(19) Europäisches Patentamt
European Patent Office
Office européen des brevets

(11) EP 4 667 464 A1

(12) **EUROPEAN PATENT APPLICATION**
published in accordance with Art. 153(4) EPC

(43) Date of publication:
**24.12.2025 Bulletin 2025/52**

(21) Application number: **24757342.1**

(22) Date of filing: **16.02.2024**

(51) International Patent Classification (IPC):
***C07D 401/14*** *(2006.01)* ***A61K 31/501*** *(2006.01)*
***A61K 31/444*** *(2006.01)* ***A61P 35/00*** *(2006.01)*

(52) Cooperative Patent Classification (CPC):
**A61K 31/444; A61K 31/501; A61P 35/00; C07D 401/14; C07D 403/14**

(86) International application number:
**PCT/KR2024/095359**

(87) International publication number:
**WO 2024/172632 (22.08.2024 Gazette 2024/34)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **16.02.2023 KR 20230020903**

(71) Applicants:
- **Cyrus Therapeutics Inc.**
  **Seoul 05836 (KR)**
- **Kanaph Therapeutics Inc.**
  **Seoul 04348 (KR)**

(72) Inventors:
- **HAN, Wooseok**
  **Hanam-si Gyeonggi-do 13015 (KR)**
- **NAM, Joonwoo**
  **Seongnam-si Gyeonggi-do 13527 (KR)**
- **YU, Jihyun**
  **Yongin-si Gyeonggi-do 16830 (KR)**
- **KIM, Ji Eun**
  **Anyang-si Gyeonggi-do 14044 (KR)**
- **KI, Dong Hyuk**
  **Gwacheon-si Gyeonggi-do 13839 (KR)**
- **KIM, Eun-Jung**
  **Seoul 06788 (KR)**
- **KIM, Sungeun**
  **Seoul 04385 (KR)**
- **CHOI, Hunmi**
  **Seoul 04712 (KR)**
- **YU, Ha Na**
  **Gwacheon-si Gyeonggi-do 13826 (KR)**
- **JO, Seongin**
  **Gwangmyeong-si Gyeonggi-do 14202 (KR)**
- **JEON, Yeejin**
  **Gwangmyeong-si Gyeonggi-do 14295 (KR)**
- **KIM, Donggeon**
  **Seoul 05629 (KR)**
- **CHOI, Sungpil**
  **Anyang-si Gyeonggi-do 14074 (KR)**

(74) Representative: **HGF**
**HGF Limited**
**4th Floor, 1 City Square**
**Leeds LS1 2AL (GB)**

(54) **AZOLYLPYRIDINE PYRIDAZINONE AMIDE AS SOS1 INHIBITOR**

(57) There is provided a novel compound of Formula I and a use thereof for the prevention or treatment of a disease associated with SOS1. In one aspect of the present invention, the novel compound is useful for the prevention or treatment of a SOS1 mediated disease such as cancer and RASopathy by inhibiting the interaction between SOS1 and RAS family proteins or between SOS1 and RAC1.

[Figure 1]

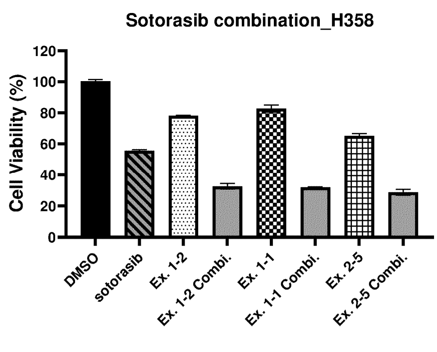


EP 4 667 464 A1

## Description

### Technical Field

The present invention relates to a novel compound having SOS1 inhibitory activity, a solvate, stereoisomer or pharmaceutically acceptable salt thereof, a pharmaceutical composition for preventing or treating a disease, comprising the same as an active ingredient, and a medicinal use thereof.

### Background Art

Mutations in the RAS gene are a major oncogene with a high incidence in human cancers, and are observed in 20 to 30% of human cancers, particularly in lung cancer, colon cancer, rectal cancer, and pancreatic cancer at high rates. RAS-family proteins include KRAS, NRAS or HRAS.

RAS proteins are small GTPases that exist in cells in either a GTP-bound or GDP-bound state, and are molecular switches that cycle between an active GTP-bound state and an inactive GDP-bound state. Mutations in the RAS gene reduce the ability of the RAS, a GTPase to hydrolyze GTP, leaving this molecular switch to maintain a constitutively active GTP-bound conformation, thereby inducing oncogenic downstream signaling (for example, Raf-MEK-ERK pathway or PI3K-PDK1-AKT pathway).

Meanwhile, binding of GTPase activating protein (GAP) such as NF1 accelerates the weak intrinsic GTPase activity of RAS proteins, thereby downregulating active RAS and returning it to an inactive form. On the other hand, binding of guanine nucleotide exchange factors (GEF) such as SOS1 promotes the release of GDP from RAS proteins and increases the GTP-bound active state.

Various studies on methods for directly or indirectly inhibiting RAS have been conducted in the prior art. However, it has been found that direct inhibition of RAS is extremely difficult due to the picomolar level of affinity of GTP for the binding site, lack of other well-defined pockets, and the fact that RAS interacts with GEFs, GAPs and effectors through the wide and flat protein-protein interaction surface, which makes difficult to apply small molecule drugs, and the like. In addition, a method of indirectly inhibiting RAS by targeting farnesyl transferase has also been attempted, but an approved drug has not yet been prepared. In view of this failure to directly or indirectly inhibit RAS, it has been generally considered to be difficult to target RAS for drug development.

Under these circumstances, a method of inhibiting RAS by inhibiting the interaction between RAS and GEF to prevent the reloading of GTP has emerged.

SOS1 (Son of Sevenless 1) is a type of guanine nucleotide exchange factor (GEF), which promotes the release of GDP from RAS family proteins to allow GTP binding, thereby regulating RAS family protein signaling. Son of Sevenless (SOS) protein exists in two isoforms, SOS1 and SOS2, and only SOS1 is phosphorylated by ERK. Growth factor-induced phosphorylation of SOS1 is mostly mediated by ERK, which phosphorylates at least four serine residues in the C-terminal region of SOS1. This suggests that SOS1 plays an important role in the regulation of negative feedback of the KRAS pathway. The SOS1 protein consists of 1333 amino acids (150 kDa). SOS1 is a multi-domain protein having two tandem N-terminal histone domains (HD) followed by a Dbl homology domain (DH), a plextrin homology domain (PH), a helical linker (HL), a RAS exchange motif (REM), a CDC25 homology domain and a C-terminal proline rich domain (PR). SOS1 has two binding sites for RAS family proteins (i.e., a catalytic site that binds GDP-binding RAS family proteins and promotes exchange of guanine nucleotides, and an allosteric site that binds GTP-binding RAS family proteins and up-regulates a catalytic site activity of SOS1) (J. Med. Chem. 2021, 64, 10, 6569-6580). Selective pharmacological inhibition of catalytic site binding of SOS1 to RAS family proteins is expected to prevent SOS 1-mediated activation of RAS-family proteins in a GTP-bound form.

Therefore, SOS1 inhibitor compounds are expected to inhibit signaling (for example, ERK phosphorylation) in cells downstream of RAS-family proteins, and thus novel SOS1 inhibitor compounds that bind to the SOS1 catalytic site and prevent binding and activation of RAS family proteins are being developed.

It has been reported that SOS1 is critically involved in mutant KRAS activation and oncogenic signaling in cancer (Current Opinion in Chemical Biology, 2021, 62: 109-118). Depletion of SOS1 levels reduced the survival of tumor cells with KRAS mutations, but no such effect was observed in KRAS wild-type cell lines. The SOS1 depletion effect cannot be rescued by the $SOS1^{F929A}$ mutation in which the catalytic site is damaged or the SOS1 mutation ($SOS1^{L687E/R688A}$) in which the GTP-KRAS binding is defective at the allosteric site, which suggests that targeting the catalytic site or the allosteric site of SOS1 may be an effective option for the treatment of KRAS mutation cancers.

In addition, SOS1 is critically involved in the activation of RAS family protein signaling in cancer through mechanisms other than mutation of RAS family proteins. SOS1 interacts with the adapter protein Grb2 to form the SOS1/Grb2 complex. The complex binds to an activated/phosphorylated receptor tyrosine kinase (for example, EGFR, ErbB2, ErbB3, ErbB4, PDGFR-A/B, FGFR1/2/3, IGF1R, INSR, ALK, ROS, TrkA, TrkB, TrkC, RET, c-MET, VEGFR1/2/3, AXL). In addition, it has been reported that SOS1 is localized to other phosphorylated cell surface receptors such as T cell

receptor (TCR), B cell receptor (BCR) and monocyte colony stimulating factor receptor, resulting in activating RAS family proteins.

Furthermore, SOS1 is a GEF for activation of the GTPase RAC1 (Ras-associated C3 botulinum toxin substrate 1). RAC1, like the RAS-family protein, is known to be involved in the pathogenesis of various cancers and other diseases.

Currently, BI-3406, BI 1701963, MRTX0902, and the like are being developed as inhibitors of SOS1 activity, but they are still in the early stages of development. Therefore, there is still a need in the art for the development of a novel compound for treating cancer by inhibiting SOS1 and a pharmaceutical composition comprising the same.

## Detailed Description of Invention

### Technical Problem

An object of the present invention is to provide a compound of Formula I, a solvate, stereoisomer or pharmaceutically acceptable salt thereof.

Another object of the present invention is to provide a pharmaceutical composition comprising a compound of Formula I, a solvate, stereoisomer or pharmaceutically acceptable salt thereof.

Another object of the present invention is to provide a method for preventing or treating a SOS1 mediated disease by administering a compound of Formula I, a solvate, stereoisomer or pharmaceutically acceptable salt thereof.

Another object of the present invention is to provide the use of a compound of Formula I, a solvate, stereoisomer or pharmaceutically acceptable salt thereof for the manufacture of a medicament for preventing or treating a SOS1 mediated disease.

### Solution to Problem

Each description and embodiment disclosed herein may also apply to each other description and embodiment. That is, all combinations of the various elements disclosed herein fall within the scope of the present application. In addition, it should not be construed that the scope of the present application is limited by the specific description set forth below.

In one aspect of the present invention, there is provided a compound of Formula I below, a solvate, stereoisomer or pharmaceutically acceptable salt thereof:

[Formula I]

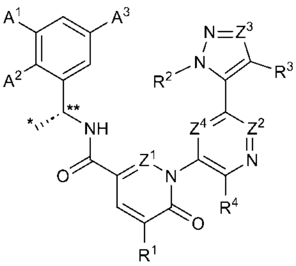

in Formula I,

- $Z^1$ and $Z^3$ are each independently N or CH;
- $Z^2$ is N or $CR^a$, and $Z^4$ is CH; or
- $Z^2$ is $CR^a$, and $Z^4$ is N;
- $R^a$ is H, halogen, OH, CN, substituted or unsubstituted $C_1$-$C_6$ alkoxy, amino, substituted or unsubstituted $C_1$-$C_6$ alkylamino, substituted or unsubstituted di($C_1$-$C_6$ alkyl)amino, substituted or unsubstituted $C_1$-$C_6$ thioalkoxy, substituted or unsubstituted $C_1$-$C_6$ alkyl, substituted or unsubstituted $C_3$-$C_7$ partially unsaturated or saturated cycloalkyl, or 4- to 7-membered heterocyclyl containing 1 or 2 heteroatoms selected from N, O and S;
- $R^1$ is H; D; halogen; OH; CN; $C_1$-$C_6$ alkyl optionally substituted with one or more deuterium, OH, oxo (=O) or halogen; $-NR^AR^B$; $-OR^A$; partially unsaturated or saturated $C_3$-$C_7$ cycloalkyl; optionally substituted $C_6$-$C_{10}$ aryl; optionally substituted 5- to 10-membered heteroaryl; or

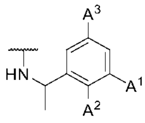

;

$R^A$ and $R^B$ are each independently $C_1$-$C_6$ alkyl optionally substituted with H or $R^a$;
$R^2$ is H; $C_1$-$C_6$ alkyl optionally substituted with one or more deuterium, OH, $C_1$-$C_6$ alkoxy or halogen; 4- to 7-membered heterocyclyl containing 1 or 2 heteroatoms selected from N, O and S, optionally substituted with one or more $R^{21}$; or $C_3$-$C_7$ partially unsaturated or saturated cycloalkyl optionally substituted with one or more $R^{21b}$;
$R^{21a}$ and $R^{21b}$ are each independently $C_1$-$C_6$ alkyl optionally substituted with one or more halogen, CN, OH, $C_1$-$C_6$ alkoxy, carboxy or oxo; $C_1$-$C_6$ acyl; carboxy; $C_1$-$C_6$ alkoxycarbonyl; or $C_1$-$C_6$ alkylsulfonyl;
$R^3$ is H, halogen, OH, $C_1$-$C_6$ alkoxy, $NH_2$, $C_1$-$C_6$ alkylamino, di($C_1$-$C_6$ alkyl)amino, $NO_2$ or CN;
$R^4$ is H, halogen, OH, $C_1$-$C_6$ alkoxy, $NH_2$, $C_1$-$C_6$ alkylamino, di($C_1$-$C_6$ alkyl)amino, $NO_2$ or CN;
$A^1$ is $C_1$-$C_6$ alkyl substituted with one or more substituents independently selected from the group consisting of deuterium, halogen, OH, $C_1$-$C_3$ alkoxy,

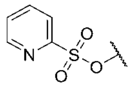

and $C_1$-$C_3$ alkyl; or partially unsaturated or saturated $C_3$-$C_7$ cycloalkyl; wherein the one or more substituents may be bonded to the same or different carbons, and two $C_1$-$C_3$ alkyl groups bonded to the same carbon atom may be optionally taken together with the carbon atom to which they are attached to form a $C_3$-$C_4$ cyclic ring;
$A^2$ is H, halogen, OH, CN or $C_1$-$C_6$ alkyl;
$A^3$ is H, amino, $C_1$-$C_6$ alkylamino or di($C_1$-$C_6$ alkyl)amino;
the carbon atoms marked with * and ** are optionally substituted with one or more deuterium atoms, provided that; the compound of Formula I is not

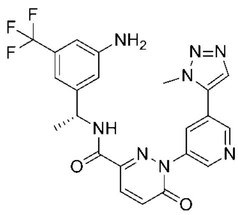

.

In Formula I above, $Z^1$ is N or CH. $Z^3$ is N or CH. In one embodiment, $Z^1$ may be N, and $Z^3$ may be N or CH. In one embodiment, $Z^1$ may be CH, and $Z^3$ may be N or CH.

In Formula I, $Z^2$ is N or $CR^a$. $Z^2$ may be $CR^a$, and $Z^4$ may be CH. $Z^2$ may be $CR^a$, and $Z^4$ may be N. Alternatively, $Z^2$ may be N, and $Z^4$ may be CH.

Said $R^a$ is H, halogen, OH, CN, substituted or unsubstituted $C_1$-$C_6$ alkoxy, amino, substituted or unsubstituted $C_1$-$C_6$ alkylamino, substituted or unsubstituted di($C_1$-$C_6$ alkyl)amino, substituted or unsubstituted $C_1$-$C_6$ thioalkoxy, substituted or unsubstituted $C_1$-$C_6$ alkyl, substituted or unsubstituted $C_3$-$C_7$ partially unsaturated or saturated cycloalkyl, or 4- to 7-membered heterocyclyl containing 1 or 2 heteroatoms selected from N, O or S. In one embodiment, $R^a$ may be H, halogen, OH, CN, $C_1$-$C_6$ alkoxy, amino, $C_1$-$C_6$ alkylamino, di($C_1$-$C_6$ alkyl)amino, or $C_1$-$C_6$ alkyl. In one embodiment, $R^a$ may be $C_3$-$C_7$ partially unsaturated or saturated cycloalkyl, or 4- to 7-membered heterocyclyl containing 1 or 2 heteroatoms selected from N, O or S. For example, the 4- to 7-membered heterocyclyl may be a 4- to 6-membered heterocyclyl containing 1 or 2 heteroatoms selected from N and O. For example, the 4- to 7-membered heterocyclyl or the 4- to 6-membered heterocyclyl may be morpholinyl, piperidinyl, piperazinyl, pyrrolidinyl, azetidinyl, or oxetanyl, but is not limited thereto. For example, the 4-to 7-membered heterocyclyl or the 4- to 6-membered heterocyclyl may be morpholinyl. In one embodiment, $R^a$ may be H, F, $-NHCH_3$, $-N(CH_3)_2$, $-OCH_3$, $-CH_3$ or

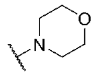

.

In Formula I above, $R^4$ is H, halogen, OH, $C_1$-$C_6$ alkoxy, $NH_2$, $C_1$-$C_6$ alkylamino, di($C_1$-$C_6$ alkyl)amino, $NO_2$ or CN. In one embodiment, $R^4$ may be H or halogen. For example, $R^4$ may be H or F.

In one embodiment, the N-containing ring bonded to the pyridazinone/pyridinone core of Formula I may not be substituted with any substituent. In one embodiment, $Z^4$ may be N, and $Z^2$ may be CH. In addition, $Z^4$ may be CH, and $Z^2$ may be N. In one embodiment, $Z^4$ may be CH, $Z^2$ may be $CR^a$, and both $R^a$ and $R^4$ may be H.

Alternatively, one or more substituents, $R^a$ and/or $R^4$ may be introduced into the N-containing ring bonded to the pyridazinone/pyridinone core of Formula I. For example, $R^a$ may be halogen, OH, CN, $C_1$-$C_6$ alkoxy, amino, $C_1$-$C_6$ alkylamino, di($C_1$-$C_6$ alkyl)amino, $C_1$-$C_6$ alkyl, $C_3$-$C_7$ partially unsaturated or saturated cycloalkyl, or 4- to 6-membered heterocyclyl containing 1 or 2 heteroatoms selected from N and O; and $R^4$ may be H or halogen. For example, $R^a$ may be H, and $R^4$ may be halogen. In one embodiment, $Z^2$ and $Z^4$ may be each CH, and $R^4$ may be H or halogen.

In Formula I above, $R^1$ is H; D; halogen; OH; CN; $C_1$-$C_6$ alkyl optionally substituted with one or more deuterium, OH, oxo (=O) or halogen; $-NR^AR^B$; $-OR^A$; partially unsaturated or saturated $C_3$-$C_7$ cycloalkyl; optionally substituted $C_6$-$C_{10}$ aryl; optionally substituted 5- to 10-membered heteroaryl; or

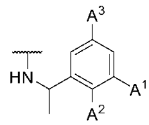

.

Said $R^A$ and $R^B$ are each independently $C_1$-$C_6$ alkyl optionally substituted with H or $R^a$. For example, $R^A$ and $R^B$ are each independently H or $C_1$-$C_6$ alkyl. Here, $A^1$, $A^2$ and $A^3$ are each as described below. In one embodiment, $R^1$ may be H; D; halogen; CN; $C_1$-$C_6$ alkyl optionally substituted with one or more of deuterium, OH, oxo (=O) or halogen; partially unsaturated or saturated $C_3$-$C_7$ cycloalkyl; $C_6$-$C_{10}$ aryl; or

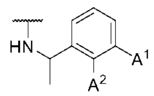

.

For example, $R^1$ may be H, D, F, Br, Cl, CN, methyl, ethyl, $CD_3$, $CF_3$, $-CH_2OH$, $-CH(OH)CH_3$, $-C(OH)(CH_3)_2$, $-C(=O)CH_3$, cyclopropyl, cyclohexyl, cyclohexenyl, phenyl, or

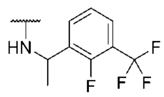

.

In one embodiment, $R^1$ may be CN. In one embodiment, $R^1$ may be $C_1$-$C_6$ alkyl substituted with OH. For example, $R^1$ may be $-CH_2OH$, $-CH(OH)CH_3$ or $-C(OH)(CH_3)_2$, but is not limited thereto. In one embodiment, $R^1$ may be $C_1$-$C_6$ alkyl substituted with halogen. For example, $R^1$ may be $CF_3$. In one embodiment, $R^1$ may be $C_1$-$C_6$ alkyl substituted with oxo (=O). For example, $R^1$ may be $-C(=O)CH_3$. In one embodiment, one or more of the carbon atoms included in $R^1$ or the carbon atom to which $R^1$ is bonded may be substituted with one or more deuterium atoms. For example, $R^1$ may be D or $CD_3$, but is not limited thereto.

In Formula I above, $R^2$ is H; $C_1$-$C_6$ alkyl optionally substituted with one or more of deuterium, OH, $C_1$-$C_6$ alkoxy or halogen; 4- to 7-membered heterocyclyl containing 1 or 2 heteroatoms selected from N, O and S, optionally substituted with one or more $R^{21a}$; or $C_3$-$C_7$ partially unsaturated or saturated cycloalkyl, optionally substituted with one or more $R^{21b}$. In one embodiment, $R^2$ may be $C_1$-$C_6$ alkyl optionally substituted with one or more of deuterium, OH, $C_1$-$C_6$ alkoxy or halogen. For example, $R^2$ may be $C_1$-$C_6$ alkyl, $C_1$-$C_6$ hydroxyalkyl, $C_1$-$C_6$ haloalkyl, $C_1$-$C_6$ alkoxy- $C_1$-$C_6$ alkyl. In this case, one or more carbon atoms included in the alkyl residue may be substituted with one or more deuterium atoms. Alternatively, $R^2$ may be 4- to 6-membered heterocyclyl containing 1 or 2 heteroatoms selected from N and O, optionally substituted with one or more $R^{21a}$. In addition, $R^2$ may be $C_3$-$C_7$ partially unsaturated or saturated cycloalkyl optionally substituted with one or more $R^{21b}$. For example, $R^2$ may be $C_3$-$C_7$ partially unsaturated or saturated cycloalkyl optionally substituted with one or more halogens (e.g., F). In one embodiment, one or more of the carbon atoms included in $R^2$ may be substituted with one or more deuterium atoms.

In the above definition of $R^2$, $R^{21a}$ and $R^{21b}$ are each independently $C_1$-$C_6$ alkyl optionally substituted with one or more of halogen, CN, OH, $C_1$-$C_6$ alkoxy, carboxy or oxo; $C_1$-$C_6$ acyl; carboxy; $C_1$-$C_6$ alkoxycarbonyl; or $C_1$-$C_6$ alkylsulfonyl. In one embodiment, $R^{21a}$ is $C_1$-$C_4$ alkyl optionally substituted with one or more of OH, $C_1$-$C_4$ alkoxy, or oxo; $C_1$-$C_4$ acyl; $C_1$-$C_4$ alkoxycarbonyl; or $C_1$-$C_4$ alkylsulfonyl. In one embodiment, $R^{21b}$ may be halogen.

In one embodiment, $R^2$ may be $C_1$-$C_6$ alkyl optionally substituted with one or more of deuterium or OH. In one embodiment, $R^2$ may be oxetanyl, azetidinyl, pyrrolidinyl, piperidinyl, morpholinyl or piperazinyl, optionally substituted with $R^{21a}$. In one embodiment, $R^2$ may be $C_3$-$C_6$ cycloalkyl optionally substituted with 1 to 3 halogens.

For example, $R^2$ may be $-CH_3$, $-CD_3$, $-CH_2CH_3$, $-CH(CH_3)_2$, $-CH_2CH_2OH$, cyclopropyl,

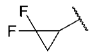, cyclobutyl, 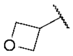, 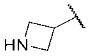, 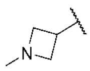, 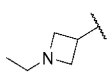, 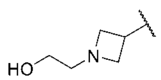, 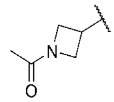,

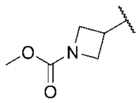, 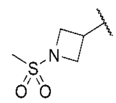, 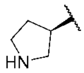, 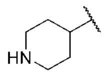, 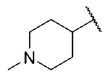, 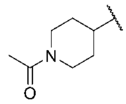, or 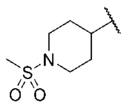,

but is not limited thereto.

In Formula I above, $R^3$ is H, halogen, OH, $C_1$-$C_6$ alkoxy, $NH_2$, $C_1$-$C_6$ alkylamino, di($C_1$-$C_6$ alkyl)amino, $NO_2$ or CN. In one embodiment, $R^3$ may be H or halogen. For example, $R^3$ may be H or Cl.

In one embodiment, one or more substituents, $R^2$ and/or $R^3$ may be introduced into the pyrazole/triazole ring of Formula I. For example, $R^2$ may be $C_1$-$C_6$ alkyl optionally substituted with one or more of deuterium, OH, $C_1$-$C_6$ alkoxy or halogen, and $R^3$ may be H. For example, $R^2$ may be $C_1$-$C_6$ alkyl optionally substituted with one or more of deuterium, OH, $C_1$-$C_6$ alkoxy, or halogen, and $R^3$ may be halogen. For example, $R^2$ may be 4- to 6-membered heterocyclyl containing 1 or 2 heteroatoms selected from N and O, optionally substituted with one or more $R^{21a}$; or $C_3$-$C_7$ partially unsaturated or saturated cycloalkyl optionally substituted with one or more $R^{21b}$; and $R^3$ may be H. For example, $R^2$ is 4- to 6-membered heterocyclyl containing 1 or 2 heteroatoms selected from N and O, optionally substituted with one or more $R^{21a}$; or $C_3$-$C_7$ partially unsaturated or saturated cycloalkyl optionally substituted with one or more $R^{21b}$; and $R^3$ may be halogen. In this case, $R^{21a}$ and $R^{21b}$ are as described above, and particular embodiments of $R^2$ and $R^3$ described above may be applied in the same manner.

In Formula I above, $A^1$ is $C_1$-$C_6$ alkyl substituted with one or more substituents independently selected from the group consisting of deuterium, halogen, OH, $C_1$-$C_3$ alkoxy,

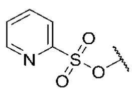

and $C_1$-$C_3$ alkyl; or partially unsaturated or saturated $C_3$-$C_7$ cycloalkyl. Said one or more substituents may be bonded to the same or different carbons. When two $C_1$-$C_3$ alkyl groups, as the one or more substituents, are bonded to the same carbon atom, they may be optionally taken together with the carbon atom to which they are attached to form a $C_3$-$C_4$ cyclic ring. In one embodiment, $A^1$ is $C_1$-$C_6$ alkyl substituted with two or more substituents selected from deuterium, F, OH, $C_1$-$C_3$ alkoxy and $C_1$-$C_3$ alkyl, wherein two of said $C_1$-$C_3$ alkyl may be attached to the same carbon atom to form a $C_3$-$C_4$ cyclic ring. For example, $A^1$ may be $-CHF_2$, $-CF_3$, $-CF_2CH_3$, $-CF_2CH_2F$, $-CF_2CH_2OH$, $-CF_2CD_2OH$, $-CF_2CH_2OCH_3$,

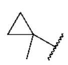, 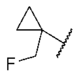,

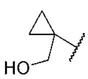 or 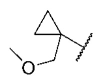,

but is not limited thereto. For example, $A^1$ may be cyclobutyl or cyclobutenyl. For example, $A^1$ may be

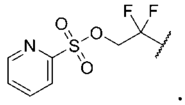.

In Formula I above, $A^2$ is H, halogen, OH, CN or $C_1$-$C_6$ alkyl. In one embodiment, $A^2$ may be H, halogen, or $C_1$-$C_4$ alkyl. For example, $A^2$ may be H, F, Cl or $-CH_3$.

In Formula I above, $A^3$ is H, amino, $C_1$-$C_6$ alkylamino or di($C_1$-$C_6$ alkyl)amino. In one embodiment, $A^3$ may be H or amino.

In one embodiment,

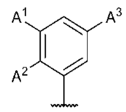

of Formula I may be selected from the following structures:

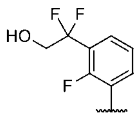
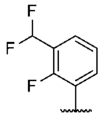
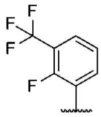
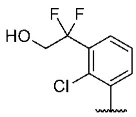
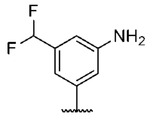
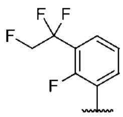
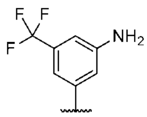

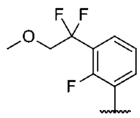
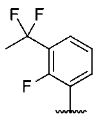
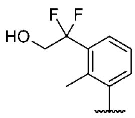
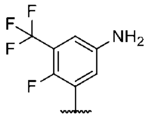
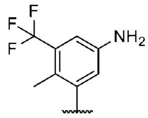
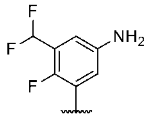
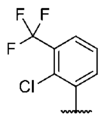

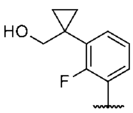
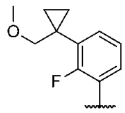
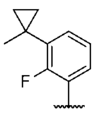
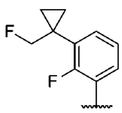
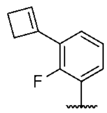
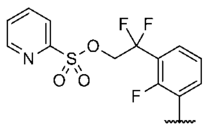
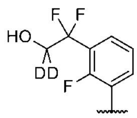 .

In a compound of Formula I according to the present invention, one or more carbon atoms may be substituted with one or more deuterium. $R^1$, $R^2$ and/or $A^1$ of Formula I may contain one or more deuterium atoms. In addition, one or more carbon atoms marked with * and ** in Formula I may be optionally substituted with one or more deuterium atoms. The deuterium substitution may provide therapeutic benefits resulting from higher metabolic stability, such as increased half-life in the body and/or reduced dosage. Even in the case where the term "deuterium" is not particularly described for hydrogen included in any other substituent, it is not excluded and should be construed to be within the scope of the present invention.

According to one embodiment, the compound represented by Formula I above may be a compound represented by Formula I-1 below:

[Formula I-1]

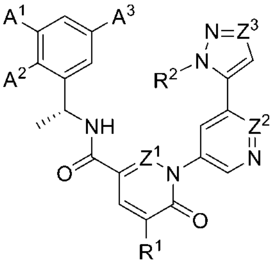

$Z^1$ and $Z^3$ are each independently N or CH;
$Z^2$ is N or $CR^a$;
$R^a$ is H, halogen, OH, CN, substituted or unsubstituted $C_1$-$C_6$ alkoxy, substituted or unsubstituted $C_1$-$C_6$ alkylamino, substituted or unsubstituted $C_1$-$C_6$ thioalkoxy, or substituted or unsubstituted $C_1$-$C_6$ alkyl;
$R^1$ is H, halogen, OH, CN, $C_1$-$C_6$ alkyl optionally substituted with one or more deuterium or halogen, $-NR^AR^B$, $-OR^A$, $C_3$-$C_6$ cycloalkyl, optionally substituted $C_6$-$C_{10}$ aryl, or optionally substituted 5- to 10-membered heteroaryl;
$R^A$ and $R^B$ are each independently $C_1$-$C_6$ alkyl optionally substituted with $R^a$;
$R^2$ is H, or $C_1$-$C_6$ alkyl optionally substituted with one or more deuterium or halogen;
$A^1$ is $C_1$-$C_6$ alkyl substituted with one or more substituents independently selected from the group consisting of halogen, OH, $C_1$-$C_3$ alkoxy and $C_1$-$C_3$ alkyl, wherein the one or more substituents may be bonded to the same or different carbons, and two $C_1$-$C_3$ alkyls bonded to the same carbon atom may be optionally taken together with the carbon atom to which they are attached to form a $C_3$-$C_4$ cyclic ring;
$A^2$ is H, halogen, OH, CN or $C_1$-$C_6$ alkyl;

$A^3$ is H or amino, provided that; the compound of Formula I-1 is not

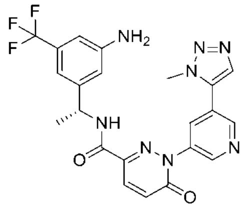

In Formula I-1, $Z^1$ and $Z^3$ are each independently N or CH.

In Formula I-1, $Z^2$ is N or $CR^a$. In this case, $R^a$ is H, halogen, OH, CN, substituted or unsubstituted $C_1$-$C_6$ alkoxy, substituted or unsubstituted $C_1$-$C_6$ alkylamino, substituted or unsubstituted $C_1$-$C_6$ thioalkoxy, or substituted or unsubstituted $C_1$-$C_6$ alkyl. For example, $R^a$ may be H, halogen, OH, CN, or $C_1$-$C_6$ alkyl. For example, $R^a$ may be H, halogen or $C_1$-$C_3$ alkyl. For example, $R^a$ may be H, F, $-CH_3$.

According to one embodiment, in Formula I-1 of the present invention, $Z^1$ may be N, $Z^2$ may be $CR^a$, and $Z^3$ may be N or CH. In this case, $R^a$ is H, halogen, OH, CN, substituted or unsubstituted $C_1$-$C_6$ alkoxy, substituted or unsubstituted $C_1$-$C_6$ alkylamino, substituted or unsubstituted $C_1$-$C_6$ thioalkoxy, or substituted or unsubstituted $C_1$-$C_6$ alkyl. For example, $R^a$ may be H, halogen, OH, CN, or $C_1$-$C_6$ alkyl. For example, $R^a$ may be H. For example, $Z^1$ may be N, $Z^2$ may be CH, and $Z^3$ may be N. In addition, $Z^1$ may be N, $Z^2$ may be CH, and $Z^3$ may be CH.

According to one embodiment, in Formula I-1 of the present invention, $Z^1$ may be CH, $Z^2$ may be $CR^a$, and $Z^3$ may be N or CH. In this case, $R^a$ is H, halogen, OH, CN, substituted or unsubstituted $C_1$-$C_6$ alkoxy, substituted or unsubstituted $C_1$-$C_6$ alkylamino, substituted or unsubstituted $C_1$-$C_6$ thioalkoxy, or substituted or unsubstituted $C_1$-$C_6$ alkyl. For example, $R^a$ may be H, halogen, OH, CN, or $C_1$-$C_6$ alkyl. For example, $R^a$ may be H. For example, $Z^1$ may be CH, $Z^2$ may be CH, and $Z^3$ may be N. In addition, all of $Z^1$, $Z^2$ and $Z^3$ may be CH.

According to one embodiment, in Formula I-1 of the present invention, both $Z^1$ and $Z^2$ may be N, and $Z^3$ may be CH. In addition, all of $Z^1$, $Z^2$ and $Z^3$ may be N.

In Formula I-1 of the present invention, $R^1$ is H, halogen, OH, CN, $C_1$-$C_6$ alkyl optionally substituted with one or more deuterium or halogen, $-NR^AR^B$, $-OR^A$, $C_3$-$C_6$ cycloalkyl, optionally substituted $C_6$-$C_{10}$ aryl, or optionally substituted 5- to 10-membered heteroaryl. In this case, $R^A$ and $R^B$ may be each independently $C_1$-$C_6$ alkyl optionally substituted with $R^a$, and the $R^a$ are as described above. For example, $R^a$ may be H, halogen, OH, CN, or $C_1$-$C_6$ alkyl.

In one embodiment, $R^1$ may be H, halogen, OH, CN, $C_1$-$C_6$ alkyl optionally substituted with one or more of deuterium or halogen, or $C_3$-$C_6$ cycloalkyl. In one embodiment, $R^1$ may be H, halogen, CN, $C_1$-$C_3$ alkyl optionally substituted with one or more of deuterium or halogen, or $C_3$-$C_5$ cycloalkyl. For example, $R^1$ may be H, F, Cl, Br, CN, methyl or cyclopropyl.

In Formula I-1 of the present invention, $R^2$ is H or $C_1$-$C_6$ alkyl optionally substituted with one or more deuterium. In one embodiment, $R^2$ may be $C_1$-$C_3$ alkyl optionally substituted with one or more deuterium atoms. For example, $R^2$ may be $-CH_3$, $-CD_3$ or $-CH_2CH_3$.

When $R^1$ and $R^2$ are alkyl substituted with one or more deuterium atoms, it may be preferable in terms of providing therapeutic benefits resulting from higher metabolic stability, such as increased half-life in the body and/or reduced dosage. In Formula I-1 of the present invention, even in the case where the term "deuterium" is not particularly described for hydrogen included in the substituents other than $R^1$ and $R^2$, it is not excluded and should be construed to be within the scope of the present invention.

In Formula I-1 of the present invention, $A^1$ is $C_1$-$C_6$ alkyl substituted with one or more substituents selected from the group consisting of halogen, OH and $C_1$-$C_3$ alkoxy. The number of substituents in $A^1$ is not limited as long as the compound is chemically stable and exhibits the desired activity. For example, $C_1$-$C_6$ alkyl may be substituted with 1, 2, 3, 4 or 5 substituents. Said one or more substituents may be different from or the same as each other. In addition, said one or more substituents may be bonded to the same or different carbons. For example, when $A^1$ is ethyl substituted with two F and one OH, two F may be bonded to the same carbon, and one OH may be bonded to a different carbon to form $-CF_2CH_2OH$. Two $C_1$-$C_3$ alkyl groups bonded to the same carbon atom may be optionally taken together with the carbon atom to which they are attached to form a $C_3$-$C_4$ cyclic ring.

According to one embodiment, in Formula I-1 of the present invention, $A^1$ may be $C_1$-$C_3$ alkyl substituted with two or more substituents independently selected from the group consisting of F, OH and methoxy. For example, said $C_1$-$C_3$ alkyl may be methyl or ethyl, and may have two F substituted on the same carbon atom, and one F, OH or methoxy optionally substituted on the same or different carbon atom. For example, $A^1$ may be $-CHF_2$, $-CF_3$, $- CF_2CH_3$, $-CF_2CH_2F$, $-CF_2CH_2OH$, and $-CF_2CH_2OCH_3$.

In Formula I-1 of the present invention, $A^2$ is H, halogen, OH, CN or $C_1$-$C_6$ alkyl. According to one embodiment, $A^2$ may be H, halogen, or $C_1$-$C_3$ alkyl. For example, $A^2$ may be H, F, Cl or $-CH_3$.

In Formula I-1 of the present invention, $A^3$ may be H or amino. In one embodiment, $A^3$ may be H. Alternatively, $A^3$ may be amino.

In one embodiment, in Formula I-1 of the present invention, $A^1$ may be $C_1$-$C_6$ alkyl substituted with one or more substituents independently selected from the group consisting of halogen, OH and $C_1$-$C_3$ alkoxy, and $A^2$ may be H, halogen, OH, CN or $C_1$-$C_6$ alkyl, and $A^3$ may be H. In this case, $A^1$ may be $C_1$-$C_3$ alkyl substituted with two or more substituents independently selected from the group consisting of F, OH and methoxy. For example, $A^1$ may be $-CHF_2$, $-CF_3$, $-CF_2CH_3$, $-CF_2CH_2F$, $-CF_2CH_2OH$, and $-CF_2CH_2OCH_3$. In this case, $A^2$ may be H, halogen, or $C_1$-$C_3$ alkyl. For example, $A^2$ may be H, F, Cl or $-CH_3$.

According to one embodiment, in Formula I-1 of the present invention, $A^1$ may be $C_1$-$C_3$ alkyl substituted with two or more F, and $A^2$ may be H, halogen or $C_1$-$C_3$ alkyl, and $A^3$ may be amino. In this case, $A^1$ may be $-CHF_2$ or $-CF_3$. In addition, said $A^2$ may be H, F or $-CH_3$.

In one embodiment, the compound of Formula I or Formula I-1 may be a compound selected from the following:

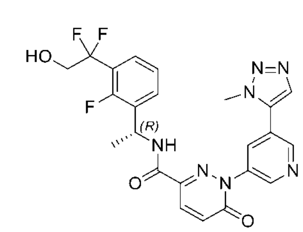
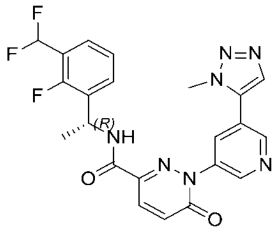
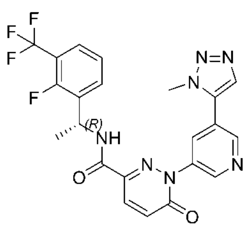
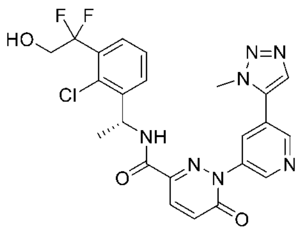

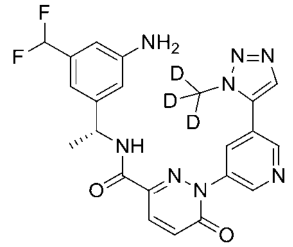
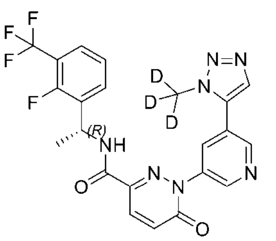
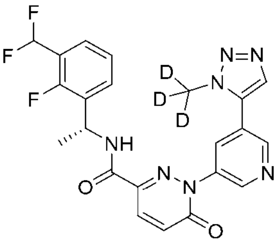
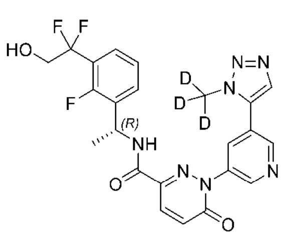

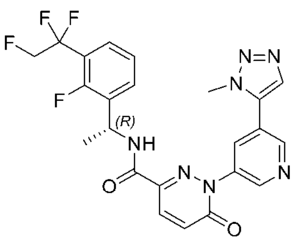
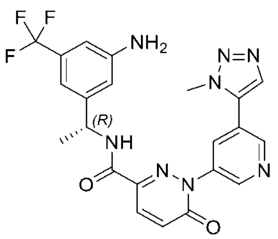
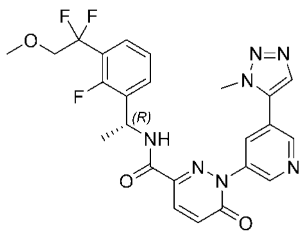
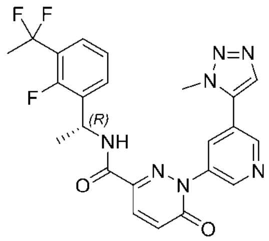

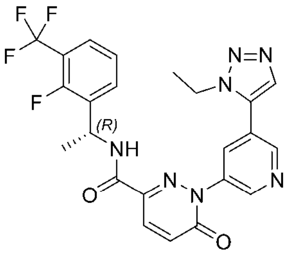
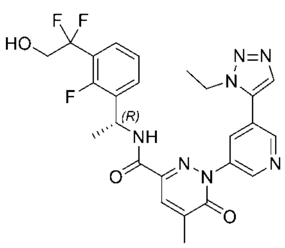
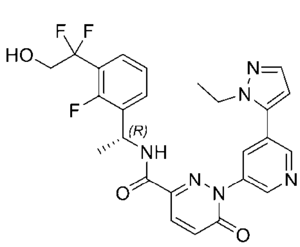
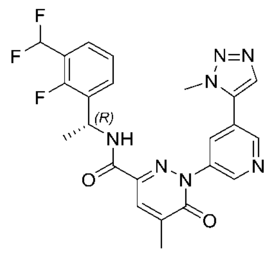

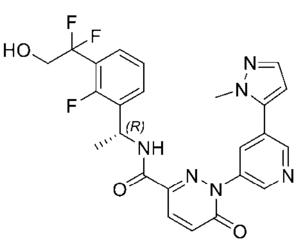
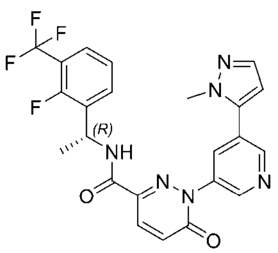
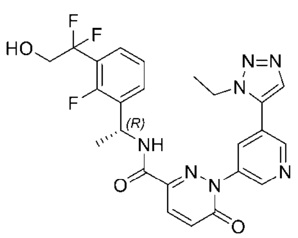
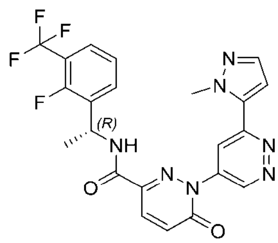

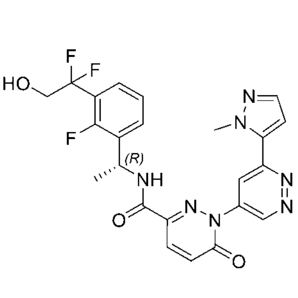
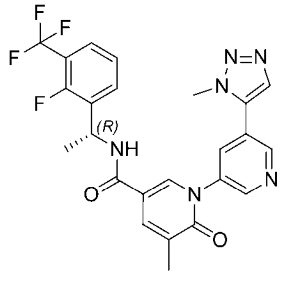
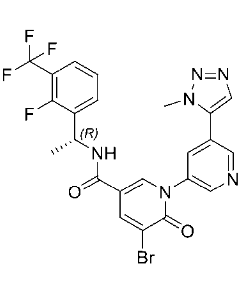
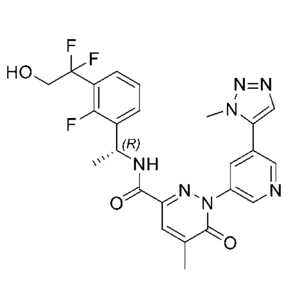

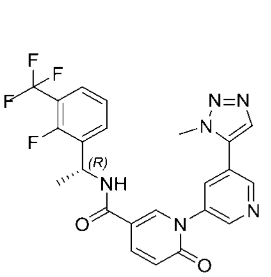
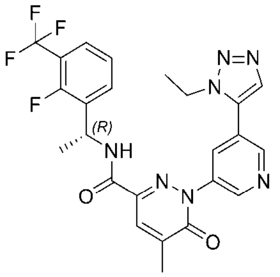
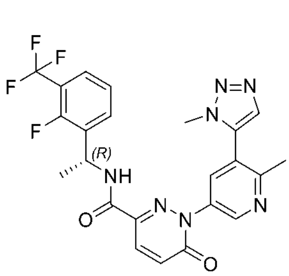
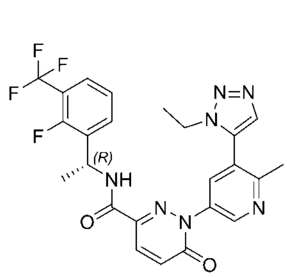

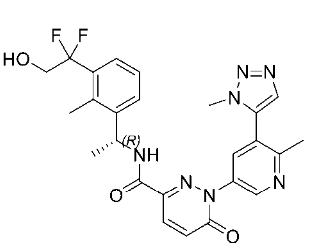
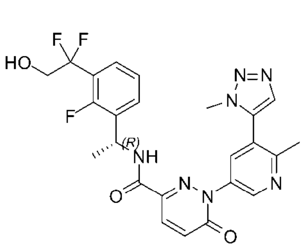
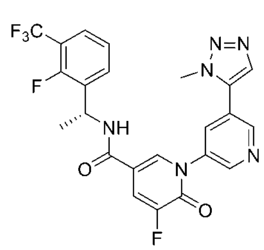
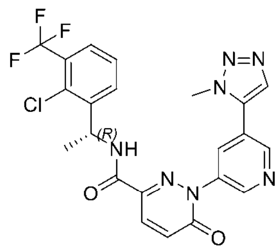

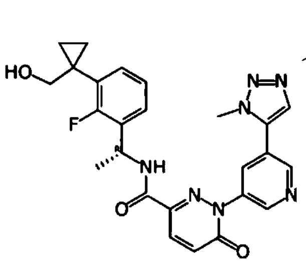
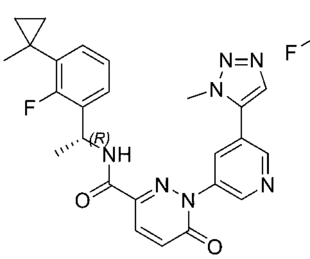
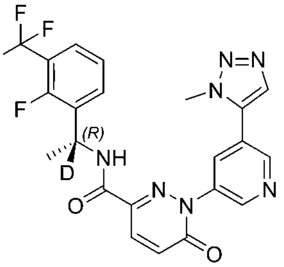
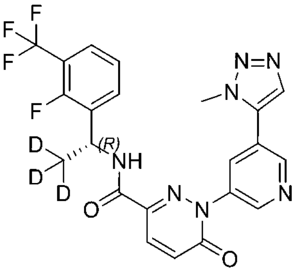

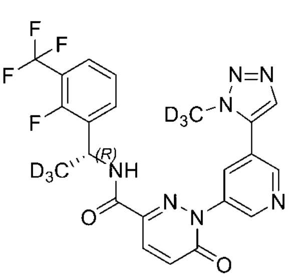
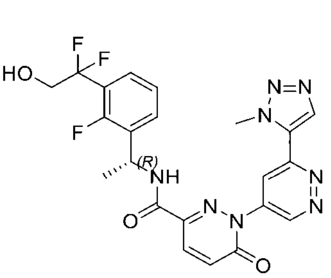
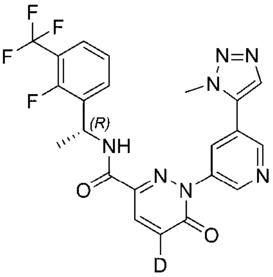
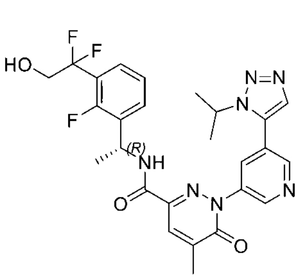

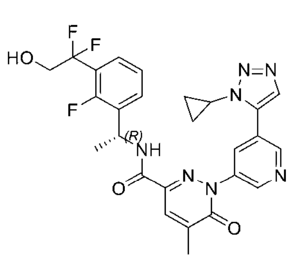
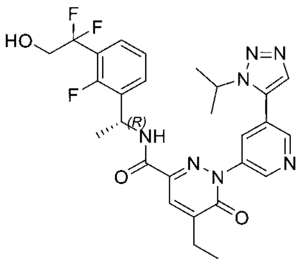
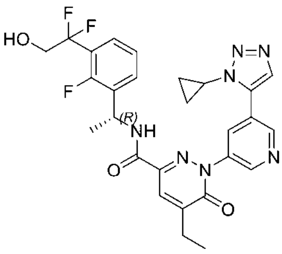
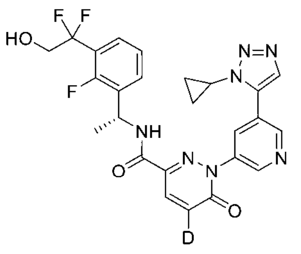

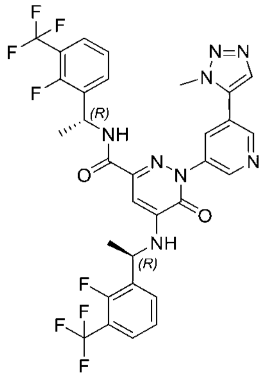
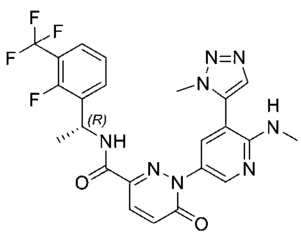
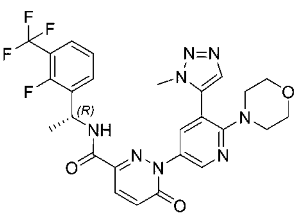
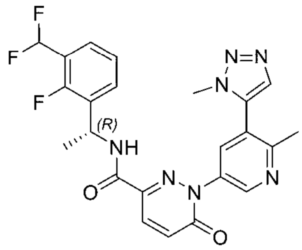

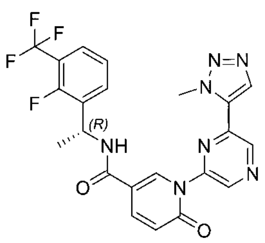
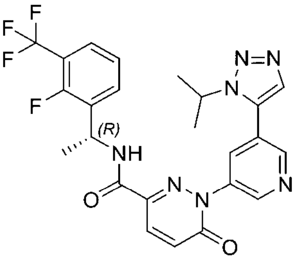
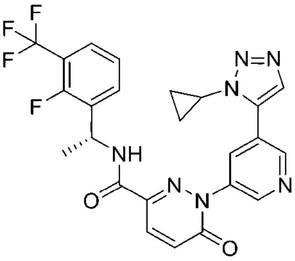
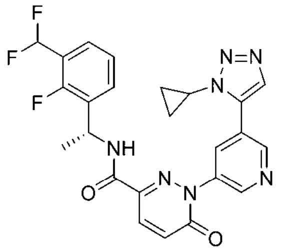

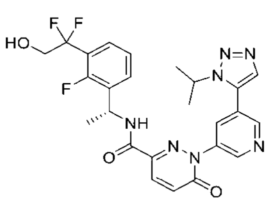
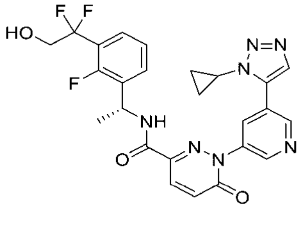
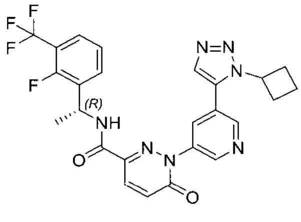
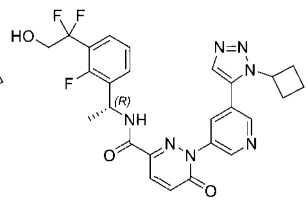

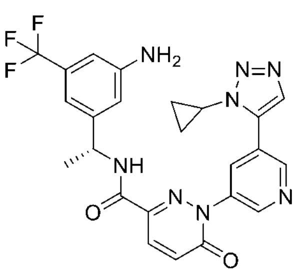
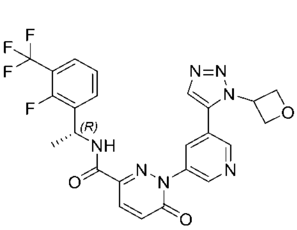
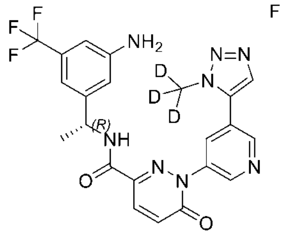
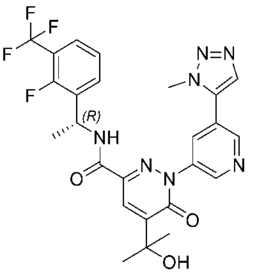

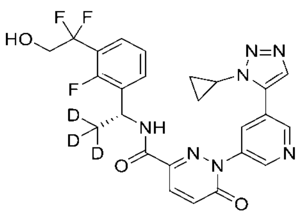
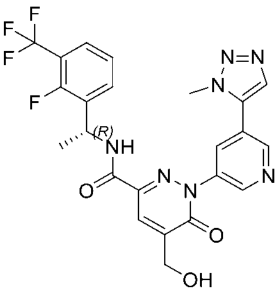
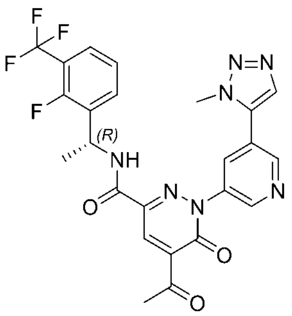
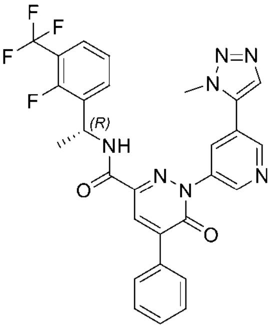

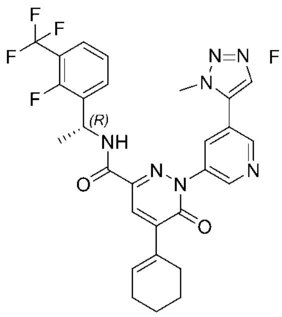
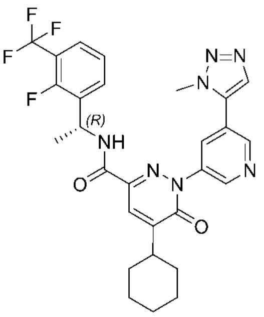
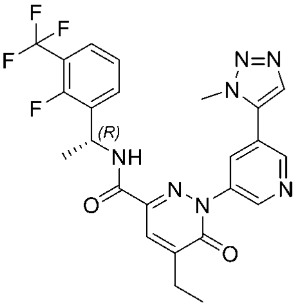
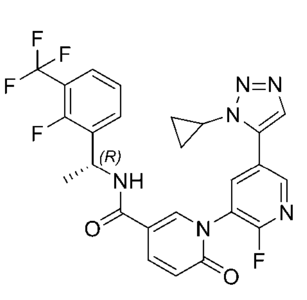

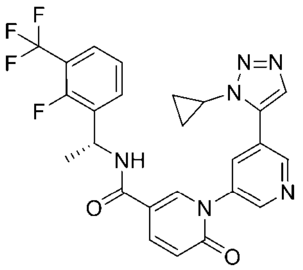
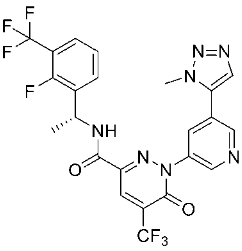
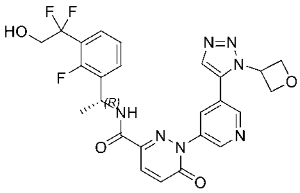
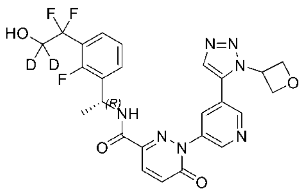

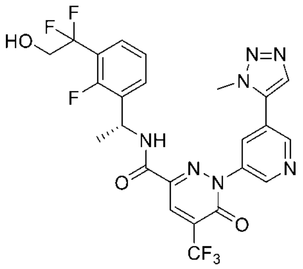
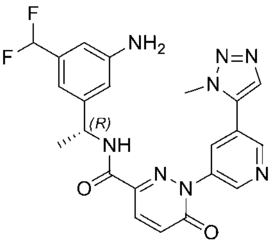
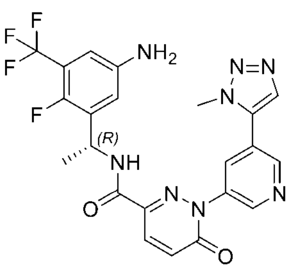
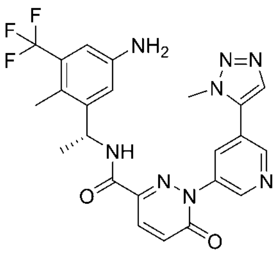

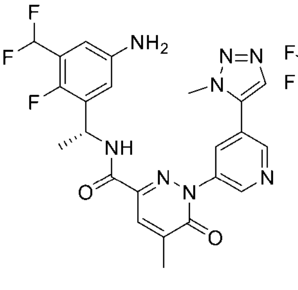
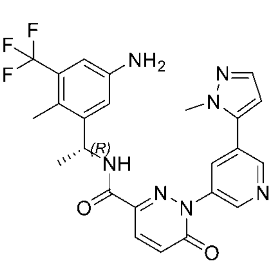
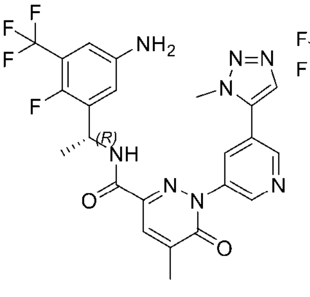
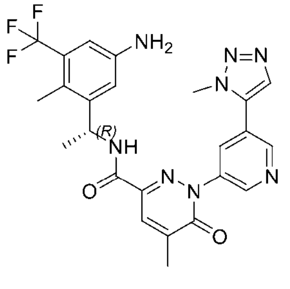

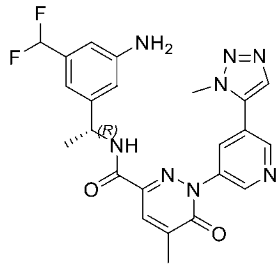
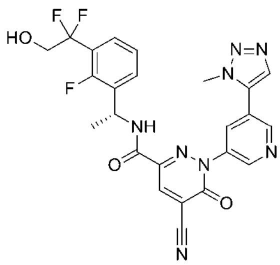
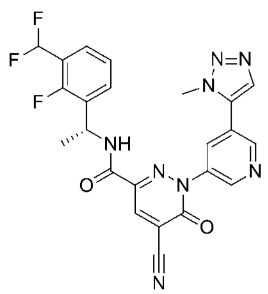
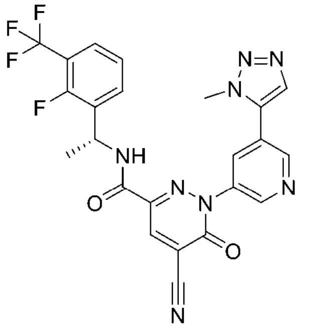

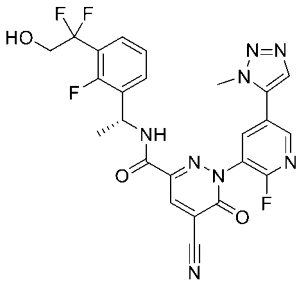
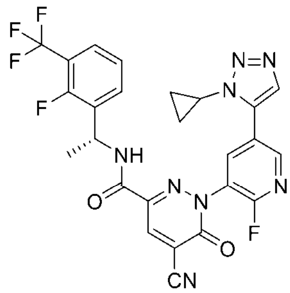
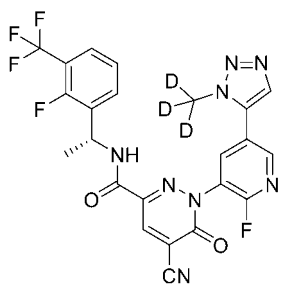
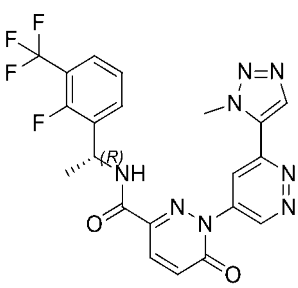

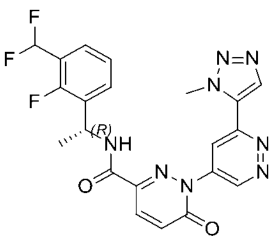
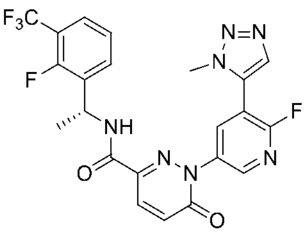
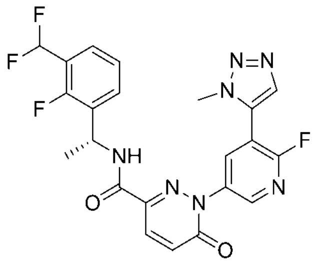
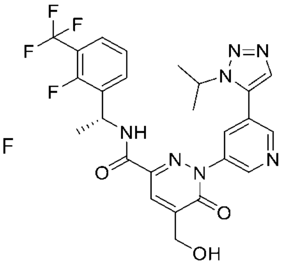

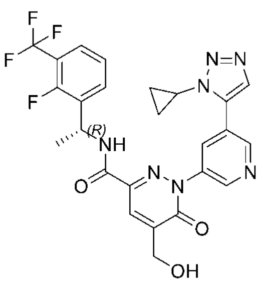
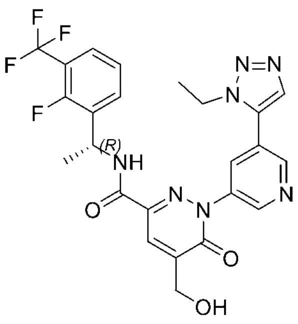
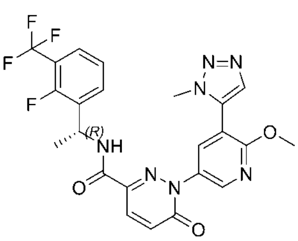
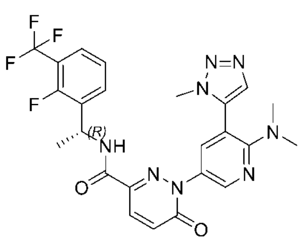

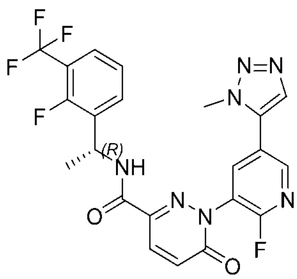
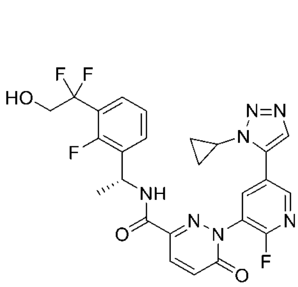
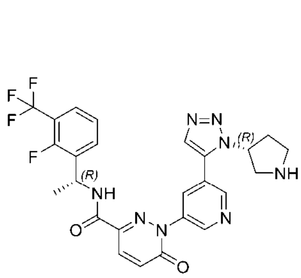
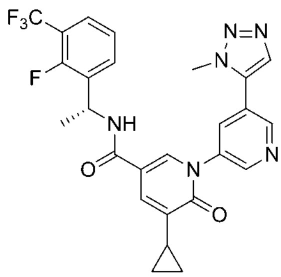

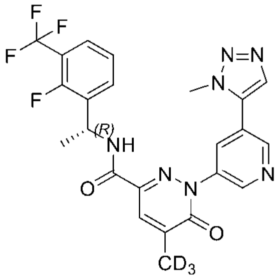
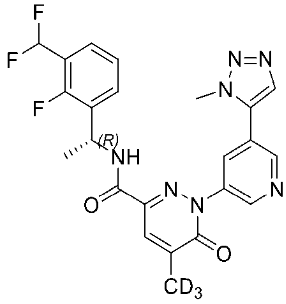
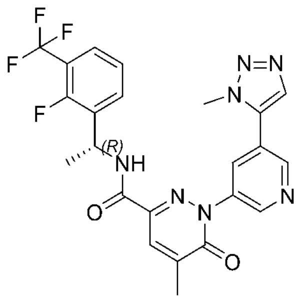
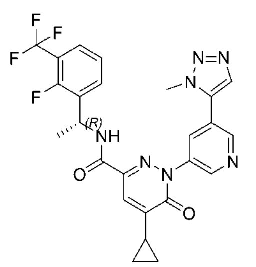
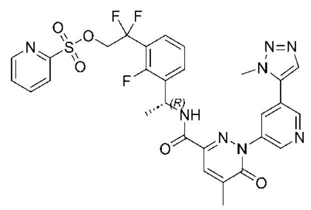
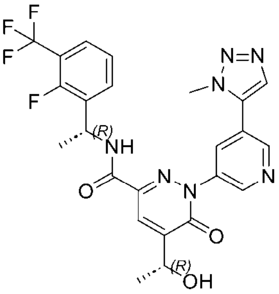
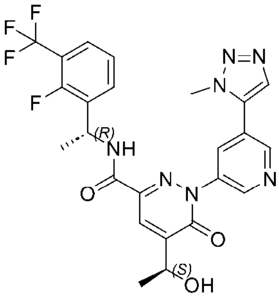
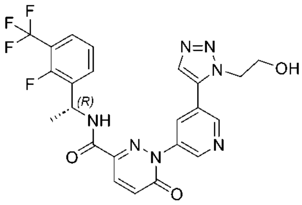
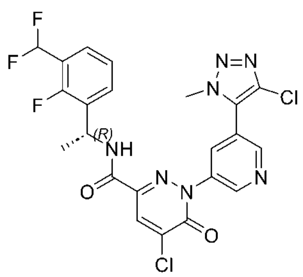
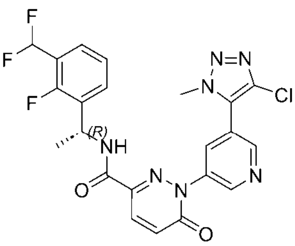
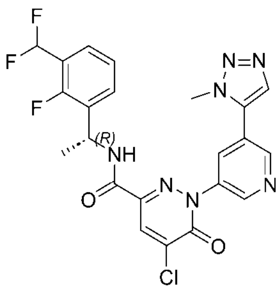
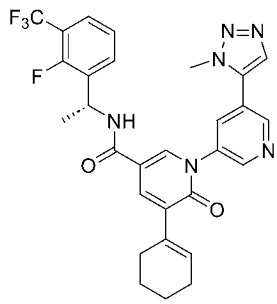
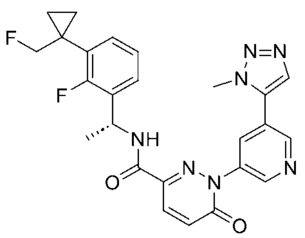
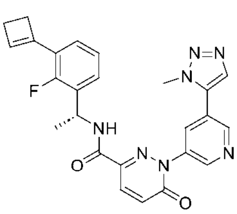
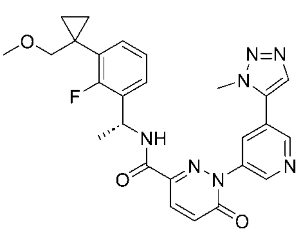
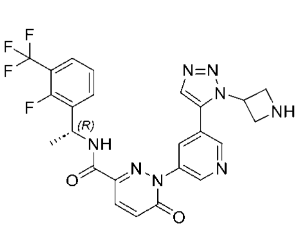
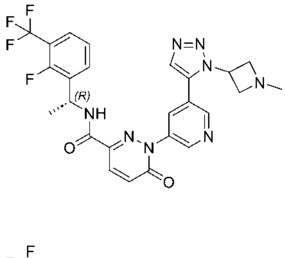
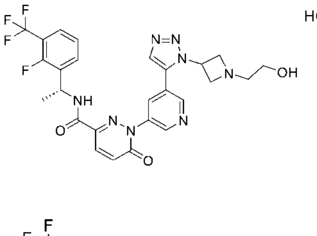
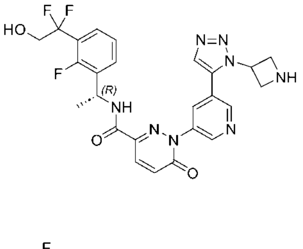
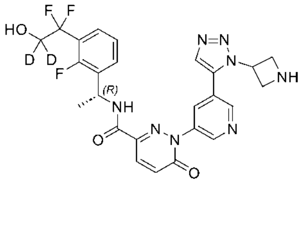
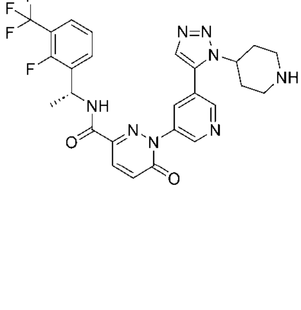
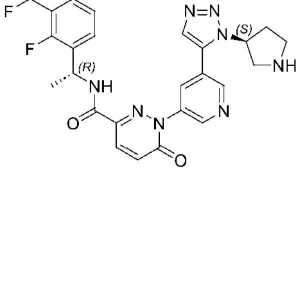
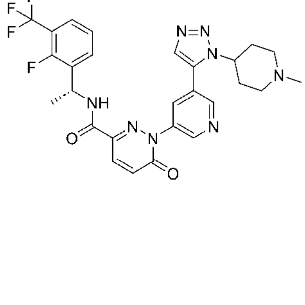
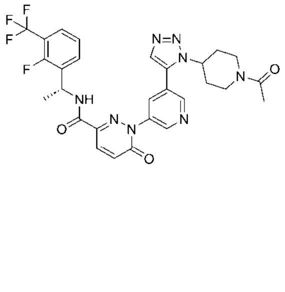

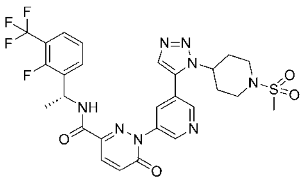
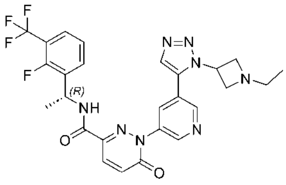
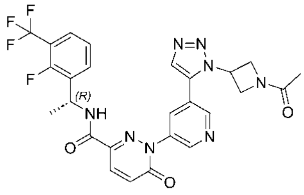
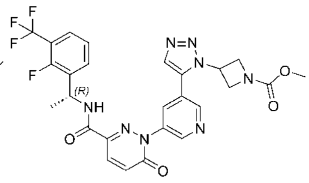

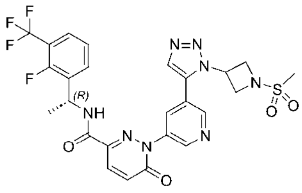
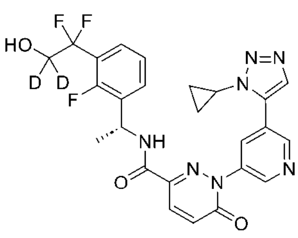
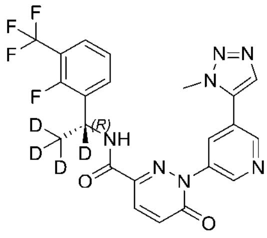
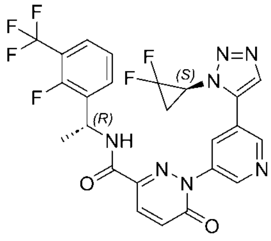

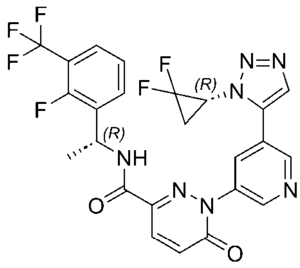
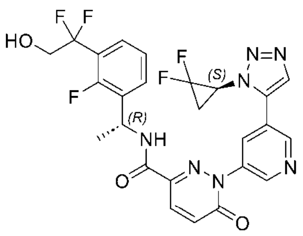
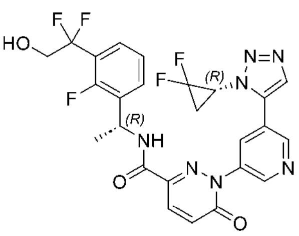
.

**Definition**

All technical and scientific terms used herein have the meanings commonly understood by one of ordinary skill in the art, and unless otherwise stated, conventional methods of measurement, methods of manufacture, conventional ingredients or substances are used based on conventional techniques such as pharmacology, pharmaceutical manufacturing chemistry, mass spectrometry, NMR, HPLC, biochemistry, and the like.

Individual features and components of each embodiment described and illustrated herein may be combined with features and components of any other embodiment without departing from the scope or spirit of the present disclosure.

Unless otherwise specified, in the present specification and the appended claims, "or" and "and" mean "and/or". The terms "include" and "included" are open-ended, and mean that a compound, composition, or method may include additional features or ingredients in addition to the listed features or ingredients. Throughout the description of the present specification and the claims, the singular encompasses the plural unless the contest requires otherwise. In particular, when an indefinite article is used, the specification is to be understood as contempolating plurality as well as singularity unless the context requires otherwise.

In the present specification, the numerical range indicated using the term "to" refers to a range including the numerical values described before and after the term "to" as the lower limit and the upper limit, respectively. In the present specification, the value expressed using the term "about" may refer to a range of $\pm$20% of the value, preferably $\pm$10% of the value.

As used herein, the term "optional" or "optionally" is intended to include that the subsequently described event or circumstance may or may not occur, and that the description includes instances where the event or circumstance occurs and instances where it does not. For example, the term "optionally substituted" is intended to include both unsubstituted or substituted with the specified substituent.

**Compound**

As used herein, the term "alkyl" refers to a fully saturated branched or unbranched (or straight chain or linear) hydrocarbon. The alkyl may be a substituted or unsubstituted alkyl group. As used herein, the term "alkyl" may refer to $C_1$-$C_6$ alkyl, $C_1$-$C_3$ alkyl, and the like. The $C_1$-$C_6$ alkyl may be a $C_1$ to $C_6$, $C_1$ to $C_5$, $C_1$ to $C_4$, $C_1$ to $C_3$, or $C_1$ to $C_2$ alkyl group. Non-limiting examples of the alkyl may be methyl, ethyl, n-propyl, isopropyl, n-butyl, isobutyl, sec-butyl, n-pentyl, isopentyl, neopentyl, iso-amyl, or n-hexyl.

As used herein, unless otherwise stated, the term "alkoxy" refers to a substituent in which a substituted or

unsubstituted straight chain or branched chain alkyl moiety is linked to another chemical structure by oxygen. As used herein, the term "alkoxy" may refer to, for example, $C_1$-$C_6$ alkoxy, $C_1$-$C_3$ alkoxy, and the like. The alkoxy may include all possible isomers thereof such as methoxy, ethoxy, propoxy, and butoxy, or isopropoxy, isobutoxy, and t-butoxy, but is not limited thereto.

As used herein, the term "thioalkoxy" refers to a substituent in which a substituted or unsubstituted straight chain or branched chain alkyl moiety is linked to another chemical structure by sulfur.

As used herein, the term "cycloalkyl" refers to a saturated hydrocarbon ring having the specified number of carbon atoms as ring elements (that is, $C_3$-$C_6$ cycloalkyl refers to a cycloalkyl group having 3, 4, 5 or 6 carbon atoms as ring elements). As used herein, the term "cycloalkyl" may refer to, for example, $C_3$-$C_6$ cycloalkyl, $C_3$-$C_5$ cycloalkyl, or $C_3$-$C_4$ cycloalkyl. The cycloalkyl may be, for example, cyclopropyl, cyclobutyl, cyclopentyl, cyclohexyl. When the cycloalkyl contains at least one double bond, it is referred to as "partially unsaturated" cycloalkyl, but does not include an aryl ring.

As used herein, the term "halogen" refers to an atom belonging to group 17 of the periodic table. The halogen atom includes fluorine, chlorine, bromine, iodine, and the like, and may be used interchangeably with the term "halo," which means a monovalent functional group composed of halogen.

As used herein, the term "cyano" refers to -CN, which is a functional group having a triple bond between a carbon atom and a nitrogen atom.

As used herein, the term "hydroxy" refers to a -OH functional group (hydroxyl group).

As used herein, the term "oxo" refers to a double bonded oxygen substituent (=O).

As used herein, the term "acyl" refers to a substituent in which any carbon of an alkyl substituent is substituted with oxo. For example, $C_1$ acyl refers to formyl (-C(=O)H), and $C_2$ acyl refers to acetyl (-C(=O)$CH_3$).

As used herein, the term "carboxy" refers to -COOH.

As used herein, the term "carbonyl" refers to a divalent functional group of -C(=O)-.

As used herein, the term "alkoxycarbonyl" refers to a substituent in which either side of carbonyl is connected to alkoxy (-C(=O)-alkoxy).

As used herein, the term "amino" refers to $-NH_2$.

As used herein, the term "alkylamino" means that one H of $-NH_2$ is substituted with alkyl.

As used herein, the term "di(alkyl)amino" means that two H of $-NH_2$ are both substituted with alkyl. The two substituted alkyls may be the same as or different from each other.

As used herein, the term "sulfonyl" refers to a divalent functional group of $-S(O)_2-$.

As used herein, the term "alkylsulfonyl" refers to a monovalent substituent ($-SO_2$-alkyl) in which either side of sulfonyl is connected to alkyl.

As used herein, the term "aryl" refers to an aromatic hydrocarbon ring group and includes groups in which the ring is fused to one or more carbon rings. For example, aryl may be a $C_6$ to $C_{10}$ aryl group. Aryl may be phenyl, naphthyl, or tetrahydronaphthyl.

As used herein, the term "heteroaryl" refers to a heterocyclic aromatic group containing at least one heteroatom selected from B, N, O, S, P(=O), Si and P as a ring-forming atom. When the heteroaryl contains N, B or P in the ring, N, B or P of the heteroaryl may be linked to another moiety. The heteroaryl may also include a form in which two or more rings are simply attached to each other (pendant) or condensed. The heteroaryl may contain 1 to 4 heteroatoms, 1 to 3 heteroatoms, 1 or 2 heteroatoms, or 1 heteroatom selected from N, O and S. The heteroaryl may contain 5 to 10, or 5 to 6 ring atoms. Examples of monocyclic heteroaryl may include thiophenyl, furanyl, pyrrolyl, pyrazolyl, thiazolyl, isothiazolyl, oxazolyl, isooxazolyl, imidazolyl, triazolyl, oxadiazolyl, thiadiazolyl, tetrazolyl, pyridinyl, pyridazinyl, pyrimidinyl, pyrazinyl, triazinyl, and the like.

As used herein, unless otherwise stated, the term "heterocyclyl" refers to a monocyclic or polycyclic, saturated or partially unsaturated ring system containing at least one heteroatom selected from B, N, O, S, Si and P and having the specified number of ring elements (that is, 3- to 7-membered heterocyclyl refers to a heterocyclyl group having 3, 4, 5, 6 or 7 ring elements, including heteroatoms). When the heterocyclyl contains N, B or P in the ring, N, B or P of the heterocyclyl may be linked to another moiety. The polycyclic heterocyclyl may also include a form in which two or more heterocyclyl rings are simply attached to each other (pendant) or bridged or condensed or spiro bonded. The heterocyclyl may contain 1 to 4 heteroatoms, 1 to 3 heteroatoms, 1 or 2 heteroatoms, or 1 heteroatom selected from N, O and S. For example, the heteroatom may be N or O. In addition, the heterocyclyl may contain 5 to 10, 4 to 7, 5 or 6 ring atoms. For example, the heterocyclyl includes morpholinyl, oxetanyl, azetidinyl, pyrrolidinyl, piperidinyl, piperazinyl, oxanyl and the like, but is not limited thereto.

As used herein, the term "deuterium" is an isotope of hydrogen with a mass number of 2, which is referred to as deuterium or the abbreviation "D." The terms "deuterated" and "substituted with deuterium" refer to a moiety or compound in which all or part of the hydrogen atoms contained in the corresponding substituent are substituted with deuterium, and they are used interchangeably.

When a derivative is formed by substituting one or more hydrogen atoms in an organic compound with another atomic group, the term "substituted" in the "substituted or unsubstituted" refers to introduction of the group in place of the

hydrogen atom and the term "substituent" refers to the introduced atomic group. Unless otherwise specified, substituents may be, for example, a halogen atom, a $C_1$-$C_6$ alkyl group substituted with a halogen atom (e.g. $CCF_3$, $CHCF_2$, $CH_2F$, $CCl_3$, etc.), $C_1$-$C_6$ alkoxy, $C_2$-$C_6$ alkoxyalkyl, hydroxyl, an amine group, an imine group, a nitro group, a cyano group, an amino group, an amidino group, hydrazine, hydrazone, a carboxyl group or salt thereof, a sulfonyl group, a sulfamoyl group, a sulfonic acid group or salt thereof, phosphoric acid or salt thereof, $C_1$-$C_6$ alkyl, $C_2$-$C_6$ alkenyl, $C_2$-$C_6$ alkynyl, a $C_1$-$C_{20}$ heteroalkyl group, a $C_6$-$C_{20}$ aryl group, a $C_6$-$C_{20}$ arylalkyl group, a $C_6$-$C_{20}$ heteroaryl group, a $C_7$-$C_{20}$ heteroarylalkyl group, a $C_6$-$C_{20}$ heteroaryloxy group, a $C_6$-$C_{20}$ heteroaryloxyalkyl group, and a $C_6$-$C_{20}$ heteroarylalkyl group.

In the present specification, when a combination of substituents is mentioned as one group, for example, arylalkyl, cycloalkylalkyl, or the like, the last-mentioned group generally contains the atom attached to the end of the molecule.

In the present specification "-" is used to indicate a position at which a substituent is bonded to the remaining moiety of the compound. For example, if - is indicated at the end of a substituent, it means that the end is attached to the remaining moiety of the compound. In addition, when two or more substituents are linked by "-," it means that the substituent immediately before "-" is bonded to a substitutable atom of the substituent immediately after "-".

As used herein, the term "solvate" may refer to a compound of the present invention or a salt thereof comprising a stoichiometric or non-stoichiometric amount of a solvent bound by non-covalent intermolecular forces. Preferred solvents therefor may be solvents that are volatile, non-toxic, and/or suitable for administration to humans. The solvent may be water, in which case the "solvate" is referred to as "hydrate."

As used herein, the term "stereoisomer" may refer to a compound of the present invention or a salt thereof that has the same chemical formula or molecular formula but is optically or sterically different, and specifically, may be a diastereomer, an enantiomer or a geometric isomer.

In some embodiments, the compound of the present invention may be in the form of a racemate, a single enantiomer, a mixture of enantiomers, a single diastereomer, a mixture of diastereomers, and the like, containing one or more asymmetric centers. In one embodiment, due to the limited rotation or nature of the asymmetric center, the compound of the present invention may be in the form of an enantiomer or a diastereomer.

When two or more asymmetric centers are present in the compound of the present invention, several diastereomers and enantiomers of the chemical structures disclosed herein may exist, and pure isomers, separated isomers, partially pure isomers, racemic mixtures or the like are all intended to fall within the scope of the present invention.

Purification of the isomers and separation of a mixture of the isomers may be achieved by standard techniques known in the art. For example, a diastereomeric mixture may be separated into its respective diastereomers by a chromatographic process or crystallization, and a racemate may be separated into its respective enantiomers by resolution or a chromatographic process on a chiral phase.

The compound of the present invention may be used in the form of a pharmaceutically acceptable salt derived from an inorganic acid or organic acid, and for example, the salt may be a salt derived from hydrochloric acid, hydrobromic acid, sulfuric acid, phosphoric acid, nitric acid, acetic acid, glycolic acid, lactic acid, pyruvic acid, malonic acid, succinic acid, glutaric acid, fumaric acid, malic acid, mandelic acid, tartaric acid, citric acid, ascorbic acid, palmitic acid, maleic acid, hydroxymaleic acid, benzoic acid, hydroxybenzoic acid, phenylacetic acid, cinnamic acid, salicylic acid, methanesulfonic acid, benzenesulfonic acid, toluenesulfonic acid, or the like.

A pharmaceutically acceptable salt of the compound may be prepared by dissolving the compound of formula I in a water-miscible organic solvent, such as acetone, methanol, ethanol, acetonitrile, or the like, and adding an excess of an organic acid or adding an aqueous acid solution of an inorganic acid, and then precipitating or crystallizing. Subsequently, after evaporating the solvent or an excess of acid from this mixture, it may be prepared by drying to obtain an addition salt or by suction filtration of the precipitated salt.

## General preparation method of compound

The compound according to the present invention can be prepared through chemical modifications well known to one of ordinary skill in the art of organic/pharmaceutical chemistry according to the method representatively shown below.

The following general reaction scheme is a general illustration of a representative preparation method of the compound of formula I. One of ordinary skill in the art will be able to easily prepare the compound of formula I by appropriately selecting a starting material, a reaction temperature, a reaction condition, a catalyst, a solvent, a treatment method, and the like suitable for the desired compound, based on the preparation methods specifically disclosed in the examples herein.

In the following reaction schemes, the designation of each substituent in a compound represented by Formula I and intermediates is the same as the substituent at the corresponding position in Formula I, unless otherwise specified. In addition, in the following reaction schemes, R' represents an alkyl group, and Hal represents a halo group (preferably Br or Cl). For convenience of explanation, each ring structure in a compound represented by Formula I is referred to separately as A-part, B-part, C-part and D-part as follows.

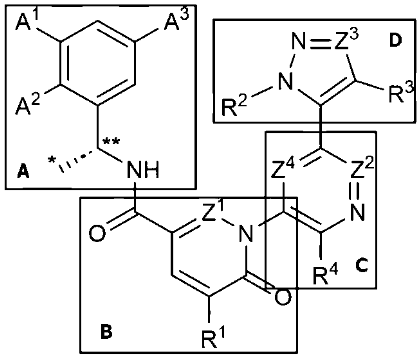


In one aspect, a compound of Formula I can be prepared according to the preparation method of Reaction Scheme I below.

[Reaction Scheme I]

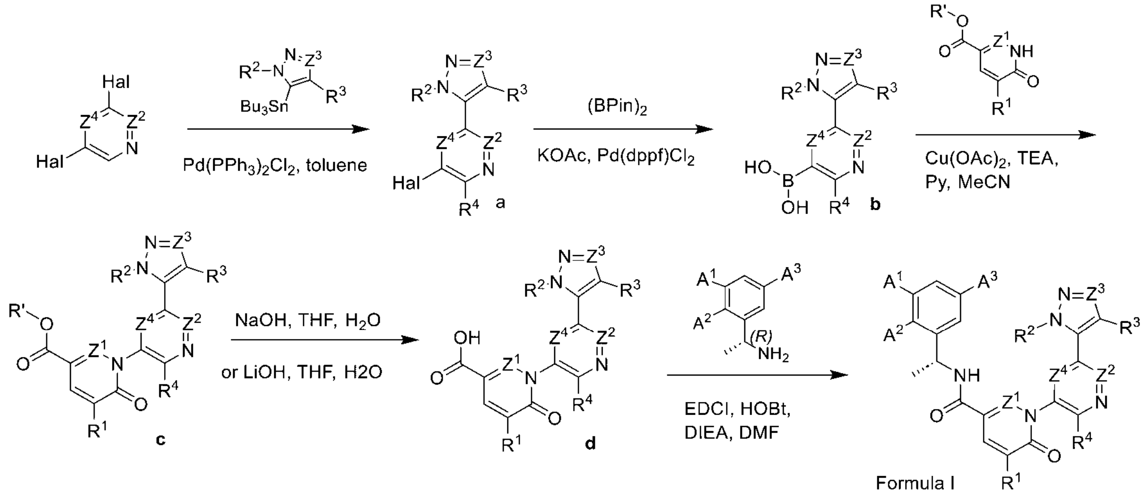


In Reaction Scheme I above, $Z^1$, $Z^2$, $Z^3$, $Z^4$, $R^1$, $R^2$, $R^3$, $R^4$, $A^1$, $A^2$ and $A^3$ are the same as in Formula I. In Reaction Scheme I, R' may be $C_1$-$C_6$ alkyl (for example, methyl or ethyl), and Hal may be halogen (for example, Br).

In Step 1 of Reaction Scheme I above, the bis-halogenated C-part intermediate and the organotin D-part intermediate can be coupled in the presence of an appropriate solvent (for example, toluene) and a Pd catalyst to prepare Intermediate a. The reaction of Step 1 can be carried out at a temperature of about 80 °C to about 120 °C, or about 90 °C to about 100 °C, for about 10 h to about 30 h, or about 15 h to about 25 h, such as 16 h or 24 h.

In Step 2, Intermediate a can be boronated using an appropriate boronating reagent (for example, bis(pinacolato) diboron) and a catalyst (for example, a Pd catalyst) to prepare Intermediate b. The reaction of Step 2 can be carried out at a temperature of about 80 °C to about 120 °C, or about 90 °C to about 100 °C, for about 10 h to about 30 h, or about 15 h to about 25 h, such as 16 h or 24 h.

In Step 3, Intermediate b can be coupled with the A-part ester intermediate in the presence of an appropriate base (for example, pyridine) and a catalyst (for example, $Cu(OAc)_2$) to prepare Intermediate c. The reaction of Step 3 can be carried out at a temperature of about 80 °C to about 120 °C, about 90 °C to about 100 °C, or about 90 °C, for about 10 h to about 30 h, or about 15 h to about 25 h, such as about 16 h.

In Step 4, the ester group of Intermediate c can be hydrolyzed using an appropriate base (for example, LiOH or NaOH) to prepare Intermediate d. The reaction of Step 4 can be carried out at a temperature of about 10 °C to about 20 °C, such as about 25 °C, for about 10 h to about 30 h, or about 15 h to about 25 h, such as about 16 h.

In Step 5, a compound represented by Formula I can be prepared by amide coupling Intermediate d with the A-part intermediate. In addition to those described in Reaction Scheme I, the amide coupling reagent can be appropriately changed as needed, and accordingly, reaction conditions such as appropriate reaction time and reaction temperature can be selected. If necessary, the amide coupling reagent can be appropriately changed to, for example, HATU, TEA, DMF, DIEA, DMAP, DCM, and the like. The amide coupling reaction of Step 5 can be carried out at about 20 °C to about 30 °C for about 2 h to about 25 h. For example, the amide coupling reaction of Reaction Scheme 1 can be carried out at about 25 °C for about 2 h to about 20 h, or about 5 h to about 16 h, or about 16 h.

Alternatively, Intermediate c in Reaction Scheme I above can be prepared according to Reaction Scheme I-1 below. The preparation method of Reaction Scheme I-1 below has a coupling sequence different from Reaction Scheme I in that the D-part intermediate is coupled after coupling the B-part intermediate and the C-part intermediate. However, the reaction reagents and reaction conditions in each step of Reaction Scheme I-1 are the same or similar to the corresponding steps in Reaction Scheme I.

[Reaction Scheme I-1]

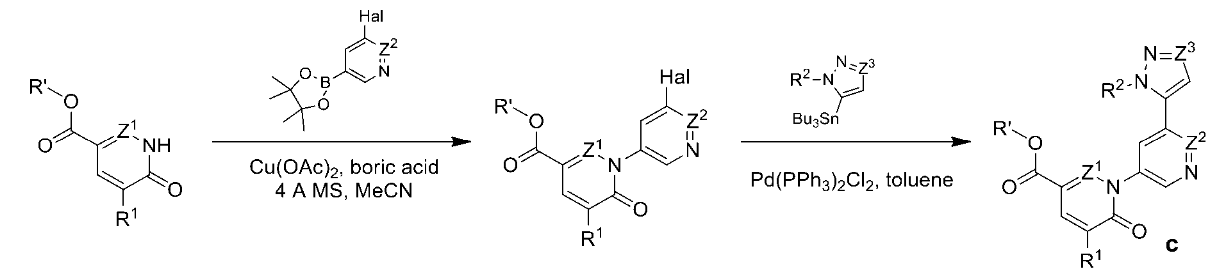


Alternatively, a compound represented by Formula I wherein $A^3$ is amino can be prepared by amide coupling the intermediate having the $NO_2$ group and Intermediate d in the same manner as in Reaction Scheme I, followed by reduction of the $NO_2$ group to the $NH_2$ group under appropriate nitro reduction conditions (for example, Fe and $NH_4Cl$) (see Reaction Scheme I-2 below).

[Reaction Scheme I-2]

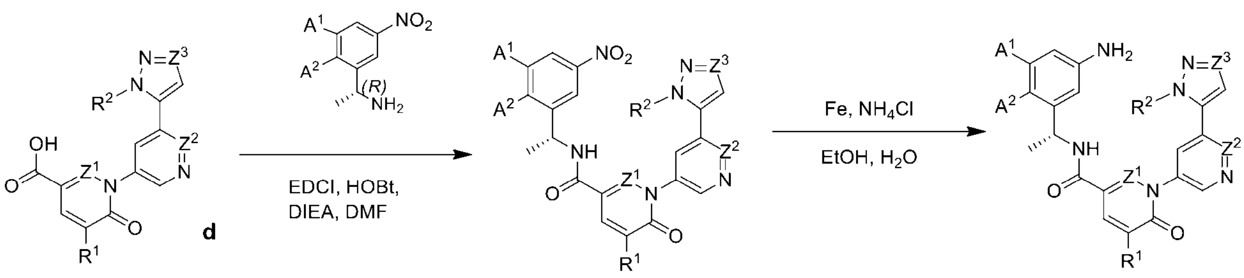


Formula I

The nitro reduction reaction can be carried out at about 60 °C to about 100 °C, or about 70 °C to about 90 °C, or about 80 °C for about 5 h to about 20 h, or about 10 h to about 15 h, or about 12 h.

Alternatively, a compound represented by Formula I wherein $R^1$ has a substituent other than halogen (for example, CN or cyclopropyl, etc.) can be prepared by introducing the desired $R^1$ group into a compound represented by Formula I wherein $R^1$ is halogen using an appropriate cyanidation reagent (for example, CuCN and NMP) or an appropriate coupling reagent (for example, $Pd(OAc)_2$, $PCy_3$, a cycloalkylboronic acid compound)

Alternatively, a compound represented by Formula I wherein $R^1$ has a substituent can be prepared by using the B-part intermediate having the corresponding substituent in Reaction Scheme I, I-1 or I-2 above.

Alternatively, Intermediate c in Reaction Scheme I can be prepared according to Reaction Scheme I-3 below.

[Reaction Scheme I-3]

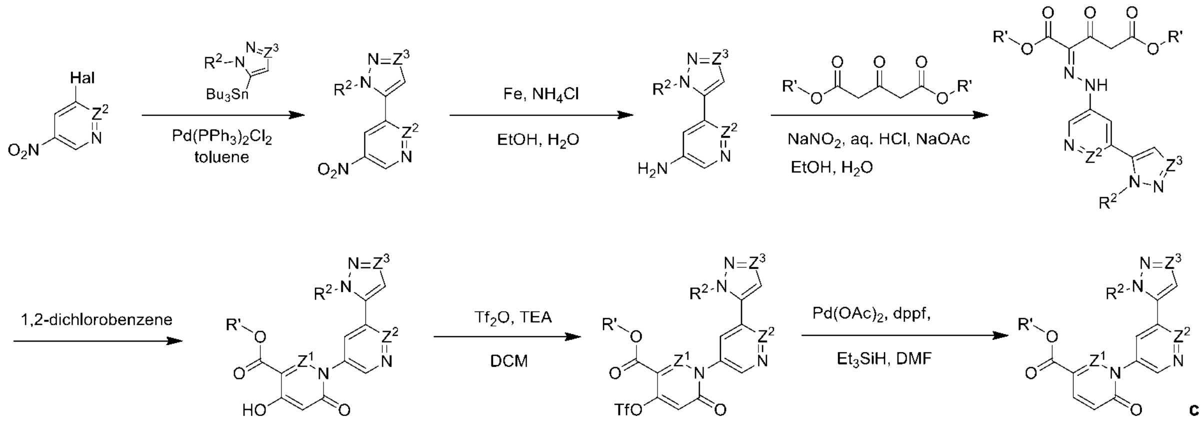

c

Step 1 of Reaction Scheme I-3 can be carried out in the same manner as in Step 1 of Reaction Scheme I except that the nitrated and halogenated C-part intermediate is used. In addition, Step 2 is a nitro reduction reaction in the same manner as in Step 2 of Reaction Scheme I-2.

In Step 3, the product of Step 2 can be reacted with 3-oxopentanedioate in the presence of an appropriate solvent and catalyst (for example, $NaNO_2$, NaOAc, aq. HCl, EtOH) to form a hydrazone compound. The reaction of Step 3 can be carried out at a temperature of about - 10 °C to about 30 °C, or about -5 °C to about 25 °C, or about 0 °C to about 20 °C for about 30 minutes to about 5 h, or about 1 h to about 2 h, or about 1 h.

In Step 4, the product of Step 3 can be heated in the presence of an appropriate solvent (for example, 1,2-dichlorobenzene) to form a dihydropyridazinone ring. The reaction of Step 4 can be carried out at a temperature of about 120 °C to about 220 °C, or about 150 °C to about 200 °C, or about 180 °C for about 5 h to about 10 h, or about 8 h.

In Step 5, $Tf_2O$ can be added to the product of Step 4 in an appropriate solvent (for example, TEA, DCM) to introduce a trifluoromethylsulfonyloxy group into the dihydropyridazinone ring. The reaction of Step 5 can be carried out at a temperature of about - 100 °C to about 50 °C, or about -80 °C to about 30 °C, or about -70 °C to about 20 °C, for about 30 minutes to about 5 h, or about 1 h to about 3 h, or about 2 h.

In Step 6, the product of Step 5 can be reacted with DPPF and $Et_3SiH$ in the presence of an appropriate catalyst (for example, $Pd(OAc)_2$) in an appropriate solvent (for example, DMF) to remove the trifluoromethylsulfonyloxy group to prepare Intermediate c. The reaction of Step 6 can be carried out at a temperature of about 50 °C to about 150 °C, or about 80 °C to about 120 °C, or about 100 °C, for about 30 minutes to about 3 h, or about 1 h to about 2 h, or about 1 h.

In one aspect, a compound represented by Formula I can be prepared as shown in Reaction Scheme IA below. The preparation method of Reaction Scheme IA below has a coupling sequence different from Reaction Scheme I and Reaction Scheme I-1 in that the A-part intermediate and the D-part intermediate are sequentially coupled after coupling the B-part intermediate and the C-part intermediate. However, the reaction reagents and reaction conditions in each step of Reaction Scheme IA are the same or similar to the corresponding steps in Reaction Scheme I or Reaction Scheme I-1.

[Reaction Scheme IA]

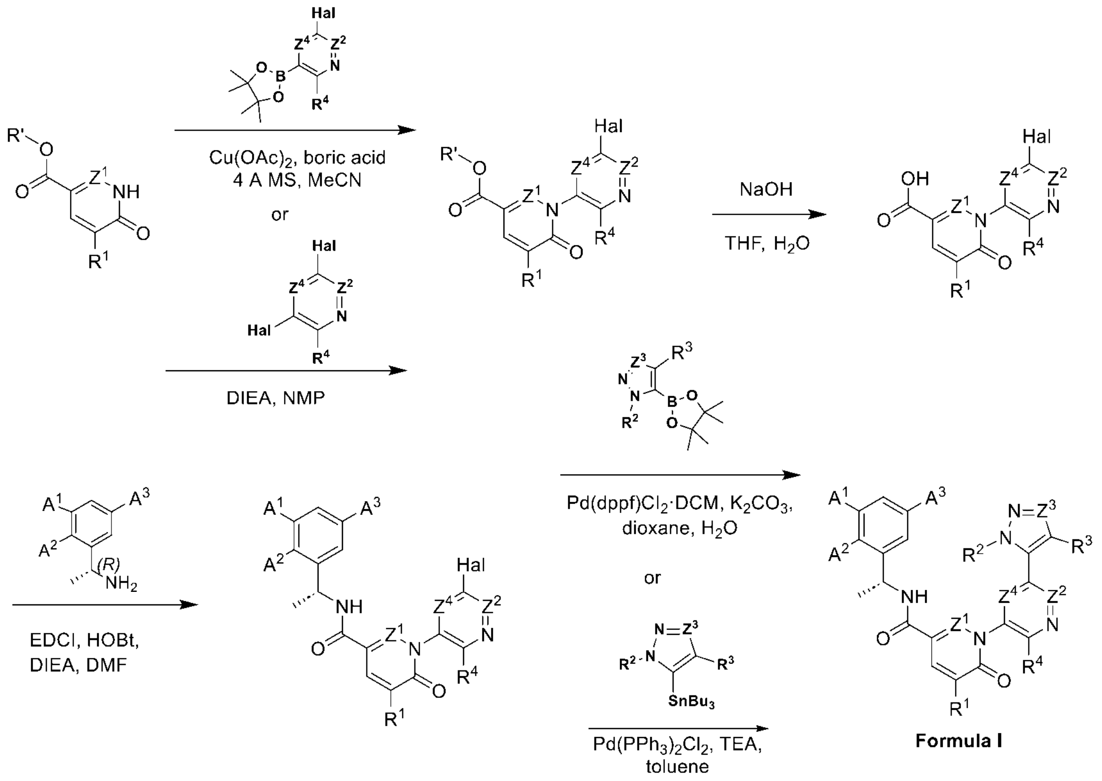

Formula I

## Medicinal use, pharmaceutical composition, and administration method

The compound of Formula I, a stereoisomer, a solvate, or a pharmaceutically acceptable salt thereof may be used for preventing or treating a SOS1 mediated disease. The compound of Formula I, a stereoisomer, a solvate, and a pharmaceutically acceptable salt thereof are as described above.

In the present specification, the term "preventing" or "prevention" refers to preventing a disease, for example, preventing a disease, condition or disorder in a subject who may be predisposed to the disease, condition or disorder but has not yet experienced or exhibited the pathology or signs of the disease.

As used herein, the term "treating" or "treatment" refers to inhibiting a disease, for example, inhibiting a disease, condition or disorder in a subject who experiences or exhibits the pathology or signs of the disease, condition or disorder, i.e., preventing recurrence of the pathology and/or signs after treatment or further development of the pathology and/or signs, or ameliorating the disease, for example, ameliorating the disease, condition or disorder in a subject who experiences or exhibits the pathology or signs of the disease, condition or disorder, i.e., reversing the pathology and/or signs, for example, reducing the severity of the disease.

The SOS1 mediated disease may include a disease that can be prevented or treated by inhibiting the interaction between SOS1 and RAS family proteins, and/or between SOS1 and RAC1. The SOS1 mediated disease may include a disease associated with the abnormal activity of SOS1 and/or RAS family proteins. The SOS1 mediated disease may be, for example, cancer. The cancer may be, for example, pancreatic cancer, lung cancer, colorectal cancer, biliary tract cancer, multiple myeloma, melanoma, uterine cancer, cervical cancer, endometrial cancer, thyroid cancer, chronic lymphocytic leukemia, acute myeloid leukemia, bladder cancer, urothelial cancer, gastric cancer, squamous cell carcinoma of the head and neck, diffuse large B cell lymphoma, esophageal cancer, hepatocellular cancer, breast cancer, ovarian cancer, prostate cancer, glioblastoma, kidney cancer or sarcoma. In one embodiment, the cancer may be pancreatic cancer, lung cancer (for example, non-small cell lung cancer), biliary tract cancer or colorectal cancer.

The cancer may be, for example, a cancer dependent on the RAS family and the MAPK signaling pathway. The cancer may include, for example, cancer having mutation of proteins or genes, gene amplification and/or overexpression in the RAS family and MAPK signaling pathway, e.g., KRAS, NRAS, HRAS, receptor tyrosine kinases (for example, EGFR, ErbB2, ErbB3, ErbB4, PDGFR-A/B, FGFR1/2/3, IGF1R, INSR, ALK, ROS, TrkA, TrkB, TrkC, RET, c-MET, VEGFR1/2/3, AXL), GAP (for example, NF1) and SOS1 (for example, mutation, amplification or overexpression of RAF, MEK). In addition, the cancer may be a RAC1 dependent cancer.

The SOS1 mediated disease may be, for example, a disease associated with dysregulation of RAS family protein pathways, i.e., RASopathy. The RASopathy may include neurofibromatosis type 1 (NF1), Noonan syndrome, Noonan syndrome with multiple lentigines (NSML, also referred to as Leopard syndrome), capillary malformation-arteriovenous

malformation syndrome (CM-AVM), Costello syndrome, CFC syndrome (Cardio-Facio-Cutaneous syndrome), Legius syndrome (also referred to as NFl-like syndrome) or hereditary gingival fibromatosis.

According to one embodiment, a compound of Formula I can be used in the treatment of a disease associated with the abnormal activity of SOS1 or RAS family proteins, or dysregulation of RAS family protein pathways by inhibiting the interaction between SOS1 and RAS family proteins, or between SOS1 and RAC1.

In one embodiment, the pharmaceutical composition may comprise conventional pharmaceutically acceptable carriers, excipients or additives. The pharmaceutical composition may be formulated according to a conventional method, and may be prepared as various oral dosage forms such as tablets, pills, powders, capsules, syrups, emulsions, microemulsions, or parenteral dosage forms such as intramuscular, intravenous or subcutaneous dosage form. The pharmaceutical composition may be a single composition or separate compositions. The pharmaceutical composition comprises the compound, stereoisomer, solvate, or pharmaceutically acceptable salt according to one aspect as an active ingredient of the pharmaceutical composition.

When the pharmaceutical composition is prepared in the form of an oral formulation, examples of additives or carriers used may include cellulose, calcium silicate, corn starch, lactose, sucrose, dextrose, calcium phosphate, stearic acid, magnesium stearate, calcium stearate, gelatin, talc, surfactant, suspending agent, emulsifying agent, diluent, and the like. When the pharmaceutical composition of the present invention is prepared in the form of an injection, the additive or carrier may include water, saline, aqueous glucose solution, similar aqueous sugar solution, alcohol, glycol, ether (for example, polyethylene glycol 400), oil, fatty acid, fatty acid ester, glyceride, surfactant, suspending agent, emulsifying agent, and the like.

The dosage of the pharmaceutical composition is an amount effective for treatment or prevention of a subject or patient, and may be administered orally or parenterally as desired. It may be administered in one to several divided doses to be administered in an amount of 0.01 to 1000 mg, more specifically 0.1 to 300 mg per kg of body weight daily based on the active ingredient when administered orally, or in an amount of 0.01 to 100 mg, more specifically 0.1 to 50 mg per kg of body weight daily based on the active ingredient when administered parenterally. The dose to be administered to a specific subject or patient should be determined in light of several related factors such as body weight, age, sex, health condition of the patient, diet, administration time, administration method, the severity of the disease, and the like, and it should be understood that it may be appropriately increased or decreased by a specialist. The above dosage is not intended to limit the scope of the present invention in any way. A physician or veterinarian of ordinary skill in the art may readily determine and prescribe the required effective amount of the pharmaceutical composition. For example, by a physician or veterinarian, a dose of the compound of the present invention used in a pharmaceutical composition may start at a level lower than that required to achieve the desired therapeutic effect, and may gradually increase until the desired effect is achieved.

In one embodiment, the pharmaceutical composition includes within its scope a pharmaceutical composition comprising, as an active ingredient, a therapeutically effective amount of at least one of the compounds according to one embodiment, alone or in combination with a pharmaceutical carrier. The term "therapeutically effective amount" or "effective amount" refers to an amount sufficient to produce a beneficial or desired clinical result, for example, an amount sufficient to alleviate, ameliorate, stabilize, reverse, slow or delay the progression of a disease.

Meanwhile, as described below, when the compound represented by Formula I or Formula I-1 according to the present invention is administered in combination with one or more other therapeutic agents, there is provided herein a pharmaceutical composition comprising the compound according to the present invention and the other therapeutic agents. Therefore, in one embodiment, there is provided a pharmaceutical composition for preventing or treating cancer, comprising a compound represented by Formula I or Formula I-1, or a solvate, stereoisomer or pharmaceutically acceptable salt thereof; and an anticancer agent as an active ingredient. The compound represented by Formula I or Formula I-1 and the anticancer agent may be included in one pharmaceutical composition or may be formulated as individual pharmaceutical compositions, respectively. The anticancer agent used in combination is as described below.

Combined administration

Optionally, the compound according to one embodiment may be administered alone, or simultaneously, separately or sequentially, in combination with the compound according to another embodiment or in combination with one or more other therapeutic agents, for example, an anticancer agent or other pharmaceutically active substances.

When used for the treatment of cancer, the compound represented by Formula I or Formula I-1 of the present invention may be administered in combination with other anticancer agents. Here, the compounds represented by Formula I and Formula I-1 are as described above.

In one embodiment, in the compound represented by Formula I according to the present invention administered in combination with the above anticancer agent, $R^2$ may be a 4-to 7-membered heterocyclyl containing 1 or 2 heteroatoms selected from N, O and S, optionally substituted with one or more $R^{21a}$; or partially unsaturated or saturated $C_3$-$C_7$ cycloalkyl optionally substituted with one or more $R^{21b}$. Here, $R^{21a}$ and $R^{21b}$ are each independently $C_1$-$C_6$ alkyl optionally

substituted with one or more halogen, CN, OH, $C_1$-$C_6$ alkoxy, carboxy or oxo; $C_1$-$C_6$ acyl; carboxy; $C_1$-$C_6$ alkoxycarbonyl; or $C_1$-$C_6$ alkylsulfonyl.

For example, in the compound represented by Formula I according to the present invention administered in combination with the above anticancer agent, $R^2$ may be a 4- to 6-membered heterocyclyl containing 1 or 2 heteroatoms selected from N and O, optionally substituted with one or more $R^{21a}$; or partially unsaturated or saturated $C_3$-$C_7$ cycloalkyl optionally substituted with one or more $R^{21b}$. Here, $R^{21a}$ may be $C_1$-$C_4$ alkyl optionally substituted with one or more OH, $C_1$-$C_4$ alkoxy or oxo; $C_1$-$C_4$ acyl; $C_1$-$C_4$ alkoxycarbonyl; or $C_1$-$C_4$ alkylsulfonyl, and $R^{21b}$ may be halogen. For example, $R^2$ may be cyclopropyl,

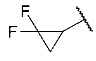,

cyclobutyl,

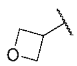, 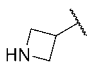, 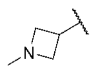, 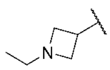, 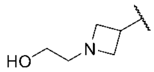, 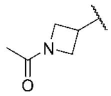, 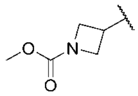, 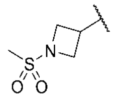,

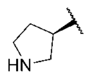, 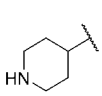, 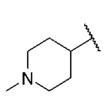, 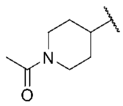, or 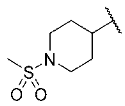.

In one embodiment, in the compound represented by Formula I according to the present invention administered in combination with the above anticancer agent, $R^1$ may be $C_1$-$C_6$ alkyl optionally substituted with one or more deuterium, OH, oxo (=O) or halogen. For example, $R^1$ may be $C_1$-$C_6$ alkyl substituted with OH. For example, $R^1$ may be $-CH_2OH$, $-CH(OH)CH_3$ or $-C(OH)(CH_3)_2$, but is not limited thereto. Alternatively, $R^1$ may be $C_1$-$C_6$ alkyl substituted with halogen. For example, $R^1$ may be $CF_3$. Alternatively, $R^1$ may be $C_1$-$C_6$ alkyl substituted with oxo (=O). For example, $R^1$ may be $-C(=O)CH_3$.

The anticancer agent that can be administered in combination with the compound represented by Formula I or Formula I-1 of the present invention may be selected from the group consisting of chemical anticancer agents, targeted anticancer agents, anticancer viruses, antibody therapeutic agents, cell therapeutic agents, immune checkpoint inhibitors, and a combination thereof.

In one embodiment, the compound represented by Formula I or Formula I-1 of the present invention may be administered alone or in combination with other anticancer therapies, such as radiation therapy, taxane derivatives (for example, paclitaxel, docetaxel), platinum compounds (for example, cisplatin, carboplatin), antimetabolites (for example, 5-FU, gemcitabine, cytarabine, 6-thioguanine), CDK4/6 inhibitors (for example, abemaciclib, palbociclib), immunotherapeutic agents (for example, anti-CTLA4 antibody, anti-PD1 antibody), angiogenesis inhibitors (for example, bevacizumab, nintedanib, regorafenib), topoisomerase inhibitors (for example, irinotecan, SN-38, doxorubicin), ERK inhibitors (for example, ulixertinib, rineterkib), MDM2 inhibitors (for example, alrizomadlin), PARP inhibitors (niraparib), MCL-1 inhibitors, mTOR inhibitors (for example, rapamycin, temsirolimus, INK-128 (sapanisertib), everolimus), BET inhibitors (JQ1), CDK9 inhibitors (for example, AT-7519), IGF1/2 or IGF1-R inhibitors (NVP-ADW742), PIK inhibitors (duvelisib), EGFR inhibitors (for example, apatinib, osimertinib, cetuximab, lazertinib, gefitinib, neratinib), ErbB2 (HER2) inhibitors (for example, trastuzumab, irbinitinib, neratinib), ALK inhibitors (for example, crizotinib, alectinib), MEK inhibitors (for example, trametinib, cobimetinib), BCR-ABL inhibitors (for example, imatinib, nilotinib, dasatinib), FGFR1, FGFR2 or FGFR3 inhibitors (for example, nintedanib), ROS1 inhibitors (for example, crizotinib, entrectinib, repotrectinib), c-MET inhibitors (for example, tepotinib), AXL inhibitors (for example, bemcentinib), NTRK1 inhibitors (for example, repotrectinib), RET inhibitors (for example, pralsetinib), KRAS G12C inhibitors (for example, sotorasib, adagrasib, trametinib, JDQ443), KRAS G12D inhibitors (for example, MRTX1133), SHP2 inhibitors (for example, TNO155), mutBRAF inhibitors (for example, dabrafenib), PI3K inhibitors (for example, alpelisib, apitolisib), Aurora A inhibitors (for example, MK-5108), pan Aurora inhibitors (for example, danusertib, AMG-900, reversine), BTK inhibitors (for example, ibrutinib), Wee1 inhibitors (for example, MK-1775), DHFR inhibitors (for example, methotrexate), HSP90 inhibitors (for example, BIIB021), A3AR antagonists (for example, reversine), ubiquitin E1 enzyme inhibitors (for example, MLN-7243), Bcl-2 inhibitors (for example, ABT-737), EZH2 inhibitors (for example, GSK-343), ARID1A inhibitors (for example, GSK-343), SUMOylation inhibitors (for example, 2-D08), CHK1 inhibitors (for example, SCH-900776), HDAC inhibitors (for example, entinostat), JAK1/2 inhibitors (for example, ruxolitinib), Proteasome inhibitors (for example, carfilzomib), AKT inhibitors (for example, ipatasertib), GR inhibitors (for example, prednisolone), PLK1 inhibitors (for example, volasertib) or pan-RAF inhibitors (for

example, sorafenib), etc.

The compound represented by Formula I or Formula I-1 of the present invention can be used together with other anticancer therapies described above to enhance the action of anticancer therapies, thereby significantly inhibiting the expression and activity of target proteins or genes. Specifically, the compound represented by Formula I or Formula I-1 not only has its own anticancer efficacy, but can also improve the anticancer efficacy of the targeted inhibitor. Therefore, when Formula I or Formula I-1 are used together with the above anticancer therapy, they can exhibit anticancer efficacy that is superior to the sum of the anticancer efficacy when they are used alone.

As used herein, the term "chemical anticancer agent" is also referred to as an antineoplastic agent or a cytotoxic agent. It is a general term for drugs that exhibit anticancer activity mainly by acting directly on DNA to block DNA replication, transcription, and translation processes, or by interfering with the synthesis of nucleic acid precursors in metabolic pathways and inhibiting cell division. The anti-tumor drug acts not only on tumor cells but also on normal cells and exhibits cytotoxicity. The chemical anticancer agent can be used for maintenance therapy. In addition, as used herein, the term "maintenance therapy" refers to treating cancer with drugs after initial anti-cancer treatment, and refers to a treatment method performed to prevent or delay the recurrence of cancer.

Specifically, chemical anticancer agent may be any one selected from the group consisting of alkylating agents, microtubule inhibitors, antimetabolites and topoisomerase inhibitors. The alkylating agent may be any one selected from the group consisting of Mechlorethamine, Cyclophosphamide, Ifosfamide, Melphalan, Chlorambucil, Thiotepa, Altretamine, Procarbazine, Busulfan, Streptozotocin, Carmustine, Lomustine, Dacarbazine, Cisplatin, Carboplatin and Oxaliplatin. The microtubule inhibitor may be any one selected from the group consisting of Docetaxel, Paclitaxel, Velban, Oncovin and Navelbine. The antimetabolite may be any one selected from the group consisting of Fluorouracil, Capecitabine, Cytarabine, Gemcitabine, Fludarabine, Methotrexate, Pemetrexed, 6-thioguanine and Mercaptopurine. The topoisomerase inhibitor may be any one selected from the group consisting of Hycamtin, Camptosar, Vepesid, Blenoxane, Adriamycin, SN-38, Doxorubicin and Cerubidine.

As used herein, the term "targeted anticancer agent" is a therapeutic agent that specifically kills cancer cells by blocking signals involved in the growth and development of cancer by targeting changes in specific proteins or specific genes that frequently appear only in cancer cells. It is classified into monoclonal antibodies that react outside cells and small molecule substances that act inside cells. Monoclonal antibodies are anticancer agents that block cancer cell-inducing signals transmitted to the outside of cells, and act on initiation signals related to proliferation, death and the like, and small molecule substances act on complex signal transduction occurring inside cells.

Specifically, a protein to be targeted may be mTOR, PI3K, EGFR, VEGFR, CD20, CD38, RNAK-L, BTK, Bcr-abl, PDGFR/FGFR family, MEK, KRAS, ERK1/2, HER2/Neu, Ubiquitin, JAK, ALK, PARP, TGFβR, Proteasome, Bcl-2, C-Met, VR1, VR2, VR3, c-kit, AXL, RET, BRAF, pan-RAF, SHP2, SRC, LCK, DNMT, CDK4/6, CDK9, BET, MDM2, IGF1/2 or IGF1-R, ROS1, NTRK1, PIK, DHFR, pan-Aurora, Aurora A, WEE1, HSP90, A3AR, EZH2, ARID1A, CHK1, ATR, HDAC1/3, AKT, PLK1, SUMOylation-related proteins, STING and the like.

The targeted anticancer agent may be any one selected from the group consisting of Rapamycin, Sirolimus, Temsilorimus, Everolimus, Ridaforolimus, INK-128, Alpelisib, Cetuximab, Trastuzumab, Pertuzumab, Gefitinib, Erlotinib, Osimertinib, Lazertinib, Panitumumab, Axitinib, Lenvatinib, Bevacizumab, Ramucirumab, Aflibercept, Rituximab, Obinutuzumab, Daratumumab, Denosumab, Ibrutinib, Dasatinib, Nilotinib, Imatinib, Bosutinib, Galunisertib, Vactosertib, Futibatinib, Nintedanib, Sunitinib, Sorafenib, Cabozantinib, Regorafenib, Masitinib, Semaxanib, Tivozanib, Vandetanib, Pazopanib, Dabrafenib, Sotorasib, Adagrasib, JDQ443, MRTX1133, Ulixertinib, Afatinib, Lapatinib, Neratinib, Lenalidomide, Ixazomib, Ruxolitinib, Lestaurtinib, Pacritinib, Trametinib, Cobimetinib, Selumetinib, Binimetinib, Alectinib, Lorlatinib, Crizotinib, Venetoclax, Bemcentinib, Gilteritinib, Selpercatinib, Pralsetinib, Encorafenib, Vemurafenib, Belvarafenib, RMC-4630, Batoprotafib, WH-4-023, Olaparib, Talazoparib, Niraparib, Rucaparib, Azacitidine, Decitabine, Guadecitabine, Abemaciclib, Ribociclib, Palbociclib, CDNs, SB11285, Rineterkib, Repotrectinib, Tepotinib, Alrizomadlin, JQ1, NVP-ADW742, Duvelisib, Irbinitinib, Danusertib, MK-1775, AMG-900, BIIB021, Reversine, MLN-7243, ABT-737, MK-5108, GSK-343, 2-D08, SCH-900776, Entinostat, Carfilzomib, Apitolisib, Ipatasertib, Volasertib, AT-7519, Methotrexate, Wortmannin, ERAS-007, PYR-41, MLN4924, RO-5503781, MK-8242, SAR-405838, CGM097, DS3032b, Lactacystin, Disulfiram, Epigallocatechin-3-gallate, Marizomib, Oprozomib, Delanzomib, Epoxomicin, MG132, Beta-hydroxy beta-methylbutyrate, Bortezomib, Navitoclax, Naporafenib, PF-07284892, TNO155, Hesperadin, LY3295668 , Tozasertib, Azenosertib, ZNL-02-096, RP-6306, GSK-1520489A, BIIB028, MPC-3100, PU-H71, Debio093, SNX-5422, AUY922, KF-26777, MRS-545, CAY10498, DZNep, EPZ005687, EI1, GSK126, UNC1999, Tazemetostat, Sinefungin, GSK-343 , Davidiin, CID9549553, SRA737, V158411, PF-477736, AZD7762, Prexasertib, Berzosertib, Gartisertib, Ceralasertib, Panobinostat, Mocetinostat, Trichostatin A, CBUD-1001, Abexinostat, VQD-002, Perifosine, Miltefosine, MK-2206, AZD5363, Rigosertib, I-BET 151, I-BET 762, OTX-015, TEN-010, CPI-203, CPI-0610, Olinone, RVX-208, ABBV-744, LY294002, AZD5153, MT-1, MS645, Figitumumab, Mecasermin, rhIGF-1, BI 885578, Buparlisib, Copanlisib, Dactolisib, Idelalisib, Parsaclisib, Paxalisib, Taselisib, Zandelisib, Inavolisib, AZD4573, Atuveciclib, VIP152, A-1592668, JSH-150, SLS009, Roscovitine and DMXAA.

As used herein, the term "mTOR (mammalian target of rapamycin)" is also referred to as mechanistic target of

rapamycin or FRAP1 (FK506 binding protein 12-rapamycin associated protein 1), and is a protein belonging to the PIKK (phosphatidylinositol 3-kinase-related kinase) family. mTOR is encoded by the FRAP1 gene in humans and is a serine/threonine protein kinase that regulates cell growth, cell proliferation, cell motility, cell survival, protein synthesis, and transcription. The mTOR inhibitor can inhibit tumor survival by inhibiting autophagy, lipogenesis, proliferation, and protein synthesis, etc. The mTOR inhibitor may be, for example, rapamycin, sirolimus, temsilorimus, everolimus, ridaforolimus or INK-128 (sapanisertib, MLN0128, TAK-228).

As used herein, the term "PI3K (Phosphoinositide 3-kinase)" is also referred to as phosphatidylinositol 3-kinase, and is an enzyme involved in cellular functions such as cell growth, proliferation, differentiation, motility, survival, and intracellular signal regulation, and is associated with cancer. PI3K includes subunits such as p110-$\alpha/\beta/\gamma/\delta$ (PI3K$\alpha/\beta/\gamma/\delta$). PI3K targeted anticancer agents may include alpelisib, wortmannin, LY294002, idelalisib, copanlisib, duvelisib, apitolisib (GDC-0980, RG7422, GNE 390), and the like.

As used herein, the term "epidermal growth factor receptor (EGFR)" is a cell membrane receptor that regulates cell growth, division, survival and death, and expression of EGFR is increased in tumor tissues in various cancers. Tumor tissues with increased EGFR are known to have high invasion, metastasis and resistance to anticancer agents. In one embodiment, a substance that inhibits the EGFR as an EGFR inhibitor may be cetuximab, trastuzumab, pertuzumab, gefitinib, erlotinib, osimertinib, lazertinib or panitumumab.

As used herein, the term "vascular endothelial growth factor receptor (VEGFR)" is a cell membrane receptor for vascular endothelial growth factor that induces angiogenesis, and a VEGFR inhibitor inhibits the angiogenesis to inhibit tumor growth and metastasis. In one embodiment, a VEGF inhibitor or a VEGFR inhibitor may be axitinib, lenvatinib, bevacizumab, ramucirumab or aflibercept.

As used herein, the term "CD20 (B lymphocyte antigen CD20)" is a protein expressed on the surface of B cells and is used as a target protein for treatment of B cell lymphoma. An inhibitor targeting CD20 may be rituximab or obinutuzumab.

As used herein, the term "CD38 (cluster of differentiation 38)" is a protein that regulates cell proliferation and death while acting as a signal transduction system receptor in immune cells, and an inhibitor targeting this protein may be daratumumab.

As used herein, the term "RNAK-L (receptor activator of nuclear factor kappa-B ligand)" is a RANK receptor expressed on the surface of osteoclasts, and when activated by binding to the ligand, it acts to cause bone destruction. A RANK-L inhibitor is mainly used for cancer patients suffering from bone metastasis or osteoporosis, and may be specifically denosumab.

As used herein, the term "BTK (Bruton's tyrosine kinase)" is an enzyme involved in the proliferation of B cells, and when overexpressed, it can develop into hematological cancer. In one embodiment, an inhibitor targeting BTK may be ibrutinib.

As used herein, the term "Bcr-abl" is a fusion protein that is highly expressed in patients with chronic myeloid leukemia, and is known to induce abnormal proliferation of blood cells. Specifically, the inhibitor of this protein may be dasatinib, nilotinib, imatinib or bosutinib.

As used herein, the term "tumor growth factor $\beta$ receptor (TGF$\beta$R)" is a cell membrane receptor for tumor growth factor, and regulates the growth, migration, differentiation and death and the like of epithelial cells and hematopoietic cells. An inhibitor targeting TGF$\beta$R may include galunisertib or vactosertib, but is not limited thereto.

As used herein, the term "PDGFR (platelet derived growth factor receptor)" is a cell membrane receptor for PDGF that is frequently expressed in cancer cells, and is known to be involved in angiogenesis to regulate cancer growth, metastasis, and drug resistance. FGFR (fibroblast growth factor receptor) is a receptor for fibroblast growth factor (FGF) and regulates various biological processes including cell growth, differentiation and migration and the like. The FGFR gene is frequently mutated, and these mutants are commonly observed in breast cancer, uterine cancer, ovarian cancer, cervical cancer, and the like. An inhibitor targeting PDGFR or FGFR may be futibatinib, nintedanib, sunitinib, imatinib, sorafenib, cabozantinib, lenvatinib, regorafenib, masitinib, semaxanib, tivozanib, vandetanib, axitinib or pazopanib.

As used herein, the term "MEK (mitogen-activated protein kinase kinase)" is a dual-specificity kinase enzyme that phosphorylates MAPK (mitogen-activated protein kinase), also referred to as MAP2K, MEK, or MAPKK. When MEK is inhibited, cell proliferation is blocked and cell death is induced. MEK targeted anticancer agents may be cobimetinib, selumetinib, trametinib or binimetinib.

As used herein, the term "KRAS (Kirsten rat sarcoma virus)" refers to a gene that produces a protein referred to as K-Ras, which is part of the RAS/MAPK pathway, and is an oncogene that instructs the growth, division, proliferation, and differentiation signals of cells. KRAS targeted anticancer agents may be sotorasib, adagrasib, JDQ443 or MRTX1133.

As used herein, the term "ERK1/2 (extracellular signal-regulated kinases 1/2)" refers to a widely expressed protein kinase intracellular signal molecule involved in functions including the regulation of meiotic, mitotic, and post-mitotic functions in cells. Disruption of the ERK pathway is commonly observed in cancer. ERK1/2 targeted anticancer agents may be rineterkib, ulixertinib (BVD-523) or ERAS-007.

As used herein, the term "HER-2/neu (human epidermal growth factor receptor 2) regulates cell proliferation by

the activation of PI3K/AKT. It is overexpressed in metastatic breast cancer and ovarian cancer and the like, and is known to induce resistance to anticancer agents. Her2/neu targeted anticancer agents may be trastuzumab, afatinib, lapatinib, irbinitinib (tucatinib) or neratinib.

As used herein, the term "ubiquitin" maintains cellular homeostasis by binding to other proteins and inducing proteolysis by proteasome, a proteolytic enzyme (ubiquitin-proteasome system, UPS). Abnormal expression or activity of the UPS is observed in various tumors, and the inhibitor of UPS exhibits anticancer activity. For example, an inhibitor targeting ubiquitin E1 enzyme may include MLN-7243 (TAK-243), PYR-41, MLN4924, and the like, and MDM2 E3 ubiquitin ligase inhibitors may include RO-5503781 (idasanutlin), MK-8242, SAR-405838, CGM097, DS3032b, and the like.

As used herein, a "proteasome inhibitor" can treat cancer by blocking the action of the proteasome, a cellular complex that degrades proteins. The inhibition of proteasome prevents the degradation of pro-apoptotic factors such as p53 protein, thereby activating programmed cell death in tumor cells that relies on inhibition of pro-apoptotic pathways. Proteasome inhibitors may include lactacystin, disulfiram, epigallocatechin-3-gallate, marizomib (salinosporamide A), oprozomib (ONX-0912), delanzomib (CEP-18770), epoxomicin, MG132, beta-hydroxy beta-methylbutyrate, bortezomib, carfilzomib, ixazomib, and the like.

As used herein, the term "JAK (Janus kinase)" is an upstream protein to STAT, which is a transcription factor that regulates cell proliferation, cell survival, cell migration and immune response, and an inhibitor of JAK is known to reduce cell proliferation and induce cell death through inhibition of STAT activity. JAK includes JAK1, JAK2, JAK3 and TYK2 (tyrosine kinase 2). An inhibitor targeting JAK may be ruxolitinib, lestaurtinib or pacritinib.

As used herein, the term "ALK (anaplastic lymphoma kinase)" is a signal transduction mediator that promotes cell proliferation, cell migration and angiogenesis and inhibits cell death, and is overactivated in various cancer tissues. An inhibitor targeting ALK may be alectinib, lorlatinib or crizotinib.

As used herein, the term "Bcl-2" is a protein that inhibits cell death, and is overexpressed or overactivated in various cancer tissues. An inhibitor targeting Bcl-2 may include venetoclax, ABT-737, navitoclax (ABT-263), and the like.

As used herein, the term "C-Met" is a receptor for hepatocyte growth factor (HGF), and activates signal transduction related to cell growth, formation, motility, survival and angiogenesis and the like. A C-Met targeted anticancer agent may be crizotinib, tepotinib or cabozantinib.

As used herein, the term "VR (vanilloid receptor) is also known as TRPV (transient receptor potential vanilloid), and exists in the form of VR1, VR2, VR3, VR4, VR5 and VR6. VR is known to regulate proliferation, death, migration, infiltration and angiogenesis of cancer cells at each stage in the cancer progression process.

As used herein, the term "c-kit" is also known as CD117, and induces signal transduction that activates cell survival, proliferation and differentiation. c-kit is a proto-oncogene, and overexpression or mutation of this gene is associated with cancer development. In one embodiment, a c-kit targeted anticancer agent may be imatinib, dasatinib or regorafenib.

As used herein, the term "AXL (tyrosine-protein kinase receptor UFO)" is a tyrosine kinase receptor present on the cell surface and mediates signal transduction involved in cell proliferation and survival. It is known to be involved in resistance to anticancer agents in anticancer treatment. In one embodiment, an AXL targeted anticancer agent may be bemcentinib or gilteritinib.

As used herein, the term "RET (rearranged during transfection)" is a receptor that mediates signals involved in cell proliferation, cell death and survival, and mutations in RET are known to be involved in cancer development. An inhibitor targeting RET may be selpercatinib or pralsetinib, but is not limited thereto.

As used herein, the term "BRAF" is a MAPK signal transduction mediator involved in cell proliferation, cell cycle regulation, cell survival, angiogenesis, cell migration, and the like, and genetic mutations are observed in cancer cells. An inhibitor targeting BRAF may be dabrafenib, encorafenib (LGX818) or vemurafenib.

As used herein, the term "pan-RAF" encompasses RAF family substances such as BRAF, ARAF, and CRAF. An inhibitor targeting pan-RAF may be naporafenib, belvarafenib or sorafenib.

As used herein, the term "SHP2 (Src homology region 2 domain-containing phosphatase-2)" is also referred to as tyrosine-protein phosphatase non-receptor type 11 (PTPN11) or protein-tyrosine phosphatase 1D/2C (PTP-1D/2C), and it is known to be a signal molecule that regulates various cellular processes, including cell growth, differentiation, mitotic cycle, and oncogenic transformation. Activation of SHP2 mutations is found in neuroblastoma, melanoma, acute myeloid leukemia, breast cancer, lung cancer, colorectal cancer, and the like. An inhibitor targeting SHP2 may include, for example, PF-07284892, RMC-4630 (SHP2-IN-7) or batoprotafib (TNO155).

As used herein, the term "SRC (proto-oncogene tyrosine-protein kinase)" refers to a non-receptor tyrosine kinase protein, also known as c-Src, that regulates embryonic development and cell growth, and an elevated level of its activity is known to be associated with cancer progression. The SRC inhibitor may be, for example, dasatinib or bosutinib.

As used herein, the term "LCK (lymphocyte-specific protein tyrosine kinase)" belongs to the SFK (Src kinase family) and activates T cell receptor signal transduction. Mutations and dysfunction of LCK inhibit T cell activation, and overexpression of LCK is known to be associated with cancer, asthma, type 1 diabetes mellitus, rheumatoid arthritis, psoriasis, systemic lupus erythematosus, inflammatory bowel disease (Crohn’s disease and ulcerative colitis), and the

like. An inhibitor targeting LCK may be, for example, WH-4-023.

As used herein, the term "PARP (poly [ADP-ribose] polymerase)" is a protein that is activated by recognition of damaged DNA in the nucleus and then activates DNA repair related proteins. An inhibitor targeting PARP inhibits the proliferation of cancer cells by inhibiting DNA repair in cancer cells. In one embodiment, an inhibitor targeting PARP may be olaparib, talazoparib, niraparib or rucaparib.

As used herein, the term "DNA methyltransferase (DNMT)" is an enzyme that attaches a methyl group to a histone protein wrapped around DNA, and through this process, the expression of the gene is inhibited. An inhibitor targeting DMNT exhibits anticancer activity by inhibiting hypermethylation of cancer suppressor genes and inducing normal expression of cancer suppressor genes. In one embodiment, an inhibitor targeting DNMT may be azacitidine, decitabine, or guadecitabine.

As used herein, the term "CDK (cyclin dependent kinase) 4/6" is a protein that promotes cell growth by regulating the cell cycle, and is overactivated during the development and progression of various malignant tumors. An inhibitor targeting CDK4/6 exhibits anticancer activity by inhibiting the cell cycle of cancer cells, inhibiting cell proliferation and inducing cell death. An inhibitor targeting CDK4/6 may be abemaciclib (LY2835219), ribociclib or palbociclib.

As used herein, the term "Aurora kinase" is a serine/threonine kinase essential for cell proliferation and refers to a phosphotransferase enzyme that helps dividing cells distribute genetic material to daughter cells. Aurora kinase plays an important role in cell division by controlling chromatid segregation, and defects in segregation can lead to tumorigenesis. Aurora A (Aurora 2) functions during mitotic prophase and is involved in the correct replication and segregation of centrosomes (microtubule-organizing centers in eukaryotic cells). Aurora B (Aurora 1) is responsible for attaching the mitotic spindle to the centromere. Aurora C (AURKC) acts in germ cells. An inhibitor targeting Aurora A may include MK-5108, hesperadin, LY3295668, and the like. An inhibitor targeting pan-Aurora may include danusertib, AMG-900, reversine, tozasertib (VX-680), and the like.

As used herein, the term "WEE1" is a 96 kDa nuclear kinase belonging to the Ser/Thr protein kinase family, also referred to as mitosis inhibitor protein kinase Wee1. Mitosis promoting factor (MPF) regulates apoptosis caused by DNA damage, and negative regulation of MPF by WEE1 causes abnormal mitosis and resistance to apoptosis caused by DNA damage. An inhibitor targeting WEE1 can reduce the sensitivity to DNA damage-induced apoptosis in cancer cells by regulating WEE1. An inhibitor targeting WEE1 may include MK-1775 (adavosertib), azenosertib (ZN-C3), ZNL-02-096, and the like.

As used herein, the term "PKMYT1 (protein kinase, membrane-associated tyrosine/threonine 1)" belongs to the Wee1 protein kinase family, and is a regulator of CDK1 phosphorylation, and is a potent therapeutic target for the treatment of certain types of DNA damage-responsive cancers through the synthetic lethality of CCNE1 amplification. An inhibitor targeting PKMYT1 may include RP-6306, GSK-1520489A, and the like.

As used herein, the term "HSP90 (heat shock protein 90)" refers to a chaperone protein that helps other proteins fold properly, stabilizes proteins from heat stress, and assists in protein degradation. It can exhibit anticancer efficacy by stabilizing a number of proteins required for tumor growth. HSP90 inhibitors may include BIIB021, BIIB028, MPC-3100, PU-H71, Debio093, SNX-5422, AUY922, and the like.

As used herein, the term "A3AR (adenosine A3 receptor; ADORA3)" refers to a G protein-coupled receptor that binds to Gi/Gq and is involved in various intracellular signaling pathways and physiological functions, and is overexpressed in pathological human cells, and can mediate cell proliferation and cell death. A therapeutic agent targeting A3AR may include reversine, KF-26777, MRS-545, CAY10498, and the like.

As used herein, the term "EZH2 (enhancer of zeste homolog 2)" refers to a histone-lysine N-methyltransferase enzyme encoded by the EZH2 gene, which participates in histone methylation and ultimately transcriptional repression. EZH2 is an attractive target for anticancer treatment because it helps cancer cells divide and proliferate, and is found in greater amounts than in healthy cells in a wide range of cancers, including breast cancer, prostate cancer, bladder cancer, uterine cancer, kidney cancer, as well as melanoma and lymphoma. An inhibitor targeting EZH2 may include DZNep, EPZ005687, EI1, GSK126, UNC1999, tazemetostat, sinefungin, and the like.

As used herein, the term "ARID1A (AT-rich interactive domain-containing protein 1A)" is a member of the SWI/SNF family, has helicase and ATPase activities, and regulates the transcription of specific genes by altering the chromatin structure around those genes. The ARID domain is a DNA binding domain that can specifically bind to AT-rich DNA sequences known to be recognized by the SWI/SNF complex at the beta-globin locus, and the C-terminus of the protein can stimulate glucocorticoid receptor-dependent transcriptional activation. Mutations in this gene are commonly found in gastric cancer, ovarian clear cell carcinoma, and pancreatic cancer. An inhibitor targeting EZH2/ARID1A may include GSK-343, and the like.

As used herein, the term "SUMOylation" is a post-translational modification that covalently attaches a small ubiquitin-like modifier (SUMO) polypeptide to a lysine residue of a target protein. The enzymatic pathway of SUMOylation is very similar to ubiquitination and includes activating enzymes, conjugation enzymes, ligases, and deconjugation enzymes. Dysregulation of the SUMOylation pathway is observed in cancer and neurological diseases, where SUMO enzymes are upregulated in many cancers and SUMO levels are directly correlated with prognosis and disease

progression. SUMOylation inhibitors may include Davidiin, CID9549553, 2-D08, and the like.

As used herein, the term "CHK1 (checkpoint kinase 1; CHEK1)" is a serine/threonine-specific protein kinase that modulates DNA damage response (DDR) and cell cycle checkpoint responses. Activation of CHK1 results in the initiation of cell cycle checkpoints, cell cycle arrest, DNA repair, and apoptosis, thereby preventing damaged cells from progressing through the cell cycle. CHK1 is overexpressed in numerous tumors, including breast cancer, colon cancer, liver cancer, gastric cancer, and nasopharyngeal cancer, and the positive correlation between CHK1 expression and tumor grade and disease recurrence suggests that CHK1 can promote tumor growth. An inhibitor targeting CHK1 may include SCH-900776, SRA737, V158411, PF-477736, AZD7762, LY2880070 (prexasertib), and the like.

As used herein, the term "ATR (ataxia telangiectasia mutated (ATM) and RAD3-related kinase)" refers to a protein kinase implicated in cellular responses to certain forms of DNA damage (for example, double-strand breaks and replication stress). Normal cells repair damaged DNA using the ATM/ATR signal transduction system, which regulates the cellular response to double-strand DNA breaks and replication stress, referred to as the DNA damage response ("DDR"). On the other hand, many cancer cells show a high dependence on DNA repair proteins, including ATR, due to defects in ATM in the DNA repair process. An inhibitor targeting ATR may be berzosertib (VX-970), gartisertib (VX-803) or ceralasertib (AZD6738).

As used herein, the term "HDAC (histone deacetylase)" refers to an enzyme that removes the acetyl group from the ε-N-acetyl lysine amino acid of histone and non-histone proteins, and histones wrap DNA more tightly to regulate the expression of DNA through acetylation and deacetylation. HDAC includes subgroups such as Class I, such as HDAC1, HDAC2, and HDAC3, and Class IIA, such as HDAC4, HDAC5, and HDAC7. An HDAC inhibitor exhibits anticancer efficacy in studies on pancreatic cancer, esophageal squamous cell carcinoma (ESCC), multiple myeloma, prostate carcinoma, gastric cancer, leukemia, breast cancer, liver cancer, ovarian cancer, nasal cancer, Hodgkin's lymphoma, and neuroblastoma. An inhibitor targeting HDAC may include panobinostat (LBH589), entinostat, mocetinostat, trichostatin A, CBUD-1001, abexinostat (PCI-24781, CRA-024781), and the like.

As used herein, the term "AKT (protein kinase B: PKB)" is a set of serine/threonine-specific protein kinases that play key roles in several cellular processes such as glucose metabolism, apoptosis, cell proliferation, and transcription, and is associated with tumor cell survival, proliferation and invasiveness. Activation of AKT is commonly observed in human cancer and tumor cells, and tumor cells depend on AKT for survival. An inhibitor targeting AKT may include VQD-002, perifosine, miltefosine, MK-2206, AZD5363, ipatasertib, and the like.

As used herein, the term "PLK1 (Polo-like kinase 1)" is also referred to as serine/threonine-protein kinase 1 or serine/threonine-protein kinase 13 (STPK13), and is a 66 kDa enzyme composed of 603 amino acids. Most colorectal cancer and lung cancer are caused by K-RAS mutations and are known to depend on PLK1. When PLK1 expression is silenced by RNA interference in cell culture, K-RAS cells can be selectively killed without harming normal cells. An inhibitor targeting PLK1 may include volasertib, rigosertib, and the like.

As used herein, the term "BET (bromodomain and extraterminal domain protein)" refers to a bromodomain composed of approximately 110 amino acid proteins that recognize acetylated lysine residues, including BRD2, BRD3, BRD4, and BRDT. It converts the signals transmitted by the acetylated lysine residues and converts them into various normal or abnormal phenotypes. Bromodomains translate the dysregulated cellular acetylome into disease phenotypes, and BETs are targets in cancer and multiple sclerosis. An inhibitor targeting BET may include JQ1, I-BET 151 (GSK1210151A), I-BET 762 (GSK525762), OTX-015, TEN-010, CPI-203, CPI-0610, Olinone, RVX-208, ABBV-744, LY294002, AZD5153, MT-1, MS645, and the like.

As used herein, the term "IGF (insulin-like growth factor)" is a protein with high sequence similarity to insulin and is involved in communication between cells and the physiological environment, and includes IGF1/2, IGF-1R, IGF-2R, and the like. IGF-1 stimulates the growth of prostate and breast cancer cells, and IGF has been found to be involved in diseases such as cancer and diabetes. An inhibitor targeting IGF1/2 or IGF-1R may include NVP-ADW742, figitumumab, mecasermin, rhIGF-1, BI 885578, and the like.

As used herein, the term "PIK (phosphatidylinositol kinase)" consists of phosphatidylinositol 3-kinase (PI3K) and phosphatidylinositol 4-kinase (PI4K). PI3K is involved in cell signal transduction by phosphorylating phosphoinositide on the 3-hydroxyl group of the inositol ring, and PI4K acts on phosphatidylinositol (PI) to produce the second messenger, inositol-1,4,5-trisphosphate, and their abnormalities are associated with cancer. An inhibitor targeting PIK may include duvelisib, buparlisib, copanlisib, dactolisib, idelalisib, parsaclisib, paxalisib, taselisib, zandelisib, inavolisib, and the like.

As used herein, the term "CDK9 (cyclin dependent kinase 9)" is a cyclin dependent kinase associated with P-TEFb and is a cell cycle regulator. CDK9 is involved in several protein-protein interaction networks that are often involved in transcriptional deregulation in cancer. An inhibitor targeting CDK9 may include, for example, AZD4573, atuveciclib, VIP152, A-1592668, JSH-150, SLS009, AT-7519, roscovitine, and the like.

As used herein, the term "DHFR (dihydrofolate reductase)" is an enzyme that reduces dihydrofolate to tetrahydrofolate using NADPH as an electron donor, and is a component of the multiprotein complex TAK/P-TEFb, an elongation factor for transcription and function by RNA polymerase II by phosphorylating the C-terminal domain of the largest subunit of RNA polymerase II. DHFR is responsible for intracellular levels of tetrahydrofolate, and inhibiting DHFR

can limit cell growth and proliferation, a hallmark of cancer and bacterial infections. An inhibitor targeting DHFR may include methotrexate, pralatrexate, pemetrexed, raltitrexed, trimetrexate, nolatrexed, piritrexim, talotrexin, and the like.

As used herein, the term "STING (stimulator of interferon genes)" is an *in vivo* sensor that recognizes DNA fragments from cancer cells, and activates immune cells in the body, such as dendritic cells, by stimulating interferon genes. A STING agonist exhibits an immune enhancing effect and cancer angiogenesis inhibitory effect. For example, a STING agonist may be CDNs, SB11285, DMXAA, and the like.

The compound represented by Formula I or Formula I-1 of the present invention can be used in combination with other targeted inhibitors described above to enhance the action of the targeted inhibitors, thereby significantly inhibiting the expression and activity of the target protein or gene. Specifically, the compound represented by Formula I or Formula I-1 not only has its own anticancer efficacy, but can also improve the anticancer efficacy of the targeted inhibitor. Therefore, when the compound represented by Formula I or Formula I-1 are used together with the targeted inhibitor, they can exhibit anticancer efficacy that is superior to the sum of the anticancer efficacy when they are used alone.

As used herein, the term "anticancer virus therapeutic agent" is a therapeutic agent that kills cancer by inserting a specific gene targeting cancer cells into a virus capable of proliferation and having infectivity. The anticancer virus therapeutic agent may be Talimogene Laherparepvec.

As used herein, the term "antibody therapeutic agent" is a therapeutic agent that exhibits an anticancer effect using an antibody that recognizes a specific protein of cancer cells as an antigen. An antibody therapeutic agent may be cetuximab, trastuzumab, emtansine, emtansine, rituximab, ibritumomab, tositumomab, brentuximab, ofatumumab, obinutuzumab, necitumumab, bevacizumab, ramucirumab, nivolumab, pembrolizumab, atezolizumab, durvalumab, ipilimumab, and the like.

As used herein, the term "immune cell therapeutic agent" is a therapeutic agent that exhibits an anticancer effect by activating an immune response in the body using immune cells such as dendritic cells, natural killer cells, Tcells, and the like. An immune cell therapeutic agent is used by extracting and enhancing immune cells in the body or genetically modifying them and then injecting them back into the body. Representative immune cell therapeutic agents may include T cell receptor-modified Tcells (TCR-T), chimeric antigen receptor-modified Tcells (CAR-T), and the like. Specifically, it may be tisagenlecleucel or axicabtagene ciloleucel, but is not limited thereto.

As used herein, the term "immune checkpoint inhibitor" is a substance that inhibits the activity of an immune checkpoint protein, which inhibits the differentiation, proliferation, and activity of immune cells, and is known to eliminate cancer cells by preventing them from exerting functions to evade the immune system. The immune checkpoint inhibitor may be any one selected from the group consisting of an anti-CTLA-4 antibody, an anti-PD-1 antibody, an anti-PD-L1 antibody, an anti-PD-L2 antibody, an anti-B7-H4 antibody, an anti-HVEM antibody, an anti-TIM3 antibody, an anti-GAL9 antibody, an anti-LAG3 antibody, an anti-VISTA antibody, an anti-KIR antibody, an anti-BTLA antibody and an anti-TIGIT antibody. In one embodiment, the immune checkpoint inhibitor may be ipilimumab, pembrolizumab, nivolumab, cemiplimab, atezolizumab, avelumab and durvalumab and the like, but is not limited thereto.

As used herein, the term "ADC (antibody drug conjugate)" is a therapeutic agent that exhibits high anticancer effects through targeted delivery by chemical binding of an antibody and a cytotoxic drug. It may be gemtuzumab-ozogamicin, brentuximab-vedotin, trastuzumab-emtansine, inotuzumab-ozogamicin, and eribulin-mesylate, and the like.

The anticancer agent may include one or more anticancer agents. Specifically, the compound, solvate, stereoisomer or pharmaceutically acceptable salt thereof may be used together with two anticancer agents. For example, two anticancer agents may be a chemical anticancer agent and a targeted anticancer agent; a chemical anticancer agent and an anticancer virus; a targeted anticancer agent and an antibody therapeutic agent; a chemical anticancer agent and a cell therapeutic agent; and a chemical anticancer agent and an immune checkpoint inhibitor. In addition, two anticancer agents may be a targeted anticancer agent and an anticancer virus; a targeted anticancer agent and an antibody therapeutic agent; a targeted anticancer agent and a cell therapeutic agent; a targeted anticancer agent and an immune checkpoint inhibitor. In addition, two anticancer agents may be an anticancer virus and an antibody therapeutic agent; an anticancer virus and a cell therapeutic agent; and an anticancer virus and an immune checkpoint inhibitor. In addition, two anticancer agents may be an antibody therapeutic agent and a cell therapeutic agent; and an antibody therapeutic agent and an immune checkpoint inhibitor.

The compound, solvate, stereoisomer or pharmaceutically acceptable salt thereof may be used together with three anticancer agents. In addition to the two anticancer agents, a different anticancer agent may be further included and used.

The compound, solvate, stereoisomer or pharmaceutically acceptable salt thereof may be used together with four anticancer agents. In addition to the three anticancer agents, a different anticancer agent may be further included and used.

The compound, solvate, stereoisomer or pharmaceutically acceptable salt thereof may be used together with five anticancer agents. In addition to the four anticancer agents, a different anticancer agent may be further included and used.

The compound, solvate, stereoisomer or pharmaceutically acceptable salt thereof may be used together with six anticancer agents.

The compound, solvate, stereoisomer or pharmaceutically acceptable salt thereof may be used in combination with an anticancer vaccine.

As used herein, the term "anticancer vaccine" is an active immunotherapy that removes cancer cells by enhancing the immune function *in vivo* by administering a tumor-specific antigen (TSA) possessed by cancer cells to a cancer patient to activate the immune system. An anticancer vaccine may include a DNA vaccine, a peptide vaccine, a cell vaccine, and the like, depending on the type of antigen and the delivery method of antigen, and a cell vaccine and a DNA vaccine developed by introducing antigens are currently being developed representatively.

The compound, solvate, stereoisomer or pharmaceutically acceptable salt thereof may be used in combination with the anticancer agent and the anticancer vaccine. Here, the compound and the anticancer agent are the same as described above.

In another aspect, there is provided a method for preventing or treating a SOS1 mediated disease, comprising administering to a subject a compound of Formula I, a solvate, stereoisomer or pharmaceutically acceptable salt thereof, or the pharmaceutical composition comprising the same.

Among the terms or elements mentioned in the description of the method, the same as those already mentioned are the same as described above.

The administration may be oral or parenteral administration. It may be administered in one to several divided doses to be administered in an amount of 0.01 to 1000 mg, more specifically 0.1 to 300 mg per kg of body weight daily based on the active ingredient when administered orally, or in an amount of 0.01 to 100 mg, more specifically 0.1 to 50 mg per kg of body weight daily based on the active ingredient when administered parenterally. The dose to be administered to a specific subject or patient should be determined in light of several related factors such as body weight, age, sex, health condition of the patient, diet, administration time, administration method, the severity of the disease, and the like, and it may be appropriately increased or decreased by a specialist.

As used herein, the term "subject" refers to a subject in need of treatment for a disease, and more specifically means a mammal such as a human or non-human primate, a mouse, a dog, a cat, a horse, and a cow.

In another aspect, there is provided a method of inhibiting the interaction of SOS1 and RAS family proteins and/or SOS1 and RAC1 in a sample or cell, comprising administering the compound represented by Formula I, solvate, stereoisomer or pharmaceutically acceptable salt thereof to the sample or cell.

In another aspect, there is provided a medicinal use of the compound of Formula I, solvate, stereoisomer or pharmaceutically acceptable salt thereof for the prevention or treatment of a SOS1 mediated disease; or a use of the compound of Formula I, solvate, stereoisomer or pharmaceutically acceptable salt thereof for the manufacture of a medicament for the prevention or treatment of a SOS1 mediated disease.

Among the terms or elements mentioned in the description of the method or use, the same as those already mentioned are the same as described above.

## Effects of Invention

The compound of Formula I, solvate, stereoisomer or pharmaceutically acceptable salt thereof has an effective inhibitory activity against SOS1, specifically, inhibits the interaction between SOS1 and RAS family proteins, or between SOS1 and RAC1, and thus, it is useful for the prevention or treatment of a SOS1 mediated disease, specifically a disease associated with the abnormal activity of SOS1 and/or RAS family proteins.

## Brief Description of Drawings

Figure 1 is a graph showing the results obtained by evaluating cell viability according to the administration of the example compounds and sotorasib alone or in combination to the lung cancer cell NCI-H358.

Figure 2 is a graph showing the results obtained by evaluating cell viability according to the administration of the example compounds and sotorasib alone or in combination to the pancreatic cancer cell MIA PaCa-2.

Figure 3 is a graph showing the results obtained by evaluating cell viability according to the administration of the example compounds and adagrasib alone or in combination to the lung cancer cell NCI-H358.

Figure 4 is a graph showing the results obtained by evaluating cell viability according to the administration of the example compounds and adagrasib alone or in combination to the pancreatic cancer cell MIA PaCa-2.

Figures 5a and 5b are graphs showing the results obtained by evaluating cell viability according to the administration of the example compounds and trametinib alone or in combination to the lung cancer cell NCI-H358.

Figures 6a and 6b are graphs showing the results obtained by evaluating cell viability according to the administration of the example compounds and trametinib alone or in combination to the gastric cancer cell SNU-1.

Figures 7a and 7b are graphs showing the results obtained by evaluating cell viability according to the administration

of the example compounds and trametinib alone or in combination to the colorectal cancer cell SW480.

Figure 8 is a graph showing the results obtained by evaluating cell viability according to the administration of the example compounds and osimertinib alone or in combination to the lung cancer cell H1975.

Figure 9 is a graph showing the results obtained by evaluating cell viability according to the administration of the example compounds and osimertinib alone or in combination to the lung cancer cell HCC827.

Figure 10 is a graph showing the results obtained by evaluating cell viability according to the administration of the example compounds and lazertinib alone or in combination to the lung cancer cell H1975.

Figure 11 is a graph showing the results obtained by evaluating cell viability according to the administration of the example compounds and lazertinib alone or in combination to the lung cancer cell HCC827.

Figure 12 is a graph showing the results obtained by evaluating cell viability according to the administration of the example compounds and alpelisib alone or in combination to the PIK3CA mutant breast cancer cell MCF7.

Figure 13 is a graph showing the results obtained by evaluating cell viability according to the administration of the example compounds and JDQ443 alone or in combination to the lung cancer cell H358 having the KRAS G12C variant.

Figure 14 is a graph showing the results obtained by evaluating cell viability according to the administration of the example compounds and TNO155 alone or in combination to the lung cancer cell H358 having the KRAS G12C variant.

Figure 15 is a graph showing the results obtained by evaluating cell viability according to the administration of the example compounds and cisplatin alone or in combination to the lung cancer cell H358 having the KRAS G12C variant.

Figure 16 is a graph showing the results obtained by evaluating cell viability according to the administration of the example compounds and rineterkib alone or in combination to the lung cancer cell H358 having the KRAS G12C variant.

Figure 17 is a graph showing the results obtained by evaluating cell viability according to the administration of the example compounds and ulixertinib alone or in combination to the lung cancer cell H358 having the KRAS G12C variant.

Figure 18 is a graph showing the results obtained by evaluating cell viability according to the administration of the example compounds and pralsetinib alone or in combination to the lung cancer cell H358 having the KRAS G12C variant.

Figure 19 is a graph showing the results obtained by evaluating cell viability according to the administration of the example compounds and repotrectinib alone or in combination to the lung cancer cell H358 having the KRAS G12C variant.

Figure 20 is a graph showing the results obtained by evaluating cell viability according to the administration of the example compounds and tepotinib alone or in combination to the c-Met overexpressing gastric cancer cell SNU-5.

Figure 21 is a graph showing the results obtained by evaluating cell viability according to the administration of the example compounds and bemcentinib alone or in combination to the lung cancer cell PC-9 with high AXL expression.

Figure 22 is a graph showing the results obtained by evaluating cell viability according to the administration of the example compounds and alrizomadlin alone or in combination to the lung cancer cell A549 having the KRAS G12S variant.

Figure 23 is a graph showing the results obtained by evaluating cell viability according to the administration of the example compounds and everolimus alone or in combination to the colorectal cancer cell LoVo having the KRAS G13D variant.

Figure 24 is a graph showing the results obtained by evaluating cell viability according to the administration of the example compounds and MRTX1133 alone or in combination to the lung cancer cell AsPC-1 having the KRAS G12D variant.

Figures 25a to 25c are graph showing the results obtained by evaluating the synergy of the combined administration of the example compounds to the lung cancer cell H358 using SynergyScreen.

Figures 26a to 26c are graph showing the results obtained by evaluating the synergy of the combined administration of the example compounds to the lung cancer cell H358 using SynergyScreen.

## Detailed Descriptions for Carrying out the Invention

Hereinafter, the present invention will be described in more detail by way of the following examples. However, the following examples are only for illustrating the present invention, and the scope of the present invention is not limited thereto.

## [Preparation Examples]

**Preparation Example** 1: **2-[3-[(1R)-1-aminoethyl]-2-fluoro-phenyl]-2,2-difluoro-ethanol** (A-1)

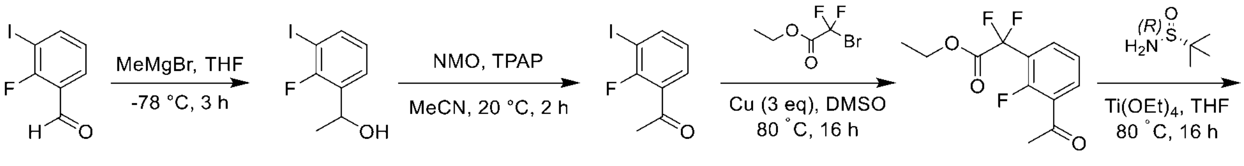


A-1-1 A-1-2 A-1-3 A-1-4

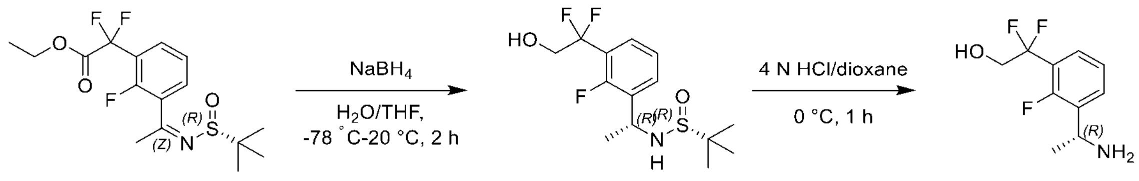


A-1-5 A-1-6 A-1

Step 1: Synthesis of 1-(2-fluoro-3-iodo-phenyl)ethanol

To a solution of 2-fluoro-3-iodo-benzaldehyde (4.0 g, 16.00 mmol) in THF (40 mL) was added dropwise MeMgBr (3 M, 8.00 mL) at -78 °C, and then the mixture was stirred for 3 h. The reaction mixture was poured into sat. aq. $NH_4Cl$ (50 mL) and extracted with EtOAc. The combined organic layers were washed with brine, dried over $Na_2SO_4$, and then concentrated under reduced pressure to give a crude product. The crude product was purified by silica gel column chromatography (7% EtOAc in petroleum ether) to give 1-(2-fluoro-3-iodo-phenyl)ethanol (4.45 g, 83.63% yield) as yellow oil. $^1$H NMR (400 MHz, CHLOROFORM-d) δ 7.72-7.64 (m, 1H), 7.55-7.45 (m, 1H), 6.93 (t, *J* = 7.6 Hz, 1H), 5.20 (q, *J*= 6.4 Hz, 1H), 1.52 (d, *J* = 6.4 Hz, 3H).

Step 2: Synthesis of 1-(2-fluoro-3-iodo-phenyl)ethanone

To a solution of 1-(2-fluoro-3-iodo-phenyl)ethanol (4.45 g, 16.73 mmol) in MeCN (50 mL) was added TPAP (Tetrapropylammonium perruthenate; 587.80 mg, 1.67 mmol) and NMO (N-methyl morpholine-N-oxide; 2.94 g, 25.09 mmol), and then the mixture was stirred at 20 °C for 2 h. The mixture was filtered and concentrated under reduced pressure to give a crude product. The crude product was purified by silica gel column chromatography (0% EtOAc in petroleum ether) to give 1-(2-fluoro-3-iodo-phenyl)ethanone (3.8 g, 12.95 mmol, 77.44% yield, 90% purity) as a white solid. $^1$H NMR (400 MHz, CHLOROFORM-d) δ 7.97-7.88 (m, 1H), 7.85-7.78 (dm, 1H), 7.00 (t, *J* = 7.6 Hz, 1H), 2.65 (d, *J* = 5.2 Hz, 3H).

Step 3: Synthesis of ethyl 2-(3-acetyl-2-fluoro-phenyl)-2,2-difluoro-acetate

To a solution of 1-(2-fluoro-3-iodo-phenyl)ethanone (3.0 g, 11.36 mmol) and ethyl-2-bromo-2,2-difluoro-acetate (6.92 g, 34.09 mmol, 4.38 mL) in DMSO (30 mL) was added Cu (2.17 g, 34.09 mmol), and then the mixture was stirred at 80 °C for 12 h. The reaction mixture was poured into water (50 mL) and extracted with EtOAc. The combined organic layers were washed with brine, dried over $Na_2SO_4$ and concentrated under reduced pressure to give a crude product. The crude product was purified by silica gel column chromatography (5% EtOAc in petroleum ether) to give ethyl 2-(3-acetyl-2-fluoro-phenyl)-2,2-difluoro-acetate (1.8 g, 56.05% yield, 92.07% purity) as colorless oil. $^1$H NMR (400 MHz, CHLOROFORM-d) δ 8.08-8.00 (m, 1H), 7.87-7.81 (m, 1H), 7.36 (t, *J* = 7.6 Hz, 1H), 4.42-4.37 (m, 2H), 2.66 (d, *J* = 5.2 Hz, 3H), 1.35 (t, *J* = 7.2 Hz, 3H); MS (ESI) m/z = 261.0 $[M+H]^+$.

Step 4: Synthesis of ethyl 2-[3-[[(R)-tert-butylsulfinyl]-C-methyl-carboimidoyl]-2-fluoro-phenyl]-2,2-difluoro-acetate

To a solution of ethyl 2-(3-acetyl-2-fluoro-phenyl)-2,2-difluoro-acetate (1.8 g, 6.92 mmol) and (R)-2-methylpropane-2-sulfinamide (1.26 g, 10.38 mmol) in THF (20 mL) was added $Ti(OEt)_4$ (4.73 g, 20.75 mmol), and then the mixture was stirred at 80 °C for 16 h. The reaction mixture was poured into water (30 mL) and EtOAc (30 mL) and filtered, and then the filtrate was extracted with EtOAc. The combined organic layers were washed with brine, dried over $Na_2SO_4$ and concentrated under reduced pressure to give a crude product. The crude product was purified by silica gel column

chromatography (6% EtOAc in petroleum ether) to give ethyl 2-[3-[[(R)-tert-butylsulfinyl]-C-methyl-carboimidoyl]-2-fluoro-phenyl]-2,2-difluoro-acetate (1.9 g, 74.09% yield, 98.03% purity) as yellow oil. $^1$H NMR (400 MHz, CHLOROFORM-d) δ 7.86-7.72 (m, 2H), 7.32 (t, $J$ = 7.6 Hz, 1H), 4.44-4.30 (m, 2H), 2.77 (s, 3H), 1.32 (s, 9H); MS (ESI) m/z = 364.0 [M+H]$^+$.

Step 5: Synthesis of (R)-N-[(1R)-1-[3-(1,1-difluoro-2-hydroxyethyl)-2-fluorophenyl]ethyl]-2-methyl-propane-2-sulfinamide

To a solution of ethyl 2-[3-[[(R)-tert-butylsulfinyl]-C-methyl-carboimidoyl]-2-fluorophenyl]-2,2-difluoro-acetate (900 mg, 2.48 mmol) in THF (10 mL) and $H_2O$ (0.2 mL) was added $NaBH_4$ (210 mg, 5.55 mmol) at -78 °C, and then the mixture was slowly warmed to 10 °C and stirred at 10 °C for 2 h. The reaction mixture was poured into ice-cold water (30 mL) and extracted with EtOAc. The combined organic layers were washed with brine, dried over $Na_2SO_4$ and concentrated under reduced pressure to give a crude product. Following the primary purification by silica gel column chromatography (50% EtOAc in petroleum ether), two diastereomers (about 3:1 ratio) were separated and purified by prep-HPLC (Xtimate C18 150*40 mm*10 μm, mobile phase: [water ($NH_3H_2O$)-ACN]; B%: 25%-55%, 10 min). MeCN was removed under reduced pressure, and the remaining solvent was removed by lyophilization to give a main product, (R)-N-[(1R)-1-[3-(1,1-difluoro-2-hydroxyethyl)-2-fluorophenyl]ethyl]-2-methyl-propane-2-sulfinamide (440 mg, 50.90% yield, 92.65% purity, >99% ee) as a white solid. $^1$H NMR (400 MHz, DMSO-$d_6$) δ 7.70 (t, $J$ = 6.8 Hz, 1H), 7.48-7.39 (m, 1H), 7.35-7.25 (m, 1H), 5.87 (d, $J$ = 7.6 Hz, 1H), 5.70 (t, $J$ = 6.4 Hz, 1H), 4.68 (quint, $J$ = 7.2 Hz, 1H), 3.90 (dt, $J$ = 6.4, 14.4 Hz, 2H), 1.40 (d, $J$ = 6.8 Hz, 3H), 1.10 (s, 9H); MS (ESI) m/z = 324.3 [M+H]$^+$.

Step 6: Synthesis of 2-[3-[(1R)-1-aminoethyl]-2-fluoro-phenyl]-2,2-difluoro-ethanol

To a solution of (R)-N-[(1R)-1-[3-(1,1-difluoro-2-hydroxy-ethyl)-2-fluorophenyl]ethyl]-2-methyl-propane-2-sulfinamide (440 mg, 1.36 mmol) in dioxane (4 mL) was added 4N HCl/dioxane (2 mL) at 0°C, and then the mixture was stirred at 0 °C for 1 h. The mixture was concentrated under reduced pressure to give **Intermediate A-1** (347 mg, 100% yield, 95% purity, HCl salt) as yellow oil. MS (ESI) m/z = 220.0 [M+H]$^+$.

**Preparation Example 2: (1R)-1-[3-(difluoromethyl)-2-fluoro-phenyl]ethanamine (A-2)**

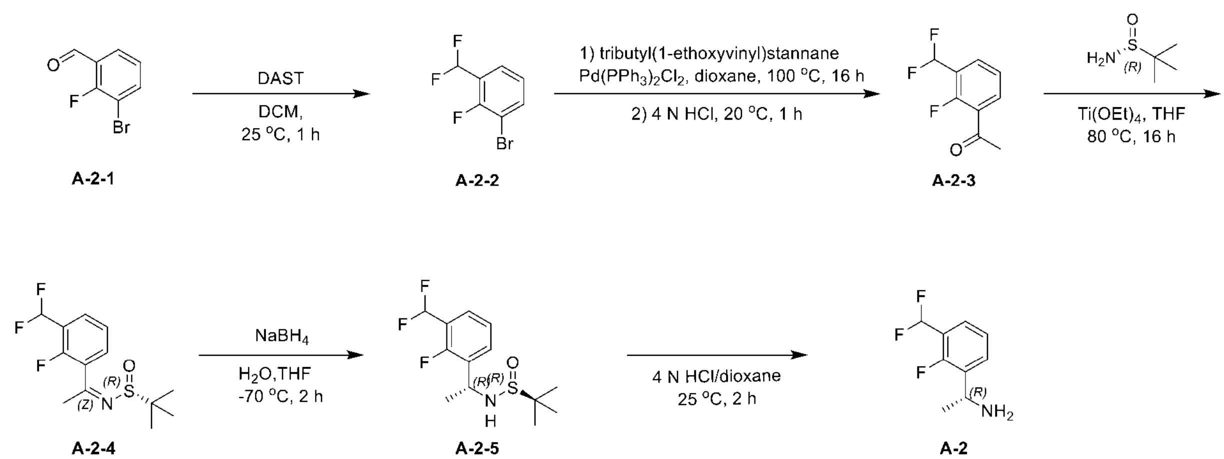


A-2-1 A-2-2 A-2-3

A-2-4 A-2-5 A-2

Step 1: Synthesis of 1-bromo-3-(difluoromethyl)-2-fluoro-benzene

To a solution of 3-bromo-2-fluoro-benzaldehyde (9 g, 44.33 mmol) in DCM (150 mL) was added DAST (Diethylaminosulfur trifluoride; 14.29 g, 88.67 mmol, 11.71 mL), and then the mixture was stirred at 25 °C for 1 h. After the addition of sat. $NaHCO_3$ (50 mL), the combined organic layers were extracted with DCM (50 mL X 3). It was washed with brine, dried over $Na_2SO_4$ and concentrated under reduced pressure to give a crude product. The organic layers were dried over anhydrous $Na_2SO_4$ and concentrated under reduced pressure to give a crude product. The crude product was purified by silica gel column chromatography (100% petroleum ether) to give 1-bromo-3-(difluoromethyl)-2-fluoro-benzene (3.9 g, 39.1% yield) as colorless oil. $^1$H NMR (400 MHz, DMSO-$d_6$) δ 7.98 - 7.79 (m, 1H), 7.72 - 7.57 (m, 1H), 7.37 (s, 1H), 7.31 (br t, $J$ = 7.8 Hz, 1H), 7.24 (s, 1H), 7.10 (s, 1H).

Step 2: Synthesis of 1-[3-(difluoromethyl)-2-fluoro-phenyllethanone

A mixture of 1-bromo-3-(difluoromethyl)-2-fluoro-benzene (17 g, 75.55 mmol), tributyl(1-ethoxyvinyl)stannane (28.65 g, 79.33 mmol, 26.80 mL) and $Pd(PPh_3)_2Cl_2$ (2.65 g, 3.78 mmol ) in dioxane (300 mL) was degassed and purged with $N_2$ for 3 times, and then the mixture was stirred at 100°C for 16 h under $N_2$ atmosphere. The mixture was quenched with 4 N HCl (30 mL) and then stirred at 20 °C for 1 h. The mixture was poured into water (200 mL) and extracted with EtOAc (100 mL X 3). The organic layers were washed with brine (100 mL X 3), dried over anhydrous $Na_2SO_4$ and concentrated under reduced pressure to give a crude product. The crude product was purified by silica gel column chromatography (2% EtOAc in petroleum ether) to give 1-[3-(difluoromethyl)-2-fluoro-phenyl]ethanone (10 g, 70.35% yield) as colorless oil. $^1H$ NMR (400 MHz, DMSO-$d_6$) δ 8.03-7.93 (m, 1H), 7.93-7.82 (m, 1H), 7.49-7.14 (m, 2H), 2.63-2.58 (m, 3H).

Step 3: Synthesis of (NZ,R)-N-[1-[3-(difluoromethyl)-2-fluoro-phenyl]ethylidene]-2-methyl-propane-2-sulfinamide

A mixture of 1-[3-(difluoromethyl)-2-fluoro-phenyl]ethanone (10 g, 53.15 mmol), 2-methylpropane-2-sulfinamide (6.44 g, 53.15 mmol) and $Ti(OEt)_4$ (36.37 g, 159.45 mmol, 33.07 mL) in THF (80 mL) was degassed and purged with $N_2$ for 3 times, and then the mixture was stirred at 80 °C for 16 h under $N_2$ atmosphere. The mixture was poured into water (100 mL) and filtered, and then the filtrate was extracted with EtOAc (100 mL X 3). The organic layers were washed with brine (50 mL X 3), dried over anhydrous $Na_2SO_4$ and concentrated under reduced pressure to give a crude product. The crude product was purified by silica gel column chromatography (35% EtOAc in petroleum ether) to give (NZ,R)-N-[1-[3-(difluoromethyl)-2-fluoro-phenyl]ethylidene]-2-methyl-propane-2-sulfinamide (13.0 g, 81.65% yield, 97.25% purity) as yellow oil. $^1H$ NMR (400 MHz, DMSO-$d_6$) δ 7.90-7.81 (m, 1H), 7.81-7.73 (m, 1H), 7.47-7.40 (m, 1H), 7.29-7.10 (m, 1H), 2.71 (br s, 3H), 1.22 (s, 9H); MS (ESI) m/z = 292.1 $[M+H]^+$.

Step 4: Synthesis of (R)-N-[(1R)-1-[3-(difluoromethyl)-2-fluoro-phenyl]ethyl]-2-methyl-propane-2-sulfinamide

A solution of (NZ,R)-N-[1-[3-(difluoromethyl)-2-fluoro-phenyl]ethylidene]-2-methyl-propane-2-sulfinamide (13 g, 44.62 mmol) in THF (200 mL) and $H_2O$ (5 mL) was cooled to -70 °C and stirred at -70 °C for 2 h under $N_2$ atmosphere after the addition of $NaBH_4$ (1.35 g, 35.70 mmol) in 3 batches. The mixture was quenched at 20 °C with sat. $NH_4Cl$ (200 mL), diluted with EtOAc (150 mL), and then extracted with EtOAc (150 mL X 3). The organic layers were dried over anhydrous $Na_2SO_4$ and concentrated under reduced pressure to give a crude product. The crude product was purified by silica gel column chromatography (30% EtOAc in petroleum ether) to give (R)-N-[(1R)-1-[3-(difluoromethyl)-2-fluoro-phenyl]ethyl]-2-methyl-propane-2-sulfinamide (4.4 g, 33.61% yield, 100% purity, 98.34% ee) as colorless oil. $^1H$ NMR (400 MHz, DMSO-$d_6$) δ 7.74 (t, $J$ = 7.2 Hz, 1H), 7.52 (t, $J$ = 6.8 Hz, 1H), 7.37-7.33 (m, 1H), 7.33-7.06 (m, 1H), 5.88 (d, $J$ = 8.0 Hz, 1H), 5.75 (s, 1H), 4.69 (quint, $J$ = 7.0 Hz, 1H), 1.41 (d, $J$ = 6.8 Hz, 3H), 1.10 (s, 9H); MS (ESI) m/z = 294.1 $[M+H]^+$.

Step 5: Synthesis of (1R)-1-[3-(difluoromethyl)-2-fluoro-phenyllethanamine

A mixture of (R)-N-[(1R)-1-[3-(difluoromethyl)-2-fluoro-phenyl]ethyl]-2-methylpropane-2-sulfinamide (2.00 g, 6.82 mmol) in 4 N HCl/dioxane (15 mL) was degassed and purged with $N_2$ for 3 times, and then the mixture was stirred at 25 °C for 2 h under $N_2$ atmosphere. The mixture was concentrated under reduced pressure to give Intermediate A-2 (1.5 g, 94.02% yield, 96.43% purity, HCl salt) as a white solid. MS (ESI) m/z = 190.1 $[M+H]^+$.

**Preparation Example 3: (1R)-1-[2-fluoro-3-(trifluoromethyl)phenyl]ethanamine (A-3)**

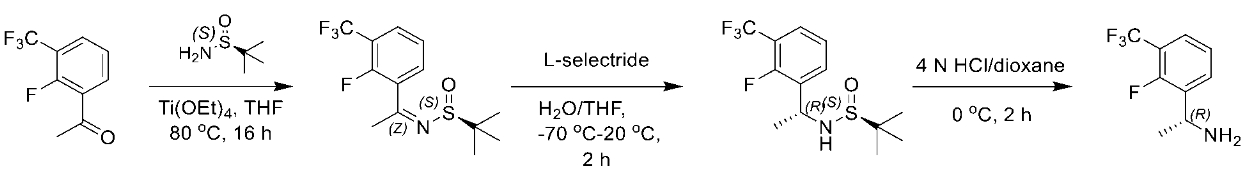


A-3-1 A-3-2 A-3-3 A-3

Step 1: Synthesis of (NZ,S)-N-[1-[2-fluoro-3-(trifluoromethylphenyl]ethylidene]-2-methyl-propane-2-sulfinamide

A mixture of 1-[2-fluoro-3-(trifluoromethyl)phenyl]ethanone (1.9 g, 9.22 mmol), (S)-2-methylpropane-2-sulfinamide (1.12 g, 9.22 mmol) and $Ti(OEt)_4$ (6.31 g, 27.65 mmol, 5.73 mL) in THF (10 mL) was degassed and purged with $N_2$ for 3 times, and then the mixture was stirred at 80 °C for 16 h under $N_2$ atmosphere. The mixture was poured into water (30 mL)

and extracted with EtOAc (30 mL X 3). The organic layers were washed with brine (50 mL), dried over anhydrous $Na_2SO_4$ and concentrated under reduced pressure to give a crude product. The crude product was purified by silica gel column chromatography (24% EtOAc in petroleum ether) to give (NZ,S)-N-[1-[2-fluoro-3-(trifluoromethyl)phenyl]ethylidene]-2-methylpropane-2-sulfinamide (2.08 g, 62.57% yield, 85.77% purity) as yellow oil. MS (ESI) m/z = 310.1 $[M+H]^+$.

Step 2: Synthesis of (S)-N-[(1R)-1-[2-fluoro-3-(trifluoromethyl)phenyl]ethyl]-2-methyl-propane-2-sulfinamide

A solution of (NZ,S)-N-[1-[2-fluoro-3-(trifluoromethyl)phenyl]ethylidene]-2-methylpropane-2-sulfinamide (2.00 g, 6.47 mmol) in THF (15 mL) was cooled to -70 °C. After the addition of L-selectride (1 M, 16.16 mL), the mixture was slowly warmed to 20 °C and stirred at 20 °C for 2 h. The reaction mixture was quenched at 20 °C with sat. aq. $NH_4Cl$ (50 mL), diluted with EtOAc (30 mL) and extracted with EtOAc (30 mL X 3). The organic layers were dried over anhydrous $Na_2SO_4$ and concentrated under reduced pressure to give a crude product. The crude product was purified by silica gel column chromatography (62% EtOAc in petroleum ether) to give a main product, (S)-N-[(1R)-1-[2-fluoro-3-(trifluoromethyl) phenyl]ethyl]-2-methyl-propane-2-sulfinamide (1.23 g, 56.49% yield, 92.35% purity, 98.28% ee) as colorless oil. MS (ESI) m/z = 312.1 $[M+H]^+$.

Step 3: Synthesis of (1R)-1-[2-fluoro-3-(trifluoromethyl)phenyl]ethanamine

A mixture of (S)-N-[(1R)-1-[2-fluoro-3-(trifluoromethyl)phenyl]ethyl]-2-methylpropane-2-sulfinamide (1.1 g, 3.53 mmol) and 4 N HCl/dioxane (5 mL) in dioxane (5 mL) was degassed and purged with $N_2$ for 3 times, and then the mixture was stirred at 25 °C for 2 h under $N_2$ atmosphere. The mixture was concentrated under reduced pressure to give **Intermediate A-3** (850 mg, 96.04% yield, 97.25% purity, HCl) as a white solid. MS (ESI) m/z = 208.0 $[M+H]^+$.

**Preparation Example 4: 2-[3-[(1R)-1-aminoethyl]-2-chloro-phenyl]-2,2-difluoroethanol (A-4)**

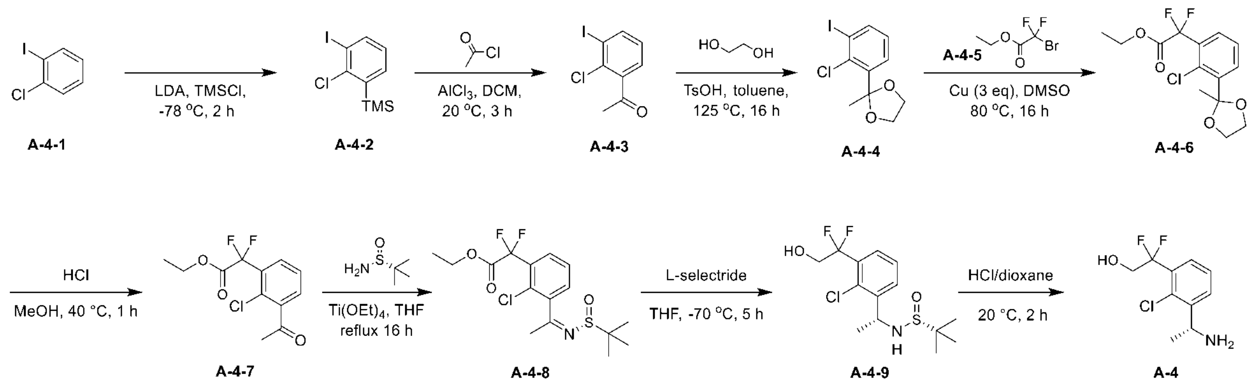


Step 1: Synthesis of (2-chloro-3-iodo-phenyl)trimethyl-silane

A solution of 1-chloro-2-iodo-benzene (27 g, 113.23 mmol, 370.37 uL) and TMSCl (14.76 g, 135.88 mmol, 17.24 mL) in THF (300 mL) was cooled to -78 °C and stirred at -78 °C for 2 h after the addition of LDA (2 M, 67.94 mL). The mixture was quenched with water (300 mL) and extracted with EtOAc (300 mL X 3). The organic layers were washed with brine (500 mL), dried over anhydrous $Na_2SO_4$ and concentrated under reduced pressure to give a crude product. The crude product was purified by silica gel column chromatography (0% EtOAc in petroleum ether) to give (2-chloro-3-iodo-phenyl)-trimethyl-silane (32 g, 90.98% yield) as colorless oil. $^1H$ NMR (400 MHz, DMSO-$d_6$) $\delta$ 7.99-7.94 (m, 1H), 7.47 (dd, $J$ = 1.2, 7.2 Hz, 1H), 7.08-7.03 (m, 1H), 0.36-0.31 (m, 9H).

Step 2: Synthesis of 1-(2-chloro-3-iodophenyl)ethanone

Acetyl chloride (9.70 g, 123.62 mmol, 8.82 mL) was slowly added dropwise to a solution of $AlCl_3$ (16.48 g, 123.62 mmol, 6.76 mL) in DCM (200 mL) at 0 °C for 0.5 h, and (2-chloro-3-iodo-phenyl)trimethyl-silane (32 g, 103.02 mmol) in DCM (200 mL) was added. The mixture was stirred at 20 °C for 2 h and quenched with aq. $Na_2CO_3$ (300 mL, 10%). The reaction mixture was poured into water (200 mL) and extracted with EtOAc (300 mL X 3). The organic layers were washed with brine (200 mL X 3), dried over anhydrous $Na_2SO_4$ and concentrated under reduced pressure to give a crude product.

The crude product was purified by silica gel column chromatography (5% EtOAc in petroleum ether) to give 1-(2-chloro-3-iodophenyl)ethanone (13 g, 33.99% yield) as colorless oil. $^1$H NMR (400 MHz, DMSO-$d_6$) δ 8.07 (dd, *J* = 1.6, 8.0 Hz, 1H), 7.61 (dd, *J* = 1.2, 7.6 Hz, 1H), 7.19 (t, *J* = 7.6 Hz, 1H), 2.55 (s, 3H).

Step 3: Synthesis of 2-(2-chloro-3-iodophenyl)-2-methyl-1,3-dioxolan

A mixture of toluene (200 mL), 1-(2-chloro-3-iodo-phenyl)ethanone (13 g, 46.35 mmol), ethylene glycol (8.63 g, 139.04 mmol) and TsOH (798.11 mg, 4.63 mmol) was degassed and purged with $N_2$ for 3 times, and then the mixture was stirred at 125 °C for 16 h under $N_2$ atmosphere. The mixture was poured into water (200 mL) and extracted with EtOAc (200 mL X 3). The organic layers were washed with brine (200 mL), dried over anhydrous $Na_2SO_4$ and concentrated under reduced pressure to give a crude product. The crude product was purified by silica gel column chromatography (6% EtOAc in petroleum ether) to give 2-(2-chloro-3-iodophenyl)-2-methyl-1,3-dioxolan (14 g, 29.08% yield, 31.25% purity) as colorless oil. MS (ESI) m/z = 324.9 [M+H]$^+$.

Step 4: Synthesis of ethyl 2-[2-chloro-3-(2-methyl-1,3-dioxolan-2-yl)phenyl]-2,2-difluoroacetate

A mixture of 2-(2-chloro-3-iodo-phenyl)-2-methyl-1,3-dioxolan (14 g, 43.14 mmol), ethyl-2-bromo-2,2-difluoroacetate (17.51 g, 86.28 mmol, 11.08 mL), Cu (8.22 g, 129.41 mmol, 917.82 μL) in DMSO (200 mL) was degassed and purged with $N_2$ for 3 times, and then the mixture was stirred at 80 °C for 16 h under $N_2$ atmosphere. The mixture was poured into water (300 mL) and extracted with EtOAc (300 mL X 3). The organic layers were washed with brine (200 mL), dried over anhydrous $Na_2SO_4$ and concentrated under reduced pressure to give a crude product. The crude product was purified by silica gel column chromatography (11% EtOAc in petroleum ether) to give ethyl 2-[2-chloro-3-(2-methyl-1,3-dioxolan-2-yl)phenyl]-2,2-difluoroacetate (6 g, 23.51% yield, 54.20% purity) as colorless oil. MS (ESI) m/z = 321.0 [M+H]$^+$.

Step 5: Synthesis of ethyl 2-(3-acetyl-2-chloro-phenyl)-2,2-difluoroacetate

To a solutiomn of ethyl 2-[2-chloro-3-(2-methyl-1,3-dioxolan-2-yl)phenyl]-2,2-difluoroacetate (6 g, 18.71 mmol) in MeOH (200 mL) was added HCl (6 M, 15 mL), and then the mixture was stirred at 40 °C for 1 h. The mixture was filtered under reduced pressure to give a residue. It was poured into water (50 mL) and extracted with EtOAc (30 mL X 3). The organic layers were washed with brine (20 mL × 3), dried over anhydrous $Na_2SO_4$ and concentrated under reduced pressure to give a crude product. The crude product was purified by silica gel column chromatography (8% EtOAc in petroleum ether) to give ethyl 2-(3-acetyl-2-chloro-phenyl)-2,2-difluoroacetate (6.4 g, 85.78% yield, 69.37% purity) as colorless oil. MS (ESI) m/z = 277.0 [M+H]$^+$.

Step 6: Synthesis of ethyl 2-[2-chloro-3-[(Z)-N-(1,1-dimethylethylsulfinyl)-C-methyl-carbonimidoyl]phenyl]-2,2-difluoroacetate

A mixture of ethyl 2-(3-acetyl-2-chloro-phenyl)-2,2-difluoroacetate (6 g, 21.69 mmol), 2-methylpropane-2-sulfinamide (3.94 g, 32.53 mmol) and $Ti(OEt)_4$ (14.84 g, 65.06 mmol, 13.49 mL) in THF (100 mL) was degassed and purged with $N_2$ for 3 times, and then the mixture was stirred at 80 °C for 16 h under $N_2$ atmosphere. The mixture was poured into water (80 mL), filtered and then extracted with EtOAc (50 mL X 6). The organic layers were washed with brine (30 mL X 3), dried over anhydrous $Na_2SO_4$ and concentrated under reduced pressure to give a crude product. The crude product was purified by silica gel column chromatography (29% MTBE in petroleum ether) to give ethyl 2-[2-chloro-3-[(Z)-N-(1,1-dimethylethylsulfinyl)-C-methyl-carbonimidoyl]phenyl]-2,2-difluoroacetate (5.2 g, 51.13% yield, 81% purity) as light yellow oil. $^1$H NMR (400 MHz, DMSO-$d_6$) δ 7.92 - 7.76 (m, 1H), 7.74 - 7.52 (m, 2H), 4.42 - 4.27 (m, 2H), 3.34 (s, 3H), 1.24 - 1.18 (m, 9H), 1.13 (s, 3H); MS (ESI) m/z = 380.1 [M+H]$^+$.

Step 7: Synthesis of (S)-N-[(1R)-1-[2-chloro-3-(1,1-difluoro-2-hydroxyethyl)phenyllethyl]-2-methyl-propane-2-sulfinamide

A mixture of ethyl 2-[2-chloro-3-[(Z)-N-(1,1-dimethylethylsulfinyl)-C-methyl-carbonimidoyl]phenyl]-2,2-difluoroacetate (2 g, 5.27 mmol) and L-selectride (1 M, 15.80 mL) in THF (50 mL) was degassed and purged with $N_2$ for 3 times, and then the mixture was stirred at -70 °C for 5 h under $N_2$ atmosphere. Sat. aq. $NH_4Cl$ (100 mL) was added dropwise to the reaction mixture at -70 °C, which was then extracted with EtOAc (50 mL X 3). The organic layers were dried over anhydrous $Na_2SO_4$ and concentrated under reduced pressure to give a crude product. After the primary purification by silica gel column chromatography (5% MeOH in DCM), two diastereomers were separated and purified by prep-HPLC (Xtimate C18 150*40 mm*10 μm, mobile phase: [water ($NH_3H_2O$)-ACN]; B%: 10%-55%, 20 min). MeCN was removed under reduced pressure, and the remaining solvent was removed by lyophilization to give a main product, (S)-N-[(1R)-1-[2-chloro-3-(1,1-difluoro-2-hydroxy-ethyl)phenyl]ethyl]-2-methyl-propane-2- sulfinamide (1.4 g, 69.64% yield, 89% purity, 100% ee) as a white solid. $^1H$ NMR (400 MHz, DMSO-$d_6$) δ 7.72 (br d, J = 6.4 Hz, 1H), 7.55 - 7.41 (m, 2H), 5.70 (br t, J = 6.4 Hz, 1H), 5.60 (br d, J = 5.2 Hz, 1H), 4.91 (quint, J = 6.0 Hz, 1H), 4.02 (dt, J = 6.4, 14.4 Hz, 2H), 1.46 (br d, J = 6.4 Hz, 3H), 1.11 (s, 9H); MS (ESI) m/z = 340.1 $[M+H]^+$.

Step 8: Synthesis of 2-[3-[(1R)-1-aminoethyl]-2-chloro-phenyl]-2,2-difluoroethanol

A mixture of (S)-N-[(1R)-1-[2-chloro-3-(1,1-difluoro-2-hydroxy-ethyl)phenyl]ethyl]-2-methyl-propane-2-sulfinamide (1.4 g, 4.12 mmol) and 4 N HCl/dioxane (10 mL) in dioxane (10 mL) was stirred at 20 °C for 2 h. The mixture was concentrated under reduced pressure to give **Intermediate A-4** (1.2 g, 88.77% yield, 82.93% purity, HCl salt) as a white solid. $^1H$ NMR (400 MHz, DMSO-$d_6$) δ 8.85 (br s, 3H), 7.96 (br d, *J* = 7.6 Hz, 1H), 7.68-7.62 (m, 1H), 7.62-7.55 (m, 1H), 5.75 (br s, 1H), 4.81 (br s, 1H), 4.03 (br t, *J* = 14.0 Hz, 2H), 1.53 (d, *J* = 6.8 Hz, 3H); MS (ESI) m/z = 236.1 $[M+H]^+$.

**Preparation Example 5: (R)-1-(2-fluoro-3-(1,1,2-trifluoroethyl)phenyl)ethan-1-amine (A-5)**

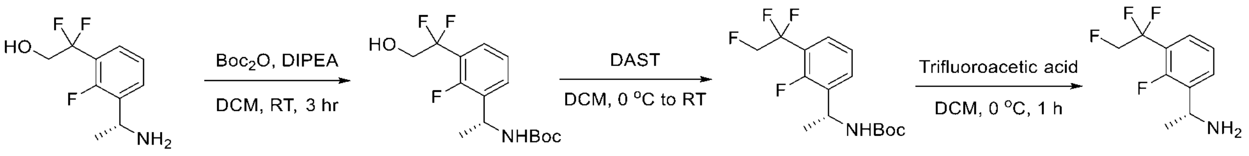


A-1 A-5-1 A-5-2 A-5

Step 1: Synthesis of (R)-(1-(3-(1,1-difluoro-2-hydroxyethyl)-2-fluorophenyl)ethyl)carbamate

To a solution of 2-{3-[(1R)-1-aminoethyl]-2-fluorophenyl}-2,2-difluoromethan-1-ol hydrochloride (500 mg, 1.96 mmol) in DCM (3.99 mL) was added di-tert-butyl dicarbonate (854 mg, 3.91 mmol) and DIPEA (0.68 mL, 3.91 mmol) at 0 °C, and then the mixture was stirred at room temperature for 3 h. The mixture was added with DCM and washed with water. The organic layers were dried over anhydrous $Na_2SO_4$, filtered and concentrated under reduced pressure. The residue was purified by silica gel column chromatography (Hex : EtOAc= 20: 1 to 10: 1) to give (R)-(1-(3-(1,1-difluoro-2-hydroxyethyl)-2-fluorophenyl)ethyl)carbamate (456 mg, 73% yield) as colorless oil. MS (ESI) m/z = 264.04 $[M+H]^+$.

Step 2: Synthesis of tert-butyl (R)-(1-(2-fluoro-3-(1,1,2-trifluoroethyl)phenyl)ethyl)carbamate

To a solution of (R)-(1-(3-(1,1-difluoro-2-hydroxyethyl)-2-fluorophenyl)ethyl)carbamate (221 mg, 0.692 mmol) in DCM (6.92 mL) was added DAST (0.37 mL, 2.77 mmol) at 0 °C under $N_2$ atmosphere, and then the mixture was warmed to room temperature and stirred overnight. The mixture was quenched with sat. $NaHCO_3$ and extracted with DCM. The organic layers were extracted with sat. $NH_4Cl$ and washed with brine. The organic layers were dried over anhydrous $Na_2SO_4$, filtered and concentrated under reduced pressure. It was purified by silica gel column chromatography (hexane : EtOAc = 20:1 to 10:1) to give tert-butyl (R)-(1-(2-fluoro-3-(1,1,2-trifluoroethyl)phenyl)ethyl)carbamate (26 mg, 12% yield) as colorless oil. MS (ESI) m/z = 265.99 $[M+H]^+$.

Step 3: Synthesis of (R)-1-(2-fluoro-3-(1,1,2-trifluoroethyl)phenyl)ethan-1-amine

TFA (0.15 mL) was added dropwise to a solution of tert-butyl (R)-(1-(2-fluoro-3-(1,1,2-trifluoroethyl)phenyl)ethyl) carbamate (56 mg, 0.174 mmol) in DCM (0.7 mL), and then the mixture was stirred at 0 °C for 1 h. The mixture was warmed to room temperature and quenched with sat. $NaHCO_3$. The organic solvent was removed by filtration under reduced pressure, and the aqueous layer was extracted with DCM. The organic layer was dried over anhydrous $Na_2SO_4$ and concentrated under reduced pressure to give **Intermediate A-5** (34.5 mg, 89% yield) as yellow oil. MS (ESI) m/z = 222.06 $[M+H]^+$.

**Preparation Example 6: (1R)-1-[3-(difluoromethyl)-5-nitro-phenyl]ethanamine (A-6)**

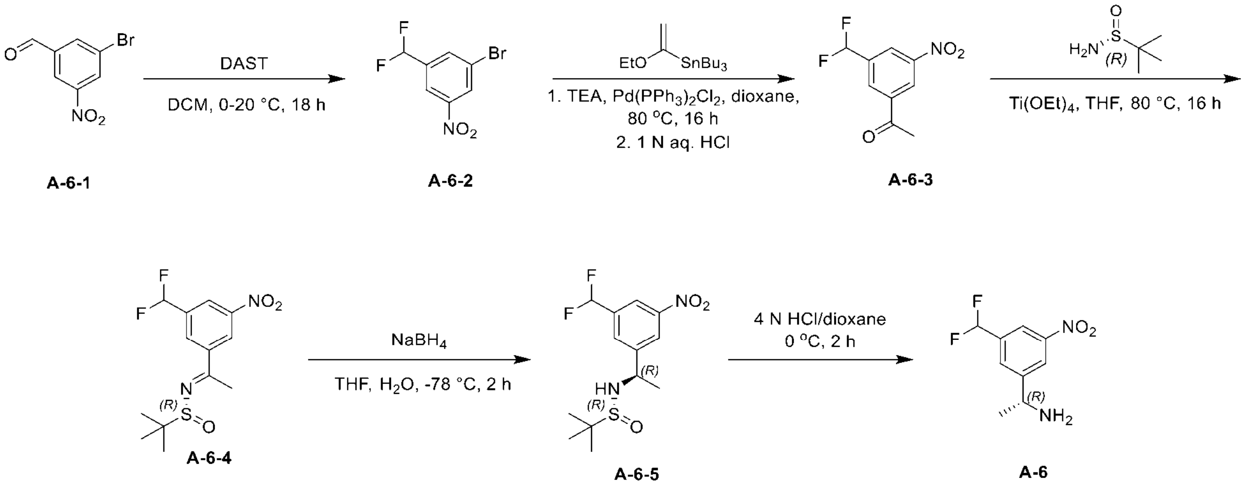


A-6-1 A-6-2 A-6-3 A-6-4 A-6-5 A-6

Step 1: Synthesis of 1-bromo-3-(difluoromethyl)-5-nitrobenzene

A mixed solution of 3-bromo-5-nitro-benzaldehyde (13.7 g, 59.56 mmol) and DAST (48.00 g, 297.81 mmol, 39.35 mL) in DCM (140 mL) was degassed and purged with $N_2$ for 3 times, and then stirred at 0 °C for 1 h. The mixture was warmed to 20 °C and stirred for 17 h under $N_2$ atmosphere. The reaction mixture was poured into ice-cold water and extracted with DCM (300 mL) and EtOAc (200 mL X 3). The combined organic layers were washed with brine (200mL X 2), dried over anhydrous $Na_2SO_4$, and then filtered under reduced pressure to give a crude product. The crude product was purified by silica gel column chromatography (0-10% EtOAc/petroleum ether) to give 1-bromo-3-(difluoromethyl)-5-nitrobenzene (14.27 g, 95.07% yield) as colorless oil. $^1$H NMR (400 MHz, DMSO-$d_6$) δ 8.57 (s, 1H), 8.40 (s, 1H), 8.29 (s, 1H), 7.34 - 7.05 (m, 1H).

Step 2: Synthesis of 1-[3-(difluoromethyl)-5-nitrophenyllethanone

A mixture of 1-bromo-3-(difluoromethyl)-5-nitrobenzene (12.27 g, 48.69 mmol), tributyl(1-ethoxyvinyl)stannane (19.5 g, 53.99 mmol, 18.22 mL), $Pd(PPh_3)_2Cl_2$ (3.42 g, 4.87 mmol) and TEA (9.85 g, 97.38 mmol, 13.55 mL) in dioxane (120 mL) was degassed and purged with $N_2$ for 3 times, and then the mixture was stirred at 80 °C for 12 h under $N_2$ atmosphere. The mixture was quenched with 4 N HCl (24.34 mL) and then stirred at 20 °C for 1 h. The mixture was poured into water (500 mL) and extracted with EtOAc (300 mL X 3). The organic layers were washed with brine (500mL), dried over anhydrous $Na_2SO_4$ and concentrated under reduced pressure to give a crude product. The aqueous layers were stirred while KF (2.5 g) was slowly added dropwise. The crude product was purified by silica gel column chromatography (0~10% EtOAc/petroleum ether) to give 1-[3-(difluoromethyl)-5-nitrophenyl]ethanone (8.74 g, 83.43% yield) as yellow oil. $^1$H NMR (400 MHz, DMSO-$d_6$) δ 8.77 (s, 1H), 8.63 (s, 1H), 8.53 (s, 1H), 7.44 - 7.14 (m, 1H), 2.73 (s, 3H).

Step 3: Synthesis of (NE,R)-N-[1-[3-(difluoromethyl)-5-nitro-phenyllethylidene]-2-methyl-propane-2-sulfinamide

A mixture of 1-[3-(difluoromethyl)-5-nitrophenyl]ethanone (10 g, 46.48 mmol), (R)-2-methylpropane-2-sulfinamide (8.45 g, 69.72 mmol) and $Ti(OEt)_4$ (31.81 g, 139.44 mmol, 28.91 mL) in THF (100 mL) was degassed and purged with $N_2$ for 3 times, and then the mixture was stirred at 80 °C for 16 h under $N_2$ atmosphere. The mixture was poured into water (200 mL X 3), filtered and extracted with EtOAc (500 mL X 6). The organic layers were washed with brine (30 mL X 3), dried over anhydrous $Na_2SO_4$ and concentrated under reduced pressure to give a crude product. The crude product was purified by silica gel column chromatography (0~30% EtOAc/petroleum ether) to give (NE,R)-N-[1-[3-(difluoromethyl)-5-nitrophenyl]ethylidene]-2-methyl-propane-2-sulfinamide (12.83 g, 82.31% yield, 94.92% purity) as yellow oil. MS (ESI) m/z = 319.1 $[M+H]^+$.

Step 4: Synthesis of (R)-N-[(1R)-1-[3-(difluoromethyl)-5-nitro-phenyl]ethyl]-2-methyl-propane-2-sulfinamide

To a solution of (NE,R)-N-[1-[3-(difluoromethyl)-5-nitro-phenyl]ethylidene]-2-methyl-propane-2-sulfinamide (4 g, 12.57 mmol) in THF (40 mL) and $H_2O$ (0.75 mL) was added $NaBH_4$ (332.76 mg, 8.80 mmol) at -78 °C in 3 batches, and then the mixture was stirred at -78 °C for 2 h under $N_2$ atmosphere. The mixture was quenched with sat. $NH_4Cl$ (150 mL), diluted with EtOAc (100 mL X 3), and then extracted with EtOAc (100 mL X 3). The organic layers were dried over anhydrous $Na_2SO_4$ and concentrated under reduced pressure to give a crude product. The crude product was purified by

silica gel column chromatography (0~25% EtOAc/petroleum ether) to give (R)-N-[(1R)-1-[3-(difluoromethyl)-5-nitro-phenyl]ethyl]-2-methyl-propane-2-sulfinamide (3.21 g, 79.26% yield, 99.39% purity, 99.00% ee) as yellow oil. $^1H$ NMR (400 MHz, DMSO-$d_6$) δ 8.51 (s, 1H), 8.30 (s, 1H), 8.11 (s, 1H), 7.37 - 7.07 (m, 1H), 6.05 (d, *J* = 8.4 Hz, 1H), 4.69 - 4.60 (m, 1H), 1.45 (d, *J* = 6.8 Hz, 3H), 1.14 (s, 9H); MS (ESI) m/z = 321.1 $[M+H]^+$.

Step 5: Synthesis of (1R)-1-[3-(difluoromethyl)-5-nitro-phenyl]ethanamine

A mixture of (R)-N-[(1R)-1-[3-(difluoromethyl)-5-nitro-phenyl]ethyl]-2-methylpropane-2-sulfinamide (532 mg, 1.66 mmol) in 4 N HCl/dioxane (30 mL) was stirred at 0 °C for 2 h. The mixture was concentrated under reduced pressure to give **Intermediate A-6** (400 mg, 98.29% yield, 88.22% purity, HCl) as a brown solid. MS (ESI) m/z = 217.0 $[M+H]^+$.

**Preparation Example 7: (1R)-1-[2-fluoro-5-nitro-3-(trifluoromethyl)phenyl]ethanamine (A-7)**

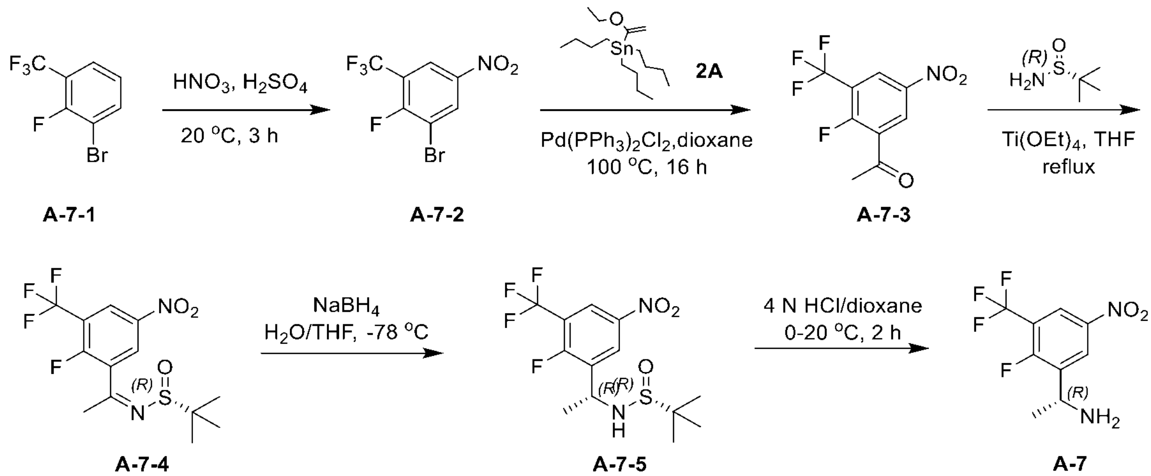


Step 1: Synthesis of 1-bromo-2-fluoro-5-nitro-3-(trifluoromethyl)benzene

To a solution of 1-bromo-2-fluoro-3-(trifluoromethyl)benzene (10 g, 41.15 mmol) in $H_2SO_4$ (40 mL) was slowly added dropwise $HNO_3$ (25.71 g, 408.01 mmol, 18.36 mL) at 0 °C, and then the mixture was warmed to 20 °C and stirred for 3 h under $N_2$ atmosphere. The mixture was quenched with ice-cold water (100 mL) and extracted with EtOAc (50 mL × 3). The organic layers were washed with brine (50 mL), dried over anhydrous $Na_2SO_4$ and concentrated under reduced pressure to give a crude product. The crude product was purified by silica column chromatography (2% EtOAc in petroleum ether) to give 1-bromo-2-fluoro-5-nitro-3-(trifluoromethyl)benzene (5.9 g, 49.78% yield) as colorless oil. $^1H$ NMR (400 MHz, DMSO-$d_6$) δ 8.95 (dd, *J* = 2.4, 5.2 Hz, 1H), 8.55 - 8.51 (m, 1H).

Step 2: Synthesis of 1-[2-fluoro-5-nitro-3-(trifluoromethyl)phenyllethanone

A mixture of 1-bromo-2-fluoro-5-nitro-3-(trifluoromethyl)benzene (5.9 g, 20.49 mmol), 1-tributyl(1-ethoxyvinyl) stannane (8.41 g, 23.29 mmol, 7.86 mL) and Pd(dppf)$Cl_2$ (1.44 g, 2.05 mmol) in dioxane (50 mL) was degassed and purged with $N_2$ for 3 times, and then the mixture was stirred at 100 °C for 16 h under $N_2$ atmosphere. The mixture was cooled to 0 °C and stirred for 1 h after the addition of HCl solution (4N, 50 mL). Following the addition of water (100 mL), the reaction mixture was extracted with EtOAc (100 mL X 3). The combined organic layers were dried over $Na_2SO_4$ and concentrated under reduced pressure to give a residue. It was purified by silica gel column chromatography (5% EtOAc in petroleum ether) to give 1-[2-fluoro-5-nitro-3-(trifluoromethyl)phenyl]ethanone (1.6 g, 31.10% yield) as colorless oil. $^1H$ NMR (400 MHz, DMSO-$d_6$) δ 8.81 (dd, *J* = 2.8, 5.6 Hz, 1H), 8.71 (dd, *J* = 2.8, 5.2 Hz, 1H), 2.70 (d, *J* = 4.0 Hz, 3H).

Step 3: Synthesis of (NZ,R)-N-[1-[2-fluoro-5-nitro-3-(trifluoromethyl)phenyl]ethylidene]-2-methyll-propane-2-sulfinamide

A mixture of 1-[2-fluoro-5-nitro-3-(trifluoromethyl)phenyl]ethanone (2.75 g, 10.95 mmol), (R)-2-methylpropane-2-sulfinamide (1.46 g, 12.05 mmol) and $Ti(OEt)_4$ (7.49 g, 32.85 mmol, 6.81 mL) in THF (30 mL) was degassed

and purged with $N_2$ for 3 times, and then the mixture was stirred at 80 °C for 16 h under $N_2$ atmosphere. The mixture was poured into water (50 mL) and extracted with EtOAc (50 mL X 6). The organic layers were washed with brine (50 mL X 2), dried over anhydrous $Na_2SO_4$ and concentrated under reduced pressure to give a crude product. The crude product was purified by silica gel column chromatography (7% EtOAc in petroleum ether) to give (NZ,R)-N-[1-[2-fluoro-5-nitro-3-(trifluoromethyl)phenyl]ethylidene]-2-methyl-1-propane-2-sulfinamide (3.43 g, 86.21% yield, 97.51% purity) as yellow oil. $^1$H NMR (400 MHz, DMSO-d$_6$) δ 8.80 (br d, *J* = 2.4 Hz, 1H), 8.64 - 8.52 (m, 1H), 2.78 - 2.54 (m, 3H), 1.24 (s, 6H), 1.21 - 1.11 (m, 3H); MS (ESI) m/z = 354.9 [M+H]$^+$.

Step 4: Synthesis of (R)-N-[(1R)-1-[2-fluoro-5-nitro-3-(trifluoromethyl)phenyllethyl]-2-methyl-propane-2-sulfinamide

A solution of (NZ,R)-N-[1-[2-fluoro-5-nitro-3-(trifluoromethyl)phenyl]ethylidene]-2-methyl-1-propane-2-sulfinamide (3.43 g, 9.68 mmol) in THF (35 mL) and $H_2O$ (1 mL) was degassed and purged with $N_2$ for 3 times. Then, $NaBH_4$ (230 mg, 6.08 mmol) was added at - 70 °C and the mixture was stirred at -70 °C for 2 h under $N_2$ atmosphere. The mixture was quenched with sat. $NH_4Cl$ (50 mL) at 20 °C, diluted with EtOAc (50 mL), and then extracted with EtOAc (50 mL X 3). The organic layers were dried over anhydrous $Na_2SO_4$ and concentrated under reduced pressure to give a crude product. The crude product was purified by silica gel column chromatography (20% EtOAc in petroleum ether) to give (R)-N-[(1R)-1-[2-fluoro-5-nitro-3-(trifluoromethyl)phenyl]ethyl]-2-methyl-propane-2-sulfinamide (1.72 g, 49.32% yield, 99.20% purity, 100% ee) as yellow oil. $^1$H NMR (400 MHz, DMSO-*d*$_6$) δ 8.83 (dd, J = 2.8, 5.6 Hz, 1H), 8.46 (dd, J = 2.8, 5.6 Hz, 1H), 6.14 (d, J =8.8 Hz, 1H), 4.83 - 4.73 (m, 1H), 1.48 (d, J = 6.8 Hz, 3H), 1.13 (s, 9H); MS (ESI) m/z = 356.9 [M+H]+.

Step 5: Synthesis of (1R)-1-[2-fluoro-5-nitro-3-(trifluoromethyl)phenyl]ethanamine

To a solution of (R)-N-[(1R)-1-[2-fluoro-5-nitro-3-(trifluoromethyl)phenyl]ethyl]-2-methyl-propane-2-sulfinamide (1.5 g, 4.21 mmol) in dioxane (10 mL) was added 4 N HCl/dioxane (10 mL), and then the mixture was stirred at 20 °C for 2 h. The mixture was concentrated under reduced pressure to give **Intermediate A-7** (1.2 g, 88.77% yield, 82.93% purity, HCl) as a white solid. MS (ESI) m/z = 252.9 [M+H]$^+$.

**Preparation Example 8: (1R)-1-[2-methyl-5-nitro-3-(trifluoromethyl)phenyl]ethanamine (A-8)**

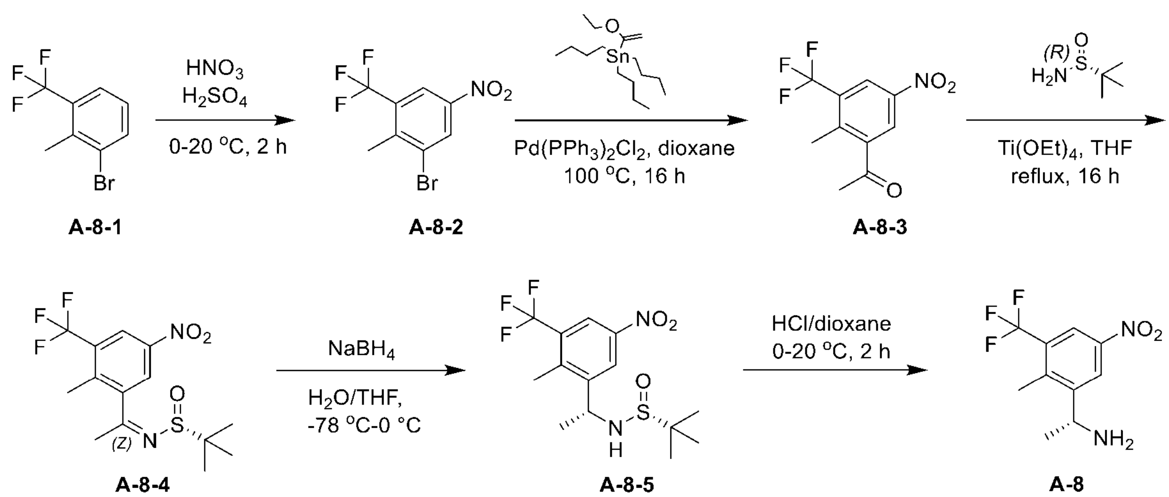

A-8-1 A-8-2 A-8-3 A-8-4 A-8-5 A-8

Step 1: Synthesis of 1-bromo-2-methyl-5-nitro-3-(trifluoromethyl)benzene

$HNO_3$ (46.45 g, 737.15 mmol, 33.18 mL) was slowly added dropwise to a solution of 1-bromo-2-methyl-3-(trifluoromethyl)benzene (10 g, 41.84 mmol) in $H_2SO_4$ (75 mL) at 0 °C, and then the mixture was heated to 20 °C and stirred for 2 h under $N_2$ atmosphere. The mixture was quenched with ice-cold water (250 mL) and extracted with EtOAc (100 mL × 3). The organic layers were washed with brine (50 mL), dried over anhydrous $Na_2SO_4$ and concentrated under reduced pressure to give a crude product. The crude product was purified by silica column chromatography (100% petroleum ether) to give 1-bromo-2-methyl-5-nitro-3-(trifluoromethyl)benzene (12 g, crude) as yellow oil. $^1$H NMR (400 MHz, DMSO-*d*$_6$) δ 8.71 (d, *J* = 2.0 Hz, 1H), 8.39 (d, *J* = 2.0 Hz, 1H), 2.59 (d, *J* = 1.2 Hz, 3H)

Step 2: Synthesis of 1-[2-methyl-5-nitro-3-(trifluoromethyl)phenyl]ethanone

A mixture of 1-bromo-2-methyl-5-nitro-3-(trifluoromethyl)benzene (12 g, 42.25 mmol), 1-tributyl(1-ethoxyvinyl) stannane (15.1 g, 41.81 mmol, 14.11 mL) and Pd(dppf)$Cl_2$ (2.97 g, 4.22 mmol) in dioxane (100 mL) was degassed and purged with $N_2$ for 3 times, and then the mixture was stirred at 100 °C for 16 under $N_2$ atmosphere. The mixture was cooled to 0 °C and stirred for 1 h after the addition of HCl solution (2N, 100 mL). Following the addition of water (100 mL), the reaction mixture was extracted with EtOAc (100 mL X 3). The combined organic layers were dried over anhydrous $Na_2SO_4$, and concentrated under reduced pressure to give a residue. It was purified by silica gel column chromatography (10% EtOAc in petroleum ether) to give 1-[2-methyl-5-nitro-3-(trifluoromethyl)phenyl]ethanone (5.3 g, 50.75% yield) as yellow oil. $^1H$ NMR (400 MHz, DMSO-$d_6$) $\delta$ 8.72 (d, *J* = 2.0 Hz, 1H), 8.47 (d, *J* = 2.0 Hz, 1H), 2.66 (s, 3H), 2.50 (s, 3H).

Step 3: Synthesis of (NZ,R)-2-methyl-N-[1-[2-methyl-5-nitro-3-(trifluoromethyl)phenyl]ethylidene]propane-2-sulfinamide

A mixture of 1-[2-methyl-5-nitro-3-(trifluoromethyl)phenyl]ethanone (5.3 g, 21.44 mmol), (R)-2-methylpropane-2-sulfinamide (2.86 g, 23.58 mmol) and $Ti(OEt)_4$ (14.67 g, 64.32 mmol, 13.34 mL) in THF (55 mL) was degassed and purged with $N_2$ for 3 times, and then the mixture was stirred at 80 °C for 16 h under $N_2$ atmosphere. The mixture was poured into water (100 mL) and EtOAc (100 mL) and filtered, and then the filtrate was extracted with EtOAc (50 mL X 3). The organic layers were washed with brine (50 mL X 2), dried over anhydrous $Na_2SO_4$, and concentrated under reduced pressure to give a crude product. The crude product was purified by silica gel column chromatography (8% EtOAc in petroleum ether) to give (NZ,R)-2-methyl-N-[1-[2-methyl-5-nitro-3-(trifluoromethyl)phenyl]ethylidene]propane-2-sulfinamide (6.7 g, 88.90% yield, 99.67% purity) as yellow oil. MS (ESI) m/z = 351.0 $[M+H]^+$.

Step 4: Synthesis of (R)-2-methyl-N-[(1R)-1-[2-methyl-5-nitro-3-(trifluoromethyl)phenyl]ethyl]propane-2-sulfinamide

To a solution of (NZ,R)-2-methyl-N-[1-[2-methyl-5-nitro-3-(trifluoromethyl)phenyl]ethylidene]propane-2-sulfinamide (6.70 g, 19.12 mmol) in THF (80 mL) and $H_2O$ (2 mL) was added $NaBH_4$ (230 mg, 6.08 mmol) at -78 °C in 3 batches, and then the mixture was slowly heated from -78 °C to 0 °C and stirred for 3 h under $N_2$ atmosphere. The mixture was quenched with sat. $NH_4Cl$ (50 mL) at 20 °C, diluted with EtOAc (50 mL) and extracted with EtOAc (50 mL X 3). The organic layers were dried over anhydrous $Na_2SO_4$ and concentrated under reduced pressure to give a crude product. The crude product was purified by silica gel column chromatography (20% EtOAc/petroleum ether) to give (R)-2-methyl-N-[(1R)-1-[2-methyl-5-nitro-3-(trifluoromethyl)phenyl]ethyl]propane-2-sulfinamide (1.4 g, 19.99% yield, 96.23% purity, 99.44% ee) as yellow oil. $^1H$ NMR (400 MHz, DMSO-*$d_6$*) $\delta$ 8.59 (d, *J* = 2.4 Hz, 1H), 8.31 (d, *J* = 2.4 Hz, 1H), 5.74 (d, *J* = 6.0 Hz, 1H), 4.85 (quint, *J* = 6.4 Hz, 1H), 2.54 (s, 3H), 1.51 (d, *J* = 6.4 Hz, 3H), 1.12 (s, 9H); MS (ESI) m/z = 353.0 $[M+H]^+$.

Step 5: Synthesis of (1R)-1-[2-methyl-5-nitro-3-(trifluoromethyl)phenyl]ethanamine

To a solution of (R)-2-methyl-N-[(1R)-1-[2-methyl-5-nitro-3-(trifluoromethyl)phenyl]ethyl]propane-2-sulfinamide (1.40 g, 3.97 mmol) in dioxane (5 L) was added HCl/dioxane (5 mL) at 0°C, and then the mixture was stirred at 20°C for 2 h. The mixture was concentrated under reduced pressure. The residue was stirred at 20 °C for 12 h with the addition of MTBE (20 mL). The mixture was filtered to give **Intermediate A-8** (1 g, 86.11% yield, 97.39% purity, HCl) as a white solid. MS (ESI) m/z = 248.9 $[M+H]^+$.

**Preparation Example 9: (1R)-1-[3-(difluoromethyl)-2-fluoro-5-nitrophenyl]ethanamine (A-9)**

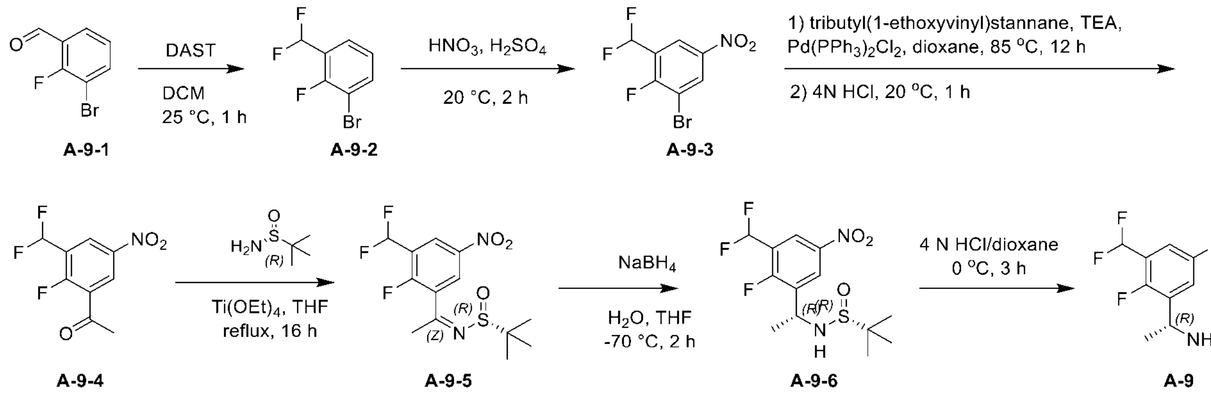


A-9-1 A-9-2 A-9-3

A-9-4 A-9-5 A-9-6 A-9

Step 1: Synthesis of 1-bromo-3-(difluoromethyl)-2-fluoro-5-nitrobenzene

$HNO_3$ (14.51 g, 230.22 mmol, 10.36 mL) was slowly added dropwise to a solution of 1-bromo-3-(trifluoromethyl)-2-fluorobenzene (3.7 g, 16.44 mmol) in $H_2SO_4$ (30 mL) at 0 °C, and then the mixture was heated to 20 °C and stirred for 2 h. The mixture was quenched with ice-cold water (120 mL) and extracted with EtOAc (40 mL × 3). The organic layers were washed with brine (120 mL), dried over anhydrous $Na_2SO_4$ and concentrated under reduced pressure to give a crude product. The crude product was purified by silica column chromatography (100% petroleum ether) to give 1-bromo-3-(difluoromethyl)-2-fluoro-5-nitrobenzene (1.8 g, 40.54% yield) as colorless oil. $^1H$ NMR (400 MHz, DMSO-$d_6$) δ 8.78 (br s, 1H), 8.47 (br d, $J$ = 2.4 Hz, 1H), 7.34 (dt, $J$ = 4.0, 53.4 Hz, 1H).

Step 2: Synthesis of 1-[3-(difluoromethyl)-2-fluoro-5-nitro-phenyllethanone

A mixture of 1-bromo-3-(difluoromethyl)-2-fluoro-5-nitrobenzene (1.8 g, 6.67 mmol), 1-tributyl(1-ethoxyvinyl) stanene (2.65 g, 7.33 mmol, 2.48 mL) and Pd(dppf)$Cl_2$ (233.96 mg, 333.33 μmol) in dioxane (30 mL) was degassed and purged with $N_2$ for 3 times, and then the mixture was stirred at 100 °C for 16 under $N_2$ atmosphere. HCl solution (4N, 30 mL) was added to the mixture, which was then stirred at 20°C for 1 h. After water (50 mL) was added, the reaction mixture was extracted with EtOAc (50 mL X 3). The combined organic layers were washed with brine (50 mL X 3), dried over anhydrous $Na_2SO_4$, and concentrated under reduced pressure to give a residue. It was purified by silica gel column chromatography (2% EtOAc in petroleum ether) to give 1-[3-(difluoromethyl)-2-fluoro-5-nitro-phenyl]ethanone (1.42 g, 91.23% yield) as yellow oil. $^1H$ NMR (400 MHz, DMSO-$d_6$) δ 8.72 - 8.68 (m, 1H), 8.68 - 8.64 (m, 1H), 7.39 (t, $J$ = 53.2 Hz, 1H), 2.69 (d, $J$ = 4.0 Hz, 3H).

Step 3: Synthesis of (NZ,R)-N-[1-[3-(difluoromethyl)-2-fluoro-5-nitrophenyl]ethylidene]-2-methyl-propane-2-sulfinamide

A mixture of 1-[3-(difluoromethyl)-2-fluoro-5-nitro-phenyl]ethanone (1.4 g, 6.00 mmol), (R)-2-methylpropane-2-sulfinamide (727.80 mg, 6.00 mmol) and $Ti(OEt)_4$ (4.11 g, 18.01 mmol, 3.74 mL) in THF (8 mL) was degassed and purged with $N_2$ for 3 times, and then the mixture was stirred at 80 °C for 16 under $N_2$ atmosphere. The mixture was poured into water (50 mL) and extracted with EtOAc (50 mL X 3). The organic layers were washed with brine (20 mL X 3), dried over anhydrous $Na_2SO_4$, and concentrated under reduced pressure to give a crude product. The crude product was purified by silica gel column chromatography (35% EtOAc in petroleum ether) to give (NZ,R)-N-[1-[3-(difluoromethyl)-2-fluoro-5-nitrophenyl]ethylidene]-2-methyl-propane-2-sulfinamide (1.42 g, 70.29% yield) as yellow oil. $^1H$ NMR (400 MHz, DMSO-$d_6$) δ 8.68 - 8.64 (m, 1H), 8.56 (br s, 1H), 7.51 - 7.24 (m, 1H), 2.76 (s, 3H), 1.24 (s, 9H).

Step 4: Synthesis of (R)-N-[(1R)-1-[3-(difluoromethyl)-2-fluoro-5-nitrophenyl]ethyl]-2-methyl-propane-2-sulfinamide

$NaBH_4$ (127.78 mg, 3.38 mmol) was added to a solution of (NZ,R)-N-[1-[3-(difluoromethyl)-2-fluoro-5-nitro-phenyl]ethylidene]-2-methyl-propane-2-sulfinamide (1.42 g, 4.22 mmol) in THF (20 mL) and $H_2O$ (0.75 mL), and then the mixture was stirred at -70 °C for 2 h under $N_2$ atmosphere. The mixture was quenched with sat. $NH_4Cl$ (30 mL) at 20°C, diluted with EtOAc (30 mL) and extracted with EtOAc (30 mL X 3). The organic layers were dried over anhydrous $Na_2SO_4$ and concentrated under reduced pressure to give a crude product. The crude product was purified by silica gel column chromatography (100% petroleum ether) to give (R)-N-[(1R)-1-[3-(difluoromethyl)-2-fluoro-5-nitro-phenyl]ethyl]-2-methyl-propane-2-sulfinamide (902 mg, 62.04% yield, 98.25% purity, 100% ee) as colorless oil. MS (ESI) m/z = 339.1 $[M+H]^+$.

Step 5: Synthesis of (1R)-1-[3-(difluoromethyl)-2-fluoro-5-nitro-phenyl]ethanamine

A mixture of (R)-N-[(1R)-1-[3-(difluoromethyl)-2-fluoro-5-nitro-phenyl]ethyl]-2-methyl-propane-2-sulfinamide (900 mg, 2.66 mmol) in 4 N HCl/dioxane (5 mL) was degassed and purged with $N_2$ for 3 times, and then the mixture was stirred at 25 °C for 2 h under $N_2$ atmosphere. The mixture was concentrated under reduced pressure to give **Intermediate A-9** (700 mg, 96.58% yield, 99.33% purity, HCl) as a white solid. $^1H$ NMR (400 MHz, DMSO-$d_6$) δ 8.90 (br s, 4H), 8.52 (br d, *J* = 1.6 Hz, 1H), 7.38 (br t, *J* = 53.2 Hz, 1H), 4.77 (br s, 1H), 1.61 - 1.52 (m, 3H); MS (ESI) m/z = 235.1 [M+H]$^+$.

**Preparation Example 10: 3-[(1R)-1-aminoethyl]-5-(trifluoromethyl)amine (A-10)**

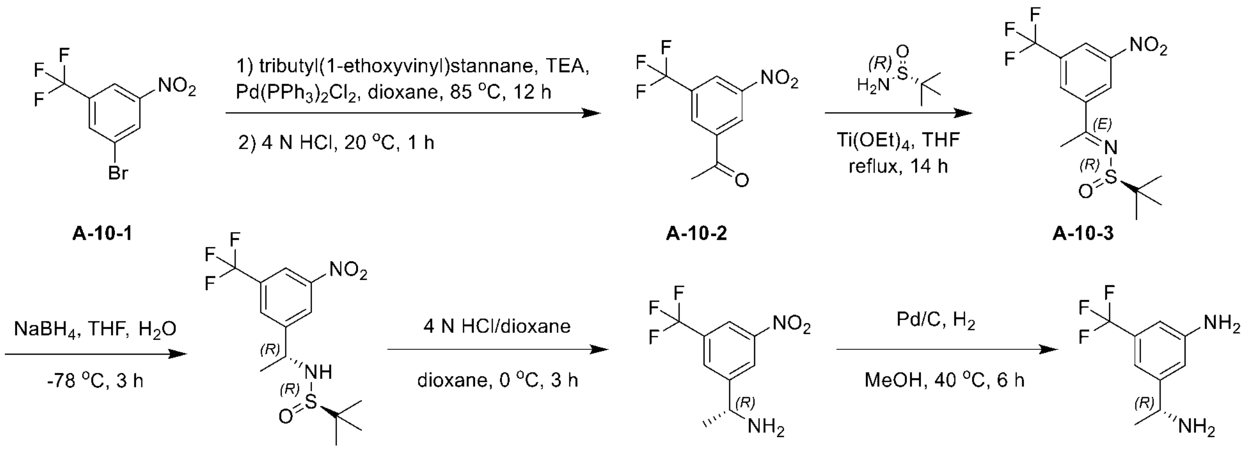


A-10-4 A-10-5 A-10

Step 1: Synthesis of 1-[3-nitro-5-(trifluoromethyl)phenyl]ethanone

A mixture of 1-bromo-3-nitro-5-(trifluoromethyl)benzene (50 g, 185.18 mmol), 1-tributyl(1-ethoxyvinyl)stannane (70.2 g, 194.38 mmol, 65.61 mL), Pd(dppf)$Cl_2$ (13.00 g, 18.52 mmol) and TEA (37.48 g, 370.37 mmol, 51.55 mL) in dioxane (500 mL) was degassed and purged with $N_2$ for 3 times, and then the mixture was stirred at 80 °C for 16h under $N_2$ atmosphere. HCl solution (4N, 200 mL) was added to the mixture, which was then stirred at 20 °C for 1 h. After water (200 mL) was added, the reaction mixture was extracted with EtOAc (150 mL X 3). The combined organic layers were washed with brine (100 mL X 3), dried over anhydrous $Na_2SO_4$ and then concentrated under reduced pressure to give a residue. It was purified by silica gel column chromatography (5% EtOAc in petroleum ether) to give 1-[3-nitro-5-(trifluoromethyl) phenyl]ethanone (66 g, 76.43% yield) as yellow oil. $^1H$ NMR (400 MHz, DMSO-$d_6$) δ 8.85 (s, 1H), 8.73 (s, 1H), 8.63 (s, 1H), 2.76 (s, 3H).

Step 2: Synthesis of (NE,R)-2-methyl-N-[1-[3-nitro-5-(trifluoromethyl)phenyl]ethylidene]propane-2-sulfinamide

A mixture of 1-[3-nitro-5-(trifluoromethyl)phenyl]ethanone (66 g, 283.09 mmol), (R)-2-methylpropane-2-sulfinamide (44.60 g, 368.01 mmol) and Ti(OEt)$_4$ (161.44 g, 707.72 mmol, 146.76 mL) in THF (650 mL) was degassed and purged with $N_2$ for 3 times, and then the mixture was stirred at 85 °C for 16 h under $N_2$ atmosphere. The mixture was poured into water (200 mL) and EtOAc (200 mL) and filtered, and the filtrate was extracted with EtOAc (200 mL X 3). The organic layers were washed with brine (200 mL X 2), dried over anhydrous $Na_2SO_4$ and concentrated under reduced pressure to give a crude product. The crude product was purified by silica gel column chromatography (12% EtOAc in petroleum ether) to give (NE,R)-2-methyl-N-[1-[3-nitro-5-(trifluoromethyl)phenyl]ethylidene]propane-2-sulfinamide (77 g, 71.07% yield, 87.88% purity) as yellow oil. $^1H$ NMR (400 MHz, DMSO-$d_6$) δ 8.84 (s, 1H), 8.65 (s, 1H), 8.54 (s, 1H), 2.86 (s, 3H), 1.25 (s, 9H); MS (ESI) m/z = 336.9 [M+H]$^+$).

Step 3: Synthesis of (R)-2-methyl-N-[(1R)-1-[3-nitro-5-(trifluoromethyl)phenyl]ethyl]propane-2-sulfinamide

To a solution of (NE,R)-2-methyl-N-[1-[3-nitro-5-(trifluoromethyl)phenyl]ethylidene]propane-2-sulfinamide (38.5 g, 114.47 mmol) in THF (350 mL) and $H_2O$ (7 mL) was added $NaBH_4$ (3.27 g, 86.43 mmol) at -78 °C in 3 batches, and then the mixture was stirred at -78 °C for 3 h under $N_2$ atmosphere. The mixture was quenched with sat. $NH_4Cl$ (150 mL) at 20

°C, and then it was poured into water (200 mL) and extracted with EtOAc (200 mL X 3). The organic layers were washed with brine (100 mL), dried over anhydrous $Na_2SO_4$ and concentrated under reduced pressure to give a crude product. The crude product was purified by silica gel column chromatography (15% EtOAc in petroleum ether) to give (R)-2-methyl-N-[(1R)-1-[3-nitro-5-(trifluoromethyl)phenyl]ethyl]propane-2-sulfinamide (30 g, 32.01 % yield, 82.663% purity, 100% ee) as a green solid. $^1H$ NMR (400 MHz, DMSO-$d_6$) δ 8.63 (s, 1H), 8.38 (s, 1H), 8.29 (s, 1H), 6.07 (d, *J* = 8.8 Hz, 1H), 4.72 - 4.65 (m, 1H), 1.45 (d, *J* = 6.8 Hz, 3H), 1.14 (s, 9H); MS (ESI) m/z = 338.9 $[M+H]^+$.

Step 4: Synthesis of (1R)-1-[3-nitro-5-(trifluoromethyl)phenyl]ethanamine

To a solution of the resulting product (R)-2-methyl-N-[(1R)-1-[3-nitro-5-(trifluoromethyl)phenyl]ethyl]propane-2-sulfinamide (30 g, 88.67 mmol) in dioxane (30 mL) was added 4 N HCl/dioxane (60 mL), and then the mixture was stirred at 20 °C for 3 h. The mixture was concentrated under reduced pressure and stirred at 20 °C for 12 h with the addition of MTBE (100 mL). It was filtered to give (1R)-1-[3-nitro-5-(trifluoromethyl)phenyl]ethanamine (23 g, 91.02% yield, 94.96% purity, HCl) as a white solid. $^1H$ NMR (400 MHz, DMSO-$d_6$) δ 8.93 - 8.61 (m, 4H), 8.51 (s, 1H), 8.45 (s, 1H), 4.74 (q, *J* = 6.8 Hz, 1H), 1.58 (d, *J* = 6.8 Hz, 3H); MS (ESI) m/z = 234.9 $[M+H]^+$.

Step 5: Synthesis of 3-[(1R)-1-aminoethyl]-5-(trifluoromethyl)amine

A mixture of (1R)-1-[3-nitro-5-(trifluoromethyl)phenyl]ethanamine (10 g, 42.70 mmol) and Pd/C (2 g, 10% purity) was degassed and purged with $N_2$ for 3 times, and then the mixture was stirred at 40 °C for 6 h under $H_2$ atmosphere. The mixture was filtered and concentrated under reduced pressure to give **Intermediate A-10** (8.5 g, 93.87% yield, 96.29% purity) as a yellow solid. It was used in the next reaction without further purification. $^1H$ NMR (400 MHz, DMSO-$d_6$) δ 8.44 (br s, 3H), 6.97 (s, 1H), 6.84 (s, 2H), 5.75 (s, 2H), 4.30 (q, *J* = 6.8 Hz, 1H), 1.47 (d, *J* = 6.8 Hz, 3H); MS (ESI) m/z = 204.9 $[M+H]^+$.

**Preparation Example 11: (R)-1-(3-(1,1-difluoro-2-methoxyethyl)-2-fluorophenyl)ethane-1-amine (A-11)**

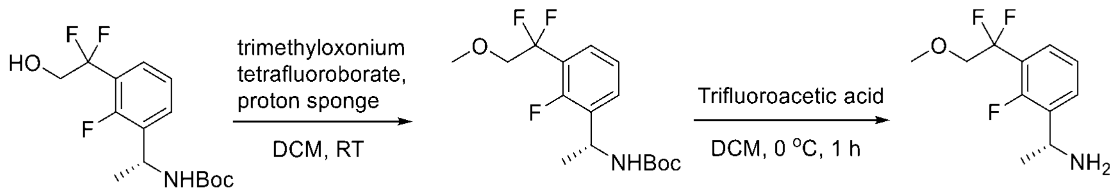


**A-5-1** **A-11-1** **A-11**

Step 1: Synthesis of tert-butyl (R)-(1-(3-(1,1-difluoro-2-methoxyethyl)-2-fluorophenyl)ethyl)carbamate

To a solution of tert-butyl *(R)*-(1-(3-(1,1-difluoro-2-hydroxyethyl)-2-fluorophenyl)ethyl)carbamate (87 mg, 0.272 mmol) in DCM (2.72 mL) was added Proton Sponge (117 mg, 0.545 mmol), trimethyloxonium tetrafluoroborate (80.6 mg, 0.545 mmol) and 4Å molecular sieve under nitrogen atmosphere, and then the mixture was stirred at 25 °C for 16 h. The reaction mixture was quenched with aq. 1 M HCl and extracted with DCM. The combined organic layers were washed with brine, dried over $Na_2SO_4$ and concentrated under reduced pressure to give a crude product. The crude product was purified by silica gel column chromatography (25% EtOAc in hexane) to give tert-butyl *(R)*-(1-(3-(1,1-difluoro-2-methoxyethyl)-2-fluorophenyl)ethyl)carbamate (61 mg, 67% yield) as colorless oil. MS (ESI) m/z = 278.02 $[M+H]^+$.

Step 2: Synthesis of (R)-1-(3-(1,1-difluoro-2-methoxyethyl)-2-fluorophenyl)ethan-1-amine

To a solution of tert-butyl *(R)*-(1-(3-(1,1-difluoro-2-methoxyethyl)-2-fluorophenyl)ethyl)carbamate (61 mg, 0.183 mmol) in DCM (0.73 mL) was added trifluoroacetic acid (0.16 mL) at 0 °C. Thereafter, the mixture was stirred at room temperature for 1 h. The reaction mixture was quenched with aq. $NaHCO_3$ and extracted with DCM. The combined organic layers were dried over $Na_2SO_4$, filtered and concentrated under reduced pressure to give **Intermediate A-11** (41 mg, 96% yield) as yellow oil. MS (ESI) m/z = 234.08 $[M+H]^+$.

**Preparation Example 12: (1R)-1-[3-(1,1-difluoroethyl)-2-fluorophenyl]ethanamine (A-12)**

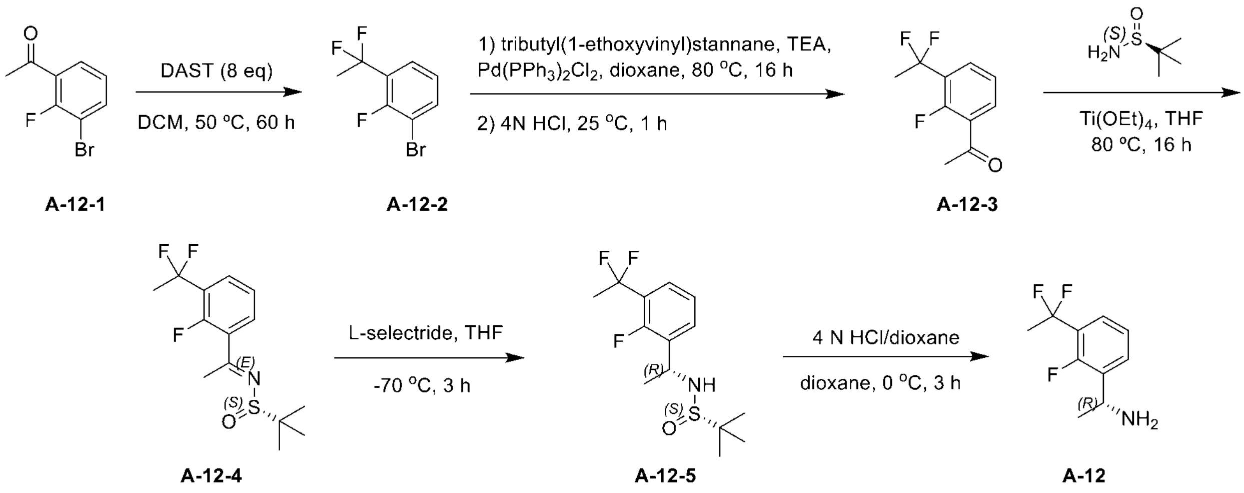


Step 1: Synthesis of 1-bromo-3-(1,1-difluoroethyl)-2-fluorobenzene

A mixture of 1-(3-bromo-2-fluoro-phenyl)ethanone (4 g, 18.43 mmol) and DAST (23.77 g, 147.44 mmol, 19.48 mL) in DCM (60 mL) was degassed and purged with $N_2$ for 3 times, and then the mixture was stirred at 50 °C for 60 h under $N_2$ atmosphere. The reaction mixture was slowly quenched with ice-cold water (40 mL) and basified (pH = 7-8) with sat. $NaHCO_3$. The aqueous layers were extracted with DCM (50 mL × 3) and the organic layers were washed with brine (20mL X 3). The organic layers were dried over anhydrous $Na_2SO_4$ and filtered under reduced pressure to give a crude product. The crude product was purified by silica gel column chromatography (0% EA in petroleum ether) to give 1-bromo-3-(1,1-difluoroethyl)-2-fluorobenzene (3.5 g, 79.45% yield) as colorless oil. $^1$H NMR (400 MHz, DMSO-$d_6$) δ 7.89 (br t, *J* = 7.2 Hz, 1H), 7.60 (br t, *J* = 7.2 Hz, 1H), 7.29 (t, *J* = 8.0 Hz, 1H), 2.02 (t, *J* = 19.2 Hz, 3H).

Step 2: Synthesis of 1-[3-(1,1-difluoroethyl)-2-fluoro-phenyl]ethanone

A mixture of 1-bromo-3-(1,1-difluoroethyl)-2-fluorobenzene (3.5 g, 14.64 mmol), 1-tributyl(1-ethoxyvinyl)stannane (5.66 g, 15.67 mmol, 5.29 mL), TEA (2.96 g, 29.28 mmol, 4.08 mL) and Pd(dppf)$Cl_2$ (1.03 g, 1.46 mmol) in dioxane (40 mL) was degassed and purged with $N_2$ for 3 times, and then the mixture was stirred at 80 °C for 16h under $N_2$ atmosphere. HCl solution (4N, 60 mL) was added to the mixture, which was then stirred at 25 °C for 1 h. After waster (30 mL) was added, the reaction mixture was extracted with EtOAc (50 mL X 3). The organic layers were washed with brine (50 mL X 3), dried over anhydrous $Na_2SO_4$ and concentrated under reduced pressure to give a residue. It was purified by silica gel column chromatography (3% EtOAc in petroleum ether) to give 1-[3-(1,1-difluoroethyl)-2-fluorophenyl]ethanone (2.5 g, 84.45% yield) as colorless oil. $^1$H NMR (400 MHz, DMSO-$d_6$) δ 7.95 (br t, *J* = 7.2 Hz, 1H), 7.82 (br t, *J* = 7.2 Hz, 1H), 7.44 (t, *J* = 7.6 Hz, 1H), 2.62 (d, *J* = 4.0 Hz, 3H), 2.05 (t, *J* = 19.2 Hz, 3H).

Step 3: Synthesis of (NE,S)-N-[1-[3-(1,1-difluoroethyl)-2-fluoro-phenyl]ethylidene]-2-methyl-propane-2-sulfinamide

A mixture of 1-[3-(1,1-difluoroethyl)-2-fluoro-phenyl]ethanone (2.7 g, 13.35 mmol), (S)-2-methylpropane-2-sulfinamide (2.43 g, 20.03 mmol) and Ti(OEt)$_4$ (9.14 g, 40.06 mmol, 8.31 mL) in THF (40 mL) was degassed and purged with $N_2$ for 3 times, and then the mixture was stirred at 80 °C for 16 h under $N_2$ atmosphere. The mixture was poured into water (50 mL) and filted, and then it was extracted with EtOAc (60 mL X 3). The organic layers were washed with brine (40 mL X 2), dried over anhydrous $Na_2SO_4$ and concentrated under reduced pressure to give a crude product. The crude product was purified by silica gel column chromatography (5% EtOAc in petroleum ether) to give (NE,S)-N-[1-[3-(1,1-difluoroethyl)-2-fluorophenyl]ethylidene]-2-methyl-propane-2-sulfinamide (3.4 g, 76.29% yield, 91.5% purity) as yellow oil. MS (ESI) m/z = 306.2 $[M+H]^+$.

Step 4: Synthesis of (S)-N-[(1R)-1-[3-(1,1-difluoroethyl)-2-fluoro-phenyllethyl]-2-methylpropane-2-sulfinamide

To a solution of (NE,S)-N-[1-[3-(1,1-difluoroethyl)-2-fluoro-phenyl]ethylidene]-2-methyl-propane-2-sulfinamide

(3.4 g, 11.13 mmol) in THF (50 mL) was added L-selectride (1 M, 27.84 mL) at -70 °C, and then the mixture was degassed and purged with $N_2$ for 3 times. Thereafter, the mixture was stirred at -70 °C for 3 h under $N_2$ atmosphere. The reaction mixture was quenched with sat. aq. $NH_4Cl$ (80 mL) added dropwise slowly at -70 °C, and then it was diluted with EtOAc (50 mL) and extracted with EtOAc (40 mL X 5). The organic layers were dried over anhydrous $Na_2SO_4$ and concentrated under reduced pressure to give a crude product. The crude product was purified by silica gel column chromatography (25% EtOAc in petroleum ether) to give a main product, (S)-N-[(1R)-1-[3-(1,1-difluoroethyl)-2-fluorophenyl]ethyl]-2-methylpropane-2-sulfinamide (2.3 g, 65.86% yield, 98% purity, 99.3% ee) as light yellow oil. $^1H$ NMR (400 MHz, DMSO-$d_6$) δ 7.63 (t, *J* = 6.8 Hz, 1H), 7.51 - 7.40 (m, 1H), 7.33 - 7.26 (m, 1H), 5.54 (d, *J* = 5.6 Hz, 1H), 4.72 (quint, *J* = 6.8 Hz, 1H), 2.00 (t, *J* = 19.2 Hz, 3H), 1.49 (d, *J* = 6.8 Hz, 3H), 1.09 (s, 9H); MS (ESI) m/z = 308.0 $[M+H]^+$.

Step 5: Synthesis of (1R)-1-[3-(1,1-difluoroethyl)-2-fluoro-phenyl]ethanamine

A mixture of (S)-N-[(1R)-1-[3-(1,1-difluoroethyl)-2-fluoro-phenyl]ethyl]-2-methylpropane-2-sulfinamide (500 mg, 1.63 mmol) and HCl/dioxane (4 N, 25 mL) in dioxane (2 mL) was degassed and purged with $N_2$ for 3 times, and then the mixture was stirred at 25 °C for 0.5 h under $N_2$ atmosphere. The mixture was concentrated under reduced pressure to give Intermediate A-12 (350 mg, 89.78% yield, HCl) as a white solid. $^1H$ NMR (400 MHz, DMSO-$d_6$) δ 8.75 (br s, 3H), 7.88 (t, *J* = 7.2 Hz, 1H), 7.59 (t, *J* = 7.2 Hz, 1H), 7.41 (t, *J* = 7.6 Hz, 1H), 4.66 (br d, *J* = 5.2 Hz, 1H), 2.02 (t, *J* = 19.2 Hz, 3H), 1.56 - 1.53 (m, 3H); MS (ESI) m/z = 204.1 $[M+H]^+$.

**Preparation Example 13: 2-[3-[(1R)-1-aminoethyl]-2-methyl-phenyl]-2,2-difluoro-ethanol (A-13)**

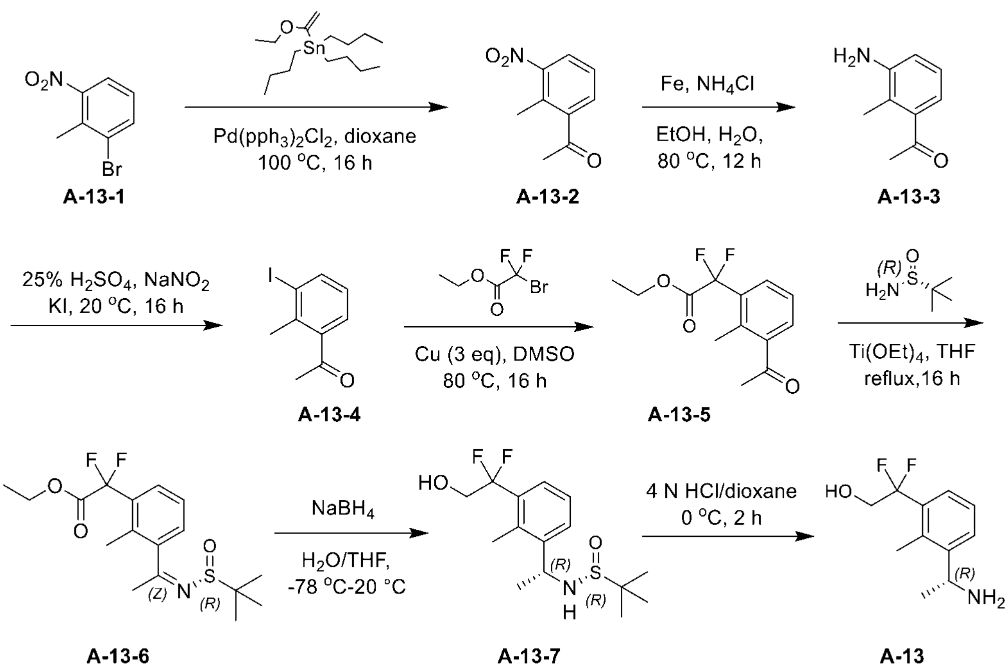


Step 1: Synthesis of 1-(2-methyl-3-nitro-phenyl)ethanone

A mixture of 1-bromo-methyl-3-nitro-benzene (10 g, 46.29 mmol), 1-tributyl(1-ethoxyvinyl)stannane (19.85 g, 54.96 mmol, 18.55 mL) and $Pd(PPh_3)_2Cl_2$ (3.25 g, 4.63 mmol) in dioxane (50 mL) was degassed and purged with $N_2$ for 3 times, and then the mixture was stirred at 100 °C for 12 h under $N_2$ atmosphere. Following the addition of water (100 mL), the reaction mixture was extracted with EtOAc (100 mL X 3). The combined organic layers were dried over anhydrous $Na_2SO_4$ and concentrated under reduced pressure to give a residue. It was purified by silica gel column chromatography (20% EtOAc in petroleum ether) to give 1-(2-methyl-3-nitro-phenyl)ethanone (8.0 g, 96.46% yield) as yellow oil. The aqueous layers were adjusted to pH = 9 with aq. NaOH, and NaClO (100 mL) was slowly added under stirring. $^1H$ NMR (400 MHz, DMSO-$d_6$) δ 8.05 - 7.89 (m, 2H), 7.56 (t, *J* = 8.0 Hz, 1H), 2.60 (s, 3H), 2.37 (s, 3H).

Step 2: Synthesis of 1-(3-amino-2-methyl-phenyl)ethanone

To a solution of 1-(2-methyl-3-nitro-phenyl)ethanone (9.2 g, 51.35 mmol) in EtOH (100 mL) and $H_2O$ (20 mL) was added Fe (28.67 g, 513.47 mmol) and $NH_4Cl$ (27.47g, 513.47 mmol). The mixture was degassed and purged with $N_2$ for 3 times, and then it was stirred at 80 °C for 12 h under nitrogen atmosphere. The reaction mixture was filtered, and the filtrate was concentrated under reduced pressure. Water (100 mL) was poured into the reaction mixture, which was then extracted with EtOAc (100 mL × 3). The combined organic layers were washed with brine (100 mL), dried over $Na_2SO_4$ and concentrated under reduced pressure to give a crude product 1-(3-amino-2-methyl-phenyl)ethanone (6.8 g, 88.77% yield) as a yellow solid. $^1H$ NMR (400 MHz, DMSO-$d_6$) δ 6.98 (t, *J* = 7.6 Hz, 1H), 6.84 (dd, *J* = 1.2, 7.6 Hz, 1H), 6.77 (dd, *J* =1.2, 8.0 Hz, 1H), 5.05 (s, 2H), 2.46 (s, 3H), 2.06 (s, 3H).

Step 3: Synthesis of 1-(3-iodo-2-methyl-phenyl)ethanone

To a suspension of 1-(3-amino-2-methyl-phenyl)ethanone (6.5 g, 43.57 mmol) in $H_2SO_4$ (95.68 g, 975.55 mmol, 21.6 mL) was added $NaNO_2$ (3.01 g, 43.57 mmol) aqueous solution (220 mL) at 0 °C, and then the mixture was stirred for 1 h. The mixture was added to KI (21.70 g, 130.71 mmol) aqueous solution (220 mL) and stirred at 20 °C for 18 h. Water (100 mL) was poured into the reaction mixture, which was then extracted with EtOAc (100 mL × 3). The organic layers were washed with brine (100 mL), dried over anhydrous $Na_2SO_4$ and concentrated under reduced pressure to give a crude product. The crude product was purified by silica column chromatography (6% EtOAc in petroleum ether) to give 1-(3-iodo-2-methylphenyl)ethanone (8.2 g, 72.37% yield) as yellow oil. $^1H$ NMR (400 MHz, DMSO-$d_6$) δ 7.99 (dd, *J* = 1.2, 8.0 Hz, 1H), 7.67 (d, *J* = 7.6 Hz, 1H), 7.07 (t, *J* = 7.6 Hz, 1H), 2.53 (s, 3H), 2.41 (s, 3H).

Step 4: Synthesis of ethyl 2-(3-acetyl-2-methyl-phenyl)-2,2-difluoroacetate

A mixture of 1-(3-iodo-2-methyl-phenyl)ethanone (4 g, 15.38 mmol), ethyl-2-bromo-2,2-difluoroacetate (3.43 g, 16.92 mmol, 2.17 mL) and Cu (2.93 g, 46.14 mmol, 327.24 μL) in DMSO (40 mL) was degassed and purged with $N_2$ for 3 times, and then the mixture was stirred at 80 °C for 16 h under $N_2$ atmosphere. The mixture was poured into water (100 mL) and extracted with EtOAc (100 mL X 3). The organic layers were washed with brine (100 mL), dried over anhydrous $Na_2SO_4$ and concentrated under reduced pressure to give a crude product. The crude product was purified by silica gel column chromatography (1-2% EtOAc in petroleum ether) to give ethyl 2-(3-acetyl-2-methyl-phenyl)-2,2-difluoroacetate (3.4 g, 74.11% yield, 85.91% purity) as colorless oil. $^1H$ NMR (400 MHz, DMSO-$d_6$) δ 7.86 (d, *J* = 7.6 Hz, 1H), 7.72 (d, *J* = 7.6 Hz, 1H), 7.49 (t, *J* = 7.6 Hz, 1H), 4.34 (q, *J* = 7.2 Hz, 2H), 2.57 (s, 3H), 2.29 (s, 3H), 1.22 (t, *J* = 7.2 Hz, 3H); MS (ESI) m/z = 257.0 $[M+H]^+$.

Step 5: Synthesis of ethyl 2-[3-[(Z)-N-[(R)-tert-butylsulfinyl]-C-methyl-carbonimidoyl]-2-methyl-phenyl)-2,2-difluoroacetate

A mixture of ethyl 2-(3-acetyl-2-methyl-phenyl)-2,2-difluoroacetate (3.4 g, 13.27 mmol), (R)-2-methylpropane-2-sulfinamide (1.77 g, 14.60 mmol) and $Ti(OEt)_4$ (9.08 g, 39.81 mmol, 8.25 mL) in THF (40 mL) was degassed and purged with $N_2$ for 3 times, and then the mixture was stirred at 80 °C for 12 h under $N_2$ atmosphere. The mixture was poured into water (50 mL) and EtOAc (50 mL) and extracted with EtOAc (50 mL X 3). The combined organic layers were washed with brine (50 mL X 2), dried over anhydrous $Na_2SO_4$ and concentrated under reduced pressure to give a crude product. The crude product was purified by silica gel column chromatography (12% EtOAc in petroleum ether) to give ethyl 2-[3-[(Z)-N-[(R)-tert-butylsulfinyl]-C-methyl-carbonimidoyl]-2-methyl-phenyl)-2,2-difluoroacetate ((2.6 g, 48.82% yield, 89.56% purity) as yellow oil. MS (ESI) m/z = 360.0 $[M+H]^+$.

Step 6: Synthesis of (R)-N-[(1R)-1-[3-(1,1-difluoro-2-hydroxy-ethyl)-2-methylphenyl]ethyl]-2-methyl-propane-2-sulfinamide

A mixture of ethyl-2-[3-[(Z)-N-[(R)-tert-butylsulfinyl]-C-methyl-carbonimidoyl]-2-methyl-phenyl)-2,2-difluoroacetate (2.6 g, 7.23 mmol) and $NaBH_4$ (830 mg, 21.94 mmol) in THF (30 mL) and $H_2O$ (1 mL) was degassed and purged with $N_2$ for 3 times, and then the mixture was stirred at -70 °C for 2 h under $N_2$ atmosphere. The mixture was quenched with sat. $NH_4Cl$ (50 mL) at 20 °C, diluted with EtOAc (50 mL), and then extracted with EtOAc (50 mL X 3). The organic layers were dried over anhydrous $Na_2SO_4$ and concentrated under reduced pressure to give a crude product. The crude product was separated and purified by prep-HPLC (YMC-Triart Prep C18 250*50 mm*10 um, mobile phase: [water ($NH_3H_2O$+$NH_4HCO_3$)-MeCN]; B%: 20%-50%, 20 min). MeCN was removed under reduced pressure, and the remaining solvent was removed by lyophilization to give a main product, (R)-N-[(1R)-1-[3-(1,1-difluoro-2-hydroxy-ethyl)-2-methyl-phenyl]ethyl]-2-methyl-propane-2-sulfinamide (900 mg, 38.08% yield, 97.77% purity, 100% ee) as a white solid. $^1H$ NMR

(400 MHz, DMSO-$d_6$) δ 7.61 (d, $J$ = 7.6 Hz, 1H), 7.39 - 7.34 (m, 1H), 7.28 (t, $J$ = 7.2 Hz, 1H), 5.73 - 5.60 (m, 2H), 4.69 (quint, $J$ = 6.8 Hz, 1H), 3.88 (dt, $J$ = 6.4, 14.8 Hz, 2H), 2.37 (s, 3H), 1.37 (d, $J$ = 6.8 Hz, 3H), 1.11 - 1.06 (m, 9H); MS (ESI) m/z = 320.0 $[M+H]^+$.

Step 7: Synthesis of 2-[3-[(1R)-1-aminoethyl]-2-methyl-phenyl]-2,2-difluoroethanol

To (R)-N-[(1R)-1-[3-(1,1-difluoro-2-hydroxy-ethyl)-2-methyl-phenyl]ethyl]-2-methyl-propane-2-sulfinamide (150 mg, 469.62 μmol) in dioxane (2 mL) was added dropwise HCl/dioxane (4 N, 4 mL) at 0 °C, and then the mixture was stirred at 0 °C for 2 h. The mixture was concentrated under reduced pressure to give **Intermediate A-13** (115 mg, 57.48% yield, 50.52% purity) as a yellow solid. MS (ESI) m/z = 216.0 $[M+H]^+$.

**Preparation Example 14: 2-[3-[(1R)-1-amino-1-deuterio-ethyl]-2-fluoro-phenyl]-2,2-difluoro-ethanol (A-14)**

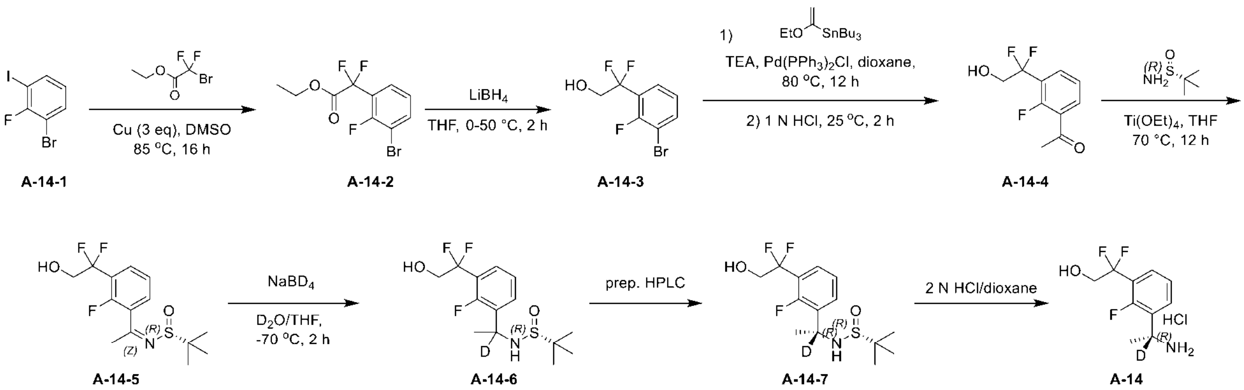

A-14-1 A-14-2 A-14-3 A-14-4
A-14-5 A-14-6 A-14-7 A-14

Step 1: Synthesis of ethyl 2-(3-bromo-2-fluoro-phenyl)-2,2-difluoro-acetate

To a solution of 1-bromo-2-fluoro-3-iodo-benzene (11 g, 36.56 mmol, 1 eq) in DMSO (200 mL) was added solid copper (6.97 g, 109.67 mmol, 777.82 μL) and ethyl 2-bromo-2,2-difluoro-acetate (22.26 g, 109.67 mmol, 14.13 mL). The reaction mixture was stirred at 85 °C for 16 h under $N_2$ atmosphere. The reaction mixture was filtered, and then the filtrate was added with water (80 mL) and extracted with EtOAc (80 mL X 3). The organic layers were washed with brine (60 mL), dried over anhydrous $Na_2SO_4$ and concentrated under reduced pressure to give a crude product. The crude product was purified by silica gel column chromatography (10 % EtOAc in petroleum ether) to give ethyl 2-(3-bromo-2-fluoro-phenyl)-2,2-difluoro-acetate (9.3 g, 77.07% yield, 90% purity) as white oil. $^1H$ NMR (400 MHz, DMSO-$d_6$) δ 8.02-7.95 (m, 1H), 7.75-7.69 (m, 1H), 7.37 (t, $J$ = 8.0 Hz, 1H), 4.40-4.31 (m, 2H), 1.23 (t, $J$ = 7.2 Hz, 3H).

Step 2: Synthesis of 2-(3-bromo-2-fluoro-phenyl)-2,2-difluoro-ethanol

To a solution of ethyl 2-(3-bromo-2-fluoro-phenyl)-2,2-difluoro-acetate (8.8 g, 29.62 mmol) in THF (140 mL) was slowly added $LiBH_4$ (2 M, 20.74 mL) at 0 °C under $N_2$ atmosphere. Thereafter, the reaction mixture was stirred at 50 °C for 2 h under $N_2$ atmosphere. Aq. $NH_4Cl$ (80 mL) was slowly poured into the mixture, which was then extracted with EtOAc (60 mL X 3). The organic layers were washed with brine (80 mL), dried over anhydrous $Na_2SO_4$ and concentrated under reduced pressure to give a crude product. The crude product was purified by silica gel column chromatography (6% EtOAc in petroleum ether) to give 2-(3-bromo-2-fluoro-phenyl)-2,2-difluoro-ethanol (7.3 g, 96.63% purity) as yellow oil. $^1H$ NMR (400 MHz, DMSO-$d_6$) δ 7.89 (t, $J$ = 7.2 Hz, 1H), 7.61-7.52 (m, 1H), 7.29 (t, $J$ = 8.0 Hz, 1H), 5.76-5.70 (m, 1H), 3.96-3.88 (m, 2H).

Step 3: Synthesis of 1-[3-(1,1-difluoro-2-hydroxy-ethyl)-2-fluoro-phenyl]ethanone

A mixture of tributyl(1-ethoxyvinyl)stannane (8.24 g, 22.82 mmol, 7.71 mL), TEA (3.17 g, 31.37 mmol, 4.37 mL) and $Pd(PPh_3)_2Cl_2$ (1.10 g, 1.57 mmol) was mixed with a solution of 2-(3-bromo-2-fluoro-phenyl)-2,2-difluoro-ethanol (4 g, 15.68 mmol) in dioxane (50 mL), degassed and purged with $N_2$ for 3 times, and then stirred at 80 °C for 12h under $N_2$ atmosphere. The mixture was added with HCl solution (50 ml, 1 M) stirring at 25 °C, and then stirred at 25 °C for 1 h. Following the addition of water (30 mL), the reaction mixture was extracted with EtOAc (50 mL X 3). The combined organic

layers were washed with brine (80 mL X 3), dried over anhydrous $Na_2SO_4$ and concentrated under reduced pressure to give a residue. It was purified by silica gel column chromatography (12% EtOAc in petroleum ether) to give 1-[3-(1,1-difluoro-2-hydroxy-ethyl)-2-fluoro-phenyl]ethanone (3.33 g, 90.43% yield, 92.93% purity) as a yellow solid. $^1$H NMR (400 MHz, DMSO-$d_6$) δ 7.99-7.91 (m, 1H), 7.82-7.74 (m, 1H), 7.43 (t, *J* = 8.0 Hz, 1H), 5.73 (t, *J* = 6.8 Hz, 1H), 3.95 (dt, *J* = 6.8, 14.3 Hz, 2H), 2.60 (d, *J* = 4.4 Hz, 3H); MS (ESI) m/z = 218.9 $[M+H]^+$.

Step 4: Synthesis of (NZ,R)-N-[1-[3-(1,1-difluoro-2-hydroxy-ethyl)-2-fluorophenyl]ethylidene]-2-methyl-propane-2-sulfinamide

To a solution of 1-[3-(1,1-difluoro-2-hydroxy-ethyl)-2-fluoro-phenyl]ethanone (3.33 g, 15.26 mmol) in THF (35 mL) was added (R)-2-methylpropane-2-sulfinamide (2.77 g, 22.89 mmol) and $Ti(OEt)_4$ (10.44 g, 45.79 mmol, 9.50 mL), and then the mixture was stirred at 70 °C for 12 h under $N_2$ atmosphere. The mixture was poured into water (50 mL) and extracted with EtOAc (50 mL X 3). The organic layers were washed with brine (50 mL), dried over anhydrous $Na_2SO_4$ and concentrated under reduced pressure to give a crude product. The crude product was purified by silica gel column chromatography (20% EtOAc in petroleum ether) to give (NZ, R)-N-[1-[3-(1,1-difluoro-2-hydroxy-ethyl)-2-fluoro-phenyl] ethylidene]-2-methylpropane-2-sulfinamide (4.3 g, 85.90% yield, 97.99% purity) as yellow oil. $^1$H NMR (400 MHz, DMSO-$d_6$) δ 7.79 (br t, *J* = 7.2 Hz, 1H), 7.68 (br t, *J* = 6.8 Hz, 1H), 7.41 (br t, *J* = 7.6 Hz, 1H), 5.75-5.71 (m, 1H), 3.93 (dt, *J* = 6.4, 14.0 Hz, 2H), 2.69 (br d, *J* = 1.6 Hz, 3H), 1.22 (s, 9H); MS (ESI) m/z = 322.1 $[M+H]^+$.

Step 5: Synthesis of (R)-N-[(1R)-1-deuterio-1-[3-(1,1-difluoro-2-hydroxy-ethyl)-2-fluoro-phenyl]ethyl]-2-methyl-propane-2-sulfinamide

To a solution of (NZ,R)-N-[1-[3-(1,1-difluoro-2-hydroxy-ethyl)-2-fluorophenyl]ethylidene]-2-methyl-propane-2-sulfinamide (4.3 g, 13.38 mmol) in THF (80 mL) and $D_2O$ (1.6 mL) was added $NaBD_4$ (506.23 mg) at -70 °C, and then the mixture was stirred at - 70 °C for 2 h under $N_2$ atmosphere. TLC (PE:EA = 1/1) indicated the reaction was complete. The mixture was quenched with sat. aq. $NH_4Cl$ (50 mL) and extracted with EtOAc (50 mL X 3). The organic layers were washed with brine (80 mL), dried over anhydrous $Na_2SO_4$ and concentrated under reduced pressure to give a crude product. The crude product was purified by silica gel column chromatography (20% EtOAc/petroleum ether) to give (R)-N-[(1R)-1-deuterio-1-[3-(1,1-difluoro-2-hydroxy-ethyl)-2-fluoro-phenyl]ethyl]-2-methyl-propane-2-sulfinamide (4.2 g, 81.28% yield, 84% purity) as yellow oil. MS (ESI) m/z = 325.0 $[M+H]^+$.

Step 6: Synthesis of (R)-N-[(1S)-1-deuterio-1-[3-(1,1-difluoro-2-hydroxy-ethyl)-2-fluoro-phenyl]ethyl]-2-methyl-propane-2-sulfinamide

(R)-N-[(1R)-1-deuterio-1-[3-(1,1-difluoro-2-hydroxy-ethyl)-2-fluoro-phenyl]ethyl]-2-methyl-propane-2-sulfinamide was purified by prep-HPLC (column: Welch Xtimate C18 250*50mm*10μm; mobile phase: [water ($NH_3H_2O+NH_4HCO_3$)-ACN]; 5%-55% B, 20 min). The solvent was concentrated under reduced pressure, and then the reside was lyophilized to give (R)-N-[(1S)-1-deuterio-1-[3-(1,1-difluoro-2-hydroxy-ethyl)-2-fluoro-phenyl]ethyl]-2-methyl-propane-2-sulfinamide (700 mg, 16.28% yield, 100% purity, peak 1) as a white solid. $^1$H NMR (400 MHz, DMSO-$d_6$) δ 7.64 (t, *J* = 6.8 Hz, 1H), 7.48-7.39 (m, 1H), 7.33-7.26 (m, 1H), 5.70 (t, *J* = 6.4 Hz, 1H), 5.52 (s, 1H), 3.90 (dt, *J* = 6.4, 14.4 Hz, 2H), 1.48 (s, 3H), 1.09 (s, 9H); MS (ESI) m/z = 325.2 $[M+H]^+$.

Step 7: Synthesis of 2-[3-[(1R)-1-amino-1-deuterio-ethyl]-2-fluoro-phenyl]-2,2-difluoro-ethanol

To (R)-N-[(1S)-1-deuterio-1-[3-(1,1-difluoro-2-hydroxy-ethyl)-2-fluorophenyl]ethyl]-2-methyl-propane-2-sulfinamide (1.14 g, 3.51 mmol) in dioxane (5 mL) was added HCl/dioxane (20 mL) at 0 °C, and then the mixture was stirred at 20 °C for 2 h. The mixture was concentrated under reduced pressure to give **Intermediate A-14** (900 mg, 94.78% yield, 95% purity, hydrochloride salt) as a white solid. $^1$H NMR (400 MHz, DMSO-$d_6$) δ 8.73 (br s, 2H), 7.87 (t, *J* = 6.8 Hz, 1H), 7.57 (t, *J* = 7.2 Hz, 1H), 7.44-7.37 (m, 1H), 3.92 (br t, *J* = 14.2 Hz, 2H), 1.54 (s, 3H), 1.10 (s, 1H).

**Preparation Example 15: (1R)-1-[2-chloro-3-(trifluoromethyl)phenyl]ethanamine (A-15)**

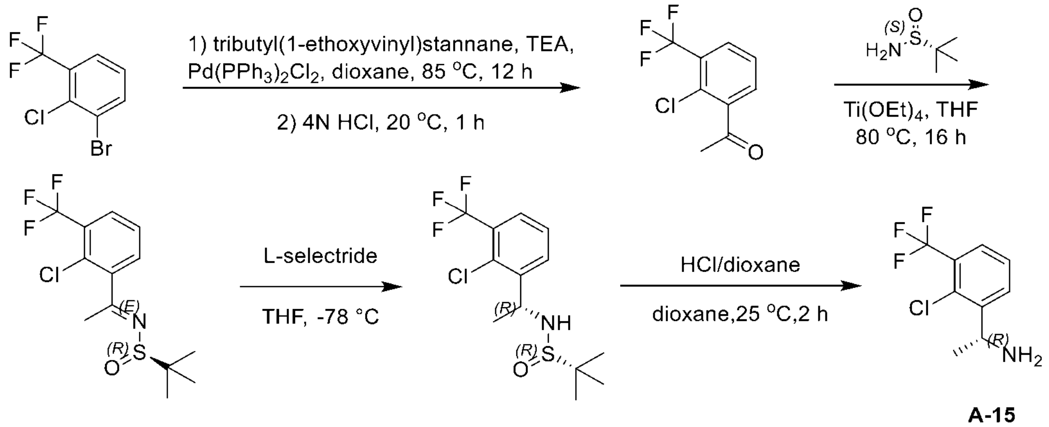


A-15

Step 1: Synthesis of 1-[2-chloro-3-(trifluoromethyl)phenyl]ethanone

A mixture of tributyl(1-ethoxyvinyl)stannane (12.520 g, 34.67 mmol, 11.71 mL), TEA (6.24 g, 61.67 mmol, 8.58 mL) and $Pd(PPh_3)_2Cl_2$ (1.10 g, 1.57 mmol) was mixed with a solution of 1-bromo-2-chloro-3-(trifluoromethyl)benzene (4 g, 15.68 mmol) in dioxane (140 mL), degassed and purged with $N_2$ for 3 times, and then stirred at 80 °C for 16 h under $N_2$ atmosphere. TLC (PE:EA = 10/1) indicated that the reaction was complete. To the mixture was added HCl solution (80 ml, 1 M) under stirring at 25 °C, and then the mixture was stirred at 25 °C for 1 h. Following the addition of water (120 mL), the reaction mixture was extracted with EtOAc (150 mL X 3). The combined organic layers were washed with brine (150 mL X 3), dried over anhydrous $Na_2SO_4$ and concentrated under reduced pressure to give a residue. The aqueous layer was stirred while slowly adding sat. aq. KF (200 mL), followed by stirring at 25 °C for 1 h. It was purified by column chromatography (6% EtOAc/petroleum ether) to give 1-[2-chloro-3-(trifluoromethyl)phenyl]ethanone (4.2 g, 58.13% yield, 95% purity) as yellow oil. $^1H$ NMR (400 MHz, DMSO-$d_6$) $\delta$ 7.97 (d, $J$ = 8.0 Hz, 1H), 7.92 (d, $J$ = 7.6 Hz, 1H), 7.71-7.63 (m, 1H), 2.61 (s, 3H).

Step 2: Synthesis of (NE,R)-N-[1-[2-chloro-3-(trifluoromethyl)phenyl]ethylidene]-2-methyl-propane-2-sulfinamide

To a solution of 1-[2-chloro-3-(trifluoromethyl)phenyl]ethanone (4.2 g, 18.87 mmol) in THF (60 mL) was added (S)-2-methylpropane-2-sulfinamide (3.43 g, 28.30 mmol) and $Ti(OEt)_4$ (12.91 g, 56.61 mmol, 11.74 mL), and then the mixture was stirred at 80 °C for 16 h under $N_2$ atmosphere. TLC (PE:EA = 10/1) indicated that the reaction was complete. The mixture was poured into water (40 mL) and filtered, and then the filtrate was extracted with EtOAc (60 mL X 3). The organic layers were washed with brine (80 mL), dried over anhydrous $Na_2SO_4$ and concentrated under reduced pressure to give a crude product. The crude product was purified by silica gel column chromatography (6% EtOAc in petroleum ether) to give (NE,R)-N-[1-[2-chloro-3-(trifluoromethyl)phenyl]ethylidene]-2-methyl-propane-2-sulfinamide (5.15 g , 75.40% yield, 90% purity) as yellow oil. $^1H$ NMR (400 MHz, DMSO-$d_6$) $\delta$ 7.98 (br d, $J$ = 7.6 Hz, 1H), 7.91 (br d, $J$ = 7.6 Hz, 1H), 7.82 (br d, $J$ = 7.6 Hz, 1H), 2.71 (s, 3H), 1.26 (s, 9H); MS (ESI) m/z = 326.0 $[M+H]^+$.

Step 3: Synthesis of N-[(1R)-1-[2-chloro-3-(trifluoromethyl)phenyllethyl]-2-methylpropane-2-sulfinamide

L-selectride (1 M, 31.62 mL) was slowly added dropwise to a solution of (NE,R)-N-[1-[2-chloro-3-(trifluoromethyl) phenyl]ethylidene]-2-methyl-propane-2-sulfinamide (5.15 g, 15.81 mmol) in THF (100 mL) at -70 °C for 0.5 h, and then the mixture was stirred at -70 °C for 2 h under $N_2$ atmosphere. The reaction mixture was quenched with sat. aq. $NH_4Cl$ (60 mL) added dropwise slowly at 20 °C under $N_2$ atmosphere, and then diluted with EtOAc (80 mL) and extracted with EtOAc (80 mL X 3). The organic layers were dried over anhydrous $Na_2SO_4$ and concentrated under reduced pressure to give a crude product. The crude product was purified by silica gel column chromatography (13% EtOAc in petroleum ether) to give the desired peak 2 fractions, N-[(1R)-1-[2-chloro-3-(trifluoromethyl)phenyl]ethyl]-2-methylpropane-2-sulfinamide (2 g, 78.17% yield, 89.89% purity, ee = 95.96%) as a yellow solid. $^1H$ NMR (400 MHz, DMSO-$d_6$) $\delta$ 7.89 (d, $J$ = 8.0 Hz, 1H), 7.76 (d, $J$ = 6.8 Hz, 1H), 7.63-7.54 (m, 1H), 5.66 (d, $J$ = 5.6 Hz, 1H), 4.93 (t, $J$ = 6.4 Hz, 1H), 1.48 (d, $J$ = 6.8 Hz, 3H), 1.10 (s, 9H).

Step 4: Synthesis of (1R)-1-[2-chloro-3-(trifluoromethyl)phenyl]ethanamine

To a solution of N-[(1R)-1-[2-chloro-3-(trifluoromethyl)phenyl]ethyl]-2-methylpropane-2-sulfinamide (200 mg, 610.14 $\mu$mol) in dioxane (1 mL) was added dropwise HCl/dioxane (1 mL), and then the mixture was stirred at 25 °C for 2 h.

TLC (PE:EtOAC = 1:1) indicated that all starting materials were consumed and one new spot was formed. The mixture was concentrated under reduced pressure to give **Intermediate A-15** (136 mg, 99.68% yield) as a yellow solid. MS (ESI) m/z = 224.1 $[M+H]^+$.

**Preparation Example 16: [1-[3-[(1R)-1-aminoethyl]-2-fluorophenyl]cyclopropyl]methanol (A-16)**

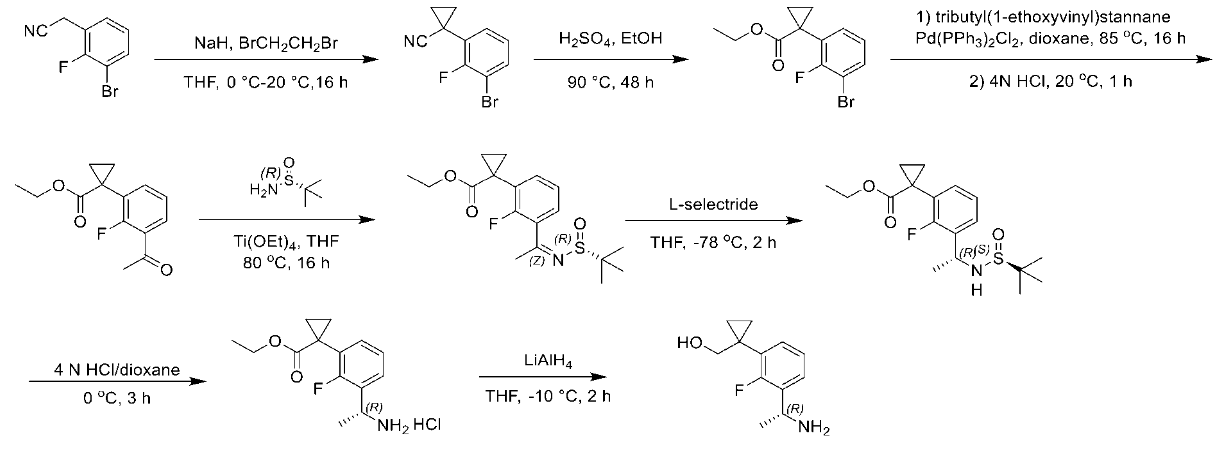


A-16

Step 1: Synthesis of 1-(3-bromo-2-fluoro-phenyl)cyclopropanecarbonitrile

A solution of 2-(3-bromo-2-fluoro-phenyl)acetonitrile (4.4 g, 20.56 mmol) and 1,2-dibromoethane (5.02 g, 26.72 mmol, 2.02 mL) in THF (80 mL) was purged with $N_2$ for 3 times, and then NaH (1.07 g, 26.72 mmol, 60% purity) was added at 0 °C. Thereafter, the mixture was stirred at 20 °C for 16 h under $N_2$ atmosphere. Sat. aq. $NH_4Cl$ was poured into the mixture, which was then extracted with EtOAc (30 mL X 3). The organic layers were washed with brine (50 mL), dried over anhydrous $Na_2SO_4$ and concentrated under reduced pressure to give a crude product. The crude product was purified by silica gel column chromatography (9% EtOAc in petroleum ether) to give 1-(3-bromo-2-fluoro-phenyl)cyclopropanecarbonitrile (4.6 g, 75.50% yield, 81% purity) as yellow oil. $^1H$ NMR (400 MHz, DMSO-$d_6$) δ 7.74 (ddd, *J* = 1.6, 6.8, 8.0 Hz, 1H), 7.57 - 7.38 (m, 1H), 7.18 (dt, *J* = 0.8, 8.0 Hz, 1H), 1.86 - 1.60 (m, 2H), 1.57 - 1.41 (m, 2H).

Step 2: Synthesis of 1-(3-bromo-2-fluoro-phenyl)cyclopropanecarboxylate

A solution of 1-(3-bromo-2-fluoro-phenyl)cyclopropanecarbonitrile (4.69 g, 19.54 mmol) in ethanol (80 mL) and sulfuric acid (10 mL) was purged with $N_2$ for 3 times, and then the mixture was stirred at 90 °C for 48 h under $N_2$ atmosphere. TLC (PE:EA = 10:1) indicated the reaction was complete. The mixture was poured into water (20 mL) and extracted with EtOAc (20 mL X 3). The organic layers were washed with brine (100 mL X 3), dried over anhydrous $Na_2SO_4$ and concentrated under reduced pressure to give a crude product. The crude product was purified by silica gel column chromatography (2% EtOAc in petroleum ether) to give 1-(3-bromo-2-fluoro-phenyl)cyclopropanecarboxylate (3.74 g, 66.68% yield) as a yellow solid. $^1H$ NMR (400 MHz, DMSO-$d_6$) δ 7.63 (ddd, *J* = 1.6, 6.8, 8.0 Hz, 1H), 7.40 - 7.29 (m, 1H), 7.20 - 7.02 (m, 1H), 4.04 (q, *J* = 7.2 Hz, 2H), 1.64 - 1.47 (m, 2H), 1.29 - 1.23 (m, 2H), 1.08 (t, *J* = 7.2 Hz, 3H).

Step 3: Synthesis of ethyl 1-(3-acetyl-2-fluoro-phenyl)cyclopropanecarboxylate

A mixture of tributyl(1-ethoxyvinyl)stannane (9.82 g, 27.20 mmol, 9.19 mL) and $Pd(PPh_3)_2Cl_2$ (1.78 g, 2.54 mmol) was mixed with a solution of 1-(3-bromo-2-fluorophenyl)cyclopropanecarboxylate (7.3 g, 25.42 mmol) in dioxane (100 mL), degassed and purged with $N_2$ for 3 times, and then stirred at 80 °C for 16 h under $N_2$ atmosphere. TLC (PE:EA = 3:1) indicated that the reaction was complete. The mixture was quenched by adding 4 N HCl solution (30 mL), and stirred at 20 °C for 1 h. TLC (PE:EA = 5:1) indicated that all intermediates were consumed and the reaction was complete. Following the addition of water (100 mL), the reaction mixture was extracted with EtOAc (100 mL X 3). The combined organic layers were washed with brine (100 mL X 3), dried over anhydrous $Na_2SO_4$ and concentrated under reduced pressure to give a residue. The aqueous layer was stirred while slowly adding sat. aq. KF (150 mL), and then stirred at 25 °C for 1h. It was purified by column chromatography (3% EtOAc in petroleum ether) to give ethyl 1-(3-acetyl-2-fluorophenyl)cyclopropanecarboxylate (4.6 g, 72.29% yield) as a yellow solid. $^1$H NMR (400 MHz, DMSO-$d_6$) δ 7.78 - 7.68 (m, 1H), 7.65 - 7.54 (m, 1H), 7.26 (dt, *J* = 1.2, 7.6 Hz, 1H), 4.04 (dq, *J* = 1.2, 7.2 Hz, 2H), 2.58 (dd, *J* = 1.2, 4.4 Hz, 3H), 1.62 - 1.49 (m, 2H), 1.32 - 1.22 (m, 2H), 1.09 (dt, *J* = 1.2, 7.2 Hz, 3H).

Step 4: Synthesis of ethyl 1-[3-[(Z)-N-[(R)-tert-butylsulfinyl]-C-methylcarbonimidoyl]-2-fluoro-phenyl]cyclopropanecarboxylate

To a solution of ethyl-1-(3-acetyl-2-fluoro-phenyl)cyclopropanecarboxylate (2 g, 7.99 mmol) was added (R)-2-methylpropane-2-sulfinamide (968.58 mg, 7.99 mmol) and $Ti(OEt)_4$ (5.47 g, 23.97 mmol, 4.97 mL), and then the mixture was degassed and purged with $N_2$ for 3 times. Thereafter, the mixture was stirred at 80 °C for 16 h under $N_2$ atmosphere. The mixture was poured into water (50 mL) and was filtered, and the filtrate was extracted with EtOAc (50 mL X 3). The organic layers were washed with brine (100 mL), dried over anhydrous $Na_2SO_4$ and concentrated under reduced pressure to give a crude product. The crude product was purified by silica gel column chromatography (6% EtOAc in petroleum ether) to give ethyl 1-[3-[(Z)-N-[(R)-tert-butylsulfinyl]-C-methylcarbonimidoyl]-2-fluorophenyl]cyclopropanecarboxylate (2.3 g, 73.28% yield, 90% purity) as yellow oil. MS (ESI) m/z = 354.3 $[M+H]^+$.

Step 5: Synthesis of ethyl 1-[3-[(1R)-1-[[(S)- tert-butylsulfinyl]amino]ethyl]-2-fluorophenyl]cyclopropanecarboxylate

L-selectride (1 M, 5.91 mL) was slowly added dropwise to a solution of ethyl 1-[3-[(Z)-N-[(R)-tert-butylsulfinyl]-C-methylcarbonimidoyl]-2-fluoro-phenyl]cyclopropanecarboxylate (1.1 g, 3.11 mmol) in THF (20 mL) at -78°C under $N_2$ atmosphere, and then the mixture was stirred at -78 °C for 2 h under $N_2$ atmosphere. The reaction mixture was quenched with sat. aq. $NH_4Cl$ (20 mL) added dropwise slowly at 20 °C under $N_2$ atmosphere, and then poured into water (20 mL) and extracted with EtOAc (20 mL X 3). The organic layers were washed with brine (10 mL), dried over anhydrous $Na_2SO_4$ and concentrated under reduced pressure to give a crude product. The crude product was purified by silica gel column chromatography (19% EtOAc in petroleum ether) to give ethyl-1-[3-[(1R)-1-[[(S)- tertbutylsulfinyl]amino]ethyl]-2-fluorophenyl]cyclopropanecarboxylate (440 mg, 36.99% yield, 93% purity, ee = 95.12%) as colorless oil. $^1$H NMR (400 MHz, DMSO-$d_6$) δ 7.46 - 7.33 (m, 1H), 7.25 - 7.17 (m, 1H), 7.15 - 7.05 (m, 1H), 5.45 (d, *J* = 6.0 Hz, 1H), 4.74 - 4.57 (m, 1H), 4.01 (dq, *J* = 2.0, 7.2 Hz, 2H), 1.51 (s, 2H), 1.47 (d, *J* = 6.8 Hz, 3H), 1.22 - 1.16 (m, 2H), 1.11 - 1.09 (m, 9H), 1.08 - 1.04 (m, 3H); MS (ESI) m/z = 356.1 $[M+H]^+$.

Step 6: Synthesis of ethyl-1-[3-[(1R)-1-aminoethyl]-2-fluoro-phenyllcyclopropanecarboxylate

To ethyl 1-[3-[(1R)-1-[[(S)- tert-butylsulfinyl]amino]ethyl]-2-fluorophenyl]cyclopropanecarboxylate (250 mg, 703.30 μmol) in dioxane (1 mL) was added dropwise HCl/dioxane (1 mL), and the mixture was degassed and purged with $N_2$ for 3 times. Thereafter, the mixture was stirred at 0 °C for 1 h under $N_2$ atmosphere. It was confirmed through LCMS that the starting materials were completely consumed. The mixture was concentrated under reduced pressure to give a crude product, ethyl-1-[3-[(1R)-1-aminoethyl]-2-fluoro-phenyl]cyclopropanecarboxylate (200 mg, 96.75% yield, 97.9% purity, hydrochloride salt) as a white solid, which was used in the next reaction without further purification. MS (ESI) m/z = 252.2 $[M+H]^+$.

Step 7: Synthesis of [1-[3-[(1R)-1-aminoethyl]-2-fluorophenylicyclopropyl methanol

To a solution of ethyl 1-[3-[(1R)-1-aminoethyl]-2-fluorophenyl]cyclopropanecarboxylate (200 mg, 795.87 μmol) in THF (3 mL) was added $LiAlH_4$ (90.62 mg, 2.39 mmol) at -10 °C, and then the mixture was stirred at -10 °C for 2 h. The mixture was quenched with aq. NaOH (15%, 0.6 mL) at 20 °C and filtered, and then the filtrate was concentrated under reduced pressure to give **Intermediate A-16** (165 mg, 74.30% yield, 75% purity) as yellow oil. $^1$H NMR (400 MHz, DMSO-$d_6$) δ 7.47 (dt, *J* = 1.6, 7.2 Hz, 1H), 7.21 (dt, *J* = 1.6, 7.2 Hz, 1H), 7.15 - 7.08 (m, 1H), 4.68 (br t, *J* = 5.6 Hz, 1H), 4.30 (q, *J* = 6.4 Hz, 1H), 3.50 (br d, *J* = 5.6 Hz, 2H), 1.29 (d, *J* = 6.4 Hz, 3H), 0.91 - 0.85 (m, 2H), 0.73 - 0.68 (m, 2H).

**Preparation Example 17: (1R)-1-[2-fluoro-3-(1-methylcyclopropyl)phenyl]ethanamine (A-17)**

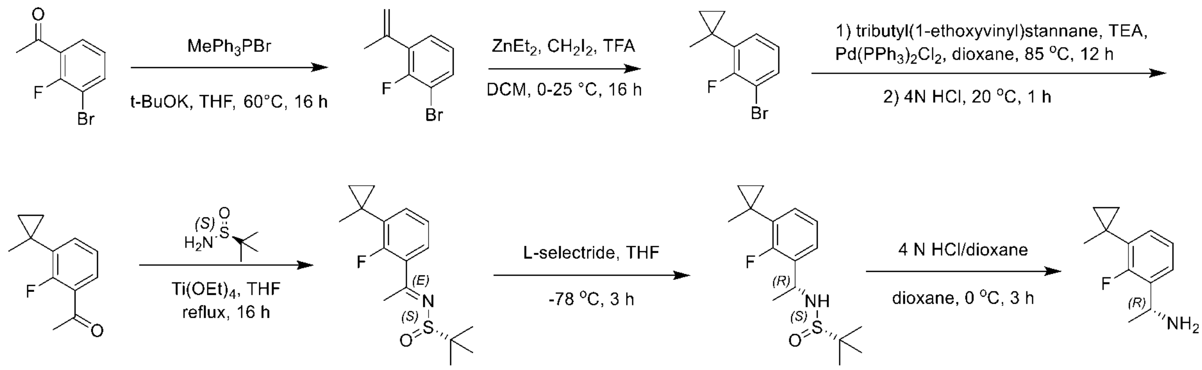


A-17

Step 1: Synthesis of 1-bromo-2-fluoro-3-isopropenyl-benzene

A solution of t-BuOK (5.17 g, 46.08 mmol, 1 eq) and methyl (triphenyl) phosphonium; bromide (16.46 g, 46.08 mmol) in THF (100 mL) was stirred at 20 °C for 1 h. After a solution of 1-(3-bromo-2-fluoro-phenyl)ethanone (10 g, 46.08 mmol) in THF (100 mL) was added, the reaction mixture was stirred at 60 °C for 16 h under $N_2$ atmosphere. The mixture was poured into water (80 mL) and extracted with EtOAc (80 mL X 3). The organic layers were washed with brine (100 mL), dried over anhydrous $Na_2SO_4$ and concentrated under reduced pressure to give a crude product. The crude product was purified by silica gel column chromatography (petroleum ether) to give 1-bromo-2-fluoro-3-isopropenyl-benzene (7.5 g, 68.12% yield, 90% purity) as white oil. $^1H$ NMR (400 MHz, DMSO-$d_6$) δ 7.66 - 7.49 (m, 1H), 7.36 (dt, *J* = 1.6, 7.4 Hz, 1H), 7.16 - 7.10 (m, 1H), 5.31 (d, *J* = 0.8 Hz, 1H), 5.22 (s, 1H), 2.08 (d, *J* = 0.8 Hz, 3H).

Step 2: Synthesis of 1-bromo-2-fluoro-3-(1-methylcyclopropyl)benzene

TFA (7.95 g, 69.75 mmol, 5.18 mL) was slowly added dropwise to diethylzinc (1 M, 69.75 mL) solution at 0 °C for 20 min. Thereafter, the mixture was stirred at 0 °C for 20 min, and then $CH_2I_2$ (18.68 g, 69.75 mmol, 5.63 mL) solution in DCM (45 mL) was added dropwise at 0 °C. The mixture was stirred at 0 °C for 20 min, and then 1-bromo-2-fluoro-3-isopropenyl-benzene (3 g, 13.95 mmol) was slowly added dropwise at 0 °C. The mixture was stirred at 25 °C for 12h. TLC (PE:EA = 10:1) indicated that the reaction was complete. The mixture was poured into water (30 mL) and extracted with DCM (30 mL X 3). The organic layers were washed with brine (50 mL), dried over anhydrous $Na_2SO_4$ and concentrated under reduced pressure to give a crude product. The crude product was purified by silica gel column chromatography (petroleum ether) to give 1-bromo-2-fluoro-3-(1-methylcyclopropyl)benzene (2.7 g, 76.04% yield, 90% purity) as colorless liquid. $^1H$ NMR (400 MHz, DMSO-$d_6$) δ 7.53 (ddd, *J* = 1.6, 6.4, 8.0 Hz, 1H), 7.32 (dt, *J* = 1.6, 7.2 Hz, 1H), 7.06 (dt, *J* = 0.8, 7.8 Hz, 1H), 1.29 (s, 3H), 0.78 - 0.75 (m, 2H), 0.73 - 0.70 (m, 2H).

Step 3: Synthesis of 1-[2-fluoro-3-(1-methylcyclopropyl)phenyllethanone

A mixture of tributyl(1-ethoxyvinyl)stannane (6.62 g, 18.33 mmol, 6.19 mL) and $Pd(PPh_3)_2Cl_2$ (612.77 mg, 873.03 μmol) was mixed with a solution of 1-bromo-2-fluoro-3-(1-methylcyclopropyl)benzene (4 g, 17.46 mmol) in dioxane (60 mL), degassed and purged with $N_2$ for 3 times, and then stirred at 100 °C for 16 h under $N_2$ atmosphere. After 4 N HCl solution (10 mL) was added, the mixture was stirred at 20 °C for 1 h. Following the addition of water (50 mL), the reaction mixture was extracted with EtOAc (30 mL X 3). The combined organic layers were washed with brine (30 mL X 3), dried over anhydrous $Na_2SO_4$ and concentrated under reduced pressure to give a residue. The residue was purified by column chromatography (petroleum ether) to give 1-[2-fluoro-3-(1-methylcyclopropyl)phenyl]ethanone (2.1 g, 60.69% yield, 97% purity) as yellow oil. $^1H$ NMR (400 MHz, DMSO-$d_6$) δ 7.64 (dt, *J* = 2.0, 7.6 Hz, 1H), 7.56 (dt, *J* = 2.0, 7.2 Hz, 1H), 7.21 (t, *J* = 7.6 Hz, 1H), 2.58 (d, *J* = 4.4 Hz, 3H), 1.32 (s, 3H), 0.79 - 0.76 (m, 2H), 0.74 - 0.71 (m, 2H); MS (ESI) m/z = 193.0 $[M+H]^+$.

Step 4: Synthesis of (NE, S)-N-[1-[2-fluoro-3-(1-methylcyclopropyl)phenyl]ethylidene]-2-methyl-propane-2-sulfinamide

To a solution of 1-[2-fluoro-3-(1-methylcyclopropyl)phenyl]ethanone (2 g, 10.40 mmol) in THF (30 mL) was added (S)-2-methylpropane-2-sulfinamide (1.51 g, 12.49 mmol) and $Ti(OEt)_4$ (4.75 g, 20.81 mmol, 4.32 mL), and then the mixture was purged with $N_2$ for 3 times. Thereafter, the mixture was stirred at 80 °C for 5 h under $N_2$ atmosphere. The mixture was poured into water (20 mL), and the reaction mixture was filtered and the filtrate was extracted with EtOAc (20 mL X 3). The organic layers were washed with brine (20 mL X 3), dried over anhydrous $Na_2SO_4$ and concentrated under

reduced pressure to give a crude product. The crude product was purified by silica gel column chromatography (2% EtOAc in petroleum ether) to give (NE, S)-N-[1-[2-fluoro-3-(1-methylcyclopropyl)phenyl]ethylidene]-2-methylpropane-2-sulfinamide (2.5 g, 77.44% yield, 95.209% purity) as yellow oil. MS (ESI) m/z = 296.2 $[M+H]^+$.

Step 5: Synthesis of (S)-N-[(1R)-1-[2-fluoro-3-(1-methylcyclopropyl)phenyl]ethyl]-2-methyl-propane-2-sulfinamide

L-selectride (1 M, 12.69 mL) was slowly added dropwise to (NE, S)-N-[1-[2-fluoro-3-(1-methylcyclopropyl)phenyl]ethylidene]-2-methyl-propane-2-sulfinamide (2.5 g, 8.46 mmol) in THF (40 mL) at -78 °C for 0.5 h under $N_2$ atmosphere, and then the mixture was stirred at -78 °C for 3 h under $N_2$ atmosphere. The reaction mixture was quenched with sat. aq. $NH_4Cl$ (50 mL) added dropwise slowly at -78 °C under $N_2$ atmosphere, and then diluted with EtOAc (60 mL) and extracted with EtOAc (60 mL X 3). The organic layers were dried over anhydrous $Na_2SO_4$ and concentrated under reduced pressure to give crude product. The crude product was purified by silica gel column chromatography (6% EtOAc in petroleum ether) to give (S)-N-[(1R)-1-[2-fluoro-3-(1-methylcyclopropyl)phenyl]ethyl]-2-methyl-propane-2-sulfinamide (2.3 g, 76.19% yield, 83.38% purity, ee = 98.7%) as yellow oil. $^1H$ NMR (400 MHz, DMSO-$d_6$) δ 7.34 - 7.25 (m, 1H), 7.19 (dt, *J* = 1.6, 7.6 Hz, 1H), 7.09 - 7.02 (m, 1H), 5.40 (d, *J* = 6.0 Hz, 1H), 4.67 (q, *J* = 6.4 Hz, 1H), 1.46 (d, *J* = 8.0 Hz, 3H), 1.28 (s, 3H), 1.09 (s, 9H), 0.74 - 0.70 (m, 2H), 0.68 (m, 2H); MS (ESI) m/z = 298.2 $[M+H]^+$.

Step 6: Synthesis of (1R)-1-[2-fluoro-3-(1-methylcyclopropyl)phenyl]ethanamine

To (S)-N-[(1R)-1-[2-fluoro-3-(1-methylcyclopropyl)phenyl]ethyl]-2-methylpropane-2-sulfinamide (200 mg, 672.43 μmol) in dioxane (2 mL) was added dropwise HCl/dioxane (2 mL), and then the mixture was degassed and purged with $N_2$ for 3 times. Thereafter, the mixture was stirred at 25 °C for 2 h under $N_2$ atmosphere. The mixture was concentrated under reduced pressure to give a crude product, which was treated with MTBE (2 mL) at 25 °C for 20 min to give **Intermediate A-17** (120 mg, 73.02% yield, 94% purity, hydrochloride) as a white solid. $^1H$ NMR (400 MHz, DMSO-$d_6$) δ 8.53 (br s, 3H), 7.52 - 7.47 (m, 1H), 7.35 (dt, *J* = 1.6, 7.2 Hz, 1H), 7.22 - 7.17 (m, 1H), 4.61 (br s, 1H), 1.51 (d, *J* = 6.8 Hz, 3H), 1.31 (s, 3H), 0.74 (br d, *J* = 10.0 Hz, 4H); MS (ESI) m/z = 194.0 $[M+H]^+$.

**Preparation Example 18: (1R)-1-deuterio-1-[2-fluoro-3-(trifluoromethyl)phenyl]ethanamine (A-18)**

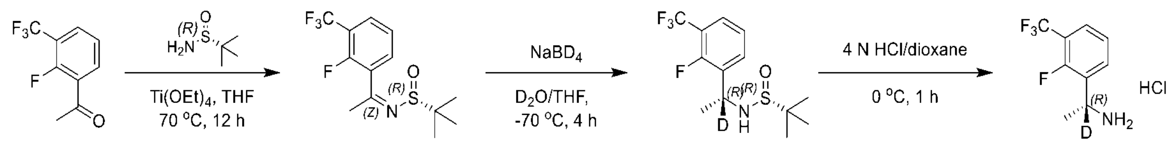


A-18

Step 1: Synthesis of (NZ,R)-N-[1-[2-fluoro-3-(trifluoromethyl)phenyl]ethylidene]-2-methyl-propane-2-sulfinamide

To a solution of 1-[2-fluoro-3-(trifluoromethyl)phenyl]ethanone (3 g, 14.55 mmol) in THF (50mL) was added $Ti(OEt)_4$ (9.96 g, 43.66 mmol, 9.05 mL) and (R)-2-propane-2-sulfinamide (2.65 g, 21.83 mmol), and then the mixture was stirred at 70 °C for 12 h under $N_2$ atmosphere. The mixture was poured into water (60 mL), and the reaction mixture was filtered and the filtrate was extracted with EtOAc (60 mL X 3). The organic layers were washed with brine (50 mL), dried over anhydrous $Na_2SO_4$ and concentrated under reduced pressure to give a crude product. The crude product was purified by silica gel column chromatography (8% EtOAc in petroleum ether) to give (NZ,R)-N-[1-[2-fluoro-3-(trifluoromethyl)phenyl]ethylidene]-2-methyl-propane-2-sulfinamide (4.36 g, 90.10% yield, 93.03% purity) as yellow oil. $^1H$ NMR (400MHz, DMSO-$d_6$) δ 7.99 (br t, *J* = 7.2 Hz, 1H), 7.92 (br t, *J* = 7.2 Hz, 1H), 7.51 (br t, *J* = 8.0 Hz, 1H), 2.72 (br d, *J* = 1.2 Hz, 3H), 1.22 (s, 9H); MS (ESI) m/z = 310.1$[M+H]^+$

Step 2: Synthesis of (R)-N-[(1R)-1-deuterio-1-[2-fluoro-3-(trifluoromethyl)phenyl]ethyl]-2-methyl-propane-2-sulfinamide

To a solution of (NZ,R)-N-[1-[2-fluoro-3-(trifluoromethyl)phenyl]ethylidene]-2-methyl-propane-2-sulfinamide (500 mg, 1.62 mmol) in THF (10 mL) and $D_2O$ (0.2 mL) was added $NaBD_4$ (61.15 mg, 1.62 mmol) at -70 °C, and then the mixture was stirred at -70 °C for 4 h under $N_2$ atmosphere. The mixture was quenched with sat. $NH_4Cl$ (50 mL) and poured into water (30 mL). It was extracted with EtOAc (40 mL X 3). The organic layers were washed with brine (40 mL), dried over anhydrous $Na_2SO_4$ and concentrated under reduced pressure to give a crude product. The crude product was

purified by silica gel column chromatography (18% EtOAc/petroleum ether) to give (R)-N-[(1R)-1-deuterio-1-[2-fluoro-3-(trifluoromethyl)phenyl]ethyl]-2-methyl-propane-2-sulfinamide (330 mg, 48.42% yield, 74.08% purity, 98.06% ee) as yellow oil. $^1$H NMR (400MHz, DMSO-$d_6$) δ 7.89 (br t, *J* = 7.2 Hz, 1H), 7.70-7.62 (m, 1H), 7.42 (t, *J* = 8.0 Hz, 1H), 5.91 (s, 1H), 1.42 (s, 3H), 1.10 (s, 9H). MS (ESI) m/z = 312.9[M+H]$^+$.

Step 3: Synthesis of (1R)-1-deuterio-1-[2-fluoro-3-(trifluoromethyl)phenyl]ethanamine

To a solution of (R)-N-[(1R)-1-deuterio-1-[2-fluoro-3-(trifluoromethyl)phenyl]ethyl]-2-methyl-propane-2-sulfinamide (330 mg, 1.06 mmol) in dioxane (4 mL) was added 4N HCl/dioxane (4 mL) 0°C, and then the mixture was stirred at 0 °C for 1h. The mixture was concentrated under reduced pressure to give **Intermediate A-18** (200 mg, 90.93% yield, 90% purity) as a yellow solid. $^1$H NMR (400MHz, DMSO-$d_6$) δ 8.80 (br s, 3H), 8.09 (t, *J* = 7.2 Hz, 1H), 7.82 (t, *J* = 7.2 Hz, 1H), 7.52 (t, *J* = 8.0 Hz, 1H), 1.56 (s, 3H); MS (ESI) m/z = 209.1 [M+H]$^+$.

**Preparation Example 19: (1R)-2,2,2-trideuterio-1-[2-fluoro-3-(trifluoromethyl)phenyl]ethanamine (A-19)**

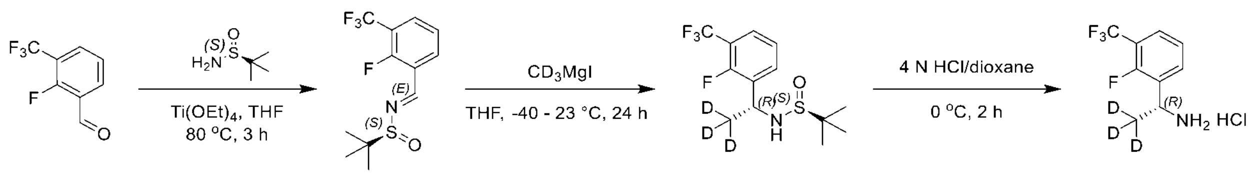


A-19

Step 1: Synthesis of (NE, S)-N-[[2-fluoro-3-(trifluoromethyl)phenyl]methylene]-2-methyl-propane-2-sulfinamide

To a solution of 2-fluoro-3-(trifluoromethyl)benzaldehyde (5 g, 26.03 mmol, 3.59 mL) in THF (60 mL) was added (S)-2-methylpropane-2-sulfinamide (4.73 g, 39.04 mmol) and $Ti(OEt)_4$ (17.81 g, 78.08 mmol, 16.19 mL), and then the mixture was degassed and purged with $N_2$ for 3 times. Thereafter, the mixture was stirred at 80 °C for 3 h under $N_2$ atmosphere. The mixture was poured into water (40 mL), and the reaction mixture was filtered and extracted with EtOAc (100 mL X 3). The organic layers were washed with brine (40 mL X 3), dried over anhydrous $Na_2SO_4$ and concentrated under reduced pressure to give a crude product. The crude product was purified by silica gel column chromatography (5% EtOAc in petroleum ether) to give (NE, S)-N-[[2-fluoro-3-(trifluoromethyl)phenyl]methylene]-2-methyl-propane-2-sulfinamide (7.3 g, 93.75% yield, 98.7% purity) as a white solid. MS (ESI) m/z = 295.9 [M+H]$^+$.

Step 2: Synthesis of (S)-2-methyl-N-[(1R)-2,2,2-trideuterio-1-[2-fluoro-3-(trifluoromethyl)phenyl]ethyl]propane-2-sulfinamide

To a solution of (NE, S)-N-[[2-fluoro-3-(trifluoromethyl)phenyl]methylene]-2-methyl-propane-2-sulfinamide (1.00 g, 3.39 mmol) in THF (15 mL) was slowly added dropwise iodo(trideuteriomethyl)magnesium (1 M, 5.08 mL) at -40 °C. Thereafter, the mixture was stirred at -40 °C for 6 h, and then it was warmed to 23 °C and stirred for 12 h. The mixture was quenched with sat. aq. $NH_4Cl$ (20 mL) added dropwise slowly at 0 °C, and extracted with EtOAc (30 mL X 3). The organic layers were washed with brine (10 mL X 3), dried over anhydrous $Na_2SO_4$ and concentrated under reduced pressure to give a crude product. The crude product was purified by silica gel column chromatography (9% EtOAc in petroleum ether) to give (S)-2-methyl-N-[(1R)-2,2,2-trideuterio-1-[2-fluoro-3-(trifluoromethyl)phenyl]ethyl]propane-2-sulfinamide (65 mg, 3.69% yield, 60.5% purity, ee = 98.26%) as yellow oil. MS (ESI) m/z = 314.9 [M+H]$^+$.

Step 3: Synthesis of (1R)-2,2,2-trideuterio-1-[2-fluoro-3-(trifluoromethyl)phenyllethanamine

To a solution of (S)-2-methyl-N-[(1R)-2,2,2-trideuterio-1-[2-fluoro-3-(trifluoromethyl)phenyl]ethyl]propane-2-sulfinamide (65 mg, 206.77 μmol) in dioxane (1 mL) was added HCl/dioxane (4 M, 1 mL) at 0°C, and then the mixture was degassed and purged with $N_2$ for 3 times. Thereafter, the mixture was stirred at 25 °C for 1h under $N_2$ atmosphere. The mixture was concentrated under reduced pressure to give **Intermediate A-19** (50 mg, 78.43% yield, 80% purity, hydrochloride) as a yellow solid. $^1$H NMR (400MHz, DMSO-$d_6$) δ 8.50 (br s, 3H), 7.97 (t, *J* = 7.2 Hz, 1H), 7.84 (t, *J* = 7.2 Hz, 1H), 7.54 (t, *J* = 8.0 Hz, 1H), 4.71 (br d, *J* = 5.2 Hz, 1H); MS (ESI) m/z = 314.3 [M+H]$^+$.

**Preparation Example 20: 2-[3-[(1R)-1-aminoethyl]-2-fluoro-phenyl]-1,1-dideuterio-2,2-difluoro-ethanol (A-20)**

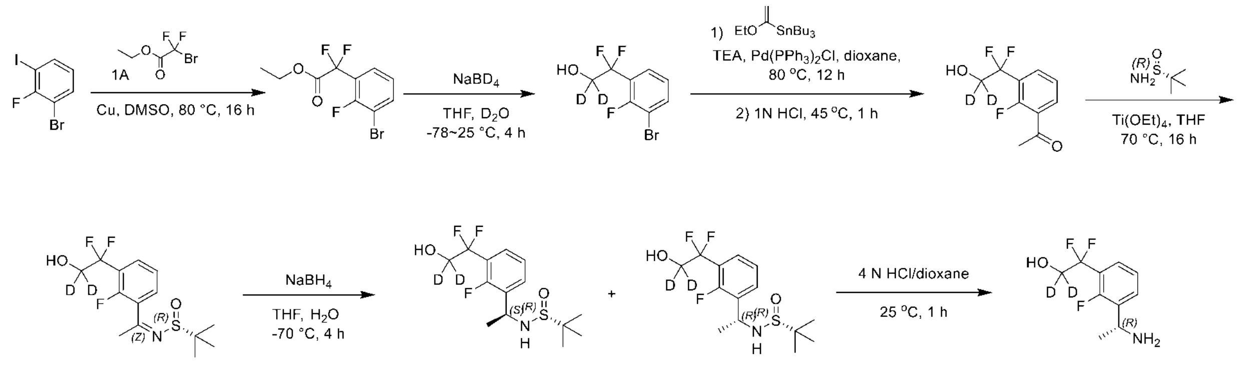


A-20

Step 1: Synthesis of ethyl 2-(3-bromo-2-fluoro-phenyl)-2,2-difluoro-acetate

To a solution of ethyl 2-bromo-2,2-difluoro-acetate (14.17 g, 69.79 mmol, 8.99 mL, 3 eq) in DMSO (200 mL) was added copper (4.43 g, 69.79 mmol, 494.98 μL, 3 eq) and 1-bromo-2-fluoro-3-iodo-benzene (7 g, 23.26 mmol, 1 eq). The reaction mixture was stirred at 85 °C for 16 h under $N_2$ atmosphere. TLC (PE:EA = 1:0) indicated that the reactants were completely consumed and one major spot was formed. The reaction mixture was filtered, and then the filtrate was added with water (80 mL) and extracted with EtOAc (80 mL X 3). The organic layers were washed with brine (60 mL), dried over anhydrous $Na_2SO_4$ and concentrated under reduced pressure to give a crude product. The crude product was purified by silica gel column chromatography (0~10% ethyl acetate/Petroleum ether) to give ethyl 2-(3-bromo-2-fluorophenyl)-2,2-difluoro-acetate (5.68 g, 78.08% yield, 95% purity) as colorless oil. $^1$H NMR (400 MHz, DMSO-$d_6$) δ 7.99 (t, *J* = 7.2 Hz, 1H), 7.75 - 7.70 (m, 1H), 7.37 (t, *J* = 8.0 Hz, 1H), 4.36 (q, *J* = 7.2 Hz, 2H), 1.23 (t, *J* = 7.2 Hz, 3H).

Step 2: Synthesis of 2-(3-bromo-2-fluoro-phenyl)-1,1-dideuterio-2,2-difluoro-ethanol

To a solution of ethyl 2-(3-bromo-2-fluoro-phenyl)-2,2-difluoro-acetate (3 g, 10.10 mmol) in THF (40 mL)/$D_2O$ (0.8 mL) was added $NaBD_4$ (496.68 mg, 13.13 mmol) at -78 °C, and then the reaction mixture was stirred at 25 °C for 4 h under $N_2$ atmosphere. The mixture was quenched with sat. aq. $NH_4Cl$ (50 mL) added dropwise slowly at 20 °C, and extracted with EtOAc (40 mL X 3) after the addition of water (30mL). The organic layers were washed with brine (50 mL), dried over anhydrous $Na_2SO_4$ and concentrated under reduced pressure to give a crude product. The crude product was purified by silica gel column chromatography (0~11% EtOAc/PE) to give 2-(3-bromo-2-fluoro-phenyl)-1,1-dideuterio-2,2-difluoro-ethanol (2.27 g, 87.45% yield) as light gray oil. $^1$H NMR (400 MHz, DMSO-*$d_6$*) δ 7.95 - 7.85 (m, 1H), 7.64 - 7.50 (m, 1H), 7.29 (t, *J* = 8.0 Hz, 1H), 5.70 (s, 1H).

Step 3: Synthesis of 1-[3-(2,2-dideuterio-1,1-difluoro-2-hydroxy-ethyl)-2-fluoro-phenyllethanone

A mixture of tributyl(1-ethoxyvinyl)stannane (4.33 g, 11.99 mmol, 4.05 mL), TEA (1.63 g, 16.11 mmol, 2.24 mL) and $Pd(PPh_3)_2Cl_2$ (565.24 mg, 805.31 μmol) was mixed with a solution of 2-(3-bromo-2-fluoro-phenyl)-1,1-dideuterio-2,2-difluoro-ethanol (2.07 g, 8.05 mmol) in dioxane (40 mL), degassed and purged with $N_2$ for 3 times, and then stirred at 80 °C for 16 h under $N_2$ atmosphere. The mixture was added with Aq. HCl (80 ml, 1 M) under stirring at 25 °C, and then stirred at 25 °C for 1 h. Following the addition of water (40 mL), the reaction mixture was extracted with EtOAc (20 mL X 3). The combined organic layers were washed with brine (40 mL X 3), dried over anhydrous $Na_2SO_4$ and concentrated under reduced pressure to give a residue. Sat. aq. KF (60 mL) was slowly added, and then the mixture was stirred at 25 °C for 1 h. The residue was purified by silica gel column chromatography (0~14% EtOAc/PE) to give 1-[3-(2,2-dideuterio-1,1-difluoro-2-hydroxy-ethyl)-2-fluorophenyl]ethanone (1.6 g, 90.23% yield) as a white solid. $^1$H NMR (400 MHz, DMSO-$d_6$) δ 7.99 - 7.90 (m, 1H), 7.83 - 7.73 (m, 1H), 7.43 (t, *J* = 7.6 Hz, 1H), 5.70 (s, 1H), 4.04 (s, 1H), 2.60 (d, *J* = 4.4 Hz, 3H); MS (ESI) m/z = 221.1 $[M+H]^+$.

Step 4: Synthesis of (NZ,R)-N-[1-[3-(2,2-dideuterio-1,1-difluoro-2-hydroxy-ethyl)-2-fluoro-phenyl]ethylidene]-2-methyl-propane-2-sulfinamide

To a solution of 1-[3-(2,2-dideuterio-1,1-difluoro-2-hydroxy-ethyl)-2-fluorophenyl]ethanone (230 mg, 1.04 mmol) in THF (5 mL) was added (R)-2-methylpropane-2-sulfinamide (189.91 mg, 1.57 mmol) and $Ti(OEt)_4$ (714.83 mg, 3.13 mmol, 649.85 μL), and then the mixture was stirred at 70 °C for 16 h under $N_2$ atmosphere. The mixture was poured into water (20 mL) and extracted with EtOAc (20 mL X 3). The organic layers were washed with brine (20 mL X 2), dried over anhydrous $Na_2SO_4$ and concentrated under reduced pressure to give a crude product. The crude product was purified by silica gel column chromatography (0~20% EtOAc/PE) to give (NZ,R)-N-[1-[3-(2,2-dideuterio-1,1-difluoro-2-hydroxy-ethyl)-2-fluoro-phenyl]ethylidene]-2-methyl-propane-2-sulfinamide (250 mg, 72.31% yield, 97.7% purity) as yellow oil. $^1H$ NMR (400 MHz, DMSO-$d_6$) δ 7.79 (br t, *J* = 6.8 Hz, 1H), 7.68 (br t, *J* = 6.8 Hz, 1H), 7.41 (br t, *J* = 7.6 Hz, 1H), 5.70 (s, 1H), 2.69 (br d, *J* = 1.6 Hz, 3H), 1.22 (s, 9H); MS (ESI) m/z = 324.0 $[M+H]^+$.

Step 5: Synthesis of (R)-N-[(1R)-1-[3-(2,2-dideuterio-1,1-difluoro-2-hydroxy-ethyl)-2-fluoro-phenyl]ethyl]-2-methyl-propane-2-sulfinamide

To a solution of (NZ,R)-N-[1-[3-(2,2-dideuterio-1,1-difluoro-2-hydroxy-ethyl)-2-fluoro-phenyl]ethylidene]-2-methyl-propane-2-sulfinamide (250 mg, 773.11 μmol) in THF (5 mL) and $H_2O$ (0.1 mL) was added $NaBH_4$ (29.25 mg, 773.11 μmol) at -70 °C, and then the mixture was stirred at -70 °C for 4 h under $N_2$ atmosphere. The mixture was quenched with sat. aq. $NH_4Cl$ (20 mL) at 20 °C, added with water (20 mL) and then extracted with EtOAc (20 mL X 3). The organic layers were washed with brine (30 mL), dried over anhydrous $Na_2SO_4$ and concentrated under reduced pressure to give a crude product. The crude product was purified by silica gel column chromatography (0-45% EtOAc/PE) and prep-HPLC (column: Welch Xtimate C18 40*200mm 7μm; mobile phase: [water ($NH_3H_2O$ + $NH_4HCO_3$)-ACN]; gradient: 16%-56% B over 25 min). The solvent was concentrated under reduced pressure, and then the residue was lyophilized to give (R)-N-[(1S)-1-[3-(2,2-dideuterio-1,1-difluoro-2-hydroxy-ethyl)-2-fluoro-phenyl]ethyl]-2-methyl-propane-2-sulfinamide (30 mg, 11.93% yield, 100% purity, ee = 100 %) as a white solid. $^1H$ NMR (400 MHz, DMSO-$d_6$) δ 7.63 (br t, *J* = 7.2 Hz, 1H), 7.43 (t, *J* = 7.2 Hz, 1H), 7.38 - 7.22 (m, 1H), 6.04 (br d, *J* = 1.6 Hz, 1H), 5.53 (d, *J* = 5.6 Hz, 1H), 4.71 (br t, *J* = 6.4 Hz, 1H), 2.54 (s, 1H), 1.48 (d, *J* = 6.8 Hz, 3H), 1.09 (s, 9H); LC/MS (ESI) m/z = 326.2 $[M+H]^+$. Additionally, the desired product (R)-N-[(1R)-1-[3-(2,2-dideuterio-1,1-difluoro-2-hydroxy-ethyl)-2-fluoro-phenyl]ethyl]-2-methyl-propane-2-sulfinamide (90 mg, 276.59 μmol, 35.78% yield, 100% purity, ee = 99.96 %) was obtained as a white solid. $^1H$ NMR (400 MHz, DMSO-$d_6$) δ 7.70 (t, *J* = 7.2Hz, 1H), 7.47 - 7.40 (m, 1H), 7.34 - 7.26 (m, 1H), 5.86 (d, *J* = 7.6 Hz, 1H), 4.68 (t, *J* = 7.2 Hz, 1H), 1.40 (d, *J* = 6.8 Hz, 3H), 1.10 (s, 9H); MS (ESI) m/z = 326.2 $[M+H]^+$.

Step 6: Synthesis of 2-[3-[(1R)-1-aminoethyl]-2-fluoro-phenyl]-1,1-dideuterio-2,2-difluoro-ethanol

HCl/dioxane (4 mL) was added to (R)-N-[(1R)-1-[3-(2,2-dideuterio-1,1-difluoro-2-hydroxy-ethyl)-2-fluoro-phenyl]ethyl]-2-methyl-propane-2-sulfinamide (90 mg, 276.59 μmol) in dioxane (1 mL). Thereafter, the mixture was stirred at 25 °C for 2 h. LCMS confirmed the complete consumption of starting materials and the formation of the desired compound. The mixture was concentrated under reduced pressure to give **Intermediate A-20** (60 mg, 94.14% yield, 96% purity) as light gray oil. $^1H$ NMR (400 MHz, DMSO-$d_6$) δ 8.66 (br s, 2H), 7.84 (t, *J* = 7.2 Hz, 1H), 7.58 (t, *J* = 6.8 Hz, 1H), 7.46 - 7.34 (m, 1H), 4.75 - 4.56 (m, 1H), 1.54 (d, *J* = 6.4 Hz, 3H).

**Preparation Example 21: (+/-)-1,2,2,2-tetradeuterio-1-[2-fluoro-3-(trifluoromethyl)phenyl]ethanamine (A-21)**

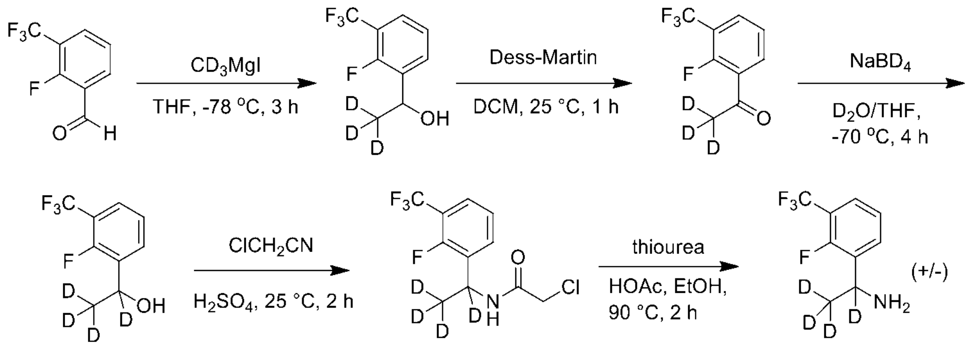


**A-21**

Step 1: Synthesis of 2,2,2-trideuterio- 1-[2-fluoro-3-(trifluoromethyl)phenyl]ethanol

To a solution of (2-fluoro-3-(trifluoromethyl)benzaldehyde (1 g, 5.21 mmol, 717.88 μL) in THF (20 mL) was slowly added dropwise iodo(trideuteriomethyl)magnesium (1 M, 10.41 mL) at -78 °C under $N_2$ atmosphere, and then the mixture was stirred at -78 °C for 3 h. The mixture was quenched by slowly adding sat. aq. $NH_4Cl$ (20 mL) dropwise and extracted with EtOAc (30 mL X 3). The organic layers were washed with brine (30 mL), dried over anhydrous $Na_2SO_4$ and concentrated under reduced pressure to give a crude product. The crude product was purified by silica gel column chromatography (2% EtOAc in petroleum ether) to give 2,2,2-trideuterio-1-[2-fluoro-3-(trifluoromethyl)phenyl]ethanol (900 mg, 81.88% yield, 95% purity) as yellow oil. $^1H$ NMR (400 MHz, DMSO-$d_6$) δ 7.85 (t, *J* = 7.2 Hz, 1H), 7.64 (t, *J*= 6.8 Hz, 1H), 7.40 (t, *J* = 8.0 Hz, 1H), 5.49 (d, *J* = 4.4 Hz, 1H), 5.03 (br d, *J* = 4.4 Hz, 1H).

Step 2: Synthesis of 2,2,2-trideuterio-1-[2-fluoro-3-(trifluoromethyl)phenyl]ethanone

To a solution of 2,2,2-trideuterio-1-[2-fluoro-3-(trifluoromethyl)phenyl]ethanol (900 mg, 4.26 mmol) in DCM (10 mL) was added (1,1-diacetoxy-3-oxo-1,2-benziodoxol-1-yl)acetate (2.17 g, 5.11 mmol, 1.58 mL) at 0 °C. Thereafter, the mixture was warmed to 25 °C and stirred at 25 °C for 1 h. The mixture was added with sat. aq. $Na_2CO_3$ (20 mL) and extracted with DCM (20 mL X 3). The organic layers were washed with brine (30 mL), dried over anhydrous $Na_2SO_4$ and concentrated under reduced pressure to give a crude product. The crude product was purified by silica gel column chromatography (1% EtOAc in petroleum ether) to give 2,2,2-trideuterio-1-[2-fluoro-3-(trifluoromethyl)phenyl]ethanone (630 mg, 70.67% yield, 90% purity) as white oil. $^1H$ NMR (400 MHz, DMSO-$d_6$) δ 8.13-8.05 (m, 1H), 7.85-7.77 (m, 1H), 7.35 (t, *J* = 8.0 Hz, 1H).

Step 3: Synthesis of 1,2,2,2-tetradeuterio-1-[2-fluoro-3-(trifluoromethyl)phenyl]ethanol

To a solution of 2,2,2-trideuterio-1-[2-fluoro-3-(trifluoromethyl)phenyl]ethanone (530 mg, 2.53 mmol) in THF (8 mL) and $D_2O$ (0.16 mL) was added $NaBD_4$ (95.87 mg, 2.53 mmol) at -70 °C under $N_2$ atmosphere, and then the mixture was stirred at -70 °C for 4 h under $N_2$ atmosphere. The mixture was quenched with sat. aq. $NH_4Cl$ (10 mL) and extracted with EtOAc (10 mL X 3). The organic layers were washed with brine (20 mL), dried over anhydrous $Na_2SO_4$ and concentrated under reduced pressure to give a crude product. The crude product was purified by silica gel column chromatography (5% EtOAc in petroleum ether) to give 1, 2, 2, 2-tetradeuterio-1-[2-fluoro-3-(trifluoromethyl)phenyl] ethanol (430 mg, 79.98% yield, 95% purity) as white oil. $^1H$ NMR (400 MHz, DMSO-$d_6$) δ 7.85 (t, *J* = 7.2 Hz, 1H), 7.64 (t, *J* = 7.2 Hz, 1H), 7.40 (t, *J* = 8.0 Hz, 1H), 5.48 (s, 1H).

Step 4: Synthesis of 2-chloro-N-[1,2,2,2-tetradeuterio-1-[2-fluoro-3-(trifluoromethyl)phenyl]ethyl]acetamide

To a solution of 1,2,2,2-tetradeuterio-1-[2-fluoro-3-(trifluoromethyl)phenyl]ethanol (400 mg, 1.89 mmol) in 2-chloroacetonitrile (2.92 g, 38.65 mmol, 2.45 mL) was slowly added sulfuric acid (2.22 g, 22.62 mmol, 1.21 mL) at 0 °C. Thereafter, the mixture was stirred at 25 °C for 2 h under $N_2$ atmosphere. The mixture was quenched with cold water (10 mL), adjusted to pH = 10-13 with sat. aq. NaOH, and then extracted with DCM (10 mL X 3). The organic layers were washed with brine (30 mL), dried over anhydrous $Na_2SO_4$ and concentrated under reduced pressure to give a crude product. The crude product was purified by silica gel column chromatography (8% EtOAc in petroleum ether) to give 2-chloro-N-[1,2,2,2-tetradeuterio-1-[2-fluoro-3-(trifluoromethyl)phenyl]ethyl]acetamide (510 mg, 71.05% yield, 75.55% purity) as yellow oil. $^1H$ NMR (400 MHz, DMSO-$d_6$) δ 8.86 (s, 1H), 7.69 (td, *J* = 7.6, 15.2 Hz, 2H), 7.41 (t, *J* = 8.0 Hz, 1H), 4.09 (s, 2H); MS (ESI) m/z = 287.9 $[M+H]^+$.

Step 5: Synthesis of (+/-)-1,2,2,2-tetradeuterio-1-[2-fluoro-3-(trifluoromethyl)phenyl]ethanamine

To a solution of 2-chloro-N-[1,2,2,2-tetradeuterio-1-[2-fluoro-3-(trifluoromethyl)phenyl]ethyl]acetamide (250 mg, 869.04 μmol) in acetic acid (0.6 mL) was added thiourea (72.77 mg, 955.94 μmol) and ethanol (3 mL), and then the mixture was stirred at 90 °C for 2 h. The mixture was diluted with water (5 mL), adjusted to pH = 9-10 with sat. aq. NaOH, and then extracted with EtOAc (5 mL X 3). The organic layers were washed with brine (10 mL), dried over anhydrous $Na_2SO_4$ and concentrated under reduced pressure to give a crude product. The crude product was purified by silica gel column chromatography (58% EtOAc in petroleum ether) to give **Intermediate A-21** (180 mg, 73.38% yield, 74.82% purity) as a yellow solid. $^1H$ NMR (400 MHz, DMSO-$d_6$) δ 8.81 (br s, 1H), 8.05 (br t, *J* = 7.2 Hz, 1H), 7.76 (br t, *J* = 7.2 Hz, 1H), 7.48 (t, *J* = 8.0 Hz, 1H); MS (ESI) m/z = 212.0 $[M+H]^+$.

**Preparation Example 22: 2-[3-[(1R)-1-amino-2,2,2-trideuterio-ethyl]-2-fluorophenyl]-2,2-difluoro-ethanol (A-22)**

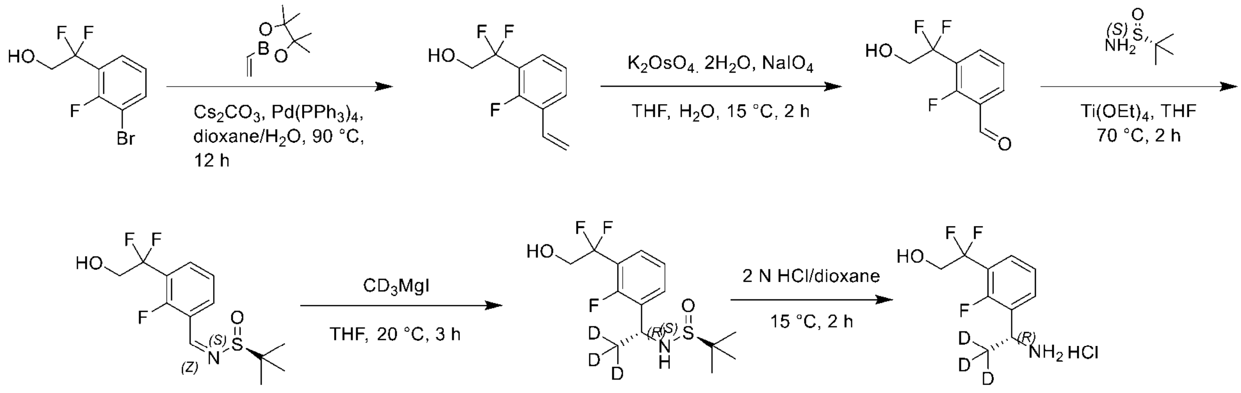


A-22

Step 1: Synthesis of 2,2-difluoro-2-(2-fluoro-3-vinyl-phenyl)ethanol

To a solution of 2-(3-bromo-2-fluoro-phenyl)-2,2-difluoro-ethanol (3 g, 11.76 mmol) in dioxane (40 mL) and $H_2O$ (5 mL) was added 4,4,5,5-tetramethyl-2-vinyl-1,3,2-dioxoborolan(4.53 g, 29.41 mmol), $Cs_2CO_3$ (15.33 g, 47.05 mmol) and $Pd(PPh_3)_4$ (2.04 g, 1.76 mmol). The mixture was degassed and substituted with nitrogen for 3 times, and then it was stirred at 90 °C for 3 h under nitrogen atmosphere. The reaction mixture was filtered and concentrated under reduced pressure to give a crude product. The crude product was purified by silica gel column chromatography (4% EtOAc in petroleum ether) to give 2,2-difluoro-2-(2-fluoro-3-vinyl-phenyl)ethanol (1.44 g, 57.52% yield, 95% purity) as yellow oil. $^1H$ NMR (400 MHz, DMSO-$d_6$) δ 7.81 (t, *J* = 7.2 Hz, 1H), 7.47 (t, *J* = 6.8 Hz, 1H), 7.33-7.27 (m, 1H), 6.88 (dd, *J* = 11.2, 17.6 Hz, 1H), 5.98 (d, *J* = 18.0 Hz, 1H), 5.69 (t, *J* = 6.4 Hz, 1H), 5.50 (d, *J* = 11.2 Hz, 1H), 3.97-3.87 (m, 2H).

Step 2: Synthesis of 3-(1,1-difluoro-2-hydroxy-ethyl)-2-fluoro-benzaldehyde

To a solution of 2,2-difluoro-2-(2-fluoro-3-vinyl-phenyl)ethanol (1.44 g, 7.12 mmol) in THF (16 mL) and $H_2O$ (4 mL) was added dipotassium;dioxido(dioxo)osmium;dihydrate (262.44 mg, 712.26 μmol) and $NaIO_4$ (5.94 g, 27.78 mmol) at 0 °C. Thereafter, the reaction mixture was degassed, substituted with nitrogen 3 times, and then stirred at 15 °C for 2 h under nitrogen atmosphere. The reaction mixture was filtered, and the filtrate was poured into $H_2O$ (4 mL) and extracted with DCM (20 mL × 3). The combined organic layers were washed with brine (30 mL), dried over anhydrous $Na_2SO_4$ and concentrated under reduced pressure to give a crude product. The crude product was purified by silica gel column chromatography (7% EtOAc in petroleum ether) to give 3-(1,1-difluoro-2-hydroxy-ethyl)-2-fluoro-benzaldehyde (990 mg, 64.68% yield, 95% purity) as yellow oil. $^1H$ NMR (400 MHz, DMSO-$d_6$) δ 10.26 (s, 1H), 8.03-7.95 (m, 1H), 7.88 (dt, *J* = 1.6, 7.6 Hz, 1H), 7.50 (t, *J* = 7.6 Hz, 1H), 5.83-5.71 (m, 1H), 4.09-3.81 (m, 2H).

Step 3: Synthesis of (NZ,S)-N-[[3-(1,1-difluoro-2-hydroxy-ethyl)-2-fluorophenyl]methylene]-2-methyl-propane-2-sulfinamide

Commercially available (S)-2-methylpropane-2-sulfinamide was used in the same manner as in Step 1 of Preparation Example 19 to give (NZ,S)-N-[[3-(1,1-difluoro-2-hydroxyethyl)-2-fluoro-phenyl]methylene]-2-methyl-propane-2-sulfinamide (850 mg, 93.01% yield, 97.19% purity) as yellow oil. MS (ESI) m/z = 308.0 $[M+H]^+$.

Step 4: Synthesis of (S)-2-methyl-N-[(1R)-2,2,2-trideuterio-1-[3-(1,1-difluoro-2-hydroxy-ethyl)-2-fluoro-phenyl]ethyl]propane-2-sulfinamide

Commercially available iodo(trideuteriomethyl)magnesium (1 M in THF) was used in the same manner as in Step 2 of Preparation Example 19 to give (S)-2-methyl-N-[(1R)-2,2,2-trideuterio-1-[3-(1,1-difluoro-2-hydroxy-ethyl)-2-fluoro-phenyl]ethyl]propane-2-sulfinamide (250 mg, 30.79% yield, 98.53% purity) as yellow oil. MS (ESI) m/z = 327.0 $[M+H]^+$.

Step 5: Synthesis of 2-[3-[(1R)-1-amino-2,2,2-trideuterio-ethyl]-2-fluoro-phenyl]-2,2-difluoro-ethanol

**Intermediate A-22** (160 mg, 64.60% yield, 80% purity, hydrochloride) was obtained in the same manner as in Step 3 of Preparation Example 19 as yellow oil. $^1H$ NMR (400 MHz, DMSO-$d_6$) δ 8.75 (br s, 3H), 7.87 (t, *J* = 6.8 Hz, 1H), 7.57 (t, *J* = 6.8 Hz, 1H), 7.40 (t, *J* = 8.0 Hz, 1H), 5.99-5.44 (m, 1H), 4.63 (br d, *J* = 4.0 Hz, 1H), 3.93 (br t, *J* = 14.4 Hz, 2H); MS (ESI) m/z = 223.1 $[M+H]^+$.

**Preparation Example 22-1: (1R)-1-[2-fluoro-3-(1,1,2-trifluoroethyl)phenyl]ethanamine (A-23)**

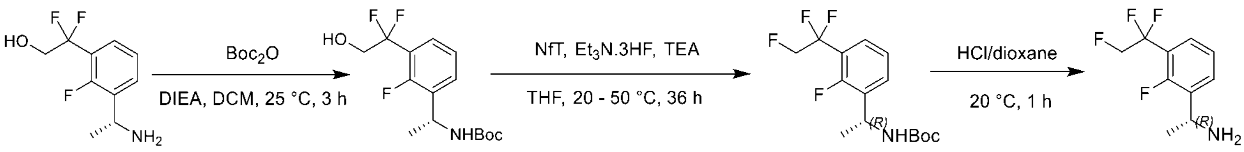


A-1 A-23

Step 1: Synthesis of tert-butyl *N*-[(1R)-1-[3-(1,1-difluoro-2-hydroxyethyl)-2-fluorophenyl]ethyl]carbamate

A mixture of **Intermediate A-1** (1 g, 3.91 mmol, HCl), $Boc_2O$ (1.71 g, 7.82 mmol, 1.80 mL) and DIEA (1.01 g, 7.82 mmol, 1.36 mL) in DCM (11 mL) was degassed and purged with nitrogen for 3 times. Thereafter, the mixture was stirred at 25 °C for 3 h under nitrogen atmosphere. The mixture was concentrated under reduced pressure to give a crude product. The crude product was purified by silica gel column chromatography (15% EA in PE) to give tert-butyl N-[(1R)-1-[3-(1,1-difluoro-2-hydroxyethyl)-2-fluorophenyl]ethyl]carbamate (848 mg, 59.75% yield, 88% purity) as colorless oil. $^1H$ NMR (400 MHz, DMSO-$d_6$) δ 7.60 - 7.50 (m, 2H), 7.44 - 7.39 (m, 1H), 7.30 - 7.26 (m, 1H), 5.70 (t, *J* = 6.4 Hz, 1H), 4.92 (br t, *J* = 7.2 Hz, 1H), 3.91 (dt, *J* = 6.4, 14.4 Hz, 2H), 1.36 (s, 9H), 1.29 (d, *J* = 7.2 Hz, 3H); MS (ESI) m/z = 264.0 $[M+H-56]^+$.

Step 2: Synthesis of tert-butyl *N*-[(1R)-1-[2-fluoro-3-(1,1,2-trifluoroethyl)phenyl]ethyl]carbamate

To a solution of tert-butyl N-[(1R)-1-[3-(1,1-difluoro-2-hydroxyethyl)-2-fluorophenyl]ethyl]carbamate (400 mg, 1.25 mmol), *N, N*-diethylethanamine;trihydrofluoride (605.82 mg, 3.76 mmol, 612.56 μL) and *N,N*-diethylethanamine (760.54 mg, 7.52 mmol, 1.05 mL) in anhydrous THF (10 mL) was slowly added dropwise NfT (1.14 g, 3.76 mmol, 661.57 μL) at 20 °C. Thereafter, the mixture was stirred at 50 °C for 36 h. The reaction mixture was concentrated under reduced pressure to give a crude product. The crude product was purified by silica gel column chromatography (5% EtOAc in PE) to give tert-butyl *N*-[(1*R*)-1-[2-fluoro-3-(1,1,2-trifluoroethyl)phenyl]ethyl]carbamate (346 mg, 82.84% yield, 96.37% purity) as a white solid. $^1H$ NMR (400 MHz, DMSO-$d_6$) δ 7.66 - 7.54 (m, 2H), 7.51 - 7.46 (m, 1H), 7.37 - 7.32 (m, 1H), 5.03 (t, *J* = 13.8 Hz, 1H), 4.96 - 4.87 (m, 2H), 1.36 (s, 9H), 1.30 (d, *J* = 7.2 Hz, 3H); MS (ESI) m/z = 266.0 $[M+H-56]^+$.

Step 3: Synthesis of (1R)-1-[2-fluoro-3-(1,1,2-trifluoroethyl)phenyllethanamine

A mixture of *N*-[(1R)-1-[2-fluoro-3-(1,1,2-trifluoroethyl)phenyl]ethyl]carbamate (346 mg, 1.08 mmol) in HCl/dioxane (4N, 2 mL) and dioxane (2 mL) was degassed and purged with nitrogen for 3 times. Thereafter, the mixture was stirred at 20 °C for 1 h under nitrogen atmosphere. The reaction mixture was concentrated under reduced pressure to give Intermediate **A-23** (248 mg, 78.66% yield, 88% purity, HCl) as a white solid. $^1H$ NMR (400 MHz, DMSO-$d_6$) δ 8.66 (br s, 3H), 7.92 (t, *J* = 7.2 Hz, 1H), 7.66 (t, *J* = 6.8 Hz, 1H), 7.53 - 7.43 (m, 1H), 5.11 - 4.89 (m, 2H), 4.68 (q, *J* = 6.8 Hz, 1H), 1.55 (d, *J* = 6.8 Hz, 3H); MS (ESI) m/z = 221.9 $[M+H]^+$.

**Preparation Example 23: 1-methyl-5-(4,4,5,5-tetramethyl-1,3,2-dioxaborolan-2-yl)triazole (T-8)**

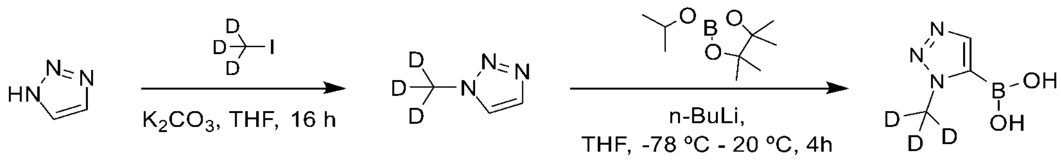


T-1 T-7 T-8

Step 1: Synthesis of 1-(trideuteriomethyl)triazole

To a solution of 1H-triazole (1.99 g, 28.74 mmol, 1.67 mL) in THF (25 mL) was added $K_2CO_3$ (7.95 g, 57.49 mmol) and trideuterio(iodo)methane (5 g, 34.49 mmol, 2.15 mL), and then the mixture was stirred at 25 °C for 12 h. The reaction mixture was poured into water (20 mL) and extracted with EtOAc (30 mL x 3). The combined organic layers were washed with brine (30 mL x 2), dried over $Na_2SO_4$ and concentrated under reduced pressure to give a crude product **Intermediate T-7** (1.18 g, 47.56% yield) as yellow oil. $^1$H NMR (400 MHz, DMSO-$d_6$) $\delta$ 8.06 (s, 1H), 7.70 (s, 1H).

Step 2: Synthesis of [3-(trideuteriomethyl)triazol-4-yl]boronic acid

To a solution of Intermediate T-7 (600 mg, 6.97 mmol) in THF (10 mL) was slowly added n-BuLi (2.5 M, 3.07 mL) at 0 °C under nitrogen atmosphere. The reaction mixture was stirred at -20 °C for 1 h and then cooled to -78 °C. To the reaction mixture was slowly added 2-isopropoxy-4,4,5,5-tetramethyl-1,3,2-dioxaborolane (1.56 g, 8.36 mmol, 1.71 mL) at -78 °C. After 15 min, the reaction mixture was warmed to 0 °C for 1 h. The reaction mixture was quenched with aq. ammonium salt. The reaction mixture was concentrated under reduced pressure to give a crude product **Intermediate T-8** (2.2 g) as a white solid. MS (ESI) m/z = 131.2 [M+H]$^+$.

**Preparation Example 24: 1-methyl-5-(4,4,5,5-tetramethyl-1,3,2-dioxaborolan-2-yl)triazole (T-5)**

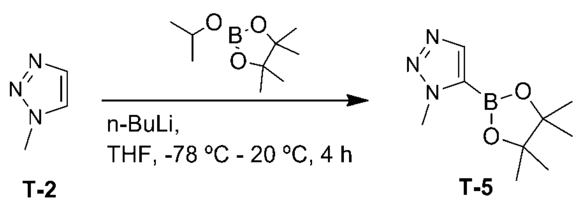


T-2 T-5

To a solution of 1-methyltriazole (2 g, 24.07 mmol) in THF (20 mL) was slowly added n-BuLi (2.5 M, 10.59 mL) at 0 °C under nitrogen atmosphere. The reaction mixture was stirred at -20 °C for 1 h and then cooled to -78 °C. To the reaction mixture was slowly added 2-isoprofoxy-4,4,5,5-tetramethyl-1,3,2-dioxaborolane (15.37 g, 28.88 mmol, 5.89 mL) at -78 °C. After 15 min, the reaction mixture was warmed to 0°C for 1 h. The reaction mixture was quenched with aq. ammonium salt (100 mg, 6 mL). The reaction mixture was concentrated under reduced pressure to give a crude product **Intermediate T-5** (7.9 g) as a light yellow solid, which was used in the next reaction without further purification. MS (ESI) m/z = 210.2 [M+H]$^+$.

**Preparation Example 25: tributyl-(3-methyltriazol-4-yl)stannane (T-3)**

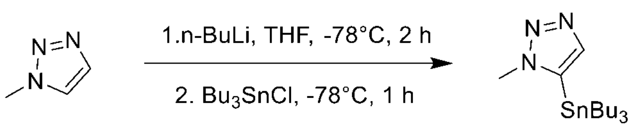


T-2 T-3

A solution of 1-methyltriazole (3 g, 36.10 mmol) in THF (30 mL) was cooled to -78 °C, and then n-BuLi (2.5 M, 16.17 mL) was slowly added and the reaction mixture was stirred at - 78 °C for 2 h. Thereafter, tributyl(chloro)stannane (16.94 g, 52.04 mmol, 14mL) was slowly added and the reaction mixture was stirred at -78 °C for 1 under nitrogen atmosphere. The reaction mixture was quenched with water added slowly at 0 °C, and extracted with EtOAc (70 mL×3). The combined organic layers were washed with brine (50 mL x 2), dried over $Na_2SO_4$ and concentrated under reduced pressure to give a crude product **Intermediate T-3** (17.35 g, 34.97 mmol, 96.85% yield, 75% purity) as light yellow oil. $^1H$ NMR (400 MHz, DMSO-$d_6$) δ 7.58 (br s, 1H), 4.06 - 4.00 (m, 3H), 1.50 - 1.44 (m, 4H), 1.28 (br d, $J$ = 7.2 Hz, 8H), 1.19 - 1.12 (m, 6H), 0.87 - 0.82 (m, 9H).

**Preparation Example 26: tributyl-[3-(trideuteriomethyl)triazol-4-yl]stannane (T-9)**

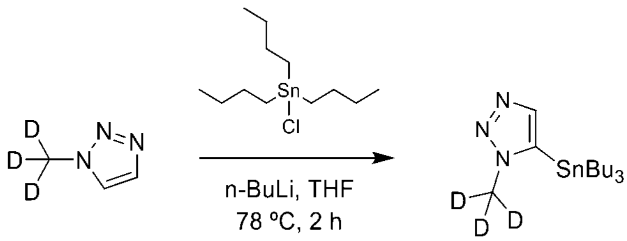


T-7 T-9

A solution of 1-(trideuteriomethyl)triazole (1 g, 11.61 mmol) in THF (20 mL) was cooled to -78 °C, and then n-BuLi (2.5 M, 5.20 mL) was slowly added, and the reaction mixture was stirred at -78 °C for 1 h. Thereafter, tributyl(chloro) stannane (5.22 g, 16.03 mmol, 4.31 mL) was slowly added, and the reaction mixture was stirred at -78 °C for 1 h under nitrogen atmosphere. The reaction mixture was quenched by slowly adding aq. $NH_4Cl$ (20 mL) dropwise at 0 °C, and extracted with EtOAc (40 mL×3). The combined organic layers were washed with brine (20 mL x 2), dried over $Na_2SO_4$ and concentrated under reduced pressure to give a crude product **Intermediate T-9** (4 g, 10.66 mmol) as yellow oil. It was used in the next reaction without further purification. $^1H$ NMR (400 MHz, DMSO-$d_6$) δ 7.60 (s, 1H), 1.49 (m, 4H), 1.35 - 1.21 (m, 8H), 1.19 - 1.09 (m, 6H), 0.88 - 0.83 (m, 9H).

**Preparation Example 27: tributyl-(3-ethyltriazol-4-yl)stannane (T-11)**

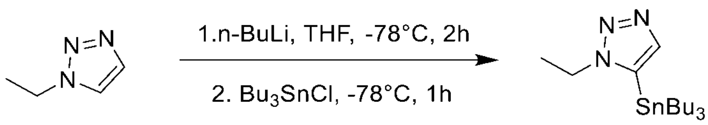


T-10 T-11

A solution of 1-ethyltriazole (500 mg, 5.15 mmol) in THF (10 mL) was cooled to - 78 °C, and then n-BuLi (2.5 M, 2.31 mL) was slowly added and the reaction mixture was stirred at -78 °C for 2 h. Thereafter, tributyl(chloro)stannane (2.14 g, 6.57 mmol, 1.77 mL) was slowly added and the reaction mixture was stirred at -78 °C for 1 h under nitrogen atmosphere. The reaction mixture was quenched with aq. ammonium salt (20 mL) added slowly at 0 °C, and extracted with EtOAc (20 mL×3). The combined organic layers were washed with brine (10 mL x 2), dried over $Na_2SO_4$ and concentrated under reduced pressure to give **Intermediate T-11** (2.4 g, 90.54% yield, 75% purity) as light yellow oil. $^1H$ NMR (400 MHz, DMSO-$d_6$) δ 7.58 (s, 1H), 4.33 (q, $J$ = 7.2 Hz, 2H), 1.37-1.21 (m, 12H), 1.20-1.13 (m, 6H), 0.86 (d, $J$ = 8.0 Hz, 12H).

**Preparation Example 27-1: tributyl-(3-cyclopropyl-4-yl)stannane (T-14)**

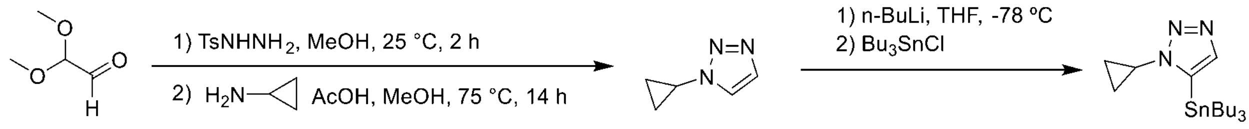


**T-14**

Step 1: Synthesis of 1-cyclopropyltriazole

To a solution of 4-methylbenzenesulfonohydrazide (5 g, 26.85 mmol) in methanol (50 mL) was added 2,2-dimethoxyacetaldehyde (4.89 g, 28.19 mmol, 4.25 mL). The reaction mixture was stirred at 25 °C for 2 h. Thereafter, acetic acid (1.61 g, 26.85 mmol, 1.54 mL) and cyclopropaneamine (1.53 g, 26.85 mmol, 1.86 mL) were added to the reaction mixture, which was then stirred at 75 °C for 14 h. The reaction mixture was concentrated under reduced pressure to give a residue. The residue was purified by silica gel column chromatography (10% EtOAc in petroleum ether) to give 1-cyclopropyltriazole (6 g, 49.15% yield, 90% purity) as yellow oil. $^1$H NMR (400 MHz, DMSO-$d_6$) δ 8.13 (s, 1H), 7.69 (d, *J* = 0.8 Hz, 1H), 4.02 - 3.92 (m, 1H), 1.15 - 1.08 (m, 4H).

Step 2: Synthesis of tributyl-(3-cyclopropyltriazol-4-yl)stannane

A solution of 1-cyclopropyltriazole (200 mg, 1.83 mmol) in THF (4 mL) was cooled to -78 °C, and then n-BuLi (2.5 M, 1.10 mL) was slowly added and the reaction mixture was stirred at -78 °C for 2 h. Thereafter, tributyl(chloro)stannane (1.14 g, 3.50 mmol, 942.15 μL) was slowly added to the reaction mixture, which was then stirred at -78 °C for 1 h under nitrogen atmosphere. The reaction mixture was quenched with aq. ammonium salt (10 mL) added slowly at 0 °C, and extracted with EtOAc (15 mL×3). The combined organic layers were washed with brine (10 mL x 2), dried over $Na_2SO_4$ and concentrated under reduced pressure to give **Intermediate T-14** (1 g, 95.93% yield, 70% purity) as white oil. $^1$H NMR (400 MHz, DMSO-$d_6$) δ 7.59 (s, 1H), 3.71 (tt, *J* = 3.6, 7.2 Hz, 1H), 1.53 - 1.46 (m, 6H), 1.29 - 1.27 (m, 6H), 1.10 - 1.05 (m, 10H), 0.87 (s, 9H).

**Preparation Example 27-2: tributyl-(3-isopropyltriazol-4-yl)stannane (T-15)**

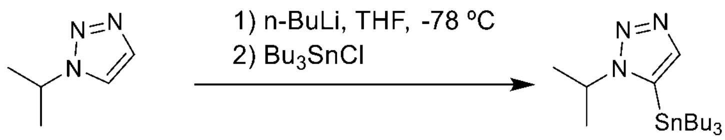


**T-15**

Commercially available 2,2-dimethoxyacetaldehyde and benzyloxyethanamine were used in the same manner as in Step 2 of Preparation Example 27-1 to give **Intermediate T-15** (1 g, 97.21% yield, 70% purity) as white oil. $^1$H NMR (400 MHz, DMSO-$d_6$) δ 7.55 (s, 1H), 4.52 (td, *J* = 6.8, 13.2 Hz, 1H), 1.48 (s, 12H), 1.29 (br d, *J* = 7.6 Hz, 6H), 1.14 - 1.09 (m, 6H), 0.86 (d, *J* = 7.6 Hz, 9H).

**Preparation Example 27-3: [3-(2-benzyloxyethyl)triazol-4-yl]-tributyl-stannane (T-16)**

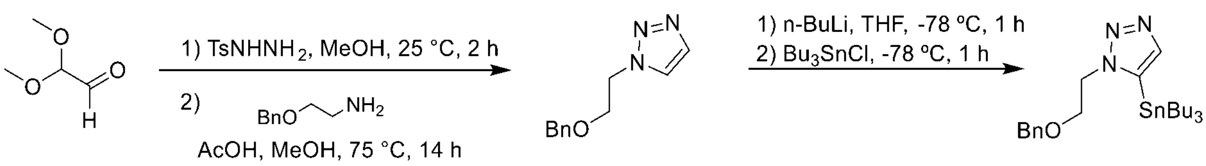


**T-16**

Commercially available 2,2-dimethoxyacetaldehyde and benzyloxyethanamine were used in the same manner as in Preparation Example 27-1 to give **Intermediate T-16** (720 mg, crude product) as yellow oil. It was used in the next reaction without further purification. $^1$H NMR (400 MHz, DMSO-$d_6$) δ 7.82 (s, 1H), 7.40 - 7.37 (m, 3H), 7.33 (br d, *J* = 6.0 Hz,

2H), 4.71 (t, $J$ = 5.2 Hz, 2H), 4.58 (s, 2H), 3.94 (t, $J$ = 5.2 Hz, 2H), 1.59 - 1.55 (m, 6H), 1.37 (m, 6H), 1.19 (m, 6H), 0.96 - 0.95 (m, 6H).

**Preparation Example 27-4: tert-butyl 3-(5-(tributylstanyl)-1H-1,2,3-triazol-1-yl)azetidine-1-carboxylate(T-17)**

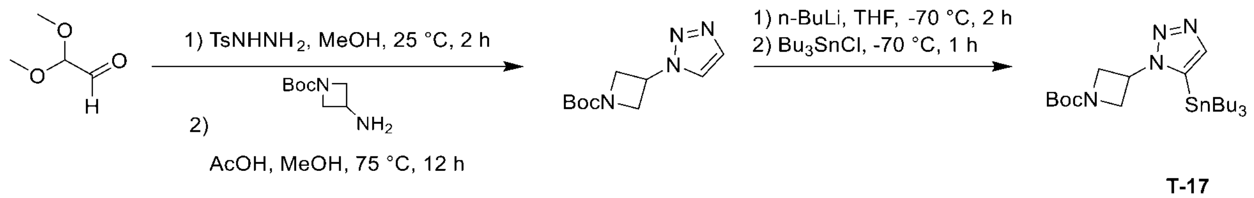


**T-17**

Commercially available 2,2-dimethoxyacetaldehyde and tert-butyl 3-aminoazetidine-1-carboxylate were used in the same manner as in Preparation Example 27-1 to give **Intermediate T-17** (720 mg, crude product) as yellow oil. It was used in the next reaction without further purification. $^1$H NMR (400 MHz, DMSO-$d_6$) δ 8.36 (d, $J$ = 1.2 Hz, 1H), 7.80 (d, $J$ = 0.8 Hz, 1H), 5.50 (tt, $J$ = 5.2, 8.0 Hz, 1H), 4.39 (br t, $J$ = 8.6 Hz, 2H), 4.17 (br s, 2H), 1.41 (s, 9H).

**Preparation Example 27-5: 1-(oxetan-3-yl)-5-(tributylstanyl)-1H-1,2,3-triazole (T-18)**

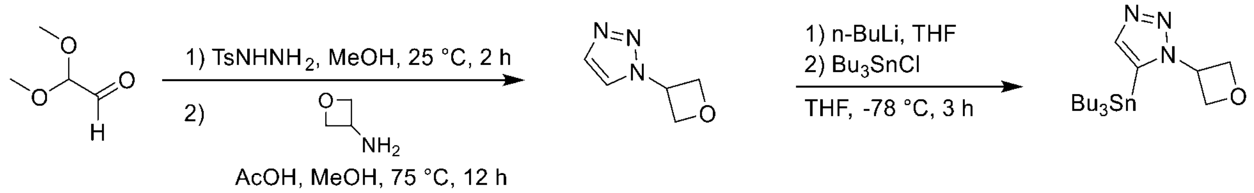


**T-18**

Commercially available 2,2-dimethoxyacetaldehyde and 3-aminooxetane were used in the same manner as in Preparation Example 27-1 to give **Intermediate T-18** (600 mg, 1.45 mmol, 90.64% yield) as yellow oil. It was used in the next reaction without further purification. $^1$H NMR (400 MHz, DMSO-$d_6$) δ 7.67 (s, 1H), 5.64 - 5.53 (m, 1H), 5.00 (d, $J$ = 6.8 Hz, 4H), 1.48 - 1.42 (m, 6H), 1.28 (br d, $J$ = 7.6 Hz, 6H), 1.17 - 1.11 (m, 6H), 0.87 - 0.84 (m, 9H).

**Preparation Example 27-6: tributyl-(3-cyclobutyltriazol-4-yl)stannane (T-19)**

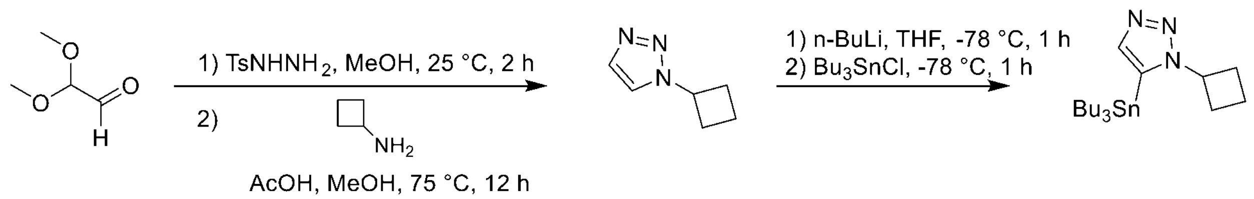


**T-19**

Commercially available 2,2-dimethoxyacetaldehyde and cyclobutaneamine were used in the same manner as in Preparation Example 27-1 to give **Intermediate T-19** (1.06 g, 79.18% yield) as yellow oil. It was used in the next reaction without further purification. $^1$H NMR (400 MHz, DMSO-$d_6$) δ 7.58 (s, 1H), 4.81 (quin, $J$ = 8.2 Hz, 1H), 2.74 - 2.58 (m, 2H), 2.47 - 2.40 (m, 2H), 1.91 (td, $J$ = 4.8, 10.0 Hz, 2H), 1.50 - 1.42 (m, 7H), 1.30 - 1.26 (m, 6H), 1.18 - 1.13 (m, 6H), 0.87 - 0.84 (m, 9H).

**Preparation Example 27-7: 1-(2,2-difluorocyclopropyl)triazole (T-20)**

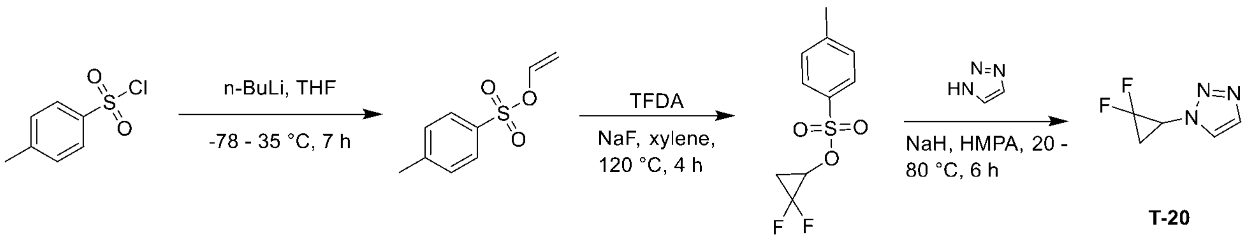


**T-20**

Step 1: Synthesis of vinyl 4-methylbenzenesulfonate

n-BuLi (2.5 M in hexane, 18.46 mL) was added under nitrogen atmosphere in anhydrous THF (70 mL) and the mixture was stirred at 35 °C for 4 h. After being cooled to - 78 °C, a solution of 4-methylbenzenesulfonyl chloride (8 g, 41.96 mmol) in anhydrous THF (20 mL) was added dropwise for 0.5 h. The resulting mixture was stirred at -78 °C for 1 h, heated to 25 °C, and then stirred for 1 h. The reaction mixture was poured into $H_2O$ (50 mL) and extracted with EtOAc (50 mL × 3). The combined organic layers were washed with brine (50 mL × 2), dried over anhydrous $Na_2SO_4$ and concentrated under reduced pressure to give a crude product. The crude product was purified by silica gel column chromatography (5% EtOAc in petroleum ether) to give vinyl 4-methylbenzenesulfonate (4.3 g, 51.69% yield) as transparent oil. $^1H$ NMR (400 MHz, DMSO-$d_6$) δ 7.82 (d, *J* = 8.4 Hz, 2H), 7.51 (d, *J* = 8.0 Hz, 2H), 6.73 (dd, *J* = 5.6, 13.2 Hz, 1H), 4.94 (dd, *J* = 2.4, 13.2 Hz, 1H), 4.82 (dd, *J* = 2.4, 5.6 Hz, 1H), 2.43 (s, 3H).

Step 2: Synthesis of (2,2-difluorocyclopropyl)4-methylbenzenesulfonate

To a mixture of vinyl 4-methylbenzenesulfonate (3.5 g, 7.66 mmol) and NaF (74.13 μL, 1.77 mmol) in o-xylene (2 mL) was added trimethylsilyl-2,2-difluoro-2-fluorosulfonyl-acetate (3.5 g, 17.66 mmol) dropwise at 120 °C for 2 h. The mixture was stirred at 120 °C for 2 h. The reaction mixture was concentrated under reduced pressure to give a crude product. The crude product was purified by silica gel column chromatography (5% EtOAc in petroleum ether) to give (2,2-difluorocyclopropyl)4-methylbenzenesulfonate (3.77 g, 82.49% yield, 95.9% purity) as a yellow solid. $^1H$ NMR (400 MHz, DMSO-*$d_6$*) δ 7.85 (d, *J* = 8.4 Hz, 2H), 7.53 (d, *J* = 8.0 Hz, 2H), 4.63 (ddt, *J* = 2.4, 4.8, 9.2 Hz, 1H), 2.44 (s, 3H), 2.11 - 1.93 (m, 1H), 1.83 (tdd, J = 5.2, 10.4, 16.8 Hz, 1H).

Step 3: Synthesis of 1-(2,2-difluorocyclopropyl)triazole

To a solution of 1H-triazole (1.63 mL, 28.20 mmol) in DMF (40 mL) was added NaH (1.13 g, 28.20 mmol, 60% purity) under nitrogen atmosphere for 3 times. The reaction mixture was stirred at 20 °C for 1 h under nitrogen atmosphere. Afterward, (2,2-difluorocyclopropyl)4-methylbenzenesulfonate (3.5 g, 14.10 mmol) in DMF (10 mL) was added dropwise for 15 min, and the mixture was stirred at 80 °C for 5 h under nitrogen conoditions. The mixture was quenched with $H_2O$ (30 mL) at 20 °C, and then extracted with EtOAc (50 mL × 4). The combined organic layers were washed with brine (20 mL × 3), dried over anhydrous $Na_2SO_4$, filtered and concentrated under reduced pressure to give a crude product. The crude product was purified by silica gel column chromatography (50% DCM in PE) to give **Intermediate T-20** (400 mg, 16.8% yield, 86.02% purity) as yellow oil. MS (ESI) m/z = 146.1 $[M+H]^+$.

**Preparation Example 27-8: tert-butyl 4-(5-tributylstanyltriazol-1-yl)piperidine-1-carboxylate (T-21)**

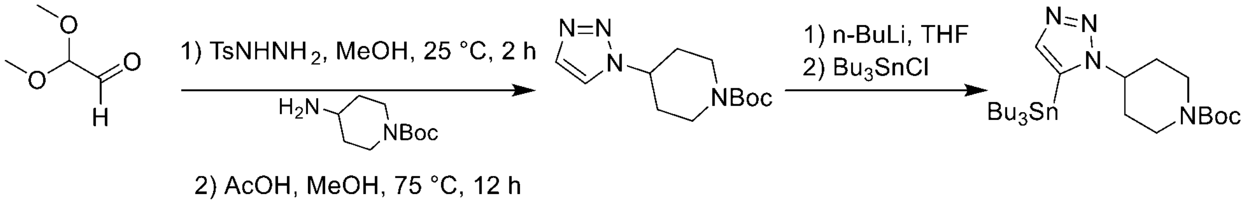


**T-21**

Commercially available 2,2-dimethoxyacetaldehyde and 4-methylbenzenesulfonohydrazide were used in the same manner as in Preparation Example 27-1 to give **Intermediate T-21** (3 g, 95.08% yield, 85% purity) as yellow oil. It was used in the next reaction without further purification. $^1H$ NMR (400 MHz, DMSO-*$d_6$*) δ 7.58 (s, 1H), 4.34 (br t, *J* = 10.8 Hz, 1H), 4.14 - 4.04 (m, 2H), 2.94 (br s, 2H), 1.98 (s, 2H), 1.94 - 1.87 (m, 2H), 1.53 - 1.45 (m, 6H), 1.42 (s, 9H), 1.31 - 1.26 (m,

6H), 1.20 - 1.15 (m, 6H), 0.87 - 0.83 (m, 9H).

**Preparation Example 27-9: tert-butyl (S)-3-(5-(tributylstanyl)-1H-1,2,3-triazol-1-yl)pyrrolidine-1-carboxylate (T-22)**

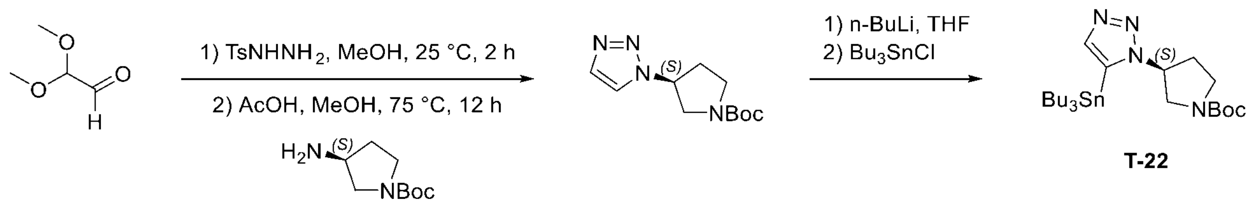


**T-22**

Commercially available 2,2-dimethoxyacetaldehyde and tert-butyl (3S)-3-aminopyrrolidine-1-carboxylate were used in the same manner as in Preparation Example 27-1 to give **Intermediate T-22** (890 mg, 12.35% yield, 71% purity) as yellow oil. It was used in the next reaction without further purification. $^1$H NMR (400 MHz, $CDCl_3$) δ 7.73 (s, 1H), 7.59 (s, 1H), 5.21 (br s, 1H), 3.91 (dd, *J* = 6.4, 12.0 Hz, 1H), 3.82 - 3.72 (m, 1H), 3.59 (br s, 2H), 2.53 - 2.40 (m, 2H), 1.46 (s, 9H); MS (ESI) m/z = 239.1 $[M+H]^+$.

**Preparation Example 28: 1-[5-(3-methyltriazol-4-yl)-3-pyridyl]-6-oxo-pyridazine-3-carboxylic acid (4-1)**

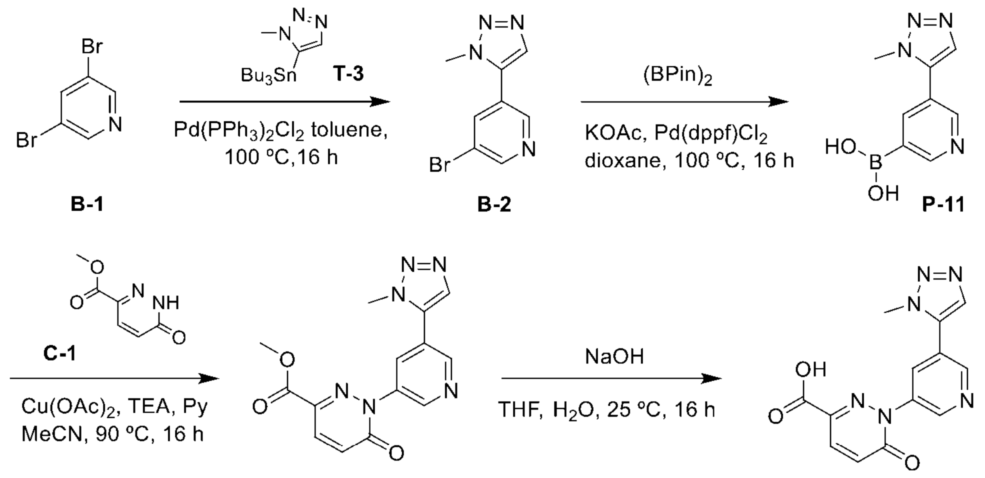


B-1, B-2, P-11, 3-1, 4-1

Step 1: Synthesis of 3-bromo-5-(3-methyltriazol-4-yl)pyridine

A solution of 3,5-dibromopyridine (10.92 g, 46.10 mmol), tributyl-(3-methyltriazol-4-yl)stannane (22.3 g, 59.92 mmol) and $Pd(PPh_3)_2Cl_2$ (3.24 g, 4.61 mmol) in toluene (200 mL) were substituted with nitrogen for 3 times, and then the mixture was stirred at 90 °C for 16 h under nitrogen atmosphere. The reaction mixture was poured into water (150 mL) and extracted with EtOAc (200 mL x 3). The combined organic layers were washed with brine (200 mL x 3), dried over $Na_2SO_4$ and concentrated under reduced pressure to give a crude product. The crude product was purified by silica gel column chromatography (80% EA in petroleum ether) to give 3-bromo-5-(3-methyltriazol-4-yl)pyridine (4.4 g, 39.93% yield) as a yellow solid. $^1$H NMR (400 MHz, DMSO-$d_6$) δ 8.83-8.83 (m, 1H), 8.40 (t, *J* = 2.0 Hz, 1H), 8.09 (s, 1H), 7.66-7.49 (m, 1H), 4.13 (s, 3H); MS (ESI) m/z = 153.0 $[M+H]^+$.

Step 2: Synthesis of [5-(3-methyltriazol-4-yl)-3-pyridyl]boronic acid

A solution of 3-bromo-5-(3-methyltriazol-4-yl)pyridine (6.76 g, 28.28 mmol), 4,4,5,5-tetramethyl-2-(4,4,5,5-tetramethyl-1,3,2-dioxoborolan-2-yl)-1,3,2-dioxoborolan (14.36 g, 56.55 mmol), $Pd(dppf)Cl_2$ (2.31 g, 2.83 mmol) and KOAc (5.55 g, 56.55 mmol) in dioxane (120 mL) was substituted with nitrogen for 3 times, and then the mixture was stirred

at 100 °C for 16 h under nitrogen atmosphere. The reaction mixture was filtered and concentrated under reduced pressure to give a crude product. The crude product was purified by silica gel column chromatography (3% MeOH in DCM) to give [5-(3-methyltriazol-4-yl)-3-pyridyl]boronic acid **(Intermediate P-11,** 6.76 g, 78.52% yield, 67% purity) as a dark brown solid. MS (ESI) m/z = 205.1 [M+H]+.

Step 3: Synthesis of methyl-1-[5-(3-methyltriazol-4-yl)-3-pyridyl]-6-oxo-pyridazine-3-carboxylate

A solution of methyl-6-oxo-1H-pyridazine-3-carboxylate (3.78 g, 24.51 mmol), [5-(3-methyltriazol-4-yl)-3-pyridyl]boronic acid (5 g, 24.51 mmol), $Cu(OAc)_2$ (4.45 g, 24.51 mmol), TEA (4.96 g, 49.02 mmol, 6.82 mL) and pyridine (3.88 g, 49.02 mmol, 3.96 mL) in MeCN (100 mL) was substituted with oxygen for 3 times, and then the mixture was stirred at 90 °C for 16 h under oxygen atmosphere. The reaction mixture was filtered and concentrated under reduced pressure to give a crude product. The crude product was purified by silica gel column chromatography (3% MeOH in DCM) to give methyl-1-[5-(3-methyltriazol-4-yl)-3-pyridyl]-6-oxo-pyridazine-3-carboxylate (1.83 g, 5.27 mmol, 21.51% yield, 89.98% purity) as a dark brwon solid. MS (ESI) m/z = 312.9 [M+H]+.

Step 4: Synthesis of 1-[5-(3-methyltriazol-4-yl)-3-pyridyl]-6-oxo-pyridazine-3-carboxylic acid

To a solution of methyl-1-[5-(3-methyltriazol-4-yl)-3-pyridyl]-6-oxo-pyridazine-3-carboxylate (1.0 g, 3.20 mmol) in THF (4 mL), $H_2O$ (2 mL) was added NaOH (256.16 mg, 6.40 mmol). The mixture was substituted with nitrogen for 3 times, and then stirred at 25 °C for 16 h under nitrogen atmosphere. The mixture was adjusted to pH = 2-3 with aq. 1 N HCl and then **Intermediate 4-1** (725 mg, 74.01% yield, 97.5% purity) was obtained by solid filtration as a yellow solid. MS (ESI) m/z = 299.0 [M+H]+.

**Preparation Example 28-1: 1-[5-(3-ethyltriazol-4-yl)-3-pyridyl]-5-methyl-6-oxo-pyridazine-3-carboxylic acid (4-9)**

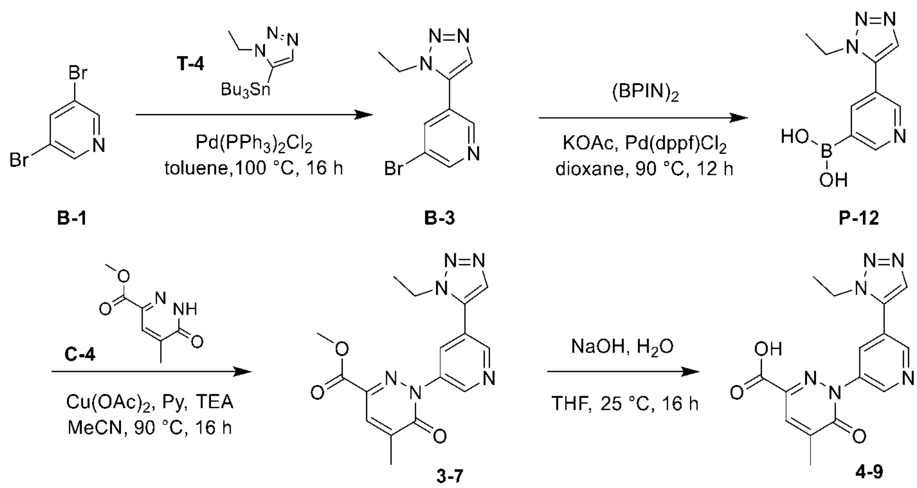


3,5-dibromopyridine and Intermediate T-4 were used in the same manner as in Preparation Example 28 to give **Intermediate 4-9** (86 mg, crude product) as a light yellow solid. MS (ESI) m/z = 327.1 [M+H]+.

**Preparation Example 28-2: (2-fluoro-5-(1-methyl-1H-1,2,3-triazol-5-yl)pyridin-3-yl)boronic acid (P-20)**

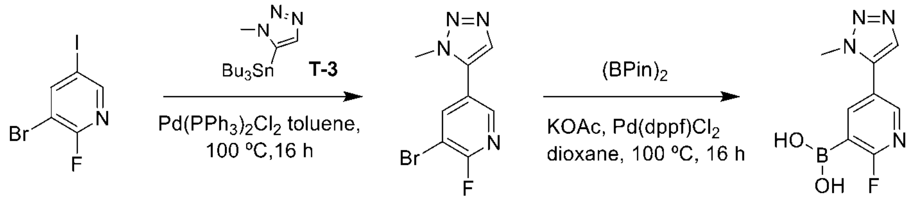


B-11 P-20

3-bromo-2-fluoro-5-iodopyridine and Intermediate T-3 were used in the same manner as in Steps 1 and 2 of Preparation Example 28 to give **Intermediate P-20** as a light yellow solid. MS (ESI) m/z = 223.1 $[M+H]^+$.

**Preparation Example 28-3: (2-fluoro-5-(1-trideuteriomethyltriazol-5-yl)pyridin-3-yl]boronic acid (P-21)**

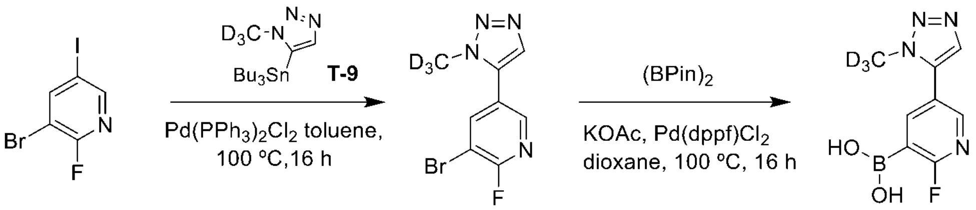


B-11 P-21

3-bromo-2-fluoro-5-iodopyridine and Intermediate T-9 were used in the same manner as in Step 1 and Step 2 of Preparation Example 28 to give **Intermediate P-21** as a light yellow solid. MS (ESI) m/z = 226.1 $[M+H]^+$.

**Preparation Example 28-4: (2-fluoro-5-(1-cyclopropyltriazol-5-yl)pyridin-3-yl]boronic acid (P-22)**

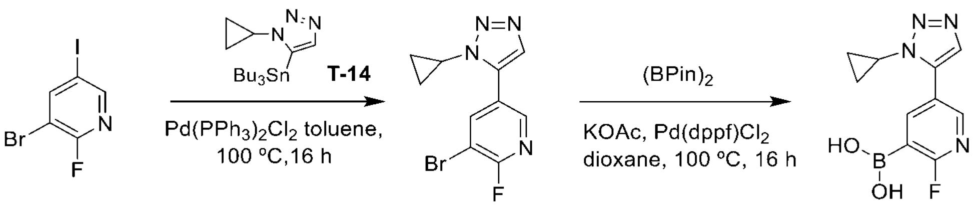


B-11 P-22

3-bromo-2-fluoro-5-iodopyridine and Intermediate T-14 were used in the same manner as in Step 1 and Step 2 of Preparation Example 28 to give Intermediate P-22 as a light yellow solid. MS (ESI) m/z = 249.1 $[M+H]^+$.

**Preparation Example 29: methyl-1-[5-(3-methyltriazol-4-yl)-3-pyridyl]-6-oxo-pyridazine-3-carboxylate (3-1): Alternative method**

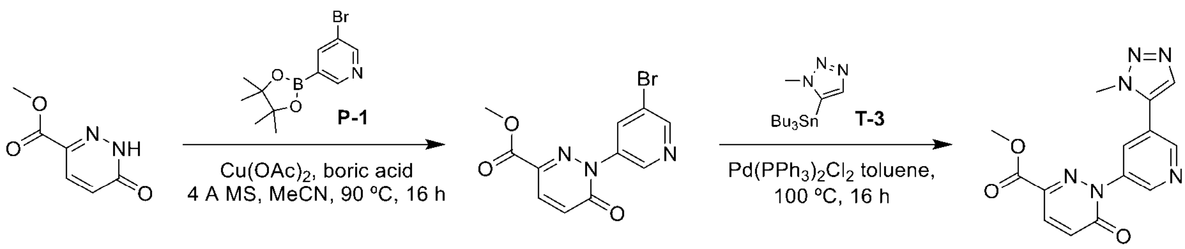


C-1 2-1 3-1

Step 1: Synthesis of methyl 1-(5-bromo-3-pyridyl)-6-oxo-pyridazine-3-carboxylate

A solution of methyl 6-oxo-1H-pyridazine-3-carboxylate (5 g, 32.44 mmol), 3-bromo-5-(4,4,5,5-tetramethyl-1,3,2-dioxaborolan-2-yl)pyridine (9.21 g, 32.44 mmol), $Cu(OAc)_2$ (2.95 g, 16.22 mmol), boric acid (4.01 g, 64.88 mmol) and 4A MS (1 g) in MeCN (120 mL) was substituted with oxygen for 3 times, and then stirred at 90 °C for 16 h under oxygen atmosphere. The reaction mixture was poured into water (30 mL) and extracted with EtOAc (30 mL x 3). The combined organic layers were washed with brine (50 mL), dried over $Na_2SO_4$ and concentrated under reduced pressure to give a crude product. The crude product was purified by silica gel column chromatography (58% DCM in petroleum ether) to give methyl 1-(5-bromo-3-pyridyl)-6-oxo-pyridazine-3-carboxylate (2.2 g, 21.54% yield, 98.49% purity) as a white solid. MS (ESI) m/z = 312.0 $[M+H]^+$.

Step 2: Synthesis of methyl-1-[5-(3-methyltriazol-4-yl)-3-pyridyl]-6-oxo-pyridazine-3-carboxylate

A solution of methyl-1-(5-bromo-3-pyridyl)-6-oxo-pyridazine-3-carboxylate (2.2 g, 7.09 mmol), tributyl-(3-methyltriazol-4-yl)stannane (7.92 g, 21.28 mmol) and $Pd(PPh_3)_2Cl_2$ (497.95 mg, 709.44 μmol) in toluene (20 mL) was substituted with nitrogen for 3 times, and then stirred at 100 °C for 16 h under nitrogen atmosphere. The reaction mixture was filtered, and then the filtrate was extracted with EtOAc (50 mL x 3) after the addition of water (20mL). The combined organic layers were washed with brine (20 mL x 2), dried over $Na_2SO_4$ and concentrated under reduced pressure to give a crude product. The crude product was purified by silica gel column chromatography (2% MeOH in DCM) to give **Intermediate 3-1** (1.0 g, 32.50% yield, 72% purity) as a white solid. MS (ESI) m/z = 327.1 $[M+H]^+$.

**Preparation Example 30: 6-oxo-1-[5-[3-(trideuteriomethyl)triazol-4-yl]-3-pyridyl]pyridazine-3-carboxylic acid (4-2)**

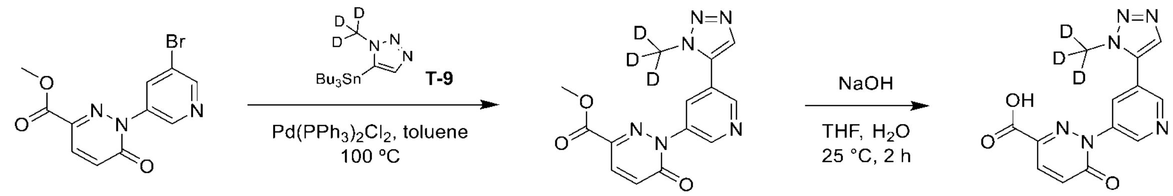


**2-1** **3-2** **4-2**

Step 1: Synthesis of methyl 6-oxo-1-[5-[3-(trideuteriomethyl)triazol-4-yl]-3-pyridyl]pyridazine-3-carboxylate

A solution of 1-(5-bromo-3-pyridyl)-6-oxo-pyridazine-3-carboxylate (1 g, 3.22 mmol), tributyl-[3-(trideuteriomethyl)triazol-4-yl]stannane (3.63 g, 9.67 mmol) and $Pd(PPh_3)_2Cl_2$ (226.34 mg, 322.47 μmol) in toluene (10 mL) was substituted with nitrogen for 3 times, and stirred at 100°C for 16 h under nitrogen atmosphere. The reaction mixture was poured into water (20mL) and extracted with EtOAc (30 mL x 3). The combined organic layers were washed with brine (20 mL x 2), dried over $Na_2SO_4$ and concentrated under reduced pressure to give a crude product. The crude product was purified by silica gel column chromatography (5% MeOH in DCM) to give methyl 6-oxo-1-[5-[3-(trideuteriomethyl)triazol-4-yl]-3-pyridyl]pyridazine-3-carboxylate (420 mg, 30.57% yield, 74% purity) as a yellow solid. MS (ESI) m/z = 316.1 $[M + H]^+$.

Step 2: Synthesis of 6-oxo-1-[5-[3-(trideuteriomethyl)triazol-4-yl]-3-pyridyl]pyridazine-3-carboxylic acid

To a solution of methyl 6-oxo-1-[5-[3-(trideuteriomethyl)triazol-4-yl]-3-pyridyl]pyridazine-3-carboxylate (420 mg, 1.33 mmol) in THF (8 mL) and $H_2O$ (2 mL) was added NaOH (79.92 mg, 2.00 mmol). After the nitrogen substitution for 3 times, the mixture was stirred at 25 °C for 2 h under nitrogen atmosphere. The mixture was adjusted to pH = 6-7 with aq. 1 N HCl and then concentrated under reduced pressure to give **Intermediate** 4-2 (420 mg, 65.93% yield, 63% purity) as a yellow solid. MS (ESI) m/z = 302.1 $[M + H]^+$.

**Preparation Example 31: 1-[5-(3-ethyltriazol-4-yl)-3-pyridyl]-6-oxo-pyridazine-3-carboxylic acid (4-3)**

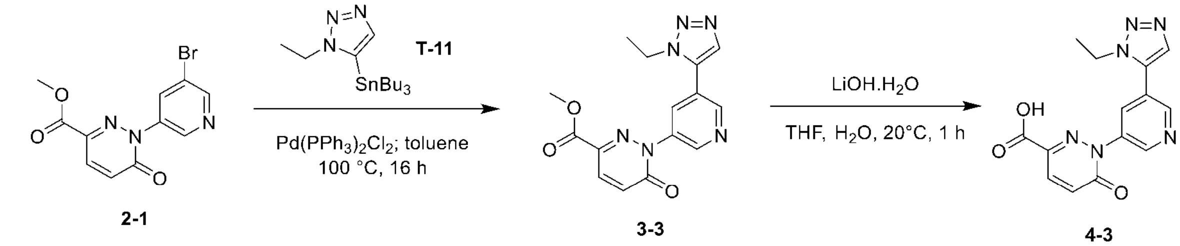


2-1 3-3 4-3

Step 1: Synthesis of 1-[5-(3-ethyltriazol-4-yl)-3-pyridyl]-6-oxo-pyridazine-3-carboxylate

A solution of 1-(5-bromo-3-pyridyl)-6-oxo-pyridazine-3-carboxylate (260 mg, 838.43 μmol), tributyl-(3-ethyltriazol-4-yl)stannane (485.66 mg, 1.26 mmol) and $Pd(PPh_3)_2Cl_2$ (58.85 mg, 83.84 μmol) in toluene (5 mL) was substituted with nitrogen for 3 times, and then stirred at 100 °C for 16 h under nitrogen atmosphere. The reaction mixture was poured into water (30 mL) and extracted with EtOAc (10 mL x 3). The combined organic layers were washed with brine (10 mL x 5), dried over $Na_2SO_4$ and concentrated under reduced pressure to give a crude product. The crude product was purified by silica gel column chromatography (3% MeOH in DCM) to give 1-[5-(3-ethyltriazol-4-yl)-3-pyridyl]-6-oxo-pyridazine-3-carboxylate (110 mg, 13.67% yield, 34% purity) as yellow oil. MS (ESI) m/z = 327.1 $[M + H]^+$.

Step 2: Synthesis of 1-[5-(3-ethyltriazol-4-yl)-3-pyridyl]-6-oxo-pyridazine-3-carboxylic acid

To a solution of 1-[5-(3-ethyltriazol-4-yl)-3-pyridyl]-6-oxo-pyridazine-3-carboxylate (110 mg, 337.10 umol) in THF (3 mL) was added $LiOH \cdot H_2O$ (28.29 mg, 674.21 umol) and $H_2O$ (1 mL), and then the mixture was stirred at 20 °C for 1 h. The reaction mixture was concentrated under reduced pressure to give a crude product. The crude product was purified by prep-HPLC (column: Welch Xtimate C18 150*30 mm*5 μm; mobile phase: [water (HCl)-MeCN]; B%: 0%-26%, 25 min). Most of MeCN was concentrated under reduced pressure, and the remaining solvent was removed by lyophilization to give **Intermediate 4-3** (47 mg, 44.65% yield, 100% purity) as a white solid. MS (ESI) m/z = 313.0 $[M + H]^+$.

**Preparation Example 32: 1-[5-(3-methyltriazol-4-yl)-3-pyridyl]-6-oxo-pyridin-3-carboxylic acid (4-4)**

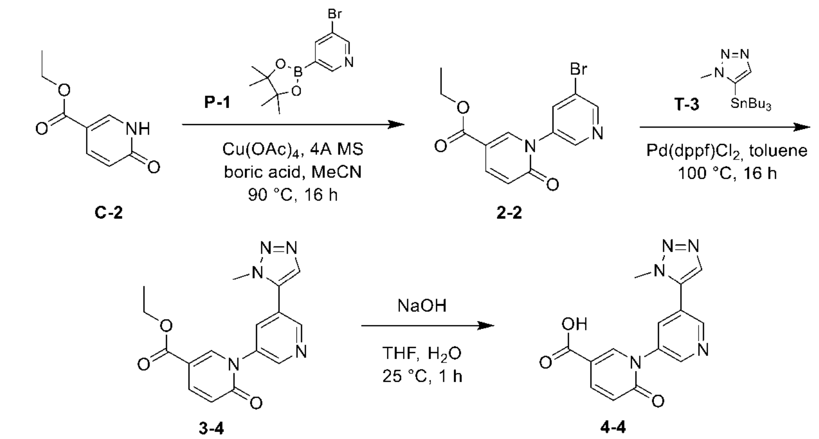


C-2 2-2 3-4 4-4

Step 1: Synthesis of ethyl 1-(5-bromo-3-pyridyl)-6-oxo-pyridine-3-carboxylate

A solution of ethyl 6-oxo-1H-pyridine-3-carboxylate (535.17 mg, 3.20 mmol), 3-bromo-5-(4,4,5,5-tetramethyl-1,3,2-dioxaborolan-2-yl)pyridine (1 g, 3.52 mmol), $Cu(OAc)_2$ (290.75 mg, 1.60 mmol), 4A MS (500 mg, 3.20 mmol) and boric acid (395.92 mg, 6.40 mmol) in MeCN (15 mL) was substituted with oxygen for 3 times, and then stirred at 90 °C for 16 h under oxygen atmosphere. The reaction mixture was filtered, and then the filtrate was concentrated under reduced pressure to give a crude product. The crude product was purified by silica gel column chromatography (30% EtOAc in petroleum ether) to give ethyl 1-(5-bromo-3-pyridyl)-6-oxo-pyridine-3-carboxylate (87 mg, 7.65% yield, 90.95% purity) as a white solid. MS (ESI) m/z = 322.9 $[M + H]^+$.

Step 2: Synthesis of ethyl 1-[5-(3-methyltriazol-4-yl)-3-pyridyl]-6-oxo-pyridine-3-carboxylate

A solution of ethyl-1-(5-bromo-3-pyridyl)-6-oxo-pyridine-3-carboxylate (87 mg, 269.23 μmol), tributyl-(3-methyltriazol-4-yl)stannane (250.48 mg, 673.08 μmol) and Pd(dppf)$Cl_2$ (19.70 mg, 26.92 μmol) in toluene (2 mL) was substituted with nitrogen for 3 times, and then stirred at 100 °C for 16 h under nitrogen atmosphere. The reaction mixture was filtered, and then the filtrate was concentrated under reduced pressure to give a crude product. The crude product was purified by silica gel column chromatography (2% MeOH in DCM) to give ethyl 1-[5-(3-methyltriazol-4-yl)-3-pyridyl]-6-oxo-pyridine-3-carboxylate (30 mg, 32.07% yield, 93.62% purity) as a white solid. MS (ESI) m/z = 326.1 $[M + H]^+$.

Step 3: Synthesis of 1-[5-(3-methyltriazol-4-yl)-3-pyridyl]-6-oxo-pyridine-3-carboxylic acid

To a solution of ethyl 1-[5-(3-methyltriazol-4-yl)-3-pyridyl]-6-oxo-pyridine-3-carboxylate (30 mg, 92.22 μmol) in THF (1 mL) and $H_2O$ (0.5 mL) was added NaOH (7.38 mg, 184.43 μmol). After nitrogen substitution for 3 times, the mixture was stirred at 25 °C for 1 h under nitrogen atmosphere. The mixture was adjusted to pH = 5-6 with aq. 1 N HCl, and then the organic layers were concentrated under reduced pressure to give **Intermediate 4-4** (25 mg, crude) as yellow oil. MS (ESI) m/z = 298.0 $[M + H]^+$.

**Preparation Example 33: 3-methyl-5'-(1-methyl-1H-1,2,3-triazol-5-yl)-2-oxo-2H-[1,3'-bipyridine]-5-carboxylic acid (4-5)**

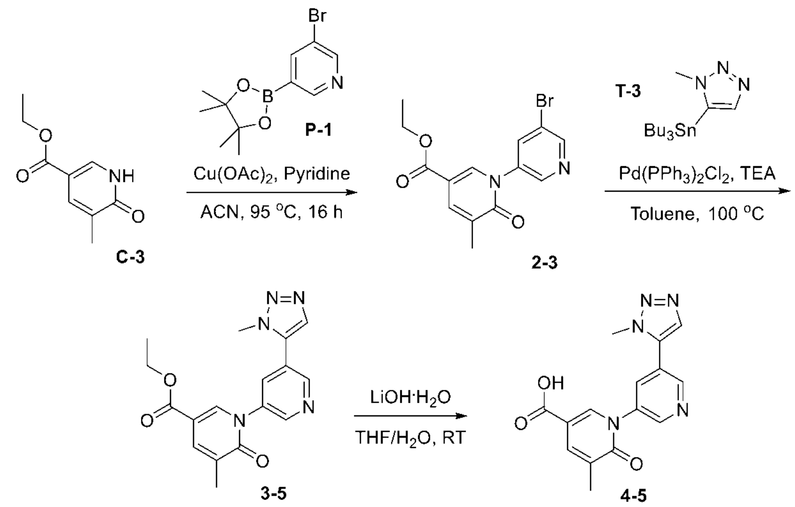


Step 1: Synthesis of ethyl 5'-bromo-3-methyl-2-oxo-2*H*-[1,3'-bipyridine]-5-carboxylate

To a solution of ethyl 5-methyl-6-oxo-1,6-dihydronicotinate (500 mg, 2.76 mmol) in MeCN (21.2 mL) was added 5-bromo-3-pyridineboronic acid pinacol ester (1.18 mg, 4.14 mmol), $Cu(OAc)_2$ (501 mg, 2.76 mmol) and pyridine (0.89 mL, 11 mmol), and then the mixture was stirred at 90 °C for 16 h. Following the addition of water, the reaction mixture was extracted with EtOAc. The combined organic layers were washed with copper sulfate(II) pentahydrate, dried over $Na_2SO_4$ and concentrated under reduced pressure to give a crude product. The crude product was purified by silica gel column chromatography (50% EtOAc in hexane) to give ethyl 5'-bromo-3-methyl-2-oxo-2*H*-[1,3'-bipyridine]-5-carboxylate (460 mg, 49% yield) as a white solid. $^1H$ NMR (400 MHz, $CDCl_3$) δ ppm 8.77 (d, J = 2.0 Hz, 1H), 8.60 (d, J = 2.4 Hz, 1H), 8.06 (d, J = 2.4 Hz, 1H), 8.01 (t, J = 2.0 Hz, 1H), 7.84 (d, J = 0.8 Hz, 1H), 4.34 (q, J = 7.0 Hz, 2H), 2.22 (s, 3H), 1.37 (t, J = 7.2 Hz, 3H); MS (ESI) m/z = 336.95, 338.95 $[M+H]^+$.

Step 2: Syynthesis of ethyl 3-methyl-5'-(1-methyl-1*H*-1,2,3-triazol-5-yl)-2-oxo-2*H*-[1,3'-bipyridine]-5-carboxylate

A solution of ethyl 5'-bromo-3-methyl-2-oxo-2H-[1,3'-bipyridine]-5-carboxylate (300 mg, 0.890 mmol), tributyl(1-methyl-1H-1,2,3-triazol-5-yl)stannane (993 mg, 2.67 mmol), TEA (124 μL, 0.890 mmol) and $Pd(PPh_3)_2Cl_2$ (62.6 mg, 0.089 mmol) in toluene (8.9 mL) was substituted with nitrogen for 3 times, and then stirred at 100 °C for 17h under nitrogen atmosphere. The reaction mixture was poured into water (30 mL) and extracted with EtOAc (10 mL x 3). The combined organic layers were washed with brine (10 mL x 5), dried over $Na_2SO_4$ and concentrated under reduced pressure to give a

crude product. The crude product was purified by silica gel column chromatography (17% EtOAc in hexane) to give ethyl 3-methyl-5'-(1-methyl-1H-1,2,3-triazol-5-yl)-2-oxo-2H-[1,3'-bipyridine]-5-carboxylate (81 mg, 27% yield) as a white solid. $^1H$ NMR (400 MHz, $CDCl_3$) δ 8.79 (d, $J$ = 2.0 Hz, 1H), 8.74 (d, $J$ = 2.4 Hz, 1H), 8.16 (d, $J$ = 2.0 Hz, 1H), 7.97 (t, $J$ = 2.2 Hz, 1H), 7.87-7.86 (m, 2H), 4.36 (q, $J$ = 7.2 Hz, 2H), 4.18 (s, 3H), 2.24 (s, 3H), 1.37 (t, $J$ = 7.2 Hz, 3H); MS (ESI) m/z = 340.07 $[M+H]^+$.

Step 3: Synthesis of 3-methyl-5'-(1-methyl-1H-1,2,3-triazol-5-yl)-2-oxo-2H-[1,3'-bipyridine]-5-carboxylic acid

To a solution of ethyl 3-methyl-5'-(1-methyl-1H-1,2,3-triazol-5-yl)-2-oxo-2H-[1,3'-bipyridine]-5-carboxylate (81 mg, 0.239 mmol) in THF (2.39 mL) was added $LiOH \cdot H_2O$ (30 mg, 0.716 mmol) amd $H_2O$ (1.19 mL), and then the mixture was stirred at 25 °C for 1 h. The reaction mixture was poured into water and adjusted to pH = 1 wtih aq. 1 N HCl, and then **Intermediate 4-5** (73 mg, 98% yield) was obtained by solid filtration as a white solid. MS (ESI) m/z = 312.04 $[M+H]^+$.

**Preparation Example 34: 1-[5-(2-methylpyrazol-3-yl)-3-pyridyl]-6-oxo-pyridazine-3-carboxylic acid (4-6)**

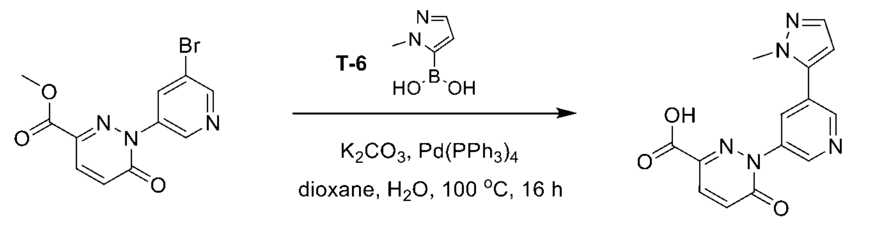


2-1 4-6

A solution of methyl-1-(5-bromo-3-pyridyl)-6-oxo-pyridazine-3-carboxylate (500 mg, 1.61 mmol), (2-methylpyrazol-3-yl)boronic acid (406.06 mg, 3.22 mmol), $K_2CO_3$ (557.09 mg, 4.03 mmol) and $Pd(PPh_3)_4$ (186.32 mg, 161.24 μmol) in dioxane (5 mL) and $H_2O$ (0.5 mL) was substituted with nitrogen for 3 times, and then stirred at 100 °C for 16 h under nitrogen atmosphere. The reaction mixture was concentrated under reduced pressure to remove the solvent, and then diluted with water (10 mL). With the addition of aq. 1 N HCl, the reaction mixture was adjusted to pH = 3-4, and then extracted with EtOAc (20 mL × 3). The organic layers were washed with brine (30 mL), dried over $Na_2SO_4$ and concentrated under reduced pressure to give a crude product, **Intermediate 4-6** (835 mg, crude) as a brown solid. MS (ESI) m/z = 298.1 $[M+H]^+$.

**Preparation Example 35: 5-methyl-1-[5-(3-methyltriazol-4-yl)-3-pyridyl]-6-oxo-pyridazine-3-carboxylic acid (4-7)**

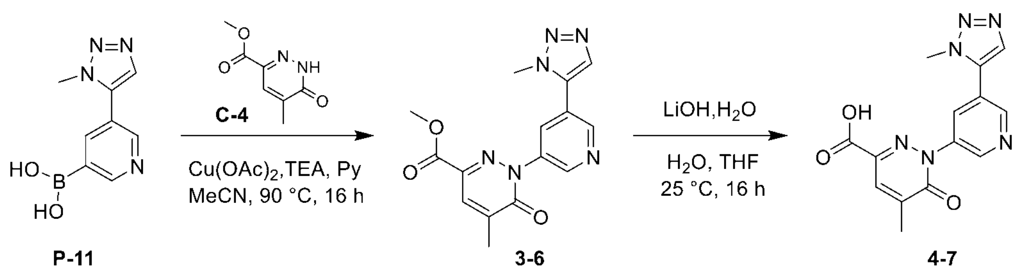


P-11 3-6 4-7

Step 1: Synthesis of methyl 5-methyl-1-[5-(3-methyltriazol-4-yl)-3-pyridyl]-6-oxo-pyridazine-3-carboxylate

A solution of methyl 5-methyl-6-oxo-1H-pyridazine-3-carboxylate (200 mg, 1.19 mmol), [5-(3-methyltriazol-4-yl)-3-pyridyl]boronic acid (242.63 mg, 1.19 mmol), $Cu(OAc)_2$ (216.03 mg, 1.19 mmol), TEA (240.71 mg, 2.38 mmol, 331.11 μL) and pyridine (188.17 mg, 2.38 mmol, 192.01 μL) in MeCN (5 mL) was substituted with nitrogen for 3 times, and then stirred at 90 °C for 16 h under nitrogen atmosphere. After the addition of water (30 mL), the reaction mixture was extracted with EtOAc (30 mL × 3). The organic layers were washed with brine (50 mL), dried over $Na_2SO_4$, and then concentrated under reduced pressure to give a crude product. The crude product was purified by silica gel column chromatography (1% MeOH in DCM) to give methyl 5-methyl-1-[5-(3-methyltriazol-4-yl)-3-pyridyl]-6-oxo-pyridazine-3-carboxylate (305 mg,

69.15% yield, 88% purity) as a yellow solid. MS (ESI) m/z = 327.1 [M+H]+.

Step 2: Synthesis of 5-methyl-1-[5-(3-methyltriazol-4-yl)-3-pyridyl]-6-oxo-pyridazine-3-carboxylic acid

LiOH·$H_2O$ (117.67 mg, 2.80 mmol) and $H_2O$ (2 mL) were added to a solution of methyl 5-methyl-1-[5-(3-methyltriazol-4-yl)-3-pyridyl]-6-oxo-pyridazine-3-carboxylate (305 mg, 934.70 μmol) in THF (4 mL), and then stirred at 25 °C for 16 h. The reaction mixture was concentrated under reduced pressure to remove solvent, and then diluted with water (10 mL). The reaction mixture was adjusted to pH = 3-4 with aq. 1 N HCl, and then extracted with EtOAc (20 mL × 3). The organic layers were washed with brine (30 mL), dried over $Na_2SO_4$, and then concentrated under reduced pressure to give **Intermediate 4-7** (63 mg, 20.29% yield, 94% purity) as a white solid. MS (ESI) m/z = 313.1 [M+H]+.

**Preparation Example 35-1: 5-methyl-1-[5-(3-methyltriazol-4-yl)-2-fluoro-3-pyridyl]-6-oxo-pyridazine-3-carboxylic acid (4-82)**

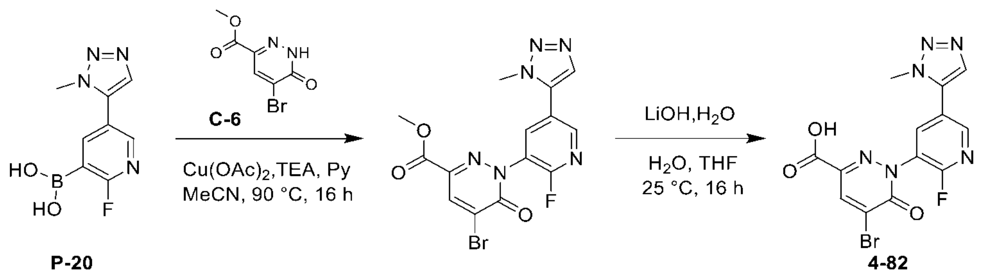


P-20 4-82

Intermediate P-20 and methyl 5-bromo-6-oxo-1,6-dihydropyridazine-3-carboxylate (C-6) were used in the same manner as in Preparation Example 35 to give **Intermediate 4-82** as a yellow solid. MS (ESI) m/z = 395.1 [M+H]+.

**Preparation Example 36: 5-deuterio-1-[5-(3-methyltriazol-4-yl)-3-pyridyl]-6-oxo-pyridazine-3- carboxylate (4-33)**

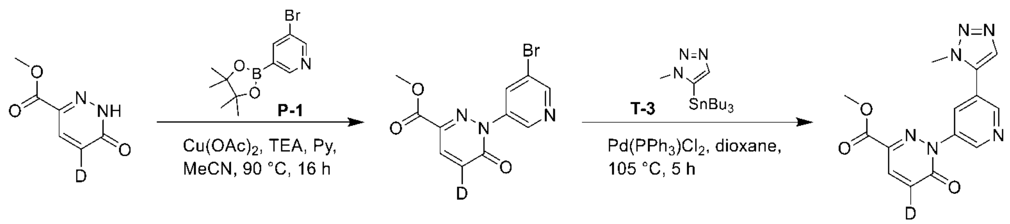


C-8 3-20 3-27

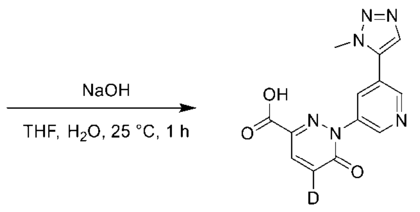


4-33

Step 1: Synthesis of methyl 1-(5-bromo-3-pyridyl)-5-deuterio-6-oxo-pyridazine-3-carboxylate

A solution of methyl 5-deuterio-6-oxo-1H-pyridazine-3-carboxylate (95 mg, 612.39 μmol), 3-bromo-5-(4,4,5,5-tetramethyl-1,3,2-dioxaborolan-2-yl)pyridine (**P-1,** 521.68 mg, 1.84 mmol), $Cu(OAc)_2$ (111.23 mg, 612.39 μmol), TEA (185.90 mg, 1.84 mmol, 255.71 μL) and pyridine (145.32 mg, 1.84 mmol, 148.29 μL) in MeCN (4 mL) was substituted with oxygen for 3 times, and then stirred at 90 °C for 16 h under oxygen atmosphere. The reaction mixture was filtered, and then the filtrate was concentrated under reduced pressure to give a crude product. The crude product was purified by silica gel

column chromatography (30% EtOAc in petroleum ether) to give methyl 1-(5-bromo-3-pyridyl)-5-deuterio-6-oxo-pyridazine-3-carboxylate (105 mg, 43.54% yield, 79% purity) as a yellow solid. $^1$H NMR (400 MHz, DMSO-$d_6$) δ 8.84 (d, *J* = 2.0 Hz, 2H), 8.39 (t, *J* = 2.0 Hz, 1H), 7.95 - 7.91 (s, 1H), 3.88 (s, 3H); MS (ESI) m/z = 312.8 [M+H]$^+$.

Step 2: Synthesis of methyl 5-deuterio-1-[5-(3-methyltriazol-4-yl)-3-pyridyl]-6-oxo-pyridazine-3-carboxylate

A solution of methyl 1-(5-bromo-3-pyridyl)-5-deuterio-6-oxo-pyridazine-3-carboxylate (105 mg, 337.50 μmol), tributyl-(3-methyltriazol-4-yl)stannane (220.00 mg, 591.18 μmol) and $Pd(PPh_3)_2Cl_2$ (23.69 mg, 33.75 μmol) in dioxane (3 mL) was substituted with nitrogen for 3 times, and then stirred at 105 °C for 5 h under nitrogen atmosphere. The reaction mixture was filtered, and then the filtrate was concentrated under reduced pressure to give a crude product. The crude product was purified by silica gel column chromatography (2% MeOH in PE) to give methyl 5-deuterio-1-[5-(3-methyltriazol-4-yl)-3-pyridyl]-6-oxo-pyridazine-3-carboxylate (100 mg, 53.62% yield, 56.7% purity) as a yellow solid. MS (ESI) m/z = 314.3 [M+H]$^+$.

Step 3: Synthesis of 5-deuterio-1-[5-(3-methyltriazol-4-yl)-3-pyridyl]-6-oxo-pyridazine-3-carboxylic acid

A solution of methyl 5-deuterio-1-[5-(3-methyltriazol-4-yl)-3-pyridyl]-6-oxo-pyridazine-3-carboxylate (50 mg, 159.60 μmol, 1 eq) and NaOH (12.77 mg, 319.19 μmol, 2 eq) in THF (1 mL) and $H_2O$ (0.5 mL) was substituted with nitrogen for 3 times, and then stirred at 25 °C for 1 h under nitrogen atmosphere. The reaction mixture was adjusted to pH = 5-6 with aq. 1 N HCl, and then concentrated under reduced pressure to give **Intermediate 4-33** (40 mg, 57.45% yield, 68.6% purity) as a yellow solid. It was used in the next reaction without further purification. MS (ESI) m/z = 299.9 [M+H]$^+$.

**Preparation Example 36-1: 1-[5-(3-cyclopropyltriazol-4-yl)-3-pyridyl]-5-deuterio-6-oxo-pyridazine-3-carboxylic acid (4-36)**

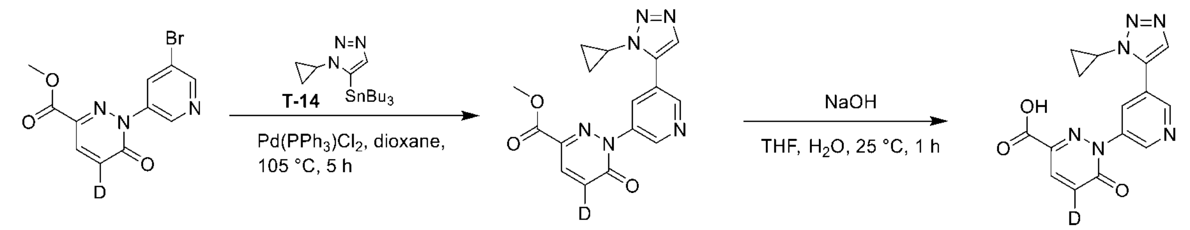


3-20 4-36

Intermediate 3-20 and intermediate T-14 were used in the same manner as in Steps 2 and 3 of Preparation Example 36 to give **Intermediate 4-36** (20 mg, 79.12% yield, 98.6% purity) as a yellow solid. MS (ESI) m/z = 326.1 [M+H]$^+$.

**Preparation Example 36-2: 1-[3-(3-isopropyltriazol-4-yl)phenyl]-5-methyl-6-oxo-pyridazine-3-carboxylic acid (4-37)**

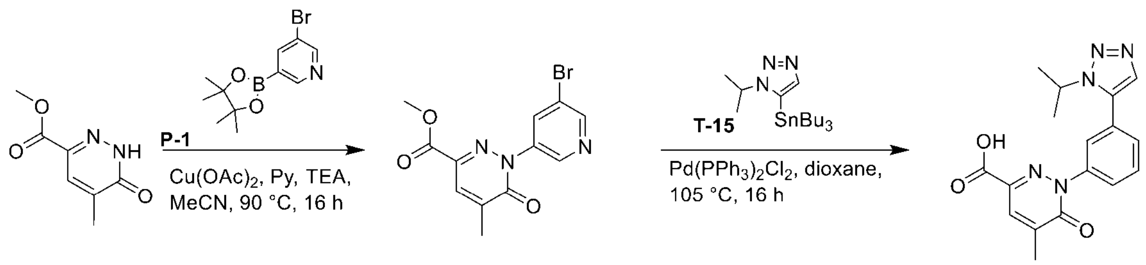


C-4 3-23 4-37

Step 1: Synthesis of methyl 1-(5-bromo-3-pyridyl)-5-methyl-6-oxo-pyridazine-3-carboxylate

A solution of methyl 5-methyl-6-oxo-1H-pyridazine-3-carboxylate (600 mg, 3.57 mmol), 3-bromo-5-(4,4,5,5-tetramethyl-1,3,2-dioxaborolan-2-yl)pyridine (1.01 g, 3.57 mmol), $Cu(OAc)_2$ (1.30 g, 7.14 mmol), TEA (722.14 mg, 7.14 mmol, 993.31 μL) and pyridine (564.50 mg, 7.14 mmol, 576.02 μL) in MeCN (15 mL) was substituted with oxygen for 3 times, and then stirred at 90 °C for 16 h under oxygen atmosphere. The reaction mixture was filtered, and then the filtrate was concentrated under reduced pressure to give a crude product. The crude product was purified by silica gel column chromatography (80% EtOAc in petroleum ether) to give methyl 1-(5-bromo-3-pyridyl)-5-methyl-6-oxo-pyridazine-3-carboxylate (Intermediate 3-23, 200 mg, 13.83% yield, 80% purity) as a yellow solid. MS (ESI) m/z = 325.9 $[M+H]^+$.

Step 2: Synthesis of 1-[3-(3-isopropyltriazol-4-yl)phenyl]-5-methyl-6-oxo-pyridazine-3-carboxylic acid

A solution of methyl 1-(5-bromo-3-pyridyl)-5-methyl-6-oxo-pyridazine-3-carboxylate (200 mg, 617.04 μmol), tributyl-(3-isopropyltriazol-4-yl)stannane (600 mg, 1.50 mmol) and $Pd(PPh_3)_2Cl_2$ (86.62 mg, 123.41 μmol) in dioxane (5 mL) was substituted with nitrogen for 3 times, and then stirred at 105 °C for 16 h under nitrogen atmosphere. The reaction mixture was filtered, and then the filtrate was concentrated under reduced pressure to give a crude product. The crude product was purified by prep-HPLC (Column: Welch Xtimate C18 40*200 mm 7 μm; mobile phase: [water (hydrochloride)-ACN]; 0%-38% B, 20.5 minute). The acetonitrile was concentrated under reduced pressure, and the remaining solvent was removed by lyophilization to give **Intermediate 4-37** (30 mg, 13.63% yield, 95.15% purity) as a yellow solid. MS (ESI) m/z = 341.0 $[M+H]^+$.

**Preparation Example 36-3: 1-[5-(3-cyclopropyltriazol-4-yl)-3-pyridyl]-5-methyl-6-oxo-pyridazine-3-carboxylic acid (4-39)**

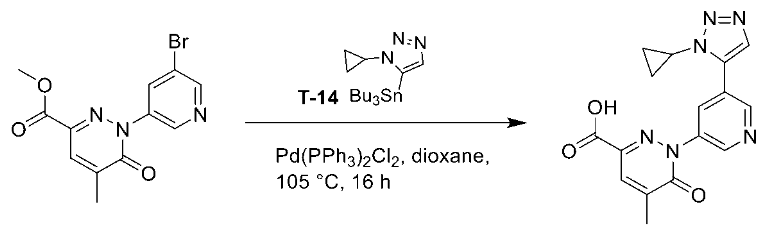


3-23 4-39

Intermediates 3-23 and T-14 were used in the same manner as in Step 2 of Preparation Example 36-2 to give **Intermediate 4-39** (40 mg, 25.04% yield, 98% purity) as a dark brown solid. MS (ESI) m/z = 339.1 $[M+H]^+$.

**Preparation Example 36-4: 1-[5-(3-cyclopropyltriazol-4-yl)-3-pyridyl]-5-ethyl-6-oxo-pyridazine-3-carboxylic acid (4-41)**

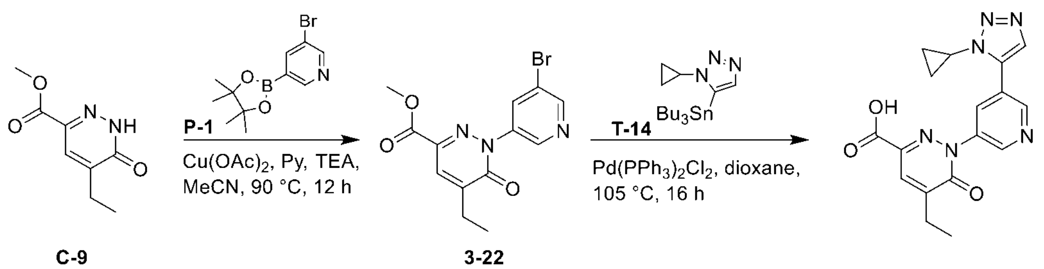


C-9 3-22 4-41

Intermediates C-9 and P-1 were used in the same manner as in Preparation Example 36-2 to give **Intermediate 4-41** (90 mg, 38.44% yield, 89% purity) as yellow oil. MS (ESI) m/z = 353.0 $[M+H]^+$.

**Preparation Example 36-5: 1-[5-(3-isopropyltriazol-4-yl)-3-pyridyl]-5-ethyl-6-oxo-pyridazine-3-carboxylic acid (4-40)**

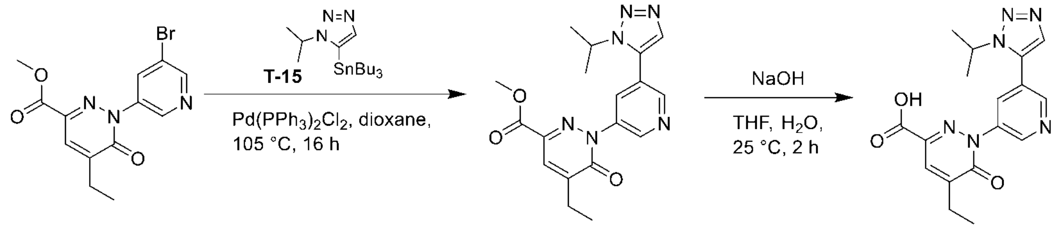


3-22 4-40

Intermediate 3-22 (the product obtained in Step 1 of Preparation Example 36-5) and Intermediate T-15 were used in the same manner as in Preparation Example 36-1 to give **Intermediate 4-40** (90 mg, 74.48% yield, 79.6% purity) as yellow oil. MS (ESI) m/z = 355.1 $[M+H]^+$.

**Preparation Example 36-6: 5-ethyl-1-[5-(3-methyltriazol-4-yl)-3-pyridyl]-6-oxo-pyridazine-3-carboxylic acid (4-31)**

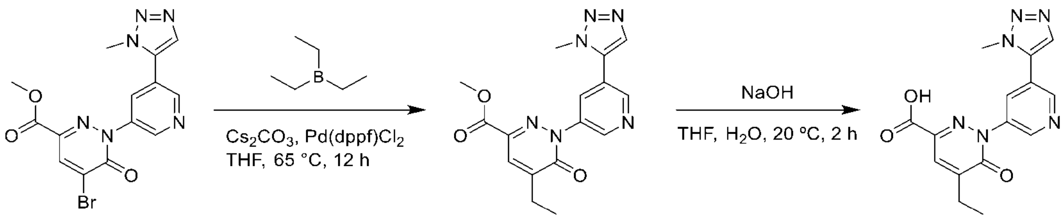


3-8 4-31

Step 1: Synthesis of methyl 5-ethyl-1-[5-(3-methyltriazol-4-yl)-3-pyridyl]-6-oxo-pyridazine-3-carboxylate

A solution of 5-bromo-1-[5-(3-methyltriazol-4-yl)-3-pyridyl]-6-oxo-pyridazine-3-carboxylate (46 mg, 117.59 μmol), $Cs_2CO_3$ (76.63 mg, 235.19 μmol), $Pd(dppf)Cl_2$ (3.44 mg, 4.70 μmol) and triethyl borate (1 M in THF, 470.37 μL) in THF (3 mL) was substituted with nitrogen for 3 times, and then stirred at 70 °C for 12 h under nitrogen atmosphere. The reaction mixture was filtered, and then the filtrate was concentrated under reduced pressure to give a crude product. The crude product was purified by silica gel column chromatography (3% DCM in MeOH) to give methyl 5-ethyl-1-[5-(3-methyltriazol-4-yl)-3-pyridyl]-6-oxo-pyridazine-3-carboxylate (26 mg, 32.48% yield, 50% purity) as a white solid. MS (ESI) m/z = 340.6 $[M+H]^+$.

Step 2: Synthesis of 5-ethyl-1-[5-(3-methyltriazol-4-yl)-3-pyridyl]-6-oxo-pyridazine-3-carboxylic acid

The intermediate, methyl 5-ethyl-1-[5-(3-methyltriazol-4-yl)-3-pyridyl]-6-oxo-pyridazine-3-carboxylate was used in the same manner as in Step 3 of Preparation Example 36 to give **Intermediate 4-31** (20 mg, 48.14% yield, 60% purity) as a yellow solid. MS (ESI) m/z = 326.9 $[M+H]^+$.

**Preparation Example 36-7: 1-[5-(3-methyltriazol-4-yl)-3-pyridyl]-6-oxo-5-phenyl-pyridazine-3-carboxylic acid (4-26)**

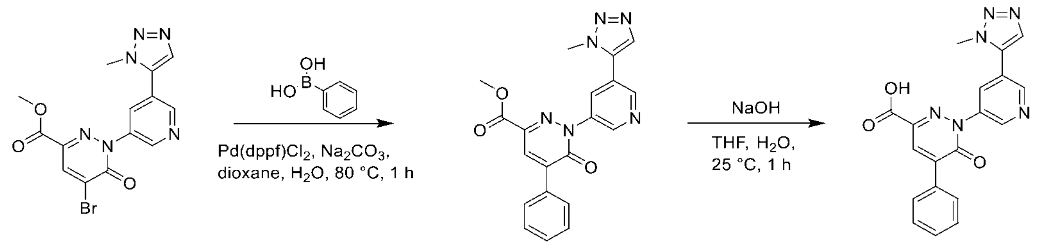


3-8 3-28 4-26

Step 1: Synthesis of methyl 1-[5-(3-methyltriazol-4-yl)-3-pyridyl]-6-oxo-5-phenyl-pyridazine-3-carboxylate

A solution of 5-bromo-1-[5-(3-methyltriazol-4-yl)-3-pyridyl]-6-oxo-pyridazine-3-carboxylate **(Intermediate 3-8,** 30 mg, 76.69 μmol), phenylboronic acid(28.05 mg, 230.07 μmol), Pd(dppf)$Cl_2$ (5.61 mg, 7.67 μmol), $Na_2CO_3$ (16.26 mg, 153.38 μmol) and $H_2O$ (0.4 mL) in dioxane (4 mL) was degassed and purged with nitrogen for 3 times. Then the mixture was stirred at 80 °C for 1 h under nitrogen atmosphere. The mixture was purified by silica column chromatography (7% MeOH in DCM) to give methyl-1-[5-(3-methyltriazol-4-yl)-3-pyridyl]-6-oxo-5-phenyl-pyridazine-3-carboxylate **(Intermediate 3-28,** 58 mg, 97.36% yield, 100% purity) as a yellow solid. MS (ESI) m/z = 389.1$[M+H]^+$.

Step 2: Synthesis of 1-[5-(3-methyltriazol-4-yl)-3-pyridyl]-6-oxo-5-phenyl-pyridazine-3-carboxylic acid

Intermediate 3-28 was used in the same manner as in Step 2 of Preparation Example 36-1 to give **Intermediate 4-26** (55 mg, 98.38% yield, 62% purity) as a yellow solid. MS (ESI) m/z = 375.1 $[M+H]^+$.

**Preparation Example 36-8: 5-(cyclohexen-1-yl)-1-[5-(3-methyltriazol-4-yl)-3-pyridyl]-6-oxo-pyridazine-3-carboxylic acid (4-28)**

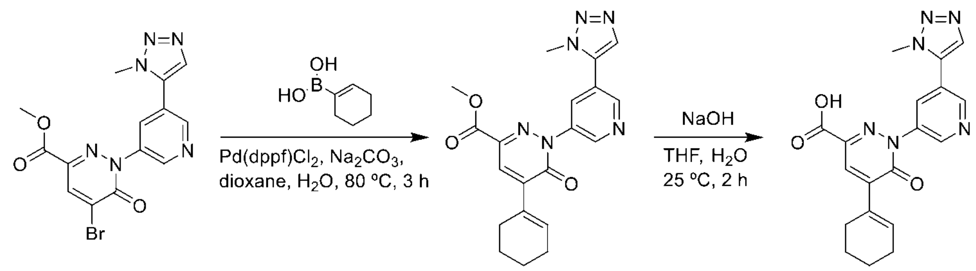


3-8 3-24 4-28

Intermediate 3-8 and (cyclohexen-1-yl)boronic acid (which is commercially available) were used in the same manner as in Preparation Example 36-7 to give **Intermediate 4-28** (56 mg, 75.46% yield, 76.66% purity) as a yellow solid. MS (ESI) m/z = 379.2 $[M+H]^+$.

**Preparation Example 36-9: 5-cyano-1-[5-(3-methyltriazol-4-yl)-3-pyridyl]-6-oxo-pyridazine-3-carboxylic acid (4-24)**

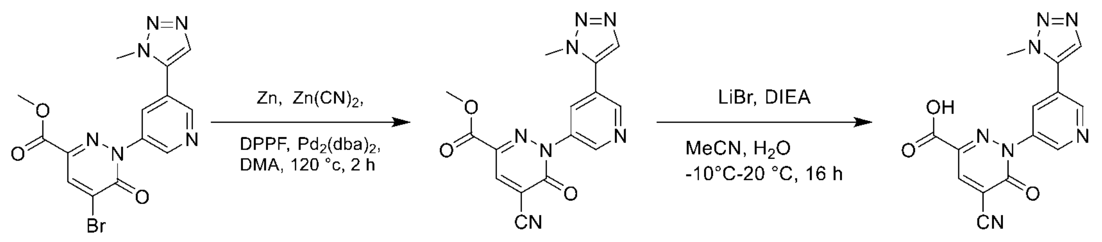


3-8 3-29 4-24

Step 1: Synthesis of methyl 5-cyano-1-[5-(3-methyltriazol-4-yl)-3-pyridyl]-6-oxo-pyridazine-3-carboxylate

$Zn(CN)_2$ (70 mg, 596 μmol) and Zn (40 mg, 611 μmol) were added to a solution of methyl 5-bromo-1-[5-(3-methyltriazol-4-yl)-3-pyridyl]-6-oxo-pyridazine-3-carboxylate (200 mg, 511 μmol), DPPF (56.69 mg, 102 μmol) and $Pd_2(dba)_3$ (46.82 mg, 51.1 μmol) in DMA (3 mL). Then the mixture was substituted with nitrogen for 3 times, and stirred at 120 °C for 2 h under nitrogen atmosphere. The reaction mixture was filtered, the filtrate was poured into water (20 mL), and then extracted with EtOAc (15 mL x 4). The combined organic layers were washed with brine (10 mL x 3), dried over $Na_2SO_4$, and then concentrated under reduced pressure to give a crude product. The aq. layers were quenched with aq. 1N HCl (5 mL) and stirred overnight. The residue was purified by silica gel column chromatography (0~100% ethyl acetate/Petroleum ether) to give methyl 5-cyano-1-[5-(3-methyltriazol-4-yl)-3-pyridyl]-6-oxo-pyridazine-3-carboxylate (73 mg, 25.23% yield, 59.6% purity) as dark brown oil. MS (ESI) m/z = 338.1 $[M+H]^+$.

Step 2: Synthesis of 5-cyano-1-[5-(3-methyltriazol-4-yl)-3-pyridyl]-6-oxo-pyridazine-3-carboxylic acid

LiBr (29.89 mg, 344 μmol) was added to a solution of $H_2O$ (1 mL) in MeCN (1 mL) at -10 °C for more than 1 minute. Then the mixture was stirred at -10 °C for 1 h to give solution A. Methyl 5-cyano-1-[5-(3-methyltriazol-4-yl)-3-pyridyl]-6-oxo-pyridazine-3-carboxylate (43 mg, 127 μmol) and DIEA (39.97 μL, 229 μmol) were added dropwise to the solution A at -10 °C, and then it was stirred at 20 °C for 1 h. The reaction mixture was poured into water (5 mL), adjusted to pH = 4 with aq. 1 N HCl, and then extracted with EtOAc (10 mL x 3). The combined organic layers were washed with brine (5 mL x 2), dried over $Na_2SO_4$, and then concentrated under reduced pressure to give **Intermediate 4-24** (25 mg, 60.06% yield, 99% purity) as brown oil. MS (ESI) m/z = 323.9 $[M+H]^+$.

**Preparation Example 36-10: 5-acetyl-1-[5-(3-methyltriazol-4-yl)-3-pyridyl]-6-oxo-pyridazine-3-carboxylic acid (4-34)**

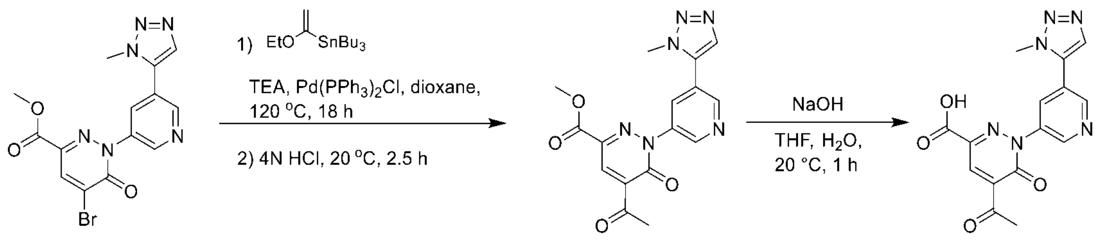


3-8 4-34

Step 1: Synthesis of methyl 5-acetyl-1-[5-(3-methyltriazol-4-yl)-3-pyridyl]-6-oxo-pyridazine-3-carboxylate

Tributyl(1-ethoxyvinyl)stannane (454.23 mg, 1.26 mmol, 424.91 μL), TEA (212.12 mg, 2.10 mmol, 291.77 μL) and $Pd(PPh_3)_2Cl_2$ (73.57 mg, 104.81 μmol) were added to a solution of methyl 5-bromo-1-[5-(3-methyltriazol-4-yl)-3-pyridyl]-6-oxo-pyridazine-3-carboxylate (410 mg, 1.05 mmol) in dioxane (6 mL). Then the mixture was stirred at 120 °C for 18 h under $N_2$ atmosphere. To the mixture was added HCl solution (50 ml, 1 M) under stirring at 20 °C, and then the mixture was stirred at 20 °C for 2.5 h. After the addition of water (10 mL), the reaction mixture was extracted with EtOAc (20 mL × 3). The combined organic layers were washed with brine (10 mL x 3), dried over anhydrous $Na_2SO_4$, and then concentrated under reduced pressure to give a residue. Sat. aq. KF (15 mL) was slowly added to the aqueous layer under stirring, and then the mixture was stirred at 25 °C for 1 h. The product was purified by silica gel column chromatography (2% MeOH in DCM) to give methyl 5-acetyl-1-[5-(3-methyltriazol-4-yl)-3-pyridyl]-6-oxo-pyridazine-3-carboxylate (200 mg, 21.54% yield, 40% purity) as a yellow solid. MS (ESI) m/z = 355.1 $[M+H]^+$.

Step 2: Synthesis of 5-acetyl-1-[5-(3-methyltriazol-4-yl)-3-pyridyl]-6-oxo-pyridazine-3-carboxylic acid

Methyl 5-acetyl-1-[5-(3-methyltriazol-4-yl)-3-pyridyl]-6-oxo-pyridazine-3-carboxylate was used in the same manner as in Step 3 of Preparation Example 36 to give **Intermediate 4-34** (80 mg, 41.57% yield, 99.8% purity) as a yellow solid. MS (ESI) m/z = 340.9 $[M+H]^+$.

**Preparation Example 36-11: 5-(hydroxymethyl)-1-[5-(3-methyltriazol-4-yl)-3-pyridyl]-6-oxo-pyridazine-3-carboxylic acid (4-25)**

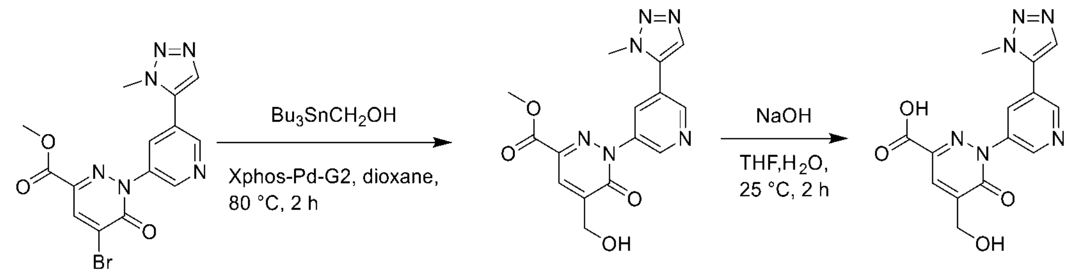


3-8 4-25

Step 1: Synthesis of methyl 5-(hydroxymethyl)-1-[5-(3-methyltriazol-4-yl)-3-pyridyl]-6-oxo-pyridazine-3-carboxylate

Xphos-Pd-G2 (10.06 mg, 12.78 μmol), tributylstanylmethanol (123.12 mg, 383.46 μmol) was added to a solution of methyl 5-bromo-1-[5-(3-methyltriazol-4-yl)-3-pyridyl]-6-oxo-pyridazine-3-carboxylate (50 mg, 127.82 μmol) in dioxane (3 mL). Then the mixture was stirred at 80 °C for 2 h. The mixture was purified by silica gel column chromatography (5% MeOH in DCM) to give methyl 5-(hydroxymethyl)-1-[5-(3-methyltriazol-4-yl)-3-pyridyl]-6-oxo-pyridazine-3-carboxylate (21 mg, 44.03% yield, 91.74% purity) as yellow oil. MS (ESI) m/z = 343.0 $[M+H]^+$.

Step 2: Synthesis of 5-(hydroxymethyl)-1-[5-(3-methyltriazol-4-yl)-3-pyridyl]-6-oxo-pyridazine-3-carboxylic acid

Methyl-5-(hydroxymethyl)-1-[5-(3-methyltriazol-4-yl)-3-pyridyl]-6-oxo-pyridazine-3-carboxylate was used in the same manner as in Step 3 of Preparation Example 36 to give **Intermediate 4-25** (20 mg, 99.31% yield, 96% purity) as a yellow solid. MS (ESI) m/z = 329.0 $[M+H]^+$.

**Preparation Example 36-12: 5-cyclohexyl-1-[5-(3-methyltriazol-4-yl)-3-pyridyl]-6-oxo-pyridazine-3-carboxylic acid (4-29)**

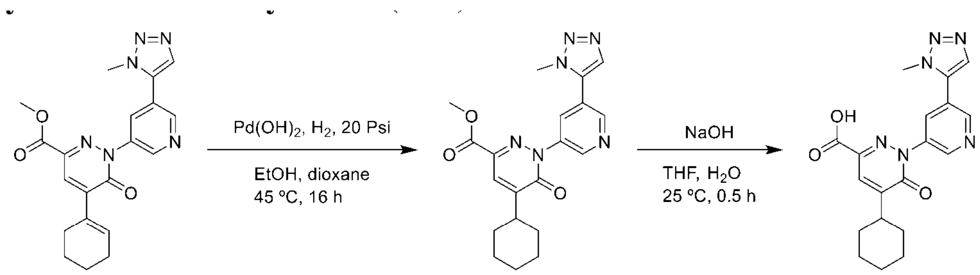


3-24 4-29

Step 1: Synthesis of methyl 5-cyclohexyl-1-[5-(3-methyltriazol-4-yl)-3-pyridyl]-6-oxo-pyridazine-3-carboxylate

$Pd(OH)_2$ (13.96 mg, 19.88 μmol, 20% purity) was added to a solution of Intermediate 3-24 (26 mg, 66.26 μmol) in dioxane (4 mL) and ethanol (4 mL) under nitrogen atmosphere. Hydrogen was purged several times under reduced pressure. Then the mixture was stirred at 45 °C for 16 h under hydrogen atmosphere (20 Psi). The reaction mixture was filtered, and then the filtrate was concentrated under reduced pressure to give a crude product. The crude product was purified by silica gel column chromatography (3% DCM in MeOH) to give methyl 5-cyclohexyl-1-[5-(3-methyltriazol-4-yl)-3-pyridyl]-6-oxo-pyridazine-3-carboxylate (30 mg, 98.56% yield, 85.860% purity) as a white solid. MS (ESI) m/z = 395.0 $[M+H]^+$.

Step 2: Synthesis of 5-cyclohexyl-1-[5-(3-methyltriazol-4-yl)-3-pyridyl]-6-oxo-pyridazine-3-carboxylic acid

Methyl 5-cyclohexyl-1-[5-(3-methyltriazol-4-yl)-3-pyridyl]-6-oxo-pyridazine-3-carboxylate was used in the same manner as in Step 3 of Preparation Example 36 to give **Intermediate 4-29** (30 mg, 93.19% yield, 89.880% purity) as a yellow solid. MS (ESI) m/z = 380.7 [M+H]+.

**Preparation Example 36-13: 1-[5-[3-(1-tert-butoxycarbonylazetidin-3-yl)triazol-4-yl]-3-pyridyl]-6-oxo-pyridazine-3-carboxylic acid (4-66)**

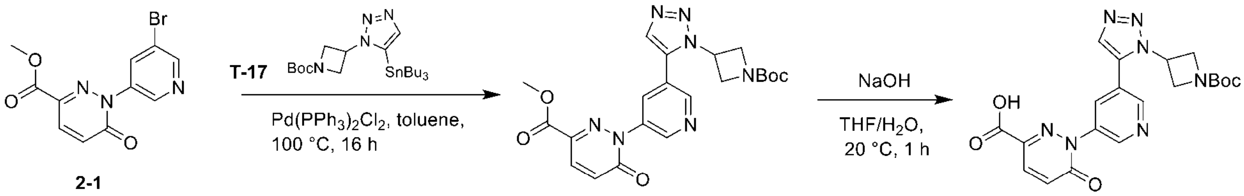


2-1

4-66

Methyl 1-(5-bromopyridin-3-yl)-6-oxo-1,6-dihydropyridazine-3-carboxylate **(2-1)** and Intermediate T-17 were used in the same manner as in Preparation Example 36-1 to give **Intermediate 4-66** (380 mg, 69.96% purity, 71.36% yield) as yellow oil. MS (ESI) m/z = 340.0 [M+H]+.

**Preparation Example 36-14: 1-[5-[3-(oxetan-3-yl)triazol-4-yl]-3- pyridyl]-6-oxo-pyridazine-3-carboxylic acid (4-35)**

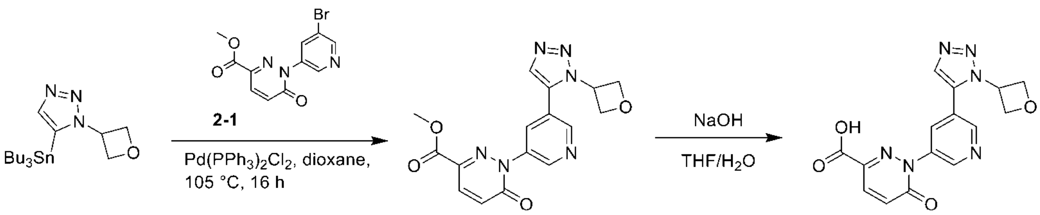


T-18

4-35

Intermediates 2-1 and T-18 were used in the same manner as in Preparation Example 36-1 to give **Intermediate 4-35** (27 mg, 32.53 μmol, 38.42 yield, 41% purity) as a light yellow solid. MS (ESI) m/z = 341.0 [M+H]+.

**Preparation Example 36-15: 1-[5-(3-cyclobutyl-4-yl)-3-pyridyl]-6-oxo-pyridazine-3-carboxylic acid (4-43)**

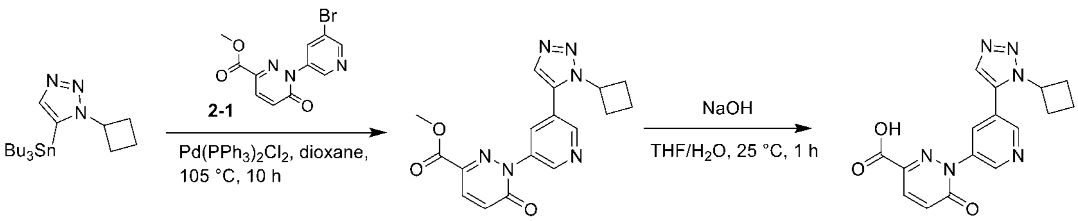


T-19

4-43

Intermediates 2-1 and T-19 were used in the same manner as in Preparation Example 36-1 to give **Intermediate 4-43** (50 mg, 81.13% yield, 93.48% purity) as a yellow solid. MS (ESI) m/z = 339.0 [M+H]+.

**Preparation Example 37: 1-[6-(methylamino)-5-(1-methyl-1H-1,2,3-triazol-5-yl)-3-pyridyl]-6-oxo-1,6-dihydropyridazine-3-carboxylic acid (4-44)**

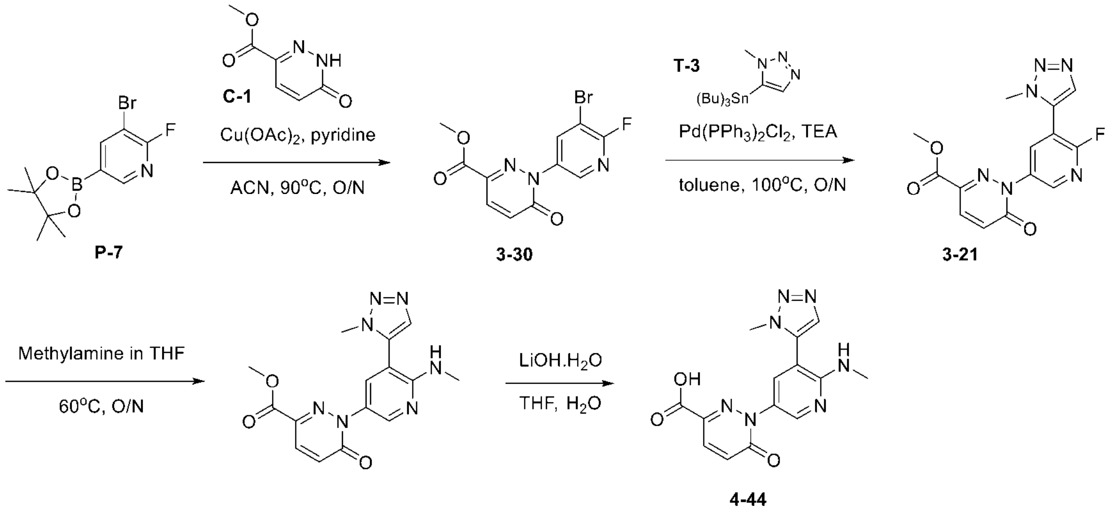


Step 1: Synthesis of methyl 1-(5-bromo-6-fluoro-3-pyridyl)-6-oxo-1,6-dihydropyridazine-3-carboxylate

Methyl 6-oxo-1,6-dihydropyridazine-3-carboxylate **(C-1)** and Intermediate P-7 were used in the same manner as in Step 1 of Preparation Example 33 to give **Intermediate 3-30** (213 mg, 100%) as a white solid. $^1$H NMR (400 MHz, $CDCl_3$) δ 8.54 (dd, *J* = 2.4, 1.2 Hz, 1H), 8.37 (dd, *J* = 7.6, 2.4 Hz, 1H), 7.95 (d, *J* = 9.6 Hz, 1H), 7.12 (d, *J* = 10.0 Hz, 1H), 3.40 (s, 3H); MS (ESI) m/z = 327.9 $[M+H]^+$, 329.9 $[M+3H]^+$.

Step 2: Synthesis of methyl 1-[6-fluoro-5-(1-methyl-1H-1,2,3-triazol-5-yl)-3-pyridyl]-6-oxo-1,6-dihydropyridazine-3-carboxylate

Intermediates 3-30 and T-3 were used in the same manner as in Step 2 of Preparation Example 33 to give **Intermediate 3-21** (145 mg, 67%) as a white solid. $^1$H NMR (400 MHz, $CDCl_3$) δ 8.73 (q, *J* = 7.3 Hz, 1H), 8.24 (dd, *J* = 8.0, 2.8 Hz, 1H), 7.99 (d, *J* = 9.6 Hz, 1H), 7.89 (s, 1H), 7.15 (d, *J* = 9.6 Hz, 1H), 4.13 (d, *J* = 1.2 Hz, 3H), 4.00 (s, 3H); MS (ESI) m/z = 331.0 $[M+H]^+$.

Step 3: Synthesis of methyl 1-[6-(methylamino)-5-(1-methyl-1H-1,2,3-triazol-5-yl)-3-pyridyl]-6-oxo-1,6-dihydropyridazine-3-carboxylate

A solution of Intermediate 3-21 (70 mg, 0.212 mmol) in methylamine (1M in THF, 1.1 mL) was stirred at 60 °C for 16 h under $N_2$ atmosphere. Methylamine (1M in THF, 1.1 mL) was further added to the reaction mixture and stirred at 60 °C for 2 h. Then the reaction mixture was concentrated under reduced pressure, crystallized by DCM and hexane to give methyl 1-[6-(methylamino)-5-(1-methyl-1H-1,2,3-triazol-5-yl)-3-pyridyl]-6-oxo-1,6-dihydropyridazine-3-carboxylate (63mg, 87%) as a yellow solid. It was used in the next reaction without further purification. MS (ESI) m/z = 342.0 $[M+H]^+$.

Step 4: Synthesis of 1-[6-(methylamino)-5-(1-methyl-1H-1,2,3-triazol-5-yl)-3-pyridyl]-6-oxo-1,6-dihydropyridazine-3-carboxylic acid

The intermediate, methyl 1-[6-(methylamino)-5-(1-methyl-1H-1,2,3-triazol-5-yl)-3-pyridyl]-6-oxo-1,6-dihydropyridazine-3-carboxylate was used in the same manner as in Step 3 of Preparation Example 33 to give **Intermediate 4-44** (25 mg, 41%) as a yellow solid. MS (ESI) m/z = 328.0 $[M+H]^+$.

**Preparation Example 38: 1-[5-(1-methyl-1H-1,2,3-triazol-5-yl)-6-morpholino-3-pyridyl]-6-oxo-1,6-dihydropyridazine-3-carboxylic acid (4-45)**

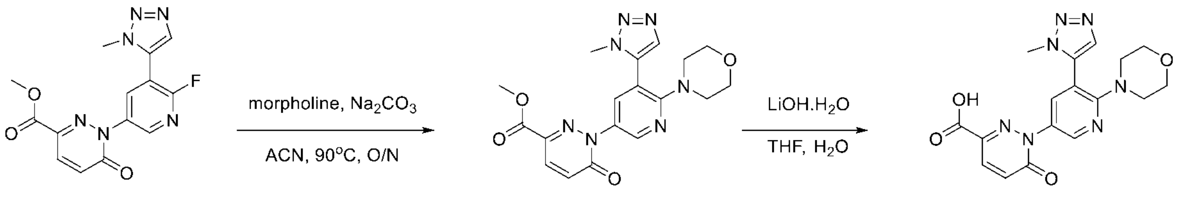


3-21

4-45

Step 1: Synthesis of methyl 1-[5-(1-methyl-1H-1,2,3-triazol-5-yl)-6-morpholino-3-pyridyl]-6-oxo-1,6-dihydropyridazine-3-carboxylate

Intermediate 3-21 (65 mg, 0.197 mmol), morpholine (25.5 μL, 0.295 mmol) and $Na_2CO_3$ (42 mg, 0.394 mmol) in MeCN (0.5 mL) were placed in a microwave vial and stirred at 90°C for 17.5 h. Then the mixture was cooled to room temperature and diluted with EtOAc. The solid was filtered with celite pad, washed with EtOAc. The filtrate was concentrated under reduced pressure. The product was purified by silica gel column chromatography to give methyl 1-[5-(1-methyl-1H-1,2,3-triazol-5-yl)-6-morpholino-3-pyridyl]-6-oxo-1,6-dihydropyridazine-3-carboxylate (54 mg, 69%) as a yellow solid. $^1$H NMR (400 MHz, $CDCl_3$) δ 8.67 (d, *J* = 2.8 Hz, 1H), 7.95 (d, *J = 9.6* Hz, 1H), 7.81 (s, 1H), 7.79 (d, *J* = 2.8 Hz, 1H), 7.11 (d, *J* = 10.0 Hz, 1H), 4.04 (s, 3H), 3.99 (s, 3H), 3.66 (t, *J* = 4.6 Hz, 4H), 3.14 (t, *J* = 4.8 Hz, 4H); MS (ESI) m/z = 398.1 $[M+H]^+$.

Step 2: Synthesis of 1-[5-(1-methyl-1H-1,2,3-triazol-5-yl)-6-morpholino-3-pyridyl]-6-oxo-1,6-dihydropyridazine-3-carboxylic acid

The intermediate, methyl 1-[5-(1-methyl-1H-1,2,3-triazol-5-yl)-6-morpholino-3-pyridyl]-6-oxo-1,6-dihydro-pyridazine-3-carboxylate was used in the same manner as in Step 3 of Preparation Example 33 to give **Intermediate 4-45** (33 mg, 63%) as a yellow solid. MS (ESI) m/z = 384.1 $[M+H]^+$.

**Preparation Example 39: methyl 5-fluoro-6-oxo-1,6-dihydropyridin-3-carboxylate (C-5)**

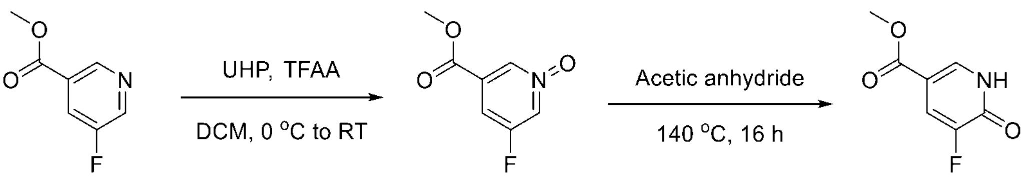


C-5

Step 1: Synthesis of 3-fluoro-5-(methoxycarbonyl)pyridine-1-oxide

Urea hydrogen peroxide (1.27 g, 13.5 mmol) and trifluoroacetic acid anhydride (4.66 mL, 33.5 mmol) were add to a solution of methyl-5-fluoronicotinate (1 g, 6.45 mmol) in DCM (14.3 mL) under nitrogen atmosphere at 0 °C. Then the reaction mixture was stirred at 25 °C for 17 h. The reaction mixture was cooled to 0 °C, added with aq. $NaHCO_3$, and then extracted with DCM. The combined organic layers were washed with aq. sodium bisulfate, dried over $Na_2SO_4$, and then concentrated under reduced pressure to give a crude product. The crude product was purified by silica gel column chromatography (5% MeOH in DCM) to give 3-fluoro-5-(methoxycarbonyl)pyridine-1-oxide (245 mg, 22% yield) as a light yellow solid. $^1$H NMR (400 MHz, $CDCl_3$) δ 9.07 (s, 1H), 8.67 (s, 1H), 8.01 (ddd, *J* = 8.6, 2.8,1.6 Hz, 1H), 3.99 (s, 3H); MS (ESI) m/z = 171.95 $[M+H]^+$.

Step 2: Synthesis of methyl 5-fluoro-6-oxo-1,6-dihydropyridine-3-carboxylate

A solution of 3-fluoro-5-(methoxycarbonyl)pyridine-1-oxide (394 mg, 2.30 mmol) in acetic anhydride (11.3 mL) was stirred at 140 °C for 17 h under nitrogen atmosphere. The reaction mixture was quenched with aq. $NaHCO_3$ and extracted with EtOAc. The combined organic layers were dried over $Na_2SO_4$, and then concentrated under reduced pressure to give a crude product. The crude product was purified by silica gel column chromatography (5% MeOH in DCM) to give **Intermediate C-5** (129 mg, 33% yield) as a white solid. $^1$H NMR (400 MHz, $CDCl_3$) δ 12.81 (brs, 1H), 8.07-8.08 (m,

1H), 7.77 (dd, *J* = 10.4, 2.2 Hz, 1H), 3.89 (s, 3H).

**Preparation Example 40: methyl 5-deuterio-6-oxo-1H-pyridazine-3-carboxylate (C-8)**

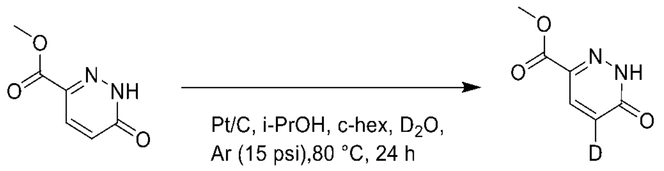


C-1 C-8

A solution of methyl 6-oxo-1H-pyridazine-3-carboxylate (200 mg, 1.30 mmol), Pt/C (163.59 mg, 38.93 μmol, 5% purity) in $D_2O$ (4 mL), isopropanol (0.2 mL) and cyclohexane (1.8 mL) was degassed and purged with argon for 3 times. Then the mixture was stirred at 90 °C for 24 h under argon atmosphere in 50 mL autoclave. The reaction mixture was diluted with DCM (20 mL), filtered, and the filter cake was washed with DCM (20 mL x 2), concentrated under reduced pressure to give **Intermediate C-8** (100 mg, 49.68% yield) as a brown solid. $^1H$ NMR (400 MHz, DMSO-d6) δ 7.89 (s, 1H), 3.91 (s, 3H); MS (ESI) m/z = 155.9 $[M+H]^+$.

**Preparation Example 41: methyl 5-ethyl-6-oxo-1H-pyridazine-3-carboxylate (C-9)**

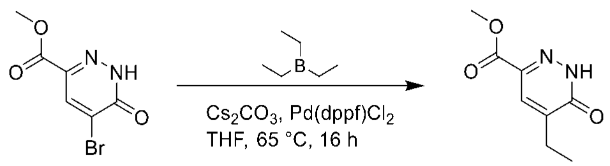


C-6 C-9

A solution of methyl 5-bromo-6-oxo-1H-pyridazine-3-carboxylate (46 mg, 117.59 μmol), $Cs_2CO_3$ (6.99 g, 21.46 mmol), $Pd(dppf)Cl_2$ (3.14 g, 4.29 mmol) and triethyl borate (1 M, 107.29 mL) in THF (60 mL) was substituted with nitrogen for 3 times, and then stirred at 65 °C for 16 h under nitrogen atmosphere. The reaction mixture was filtered, and then the filtrate was concentrated under reduced pressure to give a crude product. The crude product was purified by silica gel column chromatography (1% DCM in MeOH) to give **Intermediate C-9** (930 mg, 42.82% yield, 90% purity) as a dark brown solid. MS (ESI) m/z = 183.1 $[M+H]^+$.

**Preparation Example 42: 3-fluoro-5'-(1-methyl-1H-1,2,3-triazol-5-yl)-2-oxo-2H-[1,3'-bipyridine]-5-carboxylic acid (4-8)**

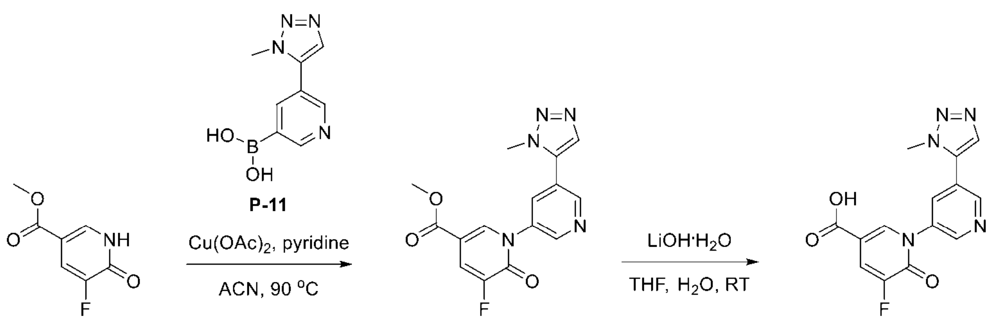


C-5 4-8

Intermediate C-5 of Preparation Example 39 was used in the same manner as in Steps 1 and 3 of Preparation Example 33 to give **Intermediate 4-8** (12.8 mg, 58% yield). MS (ESI) m/z = 316.06 [M+H]+.

**Preparation Example 42-1: 5-bromo-1-[5-(3-methyltriazol-4-yl)-3-pyridyl]-6-oxo-pyridazine-3-carboxylic acid (4-10)**

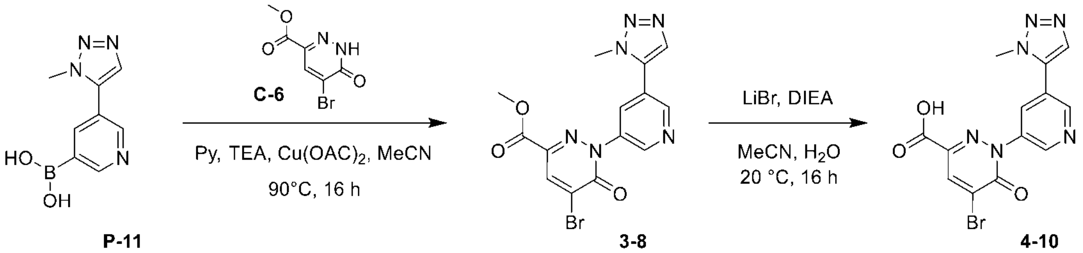


P-11 3-8 4-10

Step 1: Synthesis of methyl 5-bromo-1-[5-(3-methyltriazol-4-yl)-3-pyridyl]-6-oxo-pyridazine-3-carboxylate

Intermediates C-6 and P-11 were used in the same manner as in Step 1 of Preparation Example 36-2 to give **Intermediate 3-8** (137 mg, 13.78% yield, 93% purity) as a yellow solid. MS (ESI) m/z = 391.0, 392.9 [M+H]+.

Step 2: Synthesis of 5-bromo-1-[5-(3-methyltriazol-4-yl)-3-pyridyl]-6-oxo-pyridazine-3-carboxylic acid

Intermediate 3-8 was used in the same manner as in Step 2 of Preparation Example 36-9 **Intermediate 4-10** (90 mg, crude product) as a brown solid. MS (ESI) m/z = 376.9, 379.0 [M+H]+.

**Preparation Example 42-2: 3-bromo-5'-(1-methyl-1H-1,2,3-triazol-5-yl)-2-oxo-2H-[1,3'-bipyridine]-5-carboxylic acid (4-11)**

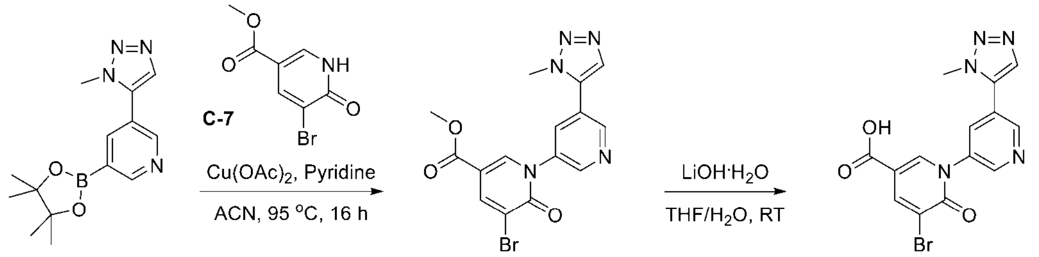


P-11 3-9 4-11

Intermediates P-11 and C-7 were used in the same manner as in Preparation Example 35 to give **Intermediate 4-11** (52 mg, 71% yield) as a light yellow solid. MS (ESI) m/z = 375.98, 377.97 [M+H]+.

**Preparation Example 42-3: 1-(5-bromo-2-fluoro-3-pyridyl)-6-oxo-pyridazine-3-carboxylic acid (4-30)**

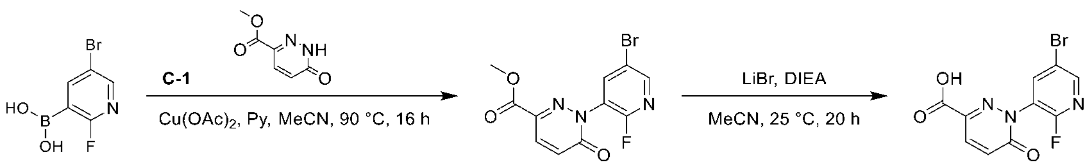


P-10 3-25 4-30

Commercially available (5-bromo-2-fluoropyridin-3-yl)boronic acid **(P-10)** and Intermediate C-1 were used in the same manner as in Preparation Example 42-1 to give **Intermediate 4-30** (45 mg, 78.85% yield, 93.6% purity, hydrochloride) as a yellow solid. MS (ESI) m/z = 313.8 $[M+H]^+$.

**Preparation Example 42-4: 1-[5-(3-methyltriazol-4-yl)-3-pyridyl]-6-oxo-5-(trifluoromethyl)pyridazine-3-carboxylic acid (4-77)**

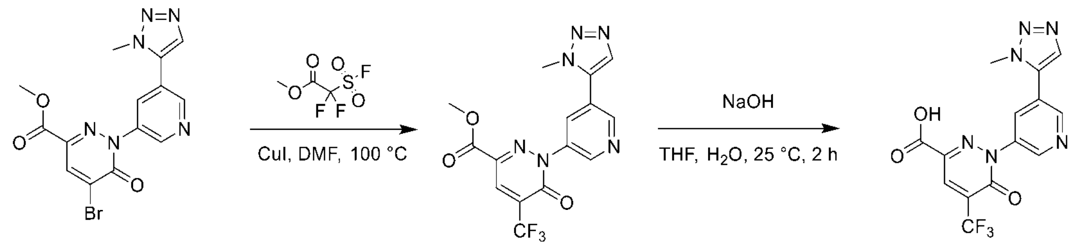


3-8

4-77

Step 1: Synthesis of methyl 1-[5-(3-methyltriazol-4-yl)-3-pyridyl]-6-oxo-5-(trifluoromethyl)pyridazine-3-carboxylate

CuI (14.61 mg, 76.69 μmol) and methyl 2,2-difluoro-2-fluorosulfonyl-acetate (736.67 mg, 3.83 mmol, 487.86 μL) were add to a solution of Intermediate 3-8 (300 mg, 766.91 μmol) in DMF (5 mL). The mixture was stirred at 80 °C for 3 h. The reaction mixture was concentrated under reduced pressure to give a crude product. The crude product was diluted with EtOAc (30 mL) and washed with brine (15 mL × 3). The organic layers were dried over anhydrous $Na_2SO_4$, and then concentrated under reduced pressure to give a crude product. The crude product was purified by silica gel column chromatography (1% MeOH in DCM) to give methyl 1-[5-(3-methyltriazol-4-yl)-3-pyridyl]-6-oxo-5-(trifluoromethyl)pyridazine-3-carboxylate (40 mg, 9.59% yield, 69.9% purity) as yellow oil. MS (ESI) m/z = 380.9 $[M+H]^+$.

Step 2: Synthesis of 1-[5-(3-methyltriazol-4-yl)-3-pyridyl]-6-oxo-5-(trifluoromethyl)pyridazine-3-carboxylic acid

The intermediate, methyl 1-[5-(3-methyltriazol-4-yl)-3-pyridyl]-6-oxo-5-(trifluoromethyl) pyridazine-3-carboxylate was used in the same manner as in Step 2 of Preparation Example 35 to give **Intermediate 4-77** (30 mg, 27.26% yield, 35% purity) as a yellow solid. MS (ESI) m/z = 366.9 $[M+H]^+$.

**Preparation Example 42-5: 1-[5-[3-(1-tert-butoxycarbonyl-4-piperidyl)triazol-4-yl]-3-pyridyl]-6-oxo-pyridazine-3-carboxylic acid (4-79)**

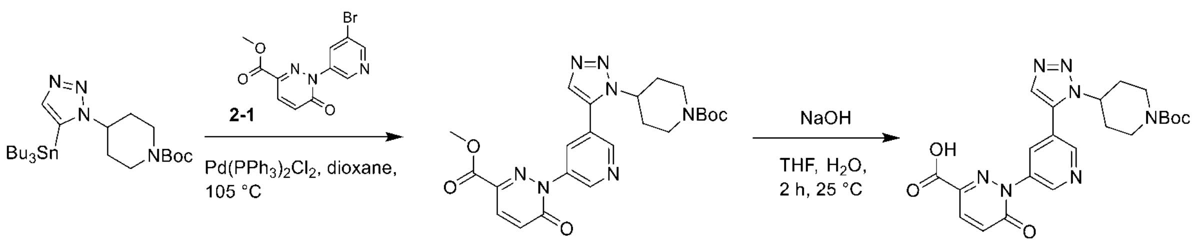


T-21

4-79

Intermediates T-21 and 2-1 were used in a similar manner as in Preparation Example 31 to give **Intermediate 4-79** (450 mg, 81.58% yield, 88% purity) as a yellow solid. MS (ESI) m/z = 468.1 [M+H] +.

**Preparation Example 43: 1-(6-methyl-5-(1-methyl-1H-1,2,3-triazol-5-yl)pyridin-3-yl)-6-oxo-1,6-dihydropyridazine-3-carboxylic acid (4-12)**

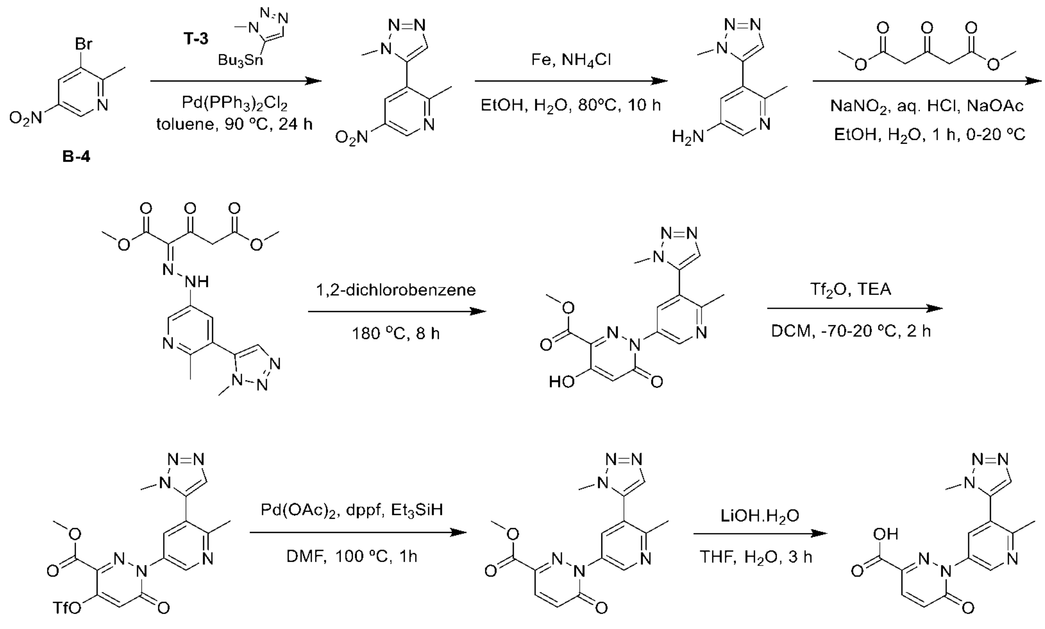


B-4

4-12

Step 1: Synthesis of 2-methyl-3-(3-methyltriazol-4-yl)-5-nitro-pyridine

A solution of 3-bromo-2-methyl-5-nitro-pyridine (10 g, 46.08 mmol), tributyl-(3-methyltriazol-4-yl)stannane (17.15 g, 46.08 mmol) and $Pd(PPh_3)_2Cl_2$ (1.62 g, 2.30 mmol) in toluene (100 mL) was substituted with nitrogen for 3 times, and then stirred at 100 °C for 16 h under nitrogen atmosphere. The reaction mixture was filtered, and then the filtrate was concentrated under reduced pressure to give a crude product. The crude product was purified by silica gel column chromatography (50% EtOAc in petroleum ether) to give 2-methyl-3-(3-methyltriazol-4-yl)-5-nitro-pyridine (7.7 g, 58.00% yield, 76.08% purity) as a yellow solid. MS (ESI) m/z = 220.1 $[M+H]^+$.

Step 2: Synthesis of 6-methyl-5-(3-methyltriazol-4-yl)pyridine-3-amine

Fe (9.81 g, 175.64 mmol) and $NH_4Cl$ (18.79 g, 351.28 mmol) were added to a solution of 2-methyl-3-(3-methyltriazol-4-yl)-5-nitro-pyridine (7.7 g, 35.13 mmol) in EtOH (150 mL) and $H_2O$ (50 mL), and then the mixture was stirred at 80 °C for 3 h under nitrogen atmosphere. The reaction mixture was filtered, and then the filtrate was concentrated under reduced pressure. After the addition of water (15 mL), the reaction mixture was extracted with EtOAc (60 mL × 3). The combined organic layers were washed with brine (20 mL x 2), dried over $Na_2SO_4$, and then concentrated under reduced pressure to give a crude product, 6-methyl-5-(3-methyltriazol-4-yl)pyridine-3-amine (5.86 g, 84.62% yield, 95.98% purity) as a yellow solid. MS (ESI) m/z = 190.1 $[M+H]^+$.

Step 3: Synthesis of dimethyl (2E)-2-[[6-methyl-5-(3-methyltriazol-4-yl)-3-pyridyllhydrazono]-3-oxo-pentanedioate

A solution of $NaNO_2$ (1.38 g, 19.98 mmol) in $H_2O$ (15 mL) was added to HCl (12 M, 8.39 mL) and 6-methyl-5-(3-methyltriazol-4-yl)pyridine-3-amine (3.78 g, 19.98 mmol) in $H_2O$ (20 mL) at 0 °C. Then dimethyl 3-oxopentanedioate (3.48 g, 19.98 mmol, 2.88 mL) in EtOH (12 mL) and NaOAc (12.00 g, 146.27 mmol) in $H_2O$ (40 mL) were added to the reaction mixture. The reaction mixture was stirred at 20 °C for 1 h. The reaction mixture was concentrated under reduced pressure, then extracted with EtOAc (30 mL × 3). The combined organic layers were washed with brine (20 mL x 2), dried over $Na_2SO_4$, and then concentrated under reduced pressure to give a crude product. The crude product was purified by silica gel column chromatography (5% MeOH in DCM) to give dimethyl (2E)-2-[[6-methyl-5-(3-methyltriazol-4-yl)-3-pyridyl] hydrazono]-3-oxo-pentanedioate (6.1 g, 70.10% yield, 85.96% purity) as yellow oil. MS (ESI) m/z = 375.1 $[M+H]^+$.

Step 4: Synthesis of methyl 4-hydroxy-1-[6-methyl-5-(3-methyltriazol-4-yl)-3-pyridyll-6-oxo-pyridazine-3-carboxylate

A mixture of dimethyl (2E)-2-[[6-methyl-5-(3-methyltriazol-4-yl)-3-pyridyl]hydrazono]-3-oxo-pentanedioate (6.1 g) in 1,2-dichlorobenzene (80 mL) was stirred at 180 °C for 8 h. The reaction mixture was filtered, and then the filtrate was concentrated under reduced pressure to give a crude product. The crude product was purified by silica gel column chromatography (4% MeOH in DCM) to give methyl 4-hydroxy-1-[6-methyl-5-(3-methyltriazol-4-yl)-3-pyridyl]-6-oxo-pyridazine-3-carboxylate (1.18 g, 15.65% yield, 74% purity) as a yellow solid. $^1$H NMR (400 MHz, DMSO-$d_6$) δ 12.22 (br s, 1H), 8.75 (d, *J* = 2.4 Hz, 1H), 8.04 (d, *J* = 2.4 Hz, 1H), 7.98 (s, 1H), 6.23 (s, 1H), 3.90 (s, 3H), 3.84 (s, 3H), 2.42 (s, 3H); MS (ESI) m/z = 343.0 $[M+H]^+$.

Step 5: Synthesis of methyl 1-[6-methyl-5-(3-methyltriazol-4-yl)-3-pyridyl]-6-oxo-4-(trifluoromethylsulfonyloxy)pyridazine-3-carboxylate

TEA (869.09 mg, 8.59 mmol, 1.20 mL) and trifluoromethylsulfonyl trifluoromethanesulfonate (1.62 g, 5.73 mmol, 944.72 μL) in DCM (5 mL) were slowly added to a mixture of methyl 4-hydroxy-1-[6-methyl-5-(3-methyltriazol-4-yl)-3-pyridyl]-6-oxo-pyridazine-3-carboxylate (0.98 g, 2.86 mmol) in DCM (15 mL) at -70 °C, and then the mixture was stirred at 20 °C for 2 h. After the addition of water (15 mL), the reaction mixture was extracted with DCM (30 mL X 3). The combined organic layers were washed with brine (10 mL x 2), dried over $Na_2SO_4$, and then concentrated under reduced pressure to give a crude product. The crude product was purified by silica gel column chromatography (50% EtOH in petroleum ether) to give methyl 1-[6-methyl-5-(3-methyltriazol-4-yl)-3-pyridyl]-6-oxo-4-(trifluoromethylsulfonyloxy)pyridazine-3-carboxylate (939 mg, 47.85% yield, 69.21% purity) as a yellow solid. MS (ESI) m/z = 475.1 $[M+H]^+$.

Step 6: Synthesis of methyl 1-[6-methyl-5-(3-methyltriazol-4-yl)-3-pyridyl]-6-oxopyridazine-3-carboxylate

A solution of methyl 1-[6-methyl-5-(3-methyltriazol-4-yl)-3-pyridyl]-6-oxo-4-(trifluoromethylsulfonyloxy)pyridazine-3-carboxylate (939 mg, 1.98 mmol), $Pd(OAc)_2$ (66.66 mg, 296.92 umol), Dppp (244.92 mg, 593.84 umol) and $Et_3SiH$ (299.22 mg, 2.57 mmol, 411.01 uL) in DMF (10 mL) was substituted with nitrogen for 3 times, and then stirred at 100 °C for 1 h under nitrogen atmosphere. After the addition of water (15 mL), the reaction mixture was extracted with EtOAc (40 mL X 3). The combined organic layers were washed with brine (20 mL x 2), dried over $Na_2SO_4$, and then concentrated under reduced pressure to give a crude product. The crude product was purified by silica gel column chromatography (3% MeOH in DCM) to give methyl 1-[6-methyl-5-(3-methyltriazol-4-yl)-3-pyridyl]-6-oxopyridazine-3-carboxylate (850 mg, 1.56 mmol, 78.96% yield, 60% purity) as a yellow solid. MS (ESI) m/z = 327.1 $[M+H]^+$.

Step 7: Synthesis of 1-[6-methyl-5-(3-methyltriazol-4-yl)-3-pyridyl]-6-oxo-pyridazine-3-carboxylic acid

$LiOH \cdot H_2O$ (173.61 mg, 4.14 mmol) was added to a solution of methyl 1-[6-methyl-5-(3-methyltriazol-4-yl)-3-pyridyl]-6-oxopyridazine-3-carboxylate (450 mg, 1.38 mmol) in THF (4 mL) and $H_2O$ (2 mL). Then the mixture was stirred at 25 °C for 1 h. After the addition of water (10 mL), the reaction mixture was extracted with EtOAc (20 mL X 3), and then the organic layers were removed. The mixture was adjusted to pH = 2-3 with aq. 1 N HCl, and then extracted with EtOAc (20 mL × 3). The combined organic layers were washed with brine (10 mL x 2), dried over $Na_2SO_4$, and then concentrated under reduced pressure to give **Intermediate 4-12** (287 mg, 48.83% yield, 73.27% purity) as a yellow solid. MS (ESI) m/z = 313.0 $[M+H]^+$.

**Preparation Example 43-1: 1-[5-(3-ethyltriazol-4-yl)-6-methyl-3-pyridyl]-6-oxo-pyridazine-3-carboxylic acid (4-13)**

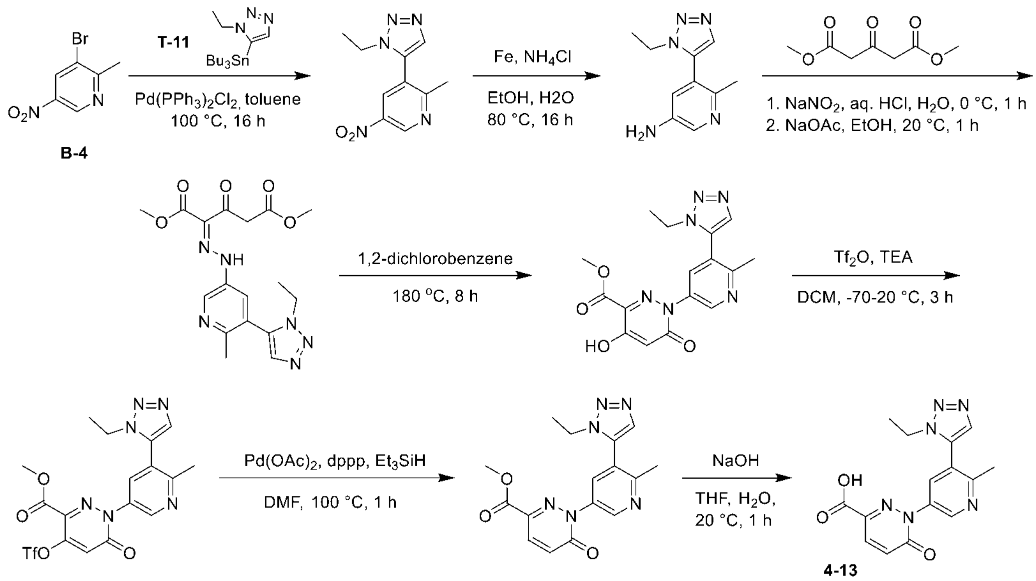

B-4

4-13

Intermediate T-11 was concentrated in the same manner as in Preparation Example 43 to give **Intermediate 4-13** (55 mg, 55.45% yield, 67.66% purity) as a white solid. MS (ESI) m/z = 327.0 $[M+H]^+$.

**Preparation Example 44: 1-(6-chloropyridazin-4-yl)-6-oxo-pyridazine-3-carboxylic acid (4-14)**

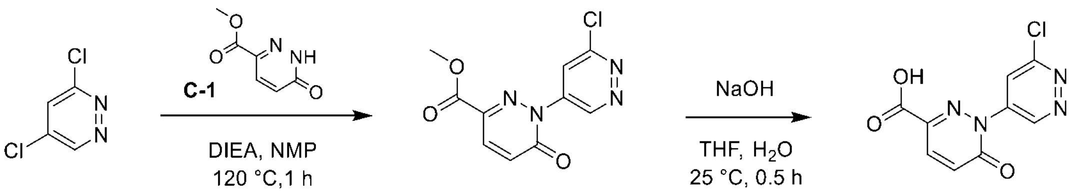

B-5 3-16 4-14

Step 1: Synthesis of methyl 1-(6-chloropyridazin-4-yl)-6-oxo-pyridazine-3-carboxylate

A solution of 3,5-dichloropyridazine (5 g, 33.56 mmol), methyl 6-oxo-1H-pyridazine-3-carboxylate (5.17 g, 33.56 mmol) and DIEA (21.69 g, 167.81 mmol, 29.23 mL) in NMP (5 mL) was stirred in microwave at 120 °C for 1 h. After the addition of water (100 mL), the reaction mixture was extracted with EtOAc (100 mL X 3). The combined organic layers were washed with brine (100 mL), dried over $Na_2SO_4$, and then concentrated under reduced pressure to give a crude product. The crude product was purified by silica gel column chromatography (30% EtOAc in petroleum ether) to give methyl 1-(6-chloropyridazin-4-yl)-6-oxo-pyridazine-3-carboxylate **(Intermediate 3-16;** 1.92 g, 19.95% yield, 93% purity) as a yellow solid. $^1H$ NMR (400 MHz, DMSO-$d_6$) δ 9.67 (d, *J* = 2.0 Hz, 1H), 8.32 (d, *J* = 2.0 Hz, 1H), 7.97 (d, *J* = 10.0Hz, 1H), 7.28 (d, *J* = 10.0 Hz, 1H), 3.92 (s, 3H); MS (ESI) m/z = 266.9 $[M+H]^+$.

Step 2: Synthesis of 1-(6-chloropyridazin-4-yl)-6-oxo-pyridazine-3-carboxylic acid

NaOH (174.00 mg, 4.35 mmol) was added to a solution of methyl 1-(6-chloropyridazin-4-yl)-6-oxo-pyridazine-3-carboxylate (580 mg, 2.18 mmol) in THF (6 mL), $H_2O$ (2 mL). Then the mixture was stirred at 20 °C for 0.5 h. The reaction mixture was concentrated under reduced pressure to give **Intermediate 4-14** (540 mg, 84.42% yield, 85.90% purity) as a yellow solid. MS (ESI) m/z = 253.1 $[M+H]^+$.

**Preparation Example 45: 1-[6-(2-methylpyrazol-3-yl)pyridazin-4-yl]-6-oxo-pyridazine-3-carboxylic acid (4-15)**

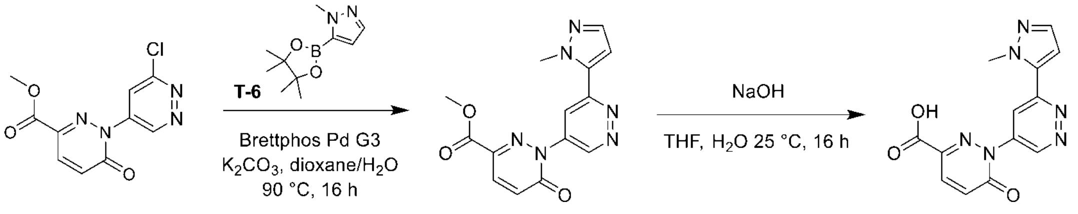


3-16 3-17 4-15

Step 1: Synthesis of methyl 1-[6-(2-methylpyrazol-3-yl)pyridazin-4-yl]-6-oxo-pyridazine-3-carboxylate

A solution of Intermediate 3-16 (300 mg, 1.13 mmol), 1-methyl-5-(4,4,5,5-tetramethyl-1,3,2-dioxaborolan-2-yl) pyrazole (351.14 mg, 1.69 mmol), BrettPhos Pd G3 (101.99 mg, 112.51 μmol) and $K_2CO_3$ (310.99 mg, 2.25 mmol) in dioxane (5 mL) and $H_2O$ (0.5 mL) was substituted with nitrogen for 3 times, and then it was stirred at 90 °C for 3 h under nitrogen atmosphere. After the addition of water (30 mL), the reaction mixture was extracted with EtOAc (30 mL X 3). The combined organic layers were washed with brine (50 mL), dried over $Na_2SO_4$, and then concentrated under reduced pressure to give a crude product. The crude product was purified by silica gel column chromatography (30% EtOAc in petroleum ether) to give methyl 1-[6-(2-methylpyrazol-3-yl)pyridazin-4-yl]-6-oxo-pyridazine-3-carboxylate (67 mg, 17.77% yield, 93.2% purity) as a yellow solid. MS (ESI) m/z = 313.1 $[M+H]^+$.

Step 2: Synthesis of 1-[6-(2-methylpyrazol-3-yl)pyridazin-4-yl]-6-oxo-pyridazine-3-carboxylic acid

NaOH (17.16 mg, 429.10 μmol) and $H_2O$ (1 mL) were added to a solution of methyl 1-[6-(2-methylpyrazol-3-yl) pyridazin-4-yl]-6-oxo-pyridazine-3-carboxylate (67 mg, 214.55 μmol) in THF (2 mL). Then the mixture was stirred at 25 °C for 2 h. The reaction mixture was concentrated under reduced pressure to give **Intermediate 4-15** (60 mg, 45.76% yield, 48.8% purity) as a white solid. MS (ESI) m/z = 299.0 $[M+H]^+$.

**Preparation Example 46: 1-[6-(3-methyltriazol-4-yl)pyrazin-2-yl]-6-oxo-pyridine-3-carboxylic acid (4-27)**

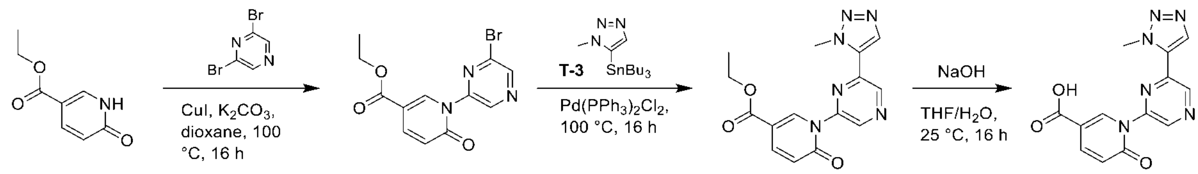


C-2 4-27

Step 1: Synthesis of ethyl 1-(6-bromopyrazin-2-yl)-6-oxo-pyridine-3-carboxylate

A solution of ethyl 6-oxo-1H-pyridine-3-carboxylate (5.46 g, 32.65 mmol), 2,6-dibromopyrazine (9.32 g, 39.18 mmol), CuI (1.24 g, 6.53 mmol), $Cs_2CO_3$ (10.64 g, 32.65 mmol) and 2-(dimethylamino)acetic acid (3.37 g, 32.65 mmol) in dioxane (80 mL) was degassed and purged with $N_2$ for 3 times. The mixture was stirred at 100 °C for 16 h under $N_2$ atmosphere. The reaction mixture was diluted with water and extracted with EtOAc (50 mL X 3). The combined organic layers were washed with brine (50 mL), dried over anhydrous $Na_2SO_4$, and then concentrated under reduced pressure to give a crude product. The crude product was purified by silica column chromatography (20% EtOAc in PE) to give ethyl 1-(6-bromopyrazin-2-yl)-6-oxo-pyridine-3-carboxylate (237 mg, 2.13% yield, 95.32% purity) as a yellow solid. $^1H$ NMR (400MHz, DMSO-$d_6$) δ 9.12 (s, 1H), 9.01 (s, 1H), 8.61 (d, *J* = 2.4 Hz, 1H), 7.94 (dd, *J* = 2.4, 9.6 Hz, 1H), 6.64 (d, *J* = 10.0 Hz, 1H), 4.29 (q, *J* = 7.2 Hz, 2H), 1.29 (t, *J* = 7.2 Hz, 3H); MS (ESI) m/z = 323.9 $[M+H]^+$.

Step 2: Synthesis of ethyl 1-[6-(3-methyltriazol-4-yl)pyrazin-2-yl]-6-oxo-pyridine-3-carboxylate

A solution of ethyl 1-(6-bromopyrazin-2-yl)-6-oxo-pyridine-3-carboxylate (150 mg, 462.78 μmol), tributyl-3-methyltriazol-4-yl)stannane (223.88 mg, 601.61 μmol) and $Pd(PPh_3)_2Cl_2$ (32.48 mg, 46.28 μmol) in dioxane (4 mL) was degassed and substituted with nitrogen for 3 times, and then it was stirred at 100 °C for 16 h under nitrogen atmosphere. The reaction mixture was filtered, and then the filtrate was concentrated under reduced pressure to give ethyl 1-[6-(3-methyltriazol-4-yl)pyrazin-2-yl]-6-oxo-pyridine-3-carboxylate (104 mg, 68.87% yield, 100% purity) as a yellow solid. $^1H$

NMR (400MHz, DMSO-$d_6$) δ 9.29 (s, 1H), 9.15 (s, 1H), 8.79 (d, *J* = 2.6 Hz, 1H), 8.56 (s, 1H), 7.95 (dd, *J* = 2.6, 9.6 Hz, 1H), 6.67 (d, *J* = 9.6 Hz, 1H), 4.34 (s, 3H), 4.28 (q, *J* = 7.2 Hz, 2H), 1.31 - 1.28 (m, 3H); MS (ESI) m/z = 327.0 $[M+H]^+$.

Step 3: Synthesis of 1-[6-(3-methyltriazol-4-yl)pyrazin-2-yll-6-oxo-pyridine-3-carboxylic acid

Ethyl 1-[6-(3-methyltriazol-4-yl)pyrazin-2-yl]-6-oxo-pyridine-3-carboxylate was used in the same manner as in Step 3 of Preparation Example 36 to give **Intermediate 4-27** (45 mg, 96.55% yield, 98.06% purity) as a yellow solid. MS (ESI) m/z = 299.1 $[M+H]^+$.

**Preparation Example 46-1: 1-[5-(3-cyclopropyltriazol-4-yl)-3-pyridyl]-6-oxo-pyridine-3-carboxylic acid (4-78)**

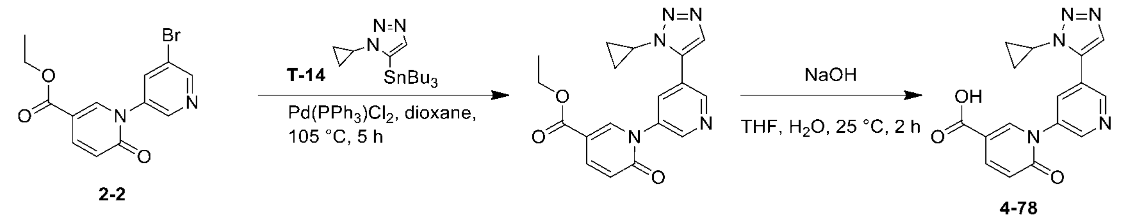


2-2

4-78

Intermediates 2-2 and T-14 were used in the same manner as in Steps 2 and 3 of Preparation Example 36 to give **Intermediate 4-78** (120 mg, 93.23% yield, 96.8% purity) as a yellow solid. MS (ESI) m/z = 323.9 $[M+H]^+$.

**Preparation Example 47: 1-[5-(3-cyclopropyltriazol-4-yl)-3-pyridyl]-6-oxo-pyridazine-3-carboxylic acid (4-47)**

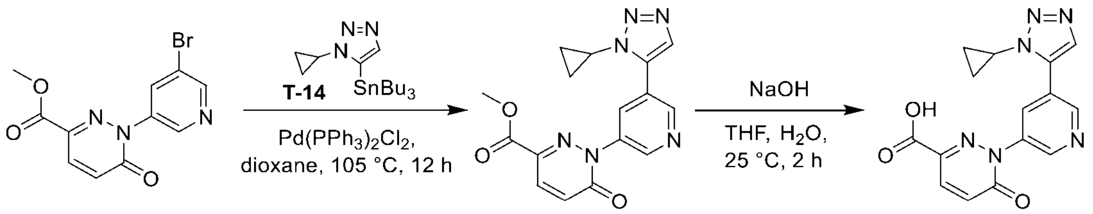


2-1

4-47

Intermediates 2-1 and T-14 were used in the same manner as in Steps 2 and 3 of Preparation Example 36 to give **Intermediate 4-47** (35 mg, 88.82% yield, 97.3% purity) as a yellow solid. $^1H$ NMR (400 MHz, DMSO-$d_6$) δ 9.03 (d, *J* = 2.0 Hz, 1H), 8.95 (d, *J* = 2.4 Hz, 1H), 8.56 (t, *J* = 2.0 Hz, 1H), 8.17 (s, 1H), 7.96 (d, *J* = 9.6 Hz, 1H), 7.22 (d, *J* = 9.6 Hz, 1H), 4.01 (td, *J* = 3.6, 7.3 Hz, 2H), 1.20 - 1.15 (m, 2H), 1.12 (br d, *J* = 2.8 Hz, 2H); MS (ESI) m/z = 324.9 $[M+H]^+$.

**Preparation Example 47-1: 1-[5-(3-isopropyltriazol-4-yl)-3-pyridyl]-6-oxo-pyridazine-3-carboxylic acid (4-32)**

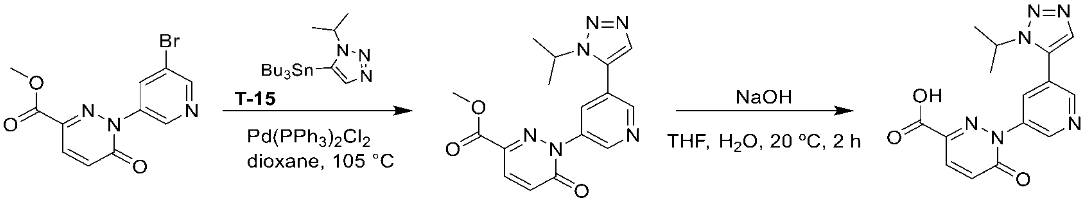


2-1

4-32

Intermediates 2-1 and T-15 were used in the same manner as in Steps 2 and 3 of Preparation Example 36 to give **Intermediate 4-32** (55 mg, 93.99% yield, 98.31 % purity) as a yellow solid. MS (ESI) m/z = 326.6 $[M+H]^+$.

**Preparation Example 48: 1-(5-bromo-2-fluoro-3-pyridyl)-6-oxo-pyridine-3-carboxylic acid (4-73)**

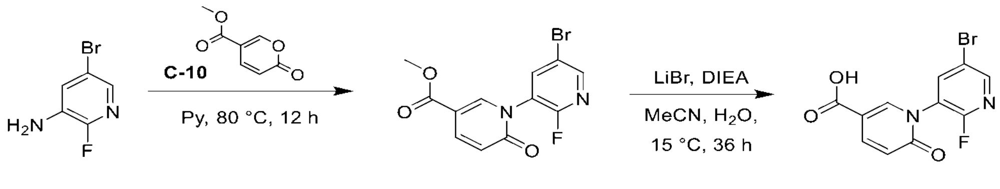


4-73

Step 1: Synthesis of methyl 1-(5-bromo-2-fluoro-3-pyridyl)-6-oxo-pyridine-3-carboxylate

5-bromo-2-fluoro-pyridine-3-amine (5 g, 26.18 mmol) was added to a solution of methyl 6-oxopyran-3-carboxylate **(C-10;** 8.07 g, 52.36 mmol) in pyridine (100 mL). The mixture was stirred at 80 °C for 12 h under nitrogen atmosphere. The reaction mixture was filtered, and then the filtrate was concentrated under reduced pressure to give a crude product. The crude product was purified by silica column chromatography (12% EtOAc in PE) to give a residue. The residue was dissolved in MeOH (5 mL) and purified by prep-HPLC (column: Welch Xtimate C18 250*50mm*10μm;mobile phase: [water($NH_4HCO_3$)-MeCN]; 5%-55% B, 20 minutes). Most of MeCN was removed under reduced pressure, and the remaining solvent was removed by lyophilization to give methyl 1-(5-bromo-2-fluoro-3-pyridyl)-6-oxo-pyridine-3-carboxylate (360 mg, 4.12% yield, 98.08% purity) as a yellow-white solid. MS (ESI) m/z = 326.8 $[M+H]^+$.

Step 2: Synthesis of 1-(5-bromo-2-fluoro-3-pyridyl)-6-oxo-pyridine-3-carboxylic acid

The intermediate, methyl 1-(5-bromo-2-fluoro-3-pyridyl)-6-oxo-pyridine-3-carboxylate was used in the same manner as in Step 2 of Preparation Example 36-9 to give **Intermediate 4-73** (60 mg, 60.43% yield, 96.39% purity) as a white solid. MS (ESI) m/z =313.0 $[M+H]^+$.

**Preparation Example 49: 1-(6-chloropyridazin-4-yl)-N-[(1R)-1-[2-fluoro-3-(trifluoromethyl)phenyl]ethyl]-6-oxo-pyridazine-3-carboxamide (D-1)**

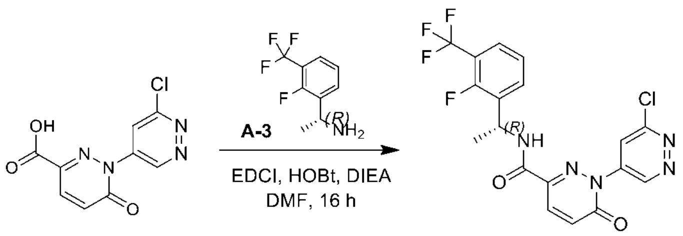


4-14 D-1

DIEA (752.07 mg, 5.82 mmol, 1.01 mL), HOBt (393.14 mg, 2.91 mmol) and EDCI (557.77 mg, 2.91 mmol) were added to a solution of 1-(6-chloropyridazin-4-yl)-6-oxo-pyridazine-3-carboxylic acid (490 mg, 1.94 mmol) and Intermediate A-3 (378.06 mg, 1.55 mmol, HCl salt) in DMF (6 mL). The mixture was degassed and purged with $N_2$ for 3 times, and then stirred at 25 °C for 5 h under $N_2$ atmosphere. After the addition of water (8 mL), the reaction mixture was extracted with EtOAc (20 mL X 3). The combined organic layers were washed with brine (10 mL x 2), dried over anhydrous $Na_2SO_4$, and then concentrated under reduced pressure to give a crude product. The crude product was purified by silica gel column chromatography (3% MeOH in DCM) to give **Intermediate D-1** (140 mg, 11.33% yield, 69.37% purity) as brown oil. MS (ESI) m/z = 442.1 $[M+H]^+$.

**Preparation Example 49-1: 1-(6-chloropyridazin-4-yl)-N-[(1R)-1-[2-fluoro-3-(difluoromethyl)phenyl]ethyl]-6-oxo-pyridazine-3-carboxamide (D-2)**

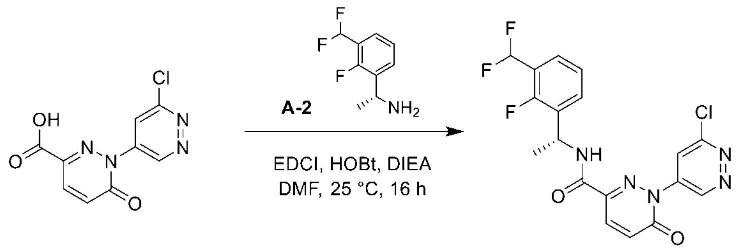


4-14 D-2

Intermediate A-2 was used in the same manner as in Preparation Example 49 to give **Intermediate D-2** (37.0 mg, 30.83% yield) as a white solid. $^{1}$H NMR (400 MHz, DMSO-$d_6$) δ 9.97 (d, *J* = 2.0 Hz, 1H), 9.21 (d, *J* = 8.0 Hz, 1H), 8.75 (d, *J* = 2.0 Hz, 1H),8.46 (s, 1H), 7.94 *(d, J* = 10.0 Hz, 1H), 7.72 (br t, *J* = 7.2 Hz, 1H), 7.57 -7.51 (m, 1H), 7.35 (d, *J* = 8.0 Hz, 1H), 7.28 (d, *J* = 10.0 Hz, 1H), 7.22 (t, *J* = 7.2 Hz, 1 H), 5.44 (quint, *J* = 7.2 Hz, 1H), 4.43 (s, 3H), 1.55 *(d, J* = 7.2 Hz, 3H); MS (ESI) m/z = 471.2 [M+H]$^+$.

**Preparation Example 49-2: 1-(5-bromo-2-fluoro-3-pyridyl)-N-[(1R)-1-[2-fluoro-3-(trifluoromethyl)phenyl] ethyl]-6-oxo-pyridazine-3-carboxamide (D-10)**

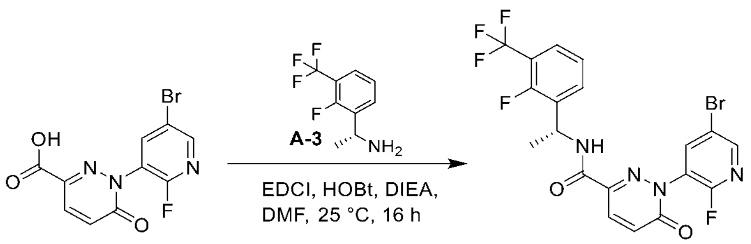


4-30 D-10

Intermediates A-3 and 4-30 were used in the same manner as in Preparation Example 49 to give **Intermediate D-10** (30 mg, 55.80% yield, 93.46% purity) as a yellow solid. $^{1}$H NMR (400 MHz, $CDCl_3$) δ 8.42 (s, 1H), 8.10 (dd, *J* = 2.4, 7.6 Hz, 1H), 8.05 (m, 1H), 7.54 (t, *J* = 7.2 Hz, 2H), 7.35 (m, 1H), 7.26 - 7.20 (m, 1H), 7.12 (dd, *J* = 1.2, 9.6 Hz, 1H), 5.43 (m, 1H), 1.62 (d, *J* = 7.2 Hz, 3H); MS (ESI) m/z = 504.9 [M+H]$^+$.

**Preparation Example 49-3: 1-(5-bromo-3-pyridyl)-N-[(1R)-1-[2-fluoro-3-[1-(hydroxymethyl)cyclopropyl]phenyl] ethyl]-6-oxo-pyridazine-3-carboxamide (D-11)**

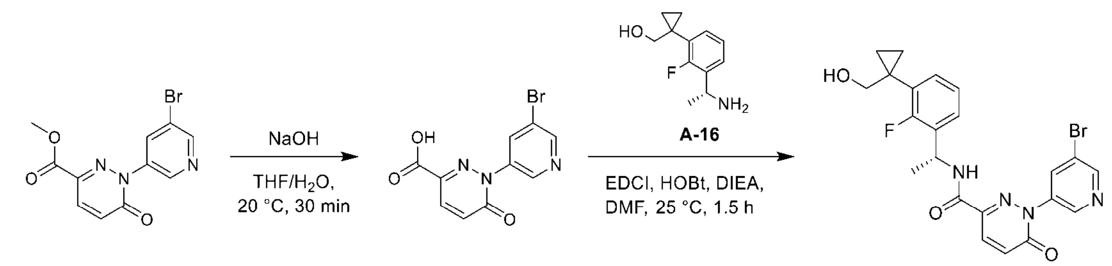


2-1 4-67 D-11

Step 1: Synthesis of 1-(5-bromo-3-pyridyl)-6-oxo-pyridazine-3-carboxylic acid

Intermediate 2-1 was used in the same manner as in Step 2 of Preparation Example 30 to give **Intermediate 4-67** (1.85 g, 92.04% yield, 95% purity) as a light yellow solid. MS (ESI) m/z = 295.9 [M+H]$^+$.

Step 2: Synthesis of 1-(5-bromo-3-pyridyl)-N-[(1R)-1-[2-fluoro-3-[1-(hydroxymethyl)cyclopropyl]phenyl]ethyl]-6-oxo-pyridazine-3-carboxamide

Intermediates 4-67 and A-16 were used in the same manner as in Preparation Example 49 to give **intermediate D-11** (240 mg, 30.80% yield, 89.82% purity) as a yellow solid. MS (ESI) m/z = 489.0 $[M+H]^+$.

**Preparation Example 49-4: 1-(5-bromo-3-pyridyl)-N-[(1R)-1-[2-fluoro-3-(trifluoromethyl)phenyl]ethyl]-6-oxo-pyridazine-3-carboxamide (D-12)**

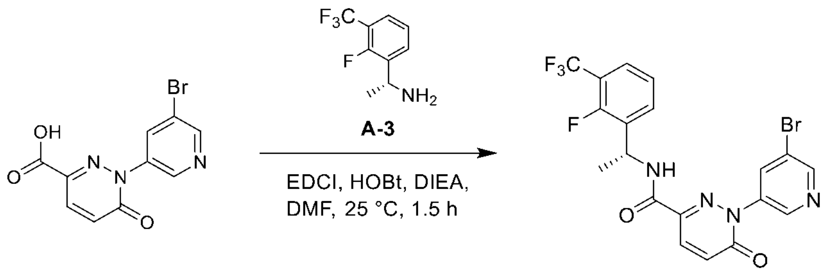


4-67 D-12

Intermediates 4-67 and A-3 were used in the same manner as in Preparation Example 49 to give **Intermediate D-12** (34.3 g, 71.01% yield, 95% purity) as a light yellow solid. $^1$H NMR (400 MHz, DMSO-$d_6$) δ 9.14 (d, *J* = 7.6 Hz, 1H), 9.01 (d, *J* = 2.0 Hz, 1H), 8.83 (d, *J* = 2.0 Hz, 1H), 8.54 (t, *J* = 2.0 Hz, 1H), 7.90 (d, *J* = 9.6 Hz, 1H), 7.83 (t, *J* = 7.2 Hz, 1H), 7.68 (t, *J* = 6.8 Hz, 1H), 7.41 (t, *J* = 7.6 Hz, 1H), 7.20 (d, *J* = 9.6 Hz, 1H), 5.43 (quin, *J* = 7.2 Hz, 1H), 1.52 (d, *J* = 7.2 Hz, 3H); MS (ESI) m/z = 486.8 $[M+H]^+$.

**Preparation Example 49-5: 1-(5-bromo-3-pyridyl)-N-[(1R)-1-[3-(1,1-difluoro-2-hydroxy-ethyl)-2-fluorophenyl]ethyl]-6-oxo-pyridazine-3-carboxamide (D-13)**

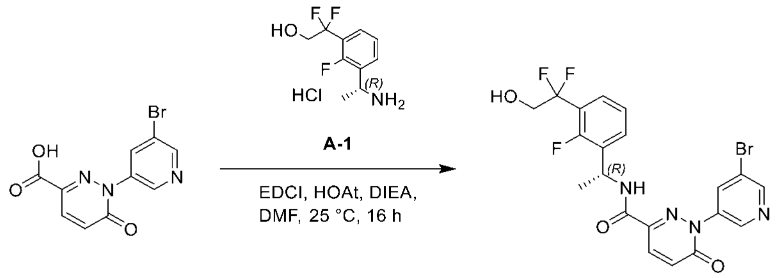


4-67 D-13

Intermediates A-1 and 4-67 were used in the same manner as in Preparation Example 49 to give **Intermediate D-13** (280 mg, 49.57% yield, 89.2% purity) as a white solid. MS (ESI) m/z = 498.8 $[M+H]^+$.

**Preparation Example 49-6: 1-(5-bromo-2-fluoro-3-pyridyl)-N-[(1R)-1-[2-fluoro-3-(trifluoromethyl)phenyl]ethyl]-6-oxo-pyridine-3-carboxamide (D-14)**

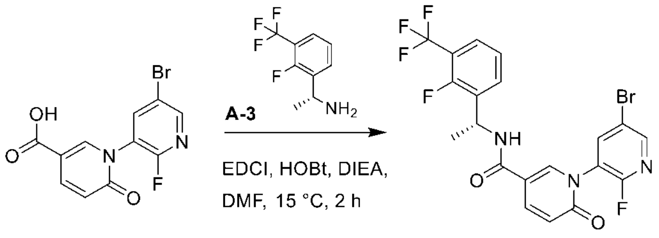


4-73 D-14

Intermediates A-3 and 4-73 were used in the same manner as in Preparation Example 49 to give **Intermediate D-14** (30 mg, 55.80% yield, 93.46% purity) as a yellow solid.

**Preparation Example 49-7: 1-(6-chloropyridazin-4-yl)-N-[(1R)-1-[3-(1,1-difluoro-2-hydroxy-ethyl)-2-fluoro-phenyl]ethyl]-6-oxo-pyridazine-3-carboxamide (D-15)**

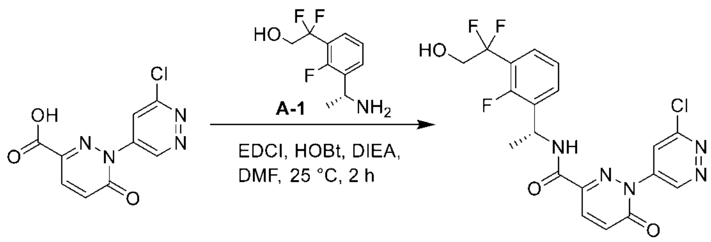


4-14 D-15

Intermediates A-1 and 4-14 were used in the same manner as in Preparation Example 49 to give **Intermediate D-15** (66 mg, 13.65% yield, 26% purity) as a yellow solid.

**Preparation Example 49-8: 1-(5-bromo-2-fluoropyridin-3-yl)-N-[(1R)-1-[3-(1,1-difluoro-2-hydroxy-ethyl)2-fluoro-phenyl]ethyl]-6-oxo-pyridazine-3-carboxamide (D-16)**

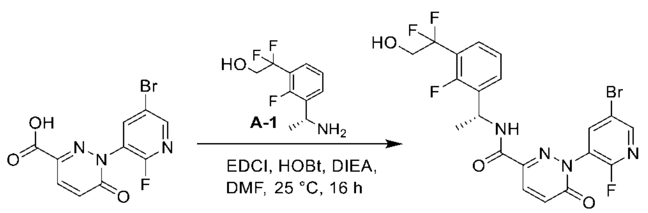


4-30 D-16

Intermediates A-1 and 4-30 were used in the same manner as in Preparation Example 49 to give **Intermediate D-16** (10 mg, 21.30% yield, 90.84% purity) as colorless oil. $^1$H NMR (400 MHz, MeOD) δ 8.48 (t, *J* = 2.8 Hz, 2H), 8.01 (d, *J* = 10.0 Hz, 1H), 7.55 (br t, *J* = 7.2 Hz, 1H), 7.48 (br t, *J* = 6.8 Hz, 1H), 7.23 (t, *J* = 7.6 Hz, 1H), 7.18 (d, *J* = 10.0 Hz, 1H), 5.48 (q, *J* = 7.2 Hz, 1H), 4.00 (t, *J* = 13.6 Hz, 2H), 1.55 *(d, J* = 7.2 Hz, 3H); MS (ESI) m/z = 516.8 $[M+H+1]^+$.

**Preparation Example 49-9: N-{(R)-1-[3-(1,1-difluoro-2-hydroxyethyl)-2-fluorophenyl]ethyl}-5-bromo-1-[5-(1-methyl-1H-1,2,3-triazol-5-yl)-2-fluoropyridin-3-yl]-6-oxo-1,6-dihydropyridazine-3-carboxamide (D-17)**

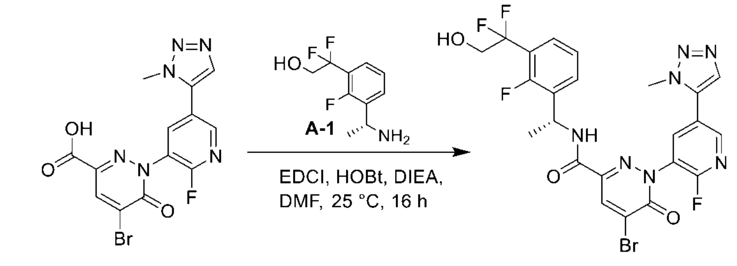


4-82 D-17

Intermediates A-1 and 4-82 were used in the same manner as in Preparation Example 49 to give **Intermediate D-17** (45 mg, 55.80% yield, 93.46% purity) as a yellow solid. MS (ESI) m/z = 596.1 [M+H] +.

**Preparation Example 50: 1-[6-(3-methyltriazol-4-yl)pyridazin-4-yl]-6-oxo-pyridazine-3-carboxylic acid (4-22)**

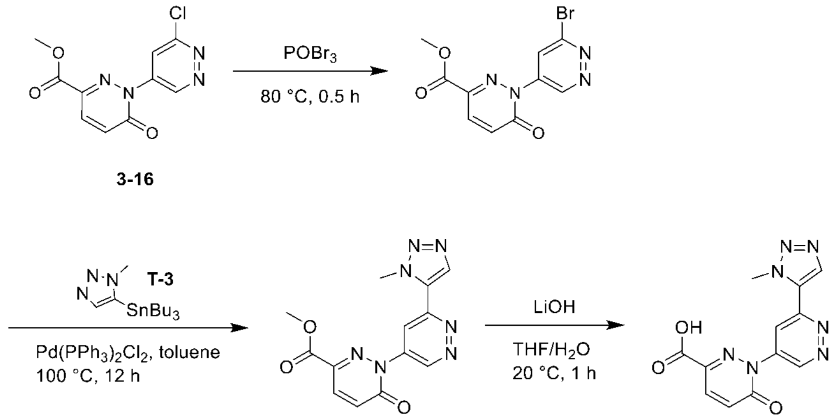


3-16

3-35 4-22

Step 1: Synthesis of methyl 1-(6-bromopyridazin-4-yl)-6-oxo-pyridazine-3-carboxylate

A solution of methyl 1-(6-chloropyridazin-4-yl)-6-oxo-pyridazine-3-carboxylic acid (250 mg, 937.59 μmol) in phosphoryl bromide (709.22 μL, 6.98 mmol) was stirred at 80 °C for 0.5 h. After the addition of water (10 mL) slowly, the reaction mixture was extracted with EtOAc (20 mL X 3). The combined organic layers were washed with brine (10 mL x 2), dried over $Na_2SO_4$, and then concentrated under reduced pressure to give methyl 1-[6-(3-methyltriazol-4-yl)pyridazin-4-yl]-6-oxo-pyridazine-3-carboxylate (145 mg, 42.75% yield, 86% purity) as black oil. MS (ESI) m/z = 311.0 [M+H] +.

Step 2: Synthesis of methyl 1-[6-(3-methyltriazol-4-yl)pyridazin-4-yl]-6-oxo-pyridazine-3-carboxylate

A solution of methyl 1-(6-bromopyridazin-4-yl)-6-oxo-pyridazine-3-carboxylate (145 mg, 466.10 μmol), tributyl-3-methyltriazol-4-yl)stannane (346.91 mg, 932.20 μmol) and $Pd(PPh_3)_2Cl_2$ (65.43 mg, 93.22 μmol) in toluene (4 mL) was degassed and substituted with nitrogen for 3 times, and then stirred at 100 °C for 12 h under nitrogen atmosphere. The reaction mixture was filtered, and then the filtrate was concentrated under reduced pressure to give methyl 1-[6-(3-methyltriazol-4-yl)pyridazin-4-yl]-6-oxo-pyridazine-3-carboxylate (146 mg, crude product) as black oil. MS (ESI) m/z = 314.1 [M+H] +

Step 3: Synthesis of 1-[6-(3-methyltriazol-4-yl)pyridazin-4-yl]-6-oxo-pyridazine-3-carboxylic acid

$LiOH \cdot H_2O$ (19.56 mg, 466.05 μmol) and $H_2O$ (1 mL) were added to a solution of methyl 1-[6-(3-methyltriazol-4-yl) pyridazin-4-yl]-6-oxo-pyridazine-3-carboxylate (146 mg, 466.05μmol) in THF (2 mL), and then the mixture was stirred at 20 °C for 1 h. The reaction mixture was adjusted to pH = 3-4 with aq. 1 N HCl, and extracted with EtOAc (20 mL × 3). The combined organic layers were washed with $H_2O$ (20 mL) and brine (10 mL x 3), dried over anhydrous $Na_2SO_4$, and then concentrated under reduced pressure to give **Intermediate 4-22** (130 mg, 57.79% yield, 62% purity) as black oil. MS (ESI) m/z = 300.1 [M+H] +.

**Preparation Example 51: 1-[5-[3-(2-benzyloxyethyl)triazol-4-yl]-3-pyridyl]-6-oxo-pyridazine-3-carboxylic acid (4-42)**

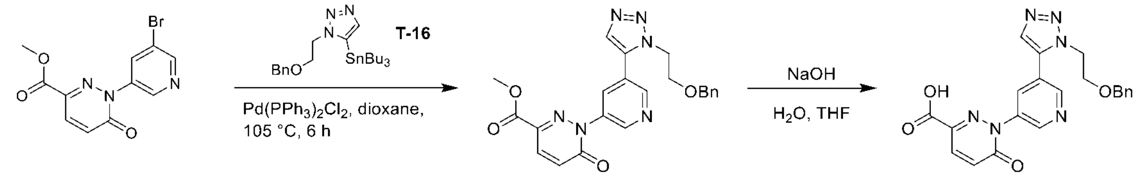


**2-1** **4-42**

Intermediates 2-1 and T-16 were used in the same manner as in Preparation Example 30 to give **Intermediate 4-42** (70 mg, 34.36% yield, 38% purity) as a brown solid. MS (ESI) m/z = 419.2 [M+H]$^+$.

**Preparation Example 52: methyl 5-isopropenyl-1-[5-(3-methyltriazol-4-yl)-3-pyridyl]-6-oxo-pyridazine-3-carboxylate (4-72)**

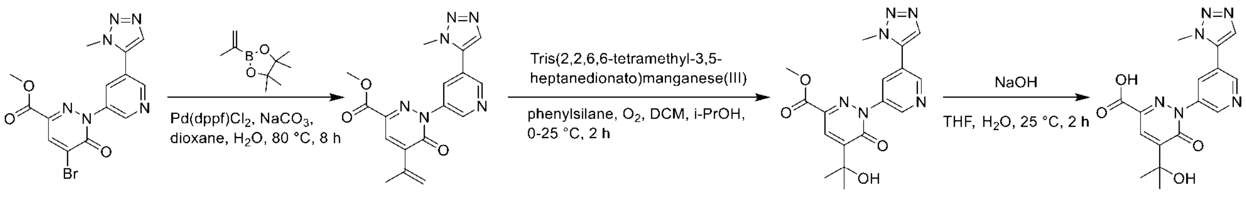


**3-8** **4-72**

Step 1: Synthesis of methyl 5-isopropenyl-1-[5-(3-methyltriazol-4-yl)-3-pyridyl]-6-oxo-pyridazine-3-carboxylate

Pd(dppf)$Cl_2$ (18.71 mg, 25.56 μmol), $Na_2CO_3$ (54.19 mg, 511.27 μmol) and 2-isopropenyl-4,4,5,5-tetramethyl-1,3,2-dioxaborolane (128.87 mg, 766.91 μmol) were added to a solution of 5-bromo-1-[5-(3-methyltriazol-4-yl)-3-pyridyl]-6-oxo-pyridazine-3-carboxylate (100 mg, 255.64 μmol) in dioxane (5 mL) and water (0.5 mL). The mixture was stirred at 80 °C for 3 h under nitrogen atmosphere. The reaction mixture was filtered, and then the filtrate was concentrated under reduced pressure to give a crude product. The crude product was purified by silica gel column chromatography (1% MeOH in DCM) to give methyl 5-isopropenyl-1-[5-(3-methyltriazol-4-yl)-3-pyridyl]-6-oxo-pyridazine-3-carboxylate (90 mg, 71.40% yield, 71.46% purity) as a yellow solid. MS (ESI) m/z = 353.0 [M+H] $^+$.

Step 2: Synthesis of methyl 5-(1-hydroxy-1-methyl-ethyl)-1-[5-(3-methyltriazol-4-yl)-3-pyridyl]-6-oxo-pyridazine-3-carboxylate

Phenylsilane (55.28 mg, 510.86 μmol, 63.04 μL) and tris[(Z)-1-tert-butyl-4,4-dimethyl-3-oxo-pent-1-enoxy] manganese (15.45 mg, 25.54 μmol) were added to a solution of methyl-5-isopropenyl-1-[5-(3-methyltriazol-4-yl)-3-pyridyl]-6-oxo-pyridazine-3-carboxylate (90 mg, 255.43 μmol) in DCM (1 mL) and i-PrOH (4 mL). The mixture was stirred at 0 °C for 5 h under oxygen atmosphere. The reaction mixture was filtered, and then the filtrate was concentrated under reduced pressure to give a crude product. The crude product was purified by silica gel column chromatography (1% MeOH in DCM) to give methyl 5-(1-hydroxy-1-methyl-ethyl)-1-[5-(3-methyltriazol-4-yl)-3-pyridyl]-6-oxo-pyridazine-3-carboxylate (60 mg, 55.26% yield, 87.14% purity) as a yellow solid. MS (ESI) m/z = 370.9 [M+H] $^+$.

Step 3: Synthesis of methyl 5-isopropenyl-1-[5-(3-methyltriazol-4-yl)-3-pyridyl]-6-oxo-pyridazine-3-carboxylate

The intermediate, methyl 5-(1-hydroxy-1-methyl-ethyl)-1-[5-(3-methyltriazol-4-yl)-3-pyridyl]-6-oxo-pyridazine-3-carboxylate was used in the same manner as in Step 4 of Preparation Example 28 to give **Intermediate 4-72** (50 mg, 81.01% yield, 93.528% purity) as a yellow solid. MS (ESI) m/z = 356.9 [M+H] $^+$.

**Preparation Example 53: 3-bromo-2-fluoro-5-(4,4,5,5-tetramethyl-1,3,2-dioxaborolan-2-yl)pyridine (P-7)**

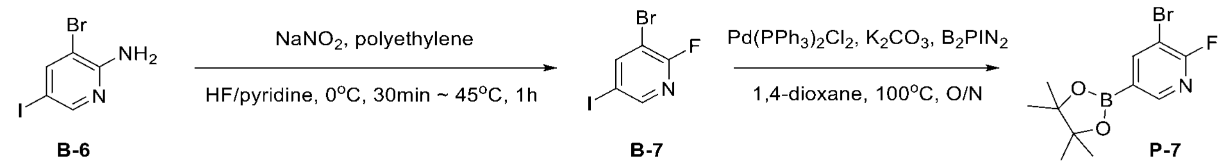


B-6 B-7 P-7

Step 1: Synthesis of 3-bromo-2-fluoro-5-iodopyridine

Sodium nitrite (402 mg, 5.82 mmol) was added dropwise to 3-bromo-5-iodopyridine-2-amine (1.5 g, 5.02 mmol) in HF/pyridine mixture (21 mL) at 0 °C in a polyethylene reaction vessel. The reaction mixture was stirred at 0 °C for 0.5 h, and then it was warmed to 45 °C and stirred for 1 h. After the addition of ice, the reaction mixture was adjusted to pH = 5 with aq. $NaHCO_3$, and then extracted with ether. The combined organic layers were washed with water, dried over $Na_2SO_4$, and then concentrated under reduced pressure to give a crude product. The crude product was purified by silica gel column chromatography to give 3-bromo-2-fluoro-5-iodopyridine (1.32 g, 87%) as a white solid. $^1H$ NMR (400 MHz, $CDCl_3$) δ 8.34-8.33 (m, 1H), 8.25 (dd, $J$ = 8.0, 2.0 Hz, 1H).

Step 2: Synthesis of 3-bromo-2-fluoro-5-(4,4,5,5-tetramethyl-1,3,2-dioxaborolan-2-yl)pyridine

Bis(pinacolato)diboron (1.22 g, 4.82 mmol) and $K_2CO_3$ (1.21 g, 8.76 mmol) were added to a solution of 3-bromo-2-fluoro-5-iodopyridine (1.32 g, 4.38 mmol) in dioxane (10 mL), and then the mixture was substituted with nitrogen for 3 times. Bis(triphenylphosphine)palladium(II) dichloride (154 mg, 0.219 mmol) was added and stirred at 100 °C for 18 h under nitrogen atmosphere. The reaction mixture was diluted with EtOAc, and then the combined organic layers were washed with aq. $NH_4Cl$, water and brine, dried over $Na_2SO_4$, and then concentrated under reduced pressure to give a crude product. The crude product was purified by silica gel column chromatography to give **Intermediate P-7** (1.04 g, 79%) as a white solid. $^1H$ NMR (400 MHz, $CDCl_3$) δ 8.47 (s, 1H), 8.32 (d, $J$ = 9.2 Hz, 1H), 1.35 (s, 12H). MS (ESI) m/z = 302.0, 304.0 $[M+H]^+$.

**Preparation Example 53-1: 3-bromo-2-methoxy-5-(4,4,5,5-tetramethyl-1,3,2-dioxaborolan-2-yl)pyridine (P-8)**

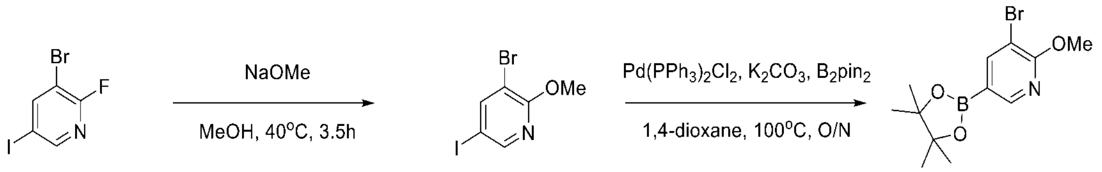


B-7 P-8

Step 1: Synthesis of 3-bromo-5-iodo-2-methoxypyridine

NaOMe (43 mg) was added to a solution of 3-bromo-2-fluoro-5-iodopyridine (200 mg, 0.663 mmol) in methanol (2.2 mL) in portions. Then the mixture was stirred at room temperature for 3.5 h. The reaction mixture was concentrated under reduced pressure. The crude product was diluted with water and extracted with EtOAc. The combined organic layers were washed with brine, dried over anhydrous $Na_2SO_4$, and then concentrated under reduced pressure to give 3-bromo-5-iodo-2-methoxypyridine (200 mg, 96%) as a gray solid. $^1H$ NMR (400 MHz, $CDCl_3$) δ 8.26 (d, $J$ = 2.0 Hz, 1H), 8.05 (d, $J$ = 2.0 Hz, 1H), 3.98 (s, 3H).

Step 2: Synthesis of 3-bromo-2-methoxy-5-(4,4,5,5-tetramethyl-1,3,2-dioxaborolan-2-yl)pyridine

Bis(pinacolato)diboron (178 mg, 0.701 mmol) and potassium carbonate (176 mg, 1.27 mmol) were added to a solution of 3-bromo-5-iodo-2-methoxypyridine (200 mg, 0.637 mmol) in dioxane (2 mL). The mixture was degassed and purged with nitrogen. The mixture was added with bis(triphenylphosphine)palladium(II)dichloride (22 mg, 0.0319 mmol), and stirred at 100 °C for 16.5 h under nitrogen atmosphere. The mixture was cooled to room temperature and diluted with

EtOAc. The mixture was washed with sat. aq. $NH_4Cl$, water and brine. The organic layers were dried over anhydrous $Na_2SO_4$, and then concentrated under reduced pressure. It was purified by silica column chromatography to give **Intermediate P-8** (90 mg, 45%) as a white solid. $^1H$ NMR (400 MHz, $CDCl_3$) δ 8.44 (d, *J* = 1.6 Hz, 1H), 8.15 (d, *J* = 1.6 Hz, 1H), 4.04 (s, 3H), 1.33 (s, 12H); MS (ESI) m/z = 314.0 $[M+H]^+$.

**Preparation Example 53-2: *N,N*-dimethyl-[3-bromo-5-(4,4,5,5-tetramethyl-1,3,2-dioxaborolan-2-yl)-2-pyridyl] amine (P-9)**

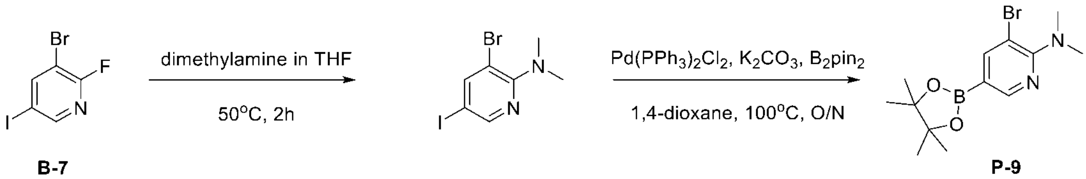


B-7 P-9

Step 1: Synthesis of *N,N*-dimethyl(3-bromo-5-iodo-2-pyridyl)amine

3-bromo-2-fluoro-5-iodopyridine (100 mg, 0.331 mmol) was added to a solution of dimethylamine (2M in THF, 0.83 mL), and the mixture was stirred at 50 °C for 2 h under nitrogen atmosphere. The resultant was cooled to room temperature, and then extracted with EtOAc and water. The organic layers were washed with water and brine, dried over anhydrous $Na_2SO_4$, and then concentrated under reduced pressure to give *N,N*-dimethyl-(3-bromo-5-iodo-2-pyridyl) amine (108 mg, 100%) as a yellow solid. It was used in the next reaction without further purification. $^1H$ NMR (400 MHz, $CDCl_3$) δ 8.30 (d, *J* = 2.0 Hz, 1H), 7.98 (d, *J* = 2.0 Hz, 1H), 2.98 (s, 6H); MS (ESI) m/z =326.7 $[M+H]^+$, 328.7 $[M+3H]^+$.

Step 2: Synthesis of *N,N*-dimethyl-[3-bromo-5-(4,4,5,5-tetramethyl-1,3,2-dioxaborolan-2-yl)-2-pyridyl]amine

The intermediate, N,N-dimethyl-(3-bromo-5-iodo-2-pyridyl)amine (108 mg, 0.330 mmol) was used in the same manner as in Step 2 of Preparation Example 53 to give **Intermediate P-9** (57 mg, 53%) as a brown solid. MS (ESI) m/z = 327.0 $[M+H]^+$, 329.0 $[M+3H]^+$.

**Preparation Example 54: (R)-1-(5-bromo-6-fluoropyridin-3-yl)-*N*-(1-(2-fluoro-3-trifluoromethyl)phenyl)ethyl)-6-oxo-1,6-dihydropyridazine-3-carboxamide (D-3)**

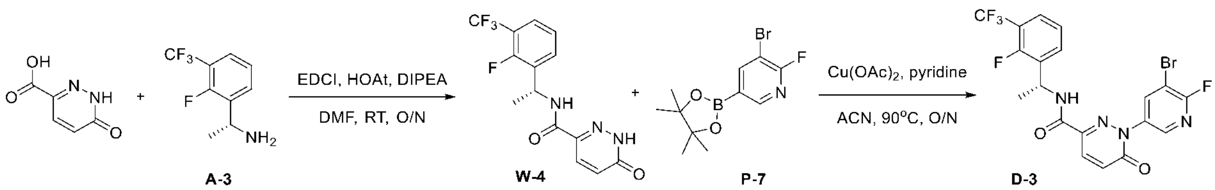


A-3 W-4 P-7 D-3

Step 1: Synthesis of (R)-N-(1-(2-fluoro-3-trifluoromethyl)phenyl)ethyl)-6-oxo-1,6-dihydropyridazine-3-carboxamide

EDCI (205 mg, 1.07 mmol), HOAt (146 mg, 1.07 mmol) and DIEA (0.37 mL, 2.14 mmol) were added to a solution of 6-oxo-1,6-dihydropyridazine-3-carboxylic acid (100 mg, 0.714 mmol) and Intermediate A-3 (174 mg, 0.714 mmol, HCl) in DMF (3 mL). The mixture was degassed and purged with $N_2$ for 3 times, and then stirred at 25 °C for 18 h under $N_2$ atmosphere. The reaction mixture was poured into water and extracted with EtOAc. The combined organic layers were washed with brine for 5 times, dried over anhydrous $Na_2SO_4$, and then concentrated under reduced pressure to give a crude product. The crude product was purified by silica gel column chromatography to give a main product, (R)-N-(1-(2-fluoro-3-trifluoromethyl)phenyl)ethyl)-6-oxo-1,6-dihydropyridazine-3-carboxamide (164 mg, 70% yield) as a white solid. $^1H$ NMR (400 MHz, $CDCl_3$) δ 12.39 (br, 1H), 8.04 (d, *J* = 10.0 Hz, 1H), 7.56-7.52 (m, 3H), 7.23 (t, *J* = 7.6 Hz, 1H), 7.06 (d, *J* = 10.0 Hz, 1H), 5.44 (quint, *J* = 7.3, 14.7 Hz, 1H), 1.63 (d, *J* = 7.2 Hz, 3H); MS (ESI) m/z = 330.1 $[M+H]^+$.

Step 2: Synthesis of (R)-1-(5-bromo-6-fluoropyridin-3-yl)-*N*-(1-(2-fluoro-3-trifluoromethyl)phenyl)ethyl)-6-oxo-1,6-dihydropyridazine-3-carboxamide

To a solution of (R)-N-(1-(2-fluoro-3-trifluoromethyl)phenyl)ethyl)-6-oxo-1,6-dihydropyridazine-3-carboxamide (164 mg, 0.498 mmol) in MeCN (8 mL) was added 3-bromo-2-fluoro-5-(4,4,5,5-tetramethyl-1-1,3,2-dioxoboron-2-yl) pyridine (301 mg, 0.996 mmol), $Cu(OAc)_2$ (91 mg, 0.498 mmol) and pyridine (0.16 mL, 1.99 mmol), and the mixture was stirred at 90 °C for 19 h. After the addition of water, the reaction mixture was extracted with EtOAc. The combined organic layers were washed with copper sulfate(II) pentahydrate, dried over $Na_2SO_4$, and then concentrated under reduced pressure to give a crude product. The crude product was purified by silica gel column chromatography to give **Intermediate D-3** (219 mg, 87% yield, 99.3% purity) as a white solid. $^1H$ NMR (400 MHz, $CDCl_3$) δ 8.46-8.45 (m, 1H), 8.37 (dd, *J* = 7.4, 2.6 Hz, 1H), 8.04 (d, *J* = 9.6 Hz, 1H), 7.55 (t, *J* = 7.4 Hz, 2H), 7.36 (d, *J* = 8.0 Hz, 1H), 7.24-7.22 (m, 1H), 7.13 (d, *J* = 9.6 Hz, 1H), 5.43 (quint, *J* = 7.3, 14.7 Hz, 1H), 1.64 (d, *J* = 7.2 Hz, 3H); MS (ESI) m/z = 503.0 $[M+H]^+$, 505.0 $[M+3H]^+$.

**Preparation Example 54-1: (R)-1-(5-bromo-6-fluoropyridin-3-yl)-*N*-(1-(3-difluoromethyl)-2-fluorophenyl) ethyl)-6-oxo-1,6-dihydropyridazine-3-carboxamide (D-4)**

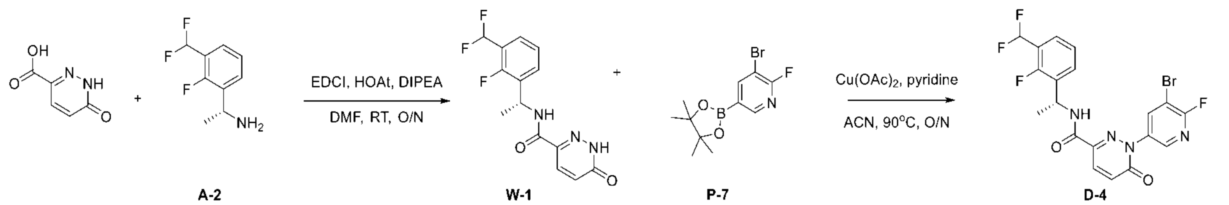


A-2 W-1 P-7 D-4

Commercially available 6-oxo-1,6-dihydropyridazine-3-carboxylic acid and Intermediate A-2 were used in the same manner as in Preparation Example 54 to give **Intermediate D-4** (87 mg, 37% 2Step yield) as a white solid. $^1H$ NMR (400 MHz, $CDCl_3$) δ 8.46-8.45 (m, 1H), 8.37 (dd, *J* = 7.6, 2.4 Hz, 1H), 8.05 (d, *J* = 10.0 Hz, 1H), 7.54 (t, *J* = 7.2 Hz, 1H), 7.46 (t, *J* = 7.2 Hz, 1H), 7.26-7.22 (m, 1H), 7.13 (d, *J* = 9.6 Hz, 1H), 6.90 (t, *J* = 55.0 Hz, 1H), 5.43 (quint, *J* = 7.4, 15.1 Hz, 1H), 1.63 (d, *J* = 7.2 Hz, 3H); MS (ESI) m/z = 484.9 $[M+H]^+$, 487.0 $[M+3H]^+$.

**Preparation Example 54-2: *N*-{(R)-1-[2-fluoro-3-(trifluoromethyl)phenyl]ethyl}-5-bromo-1-[5-(1-methyl-1H-1,2,3-triazol-5-yl)-3-pyridyl]-6-oxo-1,6-dihydropyridazine-3-carboxamide (D-5)**

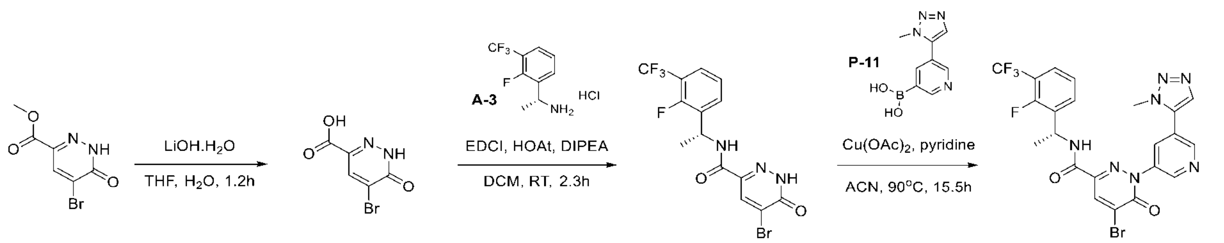


C-6 W-3 D-5

Step 1: Synthesis of 5-bromo-6-oxo-1,6-dihydro-3-pyridazinecarboxylic acid

$LiOH \cdot H_2O$ (94 mg, 2.23 mmol) was added to a solution of methyl 5-bromo-6-oxo-1,6-dihydro-3-pyridazinecarboxylate **(C-6,** 260 mg, 1.12 mmol) in THF (11.2 mL) and $H_2O$ (5.6 mL), and the mixture was stirred at 25 °C for 1.2 h. The reaction mixture was poured into water, and the aqueous layer was adjusted to pH = 4 with 1N-HCl, and then extracted with EtOAc for 3 times. The combined organic layers were washed with brine, dried over anhydrous $Na_2SO_4$, and then concentrated under reduced pressure to give 5-bromo-6-oxo-1,6-dihydro-3-pyridazinecarboxylic acid (215 mg, 88%) as a yellow solid. MS (ESI) m/z = 218.9 $[M+H]^+$, 220.9 $[M+3H]^+$.

Step 2: Synthesis of *N*-{(R)-1-[2-fluoro-3-(trifluoromethyl)phenyllethyl}-5-bromo-6-oxo-1,6-dihydropyridazine-3-carboxamide

Intermediate A-3 was used in the same manner as in Step 1 of Preparation Example 54 to give **Intermediate W-3** (120 mg, 30%) as a yellow solid. MS (ESI) m/z = 408.0 $[M+H]^+$, 409.9 $[M+3H]^+$.

Step 3: *N*-{(R)-1-[2-fluoro-3-(trifluoromethyl)phenyl]ethyl}-5-bromo-1-[5-(1-methyl-1H-1,2,3-triazol-5-yl)-3-pyridyl]-6-oxo-1,6-dihydropyridazine-3-carboxamide

Intermediates W-3 and P-11 were used in the same manner as in Step 2 of Preparation Example 54 to give **Intermediate D-5** (101 mg, 61%) as a light pink solid. $^1$H NMR (400 MHz, $CDCl_3$) δ 8.96 (s, 1H), 8.81 (s, 1H), 8.48 (s, 1H), 8.20 (t, *J* = 2.2 Hz, 1H), 7.89 (s, 1H), 7.57-7.53 (m, 2H), 7.39 (d, *J* = 8.0 Hz, 1H), 7.24-7.22 (m, 1H), 5.43 (quin, *J* = 7.4, 14.9 Hz, 1H), 4.19 (s, 3H), 1.66 (d, *J* = 6.4 Hz, 3H); MS (ESI) m/z = 566.2 $[M+H]^+$, 569.2 $[M+3H]^+$.

**Preparation Example 54-3: *N*-{(R)-1-[3-(difluoromethyl)-2-fluorophenyl]ethyl}-5-bromo-1-[5-(1-methyl-1H-1,2,3-triazol-5-yl)-3-pyridyl]-6-oxo-1,6-dihydropyridazine-3-carboxamide (D-7)**

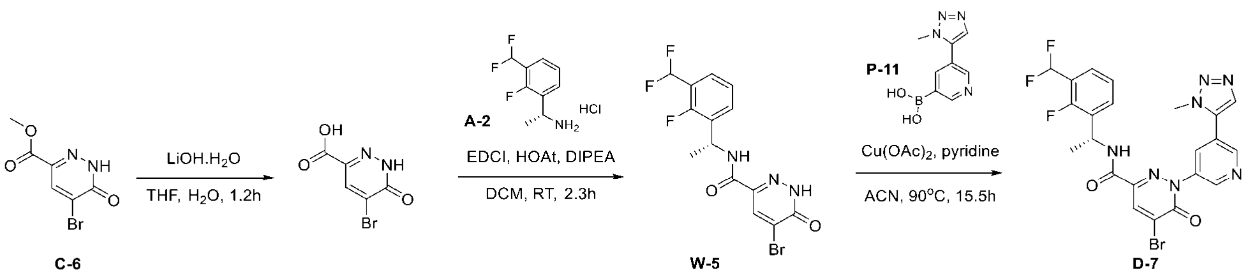


Intermediate C-6 was used in the same manner as in Preparation Example 54-2 to give **Intermediate D-7** (80 mg, 36%) as a brown solid. MS (ESI) m/z = 548.1 $[M+H]^+$, 550.1 $[M+3H]^+$.

**Preparation Example 54-4: (R)-N-(1-(3-(2-((tert-butyldimethylsilyl)oxy)-1,1-difluoroethyl)-2-fluorophenyl) ethyl)-6-oxo-1,6-dihydroxypyridazine-3-carboxamide (W)**

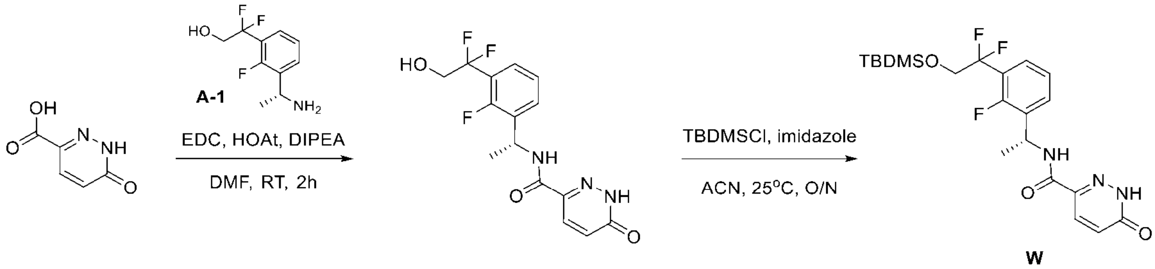


Step 1: Synthesis of N-[(1R)-1-[3-(1,1-difluoro-2-hydroxyethyl)-2-fluorophenyl]ethyl]-6-oxo-1,6-dihydropyridazine-3-carboxamide

Commercially available 6-oxo-1,6-dihydropyridazine-3-carboxylic acid and Intermediate A-1 were used in the same manner as in Step 1 of Preparation Example 54 to give the intermediate, N-[(1R)-1-[3-(1,1-difluoro-2-hydroxyethyl)-2-fluorophenyl]ethyl]-6-oxo-1,6-dihydropyridazine-3-carboxamide (281 mg, 58% yield) as a white solid. $^1$H NMR (400 MHz, DMSO-$d_6$) δ 13.44 (br, 1H), 8.98 (d, *J* = 8.4 Hz, 1H), 7.77 (d, *J* = 10.0 Hz,1H), 7.65 (t, *J* = 7.0 Hz, 1H), 7.43 (t, *J* = 6.8 Hz, 1H), 7.28 (t, *J* = 7.8 Hz, 1H), 6.95 (d, *J* = 9.6 Hz, 1H), 5.72 (t, *J* = 6.4 Hz, 1H), 5.38 (quin, *J* = 7.1, 14.5 Hz, 1H), 3.91 (td, *J* = 14.4, 6.4 Hz, 2H), 1.48 (d, *J* = 7.2 Hz, 3); MS (ESI) m/z = 342.01 $[M+H]^+$.

Step 2: Synthesis of (R)-N-(1-(3-(2-((tert-butyldimethylsilyl)oxy)-1,1-difluoroethyl)-2-fluorophenyl)ethyl)-6-oxo-1,6-dihydroxypyridazine-3-carboxamide

TBDMSCl (149 mg, 0.988 mmol) and imidazole (84 mg, 1.24 mmol) were added to a solution of N-[(1R)-1-[3-(1,1-difluoro-2-hydroxyethyl)-2-fluorophenyl]ethyl]-6-oxo-1,6-dihydropyridazine-3-carboxamide (281 mg, 0.823 mmol) in MeCN (9 mL) at room temperature, and the mixture was stirred for 14 h. The mixture was diluted with EtOAc, quenched with 10% aq. $NaHCO_3$, and then extracted. The organic layers were dried over anhydrous $Na_2SO_4$, filtered, and then concentrated under reduced pressure. The crude product was purified by silica column chromatography to give **Intermediate W** (300 mg, 80% yield) as a yellow-white solid. $^1H$ NMR (400 MHz, DMSO-d6) δ 13.41 (br, 1H), 8.94 (d, *J* = 8.0 Hz, 1H), 7.76 (d, *J* = 9.6 Hz, 1H), 7.66 (t, *J* = 7.0 Hz, 1H), 7.42 (t, *J* = 6.8 Hz, 1H), 6.93 (d, *J* = 9.6 Hz, 1H), 5.37 (quin, *J* = 7.2, 16.9 Hz, 1H), 4.12 (t, *J* = 13.4 Hz, 2H), 1.47 (d, *J* = 7.2 Hz, 3H), 0.73 (s, 9H), -0.05 (d, *J* = 2.8 Hz, 6H); MS (ESI) m/z = 456.07 $[M+H]^+$.

**Preparation Example 54-5: (*R*)-5-(cyclohex-1-en-1-yl)-N-(1-(2-fluoro-3-(trifluoromethyl)phenyl)ethyl)-6-oxo-1,6-dihydropyridine-3-carboxamide (W-2)**

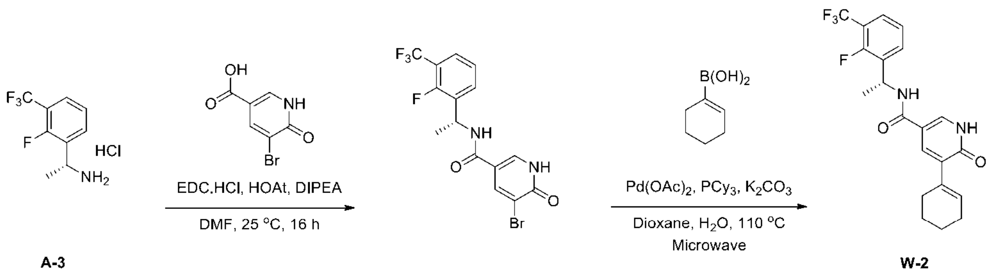


Step 1: Synthesis of (R)-5-bromo-N-(1-(2-fluoro-3-(trifluoromethyl)phenyl)ethyl)-6-oxo-1,6-dihydropyridine-3-carboxamide

Commercially available 5-bromo-6-oxo-1,6-dihydronicotinic acid and Intermediate A-3 were used in the same manner as in Step 1 of Preparation Example 54 to give the intermediate, (R)-5-bromo-N-(1-(2-fluoro-3-(trifluoromethyl)phenyl)ethyl)-6-oxo-1,6-dihydropyridine-3-carboxamide (250 mg, 75 % yield) as a yellow-white solid. $^1H$ NMR (400 MHz, DMSO-$d_6$) δ 8.79 (d, *J* = 7.2 Hz, 1H), 8.38 (d, *J* = 2.4 Hz, 1H), 8.12 (d, *J* = 2.4 Hz, 1H), 7.76 (t, *J* = 6.8 Hz, 1H), 7.66 (t, *J* = 7.2 Hz, 1H), 7.39 (t, *J* = 7.8 Hz, 1H), 5.33 (quin, *J* = 7.0 Hz, 1H), 1.46 (d, *J* = 6.8 Hz, 3H); MS (ESI) m/z = 407.01 $[M+H]^+$, 4.8.99 $[M+3H]^+$.

Step 2: Synthesis of (R)-5-(cyclohex-1-en-1-yl)-N-(1-(2-fluoro-3-(trifluoromethyl)phenyl)ethyl)-6-oxo-1,6-dihydropyridine-3-carboxamide

$K_2CO_3$ (64.1 mg, 0.463 mmol), $Pd(OAc)_2$ (5.3 mg, 0.0232 mmol) and $PCy_3$ (13 mg, 0.00463 mmol) were added to a solution of (R)-5-bromo-N-(1-(2-fluoro-3-(trifluoromethyl)phenyl)ethyl)-6-oxo-1,6-dihydropyridine-3-carboxamide and (1-cyclohexen-1-yl)boronic acid (88 mg, 0.695 mmol) in dioxane (1.2 mL) and $H_2O$ (0.23 mL), and the mixture was stirred at 110 °C for 1 h using microwave. The reaction mixture was poured into water and extracted with EtOAc. The combined organic layers were dried over anhydrous $Na_2SO_4$, and then concentrated under reduced pressure to give a crude product. The crude product was filtered by silica column chromatography (DCM: MeOH = 20: 1 to 10: 1) to give **Intermediate W-2** (64 mg, 49% yield) as an ivory solid. MS (ESI) m/z = 567.32 $[M+H]^+$.

**Preparation Example 54-6: *N*-{(R)-1-[2-fluoro-3-(trifluoromethyl)phenyl]ethyl}-5-bromo-1-[5-(1-methyl-1H-1,2,3-triazol-5-yl)-3-pyridyl]-6-oxo-1,6-dihydropyridazine-3-carboxamide (D-6)**

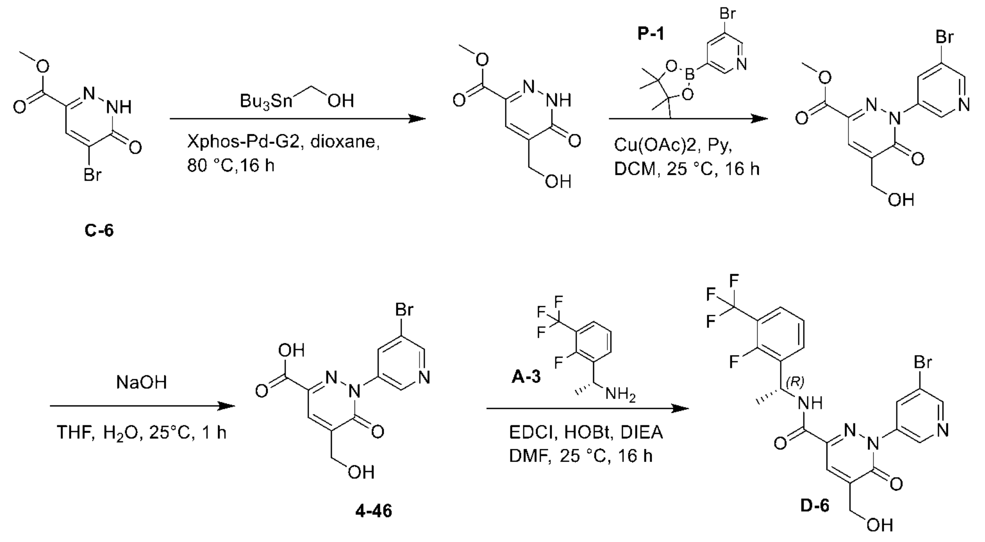


C-6

4-46

D-6

Step 1: Synthesis of methyl 5-(hydroxymethyl)-6-oxo-1*H*-pyridazine-3-carboxylate

A solution of methyl 5-bromo-6-oxo-1*H*-pyridazine-3-carboxylate (10 g, 42.91 mmol), tributylstanylmethanol (27.56 g, 85.83 mmol), [2-(2-aminophenyl)phenyl]-chloro-palladium;dicyclohexyl-[3-(2,4,6-triisopropylphenyl)phenyl] phosphan (3.38 g, 4.29 mmol) in dioxane (150 mL) was substituted with nitrogen for 3 times, and then stirred at 80 °C for 16 h under nitrogen atmosphere. The reaction mixture was concentrated under reduced pressure to give a crude product. The crude product was purified by silica gel column chromatography (2% MeOH in DCM) to give methyl 5-(hydroxymethyl)-6-oxo-1*H*-pyridazine-3-carboxylate (3.4 g, 40.44% yield, 94% purity) as a brown solid. MS (ESI) m/z = 184.9 $[M+H]^+$.

Step 2: Synthesis of methyl 1-(5-bromo-3-pyridyl)-5-(hydroxymethyl)-6-oxo-pyridazine-3-carboxylate

A solution of methyl 5-(hydroxymethyl)-6-oxo-1*H*-pyridazine-3-carboxylate (1.2 g, 6.52 mmol), 3-bromo-5-(4,4,5,5-tetramethyl-1,3,2-dioxaborolan-2-yl)pyridine (2.22 g, 7.82 mmol), $Cu(OAc)_2$ (1.18 g, 6.52 mmol) and pyridine (1.58 mL, 19.55 mmol) in DCM (30 mL) was stirred at 25 °C for 16 h under oxygen atmosphere. The reaction mixture was filtered, and then the filtrate was concentrated under reduced pressure to give a crude product. The crude product was purified by silica gel chromatography (2% MeOH in DCM) to give methyl 1-(5-bromo-3-pyridyl)-5-(hydroxymethyl)-6-oxo-pyridazine-3-carboxylate (220 mg, 8.39% yield, 84.5% purity) as a white solid. MS (ESI) m/z = 341.8 $[M+H]^+$.

Step 3: Synthesis of 1-(5-bromo-3-pyridyl)-5-(hydroxymethyl)-6-oxo-pyridazine-3-carboxylic acid

NaOH (38.81 mg, 970.22 μmol) was added to a solution of methyl 1-(5-bromo-3-pyridyl)-5-(hydroxymethyl)-6-oxo-pyridazine-3-carboxylate (220 mg, 646.81 μmol) in THF (4 mL) and $H_2O$ (0.5 mL). The reaction mixture was stirred at 20 °C for 1 h. The mixture was adjusted to pH = 6-7 with aq. 1N HCl and concentrated under reduced pressure to give **Intermediate 4-46** (200 mg, 65.90% yield, 69.5% purity) as a yellow solid. MS (ESI) m/z = 327.9 $[M+H]^+$.

Step 4: Synthesis of 1-(5-bromo-3-pyridyl)-5-(hydroxymethyl)-6-oxo-N-[rac-(1R)-1-[2-fluoro-3-(trifluoromethyl)phenyl] ethyl]pyridazine-3-carboxamide

Intermediates 4-46 and A-3 were used in the same manner as in Preparation Example 36 to give **Intermediate D-6** (200 mg, 349.26 μmol, 56.95% yield, 89.98% purity) as a yellow solid. MS (ESI) m/z = 516.9 $[M+H]^+$.

**Preparation Example 54-7: *N*-{(R)-1-[3-(trifluoromethyl)-2-fluorophenyl]ethyl}-5-bromo-1-[5-(1-cyclopropyltriazol-5-yl)-3-pyridyl]-6-oxo-1,6-dihydropyridazine-3-carboxamide (D-18)**

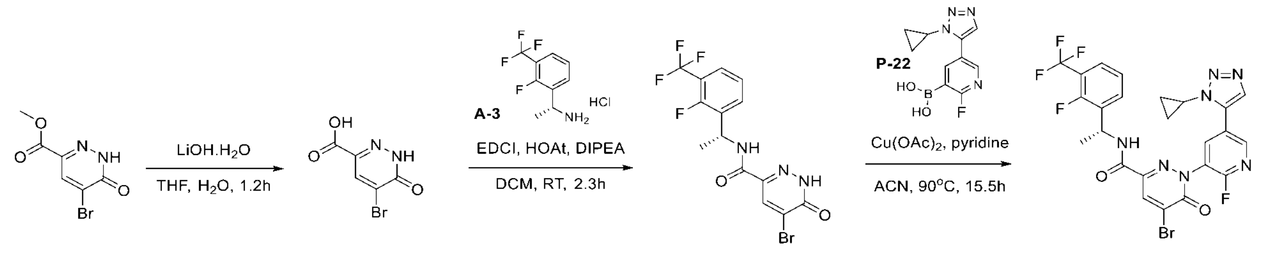


C-6 W-3 D-18

Intermediates C-6 and P-22 were used in the same manner as in Preparation Example 54-2 to give **Intermediate D-18** (80 mg, 36%) as a brown solid. MS (ESI) m/z = 584.1 $[M+H]^+$, 586.3 $[M+3H]^+$.

**Preparation Example 54-8: *N*-{(R)-1-[3-(trifluoromethyl)-2-fluorophenyl]ethyl}-5-bromo-1-[5-(1-methyl-1H-1,2,3-triazol-5-yl)-3-pyridyl]-6-oxo-1,6-dihydropyridazine-3-carboxamide (D-19)**

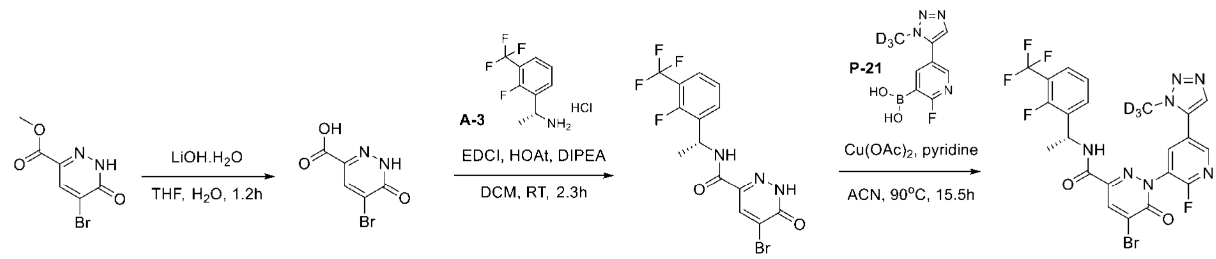


C-6 W-3 D-19

Intermediates C-6 and P-21 were used in the same manner as in Preparation Example 54-2 to give **Intermediate D-19** (80 mg, 36%) as a brown solid. MS (ESI) m/z = 584.1 $[M+H]^+$, 586.3 $[M+3H]^+$.

**[Examples]**

**Example 1: N-[(1R)-1-[3-(1,1-difluoro-2-hydroxy-ethyl)-2-fluoro-phenyl]ethyl]-1-[5-(3-methyltriazol-4-yl)-3-pyridyl]-6-oxo-pyridazine-3-carboxamide**

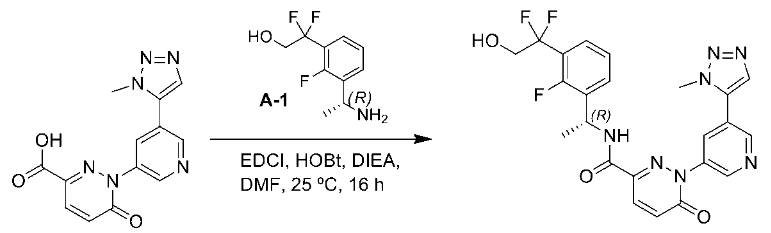


4-1 Example 1

To a solution of Intermediate 4-1 (725 mg, 2.43 mmol) and Intermediate A-1 (497.17 mg, 1.94 mmol, HCl salt) in DMF (8 mL), HOBt (492.69 mg, 3.65 mmol), DIEA (942.49 mg, 7.29 mmol, 1.27 mL) and EDCI (698.98 mg, 3.65 mmol) were added. The mixture was degassed and purged with $N_2$ for 3 times, and then stirred at 25 °C for 16 h under $N_2$ atmosphere. The reaction mixture was poured into water (10 mL) and extracted with EtOAc (30 mL×3). The combined organic layers were washed with brine (10 mL×3), dried over anhydrous $Na_2SO_4$ and concentrated under reduced pressure to give a crude product. The crude product was purified by silica gel column chromatography (2% MeOH in DCM) to give **a compound of Example 1** (1074.1 mg, 89.30% yield) as a white solid. $^1$H NMR (400 MHz, DMSO-$d_6$) δ 9.13 (d, *J* = 2.4 Hz, 1H), 9.08 (d, *J* = 8.0 Hz, 1H), 8.93 (d, *J* = 2.0 Hz, 1H), 8.52 (t, *J* = 2.0 Hz, 1H), 8.14 (s, 1H), 7.93 (d, *J* = 9.6 Hz, 1H), 7.65 (t, *J* = 7.2 Hz, 1H), 7.47 - 7.41 (m, 1H), 7.31 - 7.26 (m, 1H), 7.23 (d, J = 9.6 Hz, 1H), 5.72 (t, *J* = 6.4 Hz, 1H), 5.42 (quint, *J* = 7.2 Hz, 1H), 4.17 (s, 3H), 3.91 (dt, *J* = 6.4, 14.4 Hz, 2H), 1.49 (d, *J* = 7.2 Hz, 3H); MS (ESI) m/z = 500.1 $[M+H]^+$.

**Examples 1-1 to 1-72**

The compounds in Table 1 below were prepared in the same manner as in Example 1 through one-step amide coupling using the corresponding intermediates described in the Preparation Examples.

[Table 1]

| No. | Chemical Name/Structure | Spectra (LCMS / $^1$H NMR) |
|---|---|---|
| 1-1 | N-[(1R)-1-[3-(difluoromethyl)-2-fluoro-phenyl]ethyl]-1-[5-(3-methyltriazol-4-yl)-3-pyridyl]-6-oxo-pyridazine-3-carboxamide 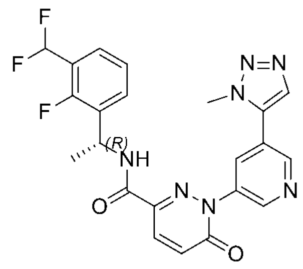 | **LCMS:** $t_R$ = 0.540 min, MS (ESI) m/z = 470.2 [M+H]$^+$. **$^1$H NMR** (400 MHz, DMSO-$d_6$) δ = 9.12 (d, *J* = 2.4 Hz, 1H), 9.08 (d, *J* = 8.0 Hz, 1H), 8.92 (d, *J* = 2.0 Hz, 1H), 8.51 (t, *J* = 2.0 Hz, 1H), 8.13 (s, 1H), 7.92 (d, *J* = 10.0 Hz, 1H), 7.69 (t, *J* = 7.2 Hz, 1H), 7.53 (t, *J* = 6.8 Hz, 1H), 7.36 - 7.32 (m, 1H), 7.24 - 7.08 (m, 2H), 5.42 (t, *J* = 7.2 Hz, 1H), 4.17 (s, 3H), 1.50 (d, *J* = 7.2 Hz, 3H). |
| 1-2 | N-[(1R)-1-[2-fluoro-3-(trifluoromethyl)phenyl]ethyl]-1-[5-(3-methyltriazol-4-yl)-3-pyridyl]-6-oxo-pyridazine-3-carboxamide 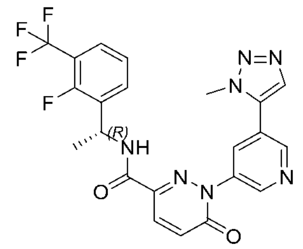 | **LCMS:** $t_R$ = 2.105 min, MS (ESI) m/z = 488.1 [M+H]$^+$. **$^1$H NMR** (400 MHz, DMSO-$d_6$) δ = 9.16-9.10 (m, 2H), 8.92 (d, *J* = 2.0 Hz, 1H), 8.51 (t, *J* = 2.0 Hz, 1H), 8.13 (s, 1H), 7.92 (d, *J* = 9.6 Hz, 1H), 7.83 (br t, *J* = 7.2 Hz, 1H), 7.68 (br t, *J* = 7.2 Hz, 1H), 7.40 (t, *J* = 7.6 Hz, 1H), 7.22 (d, *J* = 9.6 Hz, 1H), 5.46 - 5.39 (m, 1H), 4.17 (s, 3H), 1.51 (d, *J* = 7.2 Hz, 3H). |
| 1-3 | N-[(1R)-1-[2-chloro-3-(1,1-difluoro-2-hydroxy-ethyl)phenyl] ethyl]-1-[5-(3-methyltriazol-4-yl)-3-pyridyl]-6-oxo-pyridazine-3-carboxamide 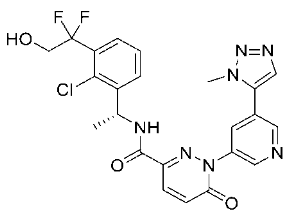 | **LCMS:** $t_R$ = 0.515 min, MS (ESI) m/z = 516.1 [M+H]$^+$. **$^1$H NMR** (400 MHz, DMSO-$d_6$) ) δ = 9.19 - 9.13 (m, 2H), 8.93 (d, *J* = 2.0 Hz, 1H), 8.53 (t, *J* = 2.0 Hz, 1H), 8.14 (s, 1H), 7.91 (d, *J* = 9.6 Hz, 1H), 7.70 (d, *J* = 7.6 Hz, 1H), 7.54 - 7.50 (m, 1H), 7.47 - 7.42 (m, 1H), 7.23 (d, *J* = 9.6 Hz, 1H), 5.70 (t, *J* = 6.4 Hz, 1H), 5.58 - 5.51 (m, 1H), 4.18 (s, 3H), 4.03 (dt, *J* = 6.4, 14.4 Hz, 2H), 1.47 (d, *J* = 7.2 Hz, 3H). |

(continued)

| No. | Chemical Name/Structure | Spectra (LCMS / $^1$H NMR) |
|---|---|---|
| 1-4 | N-[(1R)-1-[3-amino-5-(difluoromethyl)phenyl]ethyl]-6-oxo-1-[5-[3-(trideuteriomethyl)triazol-4-yl]-3-pyridyl]pyridazine-3-carboxamide 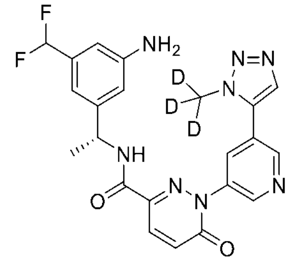 | **LCMS** : $t_R$ = 0.433 min, MS (ESI) m/z = 470.2 $[M+H]^+$. **$^1$H NMR** (400 MHz, DMSO-$d_6$) δ = 9.12 (d, *J* = 2.0 Hz, 1H), 8.94-8.90 (m, 2H), 8.50 (s, 1H), 8.12 (s, 1H), 7.95 (d, *J* = 9.6 Hz, 1H), 7.23 (d, *J* = 9.6 Hz, 1H), 6.94 (s, 1H), 6.80 (s, 1H), 6.69 (br s, 2H), 6.66 (br s, 1H), 6.58 (s, 1H), 5.36 (s, 2H), 5.07-5.02 (m, 1H), 1.45 (br d, *J* = 7.2 Hz, 3H). |
| 1-5 | N-[(1R)-1-[2-fluoro-3-(trifluoromethyl)phenyl]ethyl]-6-oxo-1-[5-[3-(trideuteriomethyl)triazol-4-yl]-3-pyridyl]pyridazine-3-carboxamide 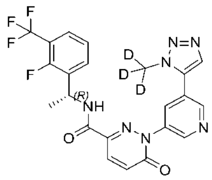 | **LCMS** : $t_R$ = 0.560 min, MS (ESI) m/z = 491.3 $[M+H]^+$. **$^1$H NMR** (400 MHz, METHANOL-$d_4$) δ = 9.10 (d, *J* = 2.4 Hz, 1H), 8.87 (*d, J* = 1.6 Hz, 1H), 8.50 (t, *J* = 2.0 Hz, 1H), 8.04 - 8.00 (m, 2H), 7.72 (br t, *J* = 7.2 Hz, 1H), 7.59 (br t, *J* = 7.2 Hz, 1H), 7.31 (t, *J* = 7.6 Hz, 1H), 7.21 (d, *J* = 9.6 Hz, 1H), 5.53 - 5.48 (m, 1H), 1.59 (d, *J* = 7.2 Hz, 3H). |
| 1-6 | N-[(1R)-1-[3-(difluoromethyl)-2-fluoro-phenyl]ethyl]-6-oxo-1-[5-[3-(trideuteriomethyl)triazol-4-yl]-3-pyridyl]pyridazine-3-carboxamide 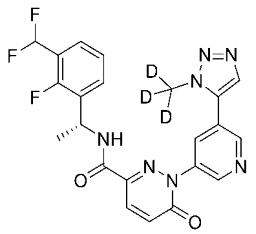 | **LCMS** : $t_R$ = 0.531 min, MS (ESI) m/z = 473.3 $[M+H]^+$. **$^1$H NMR** (400 MHz, DMSO-$d_6$) δ = 9.14-9.08 (m, 2H), 8.93 (d, *J* = 2.0 Hz, 1H), 8.52 (t, *J* = 2.0 Hz, 1H), 8.14 (s, 1H), 7.93 (d, *J* = 9.6 Hz, 1H), 7.69 (br t, *J* = 7.0 Hz, 1H), 7.53 (br t, *J* = 6.4 Hz, 1H), 7.37-7.33 (m, 1H), 7.24 (s, 1H), 7.23-7.21 (m, 1H), 5.42 (br t, *J* = 7.6 Hz, 1H), 1.50 (d, *J* = 7.2 Hz, 3H). |
| 1-7 | N-[(1R)-1-[3-(1,1-difluoro-2-hydroxy-ethyl)-2-fluoro-phenyl]ethyl]-6-oxo-1-[5-[3-(trideuteriomethyl)triazol-4-yl]-3-pyridyl]pyridazine-3-carboxamide 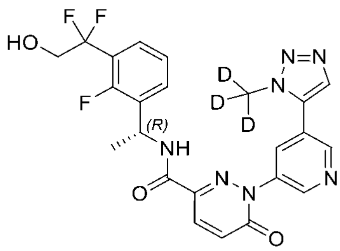 | **LCMS** : $t_R$ = 0.512 min, MS (ESI) m/z = 503.3 $[M+H]^+$. **$^1$H NMR** (400 MHz, DMSO-$d_6$) δ = 9.13 (d, *J* = 2.4 Hz, 1H), 9.08 (d, *J* = 8.4 Hz, 1H), 8.93 (d, *J* = 2.0 Hz, 1H), 8.52 (t, *J* = 2.0 Hz, 1H), 8.14 (s, 1H), 7.93 (d, *J* = 9.6 Hz, 1H), 7.65 (t, *J* = 7.2 Hz, 1H), 7.44 (t, *J* = 6.8 Hz, 1H), 7.31 - 7.26 (m, 1H), 7.23 (d, *J* = 9.86Hz, 1H), 5.72 (t, *J* = 6.4 Hz, 1H), 5.42 (quint, *J* = 7.2 Hz, 1H), 3.91 (dt, *J* = 6.4, 14.4 Hz, 2H), 1.49 (d, *J* = 7.2 Hz, 3H). |

(continued)

| No. | Chemical Name/Structure | Spectra (LCMS / $^1$H NMR) |
|---|---|---|
| 1-8 | (R)-N-(1-(2-fluoro-3-(1,1,2-trifluoroethyl)phenyl)ethyl)-1-(5-(1-methyl-1H-1,2,3-triazol-5-yl)pyridin-3-yl)-6-oxo-1,6-dihydropyridazine-3-carboxamide 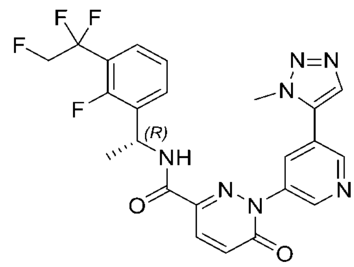 | LCMS : $t_R$ = 2.542 min, MS (ESI) m/z = 502.18 [M+H] $^+$. **$^1$H NMR** (400 MHz, DMSO-*d*$_6$) δ = 9.13-9.11 (m, 1H), 8.93 (d, *J* = 2.0 Hz, 1H), 8.52 (t, *J* = 2.0 Hz, 1H), 8.14 (s, 1H), 7.93 (d, *J = 9.6* Hz, 1H), 7.72 (t, *J* = 7.2 Hz, 1H), 7.52 (t, *J* = 6.8 Hz, 1H), 7.35 (t, *J* = 7.8 Hz, 1H), 7.23 (d, *J* = 10 Hz, 1H), 5.42 (quint, *J* = 7.3 Hz, 1H), 5.05 (t, *J* = 13.8 Hz, 1H), 4.94 (t, *J* = 13.8 Hz, 1H), 4.18 (s, 3H), 1.50(d,*J* = 7.2 Hz, 3H). |
| 1-9 | N-[(1R)-1-[3-amino-5-(trifluoromethyl)phenyl]ethyl]-1-[5-(3-methyltriazol-4-yl)-3-pyridyl]-6-oxo-pyridazine-3-carboxamide 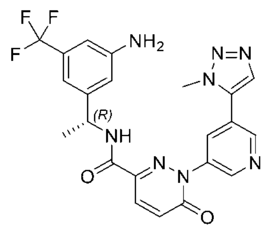 | **LCMS** : $t_R$ = 1.375 min, MS (ESI) m/z = 485.4 [M+H] $^+$. **$^1$H NMR** (400 MHz, METHANOL-*d*$_4$)*:* δ = 9.09 (d, *J* = 2.4 Hz, 1H), 8.85 (d, *J* = 2.0 Hz, 1H), 8.48 (t, *J* = 2.4 Hz, 1H), 8.05 - 8.02 (m, 2H), 7.21 (d, *J* = 9.6 Hz, 1H), 6.90 (br d, *J* = 6.0 Hz, 2H), 6.81 (s, 1H), 5.15 (q, *J* = 7.2 Hz, 1H), 4.19 (s, 3H), 1.54 (d, *J* = 7.2 Hz, 3H). |
| 1-10 | (R)-N-(1-(3-(1,1-difluoro-2-methoxyethyl)-2-fluorophenyl) ethyl)-1-(5-(1-methyl-1H-1,2,3-triazol-5-yl)pyridin-3-yl)-6-oxo-1,6-dihydropyridazine-3-carboxamide 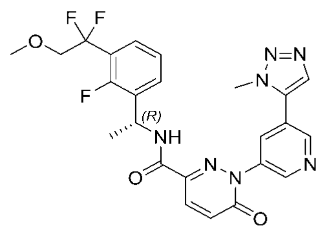 | LCMS : $t_R$ = 2.543 min, MS (ESI) m/z = 514.19 [M+H] $^+$ . **$^1$H NMR** (400 MHz, DMSO-*d*$_6$) δ = 9.13-9.11 (m, 2H), 8.93 (d, *J* = 2.0 Hz, 1H), 8.52 (t, *J* = 2.2 Hz, 1H), 8.14 (s, 1H), 7.93 (d, *J* = 10 Hz, 1H), 7.67 (t, *J* = 7.2 Hz, 1H), 7.46 (t, *J* = 6.8 Hz, 1H), 7.30 (t, *J* = 7.8 Hz, 1H), 7.23 (d, *J* = 9.6 Hz, 1H), 5.42 (quint, *J* = 7.2 Hz, 1H), 4.18 (s, 3H), 3.96 (t, *J* = 14 Hz, 1H), 3.33 (s, 3H), 1.49 (d, *J* = 8.0 Hz, 3H). |
| 1-11 | N-[(1R)-1-[3-(1,1-difluoroethyl)-2-fluoro-phenyl]ethyl]-1-[5-(3-methyltriazol-4-yl)-3-pyridyl]-6-oxo-pyridazine-3-carboxamide 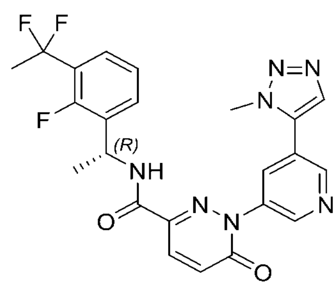 | **LCMS** : $t_R$ = 2.471 min, MS (ESI) m/z = 484.1 [M+H] $^+$. **$^1$H NMR** (400 MHz, DMSO-*d*$_6$) δ = 9.13 (d, *J* = 2.4 Hz, 1H), 9.07 (d, *J* = 8.0 Hz, 1H), 8.92 (d, *J* = 2.0 Hz, 1H), 8.52 (t, *J* = 2.0 Hz, 1H), 8.13 (s, 1H), 7.92 (d, *J* = 10.0 Hz, 1H), 7.64 (t, *J* = 7.2 Hz, 1H), 7.46 (t, *J* = 7.2 Hz, 1H), 7.28 (t, *J* = 7.6 Hz, 1H), 7.22 (d, *J* = 10.0 Hz, 1H), 5.43 (quint, *J* = 7.2 Hz, 1H), 4.17 (s, 3H), 2.01 (t, *J* = 19.2 Hz, 3H), 1.50 (d, *J* = 7.2 Hz, 3H). |

(continued)

| No. | Chemical Name/Structure | Spectra (LCMS / $^1$H NMR) |
|---|---|---|
| 1-12 | N-[(1R)-1-[2-fluoro-3-(trifluoromethyl)phenyl]ethyl]-1-[5-(3-ethyltriazol-4-yl)-3-pyridyl]-6-oxo-pyridazine-3-carboxamide 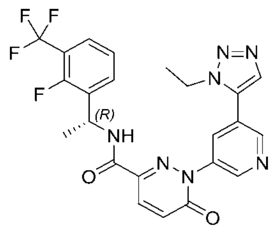 | **LCMS** : $t_R$ = 2.651 min, MS (ESI) m/z = 502.1 [M+H] $^+$. **$^1$H NMR** (400 MHz, DMSO-$d_6$) $\delta$ = 9.19-9.11 (m, 2H), 8.87 (d, $J$ = 2.0 Hz, 1H), 8.47 (t, $J$ = 2.0 Hz, 1H), 8.10 (s, 1H), 7.92 (d, $J$ = 9.6 Hz, 1H), 7.83 (br t, $J$ = 7.2 Hz, 1H), 7.68 (br t, $J$ = 7.2 Hz, 1H), 7.40 (t, $J$ = 7.6 Hz, 1H), 7.22 (d, $J$ = 9.6 Hz, 1H), 5.43 (quint, $J$ = 7.2 Hz, 1H), 4.50 (q, $J$ = 7.2 Hz, 2H), 1.52 (d, $J$ = 7.2 Hz, 3H), 1.40 (t, $J$ = 7.2 Hz, 3H). |
| 1-13 | N-[(1R)-1-[3-(1,1-difluoro-2-hydroxy-ethyl)-2-fluoro-phenyl] ethyl]-1-[5-(3-ethyltriazol-4-yl)-3-pyridyl]-5-methyl-6-oxo-pyridazine-3-carboxamide 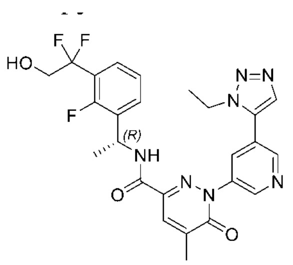 | **LCMS** : $t_R$ = 1.844 min, MS (ESI) m/z = 528.2 [M+H] $^+$. **$^1$H NMR** (400 MHz, DMSO-$d_6$) $\delta$ = 9.14 (d, $J$ = 2.4 Hz, 1H), 9.04 (d, $J$ = 8.4 Hz, 1H), 8.85 (d, $J$ = 2.0 Hz, 1H), 8.47 (t, $J$ = 2.0 Hz, 1H), 8.09 (s, 1H), 7.85 (d, $J$ = 1.6 Hz, 1H), 7.63 (t, $J$ = 6.8 Hz, 1H), 7.44 (t, $J$ = 6.8 Hz, 1H), 7.28 (t, $J$ = 7.6 Hz, 1H), 5.72 (t, $J$ = 6.4 Hz, 1H), 5.42 (t, $J$ = 7.6 Hz, 1H), 4.50 (q, $J$ = 7.6 Hz, 2H), 3.96-3.87 (m, 2H), 2.21 (d, $J$ = 1.2 Hz, 3H), 1.49 (d, $J$ = 7.2 Hz, 3H), 1.40 (t, $J$ = 7.2 Hz, 3H). |
| 1-14 | N-[(1R)-1-[3-(1,1-difluoro-2-hydroxy-ethyl)-2-fluoro-phenyl] ethyl]-1-[5-(2-ethylpyrazol-3-yl)-3-pyridyl]-6-oxo-pyridazine-3-carboxamide 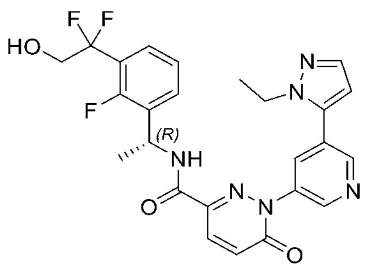 | **LCMS** : $t_R$ = 1.830 min, MS (ESI) m/z = 513.2 [M+H]$^+$. **$^1$H NMR** (400 MHz, METHANOL-$d_4$) $\delta$ = 9.05 (d, $J$ = 2.4 Hz, 1H), 8.75 (d, $J$ = 2.0 Hz, 1H), 8.38 (t, $J$ = 2.4 Hz, 1H), 8.01 (d, $J$ = 9.6 Hz, 1H), 7.62 (d, $J$ = 2.0 Hz, 1H), 7.57 (t, $J$ = 6.8 Hz, 1H), 7.51-7.46 (m, 1H), 7.25-7.19 (m, 2H), 6.56 (d, $J$ = 2.0 Hz, 1H), 5.50 (q, $J$ = 6.8 Hz, 1H), 4.60 (br s, 2H), 4.27 (q, $J$ = 7.2 Hz, 2H), 3.99 (t, $J$ = 13.6 Hz, 2H), 1.57 (d, $J$ = 7.2 Hz, 3H), 1.41 (t, $J$ = 7.2 Hz, 3H). |

(continued)

| No. | Chemical Name/Structure | Spectra (LCMS / $^1$H NMR) |
|---|---|---|
| 1-15 | N-[(1R)-1-[3-(difluoromethyl)-2-fluoro-phenyl]ethyl]-5-methyl-1-[5-(3-methyltriazol-4-yl)-3-pyridyl]-6-oxo-pyridazine-3-carboxamide 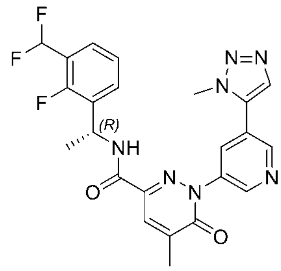 | **LCMS** : $t_R$ = 2.279 min, MS (ESI) m/z = 484.2 $[M+H]^+$. **$^1$H NMR** (400MHz, DMSO-$d_6$) δ = 9.12 (d, *J* = 2.4 Hz, 1H), 9.03 (d, *J* = 8.0 Hz, 1H), 8.91 (d, *J* = 2.0 Hz, 1H), 8.51 (t, *J* = 2.0 Hz, 1H), 8.13 (s, 1H), 7.85 (d, *J* = 1.2 Hz, 1H), 7.68 (br t, *J* = 7.6 Hz, 1H), 7.52 (s, 1H), 7.36 - 7.29 (m, 1H), 7.22 (s, 1H), 7.08 (s, 1H), 5.41 (br t, *J* = 7.6 Hz, 1H), 4.17 (s, 3H), 2.21 (s, 3H), 1.50 (d, *J* = 7.2 Hz, 3H) |
| 1-16 | N-[(1R)-1-[3-(1,1-difluoro-2-hydroxy-ethyl)-2-fluoro-phenyl]ethyl]-1-[5-(2-methylpyrazol-3-yl)-3-pyridyl]-6-oxo-pyridazine-3-carboxamide 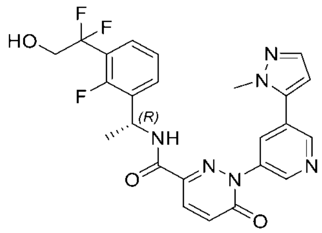 | **LCMS** : $t_R$ = 0.514 min, MS (ESI) m/z = 341.0 $[M+H]^+$. **$^1$H NMR** (400 MHz, DMSO-$d_6$) δ = 9.14-9.03 (m, 2H), 8.86 (d, *J* =2.0 Hz, 1H), 8.42 (t, *J* =2.0 Hz, 1H), 7.92 (d, *J* =9.6 Hz, 1H), 7.65 (t, *J* =6.6 Hz, 1H), 7.57 (d, *J* =2.0 Hz, 1H), 7.47-7.41 (m, 1H), 7.32-7.25 (m, 1H), 7.22 (d, *J* =9.6 Hz, 1H), 6.64 (d, *J* =2.0 Hz, 1H), 5.72 (t, *J* =6.4 Hz, 1H), 5.43 (t, *J* =7.6 Hz, 1H), 3.95 (s, 3H), 3.94-3.83 (m, 2H), 1.49 (d, *J* =7.2 Hz, 3H). |
| 1-17 | N-[(1R)-1-[2-fluoro-3-(trifluoromethyl)phenyl]ethyl]-1-[5-(2-methylpyrazol-3-yl)-3-pyridyl]-6-oxo-pyridazine-3-carboxamide 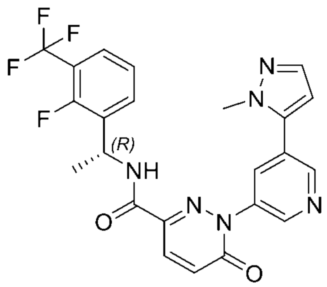 | **LCMS** : $t_R$ = 2.270 min, MS (ESI) m/z = 487.1 $[M+H]^+$. **$^1$H NMR** (400 MHz, DMSO-$d_6$) δ = 9.15 (d, *J* =7.6 Hz, 1H), 9.05 (d, *J* =2.4 Hz, 1H), 8.86 (d, *J* =2.0 Hz, 1H), 8.42 (t, *J* =2.0 Hz, 1H), 7.92 (d, *J* =9.6 Hz, 1H), 7.83 (t, *J* =7.2 Hz, 1H), 7.68 (t, *J* = 6.8 Hz, 1H), 7.57 (d, *J* =2.0 Hz, 1H), 7.40 (t, *J* =8.0 Hz, 1H), 7.22 (d, *J* =9.6 Hz, 1H), 6.64 (d, *J* =2.0 Hz, 1H), 5.46 - 5.40 (m, 1H), 3.95 (s, 3H), 1.51 (d, *J* =7.2 Hz, 3H). |
| 1-18 | N-[(1R)-1-[3-(1,1-difluoro-2-hydroxy-ethyl)-2-fluoro-phenyl]ethyl]-1-[5-(3-ethyltriazol-4-yl)-3-pyridyl]-6-oxo-pyridazine-3-carboxamide 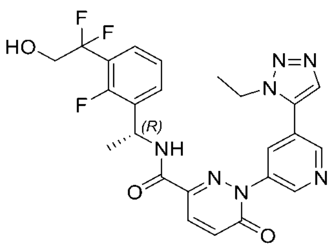 | **LCMS** : $t_R$ = 1.617 min, MS (ESI) m/z = 514.2 $[M+H]^+$. **$^1$H NMR** (400 MHz, DMSO-$d_6$) δ = 9.17-9.06 (m, 2H), 8.87 (d, *J* = 2.0 Hz, 1H), 8.48 (t, *J* = 2.0 Hz, 1H), 8.10 (s, 1H), 7.93 (d, *J* = 9.6 Hz, 1H), 7.65 (t, *J* = 7.2 Hz, 1H), 7.44 (t, *J* = 6.8 Hz, 1H), 7.31-7.26 (m, 1H), 7.23 (d, *J* = 9.6 Hz, 1H), 7.04-7.03 (m, 1H), 5.72 (t, *J* = 6.4 Hz, 1H), 5.48-5.37 (m, 1H), 4.51 (q, *J* = 7.2 Hz, 2H), 3.95-3.83 (m, 2H), 1.50 (*d, J* = 7.2 Hz, 3H), 1.41 (t, *J* = 7.2 Hz, 3H). |

(continued)

| No. | Chemical Name/Structure | Spectra (LCMS / $^1$H NMR) |
|---|---|---|
| 1-19 | N-[(1R)-1-[2-fluoro-3-(trifluoromethyl)phenyl]ethyl]-1-[6-(2-methylpyrazol-3-yl)pyridazin-4-yl]-6-oxo-pyridazine-3-carboxamide 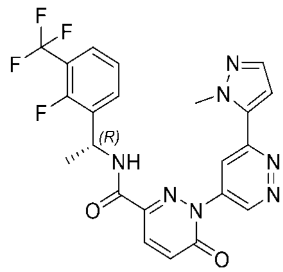 | **LCMS** : $t_R$ = 2.350 min, MS (ESI) m/z = 488.1 [M+H]$^+$. **$^1$H NMR** (400 MHz, DMSO-$d_6$) δ = 9.89 (d, *J* = 2.4 Hz, 1H), 9.26 (d, *J* = 7.6 Hz, 1H), 8.62 (d, *J* = 2.4 Hz, 1H), 7.94 (d, *J* = 9.6 Hz, 1H), 7.87 (t, *J* = 7.2 Hz, 1H), 7.70 (t, *J* = 7.2 Hz, 1H), 7.64 (d, *J* = 2.0 Hz, 1H), 7.43 (t, *J* = 7.6 Hz, 1H), 7.27 (d, *J* = 9.6 Hz, 1H), 6.98 (d, *J* = 2.0 Hz, 1H), 5.46 (t, *J* = 7.2 Hz, 1H), 4.24 (s, 3H), 1.56 (d, *J* = 7.2 Hz, 3H). |
| 1-20 | N-[(1R)-1-[3-(1,1-difluoro-2-hydroxy-ethyl)-2-fluoro-phenyl] ethyl]-1-[6-(2-methylpyrazol-3-yl)pyridazin-4-yl]-6-oxo-pyridazine-3-carboxamide 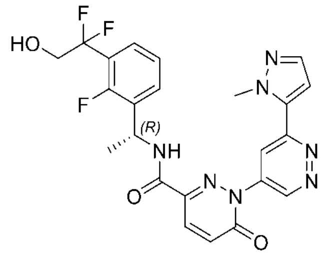 | **LCMS** : $t_R$ = 2.705 min, MS (ESI) m/z = 500.1 [M+H] $^+$. **$^1$H NMR** (400 MHz, DMSO-$d_6$) δ = 9.82 (d, *J* = 2.4 Hz, 1H), 8.63 (d, *J* = 2.4 Hz, 1H), 8.00 (d, *J* = 10.0 Hz, 1H), 7.64 - 7.58 (m, 2H), 7.50 (t, *J* = 6.8 Hz, 1H), 7.28 - 7.25 (m, 1H), 7.27 - 7.21 (m, 1H), 6.95 (d, *J* = 2.0 Hz, 1H), 5.51 (q, *J* = 6.8 Hz, 1H), 4.29 (s, 3H), 4.00 (t, *J* = 13.6 Hz, 2H), 1.61 (d, *J* = 7.2 Hz, 3H) |
| 1-21 | (R)-N-(1-(2-fluoro-3-(trifluoromethyl)phenyl)ethyl)-3-methyl-5'-(1-methyl-1H-1,2,3-triazol-5-yl)-2-oxo-2H-[1,3'-bipyridine]-5-carboxamide 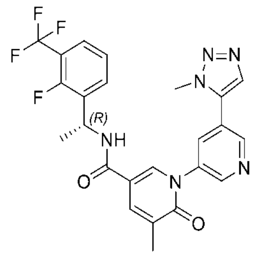 | **LCMS :** $t_R$ = 2.696 min, MS (ESI) m/z = 501.14 [M+H] $^+$. **$^1$H NMR** (400 MHz, DMSO-$d_6$) δ = 8.95 (d, *J* = 2.0 Hz, 2H), 8.86 (d, *J* = 2.0 Hz, 1H), 8.73 (d, *J* = 6.8 Hz, 1H), 8.39 (d, *J* = 2.4 Hz, 1H), 8.36 (t, *J* = 2.2 Hz, 1H), 8.13 (s, 1H), 7.91-7.90 (m, 1H), 7.77 (t, *J* = 7.0 Hz, 1H), 7.67 (t, *J* = 6.8 Hz, 1H), 7.40 (t, *J* = 7.8 Hz, 1H), 5.36 (quint, *J* = 7.0 Hz, 1H), 4.16 (s, 3H), 2.11 (s, 3H), 1.47 (d, *J* = 7.2 Hz, 3H). |
| 1-22 | (R)-3-bromo-N-(1-(2-fluoro-3-(trifluoromethyl)phenyl)ethyl)-5'-(1-methyl-1H-1,2,3-triazol-5-yl)-2-oxo-2H-[1,3'-bipyridine]-5-carboxamide 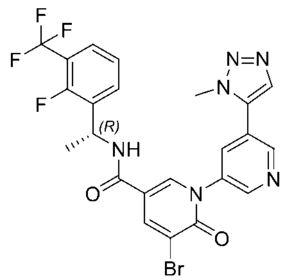 | **LCMS : $t_R$** = 2.542 min, MS (ESI) m/z = 502.18 [M+H] $^+$. **$^1$H NMR** (400 MHz, DMSO-$d_6$) δ = 9.13-9.11 (m, 1H), 8.93 (d, *J* = 2.0 Hz, 1H), 8.52 (t, *J* = 2.0 Hz, 1H), 8.14 (s, 1H), 7.93 (d, *J* = 9.6 Hz, 1H), 7.72 (t, *J* = 7.2 Hz, 1H), 7.52 (t, *J* = 6.8 Hz, 1H), 7.35 (t, *J* = 7.8 Hz, 1H), 7.23 (d, *J* = 10 Hz, 1H), 5.42 (quint, *J* = 7.3 Hz, 1H), 5.05 (t, *J* = 13.8 Hz, 1H), 4.94 (t, *J* = 13.8 Hz, 1H), 4.18 (s, 3H), 1.50 (d, *J* = 7.2 Hz, 3H). |

(continued)

| No. | Chemical Name/Structure | Spectra (LCMS / $^1$H NMR) |
|---|---|---|
| 1-23 | N-[(1R)-1-[3-(1,1-difluoro-2-hydroxy-ethyl)-2-fluoro-phenyl]ethyl]-5-methyl-1-[5-(3-methyltriazol-4-yl)-3-pyridyl]-6-oxo-pyridazine-3-carboxamide 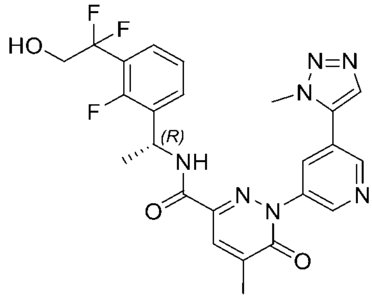 | **LCMS** : $t_R$ = 1.701 min, MS (ESI) m/z = 514.2 $[M+H]^+$. **$^1$H NMR** (400 MHz, DMSO-$d_6$) $\delta$ = 9.12 (d, *J* = 2.4 Hz, 1H), 9.03 (d, *J* = 8.0 Hz, 1H), 8.91 (d, *J* = 2.0 Hz, 1H), 8.51 (t, *J* = 2.0 Hz, 1H), 8.13 (s, 1H), 7.86 (d, *J* = 1.2 Hz, 1H), 7.64 (br t, *J* = 7.2 Hz, 1H), 7.46-7.42 (m, 1H), 7.30-7.26 (m, 1H), 5.72 (t, *J* = 6.4 Hz, 1H), 5.42 (t, *J* = 7.2 Hz, 1H), 4.17 (s, 3H), 3.91 (br d, *J* = 6.0 Hz, 2H), 2.21 (d, *J* = 1.2 Hz, 3H), 1.49 (d, *J* = 7.2 Hz, 3H). |
| 1-24 | N-[(1R)-1-[2-fluoro-3-(trifluoromethyl)phenyl]ethyl]-1-[5-(3-methyltriazol-4-yl)-3-pyridyl]-6-oxo-pyridine-3-carboxamide 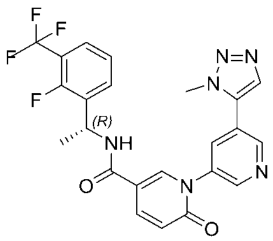 | **LCMS** : $t_R$ = 1.748 min, MS (ESI) m/z = 487.1 $[M+H]^+$). **$^1$H NMR** (400 MHz, DMSO-$d_6$) $\delta$ = 8.95 (d, *J* = 2.0 Hz, 1H), 8.87 (d, *J* = 2.4 Hz, 1H), 8.75 (d, *J* = 7.2 Hz, 1H), 8.51 (d, *J* = 2.4 Hz, 1H), 8.37 (t, *J* = 2.0 Hz, 1H), 8.13 (s, 1H), 7.99 (dd, *J* = 2.4, 9.6 Hz, 1H), 7.77 (t, *J* = 7.2 Hz, 1H), 7.67 (t, *J* = 6.8 Hz, 1H), 7.40 (t, *J* = 8.0 Hz, 1H), 6.61 (d, *J* = 9.6 Hz, 1H), 5.40-5.32 (m, 1H), 4.16 (s, 3H), 1.47 (d, *J* = 7.2 Hz, 3H). |
| 1-25 | 1-[5-(3-ethyltriazol-4-yl)-3-pyridyl]-N-[(1R)-1-[2-fluoro-3-(trifluoromethyl)phenyl]ethyl]-5-methyl-6-oxo-pyridazine-3-carboxamide 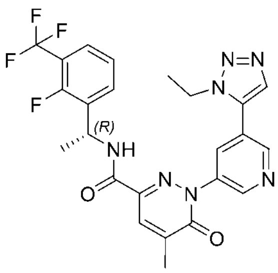 | **LCMS** : $t_R$ = 2.373 min, MS (ESI) m/z = 516.2 $[M+H]^+$. **$^1$H NMR** (400 MHz, DMSO-$d_6$) $\delta$ = 9.13 (d, *J* = 2.4 Hz, 1H), 9.09 (d, *J* = 8.0 Hz, 1H), 8.85 (d, *J* = 2.0 Hz, 1H), 8.46 (t, *J* = 2.0 Hz, 1H), 8.09 (s, 1H), 7.86 - 7.80 (m, 2H), 7.68 (t, *J* = 7.2 Hz, 1H), 7.39 (t, *J* = 7.6 Hz, 1H), 5.42 (quint, *J* = 7.2 Hz, 1H), 4.50 (q, *J* = 7.2 Hz, 2H), 2.20 (s, 3H), 1.51 (d, *J* = 7.2 Hz, 3H), 1.40 (t, *J* = 7.2 Hz, 3H). |
| 1-26 | N-[(1R)-1-[2-fluoro-3-(trifluoromethyl)phenyl]ethyl]-1-[6-methyl-5-(3-methyltriazol-4-yl)-3-pyridyl]-6-oxo-pyridazine-3-carboxamide 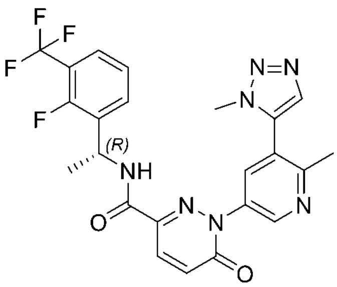 | **LCMS** : $t_R$ = 2.207 min, MS (ESI) m/z = 502.2 $[M+H]^+$. **$^1$H NMR** (400 MHz, DMSO-$d_6$) $\delta$ = 9.03 (d, *J* = 2.4 Hz, 1H), 8.82 (br d, *J* = 8.0 Hz, 1H), 8.25 (d, *J* = 2.4 Hz, 1H), 7.94 (s, 1H), 7.88 (s, 1H), 7.91 (s, 1H), 7.84 (t, *J* = 7.6 Hz, 1H), 7.66 (t, *J* = 7.2 Hz, 1H), 7.39 (t, *J* = 7.6 Hz, 1H), 7.17 (d, *J* = 9.6 Hz, 1H), 5.44 (t, *J* = 7.2 Hz, 1H), 3.95 (s, 3H), 2.47 (s, 3H), 1.56 (d, *J* = 7.2 Hz, 3H). |

(continued)

| No. | Chemical Name/Structure | Spectra (LCMS / $^1$H NMR) |
|---|---|---|
| 1-27 | 1-[5-(3-ethyltriazol-4-yl)-6-methyl-3-pyridyl]-N-[(1R)-1-[2-fluoro-3-(trifluoromethyl)phenyl]ethyl]-6-oxo-pyridazine-3-carboxamide 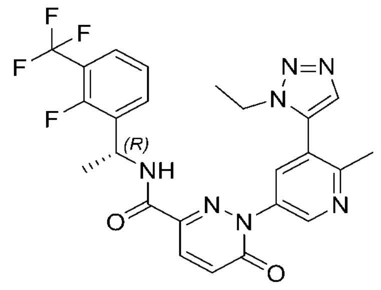 | **LCMS** : $t_R$ = 2.237 min, MS (ESI) m/z = 516.2 [M+H]$^+$). **$^1$H NMR** (400 MHz, DMSO-$d_6$) δ = 9.09 (d, *J* = 8.0 Hz, 1H), 9.03 (d, *J* = 2.4 Hz, 1H), 8.25 (d, *J* = 2.4 Hz, 1H), 7.99 (s, 1H), 7.88 (d, *J* = 9.6 Hz, 1H), 7.82 (t, *J* = 7.6 Hz, 1H), 7.68 (t, *J* = 6.8 Hz, 1H), 7.40 (t, *J* = 7.6 Hz, 1H), 7.19 (d, *J* = 9.6 Hz, 1H), 5.41 (quint, *J* = 7.2 Hz, 1H), 4.27 (q, *J* = 7.2 Hz, 2H), 2.42 (s, 3H), 1.51 (d, *J* = 7.2 Hz, 3H), 1.31 (t, *J* = 7.2 Hz, 3H). |
| 1-28 | N-[(1R)-1-[3-(1,1-difluoro-2-hydroxy-ethyl)-2-methyl-phenyl] ethyl]-1-[6-methyl-5-(3-methyltriazol-4-yl)-3-pyridyl]-6-oxo-pyridazine-3-carboxamide 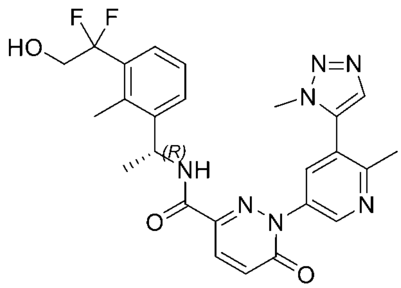 | **LCMS** : $t_R$ = 1.617 min, MS (ESI) m/z = 510.2 [M+H]$^+$. **$^1$H NMR** (400 MHz, DMSO-$d_6$) δ = 9.06-8.97 (m, 1H), 8.31 (d, *J* = 2.4 Hz, 1H), 8.01 (s, 1H), 7.87 (d, *J* = 9.6 Hz, 1H), 7.57 (d, *J* = 7.6 Hz, 1H), 7.35 (d, *J* = 7.6 Hz, 1H), 7.29-7.22 (m, 1H), 7.18 (d, *J* = 9.6 Hz, 1H), 5.41-5.32 (m, 1H), 3.92 (s, 3H), 3.90-3.82 (m, 2H), 2.46 (s, 3H), 2.43-2.43 (m, 1H), 2.41 (s, 2H), 1.43 (br d, *J* = 6.8 Hz, 3H). |
| 1-29 | (N-[(1R)-1-[3-(1,1-difluoro-2-hydroxy-ethyl)-2-fluoro-phenyl] ethyl]-1-[6-methyl-5-(3-methyltriazol-4-yl)-3-pyridyl]-6-oxo-pyridazine-3-carboxamide 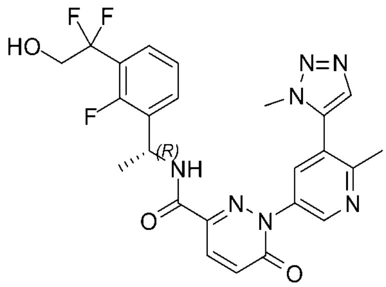 | **LCMS** : $t_R$ = 1.519 min, MS (ESI) m/z = 514.2 [M+H] $^+$. **$^1$H NMR** (400 MHz, DMSO-$d_6$) δ = 9.23-9.12 (m, 2H), 8.54 (d, *J* = 2.0 Hz, 1H), 8.07 (s, 1H), 7.90 (d, *J* = 9.6 Hz, 1H), 7.67 (br t, *J* = 6.8 Hz, 1H), 7.44 (br t, *J* = 6.8 Hz, 1H), 7.27 (t, *J* = 7.6 Hz, 1H), 7.21 (d, *J* = 9.6 Hz, 1H), 5.48 - 5.44 (m, 1H), 3.97 (s, 3H), 3.91 (br t, *J* = 14.4 Hz, 2H), 2.54 (s, 3H), 1.50 (br d, *J* = 6.8 Hz, 3H). |
| 1-30 | (R)-3-fluoro-N-(1-(2-fluoro-3-(trifluoromethyl)phenyl)ethyl)-5'-(1-methyl-1H-1,2,3-triazol-5-yl)-2-oxo-2H-[1,3'-bipyridine]-5-carboxamide 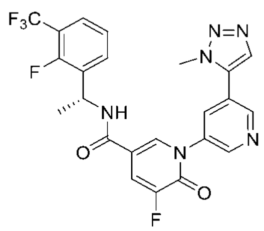 | **LCMS : $t_R$** = 2.937 min, MS (ESI) m/z = 505.16 [M+H] $^+$. **$^1$H NMR** (400 MHz, DMSO-$d_6$) δ = 9.14 (d, *J* = 6.8 Hz, 1H), 8.76 (d, *J* = 1.6 Hz, 1H), 8.70 (d, *J* = 2.4 Hz, 1H), 8.46 (d, *J* = 2.0 Hz, 1H), 8.36 (dd, *J* = 10.8, 2.0 Hz, 1H), 8.15 (t, *J* = 2.2 Hz, 1H), 8.10 (s, 1H), 7.80 (t, *J* = 7.0 Hz, 1H), 7.68 (d, *J* = 7.2 Hz, 1H), 5.20 (q, *J* = 6.4 Hz, 1H). |

(continued)

| No. | Chemical Name/Structure | Spectra (LCMS / $^1$H NMR) |
|---|---|---|
| 1-31 | N-[(1R)-1-[2-chloro-3-(trifluoromethyl)phenyl]ethyl]-1-[5-(3-methyltriazol-4-yl)-3-pyridyl]-6-oxo-pyridazine-3-carboxamide 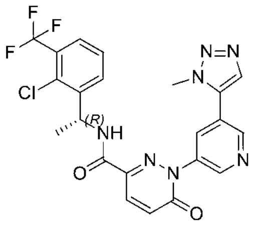 | **LCMS** : $t_R$ = 2.062 min, MS (ESI) m/z = 504.1 [M+H] $^+$. **$^1$H NMR** (400 MHz, DMSO-$d_6$) δ = 9.13 (d, *J* = 8.0 Hz, 1H), 8.60 (s, 1H), 8.35 (s, 1H), 8.20 (s, 1H), 7.66 (s, 2H), 7.43 - 7.29 (m, 4H), 7.28 - 7.20 (m, 1H), 5.14 (t, *J* = 7.2 Hz, 1H), 3.15 - 3.08 (m, 2H), 3.08 - 2.98 (m, 2H), 2.91 - 2.85 (m, 1H), 2.02 - 1.93 (m, 1H), 1.92 - 1.81 (m, 2H), 1.57 (dd, *J* = 3.6, 8.8 Hz, 1H), 1.50 (d, *J* = 7.2 Hz, 3H), 0.96 (d, *J* = 6.8 Hz, 3H). |
| 1-32 | N-[(1R)-1-[2-fluoro-3-[1-(hydroxymethyl)cyclopropyl]phenyl]ethyl]-1-[5-(3-methyltriazol-4-yl)-3-pyridyl]-6-oxo-pyridazine-3-carboxamide 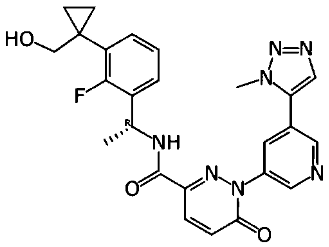 | **LCMS** : $t_R$ = 2.643 min, MS (ESI) m/z = 488.1 [M+H] $^+$. **$^1$H NMR** (400 MHz, DMSO-$d_6$) δ = 9.13 (d, *J* = 2.0 Hz, 1H), 8.98 (br d, *J* = 8.4 Hz, 1H), 8.92 (d, *J* = 1.6 Hz, 1H), 8.52 (d, *J* = 2.0 Hz, 1H), 8.13 (s, 1H), 7.93 (d, *J = 9.6* Hz, 1H), 7.34 (br t, *J* = 7.2 Hz, 1H), 7.25 - 7.17 (m, 2H), 7.06 (t, *J* = 7.6 Hz, 1H), 5.40 (quin, *J* = 7.2 Hz, 1H), 4.65 (t, *J* = 5.6 Hz, 1H), 4.17 (s, 3H), 3.43 (br d, *J* = 6.0 Hz, 2H), 1.46 (br d, *J* = 7.2 Hz, 3H), 0.82 (m, 2H), 0.65 (m, 2H). |
| 1-33 | N-[(1R)-1-[2-fluoro-3-(1-methylcyclopropyl)phenyl]ethyl]-1-[5-(3-methyltriazol-4-yl)-3-pyridyl]-6-oxo-pyridazine-3-carboxamide 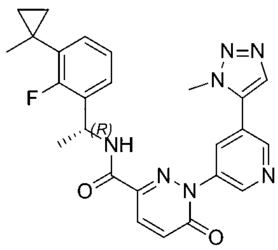 | **LCMS** : $t_R$ = 2.072 min, MS (ESI) m/z = 474.2 [M+H] $^+$. **$^1$H NMR** (400 MHz, DMSO-$d_6$) δ = 9.13 (d, *J* = 2.4 Hz, 1H), 8.96 (d, *J* = 8.4 Hz, 1H), 8.92 (d, *J* = 2.0 Hz, 1H), 8.51 (t, *J* = 2.4 Hz, 1H), 8.13 (s, 1H), 7.92 (d, *J* = 9.6 Hz, 1H), 7.32 (t, *J* = 7.6 Hz, 1H), 7.22 (d, *J* = 9.6 Hz, 2H), 7.08 - 7.04 (m, 1H), 5.42 - 5.37 (m, 1H), 4.17 (s, 3H), 1.47 (d, *J* = 7.2 Hz, 3H), 1.29 (s, 3H), 0.73 (d, *J* = 2.0 Hz, 2H), 0.69 (d, *J* = 2.4 Hz, 2H). |
| 1-34 | N-[(1R)-1-deuterio-1-[2-fluoro-3-(trifluoromethyl)phenyl]ethyl]-1-[5-(3-methyltriazol-4-yl)-3-pyridyl]-6-oxo-pyridazine-3-carboxamide 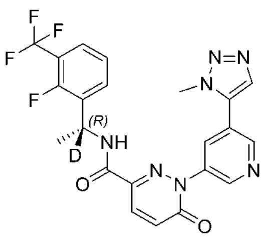 | **LCMS:** $t_R$ = 2.018 min, MS (ESI) m/z = 489.2 [M+H] $^+$. **$^1$H NMR** (400 MHz, DMSO-$d_6$) δ = 9.15-9.10 (m, 2H), 8.93 (d, *J* = 2.0 Hz, 1H), 8.51 (t, *J* = 2.4 Hz, 1H), 8.13 (s, 1H), 7.92 (d, *J* = 10.0 Hz, 1H), 7.84 (br t, *J* = 7.2 Hz, 1H), 7.68 (br t, *J* = 7.2 Hz, 1H), 7.40 (t, *J* = 8.0 Hz, 1H), 7.22 (d, *J* = 10.0 Hz, 1H), 4.17 (s, 3H), 1.51 (s, 3H). |

(continued)

| No. | Chemical Name/Structure | Spectra (LCMS / $^1$H NMR) |
|---|---|---|
| 1-35 | N-{(R)-1-[2-fluoro-3-(trifluoromethyl)phenyl]-ethyl-(2,2,2-trideuterio)}-1-[5-(1-methyl-1H-1,2,3-triazol-5-yl)-3-pyridyl]-6-oxo-pyridazine-3-carboxamide 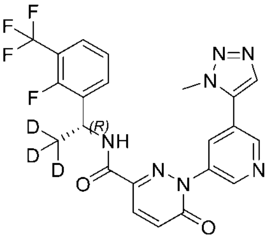 | **LCMS** : $t_R$ = 1.278 min, MS (ESI) m/z = 491.1 [M+H] $^+$. **$^1$H NMR** (400 MHz, DMSO-$d_6$) δ = 9.17 - 9.09 (m, 2H), 8.93 (d, *J* = 2.0 Hz, 1H), 8.52 (t, *J* = 2.0 Hz, 1H), 8.14 (s, 1H), 7.93 (d, *J* = 10.0 Hz, 1H), 7.83 (t, *J* = 7.2 Hz, 1H), 7.69 (t, *J* = 7.2 Hz, 1H), 7.41 (t, *J* = 7.8 Hz, 1H), 7.23 (d, *J* = 10.0 Hz, 1H), 5.42 (d, *J* = 8.0 Hz, 1H), 4.18 (s, 3H). |
| 1-36 | N-{(R)-1-[2-fluoro-3-(trifluoromethyl)phenyl]-ethyl-(2,2,2-trideuterio)}-1-(5-{1-[trideuteriomethyl]-1H-1,2,3-triazol-5-yl}-3-pyridyl)-6-oxo-1,6-dihydropyridazine-3-carboxamide 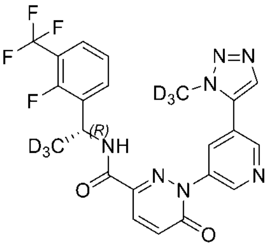 | **LCMS** : $t_R$ = 0.621 min, MS (ESI) m/z = 494.3 [M+H] $^+$. **$^1$H NMR** (400 MHz, DMSO-$d_6$) δ = 9.14 - 9.09 (m, 2H), 8.93 (d, *J* = 2.0 Hz, 1H), 8.51 (t, *J* = 2.0 Hz, 1H), 8.13 (s, 1H), 7.92 (d, *J* = 9.6 Hz, 1H), 7.83 (t, *J* = 7.2 Hz, 1H), 7.68 (t, *J* = 6.8 Hz, 1H), 7.40 (t, *J* = 7.6 Hz, 1H), 7.22 (d, *J* = 9.6 Hz, 1H), 5.41 (d, *J* = 7.6 Hz, 1H). |
| 1-37 | N-{(1R)-1-[3-(1,1-difluoro-2-hydroxyethyl)-2-fluoro-phenyl] ethyl}-6'-(1-methyl-1H-1,2,3-triazol-5-yl)-6-oxo-6H-[1,4'-bipyridazine]-1,6-dihydro-3-carboxamide 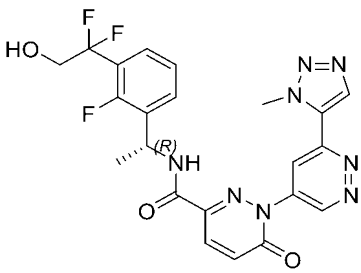 | **LCMS** : $t_R$ = 0.556 min, MS (ESI) m/z = 501.2 [M+H] $^+$. **$^1$H NMR** (400 MHz, DMSO-$d_6$) δ = 9.97 (d, *J* = 2.4 Hz, 1H), 9.20 (d, *J* = 8.0 Hz, 1H), 8.75 (d, *J* = 2.4 Hz, 1H), 8.46 (s, 1H), 7.94 (d, *J* = 10.0 Hz, 1H), 7.68 (br t, *J* = 7.2 Hz, 1H), 7.45 (br t, *J* = 6.8 Hz, 1H), 7.33 - 7.25 (m, 2H), 5.72 (br s, 1H), 5.44 (quin, *J* = 7.2 Hz, 1H), 4.43 (s, 3H), 3.92 (br t, *J* =1 4.2 Hz, 2H), 1.53 (d, *J* = *7.2* Hz, 3H). |
| 1-38 | N-{(R)-1-[2-fluoro-3-(trifluoromethyl)phenyl]ethyl}-1-[5-(1-methyl-1H-1,2,3-triazol-5-yl)-3-pyridyl]-6-oxo-1,6-dihydropyridazine-5-deuterio-3-carboxamide 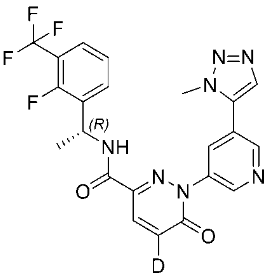 | **LCMS** : $t_R$ = 1.871 min, MS (ESI) m/z = 489.0 [M+H] $^+$. **$^1$H NMR** (400 MHz, DMSO-$d_6$) δ = 9.14 - 9.11 (m, 2H), 8.93 (d, *J* = 2.0 Hz, 1H), 8.52 (t, *J* = 2.0 Hz, 1H), 8.13 (s, 1H), 7.95 - 7.90 (m, 1H), 7.84 (t, *J* = 7.2 Hz, 1H), 7.69 (t, *J* = 6.8 Hz, 1H), 7.41 (t, *J* = 7.6 Hz, 1H), 5.44 (t, *J* = 7.2 Hz, 1H), 4.17 (s, 3H), 1.52 (d, *J* = 7.2 Hz, 3H). |

(continued)

| No. | Chemical Name/Structure | Spectra (LCMS / $^1$H NMR) |
|---|---|---|
| 1-39 | N-{(R)-1-[3-(1,1-difluoro-2-hydroxyethyl)-2-fluorophenyl] ethyl}-1-[5-(1-isopropyl-1H-1,2,3-triazol-5-yl)-3-pyridyl]-5-methyl-6-oxo-1,6-dihydropyridazine-3-carboxamide 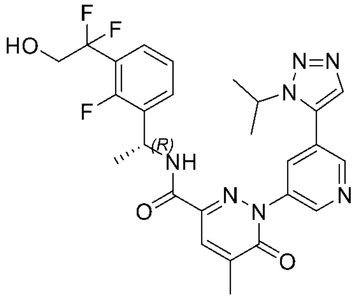 | **LCMS :** $t_R$ = 1.258 min, MS (ESI) m/z = 542.1 [M+H] $^+$. **$^1$H NMR** (400 MHz, $CDCl_3$) $\delta$ = 9.12 (t, *J* = 2.4 Hz, 1H), 8.69 (t, *J* = 2.0 Hz, 1H), 8.20 - 8.15 (m, 1H), 7.92 (d, *J* = 1.2 Hz, 1H), 7.86 (d, *J* = 2.4 Hz, 1H), 7.60 (br d, *J* = 8.4 Hz, 1H), 7.53 (br t, *J* = 7.6 Hz, 1H), 7.45 (br t, *J* = 6.8 Hz, 1H), 7.25 - 7.19 (m, 1H), 5.42 (br t, *J* = 7.2 Hz, 1H), 4.18 - 3.98 (m, 2H), 2.34 (s, 3H), 1.68 (ddd, *J* = 2.0, 6.8, 11.2 Hz, 6H), 1.63 - 1.60 (m, 3H). |
| 1-40 | N-{(R)-1-[3-(1,1-difluoro-2-hydroxyethyl)-2-fluorophenyl] ethyl}-1-[5-(1-cyclopropyl-1H-1,2,3-triazol-5-yl)-3-pyridyl]-5-methyl-6-oxo-1,6-dihydropyridazine-3-carboxamide 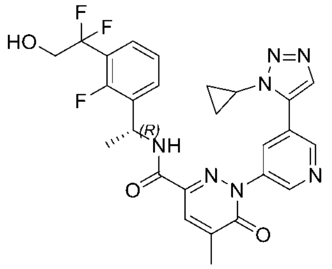 | **LCMS** : $t_R$ = 1.225 min, MS (ESI) m/z = 540.1 [M+H] $^+$. **$^1$H NMR** (400 MHz, DMSO-$d_6$) $\delta$ = 9.11 (d, *J* = 2.4 Hz, 1H), 9.03 - 8.98 (m, 2H), 8.63 (t, *J* = 2.0 Hz, 1H), 8.16 (s, 1H), 7.85 (d, *J* = 1.2 Hz, 1H), 7.63 (br t, *J* = 6.8 Hz, 1H), 7.44 (br t, *J* = 6.8 Hz, 1H), 7.27 (t, *J* = 7.6 Hz, 1H), 5.72 (t, *J* = 6.4 Hz, 1H), 5.41 (quin, *J* = 7.2 Hz, 1H), 4.07 - 3.99 (m, 1H), 3.91 (dt, *J* = 6.4, 14.3 Hz, 2H), 2.21 (d, *J* = 0.8 Hz, 3H), 1.48 (d, *J* = 7.2 Hz, 3H), 1.19 - 1.11 (m, 4H). |
| 1-41 | N-{(R)-1-[3-(1,1-difluoro-2-hydroxyethyl)-2-fluorophenyl]ethyl}-5-ethyl-1-[5-(1-isopropyl-1H-1,2,3-triazol-5-yl)-3-pyridyl]-6-oxo-1,6-dihydropyridazine-3-carboxamide 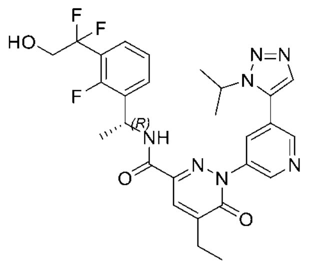 | **LCMS :** $t_R$ = 1.225 min, MS (ESI) m/z = 556.2 [M+H] $^+$. **$^1$H NMR** (400 MHz, DMSO-$d_6$) $\delta$ = 9.16 (d, *J* = 2.0 Hz, 1H), 9.05 (br d, *J* = 8.0 Hz, 1H), 8.79 (d, *J* = 1.6 Hz, 1H), 8.41 (s, 1H), 8.03 (s, 1H), 8.07 - 7.97 (m, 1H), 7.76 (s, 1H), 7.64 (br t, *J* = 6.8 Hz, 1H), 7.44 (br t, *J* = 6.8 Hz, 1H), 7.30 - 7.24 (m, 1H), 5.71 (br s, 1H), 5.42 (br t, *J* = 7.6 Hz, 1H), 4.78 (td, *J* = 6.8, 13.2 Hz, 1H), 3.91 (br t,*J* = 13.2 Hz, 2H), 2.60 (q, *J* = 7.2 Hz, 2H), 1.53 (s, 6H), 1.49 (br d, *J* = 7.2 Hz, 3H), 1.18 (t, *J* = 7.6 Hz, 3H). |

(continued)

| No. | Chemical Name/Structure | Spectra (LCMS / $^{1}$H NMR) |
|---|---|---|
| 1-42 | N-{(R)-1-[3-(1,1-difluoro-2-hydroxyethyl)-2-fluorophenyl] ethyl}-1-[5-(1-cyclopropyl-1H-1,2,3-triazol-5-yl)-3-pyridyl]-5-ethyl-6-oxo-1,6-dihydropyridazine-3-carboxamide 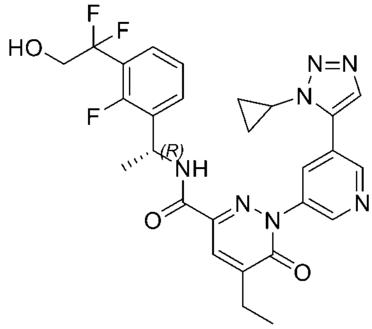 | **LCMS :** $t_R$ = 1.185 min, MS (ESI) m/z = 554.2 [M+H] $^+$. **$^{1}$H NMR** (400 MHz, DMSO-$d_6$) $\delta$ = 9.11 (d, *J* = 2.0 Hz, 1H), 9.04 - 8.99 (m, 2H), 8.63 (s, 1H), 8.16 (s, 1H), 7.76 (s, 1H), 7.64 (br t, *J* = 7.2 Hz, 1H), 7.44 (br t, *J* = 7.2 Hz, 1H), 7.31 - 7.23 (m, 1H), 5.71 (t, *J* = 6.4 Hz, 1H), 5.42 (quin, *J* = 7.2 Hz, 1H), 4.06 - 4.00 (m, 1H), 3.92 (dt, *J* = 6.0, 14.4 Hz, 2H), 2.60 (q, *J* = 7.2 Hz, 2H), 1.49 (d, *J* = 7.2 Hz, 3H), 1.21 - 1.13 (m, 7H). |
| 1-43 | N-[(1R)-1-[3-(1,1-difluoro-2-hydroxy-ethyl)-2-fluoro-phenyl]ethyl]-6-oxo-1,6-dihydropyridazine-5-deuterio-3-carboxamide 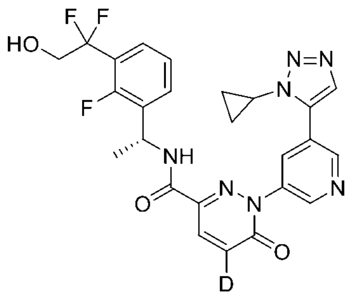 | **LCMS :** $t_R$ = 1.144 min, MS (ESI) m/z = 527.2 [M+H] $^+$. **$^{1}$H NMR** (400 MHz, DMSO-$d_6$) $\delta$ = 9.12 (d, *J* = 2.4 Hz, 1H), 9.07 (d, *J* = 8.0 Hz, 1H), 9.02 (d, *J* = 2.0 Hz, 1H), 8.65 (t, *J* = 2.0 Hz, 1H), 8.17 (s, 1H), 7.92 (s, 1H), 7.64 (br t, *J* = 7.2 Hz, 1H), 7.44 (br t, *J* = 6.8 Hz, 1H), 7.31 - 7.24 (m, 1H), 5.71 (br s, 1H), 5.49 - 5.35 (m, 1H), 4.08 - 3.99 (m, 1H), 3.96 - 3.87 (m, 2H), 1.49 (d, *J* = 7.2 Hz, 3H), 1.19 - 1.13 (m, 4H). |
| 1-44 | N-{(R)-1-[2-fluoro-3-(trifluoromethyl)phenyl]ethyl}-5-{(R)-1-[2-fluoro-3-(trifluoromethyl)phenyl]ethylamino}-1-[5-(1-methyl-1H-1,2,3-triazol-5-yl)-3-pyridyl]-6-oxo-1,6-dihydropyridazine-3-carboxamide 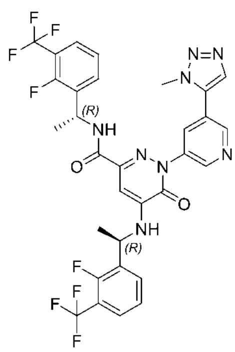 | **LCMS :** $t_R$ = 2.780 min, MS (ESI) m/z = 693.2 [M+H] $^+$. **$^{1}$H NMR** (400 MHz, DMSO-$d_6$) $\delta$ = 9.38 (d, *J* = 7.2 Hz, 1H), 9.12 (d, *J* = 8.4 Hz, 1H), 8.88 (d, *J* = 2.0 Hz, 1H), 8.83 (d, *J* = 2.4 Hz, 1H), 8.25 (t, *J* = 2.2 Hz, 1H), 8.06 (s, 1H), 7.85-7.77 (m, 2H), 7.70-7.64 (m, 3H), 7.42-7.35 (m, 2H), 5.48 (quin, *J* = 7.3, 14.7 Hz, 1H), 5.26 (quin, *J* = 7.0, 14.0 Hz, 1H), 4.01 (s, 3H), 1.51 (d, *J* = 7.2 Hz, 3H), 1.48 (d, *J* = 7.2 Hz, 3H). |
| 1-45 | N-{(R)-1-[2-fluoro-3-(trifluoromethyl)phenyl]ethyl}-1-[6-(methylamino)-5-(1-methyl-1H-1,2,3-triazol-5-yl)-3-pyridyl]-6-oxo-1,6-dihydropyridazine-3-carboxamide 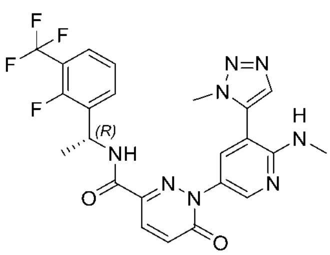 | **LCMS** : $t_R$ = 2.710 min, MS (ESI) m/z = 517.1 [M+H] $^+$. **$^{1}$H NMR** (400 MHz, DMSO-$d_6$) $\delta$ = 9.06 (d, *J* = 8.0 Hz, 1H), 8.52 (d, *J* = 2.4 Hz, 1H), 7.88-7.84 (m, 3H), 7.80 (d, *J* = 2.4 Hz, 1H), 7.68 (t, *J* = 7.2 Hz, 1H), 7.40 (t, *J* = 7.4 Hz, 1H), 7.11 (d, *J* = 9.6 Hz, 1H), 6.44-6.42 (m, 1H), 5.41 (quin, *J* = 7.4, 14.5 Hz, 1H), 3.90 (s, 3H), 2.84 (d, *J* = 4.8 Hz, 3H), 1.51 (d, *J* = 6.8 Hz, 3H). |

(continued)

| No. | Chemical Name/Structure | Spectra (LCMS / $^1$H NMR) |
|---|---|---|
| 1-46 | N-{(R)-1-[2-fluoro-3-(trifluoromethyl)phenyl]ethyl}-1-[5-(1-methyl-1H-1,2,3-triazol-5-yl)-6-morpholino-3-pyridyl]-6-oxo-1,6-dihydropyridazine-3-carboxamide 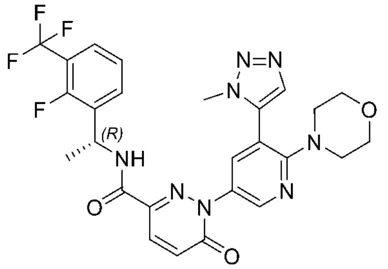 | **LCMS :** $t_R$ = 2.874 min, MS (ESI) m/z = 572.3 [M+H] $^+$. **$^1$H NMR** (400 MHz, DMSO-*d*$_6$) δ = 9.11 (d, *J* = 8.0 Hz, 1H), 8.77 (d, *J* = 2.4 Hz, 1H), 8.09 (d, *J* = 2.8 Hz, 1H), 7.97 (s, 1H), 7.88 (d, *J* = 9.6 Hz, 1H), 7.83 (t, *J* = 7.2 Hz, 1H), 7.69 (t, *J* = 7.0 Hz, 1H), 7.40 (t, *J* = 7.8 Hz, 1H), 7.17 (d, *J* = 10.0 Hz, 1H), 5.42 (quin, *J* = 7.1, 14.3 Hz, 1H), 4.02 (s, 3H), 3.58 (t, *J* = 4.6 Hz, 4H), 3.03 (t, *J* = 4.6 Hz, 4H), 1.51 (d, *J* = 6.8 Hz, 3H). |
| 1-47 | N-{(R)-1-[3-(difluoromethyl)-2-fluorophenyl]ethyl}-1-[6-methyl-5-(1-methyl-1H-1,2,3-triazol-5-yl)-3-pyridyl]-6-oxo-1,6-dihydropyridazine-3-carboxamide 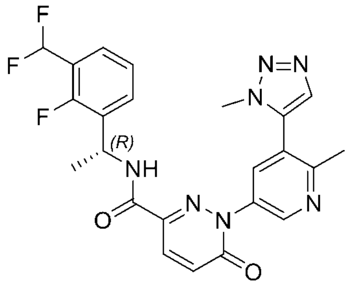 | **LCMS** : $t_R$ = 0.531 min, MS (ESI) m/z = 484.1 [M+H] $^+$. **$^1$H NMR** (400 MHz, DMSO-*d*$_6$) δ = 9.05 (d, *J* = 8.0 Hz, 1H), 9.01 (d, *J* = 2.4 Hz, 1H), 8.27 (d, *J* = 2.4 Hz, 1H), 8.03 (s, 1H), 7.89 (d, *J* = 10.0 Hz, 1H), 7.67 (t, *J* = 7.2 Hz, 1H), 7.53 (t, *J* = 6.8 Hz, 1H), 7.32 (t, J = 7.6 Hz, 1H), 7.22 (t, *J* = 54.4 Hz 1H), 7.19 (d, *J* = 10.0 Hz, 1H), 5.40 (quin, *J* = 7.2 Hz, 1H), 3.95 (s, 3H), 2.47 (s, 3H), 1.49 (d, *J* = 7.2 Hz, 3H). |
| 1-48 | N-{(R)-1-[2-fluoro-3-(trifluoromethyl)phenyl]ethyl}-1-[6-(1-methyl-1H-1,2,3-triazol-5-yl)pyrazin-2-yl]-6-oxo-1,6-dihydropyridine-3-carboxamide 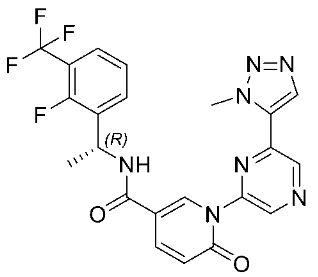 | **LCMS** : $t_R$ = 0.632 min, MS (ESI) m/z = 488.1 [M+H] $^+$. **$^1$H NMR** (400 MHz, MeOD) δ = 9.18 (d, *J* = 3.6 Hz, 2H), 8.69 (d, *J* = 2.4 Hz, 1H), 8.44 (s, 1H), 8.10 (dd, *J* = 2.8, 10.0 Hz, 1H), 7.74-7.66 (m, 1H), 7.63-7.56 (m, 1H), 7.33 (t, *J* = 8.0 Hz, 1H), 6.72 (d, *J* = 9.6 Hz, 1H), 5.46 (q, *J* = 7.2 Hz, 1H), 4.41 (s, 3H), 1.59 (d, *J* = 7.2 Hz, 3H). |
| 1-49 | N-[(1R)-1-[2-fluoro-3-(trifluoromethyl)phenyl]ethyl]-1-[5-(3-isopropyltriazol-4-yl)-3-pyridyl]-6-oxo-pyridazine-3-carboxamide 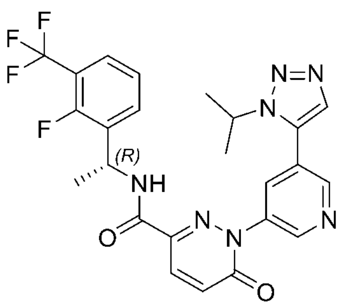 | **LCMS** : $t_R$ = 2.261 min, MS (ESI) m/z = 514.2 [M+H] $^+$. **$^1$H NMR** (400 MHz, DMSO-*d*$_6$) δ = 9.16 (d, *J* = 2.0 Hz, 1H), 9.13 (d, *J* = 8.0 Hz, 1H), 8.80 (d, *J* = 2.0 Hz, 1H), 8.41 (t, *J* = 2.0 Hz, 1H), 8.03 (s, 1H), 7.92 (d, *J* = 9.6 Hz, 1H), 7.83 (t, *J* = 7.2 Hz, 1H), 7.68 (t, *J* = 7.2 Hz, 1H), 7.40 (t, *J* = 7.6 Hz, 1H), 7.22 (d, *J* = 9.6 Hz, 1H), 5.43 (quin, *J* = 7.2 Hz, 1H), 4.78 (quin, *J* = 6.8 Hz, 1H), 1.54 - 1.51 (m, 9H). |

(continued)

| No. | Chemical Name/Structure | Spectra (LCMS / $^1$H NMR) |
| --- | --- | --- |
| 1-50 | N-[(1R)-1-[2-fluoro-3-(trifluoromethyl)phenyl]ethyl]-1-[5-(1-cyclopropyl-1H-1,2,3-triazol-5-yl)-3-pyridyl]-6-oxo-pyridazine-3-carboxamide 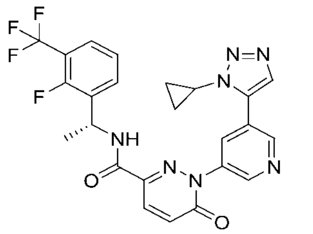 | **LCMS** : $t_R$ = 2.179 min, MS (ESI) m/z = 514.1 [M+H] $^+$. **$^1$H NMR** (400 MHz, DMSO-$d_6$) δ = 9.14 - 9.09 (m, 2H), 9.02 (d, *J* = 2.0 Hz, 1H), 8.64 (t, *J* = 2.0 Hz, 1H), 8.16 (s, 1H), 7.92 (d, *J* = 9.6 Hz, 1H), 7.82 (t, *J* = 7.2 Hz, 1H), 7.68 (t, *J* = 7.2 Hz, 1H), 7.39 (t, *J* = 7.6 Hz, 1H), 7.22 (d, *J* = 9.6 Hz, 1H), 5.42 (t, *J* = 7.2 Hz, 1H), 4.06 - 3.99 (m, 1H), 1.51 (d, *J* = 7.2 Hz, 3H), 1.18 - 1.12 (m, 4H). |
| 1-51 | N-[(1R)-1-[3-(difluoromethyl)-2-fluorophenyl]ethyl]-1-[5-(1-cyclopropyl-1H-1,2,3-triazol-5-yl)-3-pyridyl]-6-oxo-pyridazine-3-carboxamide 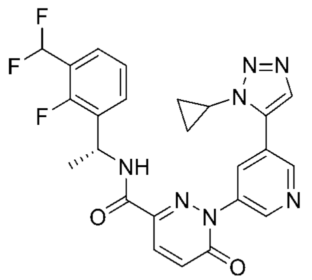 | **LCMS** : $t_R$ = 1.959 min, MS (ESI) m/z = 496.1 [M+H] $^+$. **$^1$H NMR** (400 MHz, DMSO-$d_6$) δ = 9.12 (d, *J* = 2.4 Hz, 1H), 9.07 (d, *J* = 8.0 Hz, 1H), 9.02 (d, *J* = 2.0 Hz, 1H), 8.65 (t, *J* = 2.0 Hz, 1H), 8.17 (s, 1H), 7.92 (d, *J* = 9.6 Hz, 1H), 7.68 (br t, *J* = 7.2 Hz, 1H), 7.53 (br t, *J* = 7.2 Hz, 1H), 7.35 - 7.29 (m, 1H), 7.24 - 7.20 (m, 2H), 7.08 (s, 1H), 5.42 (quin, *J* = 7.2 Hz, 1H), 4.06 - 4.00 (m, 1H), 1.50 (d, *J* = 7.2 Hz, 3H), 1.18 - 1.12 (m, 4H). |
| 1-52 | N-[(1R)-1-[3-(1,1-difluoro-2-hydroxy-ethyl)-2-fluoro-phenyl] ethyl]-1-[5-(1-isopropyl-1H-1,2,3-triazol-5-yl)-3-pyridyl]-6-oxo-pyridazine-3-carboxamide 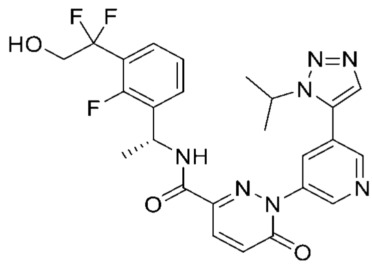 | **LCMS** : $t_R$ = 1.683 min, MS (ESI) m/z = 528.2 [M+H] $^+$. **$^1$H NMR** (400 MHz, DMSO-$d_6$) δ = 9.16 (d, *J* = 2.4 Hz, 1H), 9.08 (d, *J* = 8.2 Hz, 1H), 8.80 (d, *J* = 2.0 Hz, 1H), 8.41 (t, *J* = 2.0 Hz, 1H), 8.03 (s, 1H), 7.92 (d, *J* = 9.6 Hz, 1H), 7.64 (br t, *J* = 7.2 Hz, 1H), 7.48 - 7.41 (m, 1H), 7.27 (t, *J* = 7.6 Hz, 1H), 7.22 (d, *J* = 9.6 Hz, 1H), 5.72 (t, *J* = 6.4 Hz, 1H), 5.46 - 5.37 (m, 1H), 4.78 (quin, *J* = 6.4 Hz, 1H), 3.96 - 3.86 (m, 2H), 1.52 (d, *J* = 6.4 Hz, 6H), 1.49 (d, *J* = 7.2 Hz, 3H). |

(continued)

| No. | Chemical Name/Structure | Spectra (LCMS / $^1$H NMR) |
|---|---|---|
| 1-53 | N-[(1R)-1-[3-(1,1-difluoro-2-hydroxy-ethyl)-2-fluoro-phenyl]ethyl]-1-[5-(1-cyclopropyl-1H-1,2,3-triazol-5-yl)-3-pyridyl]-6-oxo-pyridazine-3-carboxamide 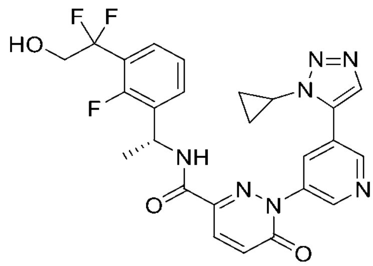 | **LCMS** : $t_R$ = 1.614 min, MS (ESI) m/z = 526.1 [M+H] $^+$. **$^1$H NMR** (400 MHz, DMSO-$d_6$) δ = 9.12 (d, *J* = 2.4 Hz, 1H), 9.07 (d, *J* = 8.0 Hz, 1H), 9.02 (d, *J* = 2.0 Hz, 1H), 8.65 (t, *J* = 2.4 Hz, 1H), 8.17 (s, 1H), 7.93 (d, *J* = 9.6 Hz, 1H), 7.64 (br t, *J* =6.8 Hz, 1H), 7.44 (br t, *J* = 6.8 Hz, 1H), 7.30 - 7.25 (m, 1H), 7.23 (d, *J* = 9.6 Hz, 1H), 5.71 (t, *J* = 6.4 Hz, 1H), 5.47 - 5.37 (m, 1H), 4.08 - 3.99 (m, 1H), 3.91 (dt, *J* = 6.4, 14.4 Hz, 2H), 1.49 (d, *J* = 7.2 Hz, 3H), 1.19 - 1.13 (m, 4H). |
| 1-54 | N-[(1R)-1-[2-fluoro-3-(trifluoromethyl)phenyl]ethyl]-1-[5-(1-cyclobutyl-1H-1,2,3-triazol-5-yl)-3-pyridyl]-6-oxo-pyridazine-3-carboxamide 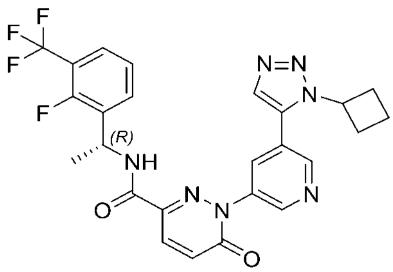 | **LCMS** : $t_R$ = 2.334 min, MS (ESI) m/z = 528.2 [M+H] $^+$. **$^1$H NMR** (400 MHz, DMSO-$d_6$) δ = 8.98 (d, *J* = 2.4 Hz, 1H), 8.69 (d, *J* = 1.6 Hz, 1H), 8.13 (t, *J* = 2.4 Hz, 1H), 8.07 (d, *J* = 9.6 Hz, 1H), 7.84 (s, 1H), 7.55 (t, *J* = 7.2 Hz, 2H), 7.44 - 7.39 (m, 1H), 7.25 - 7.21 (m, 1H), 7.15 (d, *J* = 9.6 Hz, 1H), 5.47 - 5.41 (m, 1H), 4.94 - 4.86 (m, 1H), 2.99 - 2.89 (m, 2H), 2.58 - 2.51 (m, 2H), 2.07 - 1.90 (m, 2H), 1.64 (d, *J* = 7.2 Hz, 3H). |
| 1-55 | 1-[5-(1-cyclobutyl-1H-1,2,3-triazol-5-yl)-3-pyridyl]-N-[(1R)-1-[3-(1,1-difluoro-2-hydroxy-ethyl)-2-fluoro-phenyl]ethyl]-6-oxo-pyridazine-3-carboxamide 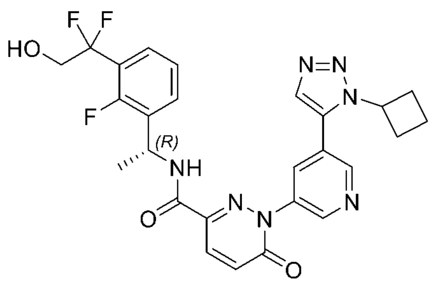 | **LCMS** : $t_R$ = 1.769 min, MS (ESI) m/z = 540.1 [M+H] $^+$. **$^1$H NMR** (400 MHz, DMSO-$d_6$) δ = 9.10 (d, *J* = 2.4 Hz, 1H), 8.68 (d, *J* = 2.0 Hz, 1H), 8.17 (t, *J* = 2.0 Hz, 1H), 8.07 (d, *J* = 9.6 Hz, 1H), 7.88 (s, 1H), 7.59 (br d, *J* = 8.4 Hz, 1H), 7.56 - 7.51 (m, 1H), 7.45 (t, *J* = 6.8 Hz, 1H), 7.22 (t, *J* = 7.6 Hz, 1H), 7.16 (d, *J* = 9.6 Hz, 1H), 5.47 - 5.39 (m, 1H), 4.92 (quin, J = 8.4 Hz, 1H), 4.20 - 4.02 (m, 2H), 3.59 (br t, *J* = 6.4 Hz, 1H), 2.94 (quin, *J* = 10.0 Hz, 2H), 2.60 - 2.51 (m, 2H), 2.08 - 1.91 (m, 2H), 1.62 (d, *J* = 7.2 Hz, 3H). |

(continued)

| No. | Chemical Name/Structure | Spectra (LCMS / $^1$H NMR) |
|---|---|---|
| 1-56 | 1-[5-(1-cyclopropyl-1H-1,2,3-triazol-5-yl)-3-pyridyl]-6-oxo-N-[(1R)-1-[3-amino-5-(trifluoromethyl)phenyl]ethyl]pyridazine-3-carboxamide 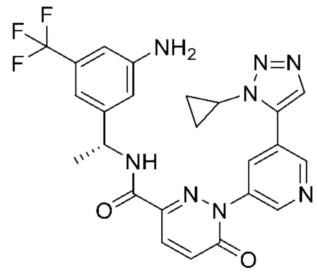 | **LCMS :** $t_R$ = 1.735 min, MS (ESI) m/z = 511.2 [M+H] $^+$. **$^1$H NMR** (400 MHz, DMSO-$d_6$) δ = 9.11 (d, *J* = 2.4 Hz, 1H), 9.01 (d, *J* = 2.0 Hz, 1H), 8.97 (d, *J* = 8.4 Hz, 1H), 8.64 (t, *J* = 2.4 Hz, 1H), 8.15 (s, 1H), 7.94 (d, *J* = 9.6 Hz, 1H), 7.23 (d, *J* = 9.6 Hz, 1H), 6.80 (br d, *J* = 11.2 Hz, 2H), 6.71 (s, 1H), 5.55 (s, 2H), 5.06 (quin, *J* = 7.2 Hz, 1H), 4.09 - 3.95 (m, 1H), 1.45 (d, *J* = 7.2 Hz, 3H), 1.18 - 1.11 (m, 4H). |
| 1-57 | 1-[5-[1-(oxetan-3-yl)triazol-5-yl]-3-pyridyl]-6-oxo-N-[(1R)-1-[2-fluoro-3-(trifluoromethyl)phenyl]ethyl]pyridazine-3-carboxamide 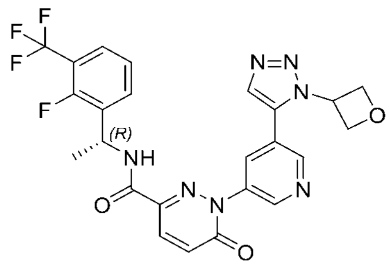 | **LCMS** : $t_R$ = 2.019 min, MS (ESI) m/z = 530.1 [M+H] $^+$. **$^1$H NMR** (400 MHz, DMSO-$d_6$) δ = 9.18 - 9.10 (m, 2H), 8.80 (d, *J* = 2.0 Hz, 1H), 8.34 (t, *J* = 2.0 Hz, 1H), 8.19 (s, 1H), 7.93 (d, *J* = 9.6 Hz, 1H), 7.84 (t, *J* = 7.6 Hz, 1H), 7.69 (t, *J* = 7.2 Hz, 1H), 7.41 (t, *J* = 8.0 Hz, 1H), 7.24 (d, *J* = 9.6 Hz, 1H), 5.93 - 5.82 (m, 1H), 5.48 - 5.40 (m, 1H), 5.08 - 5.03 (m, 2H), 5.01 - 4.97 (m, 2H), 1.53 (d, *J* = 7.2 Hz, 3H). |
| 1-58 | N-[(1R)-1-amino-5-(trifluoromethyl)phenyl]ethyl]-6-oxo-1-[5-[1-(trideuteriomethyl)triazol-5-yl]-3-pyridyl]pyridazine-3-carboxamide 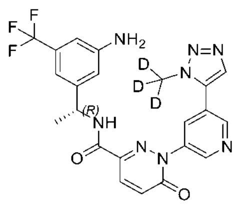 | **LCMS** : $t_R$ = 1.695 min, MS (ESI) m/z = 488.2 [M+H] $^+$. **$^1$H NMR** (400 MHz, DMSO-$d_6$) δ = 9.17 - 9.08 (m, 2H), 8.93 (d, *J* = 2.0 Hz, 1H), 8.54 (t, *J* = 2.0 Hz, 1H), 8.14 (s, 1H), 7.94 (d, *J* = 9.6 Hz, 1H), 7.41 - 7.28 (m, 2H), 7.25 - 7.20 (m, 2H), 5.21 - 5.11 (m, 3H), 1.50 (d, *J* = 7.2 Hz, 3H). |
| 1-59 | (R)-N-(1-(2-fluoro-3-(trifluoromethyl)phenyl)ethyl)-5-(2-hydroxypropane-2-yl)-1-(5-(1-methyl-1H-1,2,3-triazol-5-yl)pyridin-3-yl)-6-oxo-1,6-dihydropyridazine-3-carboxamide 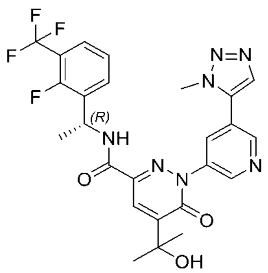 | **LCMS** : $t_R$ = 1.833 min, MS (ESI) m/z = 546.0 [M+H] $^+$. **$^1$H NMR** (400 MHz, DMSO-$d_6$) δ = 9.14 - 9.04 (m, 2H), 8.92 (d, *J* = 1.6 Hz, 1H), 8.48 (s, 1H), 8.17 - 8.02 (m, 2H), 7.82 (br t, *J* = 7.2 Hz, 1H), 7.68 (br t, *J* = 7.2 Hz, 1H), 7.40 (t, *J* = 7.6 Hz, 1H), 5.51 (s, 1H), 5.43 (quin, *J* = 7.2 Hz, 1H), 4.17 (s, 3H), 1.53 - 1.47 (m, 9H). |

(continued)

| No. | Chemical Name/Structure | Spectra (LCMS / $^1$H NMR) |
|---|---|---|
| 1-60 | 1-[5-(1-cyclopropyl-1H-1,2,3-triazol-5-yl)-3-pyridyl]-6-oxo-N-[(1R)-2,2,2-trideuterio-1-[3-(1,1-difluoro-2-hydroxy-ethyl)-2-fluoro-phenyl]ethyl]pyridazine-3-carboxamide 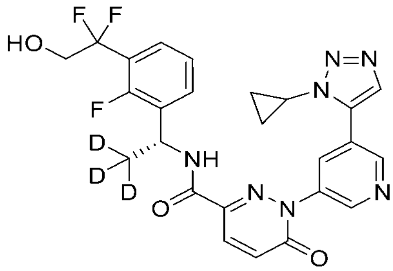 | **LCMS** : $t_R$ = 1.588 min, MS (ESI) m/z = 529.2 [M+H] $^+$. **$^1$H NMR** (400 MHz, DMSO-$d_6$) δ = 9.12 (d, *J* = 2.4 Hz, 1H), 9.06 (d, *J* = 8.0 Hz, 1H), 9.02 (d, *J* = 2.0 Hz, 1H), 8.65 (t, *J* = 2.0 Hz, 1H), 8.17 (s, 1H), 7.93 (d, *J* = 10.0 Hz, 1H), 7.64 (t, *J* = 6.8 Hz, 1H), 7.44 (t, *J* = 6.8 Hz, 1H), 7.28 (t, *J* = 7.6 Hz, 1H), 7.23 (d, *J* = 10.0 Hz, 1H), 5.71 (br s, 1H), 5.40 (d, *J* = 8.0 Hz, 1H), 4.07 - 4.00 (m, 1H), 3.91 (dt, *J* = 3.5, 14.0 Hz, 2H), 1.19 - 1.13 (m, 4H). |
| 1-61 | N-{(R)-1-[2-fluoro-3-(trifluoromethyl)phenyl]ethyl}-5-(hydroxymethyl)-1-[5-(1-methyl-1H-1,2,3-triazol-5-yl)-3-pyridyl]-6-oxo-1,6-dihydropyridazine-3-carboxamide 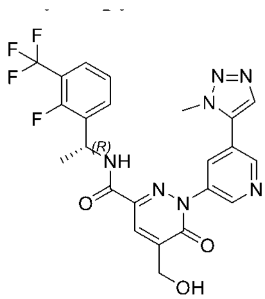 | **LCMS:** MS (ESI) m/z = 518.1 [M+H]$^+$. **$^1$H NMR** (400MHz, DMSO-$d_6$) δ = 9.15-9.09 (m, 2H), 8.92 (d, *J* = 2.0 Hz, 1H), 8.51 (t, *J* = 2.0 Hz, 1H), 8.13 (s, 1H), 7.91 (s, 1H), 7.83 (t, *J* = 7.2 Hz, 1H), 7.68 (t, *J* = 7.2 Hz, 1H), 7.40 (t, *J* = 8.0 Hz, 1H), 5.64 (t, *J* = 5.6 Hz, 1H), 5.44 (t, *J* = 7.2 Hz, 1H), 4.47 (br d, *J* = 4.4 Hz, 2H), 4.17 (s, 3H), 1.52 (d, *J* = 7.2 Hz, 3H). |
| 1-62 | N-{(R)-1-[2-fluoro-3-(trifluoromethyl)phenyl]ethyl}-5-acetyl-1-[5-(1-methyl-1H-1,2,3-triazol-5-yl)-3-pyridyl]-6-oxo-1,6-dihydropyridazine-3-carboxamide 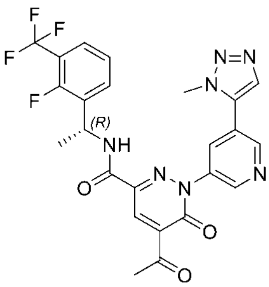 | **LCMS** : $t_R$ = 1.295 min, MS (ESI) m/z = 530.2 [M+H] $^+$. **$^1$H NMR** (400 MHz, DMSO-$d_6$) δ = 9.20 (br d, *J* = 7.6 Hz, 1H), 9.11 (d, *J* = 2.0 Hz, 1H), 8.96 (d, *J* = 1.6 Hz, 1H), 8.51 (s, 1H), 8.13 (d, *J* = 1.6 Hz, 2H), 7.82 (br t, *J* = 7.2 Hz, 1H), 7.68 (br t, *J* = 7.2 Hz, 1H), 7.40 (t, *J* = 8.0 Hz, 1H), 5.44 (quin, *J* = 7.2 Hz, 1H), 4.17 (s, 3H), 2.60 (s, 3H), 1.52 (br d, *J* = 7.2 Hz, 3H). |
| 1-63 | N-{(R)-1-[2-fluoro-3-(trifluoromethyl)phenyl]ethyl }-1-[5-(1-methyl-1H-1,2,3-triazol-5-yl)-3-pyridyl]-6-oxo-5-phenyl-1,6-dihydropyridazine-3-carboxamide 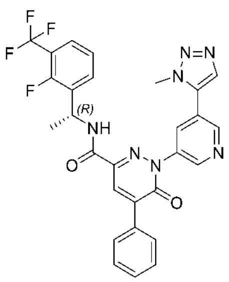 | **LCMS** : $t_R$ = 0.722 min, MS (ESI) m/z = 564.2 [M+H] $^+$. **$^1$H NMR** (400 MHz, DMSO-$d_6$) δ = 9.21-9.12 (m, 2H), 8.94 (d, *J* = 2.0 Hz, 1H), 8.56 (t, *J* = 2.0 Hz, 1H), 8.14 (s, 1H), 8.01 (s, 1H), 7.91-7.83 (m, 3H), 7.69 (t, *J* = 7.2 Hz, 1H), 7.52-7.47 (m, 3H), 7.42 (t, *J* = 8.0 Hz, 1H), 5.46 (quin, *J* = 7.2 Hz, 1H), 4.18 (s, 3H), 1.53 (d, *J* = 7.2 Hz, 3H). |

(continued)

| No. | Chemical Name/Structure | Spectra (LCMS / $^1$H NMR) |
|---|---|---|
| 1-64 | N-{(R)-1-[2-fluoro-3-(trifluoromethyl)phenyl]ethyl}-5-(1-cyclohexen-1-yl)-1-[5-(1-methyl-1H-1,2,3-triazol-5-yl)-3-pyridyl]-6-oxo-1,6-dihydropyridazine-3-carboxamide 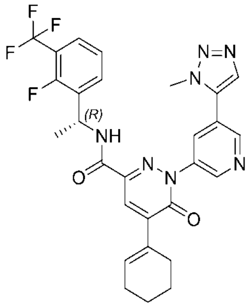 | **LCMS** : $t_R$ = 0.732 min, MS (ESI) m/z = 568.2 [M+H] $^+$. **$^1$H NMR** (400 MHz, DMSO-$d_6$) δ = 9.13 - 9.03 (m, 2H), 8.91 (d, *J* = 1.6 Hz, 1H), 8.48 (s, 1H), 8.13 (s, 1H), 7.82 (br t, *J* = 7.2 Hz, 1H), 7.70 - 7.65 (m, 2H), 7.40 (t, *J* = 7.6 Hz, 1H), 7.16 (br s, 1H), 5.43 (br t, *J* = 7.2 Hz, 1H), 4.16 (s, 3H), 2.34 (br s, 2H), 2.23 (br d, *J* = 2.4 Hz, 2H), 1.69 (br d, *J* = 4.8 Hz, 2H), 1.59 (br d, *J* = 5.6 Hz, 2H), 1.51 (br d, *J* = 7.2 Hz, 3H). |
| 1-65 | N-{(R)-1-[2-fluoro-3-(trifluoromethyl)phenyl]ethyl}-5-cyclohexyl-1-[5-(1-methyl-1H-1,2,3-triazol-5-yl)-3-pyridyl]-6-oxo-1,6-dihydro-pyridazine-3-carboxamide 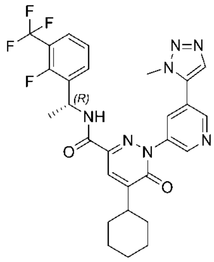 | **LCMS** : $t_R$ = 1.859 min, MS (ESI) m/z = 569.9 [M+H] $^+$. **$^1$H NMR** (400 MHz, DMSO-$d_6$) δ = 9.11 (d, *J* = 2.4 Hz, 1H), 9.08 (d, *J* = 7.6 Hz, 1H), 8.91 (d, *J* = 2.0 Hz, 1H), 8.50 (t, *J* = 2.0 Hz, 1H), 8.13 (s, 1H), 7.83 (t, *J* = 7.6 Hz, 1H), 7.71 - 7.65 (m, 2H), 7.40 (t, *J* = 7.6 Hz, 1H), 5.42 (t, *J* = 7.6 Hz, 1H), 4.17 (s, 3H), 2.87 - 2.74 (m, 1H), 1.92 - 1.59 (m, 6H), 1.51 (d, *J* = 7.2 Hz, 3H), 1.39 - 1.30 (m, 4H). |
| 1-66 | N-{(R)-1-[2-fluoro-3-(trifluoromethyl)phenyl]ethyl}-5-ethyl-1-[5-(1-methyl-1H-1,2,3-triazol-5-yl)-3-pyridyl]-6-oxo-1,6-dihydropyrida-zine-3-carboxamide 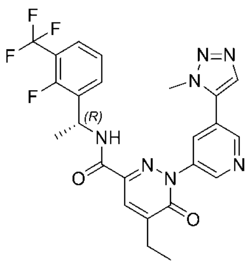 | **LCMS** : $t_R$ = 0.720 min, MS (ESI) m/z = 516.1 [M+H] $^+$. **$^1$H NMR** (400 MHz, DMSO-$d_6$) δ = 9.00 (d, *J* = 2.4 Hz, 1H), 8.77 (d, *J* = 2.0 Hz, 1H), 8.22 (t, *J* = 2.0 Hz, 1H), 7.92 (s, 1H), 7.88 (s, 1H), 7.59 - 7.53 (m, 2H), 7.45 (br d, *J* = 8.0 Hz, 1H), 7.26 - 7.22 (m, 1H), 5.45 (quin, *J* = 7.2 Hz, 1H), 4.19 (s, 3H), 2.71 (q, *J* = 7.6 Hz, 2H), 1.65 (d, *J* = 7.2 Hz, 3H), 1.29 (t, *J* = 7.6 Hz, 3H). |
| 1-67 | (R)-5'-(1-cyclopropyl-1H-1,2,3-triazol-5-yl)-2'-fluoro-N-(1-(2-fluoro-3-(trifluoromethyl)phenyl)ethyl)-2-oxo-2H-[1,3'-bipyridine]-5-car-boxamide 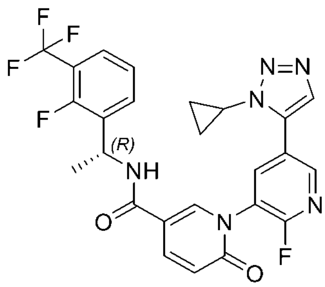 | **LCMS** : $t_R$ = 0.563 min, MS (ESI) m/z = 531.3 [M+H]$^+$. **$^1$H NMR** (400 MHz, DMSO-$d_6$) δ = 8.78-8.74 (m, 2H), 8.68 (dd, *J* = 2.4, 8.8 Hz, 1H), 8.47 (d, *J* = 2.4 Hz, 1H), 8.13 (s, 1H), 8.05 (dd, *J* = 2.8, 10.0 Hz, 1H), 7.77 (t, *J* = 7.2 Hz, 1H), 7.67 (t, *J* = 7.2 Hz, 1H), 7.39 (t, *J* = 8.0 Hz, 1H), 6.64 (d, *J* = 9.6 Hz, 1H), 5.36 (quin, *J* = 7.2 Hz, 1H), 4.08-4.00 (m, 1H), 1.47 (d, *J* = 7.2 Hz, 3H), 1.16-1.08 (m, 4H). |

(continued)

| No. | Chemical Name/Structure | Spectra (LCMS / $^1$H NMR) |
|---|---|---|
| 1-68 | 1-[5-(1-cyclopropyl-1H-1,2,3-triazol-5-yl)-3-pyridyl]-N-[(1R)-1-[2-fluoro-3-(trifluoromethyl)phenyl]ethyl]-2-oxo-2H-[1,3'-bipyridine]-5-carboxamide 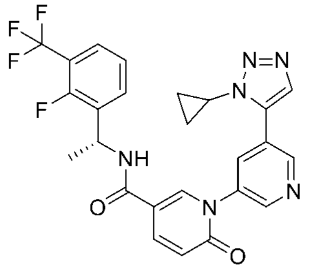 | **LCMS** : $t_R$ = 1.357 min, MS (ESI) m/z = 513.0 $[M+H]^+$. **$^1$H NMR** (400 MHz, DMSO-$d_6$) δ = 9.05 (d, *J* = 1.2 Hz, 1H), 8.87 (d, *J* = 2.0 Hz, 1H), 8.75 (br d, *J* = 6.8 Hz, 1H), 8.51 (d, *J* = 2.4 Hz, 1H), 8.46 (s, 1H), 8.15 (s, 1H), 7.99 (dd, *J* = 2.4, 9.6 Hz, 1H), 7.77 (br t, *J* = 7.2 Hz, 1H), 7.67 (br t, *J = 7.2* Hz, 1H), 7.39 (t, *J* = 7.6 Hz, 1H), 6.61 (d, *J* = 9.6 Hz, 1H), 5.36 (quin, *J* = 7.2 Hz, 1H), 4.05 (tt, *J* = 3.6, 7.2 Hz, 1H), 1.47 (d, *J* = 7.0 Hz, 3H), 1.18 - 1.09 (m, 4H). |
| 1-69 | *N*-[(R)-1-[2-fluoro-3-(trifluoromethyl)phenyl]ethyl]-1-[5-(1-methyl-1H-1,2,3-triazol-5-yl)-3-pyridyl]-6-oxo-5-(trifluoro-methyl)-pyridazine-3-carboxamide 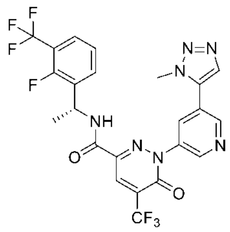 | **LCMS** : $t_R$ = 1.919 min, MS (ESI) m/z = 555.9 $[M+H]^+$. **$^1$H NMR** (400 MHz, DMSO-$d_6$) δ = 9.29 (br d, *J* = 7.2 Hz, 1H), 9.09 (br s, 1H), 8.97 (br s, 1H), 8.50 (br s, 1H), 8.25 (br s, 1H), 8.14 (s, 1H), 7.83 (br s, 1H), 7.69 (br s, 1H), 7.41 (br t, *J = 7.2* Hz, 1H), 5.50 - 5.39 (m, 1H), 4.16 (s, 3H), 1.53 (br d, *J* = 6.4 Hz, 3H). |
| 1-70 | (*R*)-N-(1-(3-(1,1-difluoro-2-hydroxyethyl)-2-fluorophenyl)ethyl)-1-(5-(1-(oxetan-3-yl)-1H-1,2,3-triazol-5-yl)pyridin-3-yl)-6-oxo-1,6-dihydropyridazine-3-carboxamide 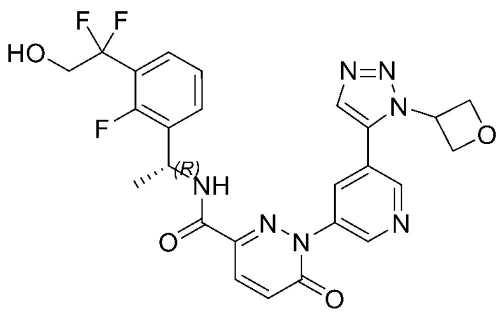 | **LCMS :** $t_R$ = 0.788 min, MS (ESI) m/z = 542.0 $[M+H]^+$. **$^1$H NMR** (400 MHz, DMSO-$d_6$) δ= 9.15 (d, *J* = 2.4 Hz, 1H), 9.09 (d, *J* = 8.0 Hz, 1H), 8.79 (d, *J* = 2.0 Hz, 1H), 8.34 (t, *J* = 2.0 Hz, 1H), 8.19 (s, 1H), 7.93 (d, *J* = 9.6 Hz, 1H), 7.65 (br t, *J* = 7.2 Hz, 1H), 7.44 (br t, *J* = 6.8 Hz, 1H), 7.28 (t, *J* = 7.6 Hz, 1H), 7.24 (d, *J* = 9.6 Hz, 1H), 5.90 - 5.83 (m, 1H), 5.71 (t, *J* = 6.4 Hz, 1H), 5.43 (t, *J* = 7.2 Hz, 1H), 5.05 (dt, *J* = 3.6, 6.4 Hz, 2H), 5.01 - 4.97 (m, 2H), 3.96 - 3.87 (m, 2H), 1.50 *(d, J* = 7.2 Hz, 3H). |

(continued)

| No. | Chemical Name/Structure | Spectra (LCMS / $^1$H NMR) |
|---|---|---|
| 1-71 | (*R*)-N-(1-(3-(1,1-difluoro-2-hydroxyethyl-2,2-dideuterio)-2-fluorophenyl)ethyl)-1-(5-(1-(oxetan-3-yl)-1H-1,2,3-triazol-5-yl)pyridin-3-yl)-6-oxo-1,6-dihydropyridazine-3-carboxamide 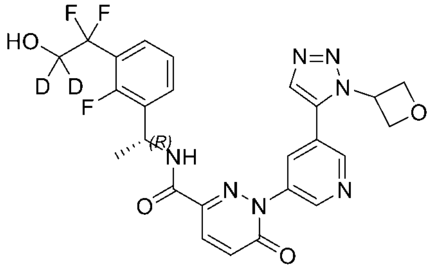 | **LCMS** : $t_R$ = 1.444 min, MS (ESI) m/z = 544.2 [M+H]$^+$. **$^1$H NMR** (400 MHz, DMSO-$d_6$) δ= 9.15 (s, 1H), 9.08 (br d, *J* = 8.4 Hz, 1H), 8.79 (s, 1H), 8.34 (s, 1H), 8.18 (s, 1H), 7.93 (d, *J* = 9.6 Hz, 1H), 7.65 (br t, *J* = 7.2 Hz, 1H), 7.44 (br t, *J* = 6.8 Hz, 1H), 7.28 (t, *J* = 7.6 Hz, 1H), 7.23 (d, *J* = 10.0 Hz, 1H), 5.91 - 5.82 (m, 1H), 5.67 (s, 1H), 5.48 - 5.38 (m, 1H), 5.07 - 5.03 (m, 2H), 5.01 - 4.96 (m, 2H), 1.50 (br d, *J* = 7.2 Hz, 3H). **Deuterated ratio:** 98.85%. |
| 1-72 | (R)-N-(1-(3-(1,1-difluoro-2-hydroxyethyl)-2-fluorophenyl) ethyl)-1-(5-(1-methyl-1H-1,2,3-triazol-5-yl)pyridin-3-yl)-6-oxo-5-(trifluoromethyl)-1,6-dihydropyridazine-3-carboxamide 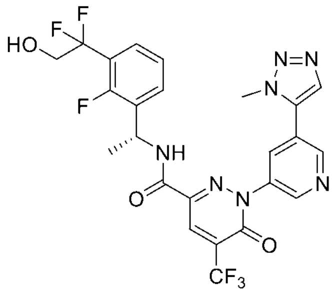 | **LCMS** : $t_R$ = 0.940 min, MS (ESI) m/z = 567.9 [M+H]$^+$. **$^1$H NMR** (400 MHz, DMSO-$d_6$) δ= 9.23 (d, *J* = 8.4 Hz, 1H), 9.10 (d, *J* = 2.4 Hz, 1H), 8.97 (d, *J* = 2.0 Hz, 1H), 8.50 (t, *J* = 2.0 Hz, 1H), 8.26 (s, 1H), 8.14 (s, 1H), 7.69 - 7.62 (m, 1H), 7.45 (br t, *J* = 6.8 Hz, 1H), 7.32 - 7.25 (m, 1H), 5.71 (t, *J* = 6.4 Hz, 1H), 5.44 (quin, *J* = 7.2 Hz, 1H), 4.17 (s, 3H), 3.91 (dt, *J* = 6.4, 14.4 Hz, 2H), 1.50 (d, *J* = 7.2 Hz, 3H). |

**Example 2: N-[(1R)-1-[3-amino-5-(difluoromethyl)phenyl]ethyl]-1-[5-(3-methyltriazol-4-yl)-3-pyridyl]-6-oxo-pyridazine-3-carboxamide**

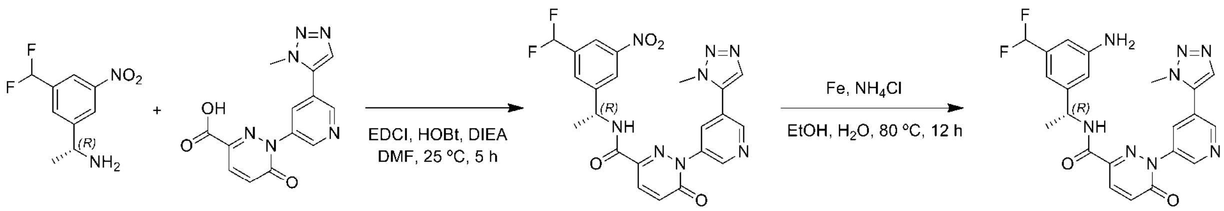


A-6 4-1 5-1 Example 2

Step 1: Synthesis of N-[(1R)-1-[3-(difluoromethyl)-5-nitro-phenyl]ethyl]-1-[5-(3-methyltriazol-4-yl)-3-pyridyl]-6-oxo-pyridazine-3-carboxamide

To a solution of **Intermediate 4-1** (88 mg, 295.05 umol) and **Intermediate A-6** (52.18 mg, 206.53 umol, HCl) in DMF (2 mL) was added HOBt (59.80 mg, 442.57 umol), EDCI (84.84 mg, 442.57 umol) and DIEA (114.40 mg, 885.14 umol, 154.18 uL). The mixture was degassed and purged with $N_2$ for 3 times, and then stirred at 30 °C for 5 h under $N_2$ atmosphere. The reaction mixture was poured into water (10 mL) and extracted with EtOAc (30 mL×3). The combined organic layers were washed with brine (10 mL×3), dried over anhydrous $Na_2SO_4$ and concentrated under reduced pressure to give a crude product. The crude product was purified by silica gel column chromatography (2% MeOH in DCM) to give N-[(1R)-1-[3-(difluoromethyl)-5-nitro-phenyl]ethyl]-1-[5-(3-methyltriazol-4-yl)-3-pyridyl]-6-oxo-pyridazine-3-carboxamide (66 mg, 33.34% yield, 74% purity) as brown oil. MS (ESI) m/z = 497.1 [M+H]$^+$.

Step 2: Synthesis of N-[(1R)-1-[3-amino-5-(difluoromethyl)phenyl]ethyl]-1-[5-(3-methyltriazol-4-yl)-3-pyridyl]-6-oxo-pyridazine-3-carboxamide

To a solution of N-[(1R)-1-[3-(difluoromethyl)-5-nitro-phenyl]ethyl]-1-[5-(3-methyltriazol-4-yl)-3-pyridyl]-6-oxo-pyridazine-3-carboxamide (62 mg, 124.89 umol) in EtOH (3 mL) and $H_2O$ (1 mL) was added Fe (34.87 mg, 624.46 umol) and $NH_4Cl$ (66.81 mg, 1.25 mmol). The mixture was degassed and purged with $N_2$ for 3 times, and then stirred at 80 °C for 12 h under $N_2$ atmosphere. The reaction mixture was concentrated by filtration under reduced pressure, and then poured into water (10 mL) and extracted with EtOAc (10 mL×3). The combined organic layers were washed with brine (10 mL×3), dried over anhydrous $Na_2SO_4$ and concentrated under reduced pressure to give a crude product. The crude product was purified by prep-HPLC (Welch Xtimate C18 150*30 mm*5 μm; mobile phase: [water($NH_3H_2O$+ $NH_4HCO_3$)-MeCN]; B%: 8%-48%, 28 min). The remaining solvent was removed by lyophilization to give a **compound of Example 2** (11.9 mg, 20.24% yield, 99.08% purity) as a white solid. $^1H$ NMR (400 MHz, DMSO-$d_6$) δ = 9.12 (d, *J* = 2.4 Hz, 1H), 8.95 - 8.90 (m, 2H), 8.51 (t, *J* = 2.4 Hz, 1H), 8.12 (s, 1H), 7.95 (d, *J* = 10.0 Hz, 1H), 7.23 (d, *J* = 10.0 Hz, 1H), 6.95 (s, 1H), 6.81 (s, 1H), 6.69 (s, 2H), 6.67 (s, 1H), 6.59 (s, 1H), 5.37 (s, 2H), 5.08 - 5.01 (m, 1H), 4.16 (s, 3H), 1.46 (d, *J* = 7.2 Hz, 3H); MS (ESI) m/z = 467.2 $[M+H]^+$.

**Examples 2-1 to 2-7**

The compounds in Table 2 below were prepared in the same manner as in Example 2 through a two-step method of amide coupling followed by reducing a nitro group, using the corresponding intermediates described in the Preparation Examples.

[Table 2]

| No. | Chemical Name/Structure | Spectra (LCMS / $^1H$ NMR) |
|---|---|---|
| 2-1 | N-[(1R)-1-[5-amino-2-fluoro-3-(trifluoromethyl)phenyl]ethyl]-1-[5-(3-methyltriazol-4-yl)-3-pyridyl]-6-oxo-pyridazine-3-carboxamide 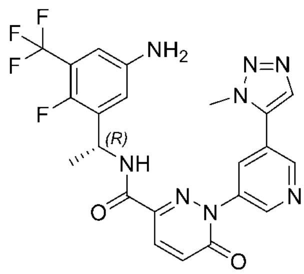 | **LCMS** : $t_R$ = 1.724 min, MS (ESI) m/z = 503.2 $[M+H]^+$. **$^1$H NMR** (400 MHz, DMSO-$d_6$) δ = 9.14 (d, *J* = 2.4 Hz, 1H), 9.02 (d, *J* = 7.6 Hz, 1H), 8.93 (d, *J* = 2.0 Hz, 1H), 8.52 (t, *J* = 2.0 Hz, 1H), 8.13 (s, 1H), 7.94 (d, *J* = 9.6 Hz, 1H), 7.24 (d, *J* = 9.6 Hz, 1H), 6.84 (dd, *J* = 2.4, 6.0 Hz, 1H), 6.73 (dd, *J* = 2.8, 5.6 Hz, 1H), 5.40 (s, 2H), 5.30-5.23 (m, 1H), 4.17 (s, 3H), 1.46 (d, *J* = 7.2 Hz, 3H). |
| 2-2 | N-[(1R)-1-[5-amino-2-methyl-3-(trifluoromethyl)phenyl]ethyl]-1-[5-(3-methyltriazol-4-yl)-3-pyridyl]-6-oxo-pyridazine-3-carboxamide 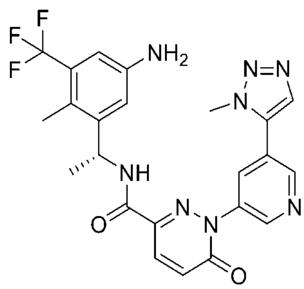 | **LCMS** : $t_R$ = 1.691 min, MS (ESI) m/z = 499.4 $[M+H]^+$. **$^1$H NMR** (400 MHz, DMSO-$d_6$) δ = 9.33 *(d, J* = 7.2 Hz, 1H), 9.16 (d, *J* = 2.4 Hz, 1H), 8.94 (d, *J* = 2.0 Hz, 1H), 8.62 (t, *J* = 2.0 Hz, 1H), 8.16 (s, 1H), 7.91 (d, *J* = 10.0 Hz, 1H), 7.73 (s, 1H), 7.61 (d, *J* = 1.6 Hz, 1H), 7.23 (d, *J* = 10.0 Hz, 1H), 5.39-5.23 (m, 3H), 4.18 (s, 3H), 2.51-2.49 (m, 3H), 2.47 (s, 3H), 1.48 (d, *J* = 6.8 Hz, 3H). |

(continued)

| No. | Chemical Name/Structure | Spectra (LCMS / $^1$H NMR) |
|---|---|---|
| 2-3 | N-[(1R)-1-[5-amino-3-(difluoromethyl)-2-fluoro-phenyl]ethyl]-5-methyl-1-[5-(3-methyltriazol-4-yl)-3-pyridyl]-6-oxo-pyridazine-3-carboxamide 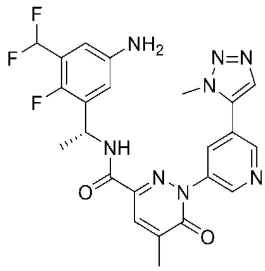 | **LCMS** : $t_R$ = 2.242 min, MS (ESI) m/z = 467.2 [M+H] $^+$. **$^1$H NMR** (400 MHz, DMSO-$d_6$) δ = 9.13 (d, *J* = 2.4 Hz, 1H), 8.93-8.89 (m, 2H), 8.52 (t, *J* = 2.4 Hz, 1H), 8.13 (s, 1H), 7.87 (d, *J* = 1.2 Hz, 1H), 7.19 (s, 1H), 7.05 (s, 1H), 6.91 (s, 1H), 6.74-6.70 (m, 1H), 6.62 (dd, *J* = 2.8, 5.2 Hz, 1H), 5.27-5.21 (m, 3H), 4.16 (s, 3H), 2.22 (d, *J* = 1.2 Hz, 3H), 1.44 (d, *J* = 7.2 Hz, 3H). |
| 2-4 | N-[(1R)-1-[5-amino-2-methyl-3-(trifluoromethyl)phenyl]ethyl]-1-[5-(2-methylpyrazol-3-yl)-3-pyridyl]-6-oxo-pyridazine-3-carboxamide 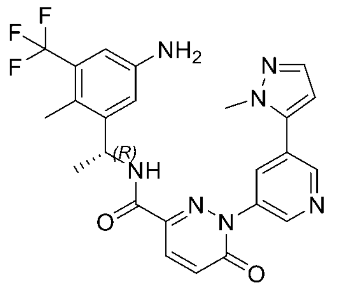 | **LCMS :** $t_R$ = 1.876 min, MS (ESI) m/z = 498.2 [M+H]$^+$. **$^1$H NMR** (400MHz, DMSO-$d_6$) δ = 9.07 (d, *J* = 2.0 Hz, 1H), 8.95 (d, *J* = 7.6 Hz, 1H), 8.84 (d, *J* = 2.0 Hz, 1H), 8.42 (t, *J* = 2.0 Hz, 1H), 7.92 (d, *J* = 10.0 Hz, 1H), 7.56 (d, *J* = 2.0 Hz, 1H), 7.22 (d, *J* = 10.0 Hz, 1H), 6.86 (s, 1H), 6.79 (d, *J* = 2.0 Hz, 1H), 6.63 (d, *J* = 2.0 Hz, 1H), 5.33 - 5.22 (m, 3H), 3.93 (s, 3H), 2.24 (s, 3H), 1.41 (d, *J* = 6.8 Hz, 3H). |
| 2-5 | N-[(1R)-1-[5-amino-2-fluoro-3-(trifluoromethyl)phenyl]ethyl]-5-methyl-1-[5-(3-methyltriazol-4-yl)-3-pyridyl]-6-oxo-pyridazine-3-carboxamide 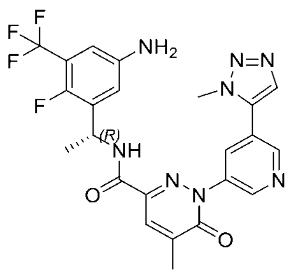 | **LCMS** : $t_R$ = 1.929 min, MS (ESI) m/z = 517.1 [M+H]$^+$. **$^1$H NMR** (400 MHz, DMSO-$d_6$) δ = 9.22 (d, *J* = 2.4 Hz, 1H), 9.05 (d, *J* = 8.0 Hz, 1H), 8.99 (d, *J* = 2.0 Hz, 1H), 8.60 (t, *J* = 2.0 Hz, 1H), 8.21 (s, 1H), 7.96 (d, *J* = 1.2 Hz, 1H), 6.91 (dd, *J* = 2.4, 5.6 Hz, 1H), 6.81 (dd, *J* = 2.8, 5.2 Hz, 1H), 5.49 (s, 2H), 5.37-5.31 (m, 1H), 4.25 (s, 3H), 2.30 *(d, J* = 1.2 Hz, 3H), 1.51 (d, *J* = 7.2 Hz, 3H). |
| 2-6 | N-[(1R)-1-[5-amino-2-methyl-3-(trifluoromethyl)phenyl]ethyl]-5-methyl-1-[5-(3-methyltriazol-4-yl)-3-pyridyl]-6-oxo-pyridazine-3-carboxamide 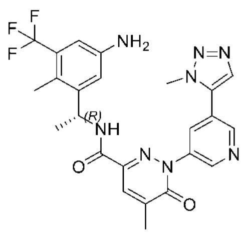 | **LCMS** : $t_R$ = 1.929 min, MS (ESI) m/z = 513.1 [M+H]$^+$. **$^1$H NMR** (400 MHz, DMSO-$d_6$) δ = 9.12 (d, *J* = 2.4 Hz, 1H), 8.91-8.86 (m, 2H), 8.50 (t, *J* = 2.0 Hz, 1H), 8.12 (s, 1H), 7.86 (s, 1H), 6.86 (d, *J* = 1.6 Hz, 1H), 6.79 (d, *J* = 2.0 Hz, 1H), 5.28 (s, 2H), 5.25 (br d, *J* = 7.6 Hz, 1H), 4.15 (s, 3H), 2.23 (s, 3H), 2.21 (s, 3H), 1.41 (d, *J* = 6.8 Hz, 3H). |

(continued)

| No. | Chemical Name/Structure | Spectra (LCMS / $^1$H NMR) |
|---|---|---|
| 2-7 | N-[(1R)-1-[3-amino-5-(difluoromethyl)phenyl]ethyl]-5-methyl-1-[5-(3-methyltriazol-4-yl)-3-pyridyl]-6-oxo-pyridazine-3-carboxamide 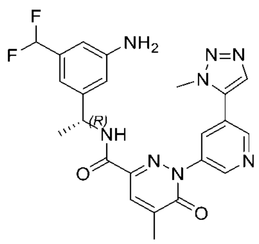 | **LCMS** : $t_R$ = 1.463 min, MS (ESI) m/z = 481.1 [M+H]$^+$. **$^1$H NMR** (400 MHz, DMSO-$d_6$) δ = 9.11 (d, *J* = 2.4 Hz, 1H), 8.90 - 8.86 (m, 2H), 8.50 (t, *J* = 2.0 Hz, 1H), 8.12 (s, 1H), 7.88 (d, *J* = 1.2 Hz, 1H), 6.94 (s, 1H), 6.80 (s, 1H), 6.69 (br s, 2H), 6.66 (s, 1H), 6.58 (s, 1H), 5.36 (s, 2H), 5.06 - 5.02 (m, 1H), 4.15 (s, 3H), 2.22 (s, 3H), 1.45 (d, *J* = 7.2 Hz, 3H) |

**Example 3: 5-cyano-N-[(1R)-1-[3-(1,1-difluoro-2-hydroxy-ethyl)-2-fluorophenyl]ethyl]-1-[5-(3-methyltriazol-4-yl)-3-pyridyl]-6-oxo-pyridazine-3-carboxamide**

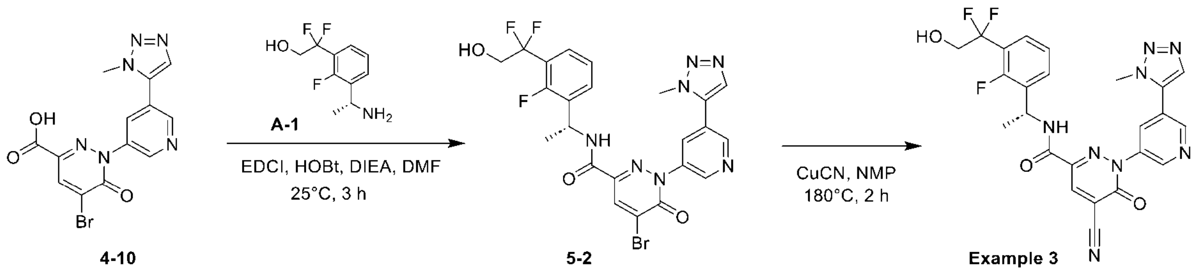


4-10 5-2 Example 3

Step 1: Synthesis of 5-bromo-N-[(1R)-1-[3-(1,1-difluoro-2-hydroxy-ethyl)-2-fluorophenyl]ethyl]-1-[5-(3-methyltriazol-4-yl)-3-pyridyl]-6-oxo-pyridazine-3-carboxamide

To a solution of Intermediate 4-10 (80 mg, 212.12 μmol) and Intermediate A-1 (54.23 mg, 212.12 μmol, HCl) in DMF (4 mL) was added EDCI (81.33 mg, 424.23 μmol), HOBt (57.32 mg, 424.23 μmol) and DIEA (82.24 mg, 636.35 μmol, 110.84 μL). The mixture was degassed and purged with $N_2$ for 3 times, and then stirred at 30 °C for 5 h under $N_2$ atmosphere. The reaction mixture was poured into water (10 mL) and extracted with EtOAc (30 mL×3). The combined organic layers were washed with brine (10 mL×3), dried over anhydrous $Na_2SO_4$ and concentrated under reduced pressure to give a crude product. The crude product was purified by silica gel column chromatography (2% MeOH in DCM) to give 5-bromo-N-[(1R)-1-[3-(1,1-difluoro-2-hydroxy-ethyl)-2-fluoro-phenyl]ethyl]-1-[5-(3-methyltriazol-4-yl)-3-pyridyl]-6-oxo-pyridazine-3-carboxamide (85 mg, 69.29% yield) as a yellow solid. MS (ESI) m/z = 580.1 [M+H]$^+$.

Step 2: Synthesis of 5-cyano-N-[(1R)-1-[3-(1,1-difluoro-2-hydroxy-ethyl)-2-fluorophenyl]ethyl]-1-[5-(3-methyltriazol-4-yl)-3-pyridyl]-6-oxo-pyridazine-3-carboxamide

To a solution of 5-bromo-N-[(1R)-1-[3-(1,1-difluoro-2-hydroxy-ethyl)-2-fluorophenyl]ethyl]-1-[5-(3-methyltriazol-4-yl)-3-pyridyl]-6-oxo-pyridazine-3-carboxamide (85 mg, 146.97 μmol) in NMP (3 mL) was added CuCN (131.63 mg, 1.47 mmol, 321.06 μL). The reaction mixture was heated in the microwave at 180 °C for 2 h. The reaction mixture was poured into water (30 mL), and extracted with EtOAc (10 mL × 3). The combined organic layers were washed with brine (20 mL × 3), dried over anhydrous $Na_2SO_4$ and concentrated under reduced pressure to give a crude product. The crude product was purified by prep-HPLC (column: Xtimate C18 150*40 mm*10 μm; mobile phase: [water ($NH_4HCO_3$)-MeCN]; gradient: 12%-52% B over 36 min). MeCN was removed under reduced pressure, and the remaining solvent was removed by lyophilization to give a **compound of Example 3** (2.4 mg, 2.69% yield) as a white solid. $^1$H NMR (400 MHz, METHANOL-$d_4$) δ = 9.09 (br s, 1H), 8.90 (br s, 1H), 8.52 (s, 1H), 8.48 (t, *J* = 2.0 Hz, 1H), 8.04 (s, 1H), 7.55 (br t, *J* = 6.8 Hz, 1H), 7.48 (br t, *J* = 6.8 Hz, 1H), 7.22 (t, *J* = 8.0 Hz, 1H), 5.49 (q, *J* = 7.2 Hz, 1H), 4.19 (s, 3H), 3.98 (t, *J* = 13.6 Hz, 2H), 1.56 (d, *J* = 7.2 Hz, 3H); MS (ESI) m/z = 525.3 [M+H]$^+$.

**Examples 3-1 to 3-5**

The compounds in Table 3 below were prepared in the same manner as in Example 3 through a two-step method of amide coupling followed by substitution with a cyano group, using the corresponding intermediates described in the Preparation Examples.

[Table 3]

| No. | Chemical Name/Structure | Spectra (LCMS / $^1$H NMR) |
|---|---|---|
| 3-1 | 5-cyano-N-[(1R)-1-[3-(difluoromethyl)-2-fluorophenyl]ethyl]-1-[5-(1-methyl-1H-1,2,3-triazol-5-yl)pyridin-3-yl]-6-oxo-1,6-dihydropyridazine-3-carboxamide 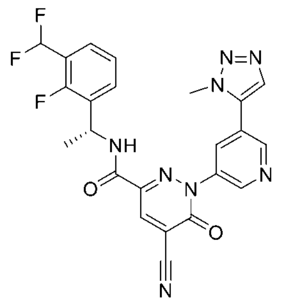 | **LCMS:** $t_R$ = 1.850 min, MS (ESI) m/z = 495.2 $[M+H]^+$. **$^1$H NMR** (400 MHz, METHANOL-$d_4$) δ = 9.09 (br s, 1H), 8.90 (br s, 1H), 8.51 (s, 1H), 8.48 (t, *J* = 2.0 Hz, 1H), 8.04 (s, 1H), 7.55 (br t, *J* = 6.8 Hz, 1H), 7.48 (br t, *J* = 6.8 Hz, 1H), 7.22 (t, *J* = 8.0 Hz, 1H), 7.15-7.08 (m, 1H), 5.49 (q, *J* = 7.2 Hz, 1H), 4.20 (s, 3H), 1.56 (d, *J* = 7.2 Hz, 3H). |
| 3-2 | 5-cyano-N-{(1R)-1-[2-fluoro-3-(trifluoromethyl)phenyl]ethyl }-1-[5-(1-methyl-1H-1,2,3-triazol-5-yl)-3-pyridyl]-6-oxo-1,6-dihydropyridazine-3-carboxamide 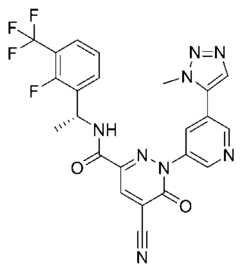 | **LCMS :** $t_R$ = 0.565 min, MS (ESI) m/z = 513.0 $[M+H]^+$. **$^1$H NMR** (400 MHz, DMSO-$d_6$) δ = 9.27 (d, *J* = 7.6 Hz, 1H), 9.09 (d, *J* = 2.4 Hz, 1H), 8.98 (d, *J* = 2.0 Hz, 1H), 8.66 (s, 1H), 8.49 (t, *J* = 2.4 Hz, 1H), 8.14 (s, 1H), 7.82 (t, *J* = 7.2 Hz, 1H), 7.69 (t, *J* =7.2 Hz, 1H), 7.41 (t, *J* = 8.0 Hz, 1H), 5.43 (quin, *J* = 7.2 Hz, 1H), 4.16 (s, 3H), 1.52 (d, *J* = 7.2 Hz, 3H). |
| 3-3 | 5-cyano-N-[(1R)-1-[2-fluoro-3-(1,1-difluoro-2-hydroxy-ethyl)-2-fluorophenyl]-1-(5-(1-methyl-1H-1,2,3-triazol-5-yl)-2-fluoropyridin-3-yl)-6-oxo-1,6-dihydropyridazine-3-carboxamide 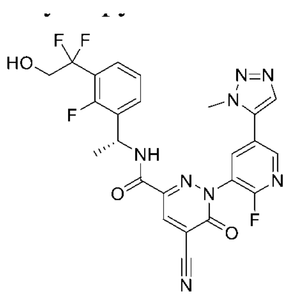 | **LCMS** : $t_R$ = 0.545 min, MS (ESI) m/z = 543.1 $[M+H]^+$. **$^1$H NMR** (400 MHz, DMSO-$d_6$) δ = 9.03 (d, *J* = 8 Hz, 1H), 8.82 - 8.73 (m, 2H), 8.67 (s, 1H), 8.15 (s, 1H), 7.59 (br t, *J* = 6.8 Hz, 1H), 7.42 (br t, *J* = 6.8 Hz, 1H), 7.31 - 7.25 (m, 1H), 5.70 (br s, 1H), 5.40 (br t, *J* = 7.2 Hz, 1H), 4.15 (s, 3H), 3.91 (br t, *J* = 14.2 Hz, 2H), 1.45 (d, *J* = 7.2 Hz, 3H). |

(continued)

| No. | Chemical Name/Structure | Spectra (LCMS / $^1$H NMR) |
|---|---|---|
| 3-4 | 5-cyano-N-[(1R)-1-[2-fluoro-3-(trifluoromethyl)phenyl]ethyl]-1-(5-(1-cyclopropyl-1H-1,2,3-triazol-5-yl)-2-fluoropyridin-3-yl)-6-oxo-1,6-dihydropyridazine-3-carboxamide 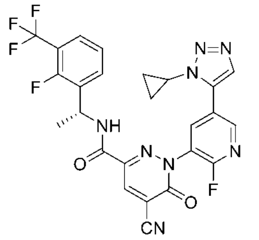 | **LCMS** : $t_R$ = 0.555 min, MS (ESI) m/z = 557.1 $[M+H]^+$. **$^1$H NMR** (400 MHz, DMSO-$d_6$) δ = 9.25 (d, *J* = 7.6 Hz, 1H), 8.76 - 8.63 (m, 3H), 8.14 (s, 1H), 7.80 (t, *J* = 7.2 Hz, 1H), 7.67 (t, *J* = 7.2 Hz, 1H), 7.39 (t, *J* = 7.6 Hz, 1H), 5.41 (quin, *J* = 7.2 Hz, 1H), 4.07 - 3.98 (m, 1H), 1.50 (d, *J* = 7.2 Hz, 3H), 1.18 - 1.12 (m, 4H). |
| 3-5 | 5-cyano-N-[(1R)-1-(2-fluoro-3-(trifluoromethyl)phenyl)ethyl]-1-[2-fluoro-5-(1-(methyl-trideuterio)-1H-1,2,3-triazol-5-yl)pyridin-3-yl]-6-oxo-1,6-dihydropyridazine-3-carboxamide 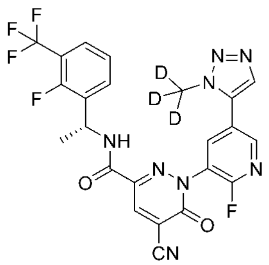 | **LCMS** : $t_R$ = 0.560 min, MS (ESI) m/z = 534.1 $[M+H]^+$. **$^1$H NMR** (400 MHz, DMSO-$d_6$) δ = 9.27 (d, *J* = 7.6 Hz, 1H), 8.76 - 8.63 (m, 3H), 8.14 (s, 1H), 7.82 (t, *J* = 7.2 Hz, 1H), 7.69 (t, *J* = 7.2 Hz, 1H), 7.41 (t, *J* = 8.0 Hz, 1H), 5.43 (quin, *J* = 7.2 Hz, 1H), 1.52 (d, *J* = 7.2 Hz, 3H). |

**Example 4: N-[(1R)-1-[2-fluoro-3-(trifluoromethyl)phenyl]ethyl]-1-[6-(3-methyltriazol-4-yl)pyridazin-4-yl]-6-oxo-pyridazine-3-carboxamide**

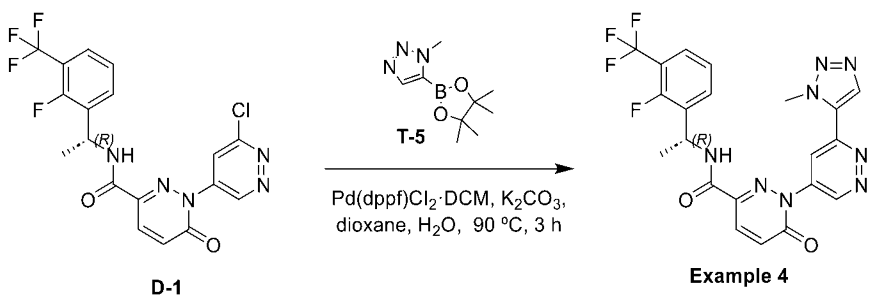


D-1

Example 4

A mixture of **Intermediate D-1** (65 mg, 147.14 μmol), **Intermediate T-5** (461.39 mg, 2.21 mmol), $K_2CO_3$ (40.67 mg, 294.27 μmol) and Pd(dppf)$Cl_2$.$CH_2Cl_2$ (12.02 mg, 14.71 μmol) in dioxane (3 mL) and $H_2O$ (0.3 mL) was degassed and purged with $N_2$ for 3 times, and then stirred at 90 °C for 3 h under $N_2$ atmosphere. The reaction mixture was poured into water (8 mL) and extracted with EtOAc (20 mL×3). The combined organic layers were washed with brine (10 mL×2), dried over anhydrous $Na_2SO_4$ and concentrated under reduced pressure, and purified by silica gel column chromatography (3% MeOH in DCM) to give a crude product. The crude product was purified by prep-HPLC (column: Welch Xtimate C18 150*30 mm*5 μm; mobile phase: [water (HCl)-MeCN]; gradient: 20%-60% B over 25 min). MeCN was removed under reduced pressure, and the remaining solvent was removed by lyophilization to give a **compound of Example 4** (4.1 mg, 5.31% yield) as a white solid. $^1$H NMR (400 MHz, DMSO-$d_6$) δ = 9.99-9.91 (m, 1H), 8.86-8.75 (m, 1H), 8.51-8.42 (m, 1H), 8.01 (d, *J* = 9.6 Hz, 1H), 7.77 (br t, *J* = 7.1 Hz, 1H), 7.61 (br t, *J* = 7.2 Hz, 1H), 7.34 (t, *J* = 7.6 Hz, 1H), 7.23 (d, *J* = 10.0 Hz, 1H), 5.53 (q, *J*

= 7.2 Hz, 1H), 4.54 (s, 3H), 1.64 (d, *J* = 7.2 Hz, 3H); MS (ESI) m/z = 489.1 [M+H] +.

**Example 5: N-[(1R)-1-[2-fluoro-3-(difluoromethyl)phenyl]ethyl]-1-[6-(3-methyltriazol-4-yl)pyridazin-4-yl]-6-oxo-pyridazine-3-carboxamide**

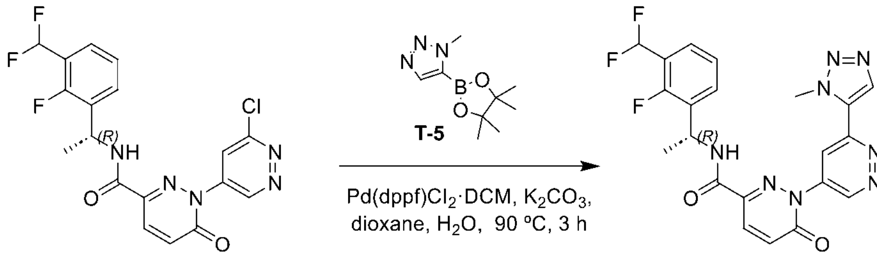


D-2 Example 5

**The compound of Example 5** was obtained as a while solid in the same manner as in Example 4 using **Intermediate D-2** (37.0 mg, 30.83% yield, 100% purity). $^1$H NMR (400 MHz, DMSO-$d_6$) δ = 9.97 (d, *J* = 2.0 Hz, 1H), 9.21 (d, *J* = 8.0 Hz, 1H), 8.75 (d, *J* = 2.0 Hz, 1H),8.46 (s, 1H), 7.94 (d, *J* = 10.0 Hz, 1H), 7.72 (br t, *J* = 7.2 Hz, 1H), 7.57 - 7.51 (m, 1H), 7.35 (d, *J* = 8.0 Hz, 1H), 7.28 (d, *J* = 10.0 Hz, 1H), 7.22 (t, *J* = 7.2 Hz, 1 H), 5.44 (quin, *J* = 7.2 Hz, 1H), 4.43 (s, 3H), 1.55 (d, *J* = 7.2 Hz, 3H); MS (ESI) m/z = 471.0 [M+H]$^+$.

**Example 6: (R)-*N*-(1-(2-fluoro-3-(trifluoromethyl)phenyl)ethyl-1-(6-fluoro-5-(1-methyl-1H-1,2,3-triazol-5-yl)pyridin-3-yl-6-oxo-1,6-dihydropyridazine-3-carboxamide**

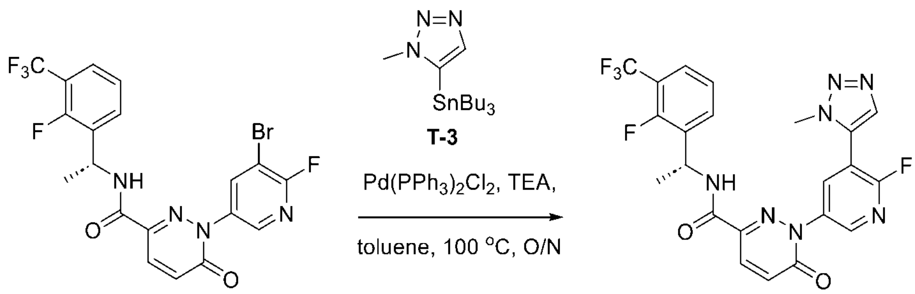


D-3 Example 6

**The compound of Example 6** was obtained as a white solid in the same manner as in Step 2 of Preparation Example 33 using **Intermediate D-3** and **Intermediate T-3** (34 mg, 28% yield, 97.5% purity) as a white solid. $^1$H NMR (400 MHz, DMSO-$d_6$) δ = 9.12 (d, *J* = 8.0 Hz, 1H), 8.86 (s, 1H), 8.68 (dd, *J* = 8.4, 2.4 Hz, 1H), 8.08 (s, 1H), 7.92 (d, *J* = 9.6 Hz, 1H), 7.82 (t, *J* = 7.0 Hz, 1H), 7.69 (t, *J* = 7.2 Hz, 1H), 7.23 (d, *J* = 9.6 Hz, 1H), 5.42 (quin, *J* = 7.2, 14.4 Hz, 1H), 4.10 (s, 3H), 1.51 (d, *J* = 6.8 Hz, 3H); MS (ESI) m/z = 506.1 [M+H]$^+$.

**Examples 6-1 to 6-9**

The compounds in Table 4 below were prepared in the same manner as in Example 6 through one-step stille coupling, using the corresponding intermediates described in the Preparation Examples.

[Table 4]

| No. | Chemical Name/Structure | Spectra (LCMS / $^1$H NMR) |
|---|---|---|
| 6-1 | *N*-{(1R)-1-(3-(difluoromethyl)-2-fluorophenyl)ethyl}-1-(6-fluoro-5-(1-methyl-1H-1,2,3-triazol-5-yl)pyridin-3-yl)-6-oxo-1,6-dihydropyridazine-3-carboxamide 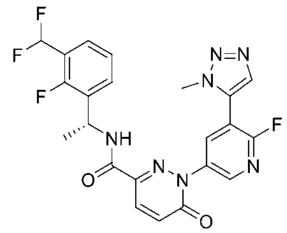 | **LCMS** : $t_R$ = 2.663 min, MS (ESI) m/z = 488.1 $[M+H]^+$. **$^1$H NMR** (400 MHz, DMSO-*$d_6$*) $\delta$= 9.09 (d, *J* = 8.0 Hz, 1H), 8.87-8.86 (m, 1H), 8.69 (dd, *J* = 8.4, 2.4 Hz, 1H), 8.09 (d, *J* = 1.2 Hz, 1H), 7.92 (d, *J* = 9.6 Hz, 1H), 7.68 (t, *J* = 7.0 Hz, 1H), 7.53 (t, *J* = 7.0 Hz, 1H), 7.33 (t, *J* = 7.6 Hz, 1H), 7.23 (t, *J* = 54.4 Hz, 1H), 7.23 (d, *J* = 9.6 Hz, 1H), 5.41 (quin, *J* = 7.3, 14.7 Hz, 1H), 4.10 (s, 3H), 1.50 *(d, J* = 7.2 Hz, 1H). |
| 6-2 | N-{(1R)-1-[2-fluoro-3-(trifluoromethyl)phenyl]ethyl}-1-[5-(1-isopropyl-1H-1,2,3-triazol-5-yl)-3-pyridyl]-5-(hydroxymethyl)-6-oxo-1,6-dihydropyridazine-3-carboxamide 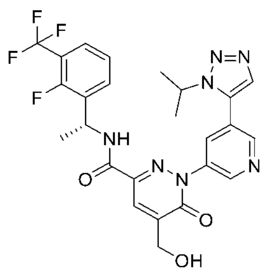 | **LCMS** : $t_R$ = 1.928 min, MS (ESI) m/z = 546.1 $[M+H]^+$. **$^1$H NMR** (400 MHz, DMSO-*$d_6$*) $\delta$= 9.16 (d, *J* = 2.4 Hz, 1H), 9.12 (d, *J* = 8.0 Hz, 1H), 8.80 (d, *J* = 2.0 Hz, 1H), 8.41 (t, *J* = 2.0 Hz, 1H), 8.02 (s, 1H), 7.90 (s, 1H), 7.82 (br t, *J* = 7.2 Hz, 1H), 7.68 (br t, *J* = 7.2 Hz, 1H), 7.39 (t, *J* = 7.6 Hz, 1H), 5.64 (t, *J* = 5.6 Hz, 1H), 5.44 (quin, *J* = 7.2 Hz, 1H), 4.77 (td, *J* = 6.8, 13.2 Hz, 1H), 4.46 (br d, *J* = 4.4 Hz, 2H), 1.52 (d, *J* = 6.4 Hz, 9H). |
| 6-3 | N-{(R)-1-[2-fluoro-3-(fluoromethyl)phenyl]ethyl}-1-[5-(1-cyclopropyl-1H-1,2,3-triazol-5-yl)-3-pyridyl]-5-(hydroxymethyl)-6-oxo-1,6-dihydropyridazine-3-carboxamide 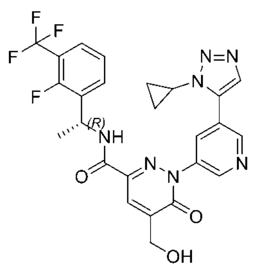 | **LCMS** : $t_R$ = 1.372 min, MS (ESI) m/z = 544.1 $[M+H]^+$. **$^1$H NMR** (400 MHz, DMSO-*$d_6$*) $\delta$= 9.11 (br s, 2H), 9.01 (br s, 1H), 8.64 (br s, 1H), 8.16 (s, 1H), 7.90 (br s, 1H), 7.82 (br t, *J* = 6.8 Hz, 1H), 7.68 (br t, *J* = 6.0 Hz, 1H), 7.40 (br t, *J* = 7.6 Hz, 1H), 5.65 (br s, 1H), 5.44 (br t, *J* = 7.2 Hz, 1H), 4.47 (br d, *J* = 4.0 Hz, 2H), 4.02 (br s, 1H), 1.51 (br d, *J* = 6.8 Hz, 3H), 1.14 (br d, *J* = 8.4 Hz, 4H). |
| 6-4 | N-{ (R)-1-[2-fluoro-3-(trifluoromethyl)phenyl]ethyl}-1-[5-(1-ethyl-1H-1,2,3-triazol-5-yl)-3-pyridyl]-5-(hydroxymethyl)-6-oxo-1,6-dihydropyridazine-3-carboxamide 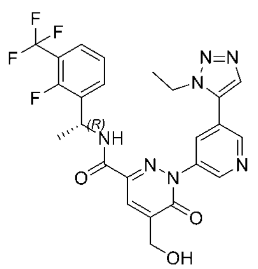 | **LCMS** : $t_R$ = 1.344 min, MS (ESI) m/z = 532.1 $[M+H]^+$. **$^1$H NMR** (400 MHz, DMSO-*$d_6$*) $\delta$= 9.14 (d, *J* = 2.0 Hz, 1H), 9.11 (br d, *J* = 8.0 Hz, 1H), 8.86 (d, *J* = 1.6 Hz, 1H), 8.47 (s, 1H), 8.08 (s, 1H), 7.90 (s, 1H), 7.83 (br t, *J* = 7.6 Hz, 1H), 7.68 (br t, *J* = 7.2 Hz, 1H), 7.40 (br t, *J* = 7.6 Hz, 1H), 5.68 - 5.59 (m, 1H), 5.44 (br t, *J* = 7.6 Hz, 1H), 4.53 - 4.45 (m, 4H), 1.52 (br d, *J* = 7.2 Hz, 3H), 1.40 (t, *J* = 7.2 Hz, 3H). |

(continued)

| No. | Chemical Name/Structure | Spectra (LCMS / $^1$H NMR) |
|---|---|---|
| 6-5 | N-{(R)-1-[2-fluoro-3-(trifluoromethyl)phenyl]ethyl}-1-[6-methoxy-5-(1-methyl-1H-1,2,3-triazol-5-yl)-3-pyridyl]-6-oxo-1,6-dihydropyridazine-3-carboxamide 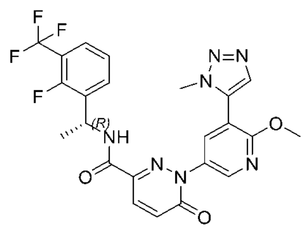 | **LCMS** : $t_R$ = 1.850 min, MS (ESI) m/z = 518.2 $[M+H]^+$. **$^1$H NMR** (400 MHz, DMSO-$d_6$) δ= 9.08 (d, *J* = 7.6 Hz, 1H), 8.75 (d, *J* = 2.4 Hz, 1H), 8.29 (d, *J* = 2.4 Hz, 1H), 7.92 (s, 1H), 7.90 (d, *J* = 9.6 Hz, 1H), 7.83 (t, *J* = 7.4 Hz, 1H), 7.69 (t, *J* = 7.2 Hz, 1H), 7.41 (t, *J* = 7.8 Hz, 2H), 7.19 (d, *J* = 9.6 Hz, 1H), 5.43 (quin, *J* = 7.2, 14.5 Hz, 1H), 4.01 (s, 3H), 4.00 (s, 3H), 1.52 (d, *J* = 7.2 Hz, 3H). |
| 6-6 | N-{(R)-1-[2-fluoro-3-(trifluoromethyl)phenyl]ethyl}-1-[6-(dimethylamino)-5-(1-methyl-1H-1,2,3-triazol-5-yl)-3-pyridyl]-6-oxo-1,6-dihydropyridazine-3-carboxamide 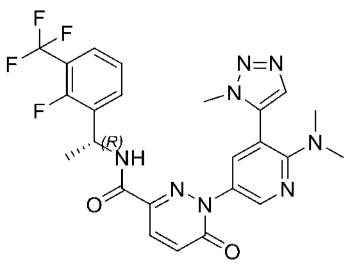 | **LCMS** : $t_R$ = 1.615 min, MS (ESI) m/z = 531.2 $[M+H]^+$. **$^1$H NMR** (400 MHz, DMSO-$d_6$) δ = 9.08 (d, *J* = 8.0 Hz, 1H), 8.66 (d, *J* = 2.4 Hz, 1H), 7.98 (d, *J* = 2.4 Hz, 1H), 7.89-7.82 (m, 3H), 7.69 (t, *J* = 7.2 Hz, 1H), 7.40 (t, *J* = 7.8 Hz, 1H), 7.15 (d, *J* = 10.0 Hz, 1H), 5.42 (quin, *J* = 7.3, 14.8 Hz, 1H), 3.92 (s, 3H), 2.72 (s, 6H), 1.51 (d, *J* = 6.8 Hz, 3H). |
| 6-7 | N-{(R)-1-[2-fluoro-3-(trifluoromethyl)phenyl]ethyl}-1-[2-fluoro-5-(1-methyl-1H-1,2,3-triazol-5-yl)-3-pyridyl]-6-oxo-1,6-dihydropyridazine-3-carboxamide 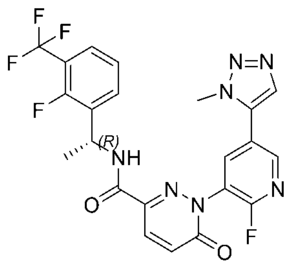 | **LCMS** : $t_R$ = 2.018 min, MS (ESI) m/z = 506.0 $[M+H]^+$. **$^1$H NMR** (400 MHz, DMSO-$d_6$) δ = 9.11 (d, *J* = 7.6 Hz, 1H), 8.76 - 8.63 (m, 2H), 8.12 (s, 1H), 7.96 (d, *J* = 9.6 Hz, 1H), 7.79 (t, *J* = 7.2 Hz, 1H), 7.67 (t, *J* = 7.2 Hz, 1H), 7.39 (t, *J* = 7.6 Hz, 1H), 7.26 (d, *J* = 10.0 Hz, 1H), 5.41 (quin, *J* = 7.2 Hz, 1H), 4.17 (s, 3H), 1.48 (d, *J* = 7.2 Hz, 3H). |
| 6-8 | N-{(R)-1-[2-fluoro-3-(1,1-difluoro-2-hydroxy-ethyl)-2-fluoro-phenyl]ethyl}-1-[2-fluoro-5-(1-cyclopropyl-1H-1,2,3-triazol-5-yl)-3-pyridyl]-6-oxo-1,6-dihydropyridazine-3-carboxamide 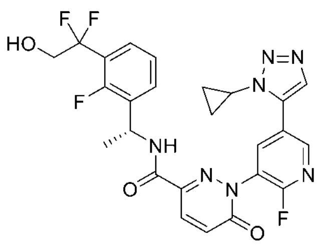 | **LCMS** : $t_R$ = 0.880 min, MS (ESI) m/z = 544.0 $[M+H]^+$. **$^1$H NMR** (400 MHz, DMSO-$d_6$) δ = 9.04 (d, *J* = 8.0 Hz, 1H), 8.83 - 8.74 (m, 2H), 8.16 (s, 1H), 7.97 (d, *J* = 10.0 Hz, 1H), 7.60 (br t, *J* = 6.8 Hz, 1H), 7.43 (br t, *J* = 6.8 Hz, 1H), 7.31 - 7.20 (m, 2H), 5.70 (br s, 1H), 5.40 (br t, *J* = 7.2 Hz, 1H), 4.06 - 3.98 (m, 1H), 3.91 (br t, *J* = 14.2 Hz, 2H), 1.45 (d, *J* = 7.2 Hz, 3H), 1.17 - 1.10 (m, 4H). |

(continued)

| No. | Chemical Name/Structure | Spectra (LCMS / $^1$H NMR) |
|---|---|---|
| 6-9 | N-{(R)-1-[2-fluoro-3-(trifluoromethyl)phenyl]ethyl}-6-oxo-1-(5-(1-((R)-pyrrolidin-3-yl)-1H-1,2,3-triazol-5-yl)pyridin-3-yl)-1,6-dihydropyridazine-3-carboxamide 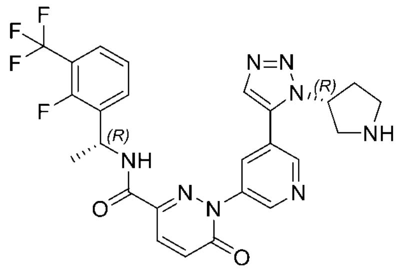 | **LCMS** : $t_R$ = 0.451 min, MS (ESI) m/z = 543.3 [M+H]$^+$. **$^1$H NMR** (400 MHz, DMSO-$d_6$) δ = 9.17 (d, *J* = 2.0 Hz, 1H), 9.14 (br d, *J* = 7.6 Hz, 1H), 8.82 (d, *J* = 1.6 Hz, 1H), 8.45 - 8.40 (m, 1H), 8.07 (s, 1H), 7.92 (d, *J* = 9.6 Hz, 1H), 7.83 (br t, *J* = 7.2 Hz, 1H), 7.68 (br t, *J* = 7.2 Hz, 1H), 7.40 (t, *J* = 7.6 Hz, 1H), 7.22 (d, *J* = 9.6 Hz, 1H), 5.43 (br t, *J* = 7.2 Hz, 1H), 5.09 - 4.93 (m, 1H), 3.28 - 3.22 (m, 1H), 3.20 (d, *J* = 6.8 Hz, 1H), 3.13 (br d, *J* = 4.4 Hz, 1H), 3.10 (br s, 1H), 2.89 (ddd, *J* = 5.6, 7.6, 11.2 Hz, 1H), 2.27 - 2.14 (m, 2H), 1.52 (d, *J* = 7.2 Hz, 3H). |

**Example 7: (R)-3-cyclopropyl-N-(1-(2-fluoro-3-(trifluoromethyl)phenyl)ethyl)-5'-(1-methyl-1H-1,2,3-triazol-5-yl)-2-oxo-2H-[1,3'-bipyridine]-5-carboxamide**

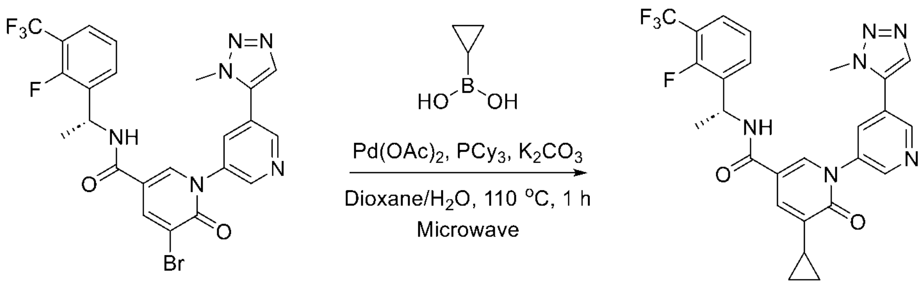


**Example 1-22** **Example 7**

**The compound of Example 7** was obtained as a white solid by reacting the compound of **Example 1-22** in a similar manner to Example 4 (4.0 mg, 21% yield). $^1$H NMR (400 MHz, DMSO-$d_6$) δ = 8.95 (d, *J* = 2.0 Hz, 1H), 8.86 (d, *J* = 2.4 Hz, 1H), 8.73 (d, *J* = 7.2 Hz, 1H), 8.37 (t, *J* = 2.2 Hz, 1H), 8.33 (d, *J* = 2.4 Hz, 1H), 8.13 (s, 1H), 7.75 (t, *J* = 7.0 Hz, 1H), 7.67 (t, *J* = 7.2 Hz, 1H), 7.50 (d, *J* = 2.0 Hz, 1H), 7.40 (t, *J* = 8.0 Hz, 1H), 5.35 (quin, *J* = 6.9 Hz, 1H), 4.16 (s, 3H), 2.08-2.03 (m, 1H), 1.47 (d, *J* = 6.8 Hz, 3H), 0.93 (dd, *J* = 8.4, 2.0 Hz, 1H), 0.75-0.72 (m, 2H); MS (ESI) m/z = 527.21 [M+H]$^+$.

**Examples 7-1 to 7-4**

The compounds in Table 5 below were prepared in the same manner as in Example 7 through one-step Suzuki coupling, using the corresponding intermediates described in the Preparation Examples.

[Table 5]

| No. | Chemical Name/Structure | Spectra (LCMS / $^1$H NMR) |
|---|---|---|
| 7-1 | N-{(R)-1-[2-fluoro-3-(trifluoromethyl)phenyl]ethyl}-5-[methyl-trideuterio]-1-[5-(1-methyl-1H-1,2,3-triazol-5-yl)-3-pyridyl]-6-oxo-1,6-dihydropyridazine-3-carboxamide 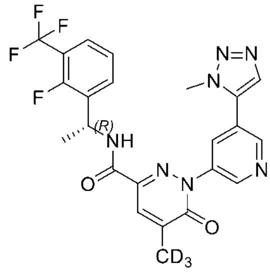 | **LCMS** : $t_R$ = 1.771 min, MS (ESI) m/z = 505.2 $[M+H]^+$. **$^1$H NMR** (400 MHz, DMSO-$d_6$) $\delta$ = 9.12 (d, *J* = 2.4 Hz, 1H), 9.09 (d, *J* = 7.6 Hz, 1H), 8.92 (d, *J* = 2.0 Hz, 1H), 8.51 (t, *J* = 2.2 Hz, 1H), 8.13 (s, 1H), 7.86 (s, 1H), 7.82 (t, *J* = 7.2 Hz, 1H), 7.68 (t, *J* =7.2 Hz, 1H), 7.40 (t, *J* = 8.0 Hz, 1H), 5.45-5.41 (m, 1H), 4.17 (s, 3H), 1.51 (d, *J* = 6.8 Hz, 3H). |
| 7-2 | 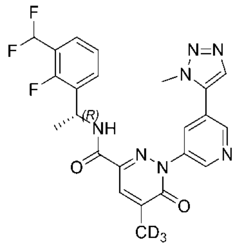 N-{(R)-1-[3-(difluoromethyl)-2-fluorophenyl]ethyl}-5-[methyl-trideuterio]-1-[5-(1-methyl-1H-1,2,3-triazol-5-yl)-3-pyridyl]-6-oxo-1,6-dihydropyridazine-3-carboxamide | **LCMS** : $t_R$ = 1.698 min, MS (ESI) m/z = 487.2 $[M+H]^+$. **$^1$H NMR** (400 MHz, DMSO-$d_6$) $\delta$ = 9.12 (d, *J* = 2.4 Hz, 1H), 9.06 (d, *J* = 8.4 Hz, 1H), 8.92 (d, *J* = 2.0 Hz, 1H), 8.14 (s, 1H), 7.86 (s, 1H), 7.68 (t, *J* = 7.2 Hz, 1H), 7.53 (t, *J* = 6.8 Hz, 1H), 7.32 (t, *J* = 7.6 Hz, 1H), 7.23 (t, *J* = 54.2 Hz, 1H), 5.43-5.40 (m, 1H), 4.17 (s, 3H), 1.49 (d, *J* = 6.8 Hz, 3H). |
| 7-3 | 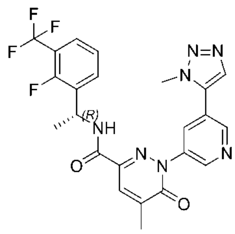 N-{ (R)-1-[2-fluoro-3-(trifluoromethyl)phenyl] ethyl }-5-methyl-1-[5-(1-methyl-1H-1,2,3-triazol-5-yl)-3-pyridyl]-6-oxo-1,6-dihydropyridazine-3-carboxamide | **LCMS** : $t_R$ = 1.797 min, MS (ESI) m/z = 502.2 $[M+H]^+$. **$^1$H NMR** (400 MHz, DMSO-$d_6$) $\delta$ = 9.12-9.09 (m, 2H), 8.92 (d, *J* = 1.6 Hz, 1H), 8.51 (t, *J* = 2.0 Hz, 1H), 8.14 (s, 1H), 7.86 (d, *J* = 1.2 Hz, 1H), 7.82 (t, *J* = 7.6 Hz, 1H), 7.69 (t, *J* = 6.8 Hz, 1H), 7.41 (t, *J* = 7.8 Hz, 1H), 5.45-5.41 (m, 1H), 4.17 (s, 3H), 2.21 (s, 3H), 1.51 (d, *J* = 6.8 Hz, 3H). |
| 7-4 | 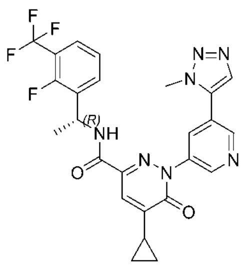 N-{(R)-1-[2-fluoro-3-(trifluoromethyl)phenyl]ethyl}-5-cyclopropyl-1-[5-(1-methyl-1H-1,2,3-triazol-5-yl)-3-pyridyl]-6-oxo-1,6-dihydropyridazine-3-carboxamide | **LCMS** : $t_R$ = 3.022 min, MS (ESI) m/z = 528.2 $[M+H]^+$. **$^1$H NMR** (400 MHz, DMSO-$d_6$) $\delta$ = 9.11 (d, *J* = 2.0 Hz, 1H), 9.08 (d, *J* = 8.0 Hz, 1H), 8.92 (d, *J* = 2.0 Hz, 1H), 8.52 (t, *J* = 2.0 Hz, 1H), 8.14 (s, 1H), 7.82 (t, *J* = 7.4 Hz, 1H), 7.68 (t, *J* = 6.8 Hz, 1H), 7.40 (t, *J* = 7.8 Hz, 1H), 7.37 (s, 1H), 5.41 (quin, *J* = 7.3, 14.7 Hz, 1H), 4.18 (s, 3H), 2.25-2.20 (m, 1H), 1.50 (d, *J* = 7.2 Hz, 3H), 1.11 (dd, *J* = 8.2, 2.2 Hz, 2H), 0.97-0.95 (m, 2H). |

**Example 8: N-[(R)-1-{3-[1,1-difluoro-2-(2-pyridylsulfonyloxy)ethyl]-2-fluorophenyl}ethyl]-5-methyl-1-[5-(1-methyl-1H-1,2,3-triazol-5-yl)-3-pyridyl]-6-oxo-1,6-dihydropyridazine-3-carboxamide**

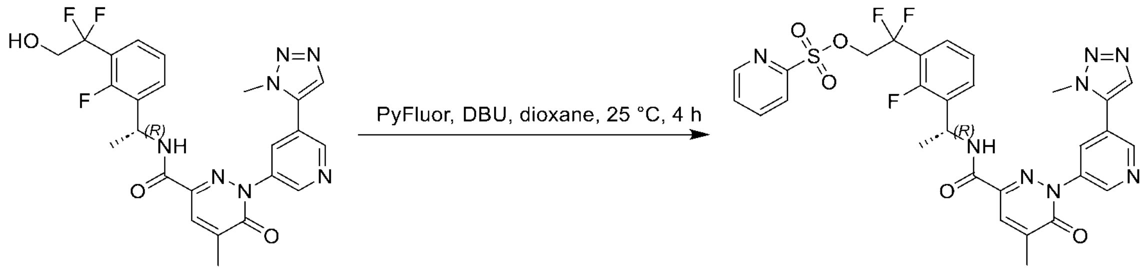


**Example 1-23** **Example 8**

A mixture of N-[(1R)-1-[3-(1,1-difluoro-2-hydroxy-ethyl)-2-fluoro-phenyl]ethyl]-5-methyl-1-[5-(3-methyltriazol-4-yl)-3-pyridyl]-6-oxo-pyridazine-3-carboxamide (20 mg, 38.95 μmol), pyridine-2-sulfonyl fluoride (18.83 mg, 116.85 μmol), DBU (35.58 mg, 233.70 μmol, 35.23 μL) in dioxane (2 mL) was degassed and purged with $N_2$ for 3 times, and then stirred at 25 °C for 4 h under $N_2$ atmosphere. The reaction mixture was concentrated under reduced pressure and diluted with EtOAc (5 mL). Then it was washed with brine (5 mL × 3), dried over anhydrous $Na_2SO_4$ and concentrated under reduced pressure to give a residue. The crude product was purified by prep-HPLC (column: Welch Xtimate C18 40 * 200 mm 7μm; mobile phase: [water ($NH_3H_2O$+$NH_4HCO_3$)-MeCN]; 22%-62% B over 25 min), and most of MeCN was removed under reduced pressure. The remaining solvent was removed by lyophilization to give **a compound of Example 8** (6.3 mg, 24.68% yield, 99.88% purity) as a white solid. $^1$H NMR (400 MHz, DMSO-$d_6$) δ = 9.11 (br s, 1H), 9.03 (br d, *J* = 7.6 Hz, 1H), 8.91 (s, 1H), 8.79 (br d, *J* = 3.6 Hz, 1H), 8.50 (br s, 1H), 8.20 - 8.10 (m, 2H), 8.07 (br d, *J* = 7.6 Hz, 1H), 7.86 (s, 1H), 7.83 - 7.78 (m, 1H), 7.72 - 7.65 (m, 1H), 7.42 (br d, *J* = 6.4 Hz, 1H), 7.34 - 7.25 (m, 1H), 5.39 (br t, *J* = 7.2 Hz, 1H), 4.94 (br t, *J* = 13.2 Hz, 2H), 4.16 (s, 3H), 2.21 (s, 3H), 1.47 (br d, *J* = 6.8 Hz, 3H); MS (ESI) m/z = 654.9 $[M+H]^+$.

**Examples 9 and 10: N-{(R)-1-[2-fluoro-3-(trifluoromethyl)phenyl]ethyl}-5-[(R)-1-hydroxyethyl]-1-[5-(1-methyl-1H-1,2,3-triazol-5-yl)-3-pyridyl]-6-oxo-1,6-dihydropyridazine-3-carboxamide and N-{(R)-1-[2-fluoro-3-(trifluoromethyl)phenyl]ethyl}-5-[(S)-1-hydroxyethyl]-1-[5-(1-methyl-1H-1,2,3-triazol-5-yl)-3-pyridyl]-6-oxo-1,6-dihydropyridazine-3-carboxamide**

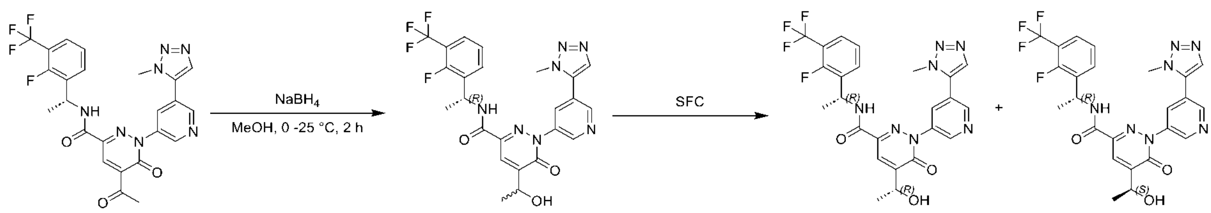


**Example 1-62** **Example 9** **Example 10**

<u>Step 1: Synthesis of N-((R)-1-(2-fluoro-3-(trifluoromethyl)phenyl)ethyl)-5-(1-hydroxyethyl)-1-(5-(1-methyl-1H-1,2,3-triazol-5-yl)pyridin-3-yl)-6-oxo-1,6-dihydropyridazine-3-carboxamide</u>

**A compound of Example 1-62** (80 mg, 151.10 μmol) in MeOH (3 mL) MeOH (3 mL) was cooled to 0 °C, $NaBH_4$ (11.43 mg, 302.20 μmol) was added dropwise under $N_2$ atmosphere, then the mixture was stirred at 0 °C for 2 h under $N_2$ atmosphere. The reaction mixture was quenched with aq. $NH_4Cl$ (4 mL) added dropwise slowly at 0 °C and extracted with DCM (10 mL × 3). The combined organic layers were washed with brine (5 mL × 2), dried over anhydrous $Na_2SO_4$ and concentrated under reduced pressure to give a crude product. The crude product was purified by silica gel column chromatography (2% MeOH in DCM) to give a crude product as a yellow solid. The crude product was dissolved in DMF (5 mL) and then purified by prep-HPLC (column: Welch Xtimate C18 40*200mm 7μm; mobile phase: [water ($NH_3H_2O$ + $NH_4HCO_3$)-MeOH]; 38%-78% B over 25 min). Most of MeCN was removed under reduced pressure, and the remaining solvent was removed by lyophilization to give N-((R)-1-(2-fluoro-3-(trifluoromethyl)phenyl)ethyl)-5-(1-hydroxyethyl)-1-(5-(1-methyl-1H-1,2,3-triazol-5-yl)pyridin-3-yl)-6-oxo-1,6-dihydropyridazine-3-carboxamide (15 mg, 15.84%

yield, 84.8% purity, 0.8% ee) as a white solid. MS (ESI) m/z = 532.1 $[M+H]^+$.

Step 2: Synthesis of N-{(R)-1-[2-fluoro-3-(trifluoromethyl)phenyl]ethyl}-5-[(R)-1-hydroxyethyl]-1-[5-(1-methyl-1H-1,2,3-triazol-5-yl)-3-pyridyl]-6-oxo-1,6-dihydropyridazine-3-carboxamide and N-{(R)-1-[2-fluoro-3-(trifluoromethyl)phenyl]ethyl}-5-[(S)-1-hydroxyethyl]-1-[5-(1-methyl-1H-1,2,3-triazol-5-yl)-3-pyridyl]-6-oxo-1,6-dihydropyridazine-3-carboxamide

N-((R)-1-(2-fluoro-3-(trifluoromethyl)phenyl)ethyl)-5-(1-hydroxyethyl)-1-(5-(1-methyl-1H-1,2,3-triazol-5-yl)pyridin-3-yl)-6-oxo-1,6-dihydropyridazine-3-carboxamide (10 mg, 18.82 μmol) was further purified by SFC separation (column: DAICEL CHIRALCEL OD (250mm*30mm,10um); mobile phase: [$CO_2$- EtOH (0.1% $NH_3H_2O$)]; B%: 40%, isocratic elution mode). Two peaks were confirmed by SFC analysis. The compound confirmed in the first peak (Rt = 1.339 min) was concentrated under reduced pressure. Then MeCN (5 mL) and $H_2O$ (25 mL) were added, and the solvent was removed by lyophilization to give a **compound of Example 9** (4 mg, 31.17% yield, 77.92% purity, 99.84% ee) as a white solid. The compound confirmed in the second peak (Rt = 1.558 min) were concentrated under reduced pressure. Then MeCN (5 mL) and $H_2O$ (25 mL) were added, and the solvent was removed by lyophilization to give a **compound of Example 10** (4.2 mg, 39.07% yield, 93.03% purity, 99.42 ee) as a white solid.

**Compound of Example 9:** $^1H$ NMR (400 MHz, $CD_3OD$) δ = 9.10 (d, *J* = 2.0 Hz, 1H), 8.86 (s, 1H), 8.50 (s, 1H), 8.11 (s, 1H), 8.03 (s, 1H), 7.74 - 7.70 (m, 1H), 7.59 (br t, *J* = 6.8 Hz, 1H), 7.31 (t, *J* = 8.0 Hz, 1H), 5.51 (q, *J* = 6.8 Hz, 1H), 4.97 (m, 1H), 4.21 (s, 3H), 1.59 (d, *J* = 7.0 Hz, 3H), 1.46 (d, *J* = 6.4 Hz, 3H); MS (ESI) m/z = 532.1 $[M+H]^+$.

**Compound of Example 10:** $^1H$ NMR (400 MHz, $CD_3OD$) δ = 9.10 (d, *J* = 1.6 Hz, 1H), 8.86 (s, 1H), 8.50 (s, 1H), 8.11 (s, 1H), 8.03 (s, 1H), 7.71 (br t, *J* = 7.2 Hz, 1H), 7.59 (br t, *J* = 7.2 Hz, 1H), 7.32 (br d, *J* = 7.6 Hz, 1H), 5.51 (q, *J* = 6.8 Hz, 1H), 4.96 (br s, 1H), 4.21 (s, 3H), 1.59 (d, *J* = 7.2 Hz, 4H), 1.47 (d, *J* = 6.4 Hz, 3H); MS (ESI) m/z = 530.2 $[M+H]^+$.

**Example 11: N-{(R)-1-[2-fluoro-3-(trifluoromethyl)phenyl]ethyl}-1-{5-[1-(2-hydroxyethyl)-1H-1,2,3-triazol-5-yl]-3-pyridyl}-6-oxo-1,6-dihydropyridazine-3-carboxamide**

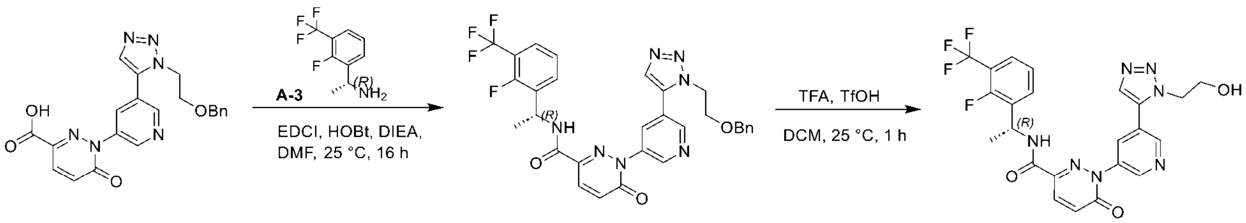


4-42

Example 11

Step 1: Synthesis of 1-[5-[3-(2-benzyloxyethyl)triazol-4-yl]-3-pyridyl]-N-[(1R)-1-[2-fluoro-3-(trifluoromethyl)phenyl]ethyl]-6-oxo-pyridazine-3-carboxamide

To a mixture of Intermediate 4-42 (70 mg, 167.30 μmol) and Intermediate A-3 (40.76 mg, 167.30 μmol, HCl) in DMF (4mL), EDCI (64.14 mg, 334.60 μmol), HOBt (45.21 mg, 334.60 μmol) and DIEA (64.87 mg, 501.91 μmol, 87.42 μL) was added, and the mixture was degassed and purged with $N_2$ for 3 times, and was stirred at 25 °C for 16 h under $N_2$ atmosphere. The reaction mixture was poured into water (10 mL) and extracted with EtOAc (15 mL × 3). The combined organic layers were washed with brine (5 mL × 3), dried over anhydrous $Na_2SO_4$ and concentrated under reduced pressure to give a residue. The residue was purified by silica gel column chromatography (1% MeOH in DCM) to give 1-[5-[3-(2-benzyloxyethyl)triazol-4-yl]-3-pyridyl]-N-[(1R)-1-[2-fluoro-3-(trifluoromethyl)phenyl]ethyl]-6-oxo-pyridazine-3-carboxamide (20 mg, 16.33% yield, 83% purity) as a yellow solid. $^1H$ NMR (400 MHz, $CD_3OD$) δ = 9.02 (d, *J* = 2.4 Hz, 1H), 8.77 (d, *J* = 2.0 Hz, 1H), 8.50 (t, *J* = 2.0 Hz, 1H), 8.00 - 7.97 (m, 1H), 7.97 - 7.96 (m, 1H), 7.71 (br t, *J* = 6.8 Hz, 1H), 7.58 (t, *J* = 6.8 Hz, 1H), 7.29 (t, *J* = 7.6 Hz, 1H), 7.17 - 7.10 (m, 4H), 7.06 - 7.00 (m, 2H), 5.48 (s, 1H), 4.66 (t, *J* = 4.8 Hz, 2H), 4.36 (s, 2H), 3.94 (t, *J* = 4.8 Hz, 2H), 1.56 (d, *J* = 7.2 Hz, 3H); MS (ESI) m/z = 608.3 $[M+H]^+$.

Step 2: Synthesis of N-{(R)-1-[2-fluoro-3-(trifluoromethyl)phenyl]ethyl}-1-{5-[1-(2-hydroxyethyl)-1H-1,2,3-triazol-5-yl]-3-pyridyl}-6-oxo-1,6-dihydropyridazine-3-carboxamide

A mixture of 1-[5-[3-(2-benzyloxyethyl)triazol-4-yl]-3-pyridyl]-N-[(1R)-1-[2-fluoro-3-(trifluoromethyl)phenyl]ethyl]-6-oxo-pyridazine-3-carboxamide (19 mg, 31.27 μmol), trifluoromethanesulfonic acid (211.20 mg, 1.41 mmol, 124.53 μL) and TFA (42.79 mg, 375.27 μmol, 27.88 μL) in DCM (1.5 mL) was degassed and purged with $N_2$ for 3 times,

and stirred at 20 °C for 1 h under $N_2$ atmosphere. The mixture was concentrated under reduced pressure to give a crude product. The crude product was dissolved in DMF (5 mL) and purified by prep-HPLC (column: Welch Xtimate C18 40*200mm 7μm; mobile phase: [water($NH_3H_2O$+$NH_4HCO_3$)-MeCN]; 16%-56% B, 25 min) to give **a compound of Example 11** (6.8 mg, 41.80% yield, 99.47% purity) as a white solid. $^1$H NMR (400 MHz, DMSO-*d*$_6$) δ = 9.17 - 9.07 (m, 2H), 8.91 (d, *J* = 2.0 Hz, 1H), 8.55 (t, *J* = 2.0 Hz, 1H), 8.08 (s, 1H), 7.92 (d, *J* = 9.6 Hz, 1H), 7.83 (br t, *J* = 7.2 Hz, 1H), 7.68 (t, *J* = 7.2 Hz, 1H), 7.40 (t, *J* = 7.6 Hz, 1H), 7.22 (d, *J* = 9.6 Hz, 1H), 5.42 (quin, *J* = 7.2 Hz, 1H), 5.13 (t, *J* = 5.2 Hz, 1H), 4.48 (t, *J* = 5.2 Hz, 2H), 3.84 (q, *J* = 5.2 Hz, 2H), 1.51 (d, *J* = 7.2 Hz, 3H); MS (ESI) m/z = 518.1 [M+H] $^+$.

**Examples 12, 13 and 14: N-{(R)-1-[3-(difluoromethyl)-2-fluorophenyl]ethyl}-5-chloro-1-[5-(4-chloro-1-methyl-1H-1,2,3-triazol-5-yl)-3-pyridyl]-6-oxo-1,6-dihydropyridazine-3-carboxamide, N-{(R)-1-[3-(difluoromethyl)-2-fluorophenyl]ethyl}-1-[5-(4-chloro-1-methyl-1H-1,2,3-triazol-5-yl)-3-pyridyl]-6-oxo-1,6-dihydropyridazine-3-carboxamide and N-{(R)-1-[3-(difluoromethyl)-2-fluorophenyl]ethyl}-5-chloro-1-[5-(1-methyl-1H-1,2,3-triazol-5-yl)-3-pyridyl]-6-oxo-1,6-dihydropyridazine-3-carboxamide**

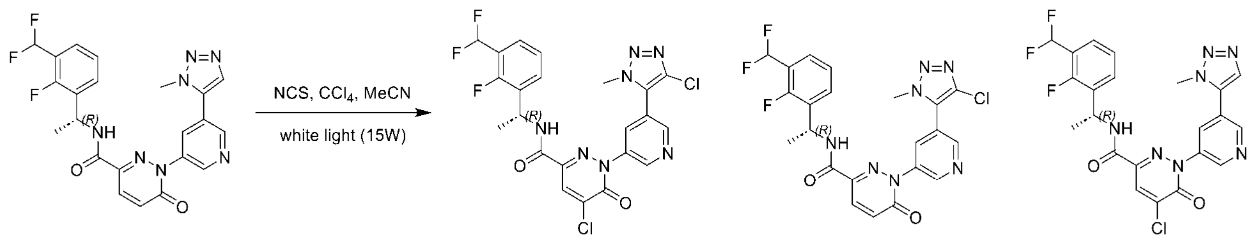


Example 1-1 Example 12 Example 13 Example 14

A mixture of the compound of Example 1-1 (100 mg, 213.03 μmol) and NCS (42.67 mg, 319.54 μmol) in $CCl_4$ (1 mL) and MeCN (0.5 mL) was degassed and purged with $N_2$ for 3 times, and then the mixture was stirred at 25 °C for 24 h under white light (15 W). The mixture was filtrated, and filtrate was poured into water (10 mL) and extracted with EtOAc (20 mL × 3). The combined organic layers were washed with $H_2O$, dried over anhydrous $Na_2SO_4$ and concentrated under reduced pressure to give a crude product. The crude product was dissolved in DMF (5 mL) and was purified by prep-HPLC (column: Welch Xtimate C18 40*200mm 7μm; mobile phase: [water ($NH_3H_2O$ + $NH_4HCO_3$)-ACN]; 22%-62% B over 25 min) to afford three products respectively. The first one: **Compound of Example 12** (6.2 mg, 5.41% yield, 100% purity) as a white solid. The second one: **Compound of Example 13 (10.8** mg, 9.89% yield, 98.3% purity) as a white solid. The third one: **Compound of Example 14** (5.6 mg, 4.38% yield, 83.95% purity) as a white solid.

**Compound of Example 12:** $^1$H NMR (400 MHz, DMSO-d6) δ = 9.18 (d, *J* = 2.4 Hz, 1H), 9.14 (d, *J* = 8.0 Hz, 1H), 8.90 (d, *J* = 2.0 Hz, 1H), 8.52 (t, *J* = 2.0 Hz, 1H), 8.19 (s, 1H), 7.68 (br t, *J* = 7.6 Hz, 1H), 7.53 (br t, *J* = 6.8 Hz, 1H), 7.36 - 7.30 (m, 1H), 7.21 (s, 1H), 7.08 (s, 1H), 5.41 (quin, *J* = 7.2 Hz, 1H), 4.11 (s, 3H), 1.50 (d, *J* = 7.2 Hz, 3H); MS (ESI) m/z = 537.9 [M+H]$^+$.

**Compound of Example 13:** $^1$H NMR (400 MHz, DMSO-d6) δ = 9.21 (d, *J* = 2.4 Hz, 1H), 9.08 (d, *J* = 8.0 Hz, 1H), 8.88 (d, *J* = 2.0 Hz, 1H), 8.54 (t, *J* = 2.0 Hz, 1H), 7.92 (d, *J* = 9.6 Hz, 1H), 7.69 (br t, *J* = 7.2 Hz, 1H), 7.54 (br t, *J* = 6.8 Hz, 1H), 7.37 - 7.30 (m, 1H), 7.26 - 7.20 (m, 2H), 7.09 (s, 1H), 5.46 - 5.37 (m, 1H), 4.12 (s, 3H), 1.51 (d, *J* = 7.2 Hz, 3H); MS (ESI) m/z = 504.0 [M+H]$^+$.

**Compound of Example 14:** $^1$H NMR (400 MHz, DMSO-d6) δ = 9.15 (d, *J* = 8.0 Hz, 1H), 9.10 (d, *J* = 2.0 Hz, 1H), 8.95 (d, *J* = 2.0 Hz, 1H), 8.52 - 8.48 (m, 1H), 8.20 (s, 1H), 8.13 (s, 1H), 7.70 - 7.66 (m, 1H), 7.53 (br t, *J* = 6.8 Hz, 1H), 7.35 - 7.31 (m, 1H), 7.21 (s, 1H), 7.08 (s, 1H), 5.41 (t, *J* = 7.6 Hz, 1H), 4.16 (s, 3H), 1.50 (d, *J* = 7.2 Hz, 3H); MS (ESI) m/z = 504.0 [M+H]$^+$.

**Example 15: N-{(R)-1-[2-fluoro-3-(trifluoromethyl)phenyl]ethyl}-5-(1-cyclohexen-1-yl)-5'-(1-methyl-1H-1,2,3-triazol-5-yl)-6-oxo-1H-[1,3'-bipyridyl]-3-carboxamide**

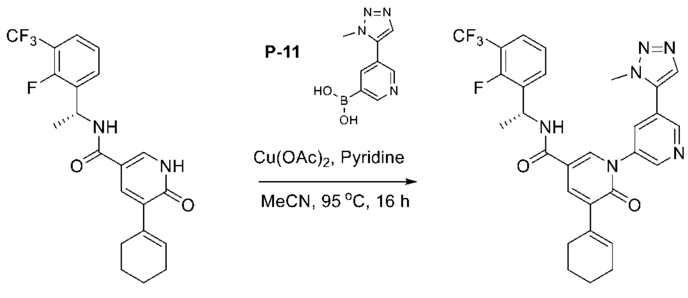


W-2

Example 15

Intermediate W-2 and Intermediate P-11 were reacted in the same manner as in Step 3 of Preparation Example 28 to obtain **a compound of Example 15** (27.3 mg, 31% yield) as a white solid. $^1$H NMR (400 MHz, DMSO-d6) δ = 8.94 (d, *J* = 2.0 Hz, 1H), 8.85 (d, *J* = 2.4 Hz, 1H), 8.80 (d, *J* = 7.2 Hz, 1H), 8.41 (d, *J* = 2.4 Hz, 1H), 8.35 (t, *J* = 2.2 Hz, 1H), 8.13 (s, 1H), 7.87 (d, *J* = 2.8 Hz, 1H), 7.77 (t, *J* = 7.4 Hz, 1H), 7.68 (t, *J* = 6.8 Hz, 1H), 7.40 (t, *J* = 7.6 Hz, 1H), 6.41 (m, 1H), 5.37 (quin, *J* = 7.0 Hz, 1H), 4.18 (s, 3H), 2.34-2.33 (m, 2H), 2.16-2.15 (m, 2H), 1.69-1.65 (m, 2H), 1.61-1.57 (m, 2H), 1.48 (d, *J* = 7.2 Hz, 3H); MS (ESI) m/z = 567.31 (M + H)$^+$.

**Examples 16 and 17: N-[(R)-1-{2-fluoro-3-[1-(fluoromethyl)cyclopropyl]phenyl}ethyl]-1-[5-(1-methyl-1H-1,2,3-triazol-5-yl)-3-pyridyl]-6-oxo-1,6-dihydropyridazine-3-carboxamide and N-{(R)-1-[3-(1-cyclobuten-1-yl)-2-fluorophenyl]ethyl}-1-[5-(1-methyl-1H-1,2,3-triazol-5-yl)-3-pyridyl]-6-oxo-1,6-dihydropyridazine-3-carboxamide**

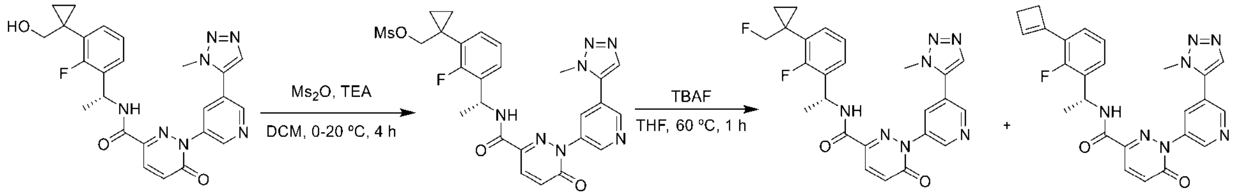


Example 1-32

Example 16

+

Example 17

Step 1: Synthesis of [1-[2-fluoro-3-[(1R)-1-[[1-[5-(3-methyltriazol-4-yl)-3-pyridyl]-6-oxo-pyridazine-3 -carbonyl]amino] ethyl]phenyl]cyclopropyl]methyl methanesulfonate

A mixture of the compound of Example 1-32 (100 mg, 204.29 μmol) and TEA (26.87 mg, 265.58 μmol, 36.96 μL) in DCM (5 mL) was degassed and purged with $N_2$ for 3 times, and then methylsulfonyl methanesulfonate (71.17 mg, 408.58 μmol) was added dropwise to the mixture at 0 °C, and then the mixture was stirred at 0 °C for 1 h under $N_2$ atmosphere. Thereafter, the mixture was allowed to warm to 20 °C, and then was stirred at 20 °C for 2 h. The reaction mixture was poured into water (10 mL) and extracted with DCM (15 mL × 3). The combined organic layers were washed with brine (10 mL × 3), dried over anhydrous $Na_2SO_4$ and concentrated under reduced pressure to give a crude product. The crude product was purified by silica gel column chromatography (1% MeOH in DCM) to give product [1-[2-fluoro-3-[(1R)-1-[[1-[5-(3-methyltriazol-4-yl)-3-pyridyl]-6-oxo-pyridazine-3-carbonyl]amino]ethyl]phenyl]cyclopropyl]-methyl methanesulfonate (60 mg, 27.94% yield, 54% purity) as a white solid. $^1$H NMR (400 MHz, DMSO-$d_6$) δ = 9.12 (d, *J* = 2.0 Hz, 1H), 9.00 (br d, *J* = 8.0 Hz, 1H), 8.92 (d, *J* = 1.6 Hz, 1H), 8.51 (s, 1H), 8.13 (s, 1H), 7.92 (d, *J* = 9.6 Hz, 1H), 7.42 - 7.36 (m, 1H), 7.26 - 7.19 (m, 2H), 7.16 - 7.08 (m, 1H), 5.40 (quin, *J* = 6.8 Hz, 1H), 4.17 (s, 3H), 2.97 (s, 2H), 1.47 (br d, *J* = 6.8 Hz, 3H), 1.03 (m, 2H), 0.92 (m, 2H); MS (ESI) m/z = 568.2 [M+H]$^+$.

Step 2: Synthesis of N-[(R)-1-{2-fluoro-3-[1-(fluoromethyl)cyclopropyl]phenyl}ethyl]-1-[5-(1-methyl-1H-1,2,3-triazol-5-yl)-3-pyridyl]-6-oxo-1,6-dihydropyridazine-3-carboxamide, N-{(R)-1-[3-(1-cyclobuten-1-yl)-2-fluorophenyl] ethyl}-1-[5-(1-methyl-1H-1,2,3-triazol-5-yl)-3-pyridyl]-6-oxo-1,6-dihydropyridazine-3-carboxamide

A mixture of [1-[2-fluoro-3-[(1R)-1-[[1-[5-(3-methyltriazol-4-yl)-3-pyridyl]-6-oxo-pyridazine-3-carbonyl]amino] ethyl]phenyl]cyclopropyl]methyl methanesulfonate (60 mg, 105.71 μmol) and TBAF (1 M in THF, 158.56 μL) in THF (2 mL) was degassed and purged with $N_2$ for 3 times, and then the mixture was stirred at 60 °C for 1h under $N_2$ atmosphere. The reaction mixture was poured into water (10 mL) and extracted with EtOAc (10 mL ×3). The combined organic layers were washed with brine (5 mL × 3), dried over anhydrous $Na_2SO_4$ and concentrated under reduced pressure to give a

residue. The residue was purified by silica gel column chromatography (1% MeOH in DCM) to give a crude product as a yellow solid. Thereafter, the crude product was further purified by prep-HPLC (column: Xtimate C18 150*40mm*10μm; mobile phase: [water ($NH_3H_2O$+$NH_4HCO_3$)-MeCN]; 18%-58% B over 32 min) to afford two peaks. The compound obtained in peak 1 was concentrated under reduced pressure to remove most of MeCN. The remaining solvent was removed by lyophilization to give **a compound of Example 16** (7.1 mg, 13.67% yield, 100% purity) as a white solid. The compound obtained in peak 2 was concentrated under reduced pressure to remove most of MeCN. The remaining solvent was removed by lyophilization to give **a compound of Example 17** (3.6 mg, 7.20% yield, 99.7% purity) as a white solid.

**Compound of Example 16:** $^1$H NMR (400 MHz, DMSO- $d_6$) δ = 9.13 (d, *J* = 2.4 Hz, 1H), 9.00 (d, *J* = 8.4 Hz, 1H), 8.92 (d, *J* = 2.0 Hz, 1H), 8.52 (t, *J* = 2.0 Hz, 1H), 8.13 (s, 1H), 7.93 (d, *J* = 9.6 Hz, 1H), 7.39 (dt, *J* = 1.2, 7.2 Hz, 1H), 7.27 - 7.21 (m, 2H), 7.13 - 7.08 (m, 1H), 5.41 (quin, *J* = 7.2 Hz, 1H), 4.47 - 4.27 (m, 2H), 4.17 (s, 3H), 1.47 (d, *J* = 7.2 Hz, 3H), 0.97 (m, 2H), 0.90 - 0.85 (m, 2H); MS (ESI) m/z = 490.0 [M+H] $^+$.

**Compound of Example 17:** $^1$H NMR (400 MHz, DMSO-$d_6$) δ = 9.13 (d, *J* = 2.4 Hz, 1H), 9.02 (d, *J* = 8.0 Hz, 1H), 8.93 (d, *J* = 2.0 Hz, 1H), 8.52 (t, *J* = 2.0 Hz, 1H), 8.14 (s, 1H), 7.93 (d, *J* = 9.6 Hz, 1H), 7.44 - 7.34 (m, 1H), 7.25 - 7.18 (m, 2H), 7.18 - 7.13 (m, 1H), 6.38 (d, *J* = 2.8 Hz, 1H), 5.41 (quin, *J* = 7.2 Hz, 1H), 4.17 (s, 3H), 2.84 - 2.79 (m, 2H), 2.55 (m, 2H), 1.49 (d, *J* = 7.2 Hz, 3H); MS (ESI) m/z = 472.2 [M+H] $^+$.

**Example 18: N-[(1R)-1-[2-fluoro-3-[1-(methoxymethyl)cyclopropyl]phenyl]ethyl]-1-[5-(3-methyltriazol-4-yl)-3-pyridyl]-6-oxo-pyridazine-3-carboxamide**

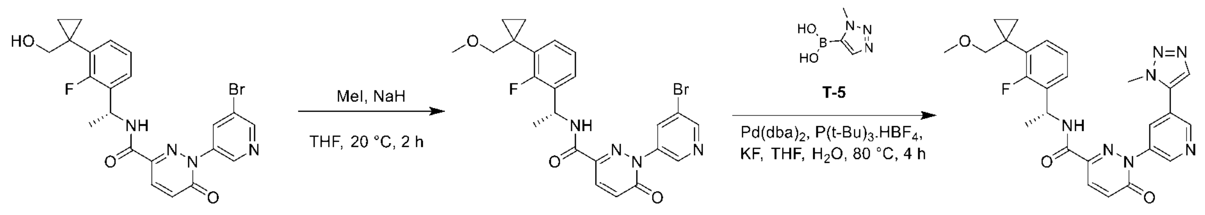


D-11

Example 18

Step 1: Synthesis of 1-(5-bromo-3-pyridyl)-N-[(1R)-1-[2-fluoro-3-[1-(methoxymethyl)cyclopropyl]phenyl]ethyl]-6-oxo-pyridazine-3-carboxamide

To a mixture of Intermediate D-11 (100 mg, 205.20 μmol) in THF (3 mL) was added NaH (24.62 mg, 615.61 μmol, 60% purity) at 50 °C. The resulting mixture was stirred at 20 °C for 15 minutes. Iodomethane (58.25 mg, 410.41 μmol, 25.55 μL) was added to the reaction mixture, and the mixture was stirred at 20 °C for 2 h. The reaction mixture was quenched with ice-cold water (10 ml) and extracted with EtOAc (30 mL × 3). The combined organic layers were washed with brine (50 mL), dried over anhydrous $Na_2SO_4$ and concentrated under reduced pressure to give a crude product. The crude product was purified by silica gel column chromatography (32% EtOAc in PE) to give 1-(5-bromo-3-pyridyl)-N-[(1R)-1-[2-fluoro-3-[1-(methoxymethyl)cyclopropyl]phenyl]ethyl]-6-oxo-pyridazine-3-carboxamide (46mg, 29.27% yield, 65.47% purity) as yellow oil. MS (ESI) m/z = 502.9 [M+H]$^+$.

Step 2: Synthesis of N-[(1R)-1-[2-fluoro-3-[1-(methoxymethyl)cyclopropyl]phenyl]ethyl]-1-[5-(3-methyltriazol-4-yl)-3-pyridyl]-6-oxo-pyridazine-3-carboxamide

A mixture of 1-(5-bromo-3-pyridyl)-N-[(1R)-1-[2-fluoro-3-[1-(methoxymethyl)cyclopropyl]phenyl]ethyl]-6-oxo-pyridazine-3-carboxamide (46 mg, 91.75 μmol), (3-methyltriazol-4-yl)boronic acid (34.93 mg, 275.26 μmol), KF (10.66 mg, 183.51 μmol, 4.30 μL), $Pd(dba)_2$ (5.28 mg, 9.18 μmol) and tri-tert-butylphosphonium;tetrafluoroborate (2.66 mg, 9.18 μmol) in THF (2 mL)/$H_2O$ (0.4 mL) was degassed and purged with $N_2$ for 3 times, and then was stirred at 80 °C for 4 h. Concentrated $NH_3 \cdot H_2O$ (51.45 mg, 367.04 μmol, 56.54 μL, 25% purity) was added, and the mixture was stirred at 20 °C for 24 h under $N_2$ atmosphere. The reaction mixture was filtrated, and the filtrate was concentrated under reduced pressure to give a crude product. The crude product was purified by prep-HPLC (column: Xtimate C18 150*40mm*10μm;mobile phase: [water($NH_3H_2O$+$NH_4HCO_3$)-ACN];18%-58% B over 32 min), and the remaining solvent was removed by lyophilization to give a **compound of Example 18** (6.5 mg, 13.44% yield, 95.53% purity) as a white solid. $^1$H NMR (400 MHz, DMSO-$d_6$) δ = 9.13 (d, *J* = 2.4 Hz, 1H), 8.98 (d, *J* = 8.4 Hz, 1H), 8.92 (d, *J* = 2.0 Hz, 1H), 8.52 (t, *J* = 2.0 Hz, 1H), 8.13 (s, 1H), 7.93 (d, *J* = 10.0 Hz, 1H), 7.35 (t, *J* = 6.8 Hz, 1H), 7.25-7.18 (m, 2H), 7.09-7.03 (m, 1H), 5.40 (q, *J* = 7.6 Hz, 1H), 4.17 (s, 3H), 3.41-3.35 (m, 2H), 3.16 (s, 3H), 1.47 (d, *J* = 7.2 Hz, 3H), 0.86-0.81 (m, 2H), 0.78-0.72 (m, 2H); MS (ESI) m/z = 504.2 [M+H]$^+$.

**Example 19: N-{(R)-1-[2-fluoro-3-(trifluoromethyl)phenyl]ethyl}-1-{5-[1-(1-methyl-3-azetidinyl)-1H-1,2,3-triazol-5-yl]-3-pyridyl}-6-oxo-1,6-dihydropyridazine-3-carboxamide**

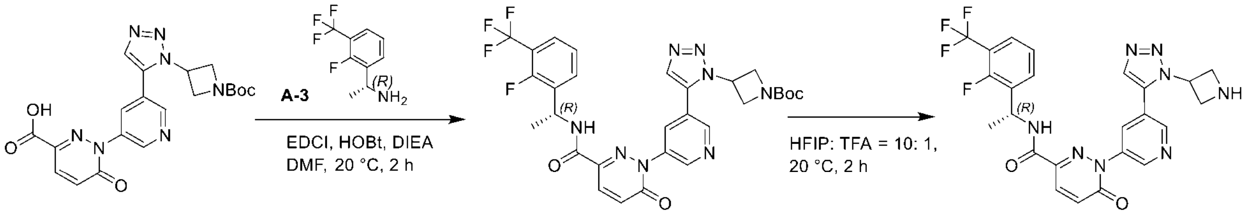


4-66

Example 19

Step 1: Synthesis of tert-butyl 3-[5-[5-[3-[[(1R)-1-[2-fluoro-3-(trifluoromethyl)phenyl]ethyl]carbarmoyl]-6-oxo-pyridazin-1-yl]-3-pyridyl]triazol-1-yl]azetidine-1-carboxylate

Intermediate 4-66 and Intermediate A-3 were used for synthesis in the same manner as in Example 1 to give tert-butyl 3-[5-[5-[3-[[(1R)-1-[2-fluoro-3-(trifluoromethyl)phenyl]ethyl]carbamoyl]-6-oxo-pyridazin-1-yl]-3-pyridyl]triazol-1-yl]azetidine-1-carboxylate (270 mg , 45.46% yield, 91.53% purity) as a yellow solid. MS (ESI) m/z = 629.4 [M+H]$^+$.

Step 2: Synthesis of N-{(R)-1-[2-fluoro-3-(trifluoromethyl)phenyl]ethyl}-1-{5-[1-(1-methyl-3-azetidinyl)-1H-1,2,3-triazol-5-yl]-3-pyridyl }-6-oxo-1,6-dihydropyridazine-3-carboxamide

To a mixture of tert-butyl 3-[5-[5-[3-[[(1R)-1-[2-fluoro-3-(trifluoromethyl)phenyl]ethyl]carbamoyl]-6-oxo-pyridazin-1-yl]-3-pyridyl]triazol-1-yl]azetidine-1-carboxylate (250 mg, 397.72 μmol) in HFIP (2 mL) was added TFA (0.2 mL), and the mixture was degassed and purged with $N_2$ for 3 times. Then the mixture was stirred at 20 °C for 2 h under $N_2$ atmosphere. The mixture was concentrated under reduced pressure to give a crude product. The crude product was purified by prep-TLC ($SiO_2$, DCM: MeOH = 5:1) to give a compound as white oil. It was further purified by prep-HPLC (column: Welch Xtimate C18 40*200mm 7μm; mobile phase: [water($NH_4HCO_3$)-MeCN]; 12%-52% B over 25 min) to give a **compound of Example 19** (7.4 mg, 24.49% yield, 99.27% purity) as a white solid. $^1$H NMR (400 MHz, DMSO-$d_6$) δ = 9.17-9.09 (m, 2H), 8.80 (d, *J* = 2.0 Hz, 1H), 8.36 (t, *J* = 2.0 Hz, 1H), 8.14 (s, 1H), 7.92 (d, *J* = 10.0 Hz, 1H), 7.84 (br t, *J* = 7.2 Hz, 1H), 7.67 (t, *J* = 6.8 Hz, 1H), 7.40 (t, *J* = 8.0 Hz, 1H), 7.22 (d, *J* = 10.0 Hz, 1H), 5.45 (quind, *J* = 7.6, 15.2 Hz, 2H), 4.06 (dt, *J* = 4.0, 7.6 Hz, 2H), 3.79 (br t, *J* = 8.0 Hz, 2H), 1.52 (d, *J* = 7.2 Hz, 3H); MS (ESI) m/z = 529.2 [M+H] $^+$.

**Example 20: N-{(R)-1-[2-fluoro-3-(trifluoromethyl)phenyl]ethyl}-1-{5-[1-(1-methyl-3-azetidinyl)-1H-1,2,3-triazol-5-yl]-3-pyridyl}-6-oxo-1,6-dihydropyridazine-3-carboxamide**

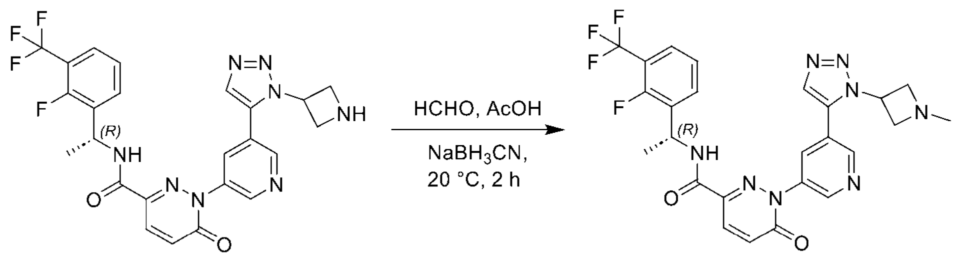


Example 19

Example 20

To a compound of Example 19 (50 mg, 94.61 μmol) in MeOH (1 mL) were added AcOH (11.36 mg, 189.23 μmol, 10.83 μL), $NaBH_3CN$ (23.78 mg, 378.46 μmol) and HCHO (15.36 mg, 189.23 μmol, 14.09 μL) in MeOH (1 mL). The mixture was degassed and purged with $N_2$ for 3 times, and then stirred at 20 °C for 2 h under $N_2$ atmosphere. The mixture was concentrated under reduced pressure to give a crude product. The crude product was dissolved in MeOH (5 mL) and purified by prep-HPLC (column: Welch Xtimate C18 40*200mm 7μm;mobile phase: [water($NH_3H_2O$+$NH_4HCO_3$)-MeCN]; 20%-60% B over 25 min) to give a **compound of Example 20** (9.5 mg, 18.51% yield, 100% purity) as a white solid. $^1$H NMR (400 MHz, DMSO-$d_6$) δ = 9.16-9.08 (m, 2H), 8.82 (d, *J* = 2.0 Hz, 1H), 8.39 (t, *J* = 2.0 Hz, 1H), 8.14 (s, 1H), 7.92 (d, *J* = 10.0

Hz, 1H), 7.83 (t, *J* = 7.2 Hz, 1H), 7.68 (t, *J* = 7.2 Hz, 1H), 7.40 (t, *J* = 8.0 Hz, 1H), 7.23 (d, *J* = 10.0 Hz, 1H), 5.43 (quin, *J* = 7.2 Hz, 1H), 5.24 (quin, *J* = 6.8 Hz, 1H), 3.83-3.77 (m, 2H), 3.45 (t, *J* = 6.8 Hz, 2H), 2.29 (s, 3H), 1.52 (d, *J* = 7.2 Hz, 3H); MS (ESI) m/z = 543.2 $[M+H]^+$.

**Example 20-1: (R)-N-(1-(2-fluoro-3-(trifluoromethyl)phenyl)ethyl)-1-(5-(1-(1-(2-hydroxyethyl)azetidin-3-yl)-1H-1,2,3-triazol-5-yl)pyridin-3-yl)-6-oxo-1,6-dihydropyridazine-3-carboxamide**

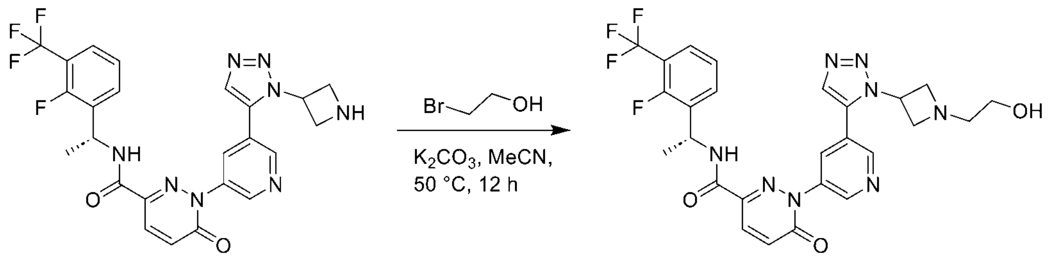


**Example 19** **Example 20-1**

A mixture of the compound of Example 19 (60 mg, 113.54 μmol), 2-bromoethanol (14.19 mg, 113.54 μmol, 8.05 μL) and $K_2CO_3$ (47.07 mg, 340.61 μmol) in MeCN (2 mL) was stirred at 50 °C for 12 h, and then concentrated under reduced pressure to give a crude product. The crude product was purified by prep-TLC ($SiO_2$, DCM:MeOH = 10:1) to give a residue. The resulting product was dissolved in MeOH (5 mL) and purified by prep-HPLC (column: F-Prepulite XP tC 18 40*200mm*7μm;mobile phase: [water($NH_3H_2O$+$NH_4HCO_3$)-ACN];gradient:16%-56% B over 20 min). Most of MeCN was concentrated under reduced pressure and the remaining solvent was removed by lyophilization to give a **compound of Example 20-1** (10.4 mg, 15.94% yield, 99.64% purity) as a white solid. $^1H$ NMR (400 MHz, DMSO-$d_6$) δ = 9.16-9.09 (m, 2H), 8.82 (d, *J* = 1.6 Hz, 1H), 8.39 (s, 1H), 8.14 (s, 1H), 7.92 (d, *J* = 10.0 Hz, 1H), 7.83 (br t, *J* = 7.2 Hz, 1H), 7.67 (br t, *J* = 7.2 Hz, 1H), 7.40 (t, *J* = 8.0 Hz, 1H), 7.23 (d, *J* = 10.0 Hz, 1H), 5.43 (quin, *J* = 7.2 Hz, 1H), 5.28 (quin, *J* = 6.8 Hz, 1H), 4.46-4.38 (m, 1H), 3.82 (br t, *J* = 7.6 Hz, 2H), 3.52 (br t, *J* = 7.2 Hz, 2H), 3.37 (br s, 2H), 2.54 (br t, *J* = 6.0 Hz, 2H), 1.52 (br d, *J* = 7.2 Hz, 3H); MS (ESI) m/z = 573.2 $[M+H]^+$.

**Example 20-2: (R)-1-(5-(1-(azetidin-3-yl)-1H-1,2,3-triazol-5-yl)pyridin-3-yl)-N-(1-(3-(1,1-difluoro-2-hydroxyethyl)-2-fluorophenyl)ethyl)-6-oxo-1,6-dihydropyridazine-3-carboxamide**

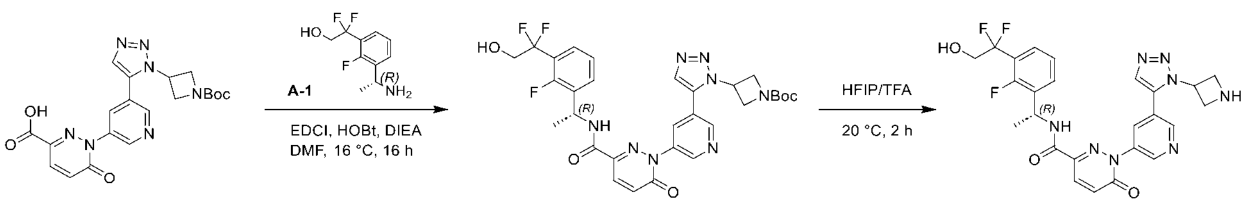


4-66 Example 20-2

Intermediate 4-66 and Intermediate A-1 were used for synthesis in the same manner as in Example 19 to give **a compound of Example 20-2** (13 mg, 34.15% yield, 99.73% purity) as a white solid. $^1H$ NMR (400 MHz, DMSO-$d_6$) δ = 9.14 (d, *J* = 2.4 Hz, 1H), 9.07 (d, *J* = 8.0 Hz, 1H), 8.80 (d, *J* = 2.0 Hz, 1H), 8.37 (t, *J* = 2.0 Hz, 1H), 8.14 (s, 1H), 7.93 (d, *J* = 10.0 Hz, 1H), 7.65 (br t, *J* = 7.2 Hz, 1H), 7.43 (br t, *J* = 6.8 Hz, 1H), 7.28 (t, *J* = 7.6 Hz, 1H), 7.23 (d, *J* = 10.0 Hz, 1H), 5.73 (br t, *J* = 5.6 Hz, 1H), 5.51 - 5.37 (m, 2H), 4.10 - 4.01 (m, 2H), 3.97 - 3.85 (m, 2H), 3.79 (br t, *J* = 8.0 Hz, 2H), 1.50 (d, *J* = 7.2 Hz, 3H); MS (ESI) m/z = 541.2 $[M+H]^+$.

**Example 20-3: (R)-1-(5-(1-(azetidin-3-yl)-1H-1,2,3-triazol-5-yl)pyridin-3-yl)-N-(1-(3-(1,1-difluoro-2-hydroxyethyl-2,2-dideuterio)-2-fluorophenyl)ethyl)-6-oxo-1,6-dihydropyridazine-3-carboxamide**

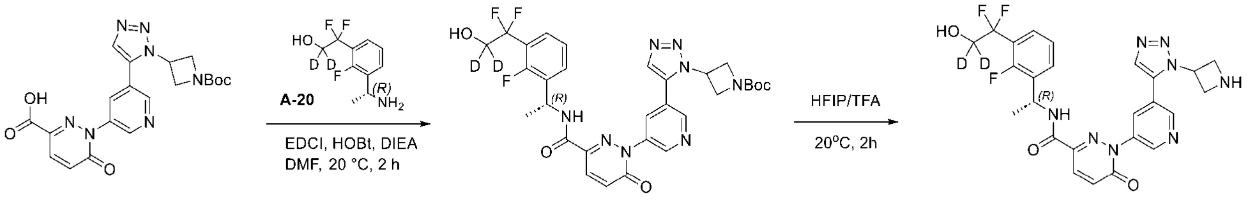


4-66

Example 20-3

Intermediate 4-66 and Intermediate A-20 were used for synthesis in the same manner as in Example 19 to give **a compound of Example 20-3** (15.2 mg, 36.01% yield, 100% purity, deuterated ratio: 98.97%) as a white solid. $^1$H NMR (400 MHz, DMSO-$d_6$) δ = 9.15 (d, *J* = 2.0 Hz, 1H), 9.08 (br d, *J* = 8.0 Hz, 1H), 8.80 (d, *J* = 2.0 Hz, 1H), 8.37 (t, *J* = 2.0 Hz, 1H), 8.14 (s, 1H), 7.93 (d, *J* = 10.0 Hz, 1H), 7.65 (br t, *J* = 6.8 Hz, 1H), 7.44 (t, *J* = 6.8 Hz, 1H), 7.28 (t, *J* = 7.6 Hz, 1H), 7.23 (d, *J* = 10.0 Hz, 1H), 5.68 (br s, 1H), 5.45 (td, *J* = 7.6, 20.0 Hz, 2H), 4.46-4.26 (m, 1H), 4.09 (br d, *J* = 3.2 Hz, 2H), 3.85 (br s, 2H), 1.50 (d, *J* = 7.2 Hz, 3H); MS (ESI) m/z = 543.2 [M+H]$^+$.

**Example 20-4: N-[(1R)-1-[2-fluoro-3-(trifluoromethyl)phenyl]ethyl]-6-oxo-1-[5-[3-(4-piperidyl)triazol-4-yl]-3-pyridyl]pyridazine-3-carboxamide**

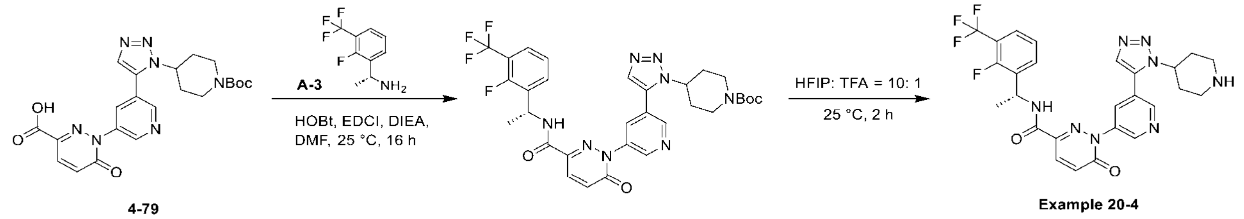


4-79

Example 20-4

Intermediate 4-79 and Intermediate A-3 were used for synthesis in the same manner as in Example 19 to give **a compound of Example 20-4** (14.2 mg, 23.70% yield, 99.037% purity) as a white solid. $^1$H NMR (400 MHz, DMSO-d6) δ = 9.17 - 9.12 (m, 2H), 8.81 (d, *J* = 2.0 Hz, 1H), 8.42 (t, *J* = 2.0 Hz, 1H), 8.04 (s, 1H), 7.92 (d, *J* = 10.0 Hz, 1H), 7.83 (t, *J* = 7.2 Hz, 1H), 7.68 (t, *J* = 7.2 Hz, 1H), 7.40 (t, *J* = 7.6 Hz, 1H), 7.23 (d, *J* = 10.0 Hz, 1H), 5.43 (quin, *J* = 7.2 Hz, 1H), 4.53 - 4.42 (m, 1H), 3.02 (br d, *J* = 12.8 Hz, 2H), 2.59 - 2.53 (m, 2H), 2.04 - 1.93 (m, 4H), 1.52 (d, *J* = 7.2 Hz, 3H); MS (ESI) m/z = 557.2 [M+H]$^+$.

**Example 20-5: N-[(1R)-1-[2-fluoro-3-(trifluoromethyl)phenyl]ethyl]-6-oxo-1-[5-[3-[(3S)-pyrrolidin-3-yl]triazol-4-yl]-3-pyridyl]pyridazine-3-carboxamide**

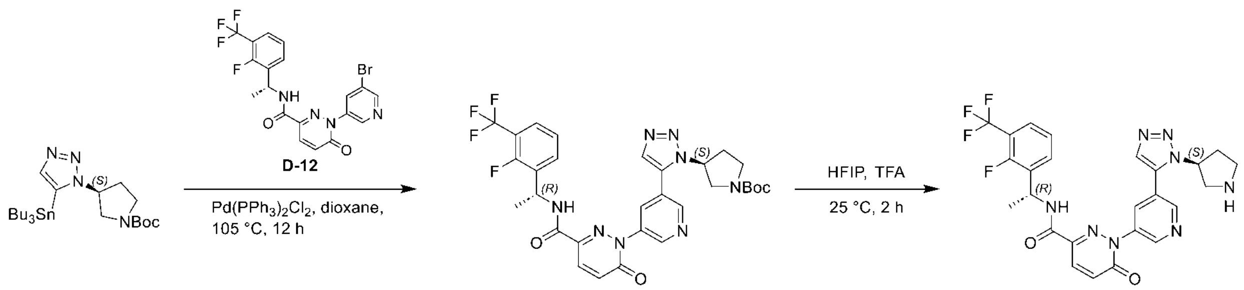


T-22

Example 20-5

Step 1 : Synthesis of tert-butyl (3S)-3-[5-[5-[3-[[(1R)-1-[2-fluoro-3-(trifluoromethyl)phenyl]ethyl]carbamoyl]-6-oxo-pyridazin-1-yl]-3-pyridyl]triazol-1-yl]pyrrolidine-1-carboxylate

A mixture of Intermediate T-22 (330 mg, 625.79 μmol), Intermediate D-12 (151.83 mg, 312.90 μmol) and $Pd(PPh_3)_2Cl_2$ (21.96 mg, 31.29 μmol) in dioxane (4 mL) was degassed and purged with nitrogen for 3 times, and then stirred at 105 °C for 12 h under $N_2$ atmosphere. The reaction mixture was filtered, and the filtrate was concentrated under reduced pressure and purified by column chromatography (0~2% MeOH/DCM) to give tert-butyl

(3S)-3-[5-[5-[3-[[(1R)-1-[2-fluoro-3-(trifluoromethyl)phenyl]ethyl]carbamoyl]-6-oxo-pyridazin-1-yl]-3-pyridyl]triazol-1-yl] pyrrolidin-1-carboxylate (170 mg, 76.94% yield, 91% purity) as a yellow solid. MS (ESI) m/z = 643.2 [M+H]+.

Step 2: Synthesis of -N-[(1R)-1-[2-fluoro-3-(trifluoromethyl)phenyl]ethyl]-6-oxo-1 [5-[3-[(3S)-pyrrolidin-3-yl]triazol-4-yl]-3-pyridyl]pyridazine-3-carboxamide

**The compound of Example 20-5** (23.9 mg, 16.65% yield, 100% purity) was obtained as a white solid in the same manner as in Step 2 of Example 19. $^1$H NMR (400 MHz, DMSO-$d_6$) δ = 9.16 (s, 1H), 9.15 - 9.11 (m, 1H), 8.82 (s, 1H), 8.43 (s, 1H), 8.07 (s, 1H), 7.92 (d, *J* = 9.6 Hz, 1H), 7.83 (br t, *J* = 7.2 Hz, 1H), 7.68 (br t, *J* = 7.2 Hz, 1H), 7.40 (t, *J* = 7.6 Hz, 1H), 7.22 (d, *J* = 9.6 Hz, 1H), 5.43 (br t, *J* = 7.2 Hz, 1H), 5.01 (br s, 1H), 3.21 (br dd, *J* = 6.8, 11.6 Hz, 2H), 3.13 - 3.07 (m, 2H), 2.92 - 2.84 (m, 1H), 2.25 - 2.14 (m, 2H), 1.52 (br d, *J* = 7.2 Hz, 3H); MS (ESI) m/z = 543.2 [M+H]+.

**Example 20-6: (R)-N-(1-(2-fluoro-3-(trifluoromethyl)phenyl)ethyl)-1-(5-(1-(1-methylpiperidin-4-yl)-1H-1,2,3-triazol-5-yl)pyridin-3-yl)-6-oxo-1,6-dihydropyridazine-3-carboxamide**

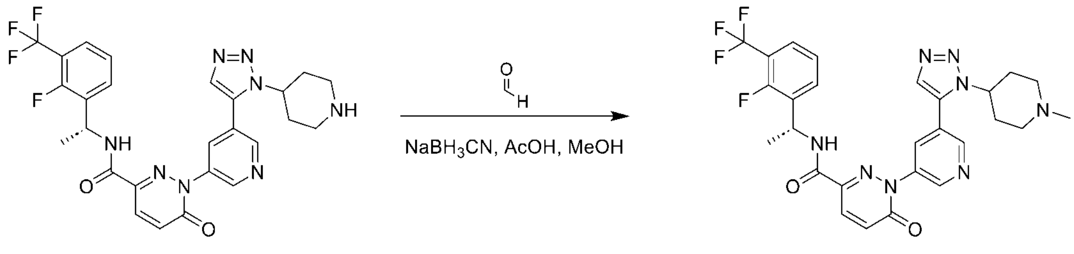


Example 20-4 Example 20-6

Example 20-4 was reacted in the same manner as in Example 30 to obtain a **compound of Example 20-6** (23.9 mg, 38.85% yield, 100% purity) as a white solid. $^1$H NMR (400 MHz, DMSO-$d_6$) δ = 9.16 (d, *J* = 2.4 Hz, 1H), 9.14 (d, *J* = 8.0 Hz, 1H), 8.81 (d, *J* = 2.0 Hz, 1H), 8.44 (t, *J* = 2.0 Hz, 1H), 8.05 (s, 1H), 7.91 (d, *J* = 10.0 Hz, 1H), 7.83 (t, *J* = 7.2 Hz, 1H), 7.68 (t, *J* = 7.2 Hz, 1H), 7.40 (t, *J* = 8.0 Hz, 1H), 7.24 (d, *J* = 10.0 Hz, 1H), 5.43 (quin, *J* = 7.2 Hz, 1H), 4.38 (ddd, *J* = 4.0, 7.2, 11.2 Hz, 1H), 2.90 - 2.80 (m, 2H), 2.20 (br d, *J* = 4.0 Hz, 1H), 2.18 (s, 3H), 2.14 (br s, 1H), 2.05 - 1.97 (m, 4H), 1.53 (d, *J* = 7.2 Hz, 3H); MS (ESI) m/z = 571.0 [M+H]+.

**Example 20-7: (R)-1-(5-(1-(1-acetylpiperidin-4-yl)-1H-1,2,3-triazol-5-yl)pyridin-3-yl)-N-(1-(2-fluoro-3-(trifluoromethyl)phenyl)ethyl)-6-oxo-1,6-dihydropyridazine-3-carboxamide**

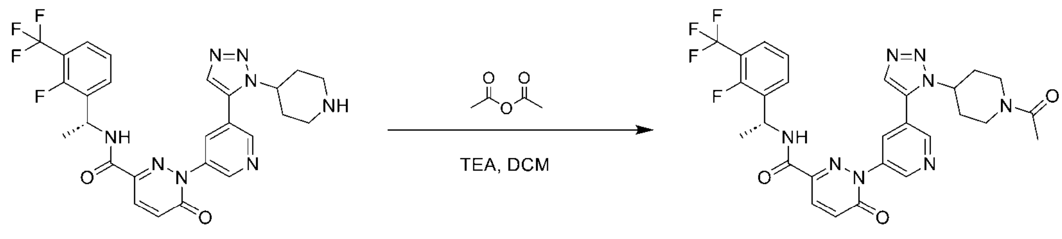


Example 20-4 Example 20-7

To a mixture of N-[(1R)-1-[2-fluoro-3-(trifluoromethyl)phenyl]ethyl]-6-oxo-1-[5-[3-(4-piperidyl)triazol-4-yl]-3-pyridyl]pyridazine-3-carboxamide (**Example 20-4,** 50.00 mg, 89.85 μmol) in DCM (2 mL) was added TEA (13.64 mg, 134.77 μmol, 18.76 μL) and acetic anhydride (11.01 mg, 107.81 μmol, 10.13 μL). The mixture was stirred at 25 °C for 2 h. The reaction mixture was filtered, and the filtrate was concentrated under reduced pressure to give a crude product. The crude product was purified by prep-HPLC (column: F-Prepulite XP tC 18 40*200mm*7um; mobile phase: [water($NH_3H_2O$+$NH_4HCO_3$)-ACN]; 22%-62% B, 20 min), and then most of ACN was removed under reduced pressure. The remaining solvent was removed by lyophilization to give a **compound of Example 20-7** (27.9 mg, 51.88% yield, 100% purity) as a white solid. $^1$H NMR (400 MHz, $CD_3OD$) δ = 9.13 (d, *J* = 2.0 Hz, 1H), 8.80 (d, *J* = 1.6 Hz, 1H), 8.51 - 8.44 (m, 1H), 8.02 (d, *J* = 10.0 Hz, 1H), 8.00 (s, 1H), 7.73 (t, *J* = 6.8 Hz, 1H), 7.60 (t, *J* = 7.2 Hz, 1H), 7.32 (t, *J* = 8.0 Hz, 1H), 7.22 (d, *J* = 9.6

Hz, 1H), 5.51 (q, *J* = 7.2 Hz, 1H), 4.82 - 4.73 (m, 2H), 4.72 - 4.48 (m, 2H), 4.08 (br d, *J* = 13.2 Hz, 1H), 2.95 - 2.73 (m, 1H), 2.36 - 2.25 (m, 1H), 2.19 (br d, *J* = 2.4 Hz, 1H), 2.15 (s, 4H), 1.60 (d, *J* = 7.2 Hz, 3H); $^1$H NMR (400 MHz, DMSO-$d_6$) δ = 9.17 (d, *J* = 2.4 Hz, 1H), 9.15 (d, *J* = 8.0 Hz, 1H), 8.84 (d, *J* = 2.0 Hz, 1H), 8.46 (t, *J* = 2.0 Hz, 1H), 8.07 (s, 1H), 7.93 (d, *J* = 10.0 Hz, 1H), 7.83 (t, *J* = 7.2 Hz, 1H), 7.68 (t, *J* = 7.2 Hz, 1H), 7.40 (t, *J* = 7.6 Hz, 1H), 7.24 (d, *J* = 10.0 Hz, 1H), 5.52 - 5.34 (m, 1H), 4.79 - 4.63 (m, 1H), 4.46 (br d, *J* = 14.0 Hz, 1H), 3.93 (br d, *J* = 13.6 Hz, 1H), 3.25 - 3.13 (m, 1H), 2.75 - 2.65 (m, 1H), 2.11 (br d, *J* = 8.8 Hz, 2H), 2.09 - 2.06 (m, 1H), 2.04 (s, 3H), 2.00 - 1.86 (m, 1H), 1.52 (d, *J* = 7.2 Hz, 3H); MS (ESI) m/z = 599.2 [M+H]$^+$.

**Example 20-8: (R)-N-(1-(2-fluoro-3-(trifluoromethyl)phenyl)ethyl)-1-(5-(1-(1-(methylsulfonyl)piperidine-4-yl)-1H-1,2,3-triazol-5-yl)pyridin-3-yl)-6-oxo-1,6-dihydropyridazine-3-carboxamide**

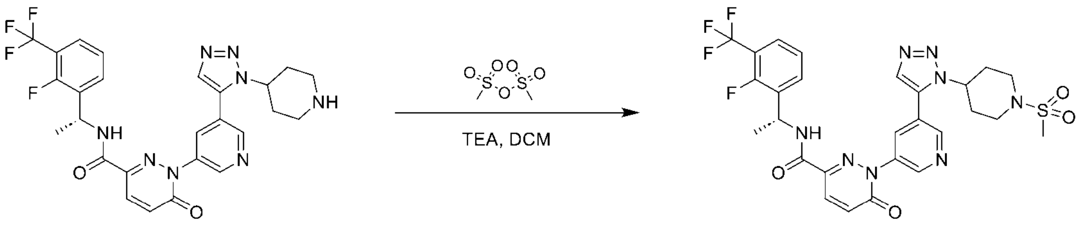


Example 20-4 Example 20-8

The compound of Example 20-4 and commercially available methanesulfonic acid anhydride were used for synthesis in the same manner as in Example 20-7 to give a **compound of Example 20-8** (41.8 mg, 73.31% yield, 100% purity) as a white solid. $^1$H NMR (400 MHz, DMSO-$d_6$) δ = 9.19 (d, *J* = 2.4 Hz, 1H), 9.14 (d, *J* = 8.0 Hz, 1H), 8.83 (d, *J* = 2.0 Hz, 1H), 8.46 (t, *J* = 2.0 Hz, 1H), 8.07 (s, 1H), 7.92 (d, *J* = 10.0 Hz, 1H), 7.83 (br t, *J* = 7.2 Hz, 1H), 7.68 (t, *J* = 7.2 Hz, 1H), 7.44 - 7.37 (m, 1H), 7.24 (d, *J* = 10.0 Hz, 1H), 5.43 (quin, *J* = 7.2 Hz, 1H), 4.66 - 4.56 (m, 1H), 3.68 (br d, *J* = 12.0 Hz, 2H), 2.99 - 2.93 (m, 2H), 2.91 (s, 3H), 2.19 (br s, 4H), 1.53 (d, *J* = 7.2 Hz, 3H); MS (ESI) m/z = 635.5 [M+H] $^+$.

**Example 20-9: (R)-1-(5-(1-(1-ethylazetidin-3-yl)-1H-1,2,3-triazol-5-yl)pyridin-3-yl)-N-(1-(2-fluoro-3-(trifluoromethyl)phenyl)ethyl)-6-oxo-1,6-dihydropyridazine-3-carboxamide**

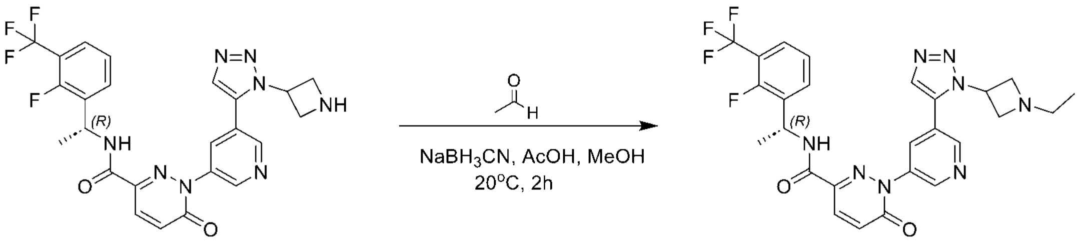


Example 19 Example 20-9

The compound of Example 19 was reacted in the same manner as in Example 30 to obtain a **compound of Example 20-9** (35.3 mg, 55.87% yield, 100% purity) as a white solid. $^1$H NMR (400 MHz, DMSO-$d_6$) δ = 9.13 (d, *J* = 2.4 Hz, 1H), 9.11 (d, *J* = 8.0 Hz, 1H), 8.82 (d, *J* = 2.0 Hz, 1H), 8.40 (t, *J* = 2.0 Hz, 1H), 8.14 (s, 1H), 7.92 (d, *J* = 10.0 Hz, 1H), 7.83 (t, *J* = 7.2 Hz, 1H), 7.68 (t, *J* = 7.2 Hz, 1H), 7.40 (t, *J* = 8.0 Hz, 1H), 7.23 (d, *J* = 10.0 Hz, 1H), 5.42 (quin, *J* = 7.2 Hz, 1H), 5.26 (quin, *J* = 6.8 Hz, 1H), 3.78 (t, *J* = 7.2 Hz, 2H), 3.44-3.40 (m, 2H), 2.48-2.43 (m, 2H), 1.53 (d, *J* = 7.2 Hz, 3H), 0.87 (t, *J* = 7.2 Hz, 3H); MS (ESI) m/z = 557.4 [M+H]$^+$.

**Example 20-10: (R)-1-(5-(1-(1-acetylazetidin-3-yl)-1H-1,2,3-triazol-5-yl)pyridin-3-yl)-N-(1-(2-fluoro-3-(trifluoromethyl)phenyl)ethyl)-6-oxo-1,6-dihydropyridazine-3-carboxamide**

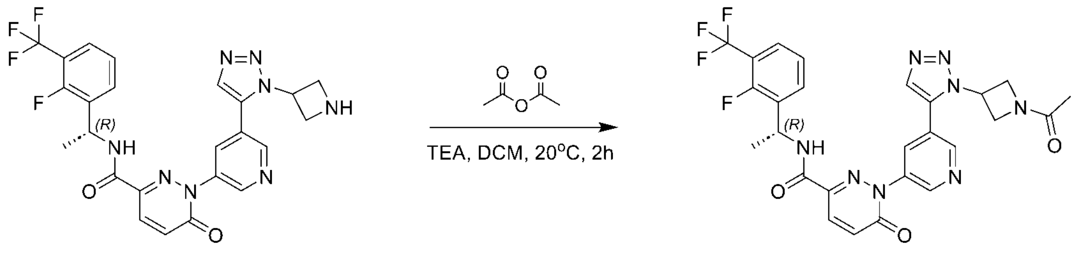


Example 19 Example 20-10

The compound of Example 19 was reacted in the same manner as in Example 20-7 to obtain a **compound of Example 20-10** (24.1 mg, 44.65% yield, 100% purity) as a white solid. $^1$H NMR (400 MHz, DMSO-$d_6$) $\delta$ = 9.20-9.07 (m, 2H), 8.82 (s, 1H), 8.38 (br s, 1H), 8.18 (s, 1H), 7.93 (d, *J* = 9.6 Hz, 1H), 7.83 (br t, *J* = 7.2 Hz, 1H), 7.68 (br t, *J* = 7.2 Hz, 1H), 7.45-7.35 (m, 1H), 7.24 (d, *J* = 10.0 Hz, 1H), 5.56-5.47 (m, 1H), 5.46-5.38 (m, 1H), 4.68-4.60 (m, 2H), 4.43-4.34 (m, 1H), 4.32-4.24 (m, 1H), 1.80 (s, 3H), 1.52 (br d, *J* = 7.2 Hz, 3H); MS (ESI) m/z = 571.2 [M+H]$^+$.

**Example 20-11: methyl (R)-3-(5-(5-(3-((1-(2-fluoro-3-(trifluoromethyl)phenyl)ethyl)carbamoyl)-6-oxopyridazin-1(6H)-yl)pyridin-3-yl)-1H-1,2,3-triazol-1-yl)azetidine-1-carboxylate**

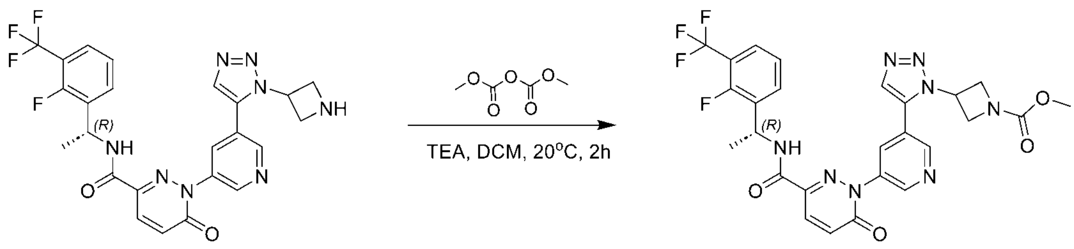


Example 19 Example 20-11

The compound of Example 19 and commercially available dimethyl dicarbonate were reacted in the same manner as in Example 20-7 to obtain a **compound of Example 20-11** (30.3 mg, 54.54% yield, 99.88% purity) as a white solid. $^1$H NMR (400 MHz, DMSO-$d_6$) $\delta$ = 9.16 (d, *J* = 2.0 Hz, 1H), 9.13 (br d, *J* = 8.0 Hz, 1H), 8.81 (d, *J* = 1.6 Hz, 1H), 8.36 (s, 1H), 8.17 (s, 1H), 7.93 (d, *J* = 9.6 Hz, 1H), 7.83 (br t, *J* = 7.2 Hz, 1H), 7.68 (br t, *J* = 7.2 Hz, 1H), 7.40 (t, *J* = 8.0 Hz, 1H), 7.24 (d, *J* = 10.0 Hz, 1H), 5.55-5.47 (m, 1H), 5.43 (br t, *J* = 7.2 Hz, 1H), 4.49-4.37 (m, 4H), 3.59 (s, 3H), 1.52 (br d, *J* = 7.2 Hz, 3H); MS (ESI) m/z = 587.0 [M+H]$^+$.

**Example 20-12: (R)-N-(1-(2-fluoro-3-(trifluoromethyl)phenyl)ethyl)-1-(5-(1-(1-(methylsulfonyl)azetidin-3-yl)-1H-1,2,3-triazol-5-yl)pyridin-3-yl)-6-oxo-1,6-dihydropyridazine-3-carboxamide**

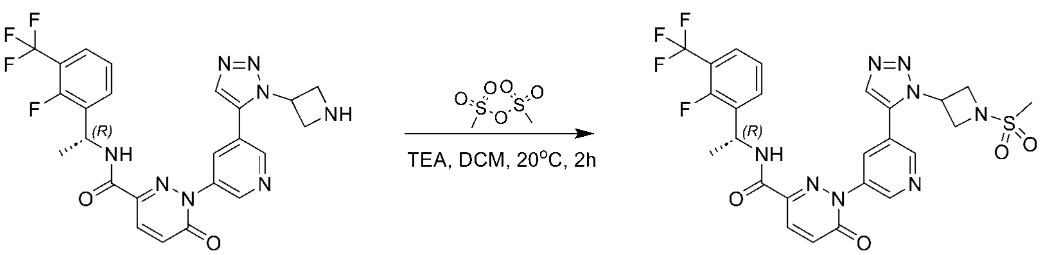


Example 19 Example 20-12

The compound of Example 19 and commercially available methanesulfonic anhydride were reacted in the same manner as in Example 20-7 to give **a compound of Example 20-12** (10.9 mg, 18.97% yield, 99.86% purity) as a white solid. $^1$H NMR (400 MHz, DMSO-$d_6$) $\delta$ = 9.22-9.06 (m, 2H), 8.82 (br s, 1H), 8.45-8.32 (m, 1H), 8.19 (s, 1H), 7.93 (br d, *J* = 9.6

Hz, 1H), 7.83 (br t, $J$ = 6.4 Hz, 1H), 7.72-7.63 (m, 1H), 7.45-7.36 (m, 1H), 7.24 (br d, $J$ = 10.0 Hz, 1H), 5.59-5.48 (m, 1H), 5.43 (br t, $J$ = 6.8 Hz, 1H), 4.48 (br s, 2H), 4.42-4.35 (m, 2H), 3.12 (s, 3H), 1.53 (br d, $J$ = 6.8 Hz, 3H); MS (ESI) m/z = 606.9 $[M+H]^+$.

**Example 21: N-[(R)-1-{3-[1,1-difluoro-2-hydroxyethyl-2,2-dideuterio]-2-fluorophenyl}ethyl]-1-[5-(1-cyclopropyl-1H-1,2,3-triazol-5-yl)-3-pyridyl]-6-oxo-1,6-dihydropyridazine-3-carboxamide**

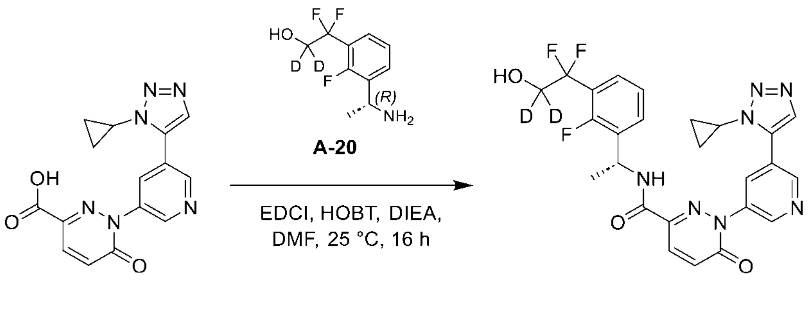


4-47

Example 21

Intermediate 4-47 and Intermediate A-20 were used for synthesis in the same manner as in Example 1 to give **a compound of Example 21** (40.6 mg, 28.38% yield, 100% purity, deuterated ratio: 99.00%) as a white solid. $^1H$ NMR (400 MHz, DMSO-$d_6$) δ = 9.12 (d, $J$ = 2.4 Hz, 1H), 9.07 (d, $J$ = 8.0 Hz, 1H), 9.02 (d, $J$ = 2.0 Hz, 1H), 8.65 (t, $J$ = 2.0 Hz, 1H), 8.17 (s, 1H), 7.93 (d, $J$ = 9.6 Hz, 1H), 7.64 (t, $J$ = 6.8 Hz, 1H), 7.44 (t, $J$ = 6.8 Hz, 1H), 7.28 (t, $J$ = 7.6 Hz, 1H), 7.22 (d, $J$ = 9.6 Hz, 1H), 5.68 (br s, 1H), 5.42 (quin, $J$ = 7.2 Hz, 1H), 4.06 - 4.00 (m, 1H), 1.49 (d, $J$ = 7.2 Hz, 3H), 1.19 - 1.13 (m, 4H); MS (ESI) m/z = 528.3 $[M+H]^+$.

**Example 22: (R)-N-(1-(2-fluoro-3-(trifluoromethyl)phenyl)ethyl-1,2,2,2-tetradeuterio)-1-(5-(1-methyl-1H-1,2,3-triazol-5-yl)pyridin-3-yl)-6-oxo-1,6-dihydropyridazine-3-carboxamide**

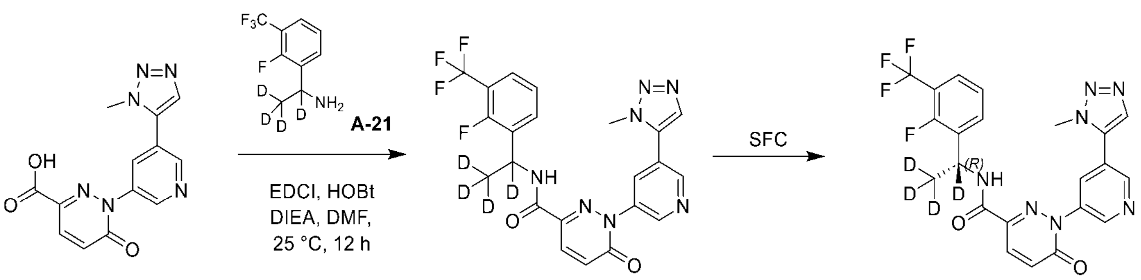


4-1

Example 22

Intermediate 4-1 and Intermediate A-21 were used for synthesis in the same manner as in Example 1 to give (+/-) N-(1-(2-fluoro-3-(trifluoromethyl)phenyl)ethyl-1,2,2,2-tetradeuterio)-1-(5-(1-methyl-1H-1,2,3-triazol-5-yl)pyridin-3-yl)-6-oxo-1,6-dihydropyridazine-3-carboxamide (50 mg, 40.47% yield, 94.18% purity) as yellow oil. It was further purified by SFC separation (column: REGIS (s,s) WHELK-O1 (250mm*30mm,5um); mobile phase: [$CO_2$-MeOH(0.1% $NH_3H_2O$)];B%:50%, isocratic elution mode). The resulting compound was added with MeCN (5 mL) and $H_2O$ (25 mL) and dried by lyophilization to obtain a white solid. It was dissolved in MeOH (5 mL) and purified by prep-HPLC (column: Welch Xtimate C18 40*200mm 7μm; mobile phase: [water ($NH_3H_2O$+$NH_4HCO_3$)-MeCN]; 18%-58% B over 25 min) to give a **compound of Example 22** (7.3 mg, 14.48% yield, 99.18% purity, 100 % ee, deuterated ratio: 99.609 %) as a white solid. $^1H$ NMR (400 MHz, DMSO-$d_6$) δ = 9.12 (d, $J$ = 2.4 Hz, 2H), 8.92 (d, $J$ = 2.0 Hz, 1H), 8.51 (t, $J$ = 2.4 Hz, 1H), 8.13 (s, 1H), 7.92 (d, $J$ = 10.0 Hz, 1H), 7.83 (t, $J$ = 7.2 Hz, 1H), 7.68 (t, $J$ = 6.8 Hz, 1H), 7.40 (t, $J$ = 8.0 Hz, 1H), 7.22 (d, $J$ = 10.0 Hz, 1H), 4.17 (s, 3H); MS (ESI) m/z = 492.1 $[M+H]^+$.

**Examples 23 and 24: 1-[5-[3-[(1S)-2,2-difluorocyclopropyl]triazol-4-yl]-3-pyridyl]-N-[(1R)-1-[2-fluoro-3-(trifluoromethyl)phenyl]ethyl]-6-oxo-pyridazine-3-carboxamide and 1-[5-[3-[(1R)-2,2-difluorocyclopropyl]triazol-4-yl]-3-pyridyl]-N-[(1R)-1-[2-fluoro-3-(trifluoromethyl)phenyl]ethyl]-6-oxo-pyridazine-3-carboxamide**

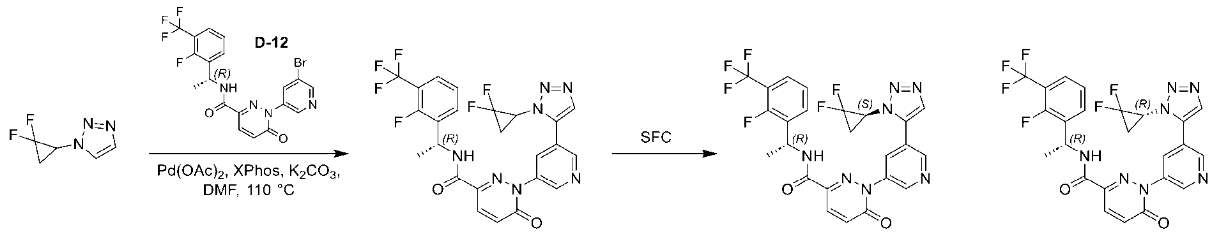


T-20 Example 23 Example 24

Step 1: Synthesis of 1-[5-[3-(2,2-difluorocyclopropyl)-triazol-4-yl]-3-pyridyl]-N-[(1R)-1-[2-fluoro-3-(trifluoromethyl)phenyl]ethyl]-6-oxo-pyridazine-3-carboxamide

A mixture of Intermediate D-12 (100 mg, 206.09 μmol) and Intermediate T-20 (89.72 mg, 618.26 μmol), $Pd(OAc)_2$ (9.25 mg, 41.22 μmol), $K_2CO_3$ (56.97 mg, 412.18 μmol) and XPhos (39.30 mg, 82.44 nmol) in DMF (4 mL) was degassed and purged with $N_2$ for 3 times, and then stirred at 110 °C for 16 h under $N_2$ atmosphere. The reaction mixture was filtered, and the filtrate was diluted with water (5 mL) and then extracted with EtOAc (15 mL × 3). The combined organic layers were washed with brine (5 mL × 3), dried over anhydrous $Na_2SO_4$ and concentrated under reduced pressure to give a crude product. The crude product was purified by silica gel column chromatography (2% MeOH in DCM) to give 1-[5-[3-(2,2-difluorocyclopropyl)-triazol-4-yl]-3-pyridyl]-N-[(1R)-1-[2-fluoro-3-(trifluoromethyl)phenyl]ethyl]-6-oxo-pyridazine-3-carboxamide (35 mg, 27.72% yield, 89.69% purity) as a yellow solid. MS (ESI) m/z = 550.0 $[M+H]^+$.

Step 2: Synthesis of 1-[5-[3-[(1S)-2,2-difluorocyclopropyl]triazol-4-yl]-3-pyridyl]-N-[(1R)-1-[2-fluoro-3-(trifluoromethyl)phenyl]ethyl]-6-oxo-pyridazine-3-carboxamide, 1-[5-[3-(1R)-2,2-difluorocyclopropylltriazol-4-yl]-3-pyridyl]-N-[(1R)-1-[2-fluoro-3-(trifluoromethyl)phenyl]ethyl]-6-oxo-pyridazine-3-carboxamide

1-[5-[3-(2,2-difluorocyclopropyl)-triazol-4-yl]-3-pyridyl]-N-[(1R)-1-[2-fluoro-3-(trifluoromethyl)phenyl]ethyl]-6-oxo-pyridazine-3-carboxamide (35 mg, 63.70 μmol) was further purified by SFC separation (column: DAICEL CHIRALCEL OD (250mm*30mm,10μm); mobile phase: [$CO_2$- EtOH (0.1% $NH_3H_2O$)]; B%: 40%, isocratic elution mode). Two peaks were confirmed by SFC analysis. The compound confirmed in the first peak (Rt = 0.838 min) was concentrated under reduced pressure, MeCN (5 mL) and $H_2O$ (10 mL) were added, and the solvent was removed by lyophilization to obtain **a compound of Example 23** (10.4 mg, 28.81% yield, 96.96% purity) as a light yellow solid. The compound confirmed in the second peak (Rt = 1.146 min) was concentrated under reduced pressure, MeCN (5 mL) and $H_2O$ (10 mL) were added, and the solvent was removed by lyophilization to give **a compound of Example 24** (14 mg, 39.80% yield, 99.50% purity) as a white solid.

**Compound of Example 23:** $^1H$ NMR (400 MHz, DMSO-d6) δ = 9.15 (d, *J* = 2.0 Hz, 1H), 9.08 (br d, *J* = 7.6 Hz, 1H), 9.00 (d, *J* = 1.6 Hz, 1H), 8.58 (s, 1H), 8.26 (s, 1H), 7.92 (d, *J* = 9.6 Hz, 1H), 7.82 (br t, *J* = 7.2 Hz, 1H), 7.68 (br t, *J* = 6.8 Hz, 1H), 7.39 (t, *J* = 7.6 Hz, 1H), 7.23 (d, *J* = 9.6 Hz, 1H), 5.42 (quin, *J* = 7.2 Hz, 1H), 5.15 - 5.06 (m, 1H), 2.66 - 2.56 (m, 1H), 2.47 - 2.39 (m, 1H), 1.51 (br d, *J* = 7.2 Hz, 3H); MS (ESI) m/z = 550.2 $[M+H]^+$.

**Compound of Example 24:** $^1H$ NMR (400 MHz, DMSO-d6) δ = 9.15 (d, *J* = 2.0 Hz, 1H), 9.08 (br d, *J* = 7.6 Hz, 1H), 8.99 (s, 1H), 8.58 (s, 1H), 8.26 (s, 1H), 7.92 (d, *J* = 9.6 Hz, 1H), 7.83 (br t, *J* = 7.2 Hz, 1H), 7.68 (br t, *J* = 6.8 Hz, 1H), 7.40 (t, *J* = 7.6 Hz, 1H), 7.23 (d, *J* = 9.6 Hz, 1H), 5.42 (br t, *J* = 7.2 Hz, 1H), 5.15 - 5.05 (m, 1H), 2.64 - 2.56 (m, 1H), 2.45 - 2.36 (m, 1H), 1.51 (br d, *J* = 7.2 Hz, 3H); MS (ESI) m/z = 550.2 $[M+H]^+$.

**Examples 25 and 26: 1-[5-[3-[(1S)-2,2-difluorocyclopropyl]triazol-4-yl]-3-pyridyl]-N-[(1R)-1-[3-(1,1-difluoro-2-hydroxy-ethyl)-2-fluoro-phenyl]ethyl]-6-oxo-pyridazine-3-carboxamide and 1-[5-[3-[(1R)-2,2-difluorocyclopropyl]triazol-4-yl]-3-pyridyl]-N-[(1R)-1-[3-(1,1-difluoro-2-hydroxy-ethyl)-2-fluoro-phenyl]ethyl]-6-oxo-pyridazine-3-carboxamide**

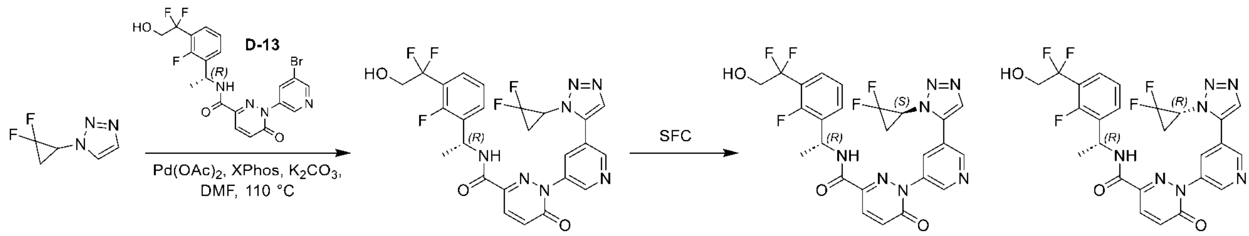


T-20 Example 25 Example 26

Step 1: Synthesis of 1-[5-[3-(2,2-difluorocyclopropyl)triazol-4-yl]-3-pyridyl]-N-[(1R)-1-[3-(1,1-difluoro-2-hydroxy-ethyl)-2-fluoro-phenyl]ethyl]-6-oxo-pyridazine-3-carboxamide

Intermediates T-20 and D-13 were used for synthesis in the same manner as in Step 1 of Example 33 to give 1-[5-[3-(2,2-difluorocyclopropyl)triazol-4-yl]-3-pyridyl]-N-[(1R)-1-[3-(1,1-difluoro-2-hydroxy-ethyl)-2-fluoro-phenyl]ethyl]-6-oxo-pyridazine-3-carboxamide (32 mg, 12.32% yield, 100% purity) as a white solid. MS (ESI) m/z = 562.2 [M+H]$^+$.

Step 2: Synthesis of 1-[5-[3-[(1S)-2,2-difluorocyclopropyl]triazol-4-yl]-3-pyridyl]-N-[(1R)-1-[3-(1,1-difluoro-2-hydroxy-ethyl)-2-fluoro-phenyl]ethyl]-6-oxo-pyridazine-3-carboxamide and 1-[5-[3-[(1R)-2,2-difluorocyclopropyl]triazol-4-yl]-3-pyridyl]-N-[(1R)-1-[3-(1,1-difluoro-2-hydroxy-ethyl)-2-fluoro-phenyl]ethyl]-6-oxo-pyridazine-3-carboxamide

1-[5-[3-(2,2-difluorocyclopropyl)triazol-4-yl]-3-pyridyl]-N-[(1R)-1-[3-(1,1-difluoro-2-hydroxy-ethyl)-2-fluoro-phenyl]ethyl]-6-oxo-pyridazine-3-carboxamide (32 mg, 56.99 μmol) was further purified by SFC separation (column: DAICEL CHIRALCEL OD (250mm*30mm,10μm); mobile phase: [$CO_2$- EtOH (0.1% $NH_3H_2O$)]; B%: 40%, isocratic elution mode). Two peaks were confirmed by SFC analysis. The compound confirmed in the first peak (Rt = 1.545 min) was concentrated under reduced pressure, MeCN (5 mL) and $H_2O$ (10 mL) were added, and the solvent was removed by lyophilization to obtain **a compound of Example 25** (9.9 mg, 30.94% yield, 100% purity) as a white solid. The compound confirmed in the second peak (Rt = 1.717 min) was concentrated under reduced pressure, MeCN (5 mL) and $H_2O$ (10 mL) were added, and the solvent was removed by lyophilization to obtain **a compound of Example 26** (11.2 mg, 35.00% yield, 100% purity) as a white solid. The absolute stereochemical configurations, S and R, of the difluorocyclopropyl triazole of the compounds of Examples 25 and 26 were arbitrarily indicated.

**Compound of Example 25:** $^1$H NMR (400 MHz, DMSO-d6) δ = 9.21 (d, *J* = 2.4 Hz, 1H), 9.08 (d, *J* = 8.0 Hz, 1H), 9.06 (d, *J* = 2.0 Hz, 1H), 8.65 (t, *J* = 2.0 Hz, 1H), 8.32 (s, 1H), 7.99 (d, *J* = 9.6 Hz, 1H), 7.70 (t, *J* = 7.2 Hz, 1H), 7.50 (t, *J* = 6.8 Hz, 1H), 7.34 (t, *J* = 7.6 Hz, 1H), 7.30 (d, *J* = 9.6 Hz, 1H), 5.77 (t, *J* = 6.4 Hz, 1H), 5.48 (t, *J* = 7.2 Hz, 1H), 5.21 - 5.10 (m, 1H), 3.98 (dt, *J* = 6.4, 14.4 Hz, 2H), 2.70 - 2.61 (m, 1H), 2.52 - 2.43 (m, 1H), 1.55 (d, *J* = 7.2 Hz, 3H); MS (ESI) m/z = 562.2 [M+H]$^+$.

**Compound of Example 26:** $^1$H NMR (400 MHz, DMSO-d6) δ = 9.15 (d, *J* = 2.4 Hz, 1H), 9.02 (d, *J* = 8.0 Hz, 1H), 8.99 (d, *J* = 2.0 Hz, 1H), 8.59 (t, *J* = 2.0 Hz, 1H), 8.26 (s, 1H), 7.93 (d, *J* = 10.0 Hz, 1H), 7.64 (t, *J* = 6.8 Hz, 1H), 7.44 (t, *J* = 6.8 Hz, 1H), 7.28 (t, *J* = 7.6 Hz, 1H), 7.23 (d, *J* = 9.6 Hz, 1H), 5.71 (t, *J* = 6.4 Hz, 1H), 5.42 (quin, *J* = 7.2 Hz, 1H), 5.16 - 5.05 (m, 1H), 3.91 (dt, *J* = 6.4, 14.4 Hz, 2H), 2.64 - 2.55 (m, 1H), 2.42 (dt, *J* = 4.8, 10.0 Hz, 1H), 1.49 (d, *J* = 7.2 Hz, 3H); MS (ESI) m/z = 562.2 [M+H]$^+$.

**[Experimental Example]**

**Experimental Example 1: SOS-catalyzed nucleotide exchange assay**

SOS1 activity was measured by SOS1-mediated GTP loading quantification of $KRAS^{G12D}$. GTP loaded $KRAS^{G12D}$ was detected by HTRF (Homogeneous Time Resolved Fluorescence) using anti-GST terbium (Cisbio, France) as a donor and a fluorescence GTP analogue (EDA-GTP-DY-647P1, [DY-647P1 labeled 2'/3'-O-(2-aminoethyl-carbamoyl)guanosine-5'-triphosphate (Jena bioscience, Germany)]) as an acceptor.

Assay buffer was composed of 10 mM HEPES pH 7.4, 150 mM NaCl, 5 mM $MgCl_2$, 1 mM DTT, 0.05% BSA Fraction V pH 7.0 (Sigma) and 0.0025% (v/v) Igepal (Sigma). $KRAS^{G12D}$ working solution was prepared by adding equal amounts of GST-$KRAS^{G12D}$ (100 nM) and anti-GST-terbium (2 nM; Cisbio, France) to the assay buffer and culturing on ice for 10 minutes. $SOS1_{cat}$ working solution was prepared by adding equal amounts of HIS-$SOS1_{cat}$ (160 nM; Cytoskeleton, US) and EDA-GTP-DY-647P1 (200 nM; Jena bioscience, Germany) to the assay buffer. Control solution was prepared by adding equal volumes of EDA-GTP-DY-647P1 and the assay buffer for normalization control to exclude GEF-independent GTP loading and background signals. In order to induce a reaction, 5 uL of the $SOS1_{cat}$ working solution or the control solution was injected into each well. 5 uL of a compound having various concentrations from 128 pM to 10 μM was added to wells containing the $SOS1_{cat}$ working solution and incubated at room temperature for 10 minutes to bind to SOS1.

10 uL of the $KRAS^{G12D}$ solution was added to each well and incubated for 30 minutes at room temperature while shaking. HTRF was measured using a Thermo Varioskan (excitation 334 nm, emission 665 nm). The $IC_{50}$ of each inhibitor was analyzed using Graphad Prism.

The SOS1 inhibitory activity of the Example compounds measured by the above method was evaluated based on the following criteria, and the results are shown in Table 6.

| $IC_{50}$ range | < 50 nM | 50 nM ≤ $IC_{50}$ < 150 nM | ≥ 150 nM |
|---|---|---|---|
| Class | A | B | C |
| NA = Not Applied | | | |

As shown in Table 6 below, the Example compounds herein exhibited excellent SOS1 inhibitory activity.

[Table 6]

| **Example No.** | **SOS-catalyzed nucleotide exchange assay** | **Example No.** | **SOS-catalyzed nucleotide exchange assay** |
|---|---|---|---|
| 1 | A | 1-68 | A |
| 1-1 | B | 1-69 | B |
| 1-2 | B | 1-70 | A |
| 1-3 | B | 1-71 | A |
| 1-4 | B | 1-72 | A |
| 1-5 | B | 2 | B |
| 1-6 | B | 2-1 | A |
| 1-7 | A | 2-2 | A |
| 1-8 | A | 2-3 | A |
| 1-9 | A | 2-4 | A |
| 1-10 | A | 2-5 | A |
| 1-11 | B | 2-6 | A |
| 1-12 | A | 2-7 | A |
| 1-13 | A | 3 | C |
| 1-14 | C | 3-1 | B |
| 1-15 | A | 3-2 | B |
| 1-16 | B | 3-3 | A |
| 1-17 | B | 3-4 | B |
| 1-18 | A | 3-5 | B |
| 1-19 | C | 4 | C |
| 1-20 | B | 5 | C |
| 1-21 | A | 6 | B |
| 1-22 | C | 6-1 | B |
| 1-23 | A | 6-2 | A |
| 1-24 | A | 6-3 | A |
| 1-25 | A | 6-4 | A |
| 1-26 | B | 6-5 | NA |
| 1-27 | B | 6-6 | NA |
| 1-28 | NA | 6-7 | A |

(continued)

| Example No. | SOS-catalyzed nucleotide exchange assay | Example No. | SOS-catalyzed nucleotide exchange assay |
|---|---|---|---|
| 1-29 | B | 6-8 | A |
| 1-30 | C | 6-9 | A |
| 1-31 | NA | 7 | A |
| 1-32 | NA | 7-1 | B |
| 1-33 | NA | 7-2 | B |
| 1-34 | B | 7-3 | B |
| 1-35 | B | 7-4 | A |
| 1-36 | A | 8 | A |
| 1-37 | B | 9 | A |
| 1-38 | B | 10 | B |
| 1-39 | A | 11 | A |
| 1-40 | A | 12 | B |
| 1-41 | A | 13 | B |
| 1-42 | A | 14 | A |
| 1-43 | A | 15 | A |
| 1-44 | NA | 16 | NA |
| 1-45 | NA | 17 | NA |
| 1-46 | B | 18 | NA |
| 1-47 | NA | 19 | A |
| 1-48 | C | 20 | A |
| 1-49 | B | 20-1 | A |
| 1-50 | A | 20-2 | A |
| 1-51 | A | 20-3 | A |
| 1-52 | A | 20-4 | A |
| 1-53 | A | 20-5 | B |
| 1-54 | A | 20-6 | A |
| 1-55 | A | 20-7 | A |
| 1-56 | A | 20-8 | A |
| 1-57 | A | 20-9 | A |
| 1-58 | A | 20-10 | A |
| 1-59 | A | 20-11 | A |
| 1-60 | A | 20-12 | A |
| 1-61 | B | 21 | A |
| 1-62 | B | 22 | A |
| 1-63 | B | 23 | B |
| 1-64 | C | 24 | B |
| 1-65 | C | 25 | A |
| 1-66 | B | 26 | A |

(continued)

| Example No. | SOS-catalyzed nucleotide exchange assay | Example No. | SOS-catalyzed nucleotide exchange assay |
|---|---|---|---|
| 1-67 | A | | |

**Experimental Example 2: KRAS $^{G12C}$::SOS1 protein-protein interaction (PPI) inhibition assay**

Protein-protein interaction between SOS1 and KRAS$^{G12c}$ was detected using KRAS G12C/SOS1 binding kit from CISBIO (64KRASG12PEH, CISBIO, FRANCE) according to the manufacturer's assay method. Briefly, the maximum concentration of an inhibitor sample was set to be 3 μM, and the inhibitor sample was diluted 7 times by 1/3, and samples having a total of 8 concentrations were prepared. In HTRF (Homogeneous Time-Resolved Fluorescence) exclusive 96-well low-volume white plate (66PL96005, CISBIO, FRANCE), 2 μL of an inhibitor sample prepared for each concentration, 5 μL of a mixed solution of Tag1 labeled KRAS$^{G12c}$ protein and GTP diluted with the dilution solution provided by the manufacturer, and 5 μL of Tag2 labeled SOS1 protein were mixed, and added with 8 μL of a diluted solution where anti-Tag1 KRAS$^{G12C}$ XL665 (HTRF acceptor) labeled antibody and anti-Tag2 SOS1 Terbium cryptate (HTRF donor) labeled antibody were mixed in an equal ratio and cultured at room temperature for 2 h. Thereafter, the HTRF signal generated by the antibody was measured using a Thermo Varioskan multi-purpose plate signal detector, and the result value was calculated as the ratio of the signals emitted at 665 nm and 620 nm as shown below.

$$\text{Result value} = 10^4 \text{ X } 665 \text{ nm signal}/620 \text{ nm signal}$$

The $IC_{50}$ value of each inhibitor was analyzed with the result values of three replicates by using the Graphad Prism 9.0 software.

The degree of inhibition of the Example compounds on SOS1 and KRAS$^{G12C}$ interaction measured by the above method was evaluated based on the following criteria, and the results are shown in Table 7.

| $IC_{50}$ range | < 50 nM | 50 nM ≤ $IC_{50}$ < 150 nM | ≥ 150 **nM** |
|---|---|---|---|
| Class | A | B | C |
| NA = Not Applied | | | |

[Table 7]

| Example No. | KRAS G12C::SOS1 PPI | Example No. | KRAS G12C::SOS1 PPI |
|---|---|---|---|
| 1 | A | 1-68 | A |
| 1-1 | A | 1-69 | A |
| 1-2 | B | 1-70 | NA |
| 1-3 | B | 1-71 | NA |
| 1-4 | A | 1-72 | NA |
| 1-5 | B | 2 | B |
| 1-6 | A | 2-1 | A |
| 1-7 | A | 2-2 | A |
| 1-8 | B | 2-3 | A |
| 1-9 | A | 2-4 | A |
| 1-10 | B | 2-5 | A |
| 1-11 | B | 2-6 | A |
| 1-12 | B | 2-7 | A |
| 1-13 | A | 3 | C |
| 1-14 | C | 3-1 | C |

(continued)

| Example No. | KRAS G12C::SOS1 PPI | Example No. | KRAS G12C::SOS1 PPI |
|---|---|---|---|
| 1-15 | A | 3-2 | B |
| 1-16 | B | 3-3 | A |
| 1-17 | C | 3-4 | B |
| 1-18 | A | 3-5 | B |
| 1-19 | C | 4 | C |
| 1-20 | C | 5 | C |
| 1-21 | A | 6 | B |
| 1-22 | B | 6-1 | C |
| 1-23 | A | 6-2 | B |
| 1-24 | A | 6-3 | A |
| 1-25 | B | 6-4 | B |
| 1-26 | B | 6-5 | B |
| 1-27 | B | 6-6 | B |
| 1-28 | C | 6-7 | A |
| 1-29 | A | 6-8 | A |
| 1-30 | C | 6-9 | A |
| 1-31 | B | 7 | A |
| 1-32 | C | 7-1 | B |
| 1-33 | C | 7-2 | A |
| 1-34 | A | 7-3 | A |
| 1-35 | B | 7-4 | A |
| 1-36 | B | 8 | A |
| 1-37 | B | 9 | B |
| 1-38 | A | 10 | B |
| 1-39 | A | 11 | B |
| 1-40 | A | 12 | C |
| 1-41 | A | 13 | C |
| 1-42 | A | 14 | B |
| 1-43 | A | 15 | A |
| 1-44 | C | 16 | C |
| 1-45 | B | 17 | C |
| 1-46 | B | 18 | C |
| 1-47 | C | 19 | A |
| 1-48 | C | 20 | A |
| 1-49 | A | 20-1 | B |
| 1-50 | A | 20-2 | A |
| 1-51 | B | 20-3 | A |
| 1-52 | B | 20-4 | B |
| 1-53 | A | 20-5 | B |

(continued)

| Example No. | KRAS G12C::SOS1 PPI | Example No. | KRAS G12C::SOS1 PPI |
|---|---|---|---|
| 1-54 | B | 20-6 | A |
| 1-55 | A | 20-7 | A |
| 1-56 | A | 20-8 | A |
| 1-57 | B | 20-9 | A |
| 1-58 | A | 20-10 | A |
| 1-59 | B | 20-11 | A |
| 1-60 | A | 20-12 | A |
| 1-61 | A | 21 | A |
| 1-62 | A | 22 | A |
| 1-63 | B | 23 | B |
| 1-64 | C | 24 | C |
| 1-65 | B | 25 | A |
| 1-66 | A | 26 | A |
| 1-67 | B | | |

**Experimental Example 3: Evaluation of cancer cell growth inhibition**

The lung cancer cell line NCI-H358 having the KRAS G12C variant was dispensed into 100 μl of cell culture solution (RPMI1640 with L-glutamine (300mg/L), 10% fetal bovine serum (FBS), 1% P/S) per well of a 96-well cell culture plate (U-shape clear bottom plate, SPL 34896) to contain approximately 2000 cells, centrifuged at 1300 RPM for 3 minutes, and then cultured for 72 h under conditions of 37 °C and 5% carbon dioxide. When three-dimensional cell spheroids were formed, the cells were treated with each compound to final concentrations of 0.002 μM 0.005 μM, 0.014 μM, 0.041 μM, 0.12 μM, 0.37 μM, 1.11 μM, 3.33 μM and 10 μM per well, and cultured under conditions of 37 °C and 5% carbon dioxide for 5 days. Thereafter, the plate with the cells cultured was adapted to room temperature for 10 minutes, and then 50 μL of CellTiter-Glo® 3D Cell Viability Assay (PROMEGA, G9683) solution was dispensed, and reacted on a horizontal stirrer at a speed of 300 RPM for 30 minutes to induce the cell lysis. 150 μL of each stirred solution was taken and transferred to a new white 96-well cell culture plate (Nunc™ MicroWell™ 96-Well, Nunclon Delta-Treated, Flat-Bottom Microplate, 136101), and the luminescence intensity was measured using a Varioskan™ LUX microplate reader. Thereafter, based on the measured values, the degree of cell growth in the well treated with each compound was calculated when the final cell density value of the well not treated with the test substance was set to 100%.

The cancer cell growth inhibition ability of the example compounds measured by the above method was evaluated based on the following criteria, and the results are shown in Table 8.

| $IC_{50}$ range | ≤ 1 μM | 1 μM < $IC_{50}$ ≤ 3 μM | > 3 μM |
|---|---|---|---|
| Class | A | B | C |
| NA = Not Applied | | | |

[Table 8]

| Example No. | H358 cell viability | Example No. | H358 cell viability |
|---|---|---|---|
| 1 | A | 1-68 | NA |
| 1-1 | A | 1-69 | NA |
| 1-2 | A | 1-70 | NA |
| 1-3 | NA | 1-71 | NA |
| 1-4 | C | 1-72 | NA |

(continued)

| Example No. | H358 cell viability | Example No. | H358 cell viability |
|---|---|---|---|
| 1-5 | A | 2 | A |
| 1-6 | C | 2-1 | A |
| 1-7 | A | 2-2 | NA |
| 1-8 | NA | 2-3 | A |
| 1-9 | A | 2-4 | A |
| 1-10 | NA | 2-5 | A |
| 1-11 | A | 2-6 | A |
| 1-12 | A | 2-7 | C |
| 1-13 | NA | 3 | NA |
| 1-14 | NA | 3-1 | NA |
| 1-15 | A | 3-2 | A |
| 1-16 | A | 3-3 | NA |
| 1-17 | A | 3-4 | NA |
| 1-18 | NA | 3-5 | NA |
| 1-19 | NA | 4 | C |
| 1-20 | C | 5 | NA |
| 1-21 | NA | 6 | A |
| 1-22 | A | 6-1 | NA |
| 1-23 | A | 6-2 | A |
| 1-24 | A | 6-3 | A |
| 1-25 | A | 6-4 | A |
| 1-26 | A | 6-5 | NA |
| 1-27 | NA | 6-6 | NA |
| 1-28 | NA | 6-7 | A |
| 1-29 | A | 6-8 | NA |
| 1-30 | NA | 6-9 | NA |
| 1-31 | NA | 7 | A |
| 1-32 | NA | 7-1 | A |
| 1-33 | C | 7-2 | A |
| 1-34 | NA | 7-3 | A |
| 1-35 | NA | 7-4 | A |
| 1-36 | NA | 8 | NA |
| 1-37 | C | 9 | NA |
| 1-38 | NA | 10 | NA |
| 1-39 | A | 11 | NA |
| 1-40 | A | 12 | NA |
| 1-41 | NA | 13 | NA |
| 1-42 | A | 14 | B |
| 1-43 | NA | 15 | A |

(continued)

| Example No. | H358 cell viability | Example No. | H358 cell viability |
|---|---|---|---|
| 1-44 | NA | 16 | NA |
| 1-45 | NA | 17 | NA |
| 1-46 | A | 18 | NA |
| 1-47 | NA | 19 | NA |
| 1-48 | NA | 20 | NA |
| 1-49 | NA | 20-1 | NA |
| 1-50 | A | 20-2 | NA |
| 1-51 | NA | 20-3 | NA |
| 1-52 | A | 20-4 | NA |
| 1-53 | A | 20-5 | NA |
| 1-54 | A | 20-6 | NA |
| 1-55 | NA | 20-7 | NA |
| 1-56 | NA | 20-8 | NA |
| 1-57 | NA | 20-9 | NA |
| 1-58 | A | 20-10 | NA |
| 1-59 | NA | 20-11 | NA |
| 1-60 | NA | 20-12 | NA |
| 1-61 | A | 21 | NA |
| 1-62 | A | 22 | A |
| 1-63 | A | 23 | NA |
| 1-64 | B | 24 | NA |
| 1-65 | NA | 25 | NA |
| 1-66 | NA | 26 | NA |
| 1-67 | A | | |

**Experimental Example 4: Evaluation of cancer cell growth inhibition according to combined administration**

When the example compound was used in combination with an anticancer agent, the effect on the growth ability of cancer cells expressing KRAS variant or EGFR variant was confirmed. In order to confirm the effect of the example compound in combination with a MEK inhibitor or a KRAS G12C inhibitor in the cell line having the KRAS variant, the cell growth inhibitory effect by the treatment of the example compound in combination with a MEK inhibitor or a KRAS G12C inhibitor was confirmed in the gastric cancer cell line SNU-1 and the pancreatic cancer cell line KP4 having the KRAS G12D variant, the lung cancer cell line NCI-H358 and the pancreatic cancer cell line MIA PaCa-2 having the KRAS G12C variant, the colorectal cancer cell line SW480 having the KRAS G12V variant, the lung cancer cell line A549 having the KRAS G12S variant, the colorectal cancer cell line LoVo having the KRAS G13D variant. In addition, in order to confirm the effect of the example compound in combination with an EGFR inhibitor in the cell line having the EGFR variant, the cell growth inhibitory effect by the treatment of the example compound in combination with an EGFR inhibitor was confirmed in the lung cancer cell line H1975 having the EGFR L858R/T790M variant and the lung cancer cell line HCC827 having the EGFR Del19 variant.

**Experimental Example 4.1: Cell dispensing well plate and test solution preparation**

The cell line stored in the liquid nitrogen tank was taken out, quickly thawed at 37 °C, then diluted with 10 times the volume of cell culture medium, and centrifuged to remove the frozen storage medium. The recovered cell pellet was mixed well with the culture medium, placed in a culture flask, and cultured under conditions of 37 °C and 5% carbon dioxide to

stabilize the cells. Each cell was cultured in RPMI 1640 Medium (1x) or Dulbecco Modified Eagle Medium (DMEM, 1x) medium containing 10% (v/v) FBS and 1% (v/v) penicillin/streptomycin. Thereafter, the cells were obtained from the flask and centrifuged to remove the culture medium, and washed with PBS (Phosphate Buffered Saline), and then centrifuged again to remove the PBS. The cells were diluted with culture medium to 1.25 x $10^4$ cells/mL and dispensed at 40 μL per well in a 384-well plate. In addition, the cells were diluted with medium to 5 x $10^4$ cells/mL and dispensed at 100 μL per well in a 96-well plate. Thereafter, the plate onto which the cells were dispensed was cultured for 24 h under conditions of 37 °C and 5% carbon dioxide.

Example 1-2, Example 1-1, Example 2-5, Example 2-4, Example 2-7, Example 1-24, Example 1-8, Example 1-21, Example 1-5, Example 20, Example 21, Example 1-51, Example 6-3, Example 9, Example 1-69 and Example 1-68, and a MEK inhibitor, a KRAS G12C inhibitor and an EGFR inhibitor (INK-128, sotorasib, adagrasib, trametinib, osimertinib, lazertinib, etc.) were each dissolved in 99.5% (v/v) dimethyl sulfoxide (hereinafter, DMSO) to a concentration of 10 mM.

**Experimental Example 4.2: Evaluation of cell viability of lung cancer cell NCI-H358 according to combined administration with sotorasib**

A well plate in which the lung cancer cell line NCI-H358 having the KRAS G12C variant was dispensed was treated with a test solution of sotorasib and the example compounds.

The cells were treated by adding 10 μL of a test solution of sotorasib, Example 1-2, Example 1-1 and Example 2-5, respectively, alone or sotorasib in combination with one of Example 1-2, Example 1-1 and Example 2-5 (Combi.) to a 384-well plate so that the final concentration of each substance was 100 nM to 97.7 pM in 50 μL of the solution per well. In a 96-well plate, the cells were treated by diluting each substance in medium at 3 times the treatment concentration and adding 50 μL per well so that the final concentration of each substance was 120 nM to 2 nM in 150 μL of a solution.

The cells treated with the test solution were evaluated in the same manner as in Experimental Example 4.1 to obtain the graph in Figure 1.

Referring to Figure 1, it was confirmed that the cell viability of the lung cancer cell NCI-H358 was decreased when treated with sotorasib and the example compound in combination compared to when treated with sotorasib and the example compound, respectively, alone.

**Experimental Example 4.3: Evaluation of cell viability of pancreatic cancer cell MIA PaCa-2 according to combined administration with sotorasib**

A well plate in which the pancreatic cancer cell line MIA PaCa-2 having the KRAS G12C variant was dispensed was treated with a test solution of sotorasib and the example compounds.

The cells were treated by adding 10 μL of a test solution of sotorasib, Example 1-2, Example 1-1 and Example 2-5, respectively, alone or sotorasib in combination with one of Example 1-2, Example 1-1 and Example 2-5 (Combi.) to a 384-well plate so that the final concentration of each substance was 100 nM to 97.7 pM in 50 μL of the solution per well. In a 96-well plate, the cells were treated by diluting each substance in medium at 3 times the treatment concentration and adding 50 μL per well so that the final concentration of each substance was 120 nM to 2 nM in 150 μL of a solution.

The cells treated with the test solution were evaluated in the same manner as in Experimental Example 4.1 to obtain the graph in Figure 2.

Referring to Figure 2, it was confirmed that the cell viability of the pancreatic cancer cell MIA PaCa-2 was decreased when treated with sotorasib and the example compound in combination compared to when treated with sotorasib and the example compound, respectively, alone.

**Experimental Example 4.4: Evaluation of cell viability of lung cancer cell NCI-H358 according to combined administration with adagrasib**

A well plate in which the lung cancer cell line NCI-H358 having the KRAS G12C variant was dispensed was treated with a test solution of adagrasib and the example compounds.

The cells were treated by adding 10 μL of a test solution of adagrasib, Example 1-2, Example 1-1 and Example 2-5, respectively, alone or adagrasib in combination with one of Example 1-2, Example 1-1 and Example 2-5 (Combi.) to a 384-well plate so that the final concentration of each substance was 100 nM to 97.7 pM in 50 μL of the solution per well. In a 96-well plate, the cells were treated by diluting each substance in medium at 3 times the treatment concentration and adding 50 μL per well so that the final concentration of each substance was 120 nM to 2 nM in 150 μL of a solution.

The cells treated with the test solution were evaluated in the same manner as in Experimental Example 4.1 to obtain the graph in Figure 3.

Referring to Figure 3, it was confirmed that the cell viability of the lung cancer cell NCI-H358 was decreased when treated with adagrasib and the example compound in combination compared to when treated with adagrasib and the

example compound, respectively, alone.

**Experimental Example 4.5: Evaluation of cell viability of pancreatic cancer cell MIA PaCa-2 according to combined administration with adagrasib**

A well plate in which the pancreatic cancer cell line MIA PaCa-2 having the KRAS G12C variant was dispensed was treated with a test solution of adagrasib and the example compounds.
The cells were treated by adding 10 μL of a test solution of adagrasib, Example 1-2, Example 1-1 and Example 2-5, respectively, alone or adagrasib in combination with one of Example 1-2, Example 1-1 and Example 2-5 (Combi.) to a 384-well plate so that the final concentration of each substance was 100 nM to 97.7 pM in 50 μL of the solution per well. In a 96-well plate, the cells were treated by diluting each substance in medium at 3 times the treatment concentration and adding 50 μL per well so that the final concentration of each substance was 120 nM to 2 nM in 150 μL of a solution.
The cells treated with the test solution were evaluated in the same manner as in Experimental Example 4.1 to obtain the graph in Figure 4.
Referring to Figure 4, it was confirmed that the cell viability of the pancreatic cancer cell MIA PaCa-2 was decreased when treated with adagrasib and the example compound in combination compared to when treated with adagrasib and the example compound, respectively, alone.

**Experimental Example 4.6: Evaluation of cell viability of lung cancer cell NCI-H358 according to combined administration with trametinib**

A well plate in which the lung cancer cell line NCI-H358 having the KRAS G12C variant was dispensed was treated with a test solution of trametinib and the example compounds.
The cells were treated by adding 10 μL of a test solution of trametinib, Example 1-2, Example 1-1, Example 2-5, Example 2-4 and Example 2-7, respectively, alone or trametinib in combination with one of Example 1-2, Example 1-1, Example 2-5, Example 2-4 and Example 2-7 (Combi.) to a 384-well plate so that the final concentration of each substance was 100 nM to 97.7 pM in 50 μL of the solution per well. In a 96-well plate, the cells were treated by diluting each substance in medium at 3 times the treatment concentration and adding 50 μL per well so that the final concentration of each substance was 120 nM to 2 nM in 150 μL of a solution.
The cells treated with the test solution were evaluated in the same manner as in Experimental Example 4.1 to obtain the graphs in Figures 5a and 5b.
Referring to Figures 5a and 5b, it was confirmed that the cell viability of the lung cancer cell NCI-H358 was decreased when treated with trametinib and the example compound in combination compared to when treated with trametinib and the example compound, respectively, alone.

**Experimental Example 4.7: Evaluation of cell viability of gastric cancer cell SNU-1 according to combined administration with trametinib**

A well plate in which the gastric cancer cell line SNU-1 having the KRAS G12D variant was dispensed was treated with a test solution of trametinib and the example compounds.
The cells were treated by adding 10 μL of a test solution of trametinib, Example 1-2, Example 1-1, Example 2-5, Example 2-4 and Example 2-7, respectively, alone or trametinib in combination with one of Example 1-2, Example 1-1, Example 2-5, Example 2-4 and Example 2-7 (Combi.) to a 384-well plate so that the final concentration of each substance was 100 nM to 97.7 pM in 50 μL of the solution per well. In a 96-well plate, the cells were treated by diluting each substance in medium at 3 times the treatment concentration and adding 50 μL per well so that the final concentration of each substance was 120 nM to 2 nM in 150 μL of a solution.
The cells treated with the test solution were evaluated in the same manner as in Experimental Example 4.1 to obtain the graphs in Figures 6a and 6b.
Referring to Figures 6a and 6b, it was confirmed that the cell viability of the gastric cancer cell SNU-1 was decreased when treated with trametinib and the example compound in combination compared to when treated with trametinib and the example compound, respectively, alone.

**Experimental Example 4.8: Evaluation of cell viability of colorectal cancer cell SW480 according to combined administration with trametinib**

A well plate in which the colorectal cancer cell line SW480 having the KRAS G12V variant was dispensed was treated with a test solution of trametinib and the example compounds.
The cells were treated by adding 10 μL of a test solution of trametinib, Example 1-2, Example 1-1, Example 2-5,

Example 2-4 and Example 2-7, respectively, alone or trametinib in combination with one of Example 1-2, Example 1-1, Example 2-5, Example 2-4 and Example 2-7 (Combi.) to a 384-well plate so that the final concentration of each substance was 100 nM to 97.7 pM in 50 μL of the solution per well. In a 96-well plate, the cells were treated by diluting each substance in medium at 3 times the treatment concentration and adding 50 μL per well so that the final concentration of each substance was 120 nM to 2 nM in 150 μL of a solution.

The cells treated with the test solution were evaluated in the same manner as in Experimental Example 4.1 to obtain the graphs in Figures 7a and 7b.

Referring to Figures 7a and 7b, it was confirmed that the cell viability of the colorectal cancer cell SW480 was decreased when treated with trametinib and the example compound in combination compared to when treated with trametinib and the example compound, respectively, alone.

### Experimental Example 4.9: Evaluation of cell viability of lung cancer cell H1975 according to combined administration with osimertinib

A well plate in which the lung cancer cell line H1975 having the EGFR L858R/T790M variant was dispensed was treated with a test solution of osimertinib and the example compounds.

The cells were treated by adding 10 μL of a test solution of osimertinib, Example 1-2, Example 1-1 and Example 2-5, respectively, alone or osimertinib in combination with one of Example 1-2, Example 1-1 and Example 2-5 (Combi.) to a 384-well plate so that the final concentration of each substance was 100 nM to 97.7 pM in 50 μL of the solution per well. In a 96-well plate, the cells were treated by diluting each substance in medium at 3 times the treatment concentration and adding 50 μL per well so that the final concentration of each substance was 120 nM to 2 nM in 150 μL of a solution.

The cells treated with the test solution were evaluated in the same manner as in Experimental Example 4.1 to obtain the graph in Figure 8.

Referring to Figure 8, it was confirmed that the cell viability of the lung cancer cell H1975 was decreased when treated with osimertinib and the example compound in combination compared to when treated with osimertinib and the example compound, respectively, alone.

### Experimental Example 4.10: Evaluation of cell viability of lung cancer cell HCC827 according to combined administration with osimertinib

A well plate in which the lung cancer cell line HCC827 having the EGFR Del19 variant was dispensed was treated with a test solution of osimertinib and the example compounds.

The cells were treated by adding 10 μL of a test solution of osimertinib, Example 1-2, Example 1-1 and Example 2-5, respectively, alone or osimertinib in combination with one of Example 1-2, Example 1-1 and Example 2-5 (Combi.) to a 384-well plate so that the final concentration of each substance was 100 nM to 97.7 pM in 50 μL of the solution per well. In a 96-well plate, the cells were treated by diluting each substance in medium at 3 times the treatment concentration and adding 50 μL per well so that the final concentration of each substance was 120 nM to 2 nM in 150 μL of a solution.

The cells treated with the test solution were evaluated in the same manner as in Experimental Example 4.1 to obtain the graph in Figure 9.

Referring to Figure 9, it was confirmed that the cell viability of the lung cancer cell HCC827 was decreased when treated with osimertinib and the example compound in combination compared to when treated with osimertinib and the example compound, respectively, alone.

### Experimental Example 4.11: Evaluation of cell viability of lung cancer cell H1975 according to combined administration with lazertinib

A well plate in which the lung cancer cell line H1975 having the EGFR L858R/T790M variant was dispensed was treated with a test solution of lazertinib and the example compounds.

The cells were treated by adding 10 μL of a test solution of lazertinib, Example 1-2, Example 1-1 and Example 2-5, respectively, alone or lazertinib in combination with one of Example 1-2, Example 1-1 and Example 2-5 (Combi.) to a 384-well plate so that the final concentration of each substance was 100 nM to 97.7 pM in 50 μL of the solution per well. In a 96-well plate, the cells were treated by diluting each substance in medium at 3 times the treatment concentration and adding 50 μL per well so that the final concentration of each substance was 120 nM to 2 nM in 150 μL of a solution.

The cells treated with the test solution were evaluated in the same manner as in Experimental Example 4.1 to obtain the graph in Figure 10.

Referring to Figure 10, it was confirmed that the cell viability of the lung cancer cell H1975 was decreased when treated with lazertinib and the example compound in combination compared to when treated with lazertinib and the example compound, respectively, alone.

**Experimental Example 4.12: Evaluation of cell viability of lung cancer cell HCC827 according to combined administration with lazertinib**

A well plate in which the lung cancer cell line HCC827 having the EGFR Del19 variant was dispensed was treated with a test solution of lazertinib and the example compounds.

The cells were treated by adding 10 μL of a test solution of lazertinib, Example 1-2, Example 1-1 and Example 2-5, respectively, alone or lazertinib in combination with one of Example 1-2, Example 1-1 and Example 2-5 (Combi.) to a 384-well plate so that the final concentration of each substance was 100 nM to 97.7 pM in 50 μL of the solution per well. In a 96-well plate, the cells were treated by diluting each substance in medium at 3 times the treatment concentration and adding 50 μL per well so that the final concentration of each substance was 120 nM to 2 nM in 150 μL of a solution.

The cells treated with the test solution were evaluated in the same manner as in Experimental Example 4.1 to obtain the graph in Figure 11.

Referring to Figure 11, it was confirmed that the cell viability of the lung cancer cell HCC827 was decreased when treated with lazertinib and the example compound in combination compared to when treated with lazertinib and the example compound, respectively, alone.

**Experimental Example 4.13: Evaluation of cell viability of breast cancer cell MCF7 according to combined administration with alpelisib**

A well plate in which PIK3CA mutant breast cancer cell line MCF7 was dispensed was treated with a test solution of alpelisib and the example compounds.

The cells were treated by adding 10 μL of a test solution of alpelisib, Example 1-2, Example 2-5, Example 1-24, Example 1-8 and Example 1-21, respectively, alone or alpelisib in combination with one of Example 1-2, Example 2-5, Example 1-24, Example 1-8 and Example 1-21 (Combi.) to a 384-well plate so that the final concentration of each substance was 100 nM to 97.7 pM in 50 μL of the solution per well.

The cells treated with the test solution were cultured under conditions of 37 °C and 5% carbon dioxide for 72 h. Thereafter, the plate with the cells cultured was adapted to room temperature for 30 minutes. Then, in order to quantify cytotoxicity, 50 μL of Cell Titer-Glo Reagent (CTG, Promega), which measures the amount of ATP, was dispensed per well into a 384-well plate. Cell lysis was induced by mixing the contents for 2 minutes, the luminescence signal was stabilized for 10 minutes, and then the luminescence intensity was measured using a Varioskan™ LUX microplate reader. The cell viability in the well treated with each compound was calculated when the final value of the well not treated with the test substance was set to 100%.

Referring to Figure 12, it was confirmed that the cell viability of the breast cancer cell MCF7 was decreased when treated with alpelisib and the example compound in combination compared to when treated with alpelisib and the example compound, respectively, alone.

**Experimental Example 4.14: Evaluation of cell viability of lung cancer cell H358 according to combined administration with JDQ443**

A well plate in which the lung cancer cell line H358 having the KRAS G12C variant was dispensed was treated with a test solution of JDQ443 and the example compounds.

The cells were treated by adding 10 μL of a test solution of JDQ443 and Example 1-2, respectively, alone or JDQ443 in combination with Example 1-2 (Combi.) to a 384-well plate so that the final concentration of JDQ443 was 100 nM to 6.1 pM and the final concentration of Example 1-2 was 30 μM to 13.7 nM in 50 μL of the solution per well.

The cells treated with the test solution were evaluated in the same manner as in Experimental Example 4.13 to obtain the graph in Figure 13.

Referring to Figure 13, it was confirmed that the cell viability of the lung cancer cell H358 was decreased when treated with JDQ443 and the example compound in combination compared to when treated with JDQ443 and the example compound, respectively, alone.

**Experimental Example 4.15: Evaluation of cell viability of lung cancer cell H358 according to combined administration with TN0155**

A well plate in which the lung cancer cell line H358 having the KRAS G12C variant was dispensed was treated with a test solution of TNO155 and the example compounds.

The cells were treated by adding 10 μL of a test solution of TNO155, Example 1-2 and Example 1-5, respectively, alone or TNO155 in combination with one of Example 1-2 and Example 1-5 (Combi.) to a 384-well plate so that the final concentration of each substance was 10 μM to 41.1 nM in 50 μL of the solution per well.

The cells treated with the test solution were evaluated in the same manner as in Experimental Example 4.13 to obtain the graph in Figure 14.

Referring to Figure 14, it was confirmed that the cell viability of the lung cancer cell H358 was decreased when treated with TNO155 and the example compound in combination compared to when treated with TNO155 and the example compound, respectively, alone.

**Experimental Example 4.16: Evaluation of cell viability of lung cancer cell H358 according to combined administration with cisplatin**

A well plate in which the lung cancer cell line H358 having the KRAS G12C variant was dispensed was treated with a test solution of cisplatin and the example compounds.

The cells were treated by adding 10 μL of a test solution of cisplatin, Example 1-2 and Example 1-5, respectively, alone or cisplatin in combination with one of Example 1-2 and Example 1-5 (Combi.) to a 384-well plate so that the final concentration of each substance was 5 μM to 156 nM in 50 μL of the solution per well.

The cells treated with the test solution were evaluated in the same manner as in Experimental Example 4.13 to obtain the graph in Figure 15.

Referring to Figure 15, it was confirmed that the cell viability of the lung cancer cell H358 was decreased when treated with cisplatin and the example compound in combination compared to when treated with cisplatin and the example compound, respectively, alone.

**Experimental Example 4.17: Evaluation of cell viability of lung cancer cell H358 according to combined administration with rineterkib**

A well plate in which the lung cancer cell line H358 having the KRAS G12C variant was dispensed was treated with a test solution of rineterkib and the example compounds.

The cells were treated by adding 10 μL of a test solution of rineterkib, Example 1-2 and Example 1-5, respectively, alone or rineterkib in combination with one of Example 1-2 and Example 1-5 (Combi.) to a 384-well plate so that the final concentration of each substance was 1 μM to 31.3 nM in 50 μL of the solution per well.

The cells treated with the test solution were evaluated in the same manner as in Experimental Example 4.13 to obtain the graph in Figure 16.

Referring to Figure 16, it was confirmed that the cell viability of the lung cancer cell H358 was decreased when treated with rineterkib and the example compound in combination compared to when treated with rineterkib and the example compound, respectively, alone.

**Experimental Example 4.18: Evaluation of cell viability of lung cancer cell H358 according to combined administration with ulixertinib**

A well plate in which the lung cancer cell line H358 having the KRAS G12C variant was dispensed was treated with a test solution of ulixertinib and the example compounds.

The cells were treated by adding 10 μL of a test solution of ulixertinib, Example 1-2 and Example 1-5, respectively, alone or ulixertinib in combination with one of Example 1-2 and Example 1-5 (Combi.) to a 384-well plate so that the final concentration of each substance was 1 μM to 31.3 nM in 50 μL of the solution per well.

The cells treated with the test solution were evaluated in the same manner as in Experimental Example 4.13 to obtain the graph in Figure 17.

Referring to Figure 17, it was confirmed that the cell viability of the lung cancer cell H358 was decreased when treated with ulixertinib and the example compound in combination compared to when treated with ulixertinib and the example compound, respectively, alone.

**Experimental Example 4.19: Evaluation of cell viability of lung cancer cell H358 according to combined administration with pralsetinib**

A well plate in which the lung cancer cell line H358 having the KRAS G12C variant was dispensed was treated with a test solution of pralsetinib and the example compounds.

The cells were treated by adding 10 μL of a test solution of pralsetinib, Example 1-2 and Example 1-5, respectively, alone or pralsetinib in combination with one of Example 1-2 and Example 1-5 (Combi.) to a 384-well plate so that the final concentration of each substance was 1 μM to 31.3 nM in 50 μL of the solution per well.

The cells treated with the test solution were evaluated in the same manner as in Experimental Example 4.13 to obtain the graph in Figure 18.

Referring to Figure 18, it was confirmed that the cell viability of the lung cancer cell H358 was decreased when treated with pralsetinib and the example compound in combination compared to when treated with pralsetinib and the example compound, respectively, alone.

**Experimental Example 4.20: Evaluation of cell viability of lung cancer cell H358 according to combined administration with repotrectinib**

A well plate in which the lung cancer cell line H358 having the KRAS G12C variant was dispensed was treated with a test solution of repotrectinib and the example compounds.
The cells were treated by adding 10 μL of a test solution of repotrectinib, Example 1-2 and Example 1-5, respectively, alone or repotrectinib in combination with one of Example 1-2 and Example 1-5 (Combi.) to a 384-well plate so that the final concentration of each substance was 1 μM to 31.3 nM in 50 μL of the solution per well.
The cells treated with the test solution were evaluated in the same manner as in Experimental Example 4.13 to obtain the graph in Figure 19.
Referring to Figure 19, it was confirmed that the cell viability of the lung cancer cell H358 was decreased when treated with repotrectinib and the example compound in combination compared to when treated with repotrectinib and the example compound, respectively, alone.

**Experimental Example 4.21: Evaluation of cell viability of gastric cancer cell SNU-5 according to combined administration with tepotinib**

A well plate in which the c-Met overexpressing gastric cancer cell line SNU-5 was dispensed was treated with a test solution of tepotinib and the example compounds.
The cells were treated by adding 10 μL of a test solution of tepotinib and Example 1-2, respectively, alone or tepotinib in combination with Example 1-2 (Combi.) to a 384-well plate so that the final concentration of each substance was 10 μM to 9.77 nM in 50 μL of the solution per well.
The cells treated with the test solution were evaluated in the same manner as in Experimental Example 4.13 to obtain the graph in Figure 20.
Referring to Figure 20, it was confirmed that the cell viability of the gastric cancer cell SNU-5 was decreased when treated with tepotinib and the example compound in combination compared to when treated with tepotinib and the example compound, respectively, alone.

**Experimental Example 4.22: Evaluation of cell viability of lung cancer cell PC-9 according to combined administration with bemcentinib**

A well plate in which the lung cancer cell line PC-9 with high AXL expression was dispensed was treated with a test solution of bemcentinib and the example compounds.
The cells were treated by adding 10 μL of a test solution of bemcentinib and Example 1-2, respectively, alone or bemcentinib in combination with Example 1-2 (Combi.) to a 384-well plate so that the final concentration of each substance was 10 μM to 9.77 nM in 50 μL of the solution per well.
The cells treated with the test solution were evaluated in the same manner as in Experimental Example 4.13 to obtain the graph in Figure 21.
Referring to Figure 21, it was confirmed that the cell viability of the lung cancer cell PC-9 was decreased when treated with bemcentinib and the example compound in combination compared to when treated with bemcentinib and the example compound, respectively, alone.

**Experimental Example 4.23: Evaluation of cell viability of lung cancer cell A549 according to combined administration with alrizomadlin**

A well plate in which the lung cancer cell line A549 having the KRAS G12S variant was dispensed was treated with a test solution of alrizomadlin and the example compounds.
The cells were treated by adding 10 μL of a test solution of alrizomadlin and Example 1-2, respectively, alone or alrizomadlin in combination with Example 1-2 (Combi.) to a 384-well plate so that the final concentration of each substance was 10 μM to 9.77 nM in 50 μL of the solution per well.
The cells treated with the test solution were evaluated in the same manner as in Experimental Example 4.13 to obtain the graph in Figure 22.
Referring to Figure 22, it was confirmed that the cell viability of the lung cancer cell A549 was decreased when treated with alrizomadlin and the example compound in combination compared to when treated with alrizomadlin and the

example compound, respectively, alone.

### Experimental Example 4.24: Evaluation of cell viability of colorectal cancer cell LoVo according to combined administration with everolimus

A well plate in which the colorectal cancer cell line LoVo having the KRAS G13D variant was dispensed was treated with a test solution of everolimus and the example compounds.

The cells were treated by adding 10 μL of a test solution of everolimus and Example 1-2, respectively, alone or everolimus in combination with Example 1-2 (Combi.) to a 384-well plate so that the final concentration of each substance was 10 μM to 9.77 nM in 50 μL of the solution per well.

The cells treated with the test solution were evaluated in the same manner as in Experimental Example 4.13 to obtain the graph in Figure 23.

Referring to Figure 23, it was confirmed that the cell viability of the colorectal cancer cell LoVo was decreased when treated with everolimus and the example compound in combination compared to when treated with everolimus and the example compound, respectively, alone.

### Experimental Example 4.25: Evaluation of cell viability of lung cancer cell AsPC-1 according to combined administration with MRTX1133

A well plate in which the lung cancer cell line AsPC-1 having the KRAS G12D variant was dispensed was treated with a test solution of MRTX1133 and the example compounds.

The cells were treated by adding 50 μL of a test solution of MRTX1133, Example 1-2 and Example 1-5, respectively, alone or MRTX1133 in combination with one of Example 1-2 and Example 1-5 (Combi.) to a 96-well plate so that the final concentration of MRTX1133 was 100 nM to 20 nM and the final concentration of the example compound was 5 μM in 150 μL of the solution per well.

The cells treated with the test solution were evaluated and the cell viability of a well treated with test solution alone or in combination was evaluated in the same manner as in Experimental Example 4.1 to obtain the graph in Figure 24.

Referring to Figure 24, it was confirmed that the cell viability of the lung cancer cell AsPC-1 was decreased when treated with MRTX1133 and the example compound in combination compared to when treated with MRTX1133 and the example compound, respectively, alone.

### Experimental Example 5.1: Determination of combination concentration by SynergyScreen™

The cells were treated by adding 5 μL of a test solution of Example 1-2 per well to a 384 well plate in which the lung cancer cell line H358 having the KRAS G12S variant was dispensed so that the final concentration of the substance was 31.6 μM to 0.00316 μM in 50 μL of the solution per well. The cells treated with Example 1-2 were cultured under conditions of 37 °C and 5% carbon dioxide for 72 h or 120 h. In order to measure cell viability, each well was treated with 24 μL of ATPlite 1Step™ (PerkinElmer) solution, then mixed for 2 minutes, and reacted by blocking light for 5 minutes, and then the luminescence signal was measured using an Envision multilabel reader (PerkinElmer). The cell viability was determined according to the formula below, and the concentration ($EFFECT_{20}$) of Example 1-2 that induces 20% cell death was calculated using IDBS XLfit 5. The $EFFECT_{20}$ concentration of Example 1-2 determined through the experiment was applied as the final treatment concentration of Example 1-2 during the combination effect experiment.

% growth= 100% × (luminescence treated wells, t=end / luminescence of untreated wells, t=end).

### Experimental Example 5.2: Evaluation of combination efficacy by SynergyScreen™

In order to confirm the effect of Example 1-2 in combination with various anticancer agents, a total of 42 anticancer agents, including cytotoxic anticancer agents and targeted anticancer agents, were used for evaluation (Table 9). A 384 well plate in which the lung cancer cell line H358 having the KRAS G12S variant was dispensed was treated with Example 1-2 at a final concentration of 1854 nM when the culture time was 72 h and was treated with Example 1-2 at a final concentration of 652 nM when the culture time was 120 h, respectively. After treatment of Example 1-2, the plate was treated with the combined anticancer agent so that the final concentration of each substance was 31.6 μM to 0.00316 μM in 50 μL of the solution per well. The cells treated with the test solution were cultured under conditions of 37 °C and 5% carbon dioxide for the culture time indicated in Table 9. In order to measure cell viability, each well was treated with 24 μL of ATPlite 1Step™ (PerkinElmer) solution, then mixed for 2 minutes, and reacted by blocking light for 5 minutes, and then the luminescence signal was measured using an Envision multilabel reader (PerkinElmer). The cell viability was determined in the same manner as in Experimental Example 4.1, and the effect of the combination treatment was evaluated by

comparing the cell viability of the group treated with the combined anticancer agent or Example 1-2 alone with the cell viability of the combined treatment.

Synergy 1 (Equation 1) was determined from the decrease in cell viability (%) (increase in cell death) when used in combination with Example 1-2 compared to the cell viability (%) when the combined anticancer agent was used alone, and Synergy 2 (Equation 2) was determined to be the difference between Synergy 1 and cell death rate (%) when Example 1-2 was used alone.

Synergy 1 (%) = cell viability (%) when the combined anticancer agent is used alone - cell viability (%) when used in combination with the example compound [Equation 1]

Synergy 2 (%) = Synergy 1 (%) - cell death rate (%) when the example compound is used alone [Equation 2]

If the synergy by treatment with Example 1-2 in combination compared to the combined anticancer agent alone is 50% or more, it is indicated as "++++", if it is less than 50% and more than 30%, it is indicated as "+++", if it is less than 30% and more than 10%, it is indicated as "++", and if it is less than 10%, it is indicated as "+". The results are shown in Figures 25a to 26c.

[Table 9]

| No | Anticancer agent | Target/ Mechanism | Culture time (h) | Concentration (log M) | | Synergy 1 | Synergy 2 |
|---|---|---|---|---|---|---|---|
| | | | | Anticancer agent | Ex. 1-2 | | |
| 1 | Paclitaxel | Taxane derivatives | 72 | -9.0 | -5.9 | ++ | ++ |
| 2 | Cytarabine | Antimetabolite | 72 | -8.5 | -5.9 | ++ | - |
| 3 | Palbociclib | CDK4/6 inhibition | 72 | -8.5 | -5.9 | ++ | - |
| 4 | Nintedanib | Inhibition of angiogenesis or FGFR1/FGFR 2/FGFR3 inhibition | 72 | -6.0 | -5.9 | ++ | ++ |
| 5 | Regorafenib | Inhibition of angiogenesis | 72 | -8.5 | -5.9 | ++ | + |
| 6 | SN-38 | TOP1 inhibition | 72 | -10.5 | -5.9 | ++ | - |
| 7 | Doxorubicin | TOP2 inhibition | 72 | -9.0 | -5.9 | ++++ | ++ |
| 8 | Niraparib | PARP inhibition | 72 | -8.5 | -5.9 | ++ | - |
| 9 | JQ1 | BET inhibition | 120 | -8.5 | -6.3 | ++ | - |
| 10 | NVP-ADW742 | IGF 1 /2 or IGF1-R inhibition | 72 | -8.5 | -5.9 | ++ | - |
| 11 | duvelisib | PIK inhibition | 72 | -7.0 | -5.9 | ++ | + |
| 12 | Gefitinib | EGFR inhibition | 72 | -7.0 | -5.9 | +++ | ++ |
| 13 | Irbinitinib (tucatinib) | ErbB2(HER2) inhibition | 72 | -6.5 | -5.9 | ++ | ++ |
| 14 | Neratinib | HER2, EGFR inhibition | 72 | -9.0 | -5.9 | +++ | ++ |
| 15 | Alectinib | ALK inhibition | 72 | -6.0 | -5.9 | ++ | - |
| 16 | Cobimetinib | MEK inhibition | 72 | -8.0 | -5.9 | +++ | ++ |
| 17 | Imatinib | BCR-ABL inhibition | 72 | -8.5 | -5.9 | ++ | + |
| 18 | Dasatinib | | 72 | -8.0 | -5.9 | +++ | ++ |
| 19 | Dabrafenib | mutBRAF inhibition | 72 | -8.5 | -5.9 | ++ | + |
| 20 | Sorafenib | pan-RAF | 72 | -8.5 | -5.9 | ++ | + |
| 21 | AT-7519 | CDK9 inhibition | 72 | -8.5 | -5.9 | ++ | - |

(continued)

| No | Anticancer agent | Target/ Mechanism | Culture time (h) | Concentration (log M) | | Synergy 1 | Synergy 2 |
|---|---|---|---|---|---|---|---|
| | | | | Anticancer agent | Ex. 1-2 | | |
| 22 | danusertib | pan Aurora | 72 | -7.0 | -5.9 | +++ | + |
| 23 | ibrutinib | BTK | 72 | -8.0 | -5.9 | +++ | + |
| 24 | MK-1775 | WEE1 | 72 | -7.0 | -5.9 | ++ | ++ |
| 25 | methotrexate | DHFR | 72 | -7.5 | -5.9 | ++ | +++ |
| 26 | AMG-900 | pan Aurora | 72 | -8.0 | -5.9 | +++ | ++ |
| 27 | BIIB021 | HSP90 | 72 | -9.5 | -5.9 | +++ | + |
| 28 | 6-thioguanine | purine antimetabolite | 72 | -7.0 | -5.9 | +++ | + |
| 29 | reversine | A3AR antagonism, pan-aurora inhibition | 120 | -8.0 | -6.3 | ++ | + |
| 30 | MLN-7243 | ubiquitin E1 enzyme | 120 | -7.5 | -6.3 | ++ | + |
| 31 | ABT-737 | Bcl-2 | 72 | -7.0 | -5.9 | +++ | ++ |
| 32 | MK-5108 | Aurora A | 72 | -7.5 | -5.9 | ++ | ++ |
| 33 | GSK-343 | EZH2/ARID1 A | 120 | -8.5 | -6.3 | ++ | - |
| 34 | 2-D08 | SUMOylation | 120 | -6.0 | -6.3 | +++ | + |
| 35 | SCH-900776 | CHK1 | 72 | -7.5 | -5.9 | ++ | + |
| 36 | entinostat | HDAC1, HDAC3 | 72 | -7.5 | -5.9 | ++ | - |
| 37 | ruxolitinib | JAK1/2 | 72 | -6.0 | -5.9 | ++ | + |
| 38 | carfilzomib | proteasome | 72 | -9.0 | -5.9 | ++ | - |
| 39 | apitolisib | PI3K$\alpha$/$\beta$/$\delta$/$\gamma$ | 72 | -8.5 | -5.9 | +++ | + |
| 40 | ipatasertib | AKT | 72 | -8.0 | -5.9 | ++ | + |
| 41 | prednisolone | GR | 72 | No Concentration -dependent change | -5.9 | NA | NA |
| 42 | volasertib | PLK1 | 72 | -8.0 | -5.9 | ++ | - |

Referring to Table 9 and Figures 25a to 26c, when the conventional anticancer agent and the example compound were used in combination, the cell death rate was higher than the simple sum of the cell death rates when the conventional anticancer agent and the example compound were used alone. That is, the example compound can exhibit synergistic anticancer activity when used in combination with an anticancer agent.

It should be understood that any feature, property, compound, compound moiety, or number described in connection with a particular aspect, embodiment, or example of the present invention is applicable to any other aspect, embodiment, or example described herein, as appropriate. All features, or individual steps of a method or process disclosed herein can be arbitrarily combined, except where such features and/or steps constitute a mutually exclusive combination.

Although specific embodiments of the present invention have been described herein, the present invention is not limited to the described embodiments, and it should be understood that it is intended to cover any equivalents, modifications or uses of known or customary techniques by those skilled in the art based on the disclosure herein.

## Claims

**1.** A compound represented by Formula I below, or a solvate, stereoisomer or pharmaceutically acceptable salt thereof:

[Formula I]

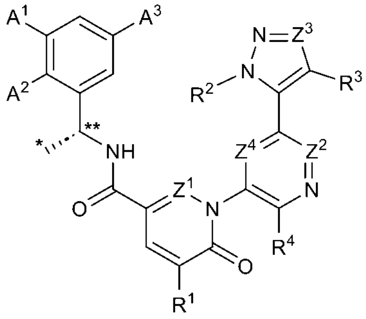

in Formula I,

$Z^1$ and $Z^3$ are each independently N or CH;
$Z^2$ is N or $CR^a$, and $Z^4$ is CH; or
$Z^2$ is $CR^a$, and $Z^4$ is N; and
$R^a$ is H, halogen, OH, CN, substituted or unsubstituted $C_1$-$C_6$ alkoxy, amino, substituted or unsubstituted $C_1$-$C_6$ alkylamino, substituted or unsubstituted di($C_1$-$C_6$ alkyl)amino, substituted or unsubstituted $C_1$-$C_6$ thioalkoxy, substituted or unsubstituted $C_1$-$C_6$ alkyl, substituted or unsubstituted partially unsaturated or saturated $C_3$-$C_7$ cycloalkyl, or a 4-to 7-membered heterocyclyl containing 1 or 2 heteroatoms selected from N, O and S;
$R^1$ is H; D; halogen; OH; CN; $C_1$-$C_6$ alkyl optionally substituted with one or more of deuterium, OH, oxo (=O) or halogen; $-NR^AR^B$; $-OR^A$; partially unsaturated or saturated $C_3$-$C_7$ cycloalkyl; optionally substituted $C_6$-$C_{10}$ aryl; optionally substituted 5- to 10-membered heteroaryl; or

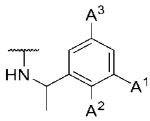;

$R^A$ and $R^B$ are each independently H or $C_1$-$C_6$ alkyl optionally substituted with $R^a$;
$R^2$ is H; $C_1$-$C_6$ alkyl optionally substituted with one or more of deuterium, OH, $C_1$-$C_6$ alkoxy or halogen; a 4- to 7-membered heterocyclyl containing 1 or 2 heteroatoms selected from N, O and S, optionally substituted with one or more $R^{21a}$; or partially unsaturated or saturated $C_3$-$C_7$ cycloalkyl optionally substituted with one or more $R^{21b}$;
$R^{21a}$ and $R^{21b}$ are each independently $C_1$-$C_6$ alkyl optionally substituted with one or more of halogen, CN, OH, $C_1$-$C_6$ alkoxy, carboxy or oxo; $C_1$-$C_6$ acyl; carboxy; $C_1$-$C_6$ alkoxycarbonyl; or $C_1$-$C_6$ alkylsulfonyl;
$R^3$ is H, halogen, OH, $C_1$-$C_6$ alkoxy, $NH_2$, $C_1$-$C_6$ alkylamino, di($C_1$-$C_6$ alkyl)amino, $NO_2$ or CN;
$R^4$ is H, halogen, OH, $C_1$-$C_6$ alkoxy, $NH_2$, $C_1$-$C_6$ alkylamino, di($C_1$-$C_6$ alkyl)amino, $NO_2$ or CN;
$A^1$ is $C_1$-$C_6$ alkyl substituted with one or more substituents independently selected from the group consisting of deuterium, halogen, OH, $C_1$-$C_3$ alkoxy,

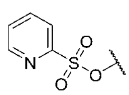

and $C_1$-$C_3$ alkyl; or partially unsaturated or saturated $C_3$-$C_7$ cycloalkyl; wherein said one or more substituents may be bonded to the same or different carbons, and two $C_1$-$C_3$ alkyl groups bonded to the same carbon atom may be optionally taken together with the carbon atom to which they are attached to form a $C_3$-$C_4$ cyclic ring;
$A^2$ is H, halogen, OH, CN or $C_1$-$C_6$ alkyl;
$A^3$ is H, amino, $C_1$-$C_6$ alkylamino or di($C_1$-$C_6$ alkyl)amino; and
the carbon atoms marked with * and ** are optionally substituted with one or more deuterium atoms; with the proviso that the compound represented by Formula I is not

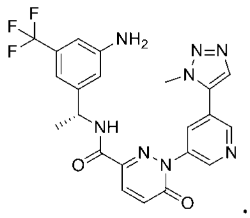

.

2. The compound, or solvate, stereoisomer or pharmaceutically acceptable salt thereof according to claim 1, wherein

one or more of $R^1$, $R^2$ and $A^1$ comprise deuterium; and/or
one or more of the carbon atoms marked with * and ** are substituted with deuterium.

3. The compound, or solvate, stereoisomer or pharmaceutically acceptable salt thereof according to claim 1, wherein $Z^4$ is CH.

4. The compound, or solvate, stereoisomer or pharmaceutically acceptable salt thereof according to claim 3, wherein

$Z^2$ is $CR^a$, and
$R^a$ and $R^4$ are both H; or
$R^a$ is halogen, OH, CN, $C_1$-$C_6$ alkoxy, amino, $C_1$-$C_6$ alkylamino, di($C_1$-$C_6$ alkyl)amino, $C_1$-$C_6$ alkyl, partially unsaturated or saturated $C_3$-$C_7$ cycloalkyl, or a 4- to 6-membered heterocyclyl containing 1 or 2 heteroatoms selected from N and O, and $R^4$ is H or halogen; or
$R^a$ is H, and $R^4$ is halogen.

5. The compound, or solvate, stereoisomer or pharmaceutically acceptable salt thereof according to claim 4, wherein

$R^a$ is H, F, $-NHCH_3$, $-N(CH_3)_2$, $-OCH_3$, $-CH_3$ or

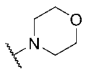

,

and
$R^4$ is H or F.

6. The compound, or solvate, stereoisomer or pharmaceutically acceptable salt thereof according to claim 1, wherein

$R^2$ is a 4- to 6-membered heterocyclyl containing 1 or 2 heteroatoms selected from N and O, optionally substituted with one or more $R^{21a}$; or partially unsaturated or saturated $C_3$-$C_7$ cycloalkyl optionally substituted with one or more $R^{21b}$; and/or
$R^3$ is halogen, and
$R^{21a}$ is $C_1$-$C_4$ alkyl optionally substituted with one or more of OH, $C_1$-$C_4$ alkoxy or oxo; $C_1$-$C_4$ acyl; $C_1$-$C_4$ alkoxycarbonyl; or $C_1$-$C_4$ alkylsulfonyl, and $R^{21b}$ is halogen.

7. The compound, or solvate, stereoisomer or pharmaceutically acceptable salt thereof according to claim 1, wherein

$R^2$ is $-CH_3$, $-CD_3$, $-CH_2CH_3$, $-CH(CH_3)_2$, $-CH_2CH_2OH$, cyclopropyl,

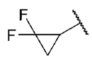

,

cyclobutyl,

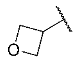, 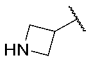, 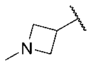, 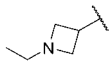, 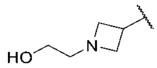, 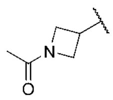, 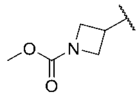, 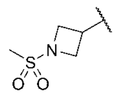,

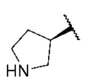, 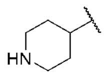, 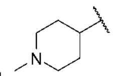, 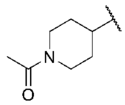, or 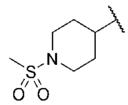,

and

$R^3$ is H or Cl.

**8.** The compound, or solvate, stereoisomer or pharmaceutically acceptable salt thereof according to claim 1, wherein $R^1$ is H; D; halogen; CN; $C_1$-$C_6$ alkyl optionally substituted with one or more of deuterium, OH, oxo (=O) or halogen; partially unsaturated or saturated $C_3$-$C_7$ cycloalkyl; $C_6$-$C_{10}$ aryl; or

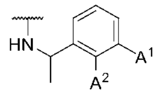.

**9.** The compound, or solvate, stereoisomer or pharmaceutically acceptable salt thereof according to claim 8, wherein $R^1$ is H, D, F, Br, Cl, CN, methyl, ethyl, $CD_3$, $CF_3$, $-CH_2OH$, $-CH(OH)CH_3$, - $C(OH)(CH_3)_2$, $-C(=O)CH_3$, cyclopropyl, cyclohexyl, cyclohexenyl, phenyl, or

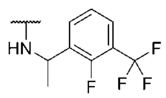.

**10.** The compound, or solvate, stereoisomer or pharmaceutically acceptable salt thereof according to claim 1, wherein

$A^1$ is $C_1$-$C_6$ alkyl substituted with two or more substituents independently selected from the group consisting of deuterium, halogen, OH, $C_1$-$C_3$ alkoxy,

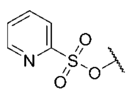

and $C_1$-$C_3$ alkyl; or partially unsaturated or saturated $C_3$-$C_7$ cycloalkyl; wherein said two $C_1$-$C_3$ alkyl groups, when bonded to the same carbon atom, are taken together with the carbon atom to which they are attached to form a $C_3$-$C_4$ cyclic ring,

$A^2$ is H, halogen or $C_1$-$C_4$ alkyl, and

$A^3$ is H or amino.

**11.** The compound, or solvate, stereoisomer or pharmaceutically acceptable salt thereof according to claim 10, wherein

$A^1$ is selected from the group consisting of $-CHF_2$, $-CF_3$, $-CF_2CH_3$, $-CF_2CH_2F$, - $CF_2CH_2OH$, $-CF_2CD_2OH$, $-CF_2CH_2OCH_3$,

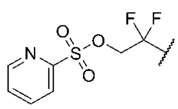, 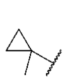, 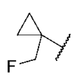, 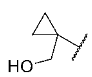, 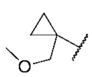

and cyclobutenyl; and

$A^2$ is H, F, Cl or $-CH_3$.

**12.** The compound, or solvate, stereoisomer or pharmaceutically acceptable salt thereof according to claim 1, wherein

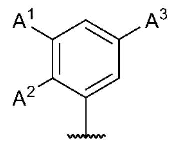

of Formula I is selected from the following structures:

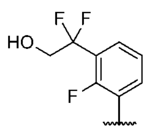 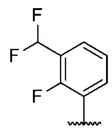 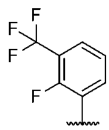 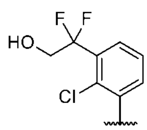 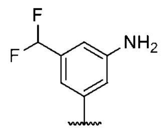 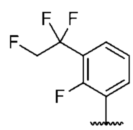 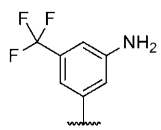

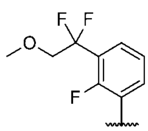 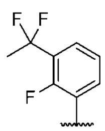 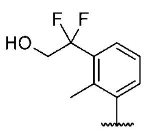 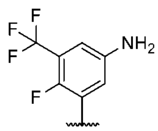 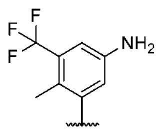 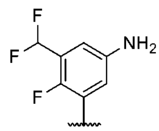 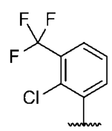

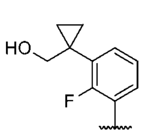 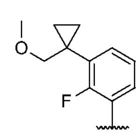 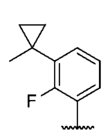 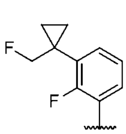 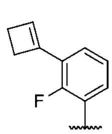 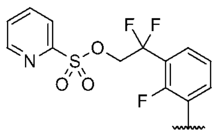 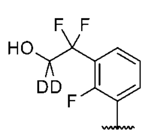 .

**13.** The compound, or solvate, stereoisomer or pharmaceutically acceptable salt thereof according to claim 1, wherein the compound is a compound represented by Formula I-1 below:

[Formula I-1]

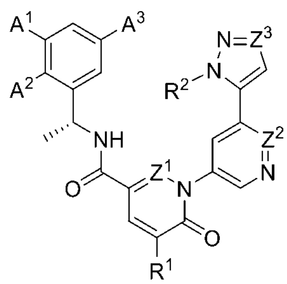

in Formula I-1,

$Z^1$ and $Z^3$ are each independently N or CH;
$Z^2$ is N or $CR^d$;
$R^a$ is H, halogen, OH, CN, substituted or unsubstituted $C_1$-$C_6$ alkoxy, substituted or unsubstituted $C_1$-$C_6$ alkylamino, substituted or unsubstituted $C_1$-$C_6$ thioalkoxy, or substituted or unsubstituted $C_1$-$C_6$ alkyl;
$R^1$ is H, halogen, OH, CN, $C_1$-$C_6$ alkyl optionally substituted with one or more deuterium or halogen, $-NR^AR^B$, $-OR^A$, $C_3$-$C_6$ cycloalkyl, optionally substituted $C_6$-$C_{10}$ aryl, or optionally substituted 5- to 10-membered heteroaryl;
$R^A$ and $R^B$ are each independently $C_1$-$C_6$ alkyl optionally substituted with $R^a$;
$R^2$ is H, or $C_1$-$C_6$ alkyl optionally substituted with one or more of deuterium or halogen;
$A^1$ is $C_1$-$C_6$ alkyl substituted with one or more substituents independently selected from the group consisting of halogen, OH, $C_1$-$C_3$ alkoxy and $C_1$-$C_3$ alkyl, wherein said one or more substituents may be bonded to the same or different carbons, and two $C_1$-$C_3$ alkyl groups bonded to the same carbon atom may be optionally taken together with the carbon atom to which they are attached to form a $C_3$-$C_4$ cyclic ring;
$A^2$ is H, halogen, OH, CN or $C_1$-$C_6$ alkyl; and

$A^3$ is H or amino, with the proviso that the compound represented by Formula I-1 is not

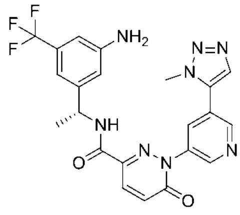.

**14.** The compound, or solvate, stereoisomer or pharmaceutically acceptable salt thereof according to claim 13, wherein

$Z^1$ is N, and $Z^2$ is CH, and $Z^3$ is N or CH; or
$Z^1$ and $Z^2$ are both CH, and $Z^3$ is N; or
$Z^1$ and $Z^2$ are both N, and $Z^3$ is N or CH.

**15.** The compound, or solvate, stereoisomer or pharmaceutically acceptable salt thereof according to claim 13, wherein

$R^a$ is H, halogen, CN, $C_1$-$C_3$ alkyl or $C_3$-$C_5$ cycloalkyl; and
$R^2$ is $C_1$-$C_3$ alkyl optionally substituted with one or more deuterium.

**16.** The compound, or solvate, stereoisomer or pharmaceutically acceptable salt thereof according to claim 13, wherein

$A^1$ is $C_1$-$C_3$ alkyl substituted with two or more substituents independently selected from the group consisting of F, OH and methoxy;
$A^2$ is H, halogen or $C_1$-$C_3$ alkyl, and
$A^3$ is H.

**17.** The compound, or solvate, stereoisomer or pharmaceutically acceptable salt thereof according to claim 13, wherein

$A^1$ is $C_1$-$C_3$ alkyl substituted with two or more F,
$A^2$ is H, halogen or $C_1$-$C_3$ alkyl, and
$A^3$ is amino.

**18.** The compound, or solvate, stereoisomer or pharmaceutically acceptable salt thereof according to claim 1, **characterized in that** the compound is selected from:

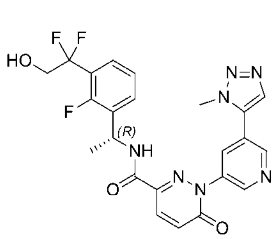 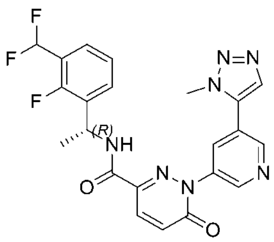 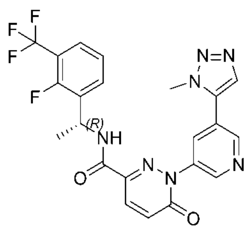 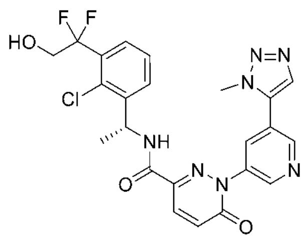

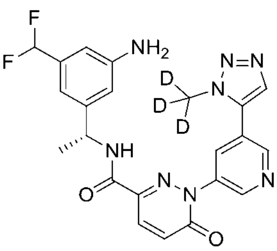 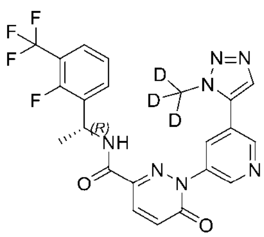 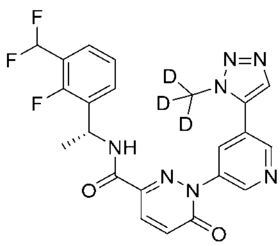 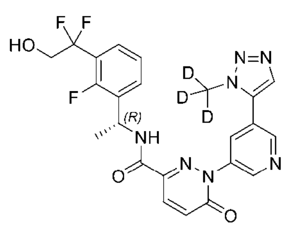

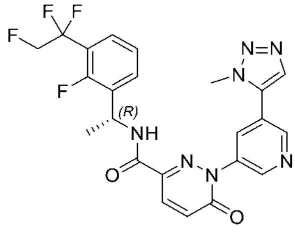
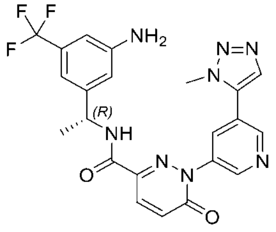
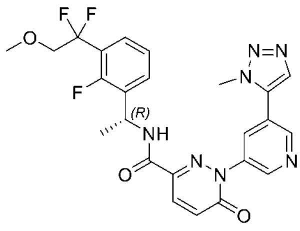
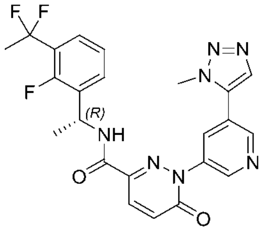

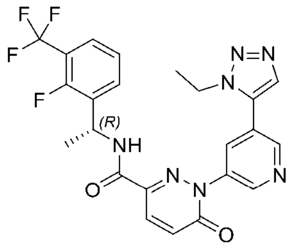
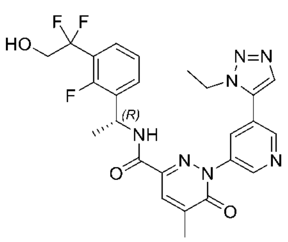
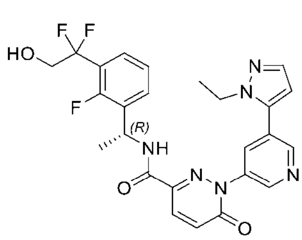
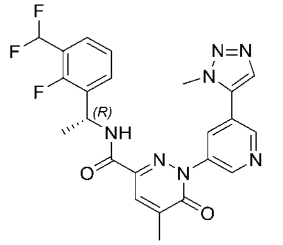

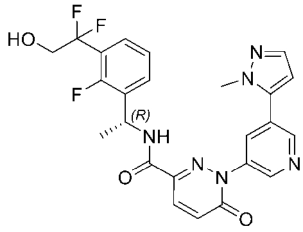
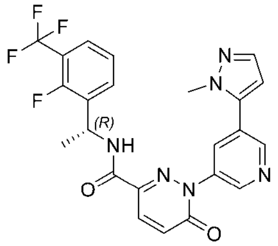
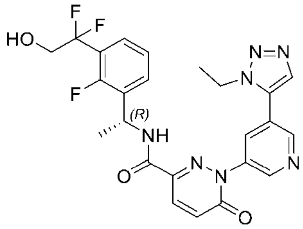
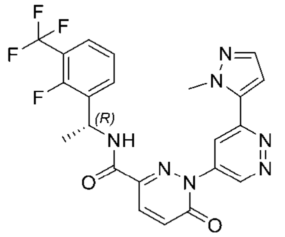

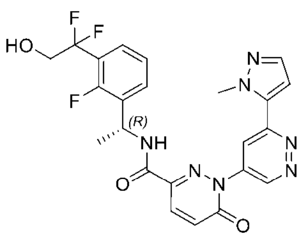
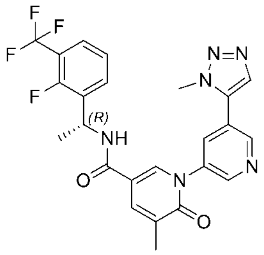
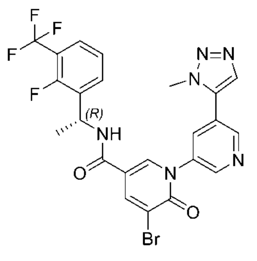
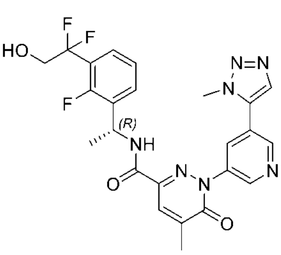

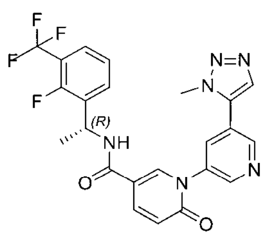
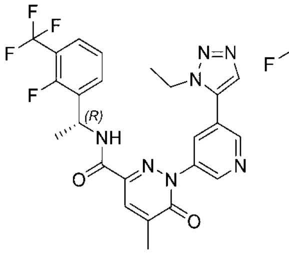
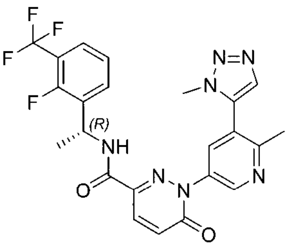
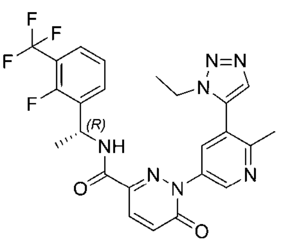

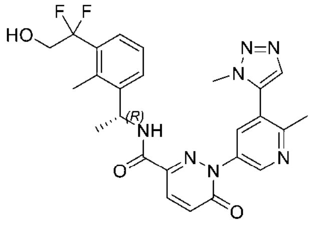
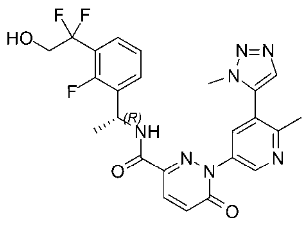
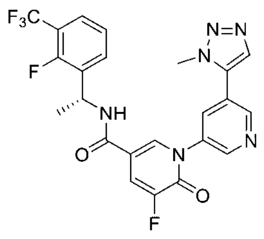
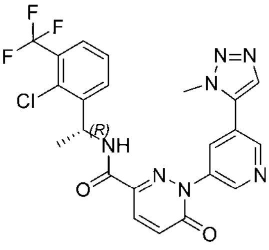

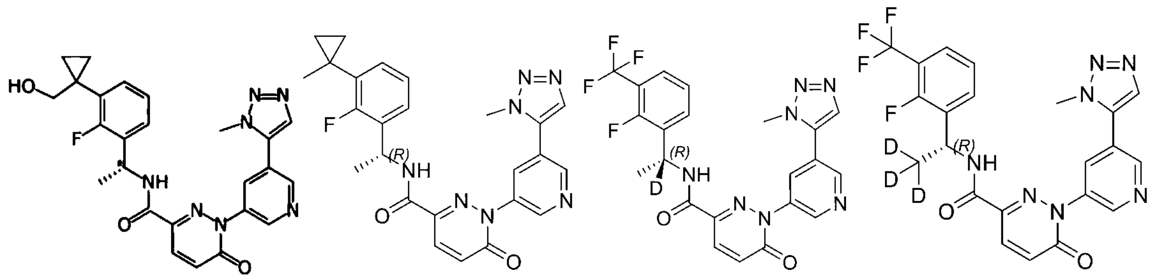

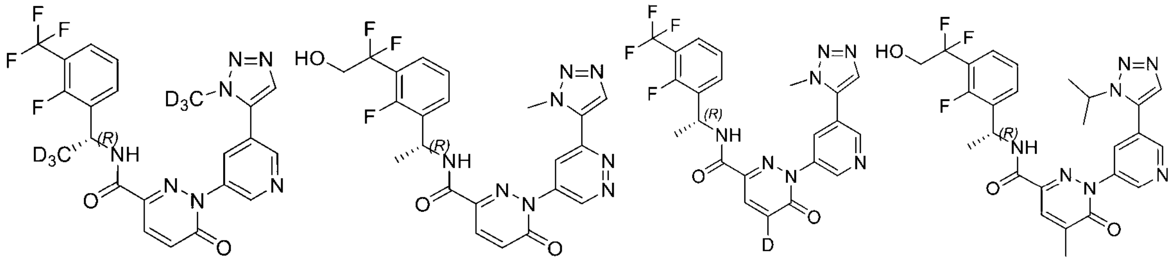

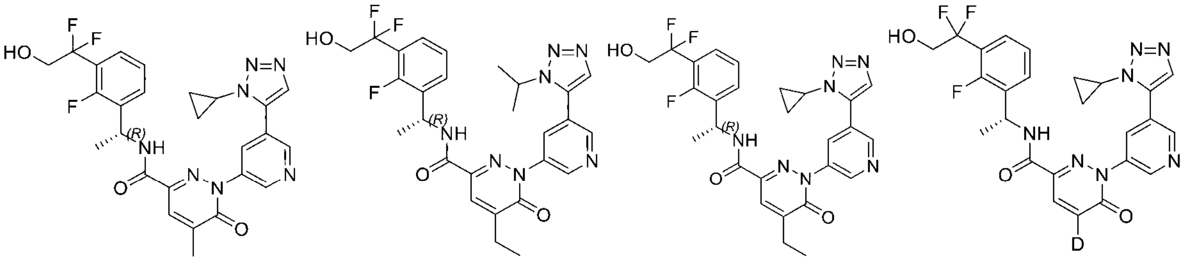

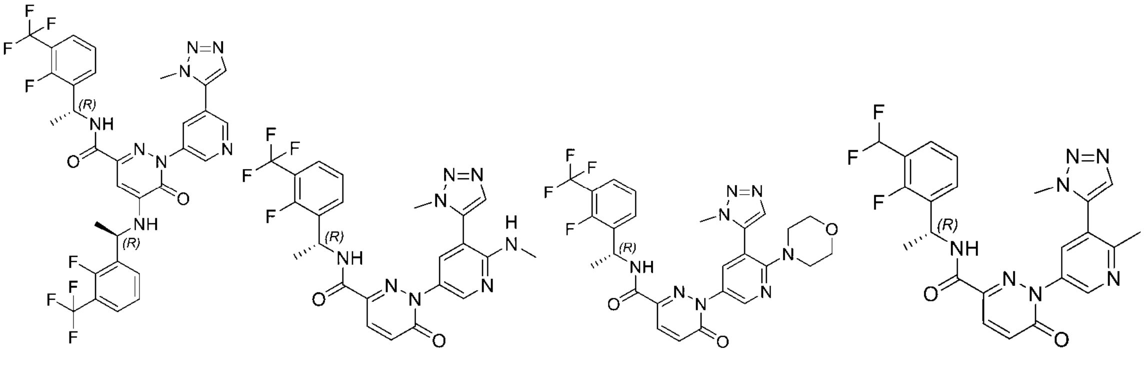

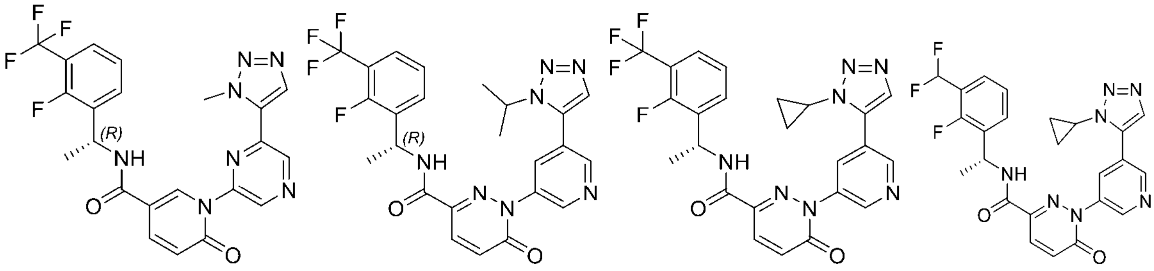

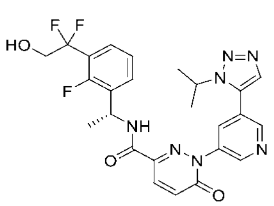
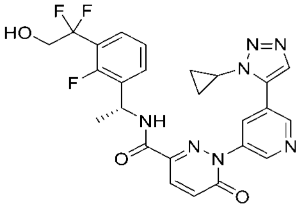
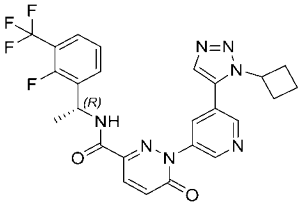
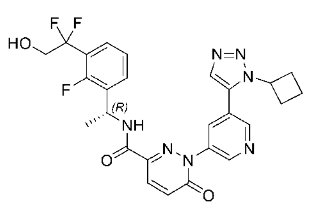

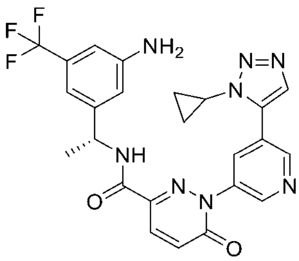
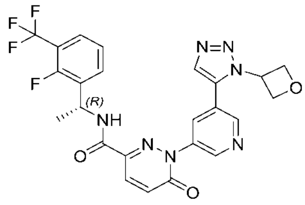
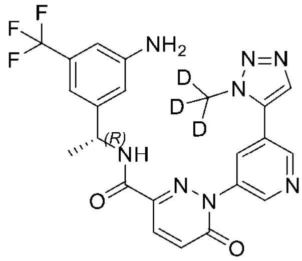
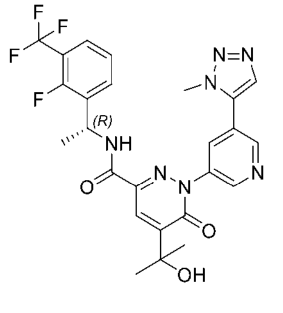

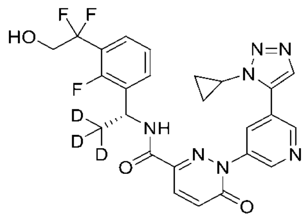
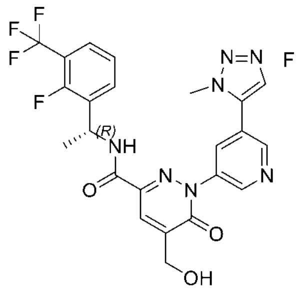
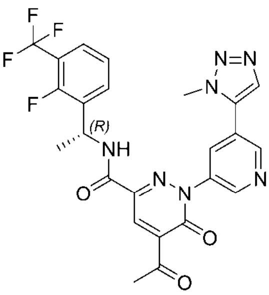
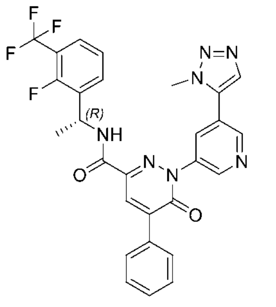

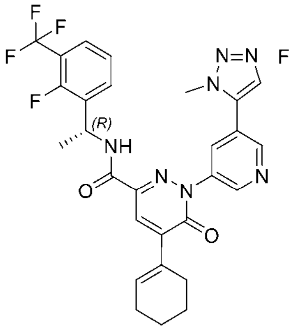
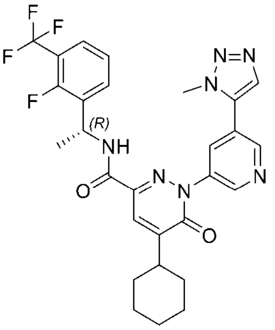
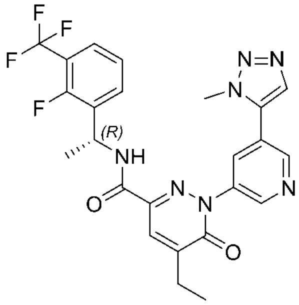
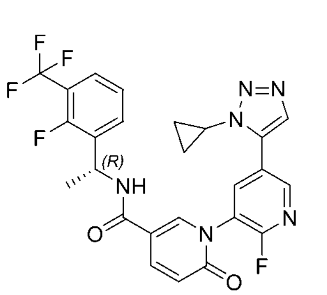

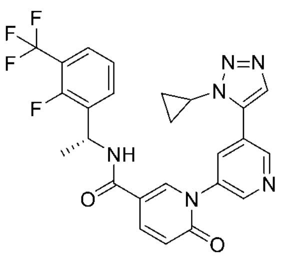
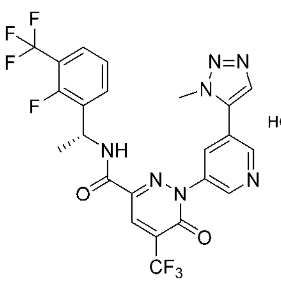
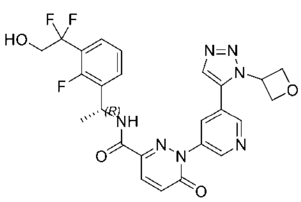
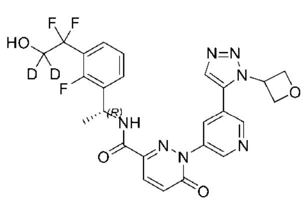

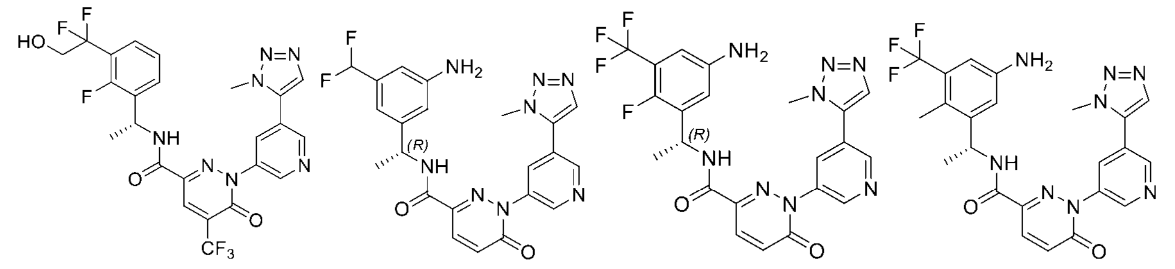

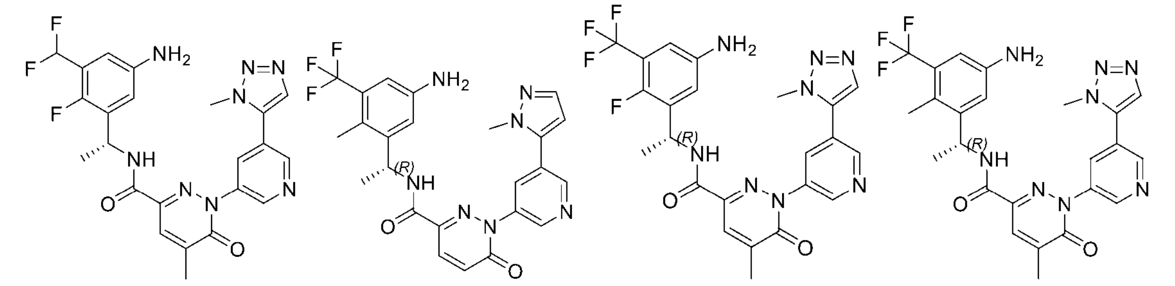

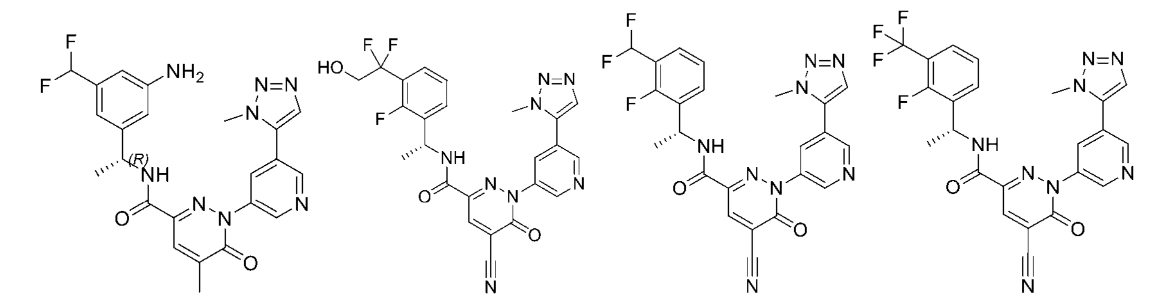

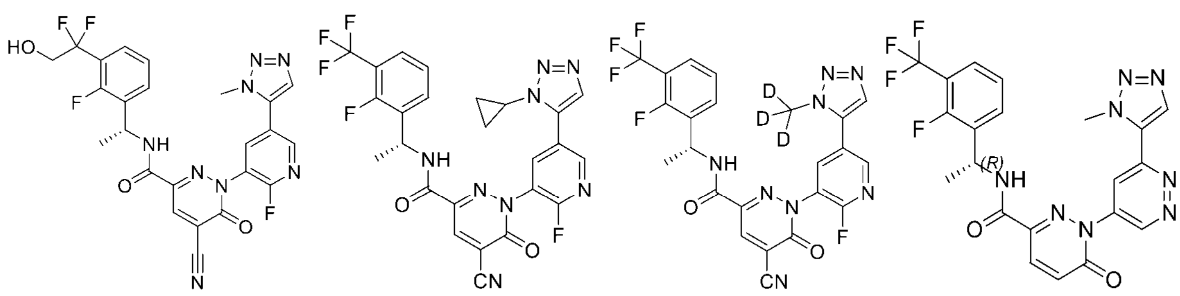

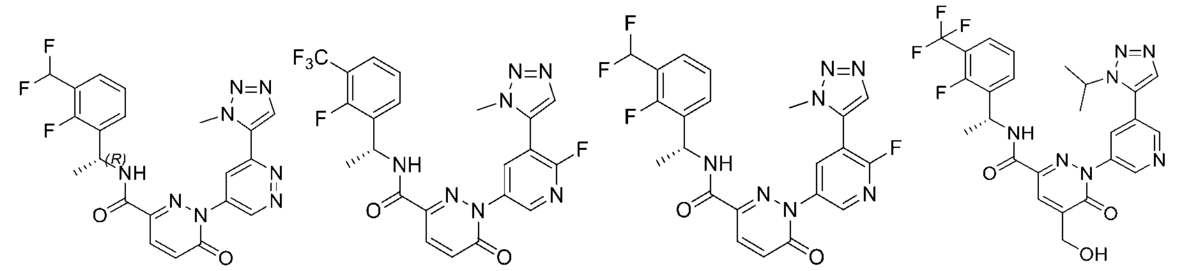

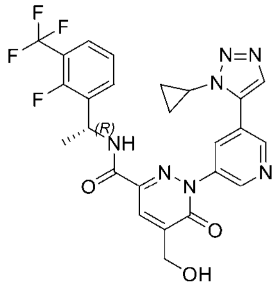
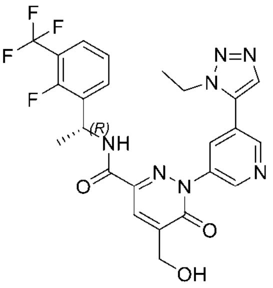
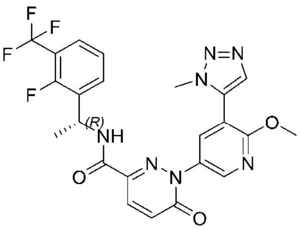
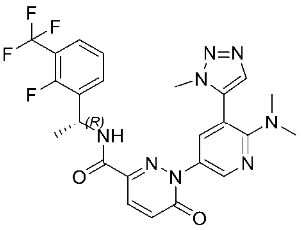

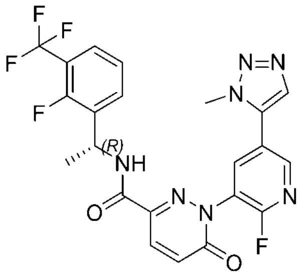
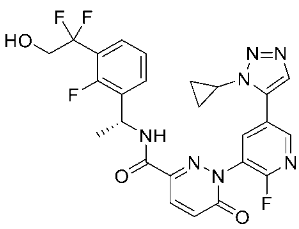
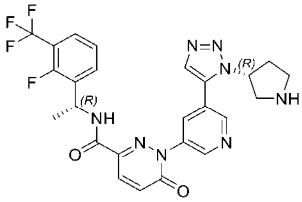
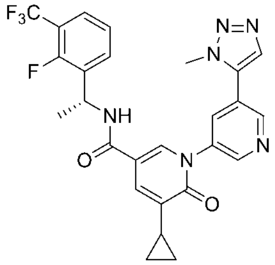

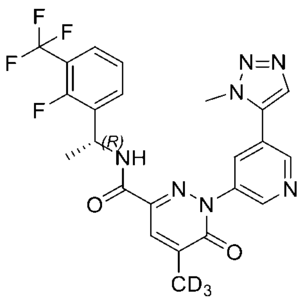
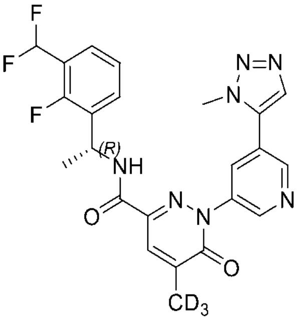
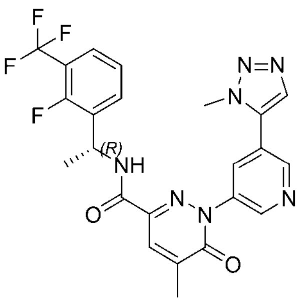
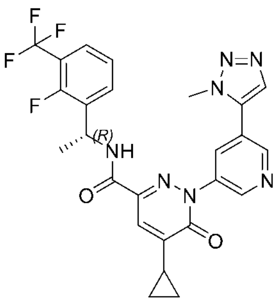

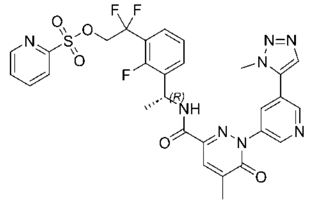
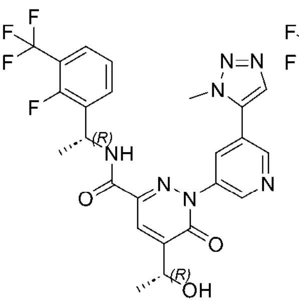
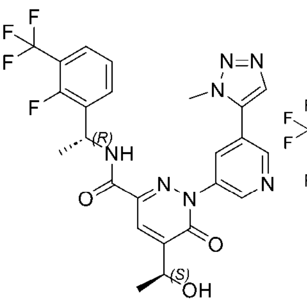
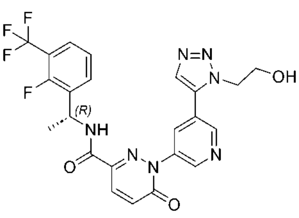

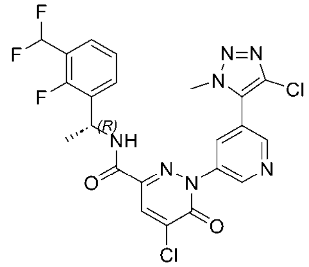
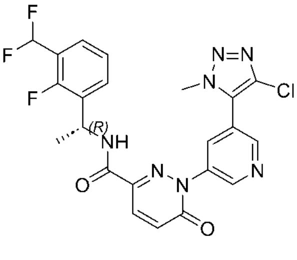
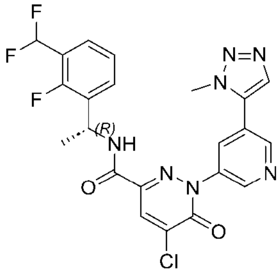
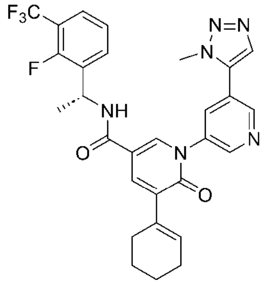

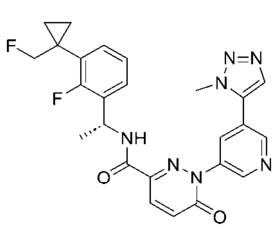 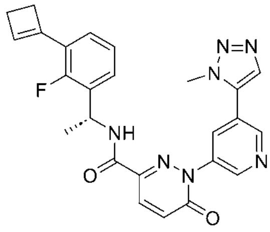 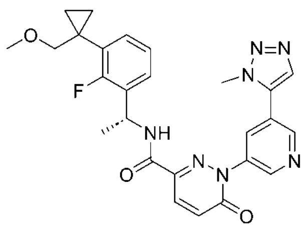 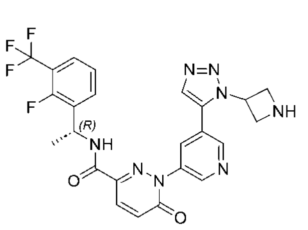

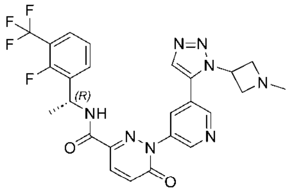 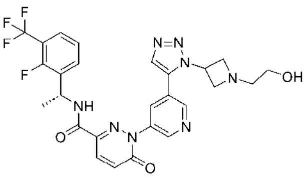 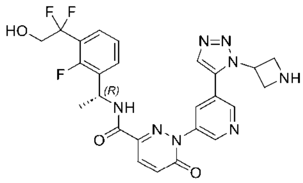 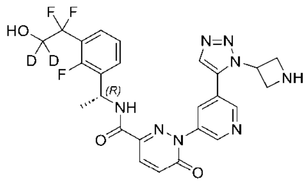

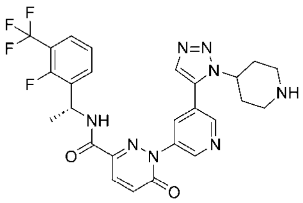 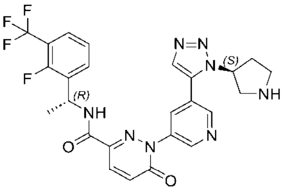 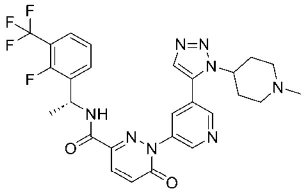 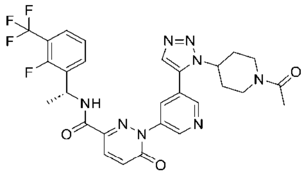

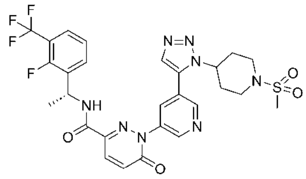 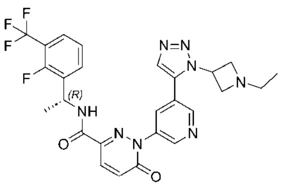 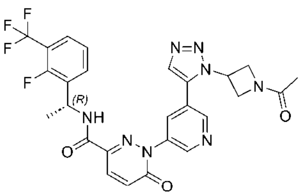 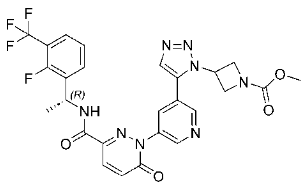

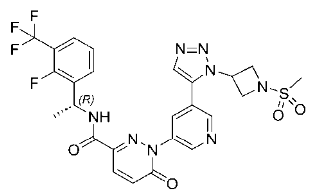 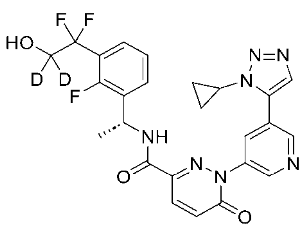 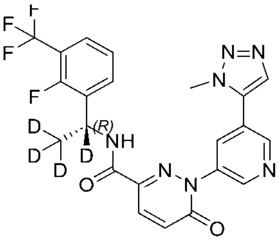 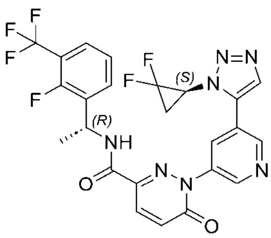

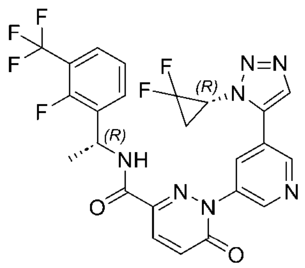 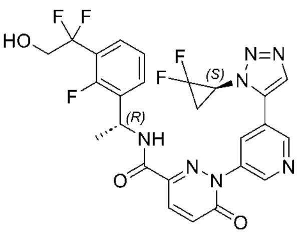 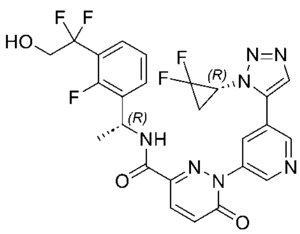.

**19.** A pharmaceutical composition comprising the compound, or solvate, stereoisomer or pharmaceutically acceptable salt thereof according to any one of claims 1 to 18 as an active ingredient.

**20.** The pharmaceutical composition according to claim 19, **characterized in that** the pharmaceutical composition is for the prevention or treatment of a SOS1 mediated disease.

**21.** The pharmaceutical composition according to claim 20, **characterized in that** the SOS1 mediated disease is cancer

or RASopathy.

**22.** The pharmaceutical composition according to claim 21, **characterized in that** the cancer is selected from the group consisting of pancreatic cancer, lung cancer, colorectal cancer, biliary tract cancer, multiple myeloma, melanoma, uterine cancer, cervical cancer, endometrial cancer, thyroid cancer, chronic lymphocytic leukemia, acute myeloid leukemia, bladder cancer, urothelial cancer, gastric cancer, squamous cell carcinoma of the head and neck, diffuse large B cell lymphoma, esophageal cancer, hepatocellular cancer, breast cancer, ovarian cancer, prostate cancer, glioblastoma, kidney cancer and sarcoma.

**23.** The pharmaceutical composition according to claim 21, **characterized in that** the RASopathy is selected from the group consisting of neurofibromatosis type 1, Noonan syndrome, Leopard syndrome, capillary malformation-arteriovenous malformation syndrome, Costello syndrome, CFC syndrome (Cardio-Facio-Cutaneous syndrome), Legius syndrome, and hereditary gingival fibromatosis.

**24.** A method for preventing or treating a SOS1 mediated disease, comprising administering to a subject the compound, or solvate, stereoisomer or pharmaceutically acceptable salt thereof according to any one of claims 1 to 18.

**25.** Use of the compound, or solvate, stereoisomer or pharmaceutically acceptable salt thereof according to any one of claims 1 to 18 for use in the prevention or treatment of a SOS1 mediated disease.

**26.** Use of the compound, or solvate, stereoisomer or pharmaceutically acceptable salt thereof according to any one of claims 1 to 18 for the manufacture of a medicament for the prevention or treatment of a SOS1 mediated disease.

**Amended claims under Art. 19.1 PCT**

**1.** A compound represented by Formula I below, or a solvate, stereoisomer or pharmaceutically acceptable salt thereof:

[Formula I]

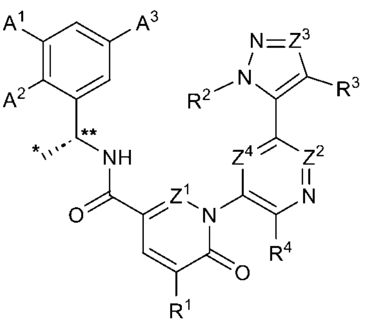

in Formula I,

$Z^1$ and $Z^3$ are each independently N or CH;
$Z^2$ is N or $CR^d$, and $Z^4$ is CH; or
$Z^2$ is $CR^a$, and $Z^4$ is N; and
$R^a$ is H, halogen, OH, CN, substituted or unsubstituted $C_1$-$C_6$ alkoxy, amino, substituted or unsubstituted $C_1$-$C_6$ alkylamino, substituted or unsubstituted di($C_1$-$C_6$ alkyl)amino, substituted or unsubstituted $C_1$-$C_6$ thioalkoxy, substituted or unsubstituted $C_1$-$C_6$ alkyl, substituted or unsubstituted partially unsaturated or saturated $C_3$-$C_7$ cycloalkyl, or a 4-to 7-membered heterocyclyl containing 1 or 2 heteroatoms selected from N, O and S;
$R^1$ is H; D; halogen; OH; CN; $C_1$-$C_6$ alkyl optionally substituted with one or more of deuterium, OH, oxo (=O) or halogen; $-NR^AR^B$; $-OR^A$; partially unsaturated or saturated $C_3$-$C_7$ cycloalkyl; optionally substituted $C_6$-$C_{10}$ aryl; optionally substituted 5- to 10-membered heteroaryl; or

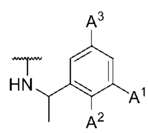
;

$R^A$ and $R^B$ are each independently H or $C_1$-$C_6$ alkyl optionally substituted with $R^a$;
$R^2$ is H; $C_1$-$C_6$ alkyl optionally substituted with one or more of deuterium, OH, $C_1$-$C_6$ alkoxy or halogen; a 4- to 7-membered heterocyclyl containing 1 or 2 heteroatoms selected from N, O and S, optionally substituted with one or more $R^{21a}$; or partially unsaturated or saturated $C_3$-$C_7$ cycloalkyl optionally substituted with one or more $R^{21b}$;
$R^{21a}$ and $R^{21b}$ are each independently $C_1$-$C_6$ alkyl optionally substituted with one or more of halogen, CN, OH, $C_1$-$C_6$ alkoxy, carboxy or oxo; $C_1$-$C_6$ acyl; carboxy; $C_1$-$C_6$ alkoxycarbonyl; or $C_1$-$C_6$ alkylsulfonyl, or $R^{21b}$ is halogen;
$R^3$ is H, halogen, OH, $C_1$-$C_6$ alkoxy, $NH_2$, $C_1$-$C_6$ alkylamino, di($C_1$-$C_6$ alkyl)amino, $NO_2$ or CN;
$R^4$ is H, halogen, OH, $C_1$-$C_6$ alkoxy, $NH_2$, $C_1$-$C_6$ alkylamino, di($C_1$-$C_6$ alkyl)amino, $NO_2$ or CN;
$A^1$ is $C_1$-$C_6$ alkyl substituted with one or more substituents independently selected from the group consisting of deuterium, halogen, OH, $C_1$-$C_3$ alkoxy,

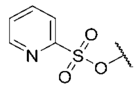

and $C_1$-$C_3$ alkyl; or partially unsaturated or saturated $C_3$-$C_7$ cycloalkyl; wherein said one or more substituents may be bonded to the same or different carbons, and two $C_1$-$C_3$ alkyl groups bonded to the same carbon atom may be optionally taken together with the carbon atom to which they are attached to form a $C_3$-$C_4$ cyclic ring;
$A^2$ is H, halogen, OH, CN or $C_1$-$C_6$ alkyl;
$A^3$ is H, amino, $C_1$-$C_6$ alkylamino or di($C_1$-$C_6$ alkyl)amino; and
the carbon atoms marked with * and ** are optionally substituted with one or more deuterium atoms; with the proviso that the compound represented by Formula I is not

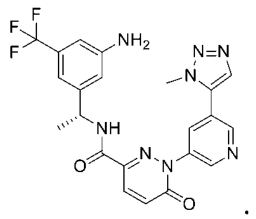.

2. The compound, or solvate, stereoisomer or pharmaceutically acceptable salt thereof according to claim 1, wherein

one or more of $R^1$, $R^2$ and $A^1$ comprise deuterium; and/or
one or more of the carbon atoms marked with * and ** are substituted with deuterium.

3. The compound, or solvate, stereoisomer or pharmaceutically acceptable salt thereof according to claim 1, wherein $Z^4$ is CH.

4. The compound, or solvate, stereoisomer or pharmaceutically acceptable salt thereof according to claim 3, wherein

$Z^2$ is $CR^a$, and
$R^a$ and $R^4$ are both H; or
$R^a$ is halogen, OH, CN, $C_1$-$C_6$ alkoxy, amino, $C_1$-$C_6$ alkylamino, di($C_1$-$C_6$ alkyl)amino, $C_1$-$C_6$ alkyl, partially unsaturated or saturated $C_3$-$C_7$ cycloalkyl, or a 4- to 6-membered heterocyclyl containing 1 or 2 heteroatoms selected from N and O, and $R^4$ is H or halogen; or
$R^a$ is H, and $R^4$ is halogen.

5. The compound, or solvate, stereoisomer or pharmaceutically acceptable salt thereof according to claim 4, wherein

$R^a$ is H, F, $-NHCH_3$, $-N(CH_3)_2$, $-OCH_3$, $-CH_3$ or

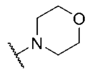,

and

$R^4$ is H or F.

**6.** The compound, or solvate, stereoisomer or pharmaceutically acceptable salt thereof according to claim 1, wherein

$R^2$ is a 4- to 6-membered heterocyclyl containing 1 or 2 heteroatoms selected from N and O, optionally substituted with one or more $R^{21a}$; or partially unsaturated or saturated $C_3$-$C_7$ cycloalkyl optionally substituted with one or more $R^{21b}$; and/or

$R^3$ is halogen, and

$R^{21a}$ is $C_1$-$C_4$ alkyl optionally substituted with one or more of OH, $C_1$-$C_4$ alkoxy or oxo; $C_1$-$C_4$ acyl; $C_1$-$C_4$ alkoxycarbonyl; or $C_1$-$C_4$ alkylsulfonyl, and $R^{21b}$ is halogen.

**7.** The compound, or solvate, stereoisomer or pharmaceutically acceptable salt thereof according to claim 1, wherein

$R^2$ is $-CH_3$, $-CD_3$, $-CH_2CH_3$, $-CH(CH_3)_2$, $-CH_2CH_2OH$, cyclopropyl,

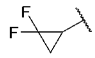,

cyclobutyl,

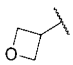, 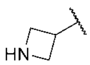, 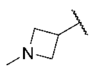, 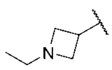, 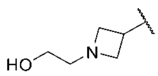, 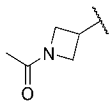, 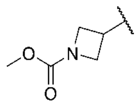, 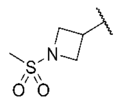,

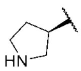, 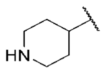, 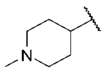, 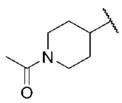, or 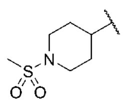,

and

$R^3$ is H or Cl.

**8.** The compound, or solvate, stereoisomer or pharmaceutically acceptable salt thereof according to claim 1, wherein $R^1$ is H; D; halogen; CN; $C_1$-$C_6$ alkyl optionally substituted with one or more of deuterium, OH, oxo (=O) or halogen; partially unsaturated or saturated $C_3$-$C_7$ cycloalkyl; $C_6$-$C_{10}$ aryl; or

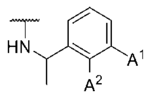.

**9.** The compound, or solvate, stereoisomer or pharmaceutically acceptable salt thereof according to claim 8, wherein $R^1$ is H, D, F, Br, Cl, CN, methyl, ethyl, $CD_3$, $CF_3$, $-CH_2OH$, $-CH(OH)CH_3$, $-C(OH)(CH_3)_2$, $-C(=O)CH_3$, cyclopropyl, cyclohexyl, cyclohexenyl, phenyl, or

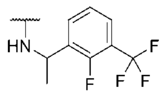.

**10.** The compound, or solvate, stereoisomer or pharmaceutically acceptable salt thereof according to claim 1, wherein

$A^1$ is $C_1$-$C_6$ alkyl substituted with two or more substituents independently selected from the group consisting of deuterium, halogen, OH, $C_1$-$C_3$ alkoxy,

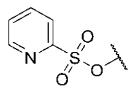

and $C_1$-$C_3$ alkyl; or partially unsaturated or saturated $C_3$-$C_7$ cycloalkyl; wherein said two $C_1$-$C_3$ alkyl groups, when bonded to the same carbon atom, are taken together with the carbon atom to which they are attached to form a $C_3$-$C_4$ cyclic ring,
$A^2$ is H, halogen or $C_1$-$C_4$ alkyl, and
$A^3$ is H or amino.

**11.** The compound, or solvate, stereoisomer or pharmaceutically acceptable salt thereof according to claim 10, wherein

$A^1$ is selected from the group consisting of $-CHF_2$, $-CF_3$, $-CF_2CH_3$, $-CF_2CH_2F$, - $CF_2CH_2OH$, $-CF_2CD_2OH$, $-CF_2CH_2OCH_3$,

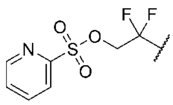 , 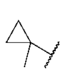 , 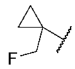 , 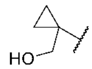 , 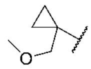

and cyclobutenyl; and
$A^2$ is H, F, Cl or $-CH_3$.

**12.** The compound, or solvate, stereoisomer or pharmaceutically acceptable salt thereof according to claim 1, wherein

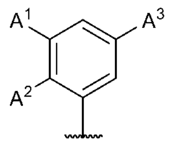

of Formula I is selected from the following structures:

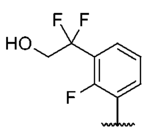 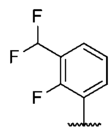 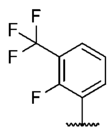 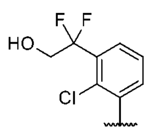 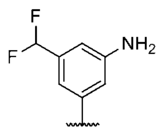 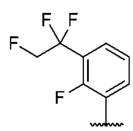 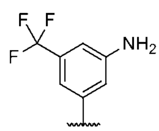

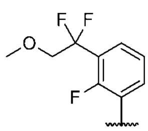 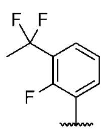 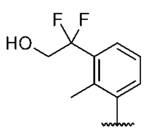 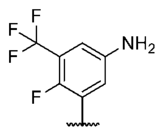 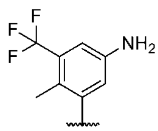 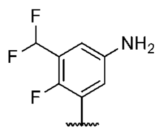 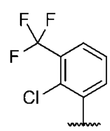

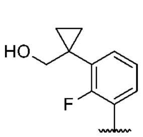 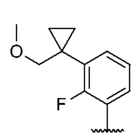 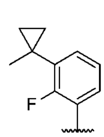 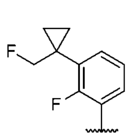 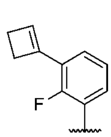 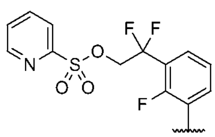 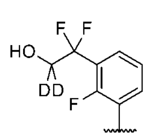 .

**13.** The compound, or solvate, stereoisomer or pharmaceutically acceptable salt thereof according to claim 1, wherein the compound is a compound represented by Formula I-1 below:

[Formula I-1]

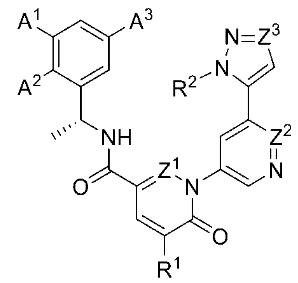

in Formula I-1,

$Z^1$ and $Z^3$ are each independently N or CH;
$Z^2$ is N or $CR^d$;
$R^a$ is H, halogen, OH, CN, substituted or unsubstituted $C_1$-$C_6$ alkoxy, substituted or unsubstituted $C_1$-$C_6$ alkylamino, substituted or unsubstituted $C_1$-$C_6$ thioalkoxy, or substituted or unsubstituted $C_1$-$C_6$ alkyl;
$R^1$ is H, halogen, OH, CN, $C_1$-$C_6$ alkyl optionally substituted with one or more deuterium or halogen, $-NR^AR^B$, $-OR^A$, $C_3$-$C_6$ cycloalkyl, optionally substituted $C_6$-$C_{10}$ aryl, or optionally substituted 5- to 10-membered heteroaryl;
$R^A$ and $R^B$ are each independently $C_1$-$C_6$ alkyl optionally substituted with $R^a$;
$R^2$ is H, or $C_1$-$C_6$ alkyl optionally substituted with one or more of deuterium or halogen;
$A^1$ is $C_1$-$C_6$ alkyl substituted with one or more substituents independently selected from the group consisting of halogen, OH, $C_1$-$C_3$ alkoxy and $C_1$-$C_3$ alkyl, wherein said one or more substituents may be bonded to the same or different carbons, and two $C_1$-$C_3$ alkyl groups bonded to the same carbon atom may be optionally taken together with the carbon atom to which they are attached to form a $C_3$-$C_4$ cyclic ring;
$A^2$ is H, halogen, OH, CN or $C_1$-$C_6$ alkyl; and
$A^3$ is H or amino, with the proviso that the compound represented by Formula I-1 is not

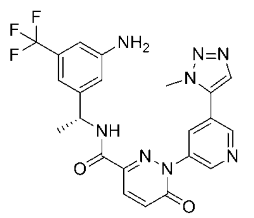

.

**14.** The compound, or solvate, stereoisomer or pharmaceutically acceptable salt thereof according to claim 13, wherein

$Z^1$ is N, and $Z^2$ is CH, and $Z^3$ is N or CH; or
$Z^1$ and $Z^2$ are both CH, and $Z^3$ is N; or
$Z^1$ and $Z^2$ are both N, and $Z^3$ is N or CH.

**15.** The compound, or solvate, stereoisomer or pharmaceutically acceptable salt thereof according to claim 13, wherein

$R^a$ is H, halogen, CN, $C_1$-$C_3$ alkyl or $C_3$-$C_5$ cycloalkyl; and
$R^2$ is $C_1$-$C_3$ alkyl optionally substituted with one or more deuterium.

**16.** The compound, or solvate, stereoisomer or pharmaceutically acceptable salt thereof according to claim 13, wherein

$A^1$ is $C_1$-$C_3$ alkyl substituted with two or more substituents independently selected from the group consisting of F, OH and methoxy;
$A^2$ is H, halogen or $C_1$-$C_3$ alkyl, and
$A^3$ is H.

**17.** The compound, or solvate, stereoisomer or pharmaceutically acceptable salt thereof according to claim 13, wherein

$A^1$ is $C_1$-$C_3$ alkyl substituted with two or more F,
$A^2$ is H, halogen or $C_1$-$C_3$ alkyl, and
$A^3$ is amino.

**18.** The compound, or solvate, stereoisomer or pharmaceutically acceptable salt thereof according to claim 1, **characterized in that** the compound is selected from:

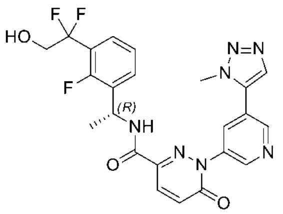 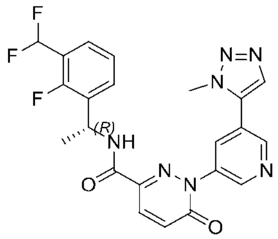 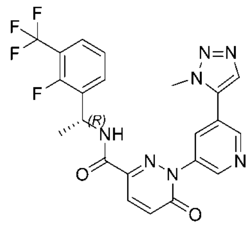 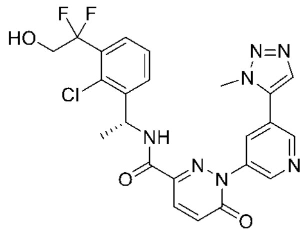

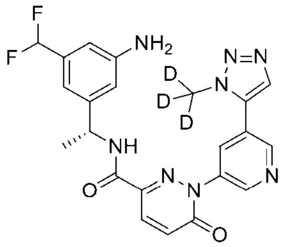 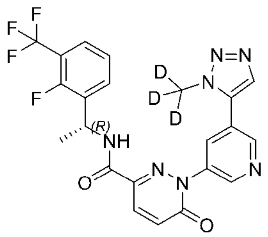 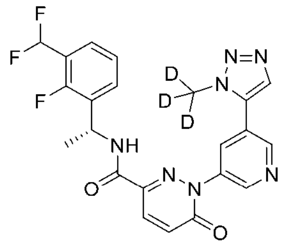 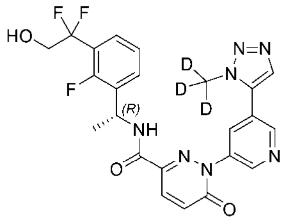

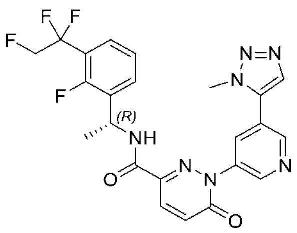 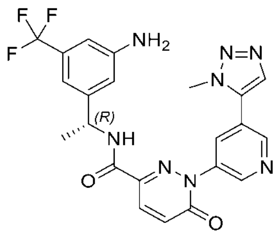 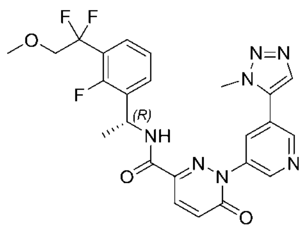 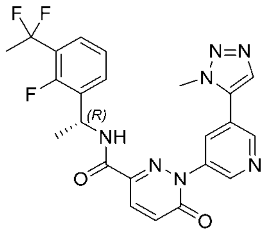

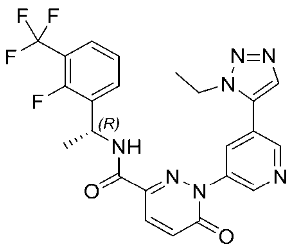 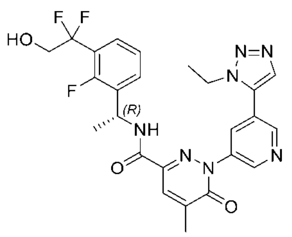 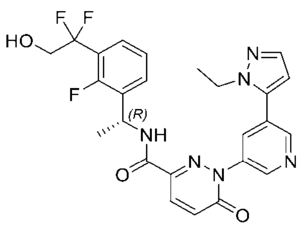 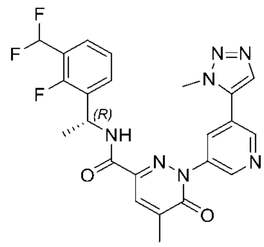

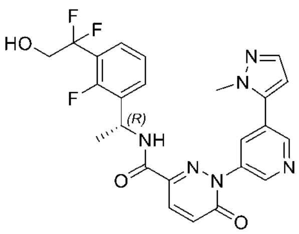 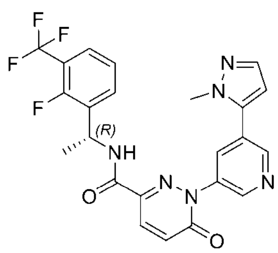 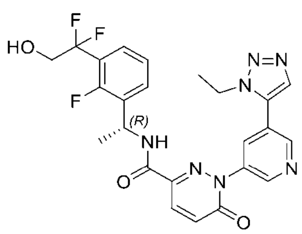 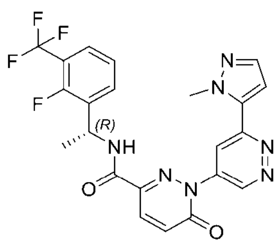

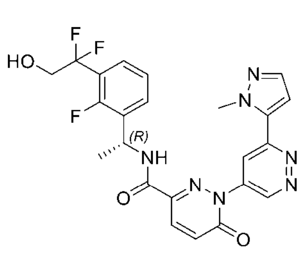
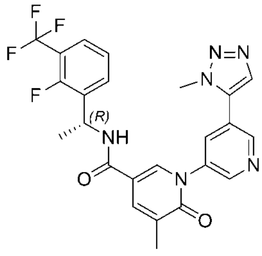
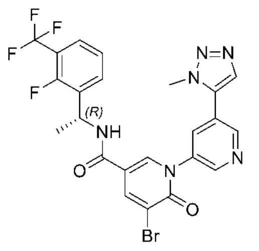
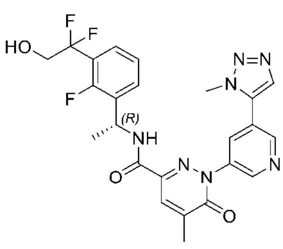
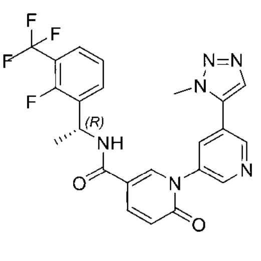
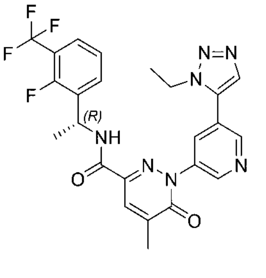
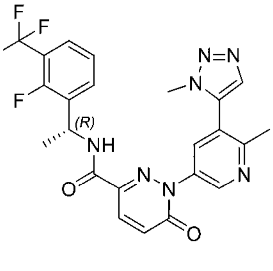
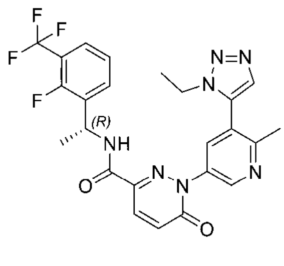
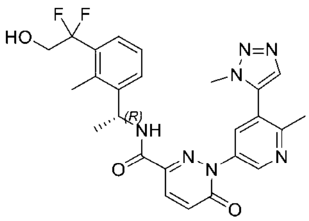
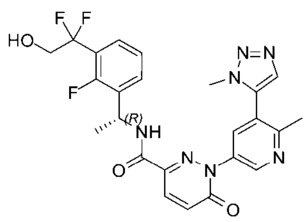
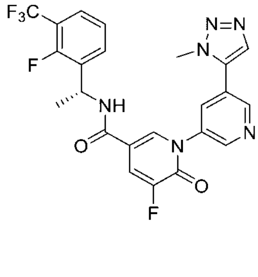
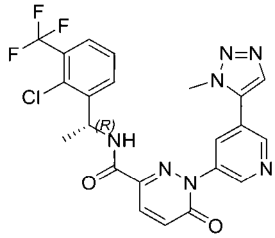
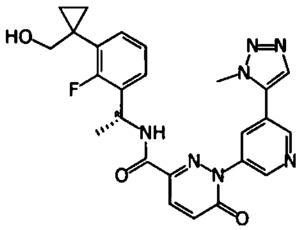
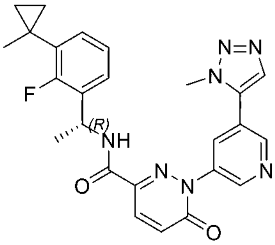
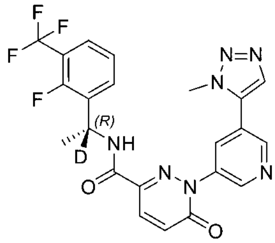
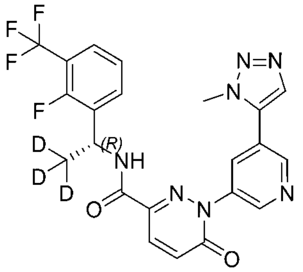
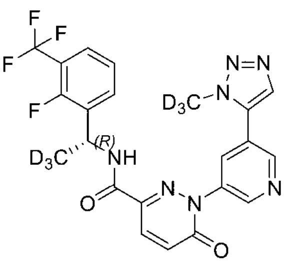
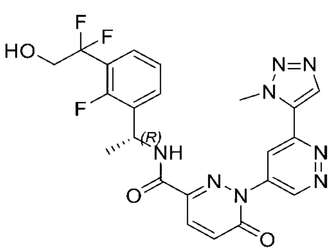
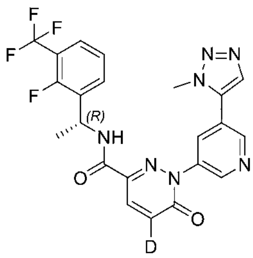
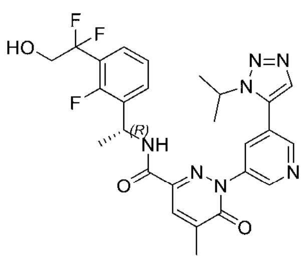
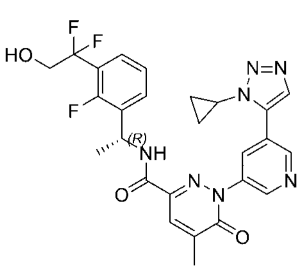
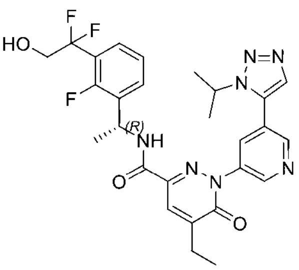
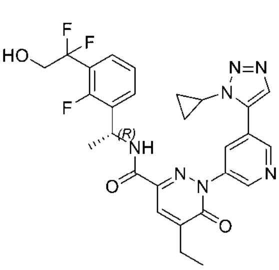
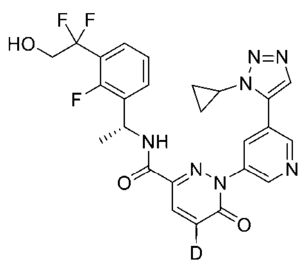

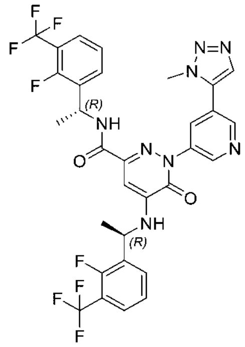
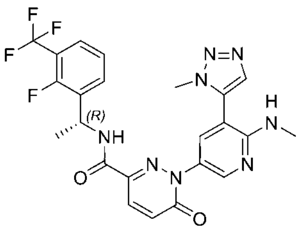
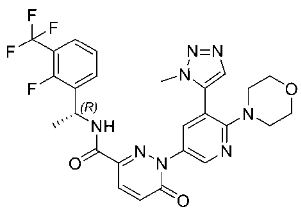
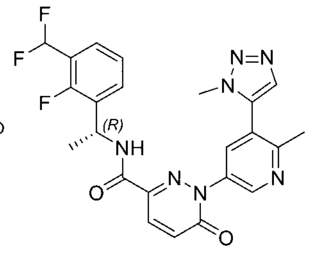

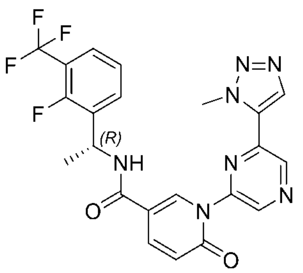
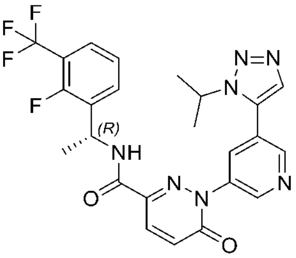
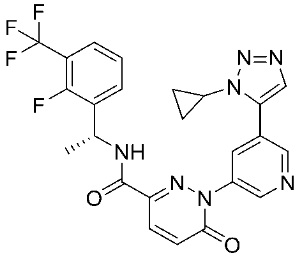
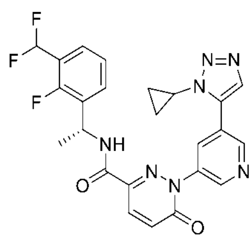

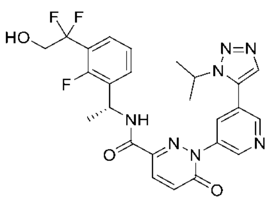
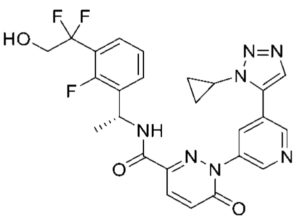
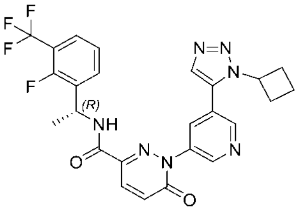
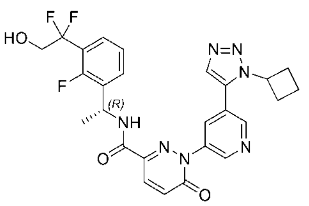

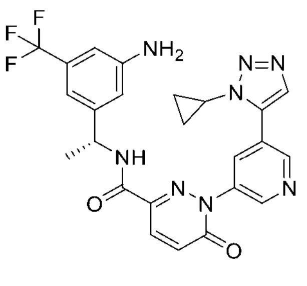
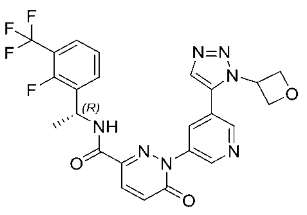
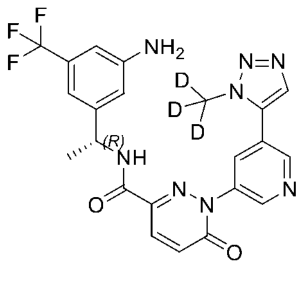
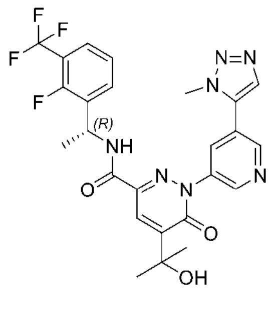

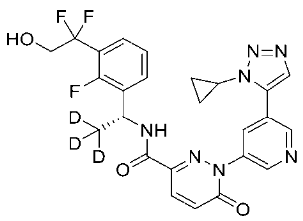
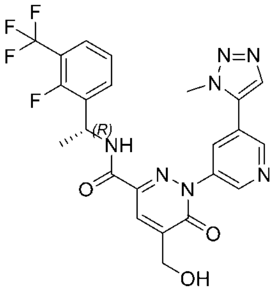
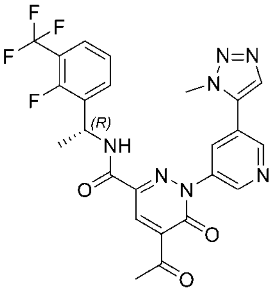
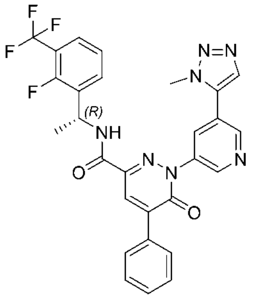

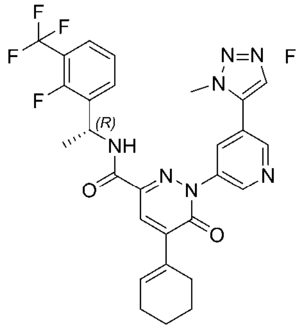
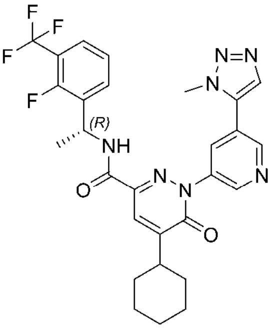
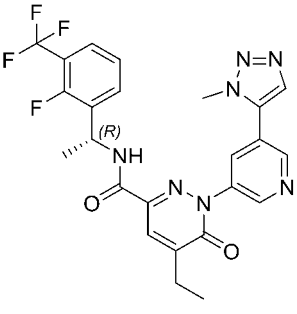
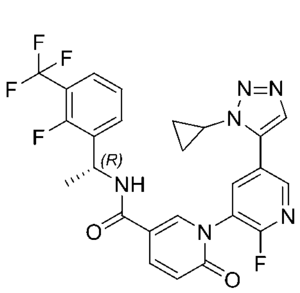

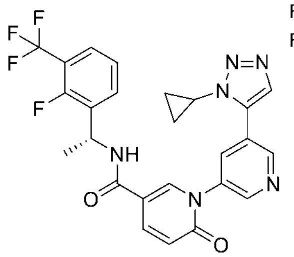
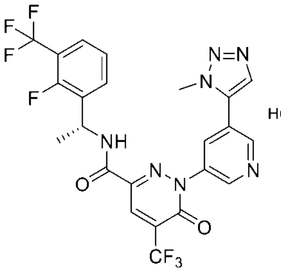
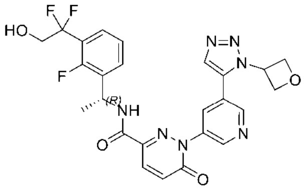
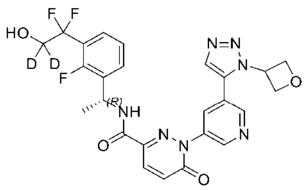

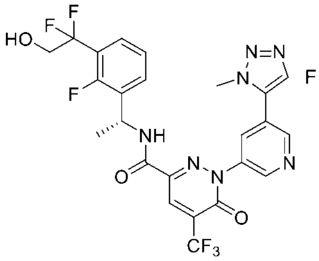
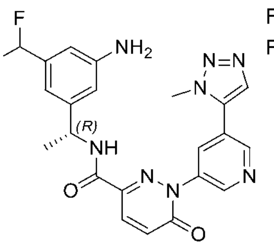
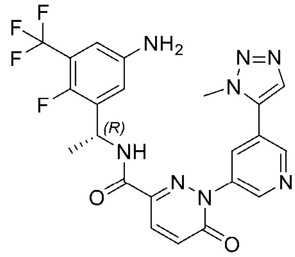
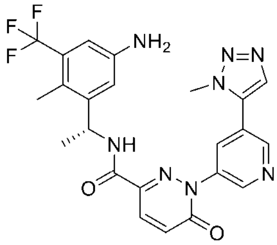

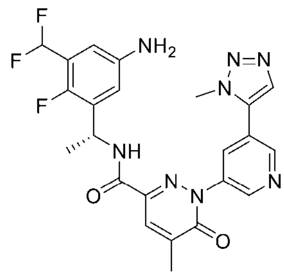
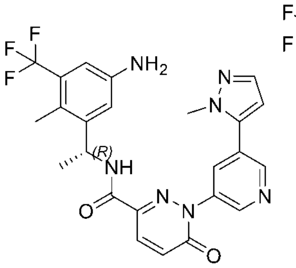
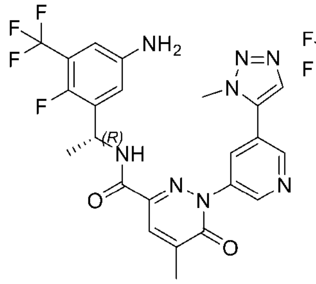
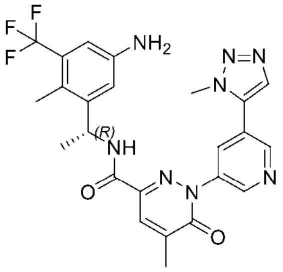

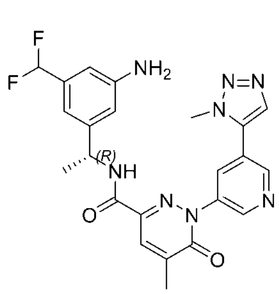
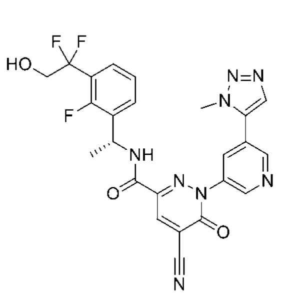
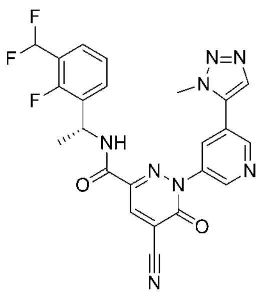
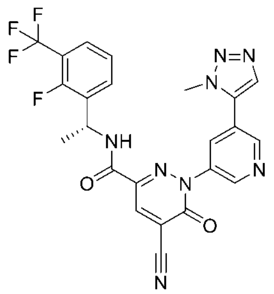

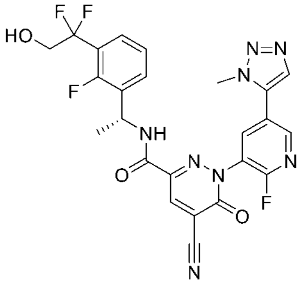
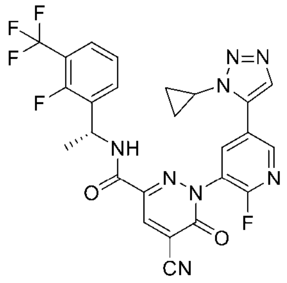
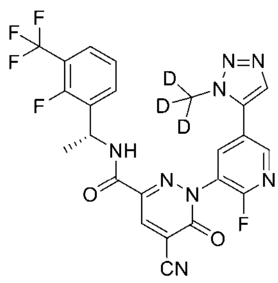
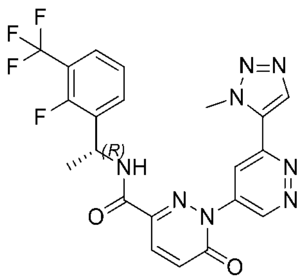

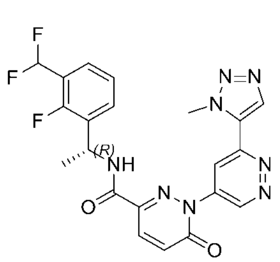
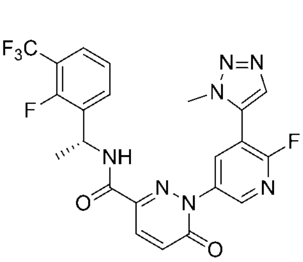
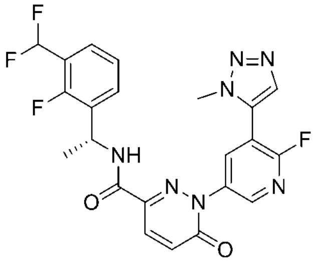
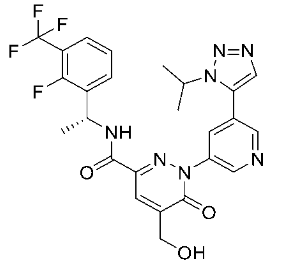

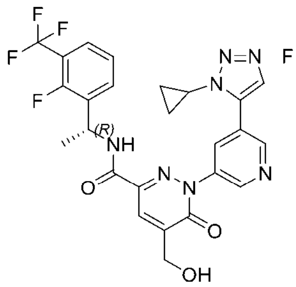
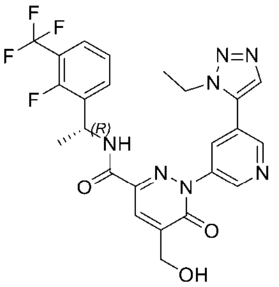
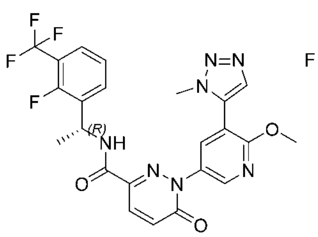
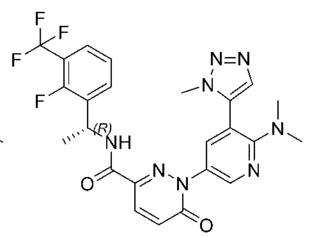

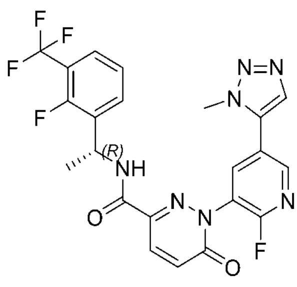
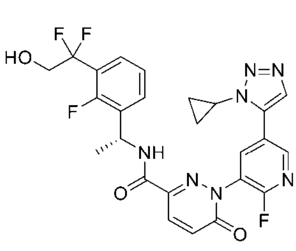
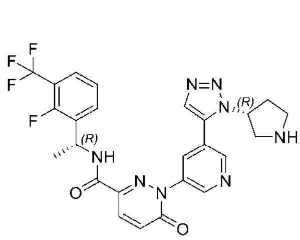
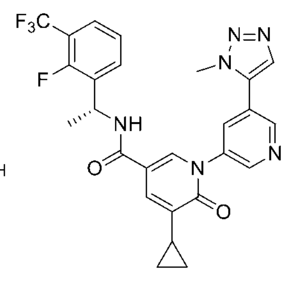

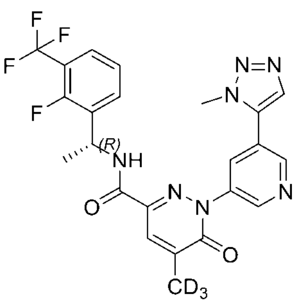
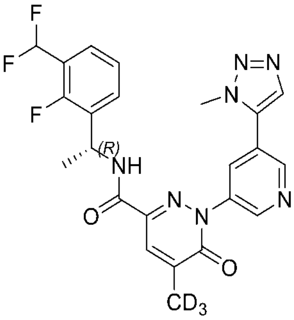
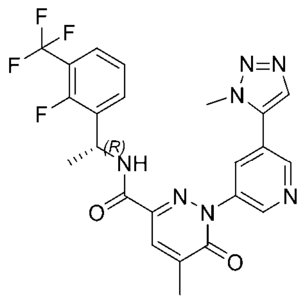
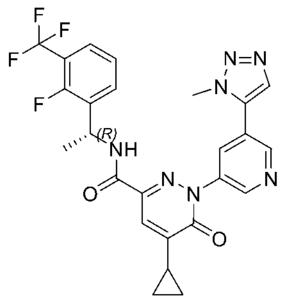

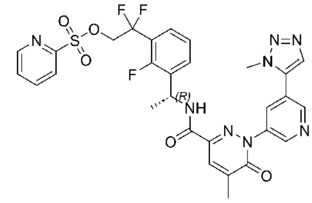
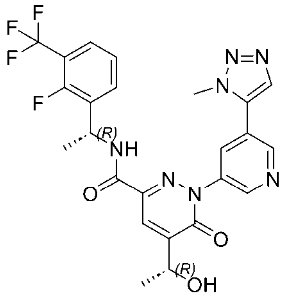
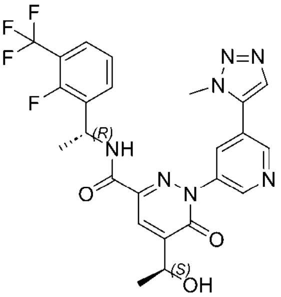
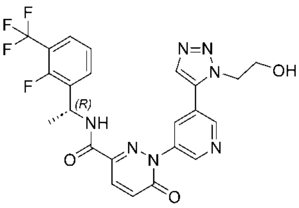

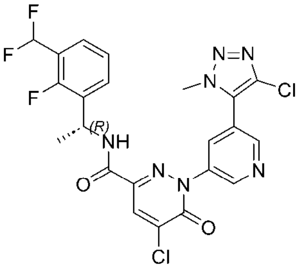
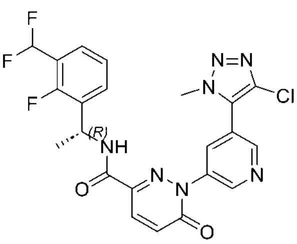
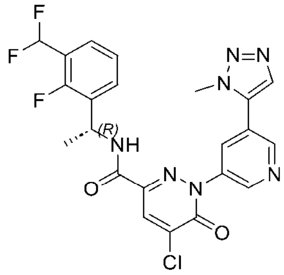
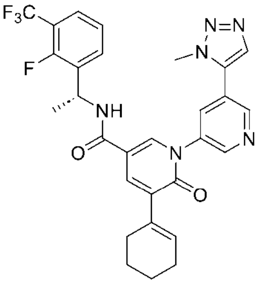

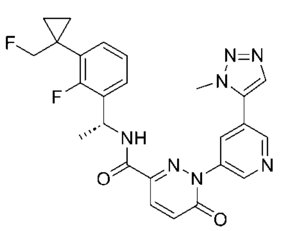
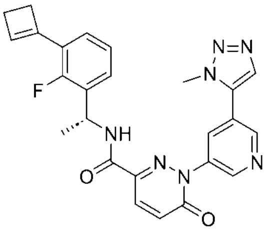
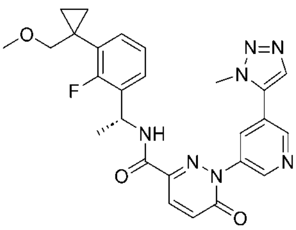
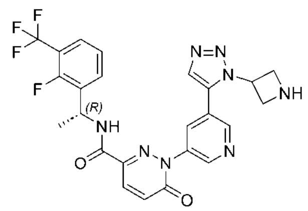

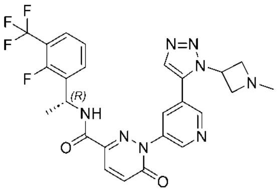
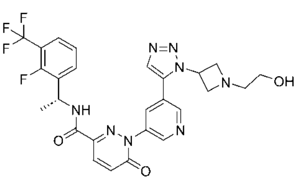
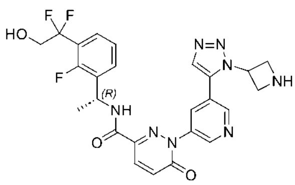
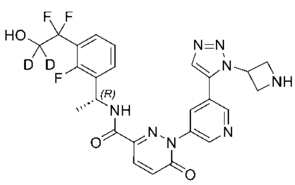

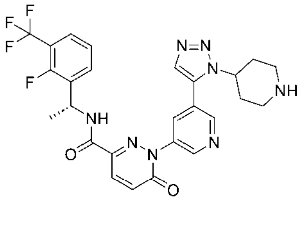
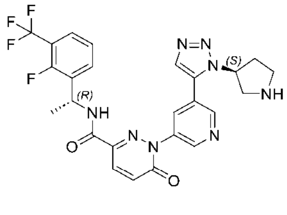
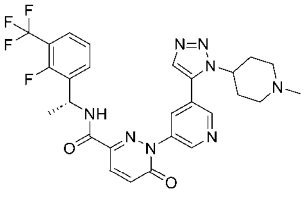
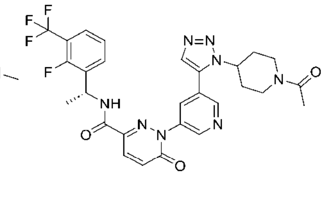

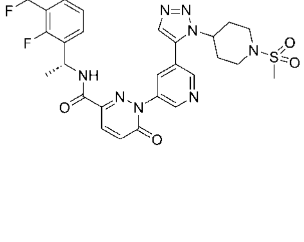
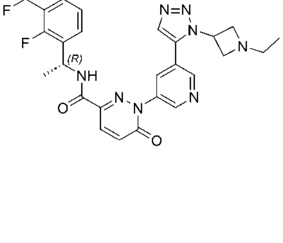
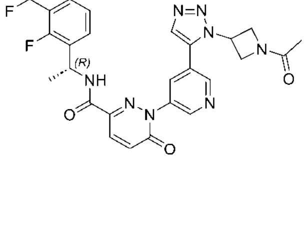
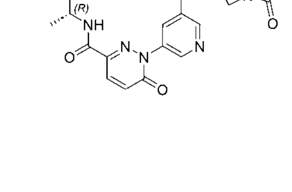

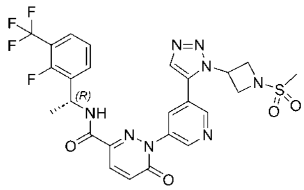 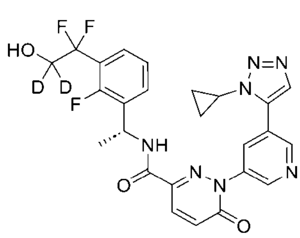 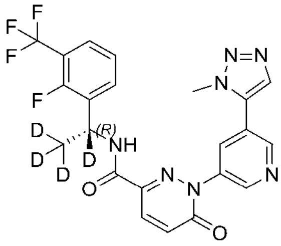 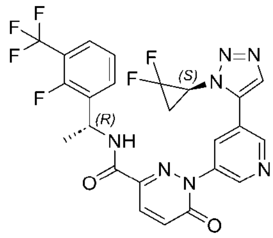

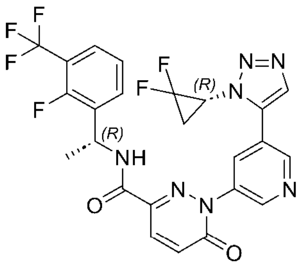 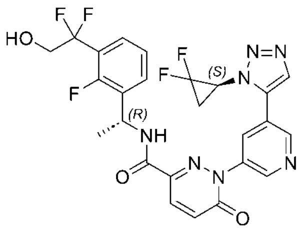 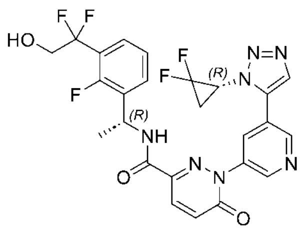 .

**19.** A pharmaceutical composition comprising the compound, or solvate, stereoisomer or pharmaceutically acceptable salt thereof according to any one of claims 1 to 18 as an active ingredient.

**20.** The pharmaceutical composition according to claim 19, **characterized in that** the pharmaceutical composition is for the prevention or treatment of a SOS1 mediated disease.

**21.** The pharmaceutical composition according to claim 20, **characterized in that** the SOS1 mediated disease is cancer or RASopathy.

**22.** The pharmaceutical composition according to claim 21, **characterized in that** the cancer is selected from the group consisting of pancreatic cancer, lung cancer, colorectal cancer, biliary tract cancer, multiple myeloma, melanoma, uterine cancer, cervical cancer, endometrial cancer, thyroid cancer, chronic lymphocytic leukemia, acute myeloid leukemia, bladder cancer, urothelial cancer, gastric cancer, squamous cell carcinoma of the head and neck, diffuse large B cell lymphoma, esophageal cancer, hepatocellular cancer, breast cancer, ovarian cancer, prostate cancer, glioblastoma, kidney cancer and sarcoma.

**23.** The pharmaceutical composition according to claim 21, **characterized in that** the RASopathy is selected from the group consisting of neurofibromatosis type 1, Noonan syndrome, Leopard syndrome, capillary malformation-arteriovenous malformation syndrome, Costello syndrome, CFC syndrome (Cardio-Facio-Cutaneous syndrome), Legius syndrome, and hereditary gingival fibromatosis.

**24.** A method for preventing or treating a SOS 1 mediated disease, comprising administering to a subject the compound, or solvate, stereoisomer or pharmaceutically acceptable salt thereof according to any one of claims 1 to 18.

**25.** Use of the compound, or solvate, stereoisomer or pharmaceutically acceptable salt thereof according to any one of claims 1 to 18 for use in the prevention or treatment of a SOS 1 mediated disease.

**26.** Use of the compound, or solvate, stereoisomer or pharmaceutically acceptable salt thereof according to any one of claims 1 to 18 for the manufacture of a medicament for the prevention or treatment of a SOS1 mediated disease.

[Figure 1]

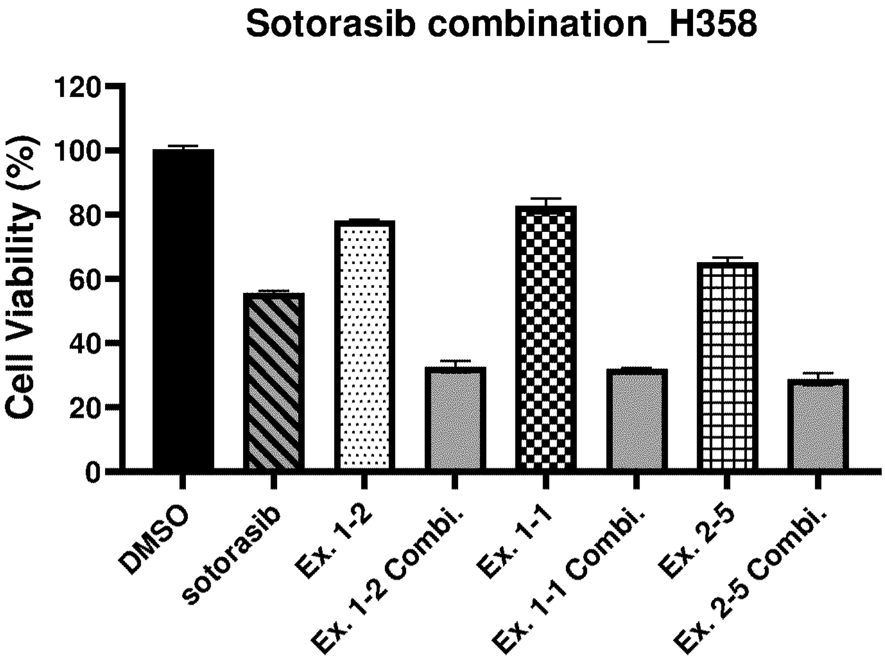


[Figure 2]

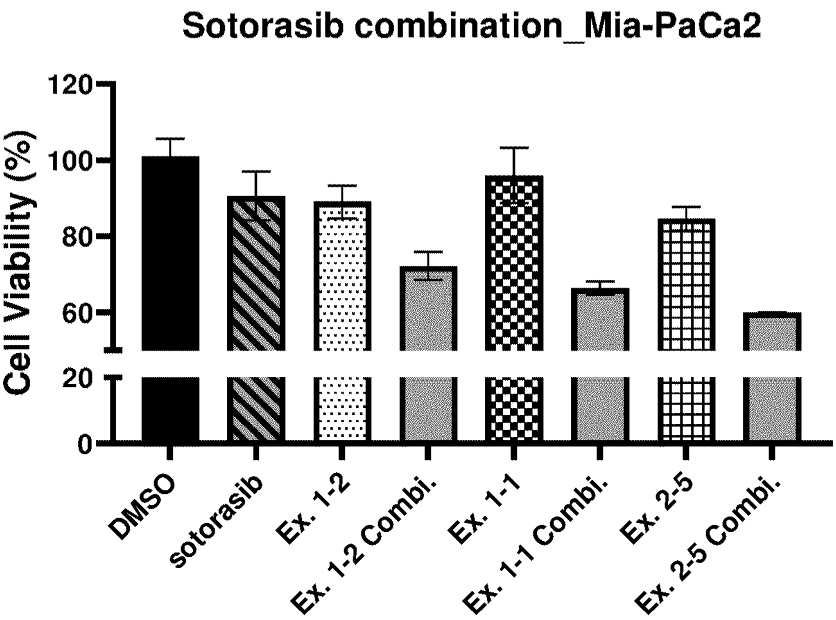

[Figure 3]

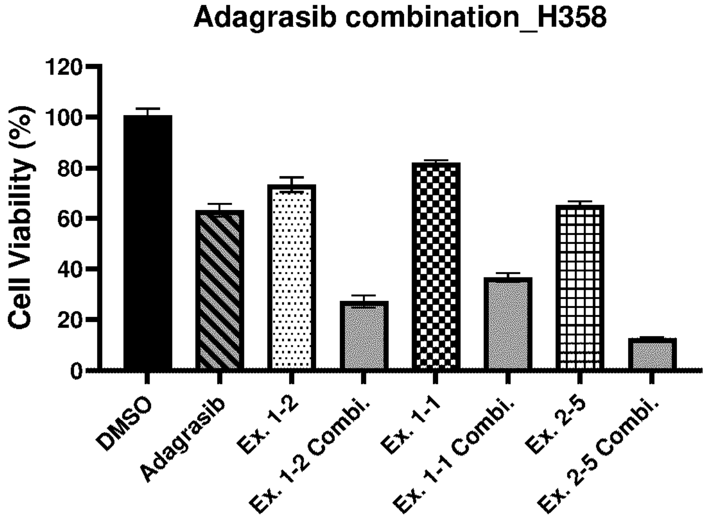


[Figure 4]

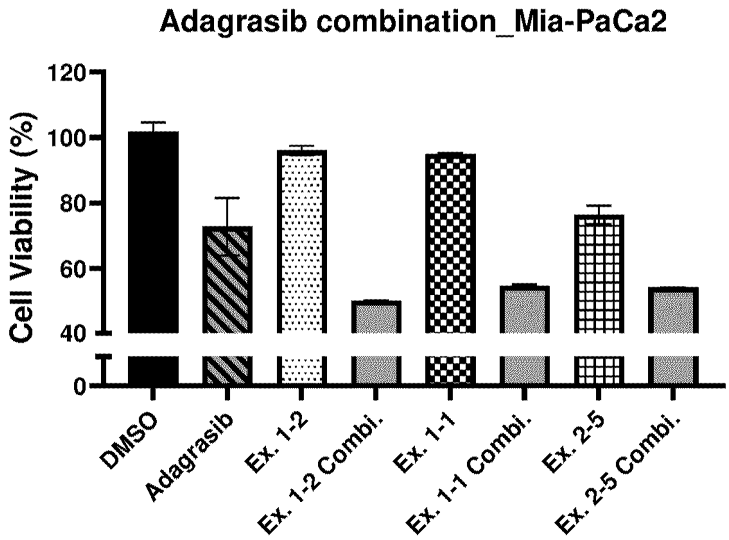


[Figure 5a]

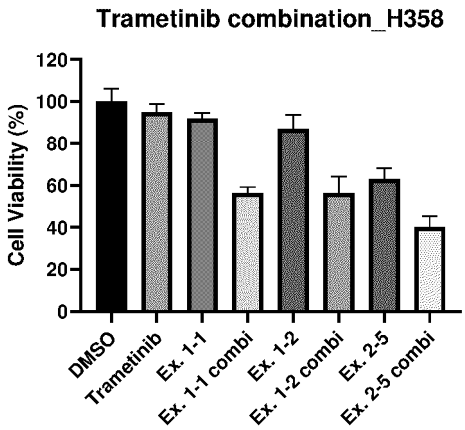

[Figure 5b]

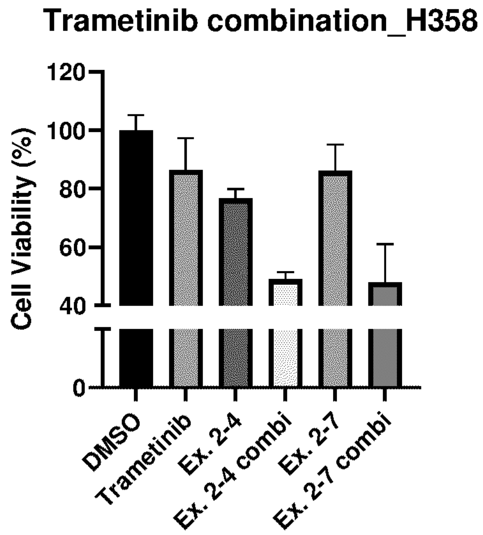


[Figure 6a]

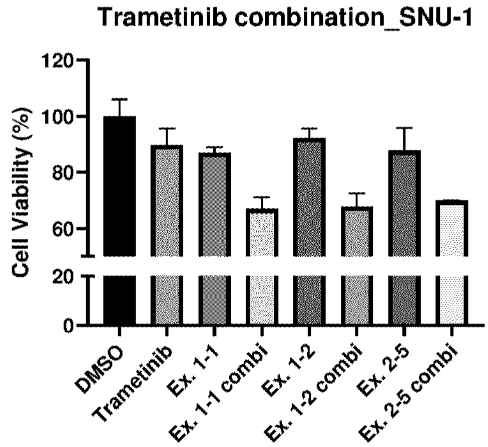


[Figure 6b]

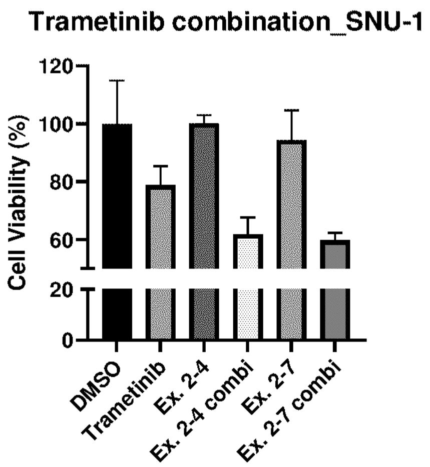

[Figure 7a]

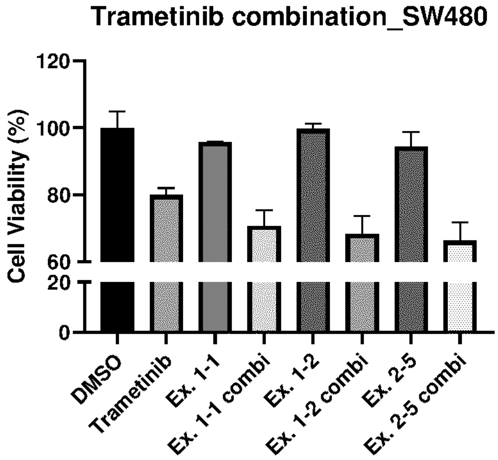


[Figure 7b]

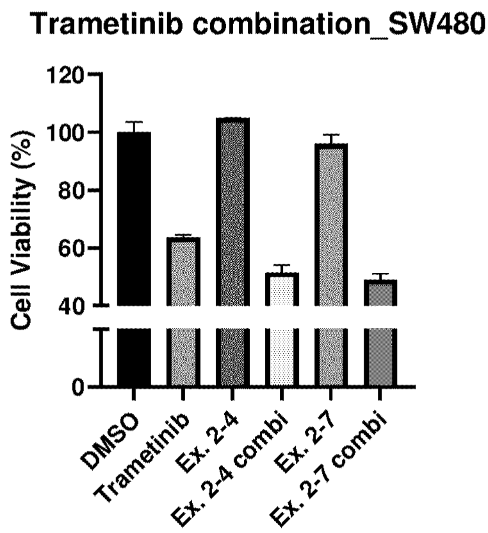


[Figure 8]

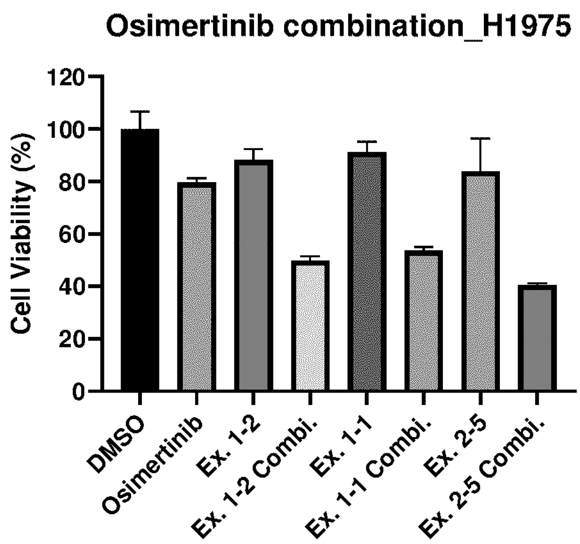

[Figure 9]

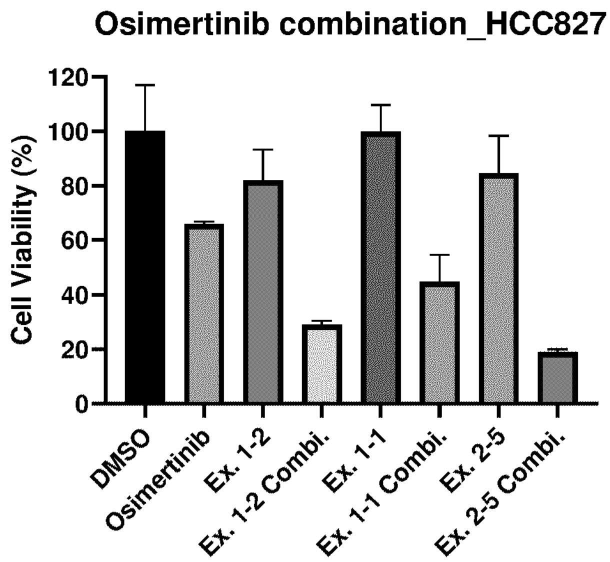


[Figure 10]

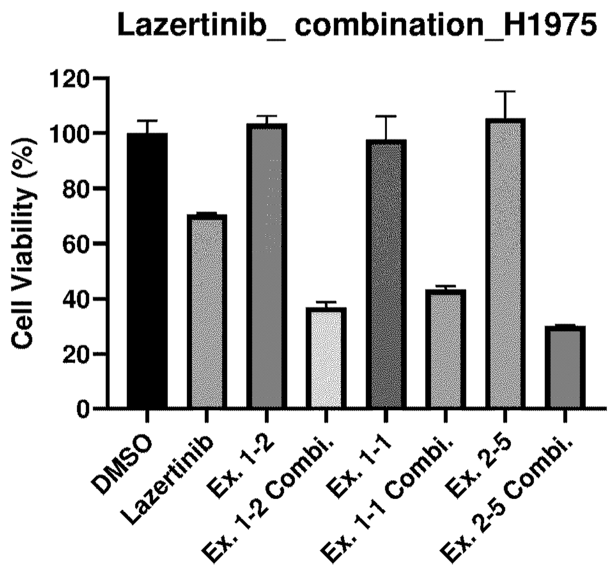

[Figure 11]

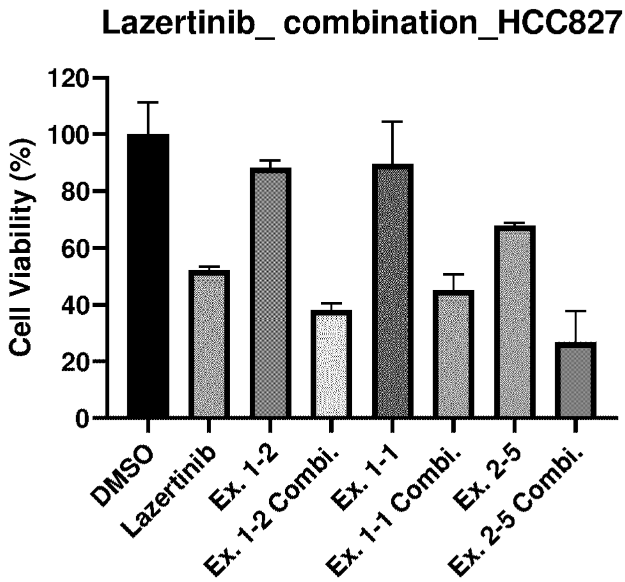


[Figure 12]

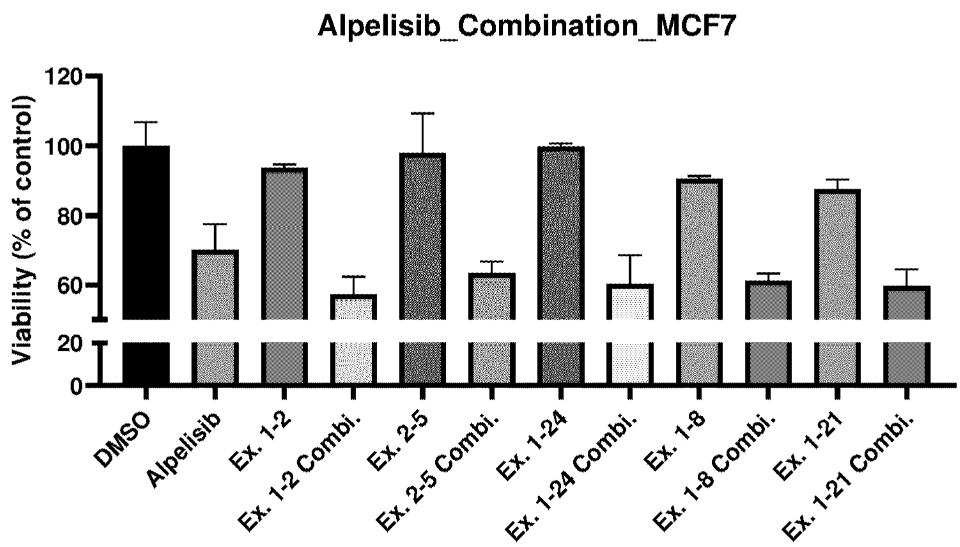

[Figure 13]

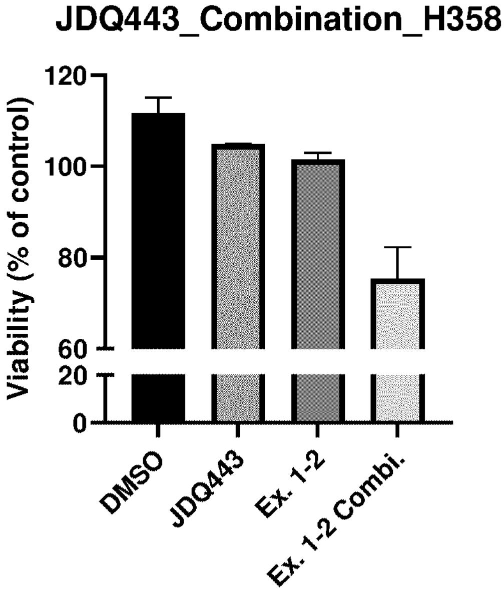


[Figure 14]

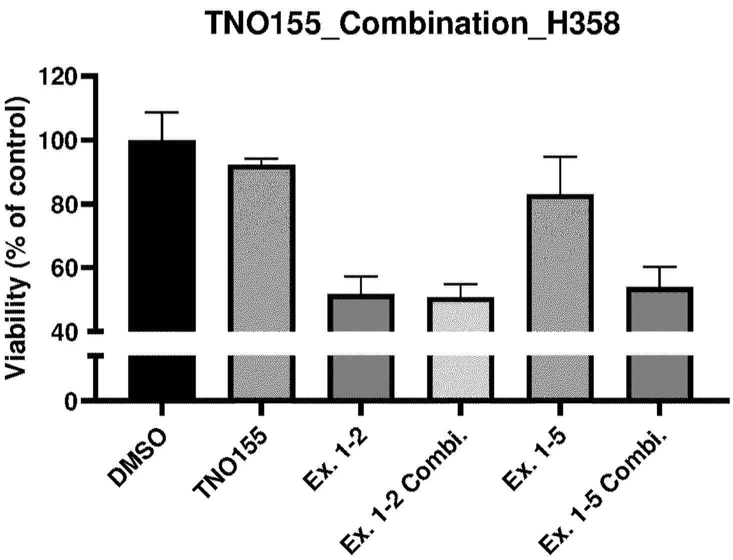

[Figure 15]

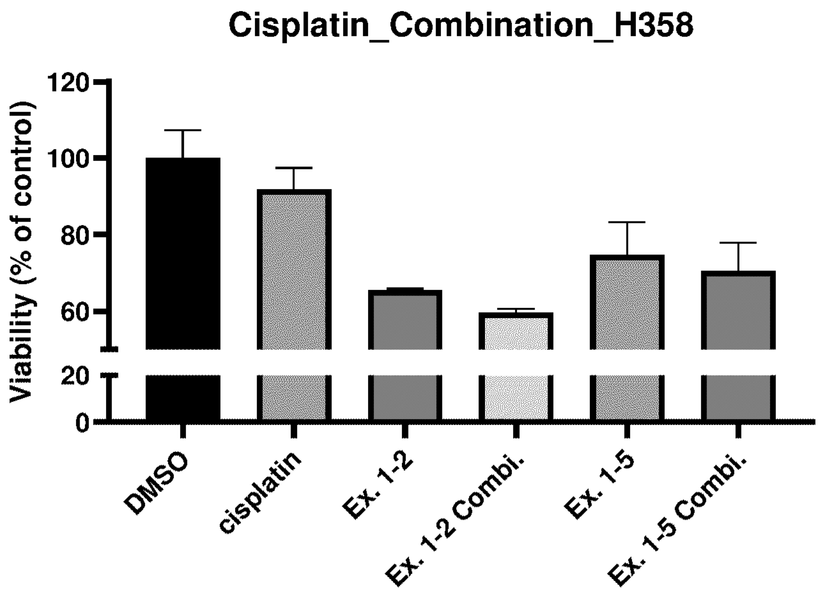


[Figure 16]

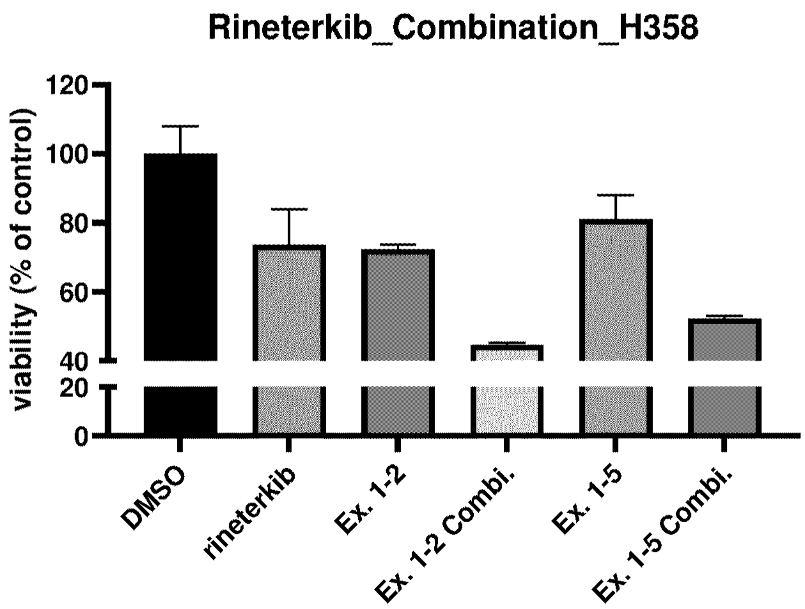

[Figure 17]

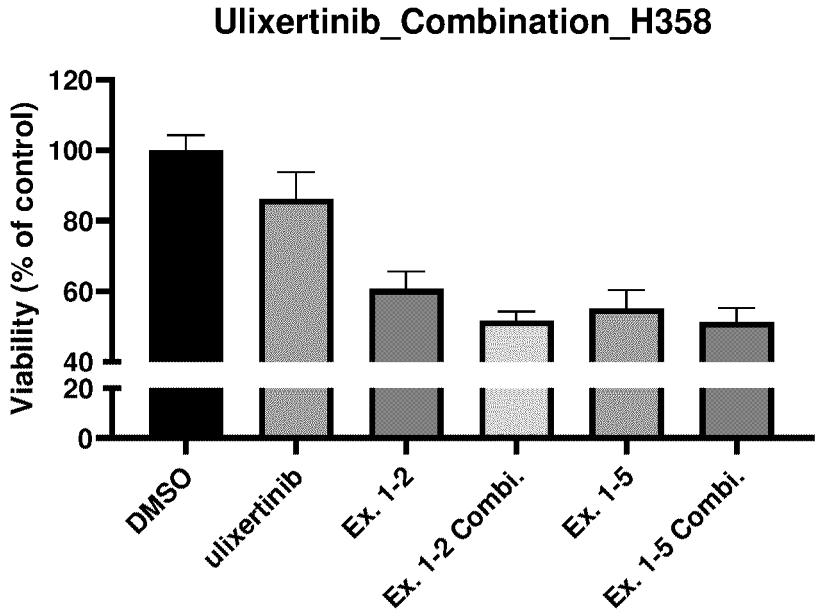


[Figure 18]

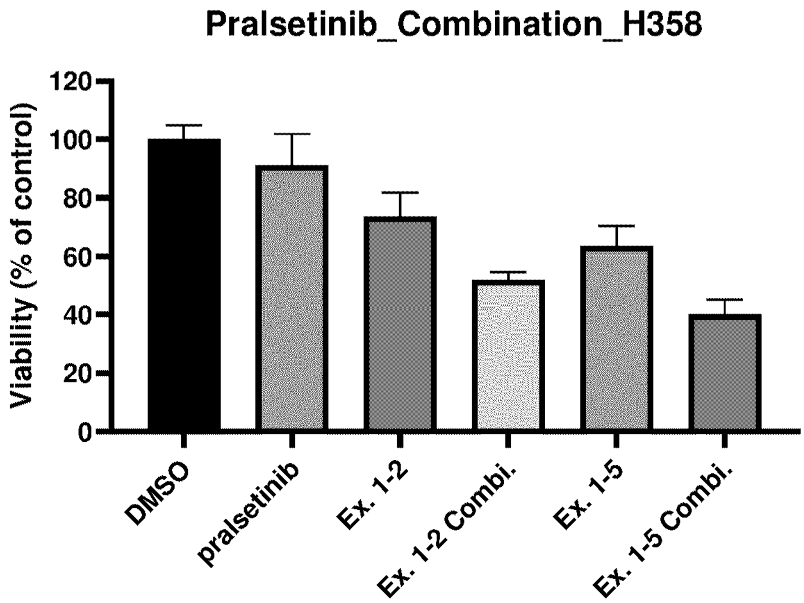

[Figure 19]

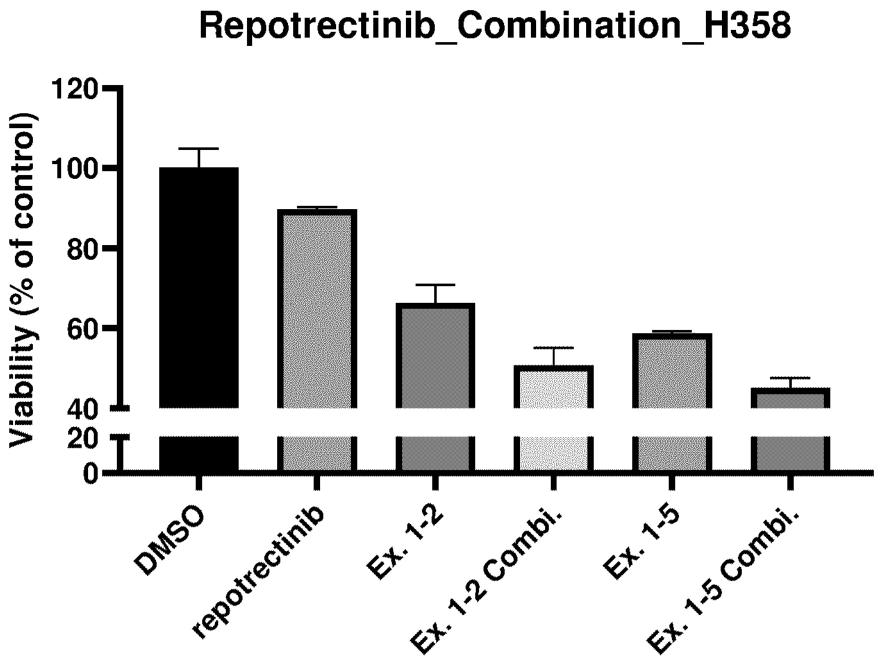


[Figure 20]

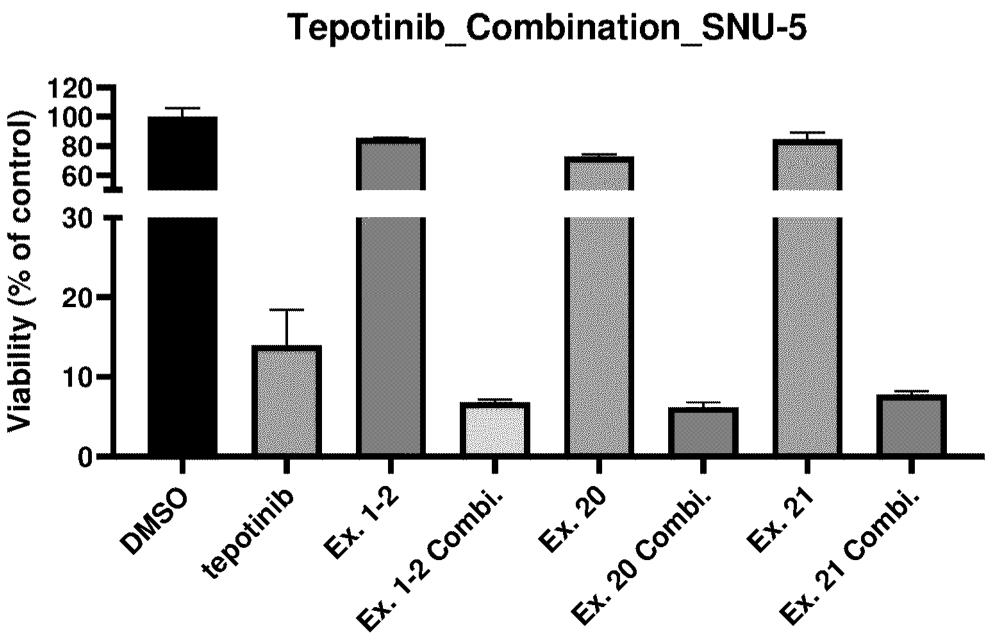

[Figure 21]

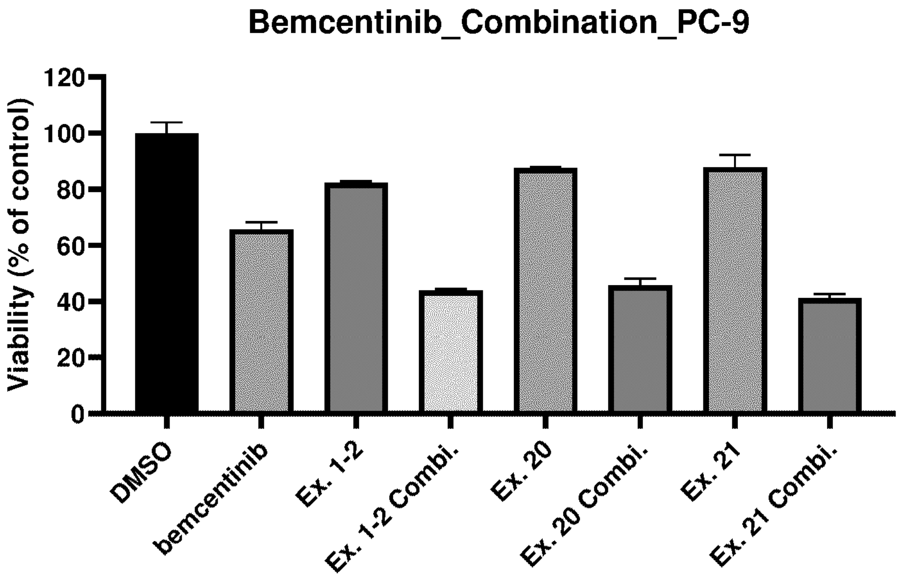


[Figure 22]

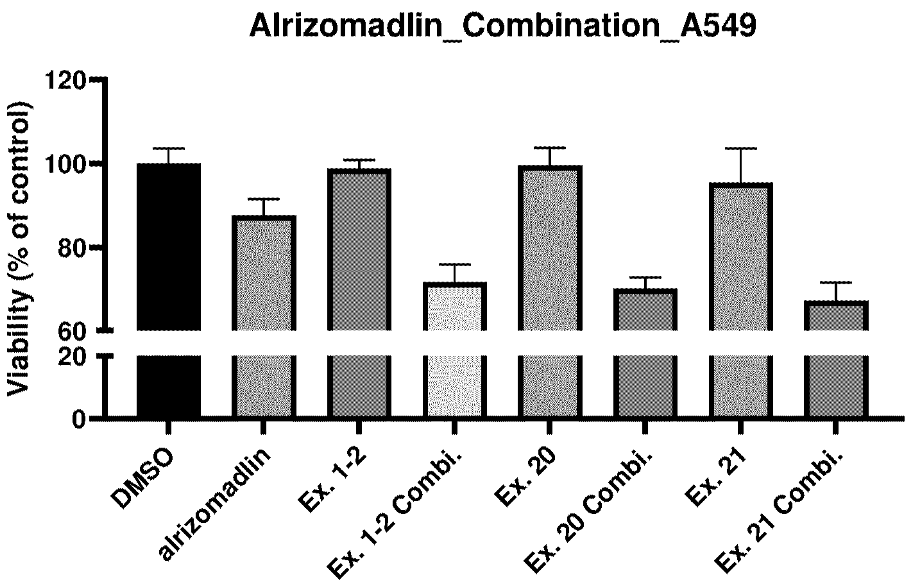

[Figure 23]

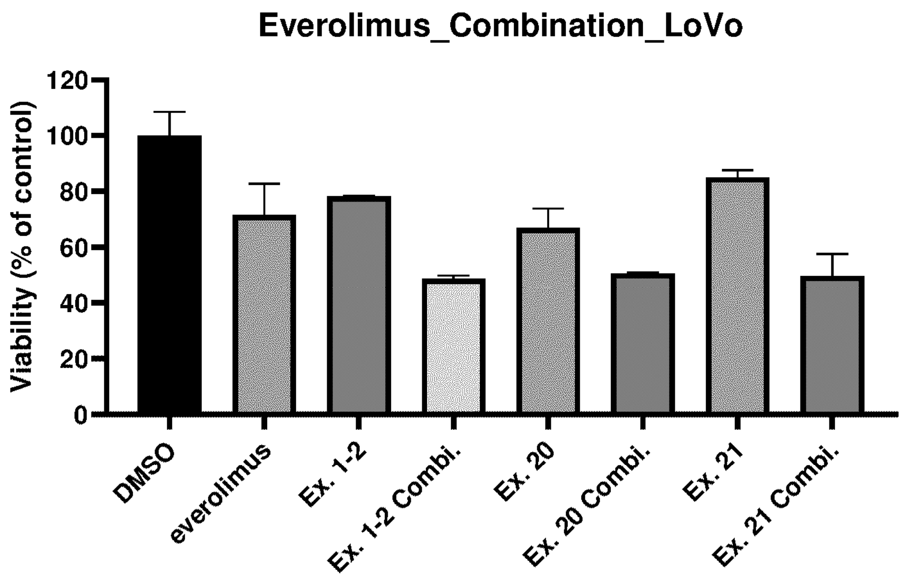


[Figure 24]

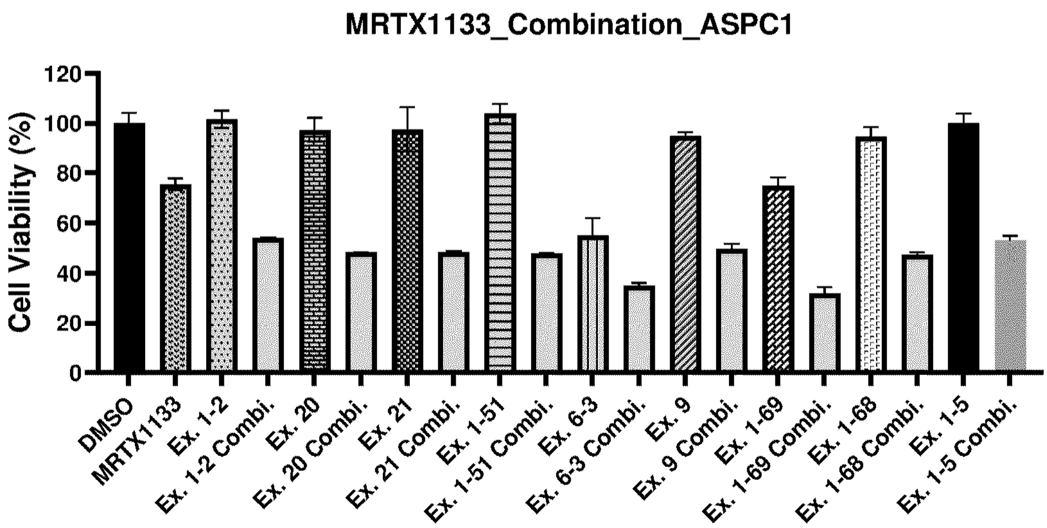


[Figure 25a]

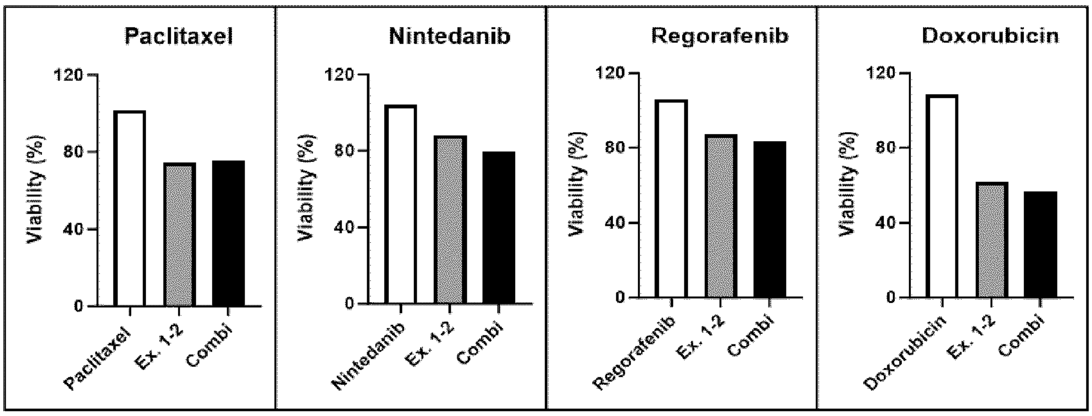

[Figure 25b]

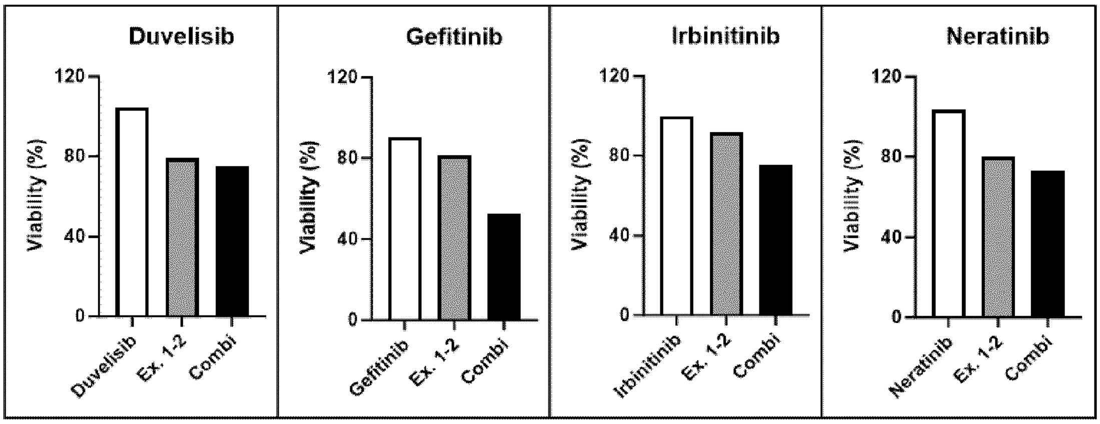


[Figure 25c]

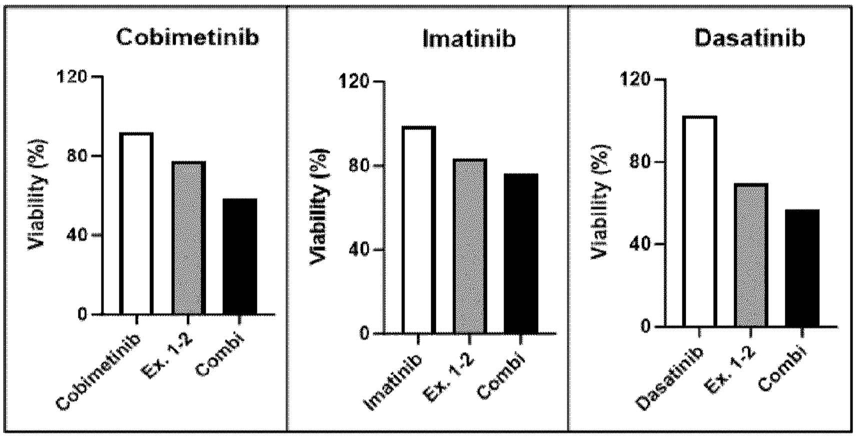


[Figure 26a]

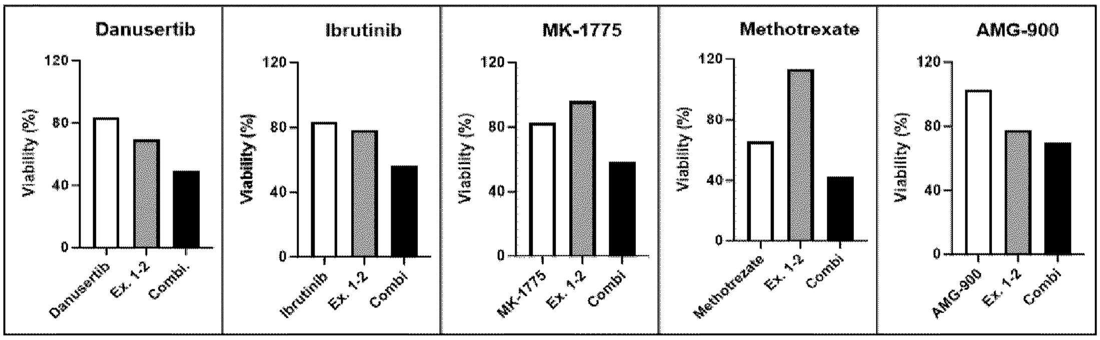


[Figure 26b]

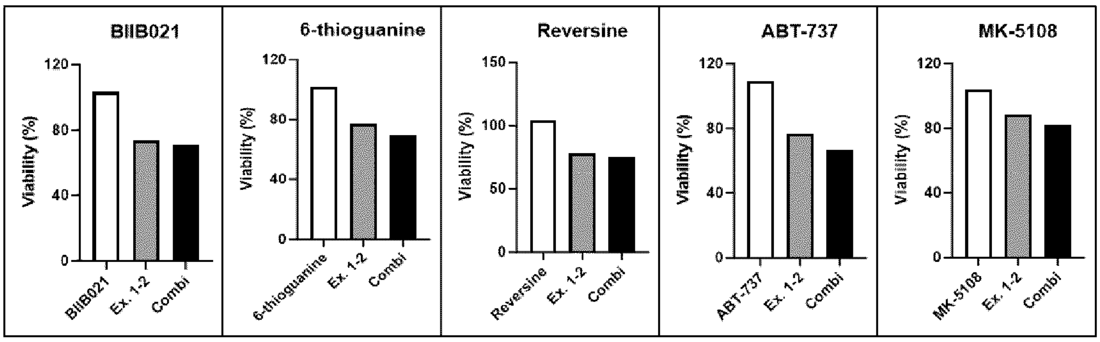

[Figure 26c]

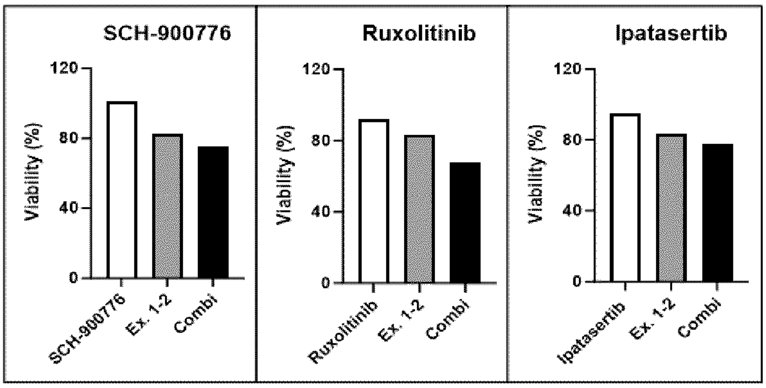
SCH-900776
120
80
40
0
Viability (%)
SCH-900776
Ex. 1-2
Combi
Ruxolitinib
120
80
40
0
Viability (%)
Ruxolitinib
Ex. 1-2
Combi
Ipatasertib
120
80
40
0
Viability (%)
Ipatasertib
Ex. 1-2
Combi

## INTERNATIONAL SEARCH REPORT

International application No. **PCT/KR2024/095359**

**A. CLASSIFICATION OF SUBJECT MATTER**

**C07D 401/14**(2006.01)i; **A61K 31/501**(2006.01)i; **A61K 31/444**(2006.01)i; **A61P 35/00**(2006.01)i

According to International Patent Classification (IPC) or to both national classification and IPC

**B. FIELDS SEARCHED**

Minimum documentation searched (classification system followed by classification symbols)

C07D 401/14(2006.01); A61K 31/496(2006.01); C07C 317/32(2006.01); C07C 317/34(2006.01); C07D 413/10(2006.01); C07D 413/14(2006.01); C07D 453/02(2006.01); C07D 471/04(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal), STN (Registry, CAplus) & keywords: SOS1(son of sevenless 1), 억제제 (inhibitor), 아졸릴피리딘 피리다지논 아미드(azolylpyridine pyridazinone amide), RAS 패밀리 단백질(RAS-family protein)

**C. DOCUMENTS CONSIDERED TO BE RELEVANT**

| Category* | Citation of document, with indication, where appropriate, of the relevant passages | Relevant to claim No. |
|---|---|---|
| A | KR 10-2015-0013563 A (CHUGAI SEIYAKU KABUSHIKI KAISHA (CHUGAI PHARMACEUTICAL CO., LTD.)) 05 February 2015 (2015-02-05)<br>See claims 1, 13 and 15; and example 689. | 1-23,25,26 |
| A | WO 2014-031928 A2 (JONES, P. et al.) 27 February 2014 (2014-02-27)<br>See entire document. | 1-23,25,26 |
| A | WO 2005-018557 A2 (PHARMACIA CORPORATION et al.) 03 March 2005 (2005-03-03)<br>See entire document. | 1-23,25,26 |
| A | WO 2013-014170 A1 (AB SCIENCE et al.) 31 January 2013 (2013-01-31)<br>See entire document. | 1-23,25,26 |
| A | WO 2019-122129 A1 (BOEHRINGER INGELHEIM INTERNATIONAL GMBH) 27 June 2019 (2019-06-27)<br>See entire document. | 1-23,25,26 |

☐ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **04 June 2024** | **04 June 2024** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

**INTERNATIONAL SEARCH REPORT**

International application No.
**PCT/KR2024/095359**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **24**
because they relate to subject matter not required to be searched by this Authority, namely:
Claim 24 pertains to a method for treatment of the human body by surgery or therapy (PCT Article 17(2)(a)(i) and PCT Rule 39.1(iv)).

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (July 2022)

**INTERNATIONAL SEARCH REPORT**
**Information on patent family members**

International application No.
**PCT/KR2024/095359**

| Patent document cited in search report | Publication date (day/month/year) | Patent family member(s) | Publication date (day/month/year) |
|---|---|---|---|
| KR 10-2015-0013563 A | 05 February 2015 | AR 090836 A1 | 10 December 2014 |
| | | AU 2013-253539 A1 | 13 November 2014 |
| | | BR 112014026266 A2 | 27 June 2017 |
| | | CA 2871237 A1 | 31 October 2013 |
| | | CN 104379560 A | 25 February 2015 |
| | | EP 2842939 A1 | 04 March 2015 |
| | | EP 2842939 B1 | 30 May 2018 |
| | | HK 1203926 A1 | 06 November 2015 |
| | | JP 6177768 B2 | 09 August 2017 |
| | | MX 2014012991 A | 14 August 2015 |
| | | RU 2014147017 A | 10 June 2016 |
| | | SG 11201406871 A | 27 November 2014 |
| | | TW 201348187 A | 01 December 2013 |
| | | US 2015-0152047 A1 | 04 June 2015 |
| | | US 9695118 B2 | 04 July 2017 |
| | | WO 2013-161851 A1 | 31 October 2013 |
| WO 2014-031928 A2 | 27 February 2014 | EP 2888256 A2 | 01 July 2015 |
| | | US 2014-0073634 A1 | 13 March 2014 |
| | | WO 2014-031928 A3 | 22 May 2014 |
| WO 2005-018557 A2 | 03 March 2005 | AR 046080 A1 | 23 November 2005 |
| | | CL 2004002050 A1 | 03 June 2005 |
| | | GT 200400161 A | 22 July 2005 |
| | | NL 1026826 A1 | 16 February 2005 |
| | | NL 1026826 C2 | 04 January 2007 |
| | | PA 8609201 A1 | 24 May 2005 |
| | | PE 20050868 A1 | 22 November 2005 |
| | | TW 200517109 A | 01 June 2005 |
| | | US 2005-0176775 A1 | 11 August 2005 |
| | | WO 2005-018557 A3 | 04 August 2005 |
| WO 2013-014170 A1 | 31 January 2013 | AR 087354 A1 | 19 March 2014 |
| | | AU 2012-288900 A1 | 16 January 2014 |
| | | AU 2012-288900 B2 | 06 October 2016 |
| | | BR 112014001977 A2 | 21 February 2017 |
| | | CA 2840029 A1 | 31 January 2013 |
| | | CA 2840029 C | 20 July 2021 |
| | | CL 2014000007 A1 | 05 September 2014 |
| | | CN 103717591 A | 09 April 2014 |
| | | CN 103717591 B | 24 August 2016 |
| | | EP 2736904 A1 | 04 June 2014 |
| | | EP 2736904 B1 | 16 March 2016 |
| | | ES 2573831 T3 | 10 June 2016 |
| | | IL 230504 A | 31 March 2014 |
| | | IL 230504 B | 30 April 2019 |
| | | JP 2014-521624 A | 28 August 2014 |
| | | JP 5944503 B2 | 05 July 2016 |
| | | KR 10-1924247 B1 | 22 February 2019 |
| | | KR 10-2014-0041609 A | 04 April 2014 |
| | | MX 2014001079 A | 12 September 2014 |
| | | NZ 618953 A | 29 May 2015 |
| | | RU 2014107463 A | 10 September 2015 |

Form PCT/ISA/210 (patent family annex) (July 2022)

**INTERNATIONAL SEARCH REPORT**
**Information on patent family members**

International application No.
**PCT/KR2024/095359**

| Patent document cited in search report | Publication date (day/month/year) | Patent family member(s) | Publication date (day/month/year) |
|---|---|---|---|
| | | RU 2612972 C2 | 14 March 2017 |
| | | TW 201311676 A | 16 March 2013 |
| | | US 2014-0179698 A1 | 26 June 2014 |
| | | US 9168245 B2 | 27 October 2015 |
| | | ZA 201400465 B | 29 April 2015 |
| WO 2019-122129 A1 | 27 June 2019 | AR 114164 A1 | 29 July 2020 |
| | | AU 2018-390927 A1 | 28 May 2020 |
| | | AU 2018-390927 B2 | 12 January 2023 |
| | | BR 112020010123 A2 | 10 November 2020 |
| | | CA 3085835 A1 | 27 June 2019 |
| | | CL 2020001501 A1 | 13 November 2020 |
| | | CL 2021000907 A1 | 29 October 2021 |
| | | CN 111372932 A | 03 July 2020 |
| | | CN 111372932 B | 21 November 2023 |
| | | CO 2020007218 A2 | 19 June 2020 |
| | | CR 20200312 A | 11 September 2020 |
| | | CR 20210307 A | 27 July 2021 |
| | | DK 3728254 T3 | 06 June 2023 |
| | | EA 202091491 A1 | 13 November 2020 |
| | | EC SP20040257 A | 31 August 2020 |
| | | EP 3728254 A1 | 28 October 2020 |
| | | EP 3728254 B1 | 15 February 2023 |
| | | EP 4219493 A1 | 02 August 2023 |
| | | ES 2944306 T3 | 20 June 2023 |
| | | FI 3728254 T3 | 05 May 2023 |
| | | HR P20230400 T1 | 23 June 2023 |
| | | HU E062076 T2 | 28 September 2023 |
| | | IL 275379 A | 30 July 2020 |
| | | IL 275379 B2 | 01 June 2023 |
| | | JO P20200154 A1 | 18 June 2020 |
| | | JP 2021-506864 A | 22 February 2021 |
| | | JP 7189956 B2 | 14 December 2022 |
| | | KR 10-2020-0111163 A | 28 September 2020 |
| | | LT 3728254 T | 10 May 2023 |
| | | MA 51290 A | 31 March 2021 |
| | | MX 2020006438 A | 17 September 2020 |
| | | PE 20210163 A1 | 26 January 2021 |
| | | PL 3728254 T3 | 12 June 2023 |
| | | PT 3728254 T | 02 May 2023 |
| | | RS 64167 B1 | 31 May 2023 |
| | | SA 520412278 B1 | 17 August 2022 |
| | | SG 11202005881 A | 29 July 2020 |
| | | SI 3728254 T1 | 30 June 2023 |
| | | TW 201938557 A | 01 October 2019 |
| | | TW I810230 B | 01 August 2023 |
| | | UA 126173 C2 | 25 August 2022 |
| | | US 10829487 B2 | 10 November 2020 |
| | | US 11814380 B2 | 14 November 2023 |
| | | US 2019-0194192 A1 | 27 June 2019 |
| | | US 2021-0009588 A1 | 14 January 2021 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- *J. Med. Chem.*, 2021, vol. 64 (10), 6569-6580
- *Current Opinion in Chemical Biology*, 2021, vol. 62, 109-118